# EUROPEAN PATENT APPLICATION

(11) **EP 4 813 463 A2**
(43) Date of publication of application: **30.09.2026**
(21) Application number: 26198580.8
(22) Date of filing: 14.06.2024
(51) Int. Cl.: A61P 37/00

(54) **TYK2 INHIBITORS AND COMPOSITIONS AND METHODS THEREOF**

(30) Priority: 15.06.2023 WO PCT/CN2023/100518; 11.12.2023 WO PCT/CN2023/137938; 04.02.2024 WO PCT/CN2024/075796; 11.03.2024 WO PCT/CN2024/081039
(62) Document number(s) of the earlier application(s) in accordance with Art. 76 EPC: 24822832.2 / 4 727 941
(71) Applicant: Lynk Pharmaceuticals Co. Ltd., Hangzhou, Zhejiang 310018 (CN)
(72) Inventor: LI, Xiaodong, Hangzhou, Zhejiang 310018 (CN); SU, Lin, Hangzhou, Zhejiang 310018 (CN); WAN, Zhaokui, Hangzhou, Zhejiang 310018 (CN); GREWAL, Gurmit, Hangzhou, Zhejiang 31008 (CN); VAZQUEZ, Michael Lawrence, Hangzhou, Zhejiang 310018 (CN); LIN, Weiyu, Hangzhou, Zhejiang 310018 (CN)
(74) Representative: J A Kemp LLP

(57) **Abstract**

The invention provides a novel class of therapeutic agents that are safe and effective TYK2 inhibitors and pharmaceutical compositions of these compounds and methods of preparation and use thereof against various TYK2-mediated diseases and disorders.

## Description

### Priority Claims and Related Patent Applications

This application claims the benefit of priority to PCT International application Nos. PCT/CN2023/100518, filed June 15, 2023; PCT/CN2023/137938, filed December 11, 2023; PCT/CN2024/075796, filed February 4, 2024; and PCT/CN2024/081039, filed March 11, 2024, the entire content of each of which is incorporated herein by reference for all purposes.

### Technical Fields of the Invention

The invention generally relates to novel compounds and methods for their therapeutic use. More particularly, the invention provides a novel class of tyrosine kinase 2 inhibitors as well as pharmaceutical compositions of these compounds and methods of preparation and use thereof against various diseases and conditions.

### Background of the Invention

Janus kinase (JAK) is a family of intracellular, nonreceptor tyrosine kinases that transduce cytokine-mediated signals via the Janus kinase - Signal Transduction Activators of Transcription (JAK-STAT) pathway. There are four members in the JAK family of enzymes in humans, *i.e*., JAKl, JAK2, JAK3 and tyrosine kinase 2 (TYK2). The family is defined by the presence of two adjacent kinase domains, JH1 and JH2, of which JH1 performs the phosphorylation involved in pathway activation whereas JH2 regulates JH1 function. (Thomas, et al., 2015 British Journal of Cancer 113, 365-371.)

These cytoplasmic tyrosine kinases are associated with membrane cytokine receptors such as common gamma-chain receptors and the glycoprotein 130 (gp130) transmembrane proteins. (Murray, et al. 2007 Immunol. 178(5):2623-2629.) About 40 cytokine receptors signal through combinations of these four JAKs and their 7 downstream substrates: the STAT family members. (Ghoreschi et al. 2009 Immunol Rev. 228(1):273-287.)

Selective inhibition of TYK2 can be utilized to treat a variety of autoimmune inflammatory diseases, such as psoriasis, psoriatic arthritis, alopecia areata, eczema, ankylosing spondylitis (AS), vitiligo, atopic dermatitis, discoid lupus erythematosus (DLE), subacute cutaneous lupus erythematosus (SCLE), systemic lupus erythematosus (SLE), Sjogren's syndrome, scleroderma, inflammatory bowel disease (IBD), Crohn's Disease (CD), rheumatoid arthritis (RA), diabetes, type I diabetes, kidney fibrosis, chronic kidney diseases such as diabetic nephropathy, polycystic kidney disease, HIV-associated nephropathy as well as cancer such as T-cell acute lymphoblastic leukemia (T-ALL) and cutaneous T-cell lymphomas (CTCL). (Ellinghaus, D. et al. 2012 Am. J. Hum. Genet. 90:636-647; Graham, D. et al. 2007 Rheumatology (Oxford). 46:927-930; Eyre, S. et al. 2012 Nat. Genet.,44:1336-1340*;* Frank C. Brosius III. et al. 2015 Curr Opin Nephrol Hypertens. 24(1): 88-95; Keiichiro Mine. et al. 2024 Nature Communications. 15:1337-1350; Calliope A. Dendrou. et al. 2016 Sci Transl Med. 8(363):149-180; Tao, J.H. et al. 2011 Mol. Biol. Rep. 38:4663-4672).

TYK2 plays a crucial role in the immune system, particularly in mediating inflammatory responses. Primarily studied in the context of immune-related disorders, emerging research indicates its involvement in various brain diseases. In multiple sclerosis (MS) patients, TYK2 regulates T cell activation and cytokine production, particularly interleukin-12 (IL-12) and interferon-alpha (IFN-α), which are crucial for immune responses. Th17 cells have been identified in active lesions in the brain from MS patients. Dysregulation of TYK2 can exacerbate inflammation in MS, contributing to central nervous system damage (Beecham, et al. 2013 Nature genetics. 45(11): 1353-1360; Couturier, N. et al. 2011 Brain. 134:693-703; Murphy, C. A. et al. 2003 Nature, 421:744-748;); TYK2 has been identified as a potential risk factor for AD (Alzheimer's Disease), a neurodegenerative disorder characterized by cognitive decline and neuronal loss. Research indicates that TYK2 may modulate microglial activation and neuroinflammation, processes implicated in the progression of AD pathology (Shi et al. 2019 J Exp Med. 216 (11):2546-2561); TYK2 has been linked to PD (Parkinson's Disease), a progressive neurodegenerative disorder primarily affecting movement. Studies suggest that TYK2 may influence neuroinflammation and dopaminergic neuron degeneration, key processes in PD pathology (Qin, et al. 2016, Journal of Neuroscience 36(18): 5144-5159).

The selectivity against other JAK family subtypes is regarded as crucial in order to increase the intended pharmacological effects and to reduce side effects. Identifying kinase inhibitors with a high degree of TYK2 selectivity has posed a significant challenge partly due to the high sequence homology of the active site among the JAK family kinases. TYK2 specificity is critical for clinical application of TYK2 kinase inhibitors, because Tyk2 knockout mice are viable with normal blood cell counts, whereas deficiency of JAK3 results in severe combined immunodeficiency in mice, and JAK1 or JAK2 knockout mice show perinatal lethality. (Ghoreschi, et al. 2009 Immunol Rev. 228:273-287; Karaghiosoff, et al. 2000 Immunity. 13:549-560; Shimoda, et al. 2000 Immunity. 13:561-571.) Genetic evidence suggests that pharmacological inhibition of TYK2 should not result in acute toxicity in human patients, but careful monitoring for viral or mycobacterial infections would be warranted in patients treated for prolonged periods. (Akahane, et al. 2017 Br J Haematol. 177(2): 271-282.)

The ability of a drug to penetrate the blood-brain barrier (BBB) is crucial for treating central nervous system (CNS) diseases. The BBB serves as a highly selective barrier, regulating the passage of substances between the bloodstream and the brain to maintain brain homeostasis. However, this barrier also poses a significant challenge for drug delivery to the brain. For effective treatment of CNS disorders such as Alzheimer's disease, Parkinson's disease, and brain tumors, drugs must be able to cross the BBB to reach their targets within the brain. Compounds with poor brain penetrability may require high doses, leading to systemic side effects and reduced therapeutic efficacy. Developing drugs with enhanced brain penetrability involves strategies such as optimizing molecular properties, utilizing drug delivery systems, and designing prodrugs that can be metabolically activated to increase BBB permeability. Additionally, the discovery of transport mechanisms that facilitate the passage of specific molecules across the BBB has opened new avenues for drug delivery. In summary, the ability of a drug to penetrate the BBB is essential for the successful treatment of CNS diseases. Improving brain penetrability enhances drug efficacy, reduces side effects, and holds promise for the development of more effective therapies for challenging neurological conditions.

Gastrointestinal restricted or enriched Janus kinase (JAK) inhibitors represent a specialized class of medications designed to target the JAK-STAT signaling pathway specifically within the gastrointestinal tract. These inhibitors offer several advantages in the treatment of inflammatory bowel disease (IBD) and Crohn's disease, including: *Localized Action: By* targeting JAK inhibition specifically in the gastrointestinal tract, these inhibitors minimize systemic exposure, potentially reducing the risk of systemic side effects associated with broader JAK inhibitors. *Enhanced Efficacy:* By concentrating the therapeutic effect within the gut, gastrointestinal restricted or enriched JAK inhibitors may provide enhanced efficacy in controlling inflammation and promoting mucosal healing in patients with IBD and Crohn's disease. *Reduced Systemic Side Effects:* The localized action of these inhibitors may lead to a reduced incidence of systemic adverse effects such as infections or hematologic abnormalities, which are associated with systemic JAK inhibitors. *Potential for Lower Doses:* Targeting JAK inhibition to the gastrointestinal tract may allow for lower doses of medication to achieve therapeutic effects, further reducing the risk of systemic side effects. *Combination Therapy:* Gastrointestinal restricted or enriched JAK inhibitors can be used alone or in combination with other therapies for IBD management, offering flexibility in treatment approaches while potentially minimizing systemic exposure to medications.

Solubility is a critical factor in the development of small molecule drugs as it directly impacts their bioavailability, efficacy, and ultimately, their clinical success. Poor solubility can lead to insufficient drug absorption, erratic pharmacokinetics, and reduced therapeutic effectiveness. Consequently, enhancing solubility is a primary focus in drug development to ensure adequate drug delivery and optimal therapeutic outcomes. Several drug candidates have faced setbacks or failures in clinical development due to solubility issues. These cases underscore the importance of addressing solubility early in the drug development process to mitigate risks and optimize the clinical potential of small molecule therapeutics.

An urgent and unmet need exists and challenges remain across broad therapeutic areas for selective TYK2 inhibitors with improved potency and minimal side effects.

### Summary of the Invention

The invention provides novel, selective and potent compounds that are orally and/or topically available and/or suitable for CNS penetrable, gastrointestinal (GI) tract restricted and/or topical administration. These therapeutic agents are safe and effective TYK2 inhibitors and may exhibit fewer and/or lesser side effects than currently available drugs. The invention also provides pharmaceutical compositions of these compounds and methods of their preparation and use.

Disclosed herein are a series of novel TYK2 inhibitors that were specifically designed to fit in the profiles that are potentially suitable for (I) CNS penetrable or (II) oral administrations or (III) GI and/or skin topical uses. For compounds designed for oral administration, they are potent for TYK2 with an array of selectivity against other JAK kinases and JAK1 JH2 domain with good overall drug profiles. For the compounds that are potentially suitable for GI restricted use, their properties such as Caco-2 data, solubility, PK *etc.* are suitable for limiting them to the intestinal action site. For the compounds that are potentially suitable for topical uses, they are designed to have good skin permeability and high retention amount in epidermis and dermis. The new class of inhibitors exhibits exceptional potency profiles with TYK2 IC50 values in the low picomolar or nanomolar range. These therapeutic agents are safe and effective TYK2 inhibitors and exhibit fewer and/or lesser side effects than currently available drugs. The invention also provides pharmaceutical compositions of these compounds and methods of their preparation and use.

In one aspect, the invention generally relates to a compound having the structural formula (I): 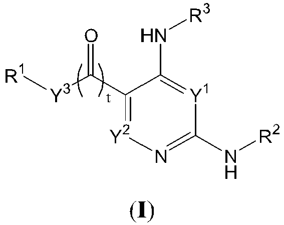 or a pharmaceutically acceptable form or an isotope derivative thereof,
wherein
Y¹ is CH, CF or N;
Y² is CH or N;
Y³ is NR, O, CH₂, CD₂, CF₂ or O-NH;
t is 0 or 1;
R¹ is H, F, CD₃, or C₁-C₃ alkyl, provided that R¹ is not F when Y³ is N, O or O-NH;
R² is
   R^{2'}, wherein R^{2'} is C₁-C₆ alkyl, C₃-C₆ cycloalkyl, C₅-C₇ spirocycloalkyl, or C₃-C₆ heterocycloalkyl, each substituted with 0-2 R^{2a}, wherein R^{2a} is selected from the group consisting of halogen, CN, OR, NRR', alkyl, cycloalkyl, heterocyclic;
   an aryl or heteroaryl group, each substituted with 0-2 R^{2a};
   (C=O)R^{2b}; or
   (C=O)NHR^{2b};
R³ is 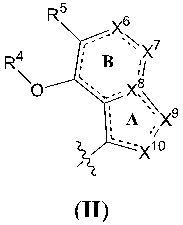 wherein
X⁶ is CR⁶ or N;
X⁷ is CR⁷ or N;
X⁸ is C or N;
X⁹ is CR⁹, O, S, N or NR⁹;
X¹⁰ is CR¹⁰, O, S, N or NR¹⁰; and
   wherein each of Ring A and Ring B is independently an aryl or heteroaryl group;
R^{2b} is a C₁₋₆ alkyl or C₃₋₆ cycloalkyl, C₅₋₇ spirocycloalkyl, aryl or heteroaryl, each substituted with 0-4 R^{2c};
R^{2c} at each occurrence is independently halo, CN, OR, NRR', OCF₃, CF₃, C₁₋₆ alkyl, C₁₋₆ haloalkyl, C₂₋₆ alkenyl, C₂₋₆ alkynyl, wherein said alkyl, haloalkyl, alkenyl, alkynyl, R and R' are substituted with 0-3 R^{2a}; and
R⁴ is a C₁₃ alkyl, substituted with 0-5 R^{4a}, wherein R^{4a} is selected from D, F and Cl;
R⁵ is H, CN, halo, OCH₃, C(=O)OR, NHC(=O)R, NRR', NO₂, C₁₋₆ alkyl, C₃-C₆ cycloalkyl or heterocyclic, wherein said alkyl, cycloalkyl or heterocyclic is substituted with 0-3 R^{5a}, wherein each R^{5a} is independently selected from OH, D, F, Cl, CN, CH₂F, CHF₂, CF₃, OCH₃, OCD₃, OCF₃ and OC(=O)CH₃;
each of R⁶, R⁷, R⁹ and R¹⁰ is independently selected from H, F, Cl, CN, CD₃, CH₂CF₃, CF₃, OR, NRR', C₁-C₃ alkyl and C₃-C₅ cycloalkyl, wherein said alkyl, cycloalkyl, R and R' are substituted with 0-2 R^{2a}; and
each of R and R' is independently H or a C₁-C₆ alkyl or acyl, or R and R', together with the nitrogen atom to which they are bonded, form a 4- to 7-membered ring comprising 0-2 heteroatoms selected from O, NR, S and SO₂.

In certain embodiments of **(I),** *t is* 1, and the compound has the structural formula:

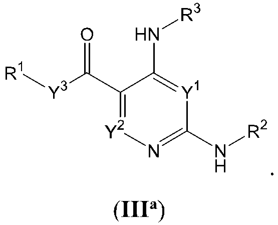

In certain embodiments of (I), *t is* 0, and the compound has the structural formula:

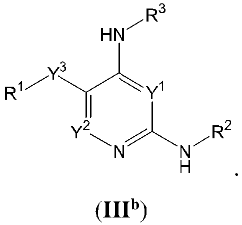

In another aspect, the invention generally relates to a compound having the structural formula (VIII): 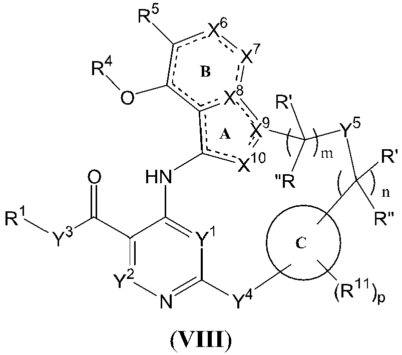 or a pharmaceutically acceptable form or an isotope derivative thereof, wherein
X⁶ is CR⁶ or N;
X⁷ is CR⁷ or N;
X⁸ is C or N;
X⁹ is CR⁹, O, S, N or NR⁹;
X¹⁰ is CR¹⁰, O, S, N or NR¹⁰;
Y¹ is CH, CF or N;
Y² is CH or N;
Y³ is NR, O, CH₂, CD₂, CF₂ or O-NH;
Y⁴ is NR, CH₂ or CF₂;
Y⁵ is NR, CH₂, O, S, SO or SO₂;
*m* is 0, 1, 2 and 3;
*n is* 0, 1, 2 and 3;
*p is 0, 1, 2 and 3;*
each of Ring A and Ring B is independently an aryl or heteroaryl group;
Ring C is a 5- or 6-membered aryl or heteroaryl group;
R¹ is H, F, CD₃, or C₁₋₃ alkyl, provided that R¹ is not F when Y³ is N, O or O-NH;
R⁴ is a C₁₋₃ alkyl, substituted with 0-5 R^{4a}, wherein R^{4a} is selected from D, F and Cl;
R⁵ is H, CN, halo, OCH₃, C(=O)OR, NHC(=O)R, NRR', NO₂, C₁₋₆ alkyl, C₃₋₆ cycloalkyl or heterocyclic, wherein said alkyl, cycloalkyl or heterocyclic is substituted with 0-3 R^{5a}, wherein each R^{5a} is independently selected from OH, D, F, Cl, CN, CH₂F, CHF₂, CF₃, OCH₃, OCD₃, OCF₃ and OC(=O)CH₃;
each of R⁶, R⁷, R⁹, R¹⁰ and R¹¹ is independently selected from H, F, Cl, CN, CD₃, CH₂CF₃, CF₃, OR, NRR', C₁₋₃ alkyl and C₃₋₅ cycloalkyl, wherein said alkyl, cycloalkyl, R and R' are substituted with 0-2 R^{2a};
R^{2a} is selected from F, OCF₃, CF₃, CN, NO₂, OR, NRR' and C₁₋₆ alkyl; and
each of R and R' independently H, C₁-C₆ alkyl or acyl, or R and R' together with the nitrogen or carbon atom to which they are bonded, form a 3- to 6-membered ring comprising 0-2 heteroatoms selected from O, NR, S and SO₂.

In yet another aspect, the invention generally relates to a method for preparing a compound disclosed herein, as exemplified by the synthetic schemes and experimental procedure disclosed herein.

In yet another aspect, the invention generally relates to a pharmaceutical composition comprising a compound disclosed herein, effective to treat or reduce one or more diseases or disorders, in a mammal, including a human, and a pharmaceutically acceptable excipient, carrier, or diluent.

In yet another aspect, the invention generally relates to a unit dosage form comprising a pharmaceutical composition disclosed herein.

In yet another aspect, the invention generally relates to a method for treating, reducing or preventing a disease or disorder, comprising administering to a subject in need thereof a therapeutically effective amount of a compound disclosed herein, wherein the disease or disorder is selected from inflammatory diseases, immune-mediated diseases, cancer, or a related disease or disorder thereof, in a mammal, including a human.

In yet another aspect, the invention generally relates to use of a compound disclosed herein, and a pharmaceutically acceptable excipient, carrier, or diluent, in preparation of a medicament for treating a disease or disorder.

### Brief Description of the Drawings

**FIG.** 1 shows certain exemplary data on changes in mouse weight (A) and DAI score (B). n = 10 mice per group, data are shown as mean values ± SEM are presented, * *p*<0.05, ** *p<0.01,* *** *p<0.005,* **** *p*<0.0001, versus Vehicle, two way-ANOVA, Dunnet's test for multiple comparisons.
FIG. 2 shows certain exemplary data on changes in mouse weight (A) and DAI score (B). n = 8 mice per group, data are shown as mean values ± SEM are presented, * *p*<0.05, ** *p<0.01,* *** *p<0.005,* **** *p*<0.0001, versus Vehicle, two way-ANOVA, Dunnet's test for multiple comparisons.
FIG. 3 shows certain exemplary data on immune cells in whole blood. n = 5 mice per group, data are shown as mean values ± SEM are presented, * *p*<0.05, ** *p<0.01,* *** *p<0.005,* **** *p*<0.0001, versus Vehicle, two way-ANOVA, Dunnet's test for multiple comparisons.
FIG. 4 shows certain exemplary data on CD4⁺T cell count (A) and histology score in colon (B). n = 8 mice per group, data are shown as mean values ± SEM are presented, * *p*<0.05, ** *p<0.01,* *** *p<0.005,* **** *p*<0.0001, versus Vehicle, two way-ANOVA, Dunnet's test for multiple comparisons.

### Definitions

Unless defined otherwise, all technical and scientific terms used herein have the same meaning as commonly understood by one of ordinary skill in the art to which this invention belongs. General principles of organic chemistry, as well as specific functional moieties and reactivity, are described in "Organic Chemistry", Thomas Sorrell, University Science Books, Sausalito: 2006.

The following terms, unless indicated otherwise according to the context wherein the terms are found, are intended to have the following meanings.

Ranges provided herein are understood to be shorthand for all of the values within the range. For example, a range of 1 to 16 is understood to include any number, combination of numbers, or sub-range from the group consisting 1, 2, 3, 4, 5, 6, 7, 8, 9, 10, 11, 12, 13, 14, 15 or 16.

As used herein, "at least" a specific value is understood to be that value and all values greater than that value.

As used herein, "more than one" is understood as 2, 3, 4, 5, 6, 7, 8, 9, 10, 11, 12, 13, 14, 15, 16, 17, 18, 19, 20, 25, 30, 40, 50, 100, etc., or any value therebetween.

In this specification and the appended claims, the singular forms "a," "an," and "the" include plural reference, unless the context clearly dictates otherwise.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein can be modified by the term about.

Unless specifically stated or obvious from context, as used herein, the term "or" is understood to be inclusive.

Any compositions or methods disclosed herein can be combined with one or more of any of the other compositions and methods provided herein.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

The term "comprising", when used to define compositions and methods, is intended to mean that the compositions and methods include the recited elements, but do not exclude other elements. The term "consisting essentially of", when used to define compositions and methods, shall mean that the compositions and methods include the recited elements and exclude other elements of any essential significance to the compositions and methods. For example, "consisting essentially of" refers to administration of the pharmacologically active agents expressly recited and excludes pharmacologically active agents not expressly recited. The term consisting essentially of does not exclude pharmacologically inactive or inert agents, *e*.*g*., pharmaceutically acceptable excipients, carriers or diluents. The term "consisting of", when used to define compositions and methods, shall mean excluding trace elements of other ingredients and substantial method steps. Embodiments defined by each of these transition terms are within the scope of this invention.

Certain compounds of the present invention may exist in particular geometric or stereoisomeric forms. The present invention contemplates all such compounds, including *cis-* and trans-isomers, atropisomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess of either the R- or S-configuration. For optically active compounds, it is often preferred to use one enantiomer to the substantial exclusion of the other enantiomer.

Isomeric mixtures containing any of a variety of isomer ratios may be utilized in accordance with the present invention. For example, where only two isomers are combined, mixtures containing 50:50, 60:40, 70:30, 80:20, 90:10, 95:5, 96:4, 97:3, 98:2, 99:1, or 100:0 isomer ratios are contemplated by the present invention. Those of ordinary skill in the art will readily appreciate that analogous ratios are contemplated for more complex isomer mixtures.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic methods well known in the art, and subsequent recovery of the pure enantiomers.

A mixture of isomers can be separated on the basis of the physicochemical differences of the constituents, into the pure or substantially pure geometric or optical isomers, diastereomers, racemates, for example, by chromatography and/or fractional crystallization.

Definitions of specific functional groups and chemical terms are described in more detail below. When a range of values is listed, it is intended to encompass each value and sub-range within the range. For example, "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

Where substituent groups are specified by their conventional chemical formulae, written from left to right, they equally encompass the chemically identical substituents that would result from writing the structure from right to left, *e.g.,* -C(=O)-O- is equivalent to -O-C(=O)-.

Structures of compounds of the invention are limited by principles of chemical bonding known to those skilled in the art. Accordingly, where a group may be substituted by one or more of a number of substituents, such substitutions are selected so as to comply with principles of chemical bonding and to give compounds that are not inherently unstable and/or would be known to one of ordinary skill in the art as likely to be unstable under ambient conditions (*e.g.,* aqueous, neutral, and several known physiological conditions).

Solvates and polymorphs of the compounds of the invention are also contemplated herein. Solvates of the compounds of the present invention include, for example, hydrates.

As used herein, the term "alkyl" refers to a straight, branched or cyclic hydrocarbon chain radical consisting solely of carbon and hydrogen atoms, containing no unsaturation, having from one to ten carbon atoms (*e.g.,* C₁₋₁₀ alkyl). Whenever it appears herein, a numerical range such as "1 to 10" refers to each integer in the given range; *e.g*., "1 to 10 carbon atoms" means that the alkyl group can consist of 1 carbon atom, 2 carbon atoms, 3 carbon atoms, *etc.,* up to and including 10 carbon atoms, although the present definition also covers the occurrence of the term "alkyl" where no numerical range is designated. In some embodiments, "alkyl" can be a C₁₋₆ alkyl group. In some embodiments, alkyl groups have 1 to 10, 1 to 8, 1 to 6, or 1 to 3 carbon atoms. Representative saturated straight chain alkyls include, but are not limited to, -methyl, - ethyl, -n-propyl, -n-butyl, -n-pentyl, and -n-hexyl; while saturated branched alkyls include, but are not limited to, -isopropyl, -sec-butyl, -isobutyl, -tert-butyl, -isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, and the like. The alkyl is attached to the parent molecule by a single bond. Unless stated otherwise in the specification, an alkyl group is optionally substituted by one or more of substituents which independently include: acyl, alkyl, alkenyl, alkynyl, alkoxy, alkylaryl, cycloalkyl, aralkyl, aryl, aryloxy, amino, amido, amidino, imino, azide, carbonate, carbamate, carbonyl, heteroalkyl, heteroaryl, heteroarylalkyl, heterocycloalkyl, hydroxy, cyano, halo, haloalkoxy, haloalkyl, ester, ether, mercapto, thio, alkylthio, arylthio, thiocarbonyl, nitro, oxo, phosphate, phosphonate, phosphinate, silyl, sulfinyl, sulfonyl, sulfonamidyl, sulfoxyl, sulfonate, urea, -Si(R^{a})₃ , -OR^{a}, -SR^{a}, -OC(O)-R^{a}, -N(R^{a})₂, - C(O)Rₐ, -C(O)OR^{a}, -OC(O)N(R^{a})₂, -C(O)N(R^{a})₂, -N(R^{a})C(O)OR^{a}, -N(R^{a})C(O)R^{a}, - N(R^{a})C(O)N(R^{a})₂, -N(R^{a})C(NR^{a})N(R^{a})₂, -N(R^{a})S(O)ₜN(R^{a})₂ (where t is 1 or 2), -P(=O)(R^{a})(R^{a}), or -O-P(=O)(OR^{a})₂ where each R^{a} is independently hydrogen, alkyl, haloalkyl, carbocyclyl, carbocyclylalkyl, aryl, aralkyl, heterocycloalkyl, heterocycloalkylalkyl, heteroaryl or heteroarylalkyl, and each of these moieties can be optionally substituted as defined herein. In a non-limiting embodiment, a substituted alkyl can be selected from fluoromethyl, difluoromethyl, trifluoromethyl, 2-fluoroethyl, 3-fluoropropyl, hydroxymethyl, 2-hydroxyethyl, 3-hydroxypropyl, benzyl, and phenethyl.

As used herein, the term "alkoxy" refers to the group -O-alkyl, including from 1 to 10 carbon atoms (C₁₋₁₀) of a straight, branched, saturated cyclic configuration and combinations thereof, attached to the parent molecular structure through an oxygen. Unless stated otherwise in the specification, the term is intended to include both substituted and unsubstituted alkoxy groups. Examples include methoxy, ethoxy, propoxy, isopropoxy, butoxy, t-butoxy, pentoxy, cyclopropyloxy, cyclohexyloxy and the like. "Lower alkoxy" refers to alkoxy groups containing one to six carbons. In some embodiments, C₁₋₃ alkoxy is an alkoxy group that encompasses both straight and branched chain alkyls of from 1 to 3 carbon atoms. Unless stated otherwise in the specification, an alkoxy group can be optionally substituted by one or more substituents which independently include: acyl, alkyl, alkenyl, alkynyl, alkoxy, alkylaryl, cycloalkyl, aralkyl, aryl, aryloxy, amino, amido, amidino, imino, azide, carbonate, carbamate, carbonyl, heteroalkyl, heteroaryl, heteroarylalkyl, heterocycloalkyl, hydroxy, cyano, halo, haloalkoxy, haloalkyl, ester, ether, mercapto, thio, alkylthio, arylthio, thiocarbonyl, nitro, oxo, phosphate, phosphonate, phosphinate, silyl, sulfinyl, sulfonyl, sulfonamidyl, sulfoxyl, sulfonate, urea, -Si(R^{a})₃ , -OR^{a}, - SR^{a}, -OC(O)-R^{a}, -N(R^{a})₂, -C(O)Rₐ, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, -N(R^{a})C(NR^{a})N(R^{a})₂, -N(R^{a})S(O)ₜN(R^{a})₂ (where t is 1 or 2), - P(=O)(R^{a})(R^{a}), or -O-P(=O)(OR^{a})₂ where each R^{a} is independently hydrogen, alkyl, haloalkyl, carbocyclyl, carbocyclylalkyl, aryl, aralkyl, heterocycloalkyl, heterocycloalkylalkyl, heteroaryl or heteroarylalkyl, and each of these moieties can be optionally substituted as defined herein.

As used herein, the terms "aromatic" or "aryl" refer to a radical with 6 to 14 ring atoms (*e.g.,* C₆₋₁₄ aromatic or C₆₋₁₄ aryl) that has at least one ring having a conjugated pi electron system which is carbocyclic (*e.g*., phenyl, fluorenyl, and naphthyl). Unless stated otherwise in the specification, the term is intended to include both substituted and unsubstituted aryl groups. In some embodiments, the aryl is a C₆₋₁₀ aryl group. For example, bivalent radicals formed from substituted benzene derivatives and having the free valences at ring atoms are named as substituted phenylene radicals. In other embodiments, bivalent radicals derived from univalent polycyclic hydrocarbon radicals whose names end in"-yl" by removal of one hydrogen atom from the carbon atom with the free valence are named by adding "-idene" to the name of the corresponding univalent radical, *e.g.,* a naphthyl group with two points of attachment is termed naphthylidene. Whenever it appears herein, a numerical range such as "6 to 14 aryl" refers to each integer in the given range; *e.g.,* "6 to 14 ring atoms" means that the aryl group can consist of 6 ring atoms, 7 ring atoms, *etc.,* up to and including 14 ring atoms. The term includes monocyclic or fused-ring polycyclic (i.e., rings which share adjacent pairs of ring atoms) groups. Polycyclic aryl groups include bicycles, tricycles, tetracycles, and the like. In a multi-ring group, only one ring is required to be aromatic, so groups such as indanyl are encompassed by the aryl definition. Non-limiting examples of aryl groups include phenyl, phenalenyl, naphthalenyl, tetrahydronaphthyl, phenanthrenyl, anthracenyl, fluorenyl, indolyl, indanyl, and the like. Unless stated otherwise in the specification, an aryl moiety can be optionally substituted by one or more substituents which independently include: acyl, alkyl, alkenyl, alkynyl, alkoxy, alkylaryl, cycloalkyl, aralkyl, aryl, aryloxy, amino, amido, amidino, imino, azide, carbonate, carbamate, carbonyl, heteroalkyl, heteroaryl, heteroarylalkyl, heterocycloalkyl, hydroxy, cyano, halo, haloalkoxy, haloalkyl, ester, ether, mercapto, thio, alkylthio, arylthio, thiocarbonyl, nitro, oxo, phosphate, phosphonate, phosphinate, silyl, sulfinyl, sulfonyl, sulfonamidyl, sulfoxyl, sulfonate, urea, -Si(R^{a})₃ , -OR^{a}, -SR^{a}, -OC(O)-R^{a}, -N(R^{a})₂, -C(O)Rₐ, -C(O)OR^{a}, -OC(O)N(R^{a})₂, - C(O)N(R^{a})₂, -N(R^{a})C(O)OR^{a}, -N(R^{a})C(O)R^{a}, -N(R^{a})C(O)N(R^{a})₂, -N(R^{a})C(NR^{a})N(R^{a})₂, - N(R^{a})S(O)ₜN(R^{a})₂ (where t is 1 or 2), -P(=O)(R^{a})(R^{a}), or -O-P(=O)(OR^{a})₂ where each R^{a} is independently hydrogen, alkyl, haloalkyl, carbocyclyl, carbocyclylalkyl, aryl, aralkyl, heterocycloalkyl, heterocycloalkylalkyl, heteroaryl or heteroarylalkyl, and each of these moieties can be optionally substituted as defined herein.

As used herein, the terms "cycloalkyl" and "carbocyclyl" each refers to a monocyclic or polycyclic radical that contains only carbon and hydrogen, and can be saturated or partially unsaturated. Partially unsaturated cycloalkyl groups can be termed "cycloalkenyl" if the carbocycle contains at least one double bond, or "cycloalkynyl" if the carbocycle contains at least one triple bond. Cycloalkyl groups include groups having from 3 to 13 ring atoms (i.e., C₃₋₁₃ cycloalkyl). Unless stated otherwise in the specification, the term is intended to include both substituted and unsubstituted cycloalkyl groups. Whenever it appears herein, a numerical range such as "3 to 10" refers to each integer in the given range; *e*.*g*., "3 to 13 carbon atoms" means that the cycloalkyl group can consist of 3 carbon atoms, 4 carbon atoms, 5 carbon atoms, *etc.,* up to and including 13 carbon atoms. The term "cycloalkyl" also includes bridged and spiro-fused cyclic structures containing no heteroatoms. The term also includes monocyclic or fused-ring polycyclic (i.e., rings which share adjacent pairs of ring atoms) groups. Polycyclic aryl groups include bicycles, tricycles, tetracycles, and the like. In some embodiments, "cycloalkyl" can be a C₃₋₈ cycloalkyl radical. In some embodiments, "cycloalkyl" can be a C₃₋₅ cycloalkyl radical. Illustrative examples of cycloalkyl groups include, but are not limited to the following moieties: C₃₋₆ carbocyclyl groups include, without limitation, cyclopropyl (C₃), cyclobutyl (C₄), cyclopentyl (C₅), cyclopentenyl (C₅), cyclohexyl (C₆), cyclohexenyl (C₆), cyclohexadienyl (C₆) and the like. Examples of C₃₋₇ carbocyclyl groups include norbornyl (C₇). Examples of C₃₋₈ carbocyclyl groups include the aforementioned C₃₋₇ carbocyclyl groups as well as cycloheptyl (C₇), cycloheptadienyl (C₇), cycloheptatrienyl (C₇), cyclooctyl (C₈), bicyclo[2.2.1]heptanyl, bicyclo[2.2.2]octanyl, and the like. Examples of C₃₋₁₃ carbocyclyl groups include the aforementioned C₃₋₈ carbocyclyl groups as well as octahydro-1H indenyl, decahydronaphthalenyl, spiro[4.5]decanyl and the like. Unless stated otherwise in the specification, a cycloalkyl group can be optionally substituted by one or more substituents which independently include: acyl, alkyl, alkenyl, alkynyl, alkoxy, alkylaryl, cycloalkyl, aralkyl, aryl, aryloxy, amino, amido, amidino, imino, azide, carbonate, carbamate, carbonyl, heteroalkyl, heteroaryl, heteroarylalkyl, heterocycloalkyl, hydroxy, cyano, halo, haloalkoxy, haloalkyl, ester, ether, mercapto, thio, alkylthio, arylthio, thiocarbonyl, nitro, oxo, phosphate, phosphonate, phosphinate, silyl, sulfinyl, sulfonyl, sulfonamidyl, sulfoxyl, sulfonate, urea, -Si(R^{a})₃ , -OR^{a}, - SR^{a}, -OC(O)-R^{a}, -N(R^{a})₂, -C(O)Rₐ, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, -N(R^{a})C(NR^{a})N(R^{a})₂, -N(R^{a})S(O)ₜN(R^{a})₂ (where t is 1 or 2), - P(=O)(R^{a})(R^{a}), or -O-P(=O)(OR^{a})₂ where each R^{a} is independently hydrogen, alkyl, haloalkyl, carbocyclyl, carbocyclylalkyl, aryl, aralkyl, heterocycloalkyl, heterocycloalkylalkyl, heteroaryl or heteroarylalkyl, and each of these moieties can be optionally substituted as defined herein. The terms "cycloalkenyl" and "cycloalkynyl" mirror the above description of "cycloalkyl" wherein the prefix "alk" is replaced with "alken" or "alkyn" respectively, and the parent "alkenyl" or "alkynyl" terms are as described herein. For example, a cycloalkenyl group can have 3 to 13 ring atoms, such as 5 to 8 ring atoms. In some embodiments, a cycloalkynyl group can have 5 to 13 ring atoms.

As used herein, the terms "carbocycle", "carbocyclic" and "carbocyclyl" each refers to a monocyclic or polycyclic radical that contains only carbon as ring atoms, and can be saturated or partially unsaturated. Fully saturated carbocyclic is termed cycloalkyl. Partially unsaturated cycloalkyl groups can be termed "cycloalkenyl" if the carbocycle contains at least one double bond, or "cycloalkynyl" if the carbocycle contains at least one triple bond. Unless stated otherwise in the specification, the term is intended to include both substituted and unsubstituted carbocyclic groups. The term "carbocyclic" also includes bridged and spiro-fused cyclic structures containing no hetero ring atoms. The term also includes monocyclic or fused-ring polycyclic (i.e., rings which share adjacent pairs of ring atoms) groups. Polycyclic groups include bicycles, tricycles, tetracycles, and the like. Unless stated otherwise in the specification, a carbocyclic group can be optionally substituted by one or more substituents.

As used herein, the terms "heterocycle", "heterocyclic" or "heterocyclyl" refer to fully saturated or partially unsaturated cyclic groups, for example, 3 to 7 membered monocyclic, 7 to 12 membered bicyclic, or 10 to 15 membered tricyclic ring systems, which have at least one heteroatom in at least one ring, wherein 0, 1, 2 or 3 atoms of each ring may be substituted by a substituent. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system.

As used herein, the term "halogen" refers to fluorine (F), chlorine (Cl), bromine (Br), or iodine (I). As used herein, the term "halide" or "halo", means fluoro, chloro, bromo or iodo. The terms "haloalkyl," "haloalkenyl," "haloalkynyl" and "haloalkoxy" include alkyl, alkenyl, alkynyl and alkoxy structures that are substituted with one or more halo groups or with combinations thereof. For example, the terms "fluoroalkyl" and "fluoroalkoxy" include haloalkyl and haloalkoxy groups, respectively, in which the halo is fluorine, such as, but not limited to, trifluoromethyl, difluoromethyl, 2,2,2-trifluoroethyl, 1-fluoromethyl-2-fluoroethyl, and the like. Each of the alkyl, alkenyl, alkynyl and alkoxy groups are as defined herein and can be optionally further substituted as defined herein.

As used herein, the term "heteroatom" refers to oxygen (O), nitrogen (N), sulfur (S), and phosphorus (P).

As used herein, the term "heteroalkyl" refers to an alkyl radical, which have one or more skeletal chain atoms selected from an atom other than carbon, *e*.*g*., oxygen, nitrogen, sulfur, phosphorus or combinations thereof. Unless stated otherwise in the specification, the term is intended to include both substituted and unsubstituted heteroalkyl groups. A numerical range can be given, *e.g.,* C₁₋₄ heteroalkyl, which refers to the chain length in total, which in this example is 4 atoms long. For example, a -CH₂OCH₂CH₃ radical is referred to as a "C₄" heteroalkyl, which includes the heteroatom center in the atom chain length description. Connection to the parent molecular structure can be through either a heteroatom or a carbon in the heteroalkyl chain. For example, an N-containing heteroalkyl moiety refers to a group in which at least one of the skeletal atoms is a nitrogen atom. One or more heteroatom(s) in the heteroalkyl radical can be optionally oxidized. One or more nitrogen atoms, if present, can also be optionally quaternized. For example, heteroalkyl also includes skeletal chains substituted with one or more nitrogen oxide (-O-) substituents. Exemplary heteroalkyl groups include, without limitation, ethers such as methoxyethanyl (-CH₂CH₂OCH₃), ethoxymethanyl (-CH₂OCH₂CH₃), (methoxymethoxy)ethanyl (-CH₂CH₂OCH₂OCH₃), (methoxymethoxy) methanyl (-CH₂OCH₂OCH₃) and (methoxyethoxy)methanyl (-CH₂OCH₂CH₂OCH₃) and the like; amines such as (-CH₂CH₂NHCH₃, -CH₂CH₂N(CH₃)₂, -CH₂NHCH₂CH₃, -CH₂N(CH₂CH₃)(CH₃)) and the like.

As used herein, the term "heterocycloalkyl" refers to a cycloalkyl radical, which have one or more skeletal chain atoms selected from an atom other than carbon, *e*.*g*., oxygen, nitrogen, sulfur, phosphorus or combinations thereof. Unless stated otherwise in the specification, the term is intended to include both substituted and unsubstituted heterocycloalkyl groups. Illustrative examples of heterocycloalkyl include 2-hydroxy-aziridin-1-yl, 3-oxo-1-oxacyclobutan-2-yl, 2,2-dimethyl-tetrahydrofuran-3-yl, 3-carboxy-morpholin-4-yl, 1-cyclopropyl-4-methyl-piperazin-2-yl. 2-pyrrolinyl, 3-pyrrolinyl, dihydro-2H-pyranyl, 1,2,3,4-tetrahydropyridine, 3,4-dihydro-2H-[1,4]oxazine, etc.

As used herein, the term "heteroaryl" or, alternatively, "heteroaromatic" refers to a radical of a 5-18 membered monocyclic or polycyclic (*e.g.,* bicyclic, tricyclic, tetracyclic and the like) aromatic ring system (*e.g.,* having 6, 10 or 14 π electrons shared in a cyclic array) having ring carbon atoms and 1-6 ring heteroatoms provided in the aromatic ring system, wherein each heteroatom is independently selected from nitrogen, oxygen, phosphorous and sulfur ("5-18 membered heteroaryl"). Unless stated otherwise in the specification, the term is intended to include both substituted and unsubstituted heteroaryl groups. Heteroaryl polycyclic ring systems can include one or more heteroatoms in one or both rings. Whenever it appears herein, a numerical range such as "5 to 18" refers to each integer in the given range; *e.g.,* "5 to 18 ring atoms" means that the heteroaryl group can consist of 5 ring atoms, 6 ring atoms, *etc.,* up to and including 18 ring atoms. In some instances, a heteroaryl can have 5 to 14 ring atoms. In some embodiments, the heteroaryl has, for example, bivalent radicals derived from univalent heteroaryl radicals whose names end in "-yl" by removal of one hydrogen atom from the atom with the free valence are named by adding "-ene" to the name of the corresponding univalent radical, *e.g.,* a pyridyl group with two points of attachment is a pyridylene.

For example, an N-containing "heteroaromatic" or "heteroaryl" moiety refers to an aromatic group in which at least one of the skeletal atoms of the ring is a nitrogen atom. One or more heteroatom(s) in the heteroaryl radical can be optionally oxidized. One or more nitrogen atoms, if present, can also be optionally quaternized. Heteroaryl also includes ring systems substituted with one or more nitrogen oxide (-O-) substituents, such as pyridinyl N-oxides. The heteroaryl is attached to the parent molecular structure through any atom of the ring(s).

"Heteroaryl" also includes ring systems wherein the heteroaryl ring, as defined above, is fused with one or more aryl groups wherein the point of attachment to the parent molecular structure is either on the aryl or on the heteroaryl ring, or wherein the heteroaryl ring, as defined above, is fused with one or more cycloalkyl or heterocycyl groups wherein the point of attachment to the parent molecular structure is on the heteroaryl ring. For polycyclic heteroaryl groups wherein one ring does not contain a heteroatom (*e.g.,* indolyl, quinolinyl, carbazolyl and the like), the point of attachment to the parent molecular structure can be on either ring, i.e., either the ring bearing a heteroatom (*e.g.,* 2-indolyl) or the ring that does not contain a heteroatom (*e.g.,* 5-indolyl). In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, phosphorous, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, phosphorous, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, phosphorous, and sulfur ("5-6 membered heteroaryl"). In some embodiments, the 5-6 membered heteroaryl has 1-3 ring heteroatoms selected from nitrogen, oxygen, phosphorous, and sulfur. In some embodiments, the 5-6 membered heteroaryl has 1-2 ring heteroatoms selected from nitrogen, oxygen, phosphorous, and sulfur. In some embodiments, the 5-6 membered heteroaryl has 1 ring heteroatom selected from nitrogen, oxygen, phosphorous, and sulfur.

Examples of heteroaryls include, but are not limited to, azepinyl, acridinyl, benzimidazolyl, benzindolyl, 1,3-benzodioxolyl, benzofuranyl, benzooxazolyl, benzo[d]thiazolyl, benzothiadiazolyl, benzo[b][1,4]dioxepinyl, benzo[b][1,4] oxazinyl, 1,4-benzodioxanyl, benzonaphthofuranyl, benzoxazolyl, benzodioxolyl, benzodioxinyl, benzoxazolyl, benzopyranyl, benzopyranonyl, benzofuranyl, benzopyranonyl, benzofurazanyl, benzothiazolyl, benzothienyl (benzothiophenyl), benzothieno[3,2-d]pyrimidinyl, benzotriazolyl, benzo[4,6]imidazo[ 1,2-a]pyridinyl, carbazolyl, cinnolinyl, cyclopenta[d]pyrimidinyl, 6,7-dihydro-5H-cyclopenta[4,5]thieno [2,3-d]pyrimidinyl, 5,6-dihydrobenzo[h]quinazolinyl, 5,6-dihydrobenzo[h]cinnolinyl, 6,7-dihydro-5H benzo[6,7]cyclohepta[ 1,2-c]pyridazinyl, dibenzofuranyl, dibenzothiophenyl, furanyl, furazanyl, furanonyl, furo [3,2 -c]pyridinyl, 5,6,7,8,9,10-hexahydrocycloocta[d] pyrimidinyl, 5,6,7,8,9,10-hexahydrocycloocta[d]pyridazinyl, 5,6,7,8,9,10- hexahydrocycloocta[d]pyridinyl, isothiazolyl, imidazolyl, indazolyl, indolyl, indazolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyranyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, isoquinolinyl, tetrahydroquinolinyl, 5,6,7,8-tetrahydroquinazolinyl, 5,6,7,8-tetrahydrobenzo [4,5 ] thieno [2,3 -d]pyrimdinyl, 6,7,8,9-tetrahydro-5H-cyclohepta[4,5]thieno [2,3-d]pyrimidinyl, 5,6,7,8-tetrahydropyrido[4,5-c]pyridazinyl, thiazolyl, thiadiazolyl, thiapyranyl, triazolyl, tetrazolyl, triazinyl, thieno[2,3-d]pyrimidinyl, thieno[3,2-d]pyrimidinyl, thieno [2,3-c]pridinyl, and thiophenyl (*i.e.,* thienyl). Unless stated otherwise in the specification, a heteroaryl moiety can be optionally substituted by one or more substituents which independently include: acyl, alkyl, alkenyl, alkynyl, alkoxy, alkylaryl, cycloalkyl, aralkyl, aryl, aryloxy, amino, amido, amidino, imino, azide, carbonate, carbamate, carbonyl, heteroalkyl, heteroaryl, heteroarylalkyl, heterocycloalkyl, hydroxy, cyano, halo, haloalkoxy, haloalkyl, ester, ether, mercapto, thio, alkylthio, arylthio, thiocarbonyl, nitro, oxo, phosphate, phosphonate, phosphinate, silyl, sulfinyl, sulfonyl, sulfonamidyl, sulfoxyl, sulfonate, urea, -Si(R^{a})₃ , -OR^{a}, - SR^{a}, -OC(O)-R^{a}, -N(R^{a})₂, -C(O)Rₐ, -C(O)OR^{a}, -OC(O)N(R^{a})₂, -C(O)N(R^{a})₂, -N(R^{a})C(O)OR^{a}, - N(R^{a})C(O)R^{a}, -N(R^{a})C(O)N(R^{a})₂, -N(R^{a})C(NR^{a})N(R^{a})₂, -N(R^{a})S(O)ₜN(R^{a})₂ (where t is 1 or 2), - P(=O)(R^{a})(R^{a}), or -O-P(=O)(OR^{a})₂ where each R^{a} is independently hydrogen, alkyl, haloalkyl, carbocyclyl, carbocyclylalkyl, aryl, aralkyl, heterocycloalkyl, heterocycloalkylalkyl, heteroaryl or heteroarylalkyl, and each of these moieties can be optionally substituted as defined herein.

As used herein, the term "administering" refers to oral administration, administration as a suppository, topical contact, intravenous, parenteral, intraperitoneal, intramuscular, intralesional, intrathecal, intracranial, intranasal or subcutaneous administration, or the implantation of a slow-release device, *e.g.,* a mini-osmotic pump, to a subject. Suitable routes of administration for a particular patient will depend on the nature and severity of the disease or condition being treated or the nature of the therapy being used and on the nature of the active compound.

Administration may be by any suitable route, including parenteral and transmucosal (*e.g.,* buccal, sublingual, palatal, gingival, nasal, vaginal, rectal, or transdermal). Parenteral administration includes, *e*.*g*., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, *etc.*

By "co-administer" it is meant that a composition described herein is administered at the same time, just prior to, or just after the administration of one or more additional therapies.

The compound of the invention can be administered alone or can be co-administered to the patient. Co-administration is meant to include simultaneous or sequential administration of the compound individually or in combination (more than one compound or agent). Thus, the preparations can also be combined, when desired, with other active substances (*e.g.,* to reduce metabolic degradation).

The compositions of the present invention can be delivered transdermally, by a topical route, formulated as applicator sticks, solutions, suspensions, emulsions, gels, creams, ointments, pastes, jellies, paints, powders, and aerosols. Oral preparations include tablets, pills, powder, dragees, capsules, liquids, lozenges, cachets, gels, syrups, slurries, suspensions, etc., suitable for ingestion by the patient. Solid form preparations include powders, tablets, pills, capsules, cachets, suppositories, and dispersible granules. Liquid form preparations include solutions, suspensions, and emulsions, gels, for example, water or water/propylene glycol solutions.

The compositions of the present invention may additionally include components to provide sustained release and/or comfort. Such components include high molecular weight, anionic mucomimetic polymers, gelling polysaccharides and finely-divided drug carrier substrates. These components are discussed in greater detail in U.S. Pat. Nos. 4,911,920; 5,403,841; 5,212,162; and 4,861,760. The entire contents of these patents are incorporated herein by reference in their entirety for all purposes. The compositions of the present invention can also be delivered as microspheres for slow release in the body. For example, microspheres can be administered via intradermal injection of drug-containing microspheres, which slowly release subcutaneously (see Rao, 1995 J. Biomater Sci. Polym. Ed. 7:623-645; as biodegradable and injectable gel formulations (see, *e.g.,* Gao 1995 Pharm. Res. 12:857-863); or, as microspheres for oral administration (see, *e.g.,* Eyles 1997 J. Pharm. Pharmacol. 49:669-674).

As used herein, the terms "disease," "condition," and "disorder" are used interchangeably herein and refer to a state of being or health status of a patient or subject capable of being treated with a compound, pharmaceutical composition, or method provided herein.

As used herein, the term "effective amount" of an active agent refers to an amount sufficient to elicit the desired biological response. As will be appreciated by those of ordinary skill in this art, the effective amount of a compound of the invention may vary depending on such factors as the desired biological endpoint, the pharmacokinetics of the compound, the disease being treated, the mode of administration, and the patient.

As used herein, the terms "inhibition," "inhibit" and "inhibiting" and the like in reference to a biological target (*e.g.,* TYK2) inhibitor interaction refers to negatively affecting (*e.g.,* decreasing) the activity or function of the protein relative to the activity or function of the protein in the absence of the inhibitor. In embodiments, inhibition means negatively affecting (*e.g.* decreasing) the concentration or levels of the protein relative to the concentration or level of the protein in the absence of the inhibitor. In embodiments, inhibition refers to reduction of a disease or symptoms of disease. In embodiments, inhibition refers to a reduction in the activity of a particular protein target. Inhibition includes, at least in part, partially or totally blocking stimulation, decreasing, preventing, or delaying activation, or inactivating, desensitizing, or down-regulating signal transduction or enzymatic activity or the amount of a protein. In embodiments, inhibition refers to a reduction of activity of a target protein resulting from a direct interaction (*e.g.,* an inhibitor binds to the target protein). In embodiments, inhibition refers to a reduction of activity of a target protein from an indirect interaction *(e.g.,* an inhibitor binds to a protein that activates the target protein, thereby preventing target protein activation).

As used herein, the terms "isolated" or "purified" refer to a material that is substantially or essentially free from components that normally accompany it in its native state. Purity and homogeneity are typically determined using analytical chemistry techniques such as polyacrylamide gel electrophoresis or high-performance liquid chromatography.

As used herein, the term "modulate" refers to the production, either directly or indirectly, of an increase or a decrease, a stimulation, inhibition, interference, or blockage in a measured activity when compared to a suitable control. A "modulator" of a polypeptide or polynucleotide refers to a substance that affects, for example, increases, decreases, stimulates, inhibits, interferes with, or blocks a measured activity of the polypeptide or polynucleotide, when compared to a suitable control. For example, a "modulator" may bind to and /or activate or inhibit the target with measurable affinity, or directly or indirectly affect the normal regulation of a receptor activity.

As used herein, a "pharmaceutically acceptable form" of a disclosed compound includes, but is not limited to, pharmaceutically acceptable salts, esters, hydrates, solvates, isomers, prodrugs, and isotopically labeled derivatives thereof. In one embodiment, a "pharmaceutically acceptable form" includes, but is not limited to, pharmaceutically acceptable salts, esters, prodrugs and isotopically labeled derivatives thereof. In some embodiments, a "pharmaceutically acceptable form" includes, but is not limited to, pharmaceutically acceptable isomers and stereoisomers, prodrugs and isotopically labeled derivatives thereof.

In certain embodiments, the pharmaceutically acceptable form is a pharmaceutically acceptable salt. As used herein, the term "pharmaceutically acceptable salt" refers to those salts which are, within the scope of sound medical judgment, suitable for use in contact with the tissues of subjects without undue toxicity, irritation, allergic response and the like, and are commensurate with a reasonable benefit/risk ratio. Pharmaceutically acceptable salts are well known in the art. For example, Berge et al. describes pharmaceutically acceptable salts in detail in J. Pharmaceutical Sciences (1977) 66:1-19. Pharmaceutically acceptable salts of the compounds provided herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchlorate acid or with organic acids such as acetic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, besylate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, p-toluenesulfonate, undecanoate, valerate salts, and the like. In some embodiments, organic acids from which salts can be derived include, for example, acetic acid, propionic acid, glycolic acid, pyruvic acid, lactic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

The salts can be prepared in situ during the isolation and purification of the disclosed compounds, or separately, such as by reacting the free base or free acid of a parent compound with a suitable base or acid, respectively. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, iron, zinc, copper, manganese, aluminum, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate. Organic bases from which salts can be derived include, for example, primary, secondary, and tertiary amines, substituted amines, including naturally occurring substituted amines, cyclic amines, basic ion exchange resins, and the like, such as isopropylamine, trimethylamine, diethylamine, triethylamine, tripropylamine, and ethanolamine. In some embodiments, the pharmaceutically acceptable base addition salt can be chosen from ammonium, potassium, sodium, calcium, and magnesium salts.

In certain embodiments, the pharmaceutically acceptable form is a "solvate" (*e.g.,* a hydrate). As used herein, the term "solvate" refers to compounds that further include a stoichiometric or non-stoichiometric amount of solvent bound by non-covalent intermolecular forces. The solvate can be of a disclosed compound or a pharmaceutically acceptable salt thereof. Where the solvent is water, the solvate is a "hydrate." Pharmaceutically acceptable solvates and hydrates are complexes that, for example, can include 1 to about 100, or 1 to about 10, or 1 to about 2, about 3 or about 4, solvent or water molecules. It will be understood that the term "compound" as used herein encompasses the compound and solvates of the compound, as well as mixtures thereof.

In certain embodiments, the pharmaceutically acceptable form is a prodrug. As used herein, the term "prodrug" (or "pro-drug") refers to compounds that are transformed *in vivo* to yield a disclosed compound or a pharmaceutically acceptable form of the compound. A prodrug can be inactive when administered to a subject, but is converted *in vivo* to an active compound, for example, by hydrolysis (*e.g.,* hydrolysis in blood). In certain cases, a prodrug has improved physical and/or delivery properties over the parent compound. Prodrugs can increase the bioavailability of the compound when administered to a subject (*e.g.,* by permitting enhanced absorption into the blood following oral administration) or which enhance delivery to a biological compartment of interest *(e.g.,* the brain or lymphatic system) relative to the parent compound. Exemplary prodrugs include derivatives of a disclosed compound with enhanced aqueous solubility or active transport through the gut membrane, relative to the parent compound.

The prodrug compound often offers advantages of solubility, tissue compatibility or delayed release in a mammalian organism (see, *e.g.,* Bundgard, H., Design of Prodrugs (1985), pp. 7- 9, 21-24 (Elsevier, Amsterdam). A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987, both of which are incorporated in full by reference herein.

Prodrug forms often offer advantages of solubility, tissue compatibility, or delayed release in the mammalian organism. (See, Bundgard, Design of Prodrugs, pp. 7-9,21-24, Elsevier, Amsterdam 1985 and Silverman, The Organic Chemistry of Drug Design and Drug Action, pp. 352-401, Academic Press, San Diego, Calif., 1992.) Prodrugs commonly known in the art include well-known acid derivatives, such as, for example, esters prepared by reaction of the parent acids with a suitable alcohol, amides prepared by reaction of the parent acid compound with an amine, basic groups reacted to form an acylated base derivative, *etc.* Other prodrug derivatives may be combined with other features disclosed herein to enhance bioavailability. As such, those of skill in the art will appreciate that certain of the presently disclosed compounds having free amino, amido, hydroxy or carboxylic groups can be converted into prodrugs. Prodrugs include compounds having a carbonate, carbamate, amide or alkyl ester moiety covalently bonded to any of the above substituents disclosed herein.

Exemplary advantages of a prodrug can include, but are not limited to, its physical properties, such as enhanced water solubility for parenteral administration at physiological pH compared to the parent compound, or it can enhance absorption from the digestive tract, or it can enhance drug stability for long-term storage.

As used herein, the term "pharmaceutically acceptable" excipient, carrier, or diluent refers to a pharmaceutically acceptable material, composition or vehicle, such as a liquid or solid filler, diluent, excipient, solvent or encapsulating material, involved in carrying or transporting the subject pharmaceutical agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations. Wetting agents, emulsifiers and lubricants, such as sodium lauryl sulfate, magnesium stearate, and polyethylene oxide-polypropylene oxide copolymer as well as coloring agents, release agents, coating agents, sweetening, flavoring and perfuming agents, preservatives and antioxidants can also be present in the compositions.

As used herein, the term "subject" refers to any animal (*e.g.,* a mammal), including, but not limited to humans, non-human primates, rodents, and the like, which is to be the recipient of a particular treatment. A subject to which administration is contemplated includes, but is not limited to, humans (*e.g.,* a male or female of any age group, *e.g.,* a pediatric subject (*e.g.,* infant, child, adolescent) or adult subject (*e.g.,* young adult, middle-aged adult or senior adult)) and/or other non-human animals, for example, non-human mammals (e.g., primates (e.g., cynomolgus monkeys, rhesus monkeys); commercially relevant mammals such as cattle, pigs, horses, sheep, goats, cats, and/or dogs), rodents (e.g., rats and/or mice), etc. In certain embodiments, the non-human animal is a mammal. The non-human animal may be a male or female at any stage of development. A non-human animal may be a transgenic animal. Typically, the terms "subject" and "patient" are used interchangeably herein in reference to a human subject.

As used herein, the terms "treatment" or "treating" a disease or disorder refers to a method of reducing, delaying or ameliorating such a condition before or after it has occurred. Treatment may be directed at one or more effects or symptoms of a disease and/or the underlying pathology. The treatment can be any reduction and can be, but is not limited to, the complete ablation of the disease or the symptoms of the disease. Treating or treatment thus refers to any indicia of success in the therapy or amelioration of an injury, disease, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. The treatment or amelioration of symptoms can be based on objective or subjective parameters, for example, the results of a physical examination, neuropsychiatric exams, and/or a psychiatric evaluation. As compared with an equivalent untreated control, such reduction or degree of amelioration may be at least 5%, 10%, 20%, 40%, 50%, 60%, 80%, 90%, 95%, or 100% as measured by any standard technique.

Treatment methods include administering to a subject a therapeutically effective amount of a compound described herein. The administering step may be a single administration or may include a series of administrations. The length of the treatment period depends on a variety of factors, such as the severity of the condition, the patient's age, the concentration of the compound, the activity of the compositions used in the treatment, or a combination thereof. It will also be appreciated that the effective dosage of an agent used for the treatment may increase or decrease over the course of a particular treatment regime. Changes in dosage may result and become apparent by standard diagnostic assays known in the art. In some instances, chronic administration may be required. For example, the compositions are administered to the subject in an amount and for a duration sufficient to treat the patient.

### Detailed Description of the Invention

The invention is based on an unexpected discovery of novel, selective and potent compounds that are TYK2 inhibitors. The invention also provides pharmaceutical compositions of these compounds and methods of their preparation and use. The compounds are orally available and exhibit fewer and/or lesser side effects than currently available drugs.

The new class of TYK2 inhibitors disclosed herein exhibit exceptional potency profiles and are useful in treating one or more TYK2-mediated diseases and conditions, such as allergic, autoimmune, inflammatory, metabolic, neurological and proliferative diseases and conditions. Without wishing to be bound by the theory, compounds of the invention are modulators of interleukins *(e.g.,* IL-12, IL-23) and interferons *(e.g.,* IFN-a) by inhibiting TYK2-mediated signal transduction.

These compounds are designed to show good potency against TYK2 with good oral absorption and good *in vivo* stability. The invention also provides pharmaceutical compositions of these compounds and methods of preparation and use thereof. The TYK2 inhibitors disclosed herein exhibit favorable pharmacokinetic profiles and drug properties that are suitable for the target indications.

In one aspect, the invention generally relates to a compound having the structural formula (I): 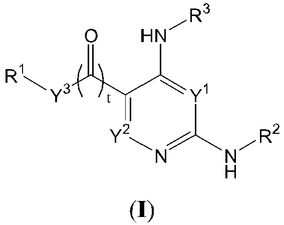 or a pharmaceutically acceptable form or an isotope derivative thereof,
wherein
Y¹ is CH, CF or N;
Y² is CH or N;
Y³ is NR, O, CH₂, CD₂, CF₂ or O-NH;
*t* is 0 or 1;
R¹ is H, F, CD₃, or C₁-C₃ alkyl, provided that R¹ is not F when Y³ is N, O or O-NH;
R² is
   R^{2'}, wherein R^{2'} is C₁-C₆ alkyl, C₃-C₆ cycloalkyl, C₅-C₇ spirocycloalkyl, or C₃-C₆ heterocycloalkyl, each substituted with 0-2 R^{2a}, wherein R^{2a} is selected from the group consisting of halogen, CN, OR, NRR', alkyl, cycloalkyl, heterocyclic;
   an aryl or heteroaryl group, each substituted with 0-2 R^{2a};
   (C=O)R^{2b}; or
   (C=O)NHR^{2b};
R³ is 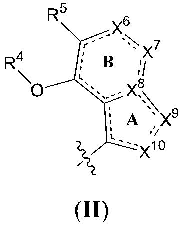 wherein
   X⁶ is CR⁶ or N;
   X⁷ is CR⁷ or N;
   X⁸ is C or N;
   X⁹ is CR⁹, O, S, N or NR⁹;
   X¹⁰ is CR¹⁰ , O, S, N or NR¹⁰; and
wherein each of Ring A and Ring B is independently an aryl or heteroaryl group;
   R^{2b} is a C₁₋₆ alkyl or C₃₋₆ cycloalkyl, C₅₋₇ spirocycloalkyl, aryl or heteroaryl, each substituted with 0-4 R^{2c};
   R^{2c} at each occurrence is independently halo, CN, OR, NRR', OCF₃, CF₃, C₁₋₆ alkyl, C₁₋₆ haloalkyl, C₂₋₆ alkenyl, C₂₋₆ alkynyl, wherein said alkyl, haloalkyl, alkenyl, alkynyl, R and R' are substituted with 0-3 R^{2a}; and
   R⁴ is a C₁₋₃ alkyl, substituted with 0-5 R^{4a}, wherein R^{4a} is selected from D, F and Cl;
   R⁵ is H, CN, halo, OCH₃, C(=O)OR, NHC(=O)R, NRR', NO₂, C₁₋₆ alkyl, C₃-C₆ cycloalkyl or heterocyclic, wherein said alkyl, cycloalkyl or heterocyclic is substituted with 0-3 R^{5a}, wherein each R^{5a} is independently selected from OH, D, F, Cl, CN, CH₂F, CHF₂, CF₃, OCH₃, OCD₃, OCF₃ and OC(=O)CH₃;
   each of R⁶, R⁷, R⁹ and R¹⁰ is independently selected from H, F, Cl, CN, CD₃, CH₂CF₃, CF₃, OR, NRR', C₁-C₃ alkyl and C₃-C₅ cycloalkyl, wherein said alkyl, cycloalkyl, R and R' are substituted with 0-2 R^{2a}; and
   each of R and R' is independently H or a C₁-C₆ alkyl or acyl, or R and R', together with the nitrogen atom to which they are bonded, form a 4- to 7-membered ring comprising 0-2 heteroatoms selected from O, NR, S and SO₂.

In certain embodiments of (**I**), *t is* 1, and the compound has the structural formula:

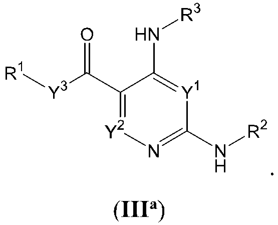

In certain embodiments of (**I**), *t is* 0, and the compound has the structural formula:

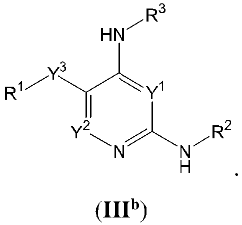

In certain embodiments of (**II**), Ring A is a heteroaryl group.

In certain embodiments of (**II**), X⁶ is CH and X⁷ is CH, with R³ having the structure:

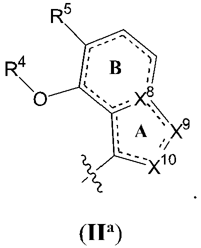

In certain embodiments of (**II**), X⁷ is CH and X⁸ is C, with R³ having the structure:

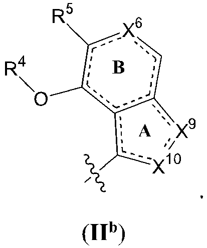

In certain embodiments of (**II**), X⁶ is CH and X³ is C, with R³ having the structure:

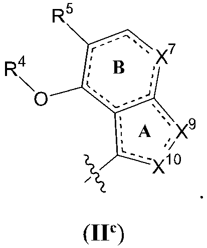

In certain embodiments of (**II**), X⁷ is CH and X¹⁰ is CH, with R³ having the structure:

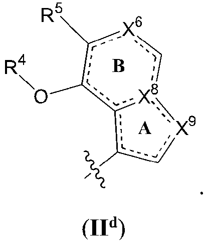

In certain embodiments of **(II)-(II^{d}),** R⁴ is CH₃.

In certain embodiments of **(II)-(II^{d}),** R⁴ is CD₃.

In certain embodiments of **(III^{a})-(III^{b}),** R³ is selected from:

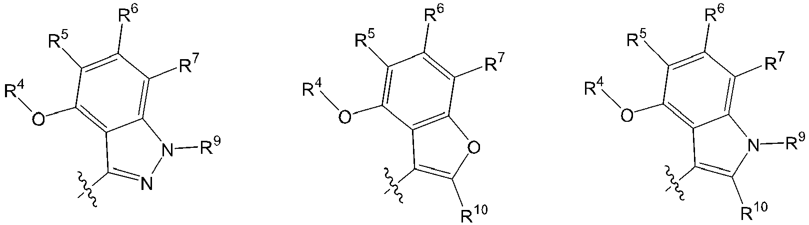

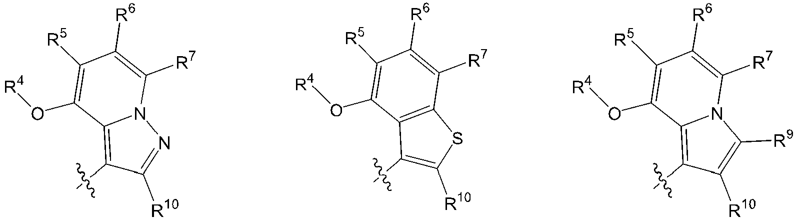

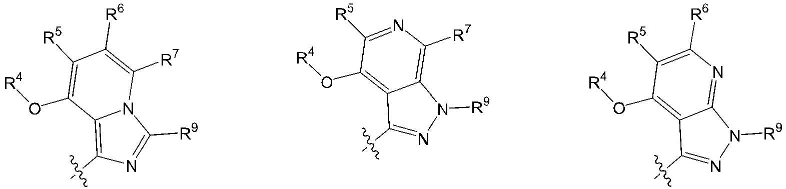

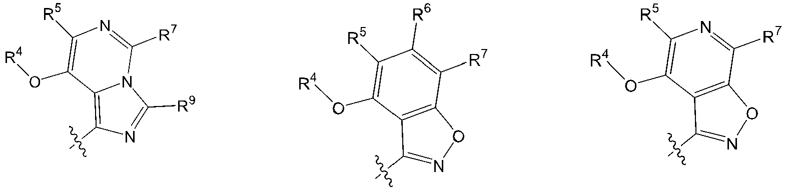

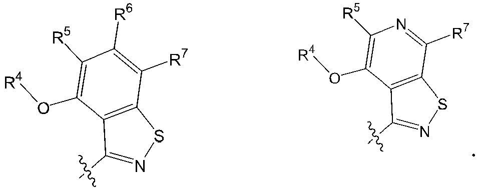

In certain embodiments, R⁴ is CD₃.

In certain embodiments, R⁴ is CH₃, and R³ is selected from:

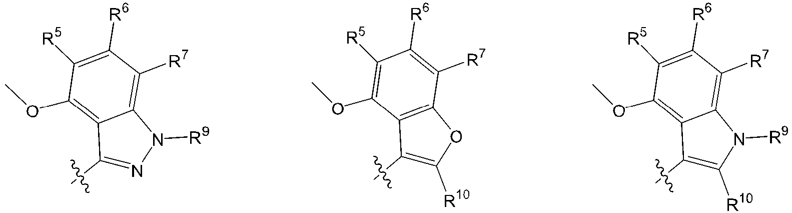

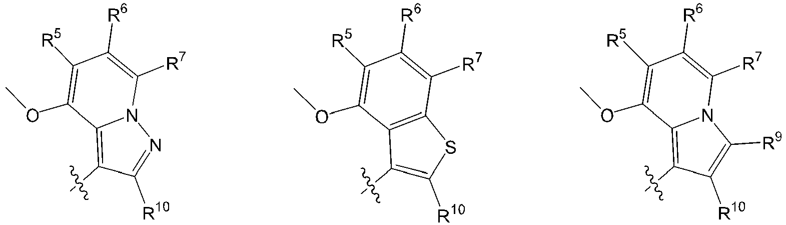

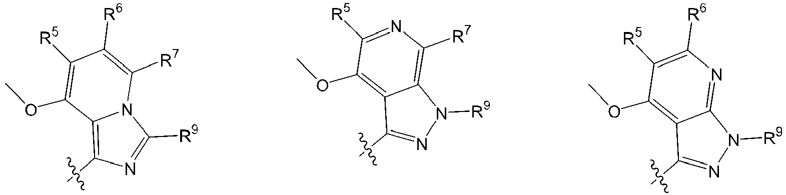

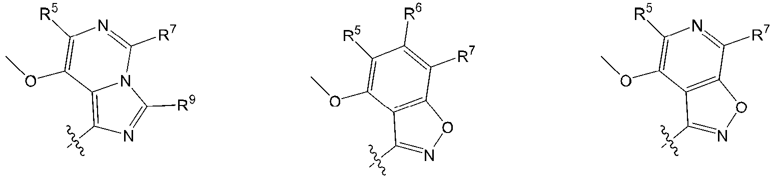

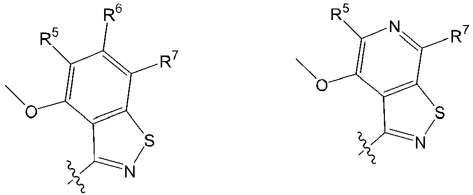

In certain embodiments of **(III^{a})-(III^{b}),** R³ is:

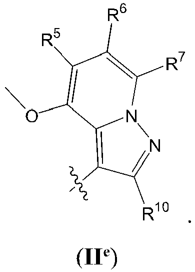

In certain embodiments of R³, R¹⁰ is H.

In certain embodiments of **(III^{a})-(III^{b}),** R³ is:

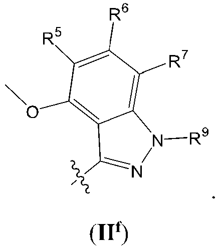

In certain embodiments of R³, R⁹ is C₁₋₃ alkyl or cyclopropyl, each optionally substituted with C₁₋₃ alkoxy, CF₃ or NRR'.

In certain embodiments of R³, R⁹ is C₁₋₃ alkyl.

In certain embodiments of R³, R⁹ is CH₃.

In certain embodiments of R³, R⁹ is CD₃.

In certain embodiments of R³, R⁵ is C₁₋₄ alkyl, substituted with an OH.

In certain embodiments of R³, R⁵ is: 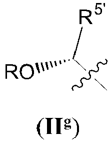 wherein
R^{5'} is a C₁₋₃ alkyl or cyclopropyl, substituted with 0-5 F's; and
R is H, C₁₋₃ alkyl or acyl.

In certain embodiments of R³, R⁵ is: 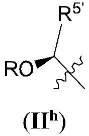 wherein
R^{5'} is a C₁₋₃ alkyl or cyclopropyl, substituted with 0-5 F's; and
R is H, C₁₋₃ alkyl or acyl.

In certain embodiments of **(II^{g})-(II^{h}),** R is CH₃.

In certain embodiments of **(II^{g})-(II^{h}),** R is CD₃.

In certain embodiments of **(II^{g})-(II^{h}),** R is C(=O)CH₃.

In certain embodiments of **(II^{g})-(II^{h}),** R is C(=O)CD₃.

In certain embodiments of **(II^{g})-(II^{h}),** R^{5'} is CF₃.

In certain embodiments of **(II^{g})-(II^{h}),** R^{5'} is CHF₂.

In certain embodiments of (I), **(III^{a})** and **(III^{b}),** Y³ is NH.

In certain embodiments of **(III^{a}),** Y¹ is CH, Y² is CH, and the compound has the structural formula:

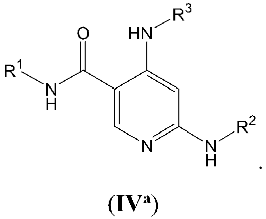

In certain embodiments of **(III^{a}),** Y¹ is CH, Y² is N, and the compound has the structural formula:

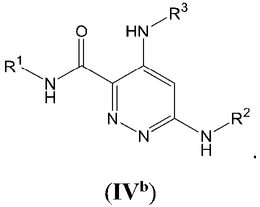

In certain embodiments of **(III^{a}),** Y¹ is N, Y² is CH, and the compound has the structural formula:

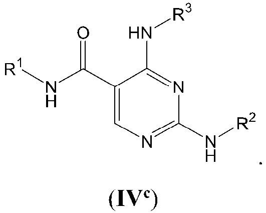

In certain embodiments of **(III^{a}),** Y¹ is N, Y² is N, and the compound has the structural formula:

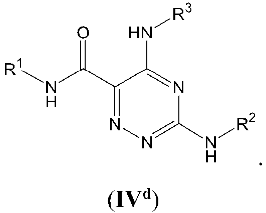

In certain embodiments of (I), **(III^{a})** and **(III^{b}),** Y³ is O.

In certain embodiments of **(III^{a}),** Y¹ is CH, Y² is CH, and the compound has the structural formula:

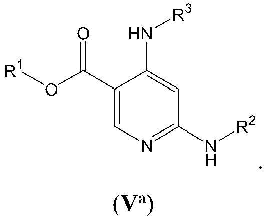

In certain embodiments of **(III^{a}),** Y¹ is CH, Y² is N, and the compound has the structural formula:

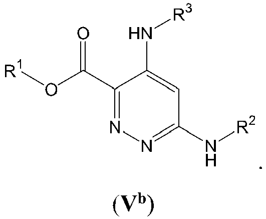

In certain embodiments of **(III^{a}),** Y¹ is N, Y² is CH, and the compound has the structural formula:

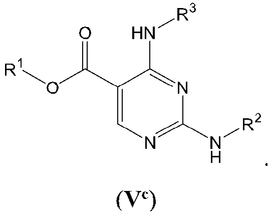

In certain embodiments of **(III^{a}),** Y¹ is N, Y² is N, and the compound has the structural formula:

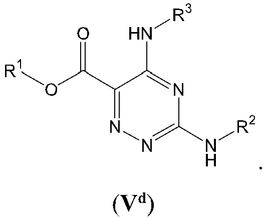

In certain embodiments of (I), **(III^{a})** and **(III^{b}),** Y³ is CH₂.

In certain embodiments of **(III^{a}),** Y¹ is CH, Y² is CH, and the compound has the structural formula:

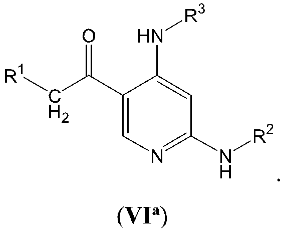

In certain embodiments of **(III^{a}),** Y¹ is CH, Y² is N, and the compound has the structural formula:

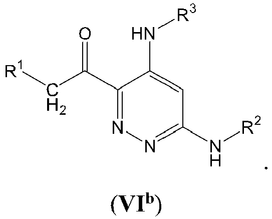

In certain embodiments of **(III^{a}),** Y¹ is N, Y² is CH, and the compound has the structural formula:

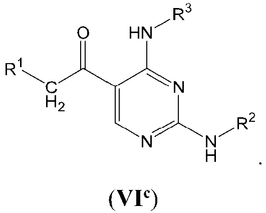

In certain embodiments of **(III^{a}),** Y¹ is N, Y² is N, and the compound has the structural formula:

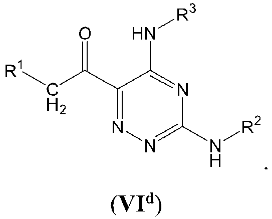

In certain embodiments of (I), wherein t is 0 and the compound has the structural formula **(III^{b}).**

In certain embodiments of **(III^{b}),** Y³ is CD₂.

In certain embodiments of **(III^{b}),** Y³ is CF₂.

In certain embodiments of **(III^{b}),** Y¹ is CH and Y² is CH, and the compound has the structural formula:

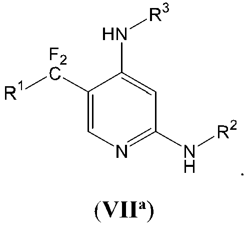

In certain embodiments of **(III^{b}),** Y¹ is CH and Y² is N, and the compound has the structural formula:

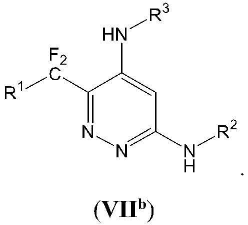

In certain embodiments of **(III^{b}),** Y¹ is N and Y² is CH, and the compound has the structural formula:

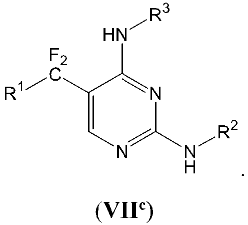

In certain embodiments of **(III^{b}),** Y¹ is N and Y² is N, and the compound has the structural formula:

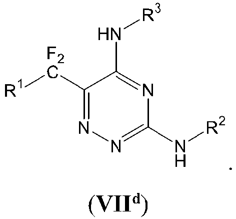

In certain embodiments of R³, each of R⁶ and R⁷, if present, is H.

In certain embodiments of **(III^{a})-(III^{b}),** R² is R^{2'}.

In certain embodiments of **(III^{a})-(III^{b}),** R² is (C=O)R^{2b}.

In certain embodiments, R^{2b} is selected from C₁-C₆ alkyl, substituted with 0-3 R^{2c}.

In certain embodiments, R^{2b} is C₃₋₆ cycloalkyl, substituted with 0-3 R^{2c}.

In certain embodiments, R²⁶ is cyclopropyl.

In certain embodiments, R^{2b} is cyclopropyl, substituted with F.

In certain embodiments, R^{2b} is C₅₋₇ spirocycloalkyl, substituted with 0-3 R^{2c}.

In certain embodiments, R^{2b} is C₅ spiro[2.2]pentyl.

In certain embodiments of **(III^{a})-(III^{b}),** R² is (C=O)NHR^{2b}.

In certain embodiments of **(III^{a})-(III^{b}),** R² is pyridinyl substituted with 0-2 R^{2c}.

In certain embodiments of **(III^{a})-(III^{b}),** R² is phenyl substituted with 0-2 R^{2b}.

In certain embodiments of **(III^{a})-(III^{b}),** R² is pyrazolyl substituted with 0-2 R^{2c}.

In certain embodiments of **(III^{a})-(III^{b}),** R² is pyrimidyl substituted with 0-2 R^{2c}.

In certain embodiments of **(III^{a})-(III^{b}),** R¹ is CH₃.

In certain embodiments of **(III^{a})-(III^{b}),** R¹ is CD₃.

In certain embodiments of **(III^{a}),** the compound has the structural formula: 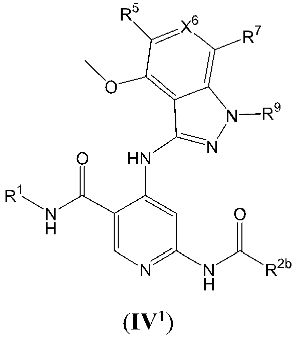 wherein X⁶ is N or CH.

In certain embodiments of **(III^{a}),** the compound has a structural formula selected from:

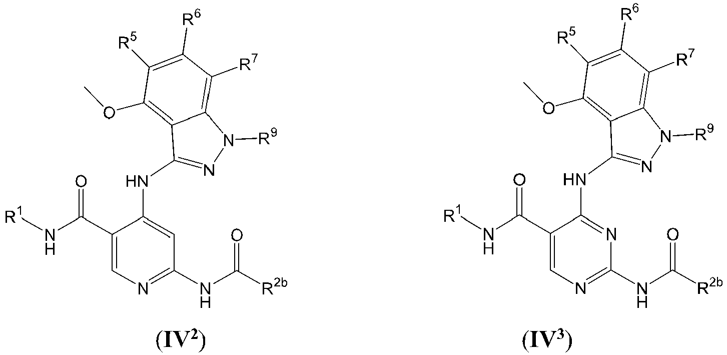

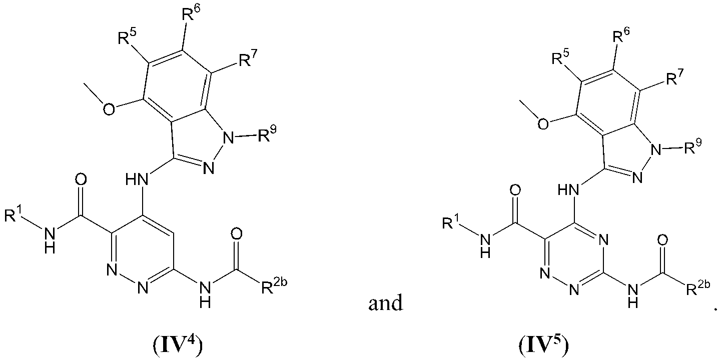

In certain embodiments of **(III^{a}),** the compound has a structural formula selected from:

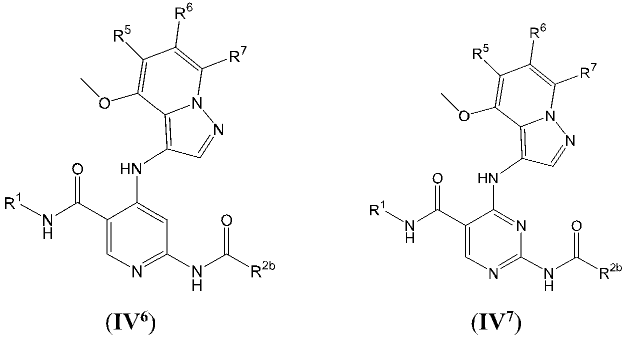

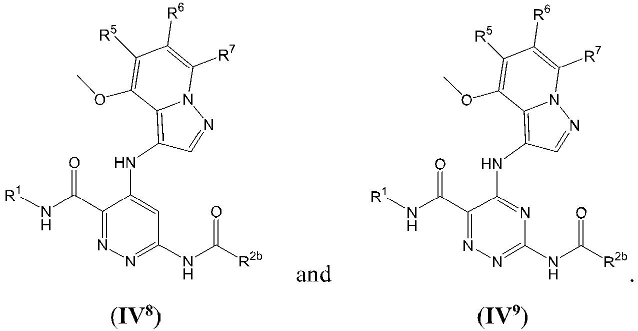

In certain embodiments of **(III^{a}),** the compound has a structural formula selected from:

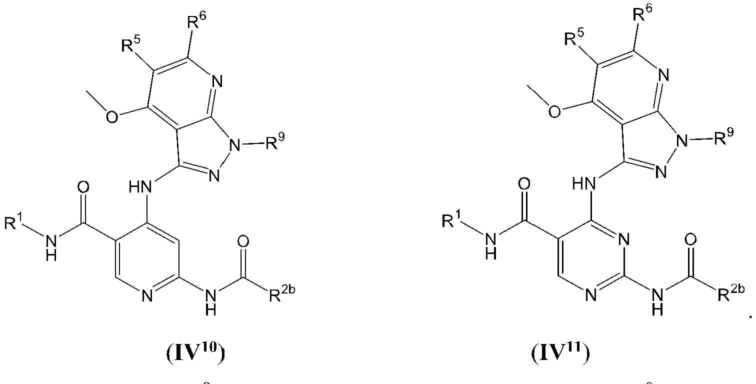

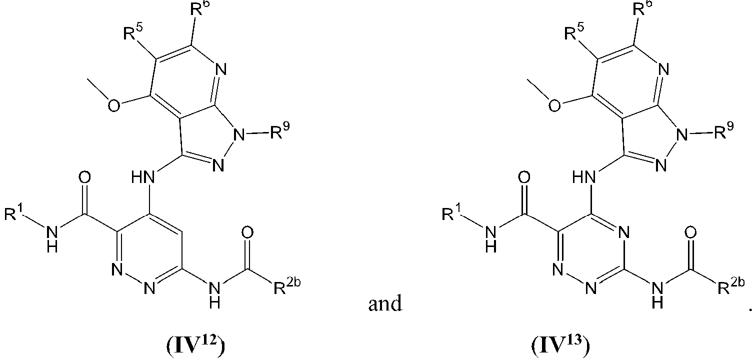

In certain embodiments of **(III^{a}),** the compound has a structural formula selected from:

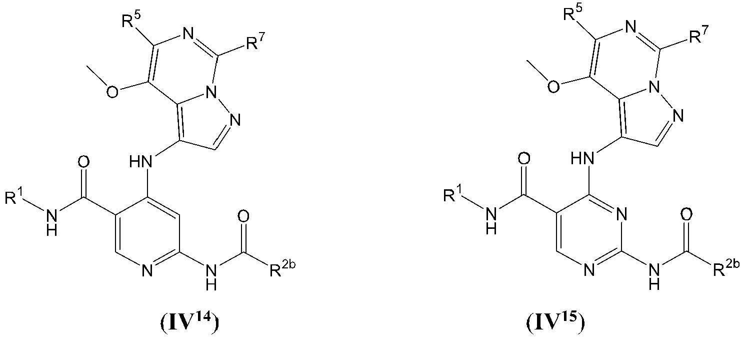

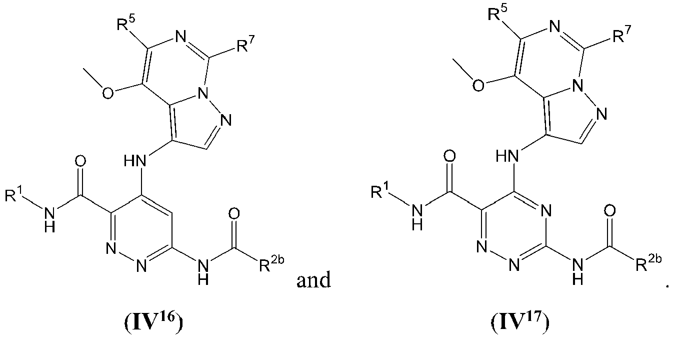

In certain embodiments of **(III^{a}),** the compound has a structural formula selected from:

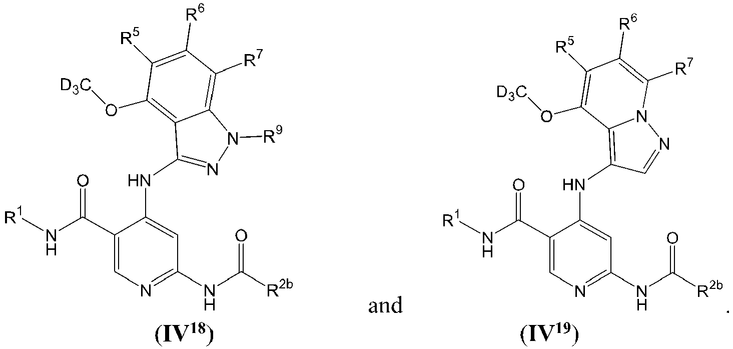

In certain embodiments of **(III^{a}),** the compound has a structural formula selected from: 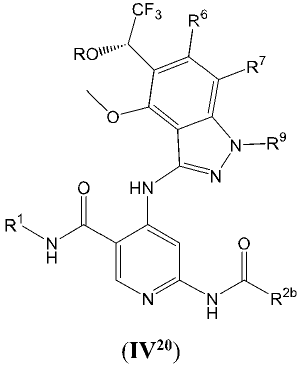 wherein R is H, CD₃, C₁₋₃ alkyl or acyl.

In certain embodiments of **(III^{a}),** the compound has a structural formula selected from: 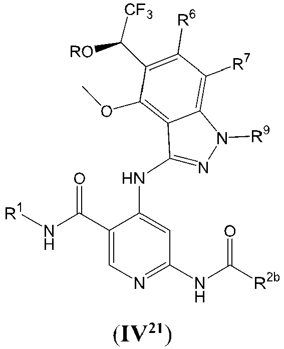 wherein R is H, CD₃, C₁₋₃ alkyl or acyl.

In certain embodiments of **(III^{a}),** the compound has a structural formula selected from: 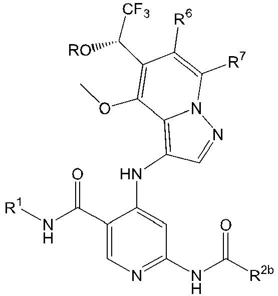 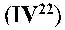 wherein R is H, CD₃, C₁₋₃ alkyl or acyl.

In certain embodiments of **(III^{a}),** the compound has a structural formula selected from: 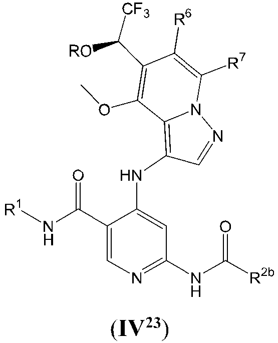 wherein R is H, CD₃, C₁₋₃ alkyl or acyl.

In certain embodiments of **(III^{a}),** the compound has the structural formula: 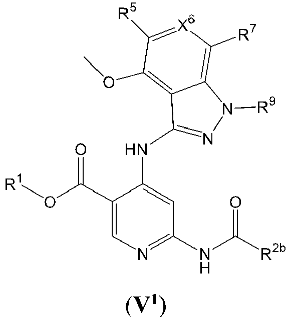 wherein X⁶ is N or CH.

In certain embodiments of **(III^{a}),** the compound has a structural formula selected from:

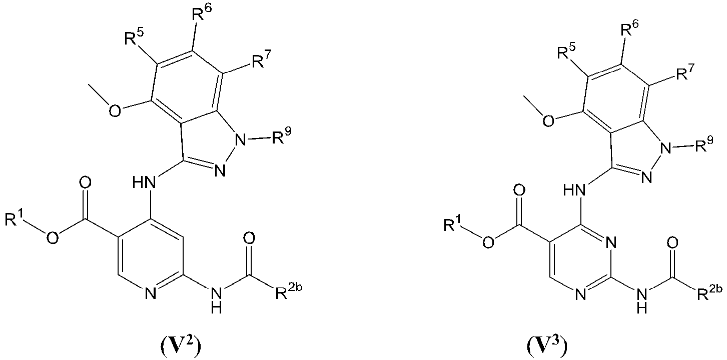

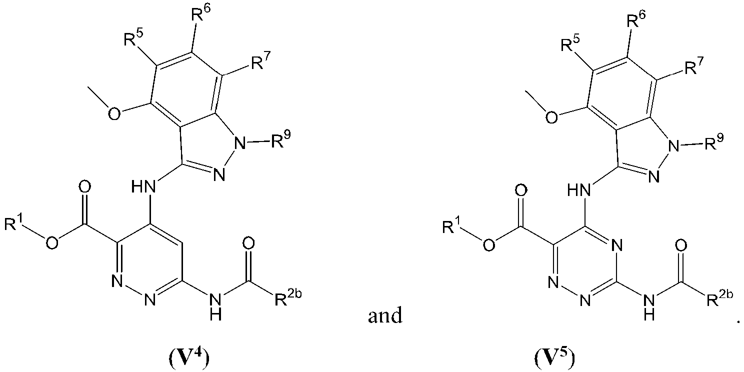

In certain embodiments of **(III^{a}),** the compound has a structural formula selected from:

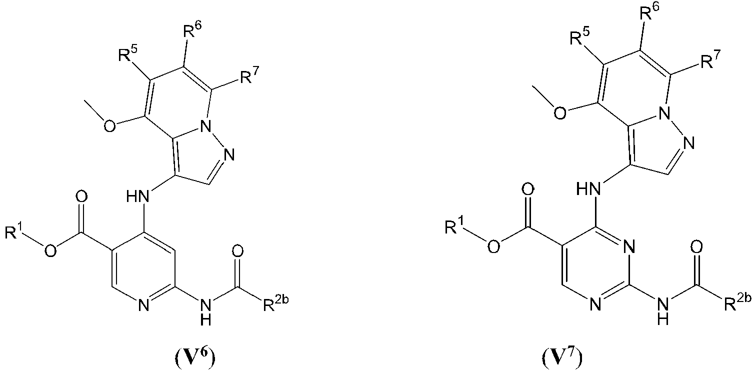

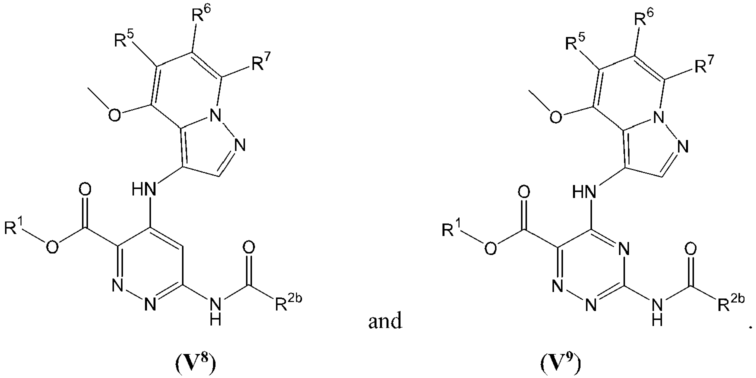

In certain embodiments of **(III^{a}),** the compound has a structural formula selected from:

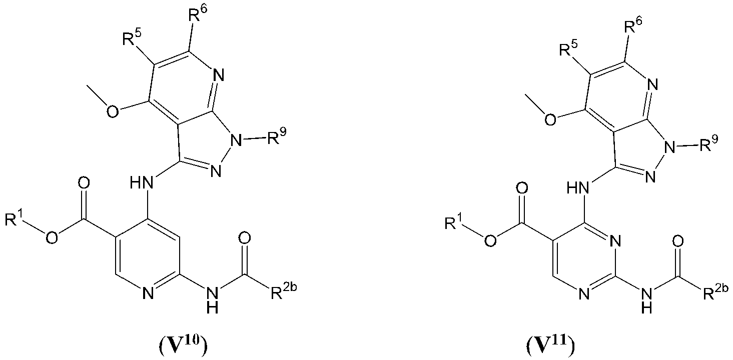

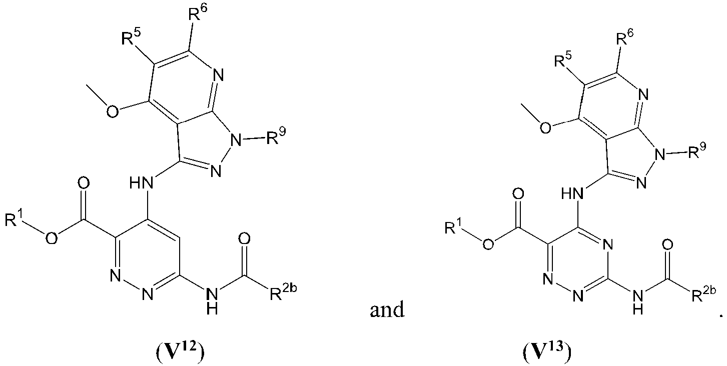

In certain embodiments of **(III^{a}),** the compound has a structural formula selected from:

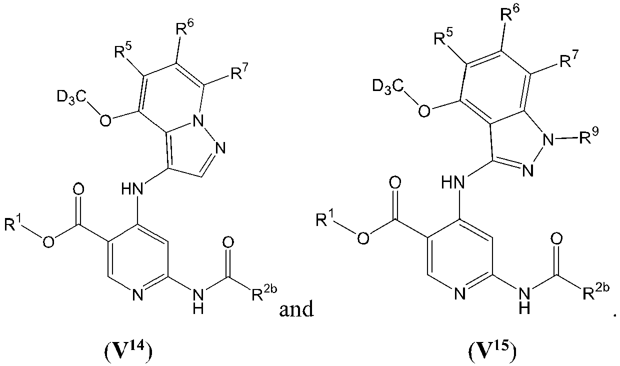

In certain embodiments of **(III^{a}),** the compound has a structural formula selected from:

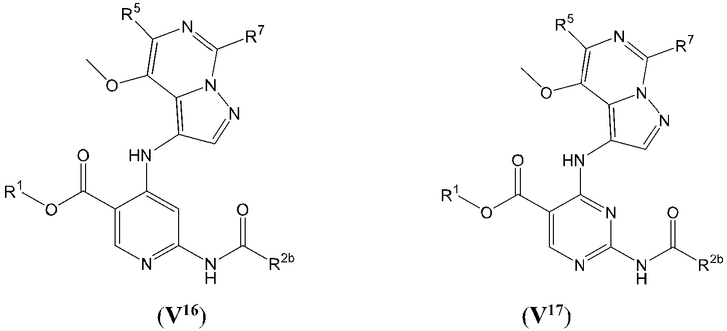

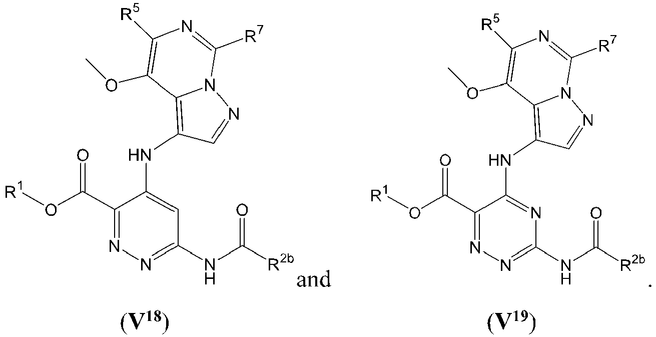

In certain embodiments of **(III^{a}),** the compound has a structural formula selected from: 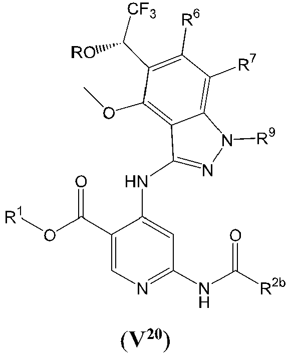 wherein R is H, CD₃, C₁₋₃ alkyl or acyl.

In certain embodiments of **(III^{a}),** the compound has a structural formula selected from: 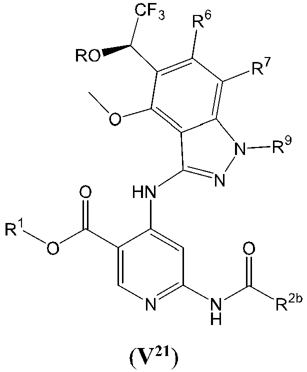 wherein R is H, CD₃, C₁₋₃ alkyl or acyl.

In certain embodiments of **(III^{a}),** the compound has a structural formula selected from: 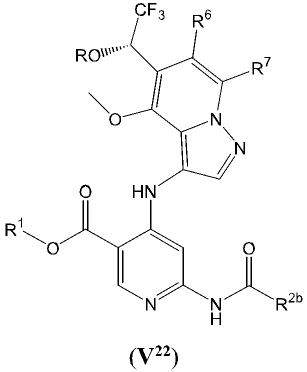 wherein R is H, CD₃, C₁₋₃ alkyl or acyl.

In certain embodiments of **(III^{a}),** the compound has a structural formula selected from: 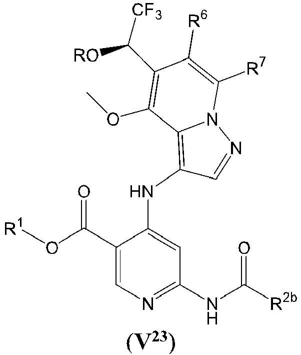 wherein R is H, CD₃, C₁₋₃ alkyl or acyl.

In certain embodiments of **(III^{a}),** the compound has a structural formula selected from: 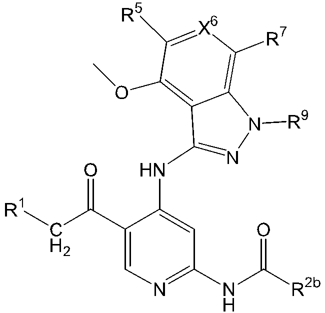 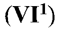 wherein X⁶ is N or CH.

In certain embodiments of **(III^{a}),** the compound has the structural formula: 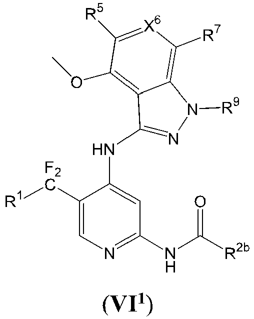 wherein X⁶ is N or CH.

In certain embodiments of **(III^{a}),** the compound has a structural formula selected from:

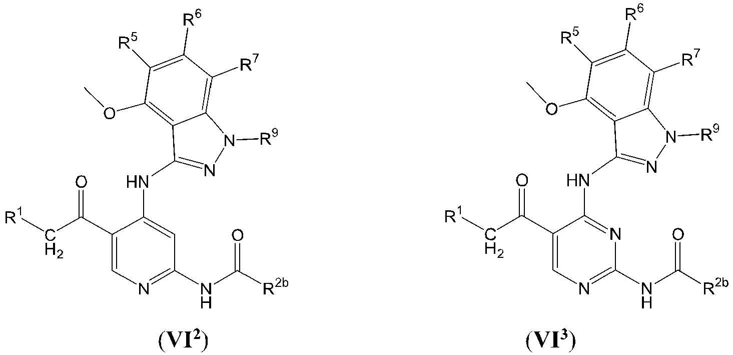

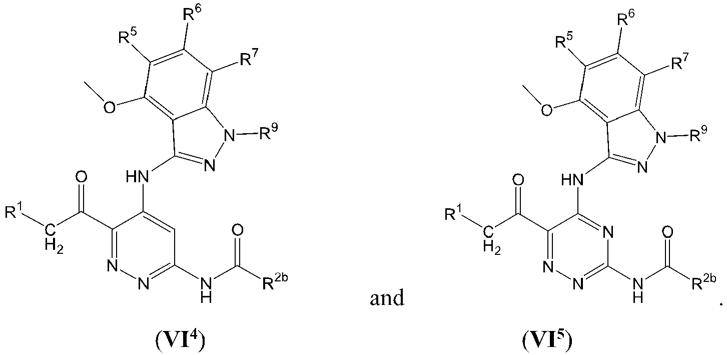

In certain embodiments of **(III^{a}),** the compound has a structural formula selected from:

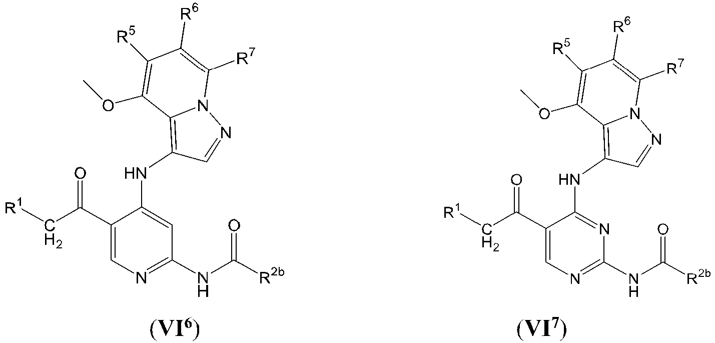

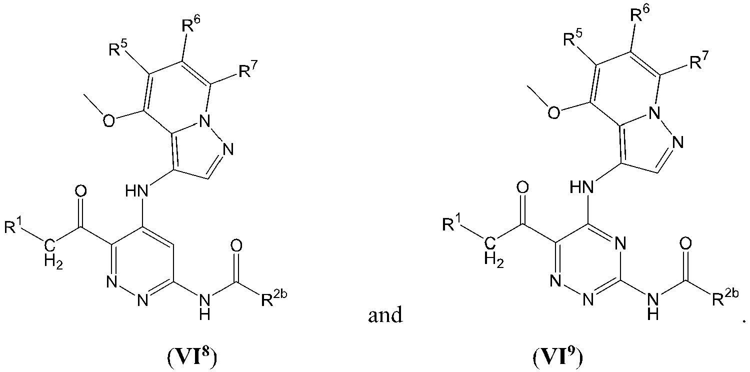

In certain embodiments of **(III^{a}),** the compound has a structural formula selected from:

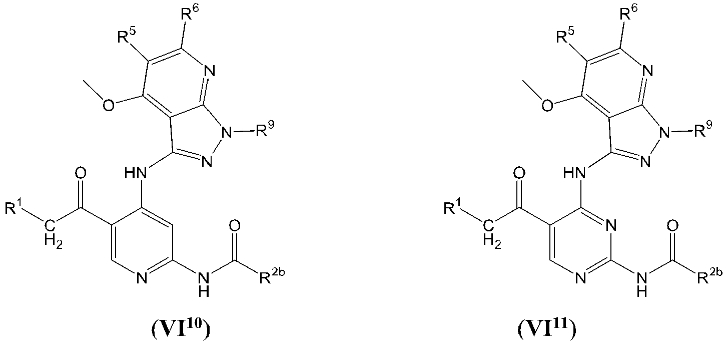

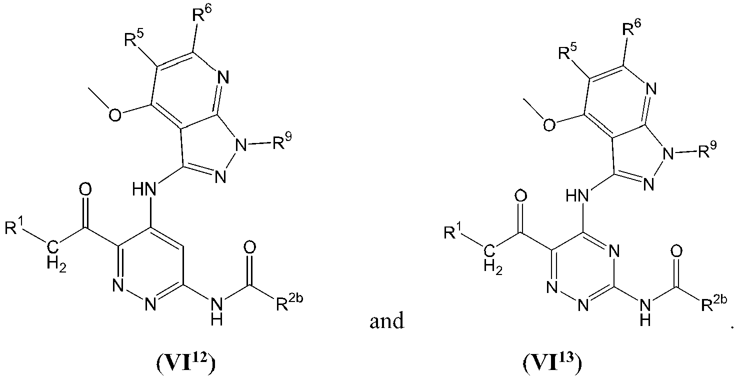

In certain embodiments of **(III^{a}),** the compound has a structural formula selected from:

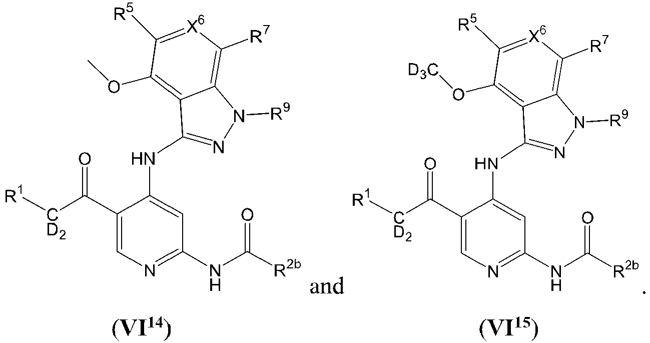

In certain embodiments of **(III^{a}),** the compound has a structural formula selected from:

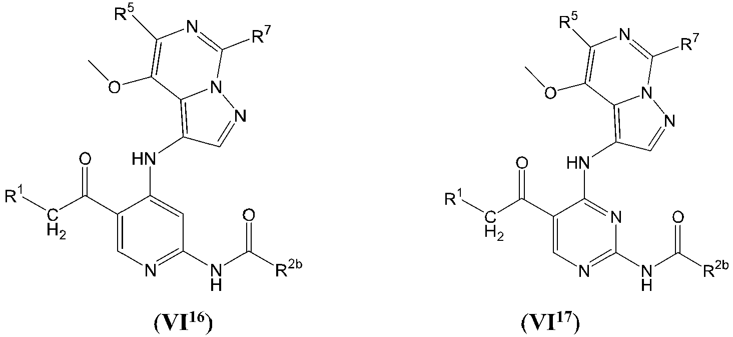

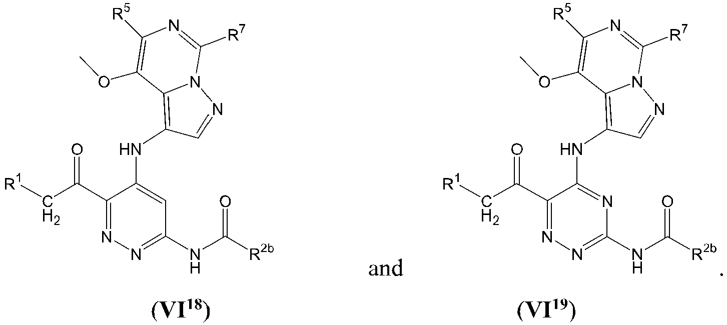

In certain embodiments of **(III^{b}),** the compound has the structural formula: 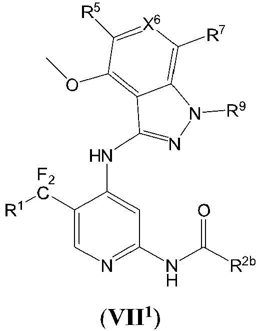 wherein X⁶ is N or CH.

In certain embodiments of **(III^{b}),** the compound has the structural formula: 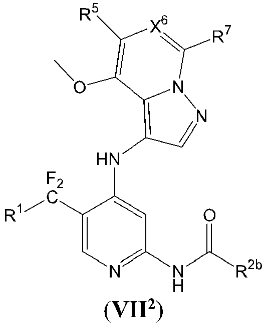 wherein X⁶ is N or CH.

In certain embodiments of **(IV¹)-(VII),** each of R⁶ and R⁷, if present, is H.

In certain embodiments of **(IV¹)-(VII),** wherein R⁹ if present is C₁₋₃ alkyl or cyclopropyl, each optionally substituted with C₁-C₃ alkoxy, CF₃ or NRR'.

In certain embodiments of **(IV¹)-(VII),** R⁹ is C₁₋₃ alkyl or cyclopropyl.

In certain embodiments of **(IV¹)-(VII),** R⁹ is CH₃.

In certain embodiments of **(IV¹)-(VII),** R⁹ is CD₃.

In certain embodiments of **(IV¹)-(VII),** R^{2b} is C₁-C₆ alkyl, cyclopropyl or cyclobutyl, substituted with 0-2 R^{2c}.

In certain embodiments of **(IV¹)-(VII),** R^{2b} is cyclopropyl.

In certain embodiments of **(IV¹)-(VII),** R⁵ is: 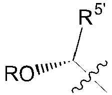 wherein
R^{5'} is a C₁₋₃ alkyl or cyclopropyl, substituted with 0-5 F's; and
R is H, C₁₋₃ alkyl or acyl.

In certain embodiments of **(IV¹)-(VII),** R⁵ is: 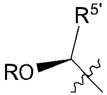 wherein
R^{5'} is a C₁₋₃ alkyl or cyclopropyl, substituted with 0-5 F's; and
R is H, C₁₋₃ alkyl or acyl.

In certain embodiments, R is H.

In certain embodiments, R is CH₃.

In certain embodiments, R is CD₃.

In certain embodiments, R^{5'} is CF₃.

In certain embodiments, R^{5'} is CHF₂.

In certain embodiments, R^{5'} is a C₂₋₃ alkyl, substituted with 2-5 F's.

In certain embodiments of **(IV¹)-(VII),** R¹ is CH₃.

In certain embodiments of **(IV¹)-(VII),** R¹ is CD₃.

In another aspect, the invention generally relates to a compound having the structural formula **(VIII):** 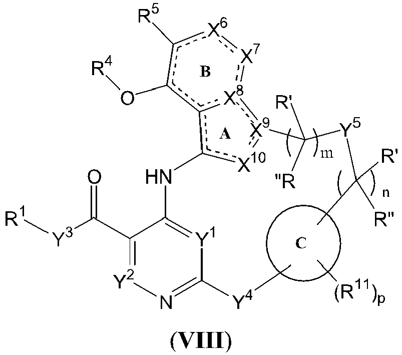 or a pharmaceutically acceptable form or an isotope derivative thereof,
wherein
X⁶ is CR⁶ or N;
X⁷ is CR⁷ or N;
X⁸ is C or N;
X⁹ is CR⁹, O, S, N or NR⁹;
X¹⁰ is CR¹⁰, O, S, N or NR¹⁰;
Y¹ is CH, CF or N;
Y² is CH or N;
Y³ is NR, O, CH₂, CD₂, CF₂ or O-NH;
Y⁴ is NR, CH₂ or CF₂;
Y⁵ is NR, CH₂, O, S, SO or SO₂;
m is 0, 1, 2 and 3;
*n is* 0, 1, 2 and 3;
*p* is 0, 1, 2 and 3;
each of Ring A and Ring B is independently an aryl or heteroaryl group;
Ring C is a 5- or 6-membered aryl or heteroaryl group;
R¹ is H, F, CD₃, or C₁₋₃ alkyl, provided that R¹ is not F when Y³ is N, O or O-NH;
R⁴ is a C₁₋₃ alkyl, substituted with 0-5 R^{4a}, wherein R^{4a} is selected from D, F and Cl;
R⁵ is H, CN, halo, OCH₃, C(=O)OR, NHC(=O)R, NRR', NO₂, C₁₋₆ alkyl, C₃₋₆ cycloalkyl or heterocyclic, wherein said alkyl, cycloalkyl or heterocyclic is substituted with 0-3 R^{5a}, wherein each R^{5a} is independently selected from OH, D, F, Cl, CN, CH₂F, CHF₂, CF₃, OCH₃, OCD₃, OCF₃ and OC(=O)CH₃ ;
each of R⁶, R⁷, R⁹, R¹⁰ and R¹¹ is independently selected from H, F, Cl, CN, CD₃, CH₂CF₃, CF₃, OR, NRR', C₁₋₃ alkyl and C₃₋₅ cycloalkyl, wherein said alkyl, cycloalkyl, R and R' are substituted with 0-2 R^{2a};
R^{2a} is selected from F, OCF₃, CF₃, CN, NO₂, OR, NRR' and C₁₋₆ alkyl; and
each of R and R' independently H, C₁-C₆ alkyl or acyl, or R and R' together with the nitrogen or carbon atom to which they are bonded, form a 3- to 6-membered ring comprising 0-2 heteroatoms selected from O, NR, S and SO₂.

In certain embodiments of **(VIII),** Y¹ is CH and Y² is CH.

In certain embodiments of **(VIII),** Y¹ is N and Y² is CH.

In certain embodiments of **(VIII),** Y¹ is CH and Y² is N.

In certain embodiments of **(VIII),** Y¹ is N and Y² is N.

In certain embodiments of **(VIII),** Y³ is NH.

In certain embodiments of **(VIII),** Y³ is O.

In certain embodiments of **(VIII),** Y³ is CH₂.

In certain embodiments of **(VIII),** Y³ is CD₂.

In certain embodiments of **(VIII),** Y⁴ is NH.

In certain embodiments of **(VIII),** Y⁴ is CH₂.

In certain embodiments of **(VIII),** R¹ is CH₃.

In certain embodiments of **(VIII),** R¹ is CD₃.

In certain embodiments of **(VIII),** R⁴ is CH₃.

In certain embodiments of **(VIII),** R⁴ is CD₃.

Non-limiting examples of compounds of the invention include those listed in **Table 1** in the Examples section herein.

In yet another aspect, the invention generally relates to a method for preparing a compound disclosed herein, as exemplified by the synthetic schemes and experimental procedure disclosed herein.

In another aspect, the invention generally relates to a pharmaceutical composition comprising a compound disclosed herein, effective to treat or reduce one or more diseases or disorders, in a mammal, including a human, and a pharmaceutically acceptable excipient, carrier, or diluent.

In yet another aspect, the invention generally relates to a pharmaceutical composition comprising an amount of a compound having the structural formula of (I): 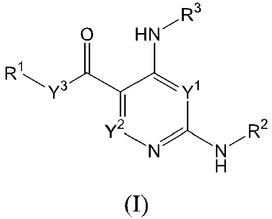 or a pharmaceutically acceptable form or an isotope derivative thereof,
wherein
Y¹ is CH, CF or N;
Y² is CH or N;
Y³ is NR, O, CH₂, CD₂, CF₂ or O-NH;
R¹ is H, F, C₁-C₃ alkyl and CD₃, provided that R¹ is not F when Y³ is N, O or O-NH;
R² is
   R^{2'}, wherein R^{2'} is C₁-C₆ alkyl, C₃₋₆ cycloalkyl, C₅₋₇ spirocycloalkyl, or C₃-C₆ heterocycloalkyl, each substituted with 0-2 R^{2a}, wherein R^{2a} is selected from the group consisting of halogen, CN, OR, NRR', alkyl, cycloalkyl, heterocyclic;
   an aryl or heteroaryl group, each substituted with 0-2 R^{2a};
   (C=O)R^{2b}; or
   (C=O)NHR^{2b};
R³ is 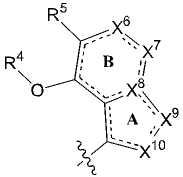 wherein
X⁶ is CR⁶ or N;
X⁷ is CR⁷ or N;
X⁸ is C or N;
X⁹ is CR⁹, O, S, N or NR⁹;
X¹⁰ is CR¹⁰, O, S, N or NR¹⁰; and
   wherein each of Ring A and Ring B is independently an aryl or heteroaryl group;
R^{2b} is a C₁₋₆ alkyl or C₃₋₆ cycloalkyl, C₅₋₇ spirocycloalkyl, aryl or heteroaryl, each substituted with 0-2 R^{2c};
R^{2c} at each occurrence is independently halo, CN, OR, NRR', OCF₃, CF₃, C₁₋₆ alkyl, C₁₋₆ haloalkyl, C₂₋₆ alkenyl, C₂₋₆ alkynyl, wherein said alkyl, haloalkyl, alkenyl, alkynyl, R and R' are substituted with 0-3 R^{2a}; and
R⁴ is a C₁₋₃ alkyl, substituted with 0-5 R^{4a}, wherein R^{4a} is selected from D, F and Cl;
R⁵ is H, CN, halo, OCH₃, C(=O)OCH₃, C₁₋₆ alkyl, C₃-C₆ cycloalkyl or heterocyclic, wherein said alkyl, cycloalkyl or heterocyclic is substituted with 0-3 R^{5a}, wherein each R^{5a} is independently selected from OH, D, F, Cl, CN, OCH₃, OCD₃, OCF₃ and OC(=O)CH₃;
each of R⁶, R⁷, R⁹ and R¹⁰ is independently selected from H, F, Cl, CN, CD₃, CH₂CF₃, CF₃, OR, NRR', C₁-C₃ alkyl and C₃-C₅ cycloalkyl, wherein said alkyl, cycloalkyl, R and R' are substituted with 0-2 R^{2a}; and
each of R and R' is independently H or a C₁-C₆ alkyl or acyl, or R and R', together with the nitrogen atom to which they are bonded, form a 4- to 7-membered ring comprising 0-2 heteroatoms selected from O, NR, S and SO₂,
effective to treat, or reduce one or more diseases or disorders, in a mammal, including a human, and a pharmaceutically acceptable excipient, carrier, or diluent.

In certain embodiments, the pharmaceutical composition is suitable for oral administration.

In certain embodiments, the pharmaceutical composition is suitable for topical administration.

In certain embodiments, the pharmaceutical composition is suitable for GI-restricted administration.

In certain embodiments, the pharmaceutical composition is useful to treat or reduce one or more of inflammatory diseases, immune-mediated diseases and cancers, or a related disease or disorder.

In certain embodiments, the disease or disorder is an inflammatory disease.

In certain embodiments, the disease or disorder is an immune-mediated disease.

In certain embodiments, the disease or disorder is cancer.

In certain embodiments, the disease or disorder is selected from: inflammatory bowel disease, psoriasis, psoriatic arthritis, alopecia areata, eczema, ankylosing spondylitis (AS), vitiligo, atopic dermatitis, discoid lupus erythematosus (DLE), subacute cutaneous lupus erythematosus (SCLE), systemic lupus erythematosus (SLE), Sjogren's syndrome, scleroderma, Crohn's Disease (CD), rheumatoid arthritis (RA), T-cell acute lymphoblastic leukemia (T-ALL), cutaneous T-cell lymphomas (CTCL), multiple sclerosis (MS), Alzheimer's Disease (AD), Parkinson's Disease (PD), type I diabetes, asthma, kidney fibrosis, diabetic nephropathy, polycystic kidney disease, HIV-associated nephropathy, chronic myelogenous leukemia (CML), essential thrombocythemia (ET), polycythemia vera (PV), myelofibrosis (MF), breast cancer and ovarian cancer.

In yet another aspect, the invention generally relates to a unit dosage form comprising a pharmaceutical composition disclosed herein.

In certain embodiments, the unit dosage form is a tablet.

In certain embodiments, the unit dosage form is a capsule.

In certain embodiments, the unit dosage form is a topical formulation.

In yet another aspect, the invention generally relates to a method for treating, reducing or preventing a disease or disorder, comprising administering to a subject in need thereof a therapeutically effective amount of a compound disclosed herein, wherein the disease or disorder is selected from inflammatory diseases, immune-mediated diseases, cancer, or a related disease or disorder thereof, in a mammal, including a human.

In yet another aspect, the invention generally relates to a method for treating, reducing or preventing a disease or disorder, comprising administering to a subject in need thereof a therapeutically effective amount of a compound having the structural formula of (I): 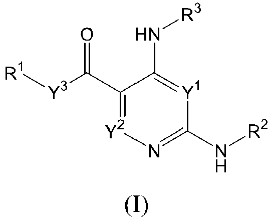 or a pharmaceutically acceptable form or an isotope derivative thereof,
wherein
Y¹ is CH, CF or N;
Y² is CH or N;
Y³ is NR, O, CH₂, CD₂, CF₂ or O-NH;
R¹ is H, F, C₁-C₃ alkyl and CD₃, provided that R¹ is not F when Y³ is N, O or O-NH;
R² is
   R^{2'}, wherein R^{2'} is C₁₋₆ alkyl, C₃₋₆ cycloalkyl, C₅₋₇ spirocycloalkyl, or C₃-C₆ heterocycloalkyl, each substituted with 0-2 R^{2a}, wherein R^{2a} is selected from the group consisting of halogen, CN, OR, NRR', alkyl, cycloalkyl, heterocyclic;
   an aryl or heteroaryl group, each substituted with 0-2 R^{2a};
   (C=O)R^{2b}; or
   (C=O)NHR^{2b};
R³ is 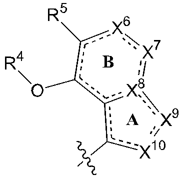 wherein
X⁶ is CR⁶ or N;
X⁷ is CR⁷ or N;
X⁸ is C or^{N};
X⁹ is CR⁹, O, S, N or NR⁹;
X¹⁰ is CR¹⁰, O, S, N or NR¹⁰; and wherein each of Ring A and Ring B is independently an aryl or heteroaryl group;
R^{2b} is a C₁₋₆ alkyl or C₃₋₆ cycloalkyl, C₅₋₇ spirocycloalkyl, aryl or heteroaryl, each substituted with 0-2 R^{2c};
R^{2c} at each occurrence is independently halo, CN, OR, NRR', OCF₃, CF₃, C₁₋₆ alkyl, C₁₋₆ haloalkyl, C₂₋₆ alkenyl, C₂₋₆ alkynyl, wherein said alkyl, haloalkyl, alkenyl, alkynyl, R and R' are substituted with 0-3 R^{2a}; and
R⁴ is a C₁₋₃ alkyl, substituted with 0-5 R^{4a}, wherein R^{4a} is selected from D, F and Cl;
R⁵ is H, CN, halo, OCH₃, C(=O)OCH₃, C₁₋₆ alkyl, C₃-C₆ cycloalkyl or heterocyclic, wherein said alkyl, cycloalkyl or heterocyclic is substituted with 0-3 R^{5a}, wherein each R^{5a} is independently selected from OH, D, F, Cl, CN, OCH₃, OCD₃, OCF₃ and OC(=O)CH₃;
each of R⁶, R⁷, R⁹ and R¹⁰ is independently selected from H, F, Cl, CN, CD₃, CH₂CF₃, CF₃, OR, NRR', C₁-C₃ alkyl and C₃-C₅ cycloalkyl, wherein said alkyl, cycloalkyl, R and R' are substituted with 0-2 R^{2a}; and
each of R and R' is independently H or a C₁-C₆ alkyl or acyl, or R and R', together with the nitrogen atom to which they are bound, form a 4- to 7-membered ring comprising 0-2 heteroatoms selected from O, NR, S and SO₂,
wherein the disease or disorder is selected from inflammatory diseases, immune-mediated diseases, cancer, or a related disease or disorder thereof, in a mammal, including a human.

In certain embodiments, the disease or disorder is an inflammatory disease.

In certain embodiments, the disease or disorder is an immune-mediated disease.

In certain embodiments, the disease or disorder is cancer.

In certain embodiments, the disease or disorder is selected from: inflammatory bowel disease, psoriasis, psoriatic arthritis, alopecia areata, eczema, ankylosing spondylitis (AS), vitiligo, atopic dermatitis, discoid lupus erythematosus (DLE), subacute cutaneous lupus erythematosus (SCLE), systemic lupus erythematosus (SLE), Sjogren's syndrome, scleroderma, Crohn's Disease (CD), rheumatoid arthritis (RA), T-cell acute lymphoblastic leukemia (T-ALL), cutaneous T-cell lymphomas (CTCL), multiple sclerosis (MS), Alzheimer's Disease (AD), Parkinson's Disease (PD), type I diabetes, asthma, kidney fibrosis, diabetic nephropathy, polycystic kidney disease, HIV-associated nephropathy, chronic myelogenous leukemia (CML), essential thrombocythemia (ET), polycythemia vera (PV), myelofibrosis (MF), breast cancer and ovarian cancer.

In certain embodiments, administration is via oral administration.

In certain embodiments, administration is via topical administration.

In certain embodiments, administration is via GI-restricted administration.

In yet another aspect, the invention generally relates to use of a compound disclosed herein, and a pharmaceutically acceptable excipient, carrier, or diluent, in preparation of a medicament for treating a disease or disorder.

In certain embodiments of the use, the disease or disorder is one or more of inflammatory diseases, immune-mediated diseases and cancer.

In certain embodiments of the use, the disease or disorder is an inflammatory disease.

In certain embodiments of the use, the disease or disorder is an immune-mediated disease.

In certain embodiments of the use, the disease or disorder is cancer.

In certain embodiments of the use, the medicament is for oral administration.

In certain embodiments of the use, the medicament is for topical administration.

In certain embodiments of the use, the medicament is for GI restriction administration.

As discussed herein, isotope derivative compounds having one or more hydrogen atoms (*e.g.,* 1, 2, 4, 5, 6, 7, 8, 9, 10, *etc.*) replaced with deuterium atoms are contemplated in the presented invention.

The term "inflammatory disease" refers to a disease or condition characterized by aberrant inflammation, e.g. an increased level of inflammation compared to a control such as a healthy person not suffering from a disease. Examples of inflammatory diseases that may be treated with a compound, pharmaceutical composition, or method described herein include autoimmune diseases, traumatic brain injury, arthritis, rheumatoid arthritis, psoriatic arthritis, juvenile idiopathic arthritis, multiple sclerosis, systemic lupus erythematosus (SLE), myasthenia gravis, juvenile onset diabetes, diabetes mellitus type 1, Guillain-Barre syndrome, Hashimoto's encephalitis, Hashimoto's thyroiditis, ankylosing spondylitis, psoriasis, Sjogren's syndrome, vasculitis, glomerulonephritis, auto-immune thyroiditis, Behcet's disease, Crohn's disease, ulcerative colitis, bullous pemphigoid, sarcoidosis, ichthyosis, Graves ophthalmopathy, inflammatory bowel disease, Addison's disease, Vitiligo, asthma, allergic asthma, acne vulgaris, celiac disease, chronic prostatitis, inflammatory bowel disease, pelvic inflammatory disease, reperfusion injury, ischemia reperfusion injury, stroke, sarcoidosis, transplant rejection, interstitial cystitis, atherosclerosis, scleroderma, and atopic dermatitis. Such conditions are frequently inextricably intertwined with other diseases, disorders and conditions. A non-limiting list of inflammatory-related diseases, disorders and conditions which may, for example, be caused by inflammatory cytokines, include, arthritis, kidney failure, lupus, asthma, psoriasis, colitis, pancreatitis, allergies, fibrosis, surgical complications (*e.g*., where inflammatory cytokines prevent healing), anemia, and fibromyalgia. Other diseases and disorders, which may be associated with chronic inflammation include Alzheimer's disease, congestive heart failure, stroke, aortic valve stenosis, arteriosclerosis, osteoporosis, Parkinson's disease, infections, inflammatory bowel disease (IBD), allergic contact dermatitis and other eczemas, systemic sclerosis, transplantation and multiple sclerosis. Some of the aforementioned diseases, disorders and conditions for which a compound of the present disclosure may be particularly efficacious (due to, for example, limitations of current therapies) are described in more detail hereafter.

The term "autoimmune disease" refers to a disease or condition in which a subject's immune system has an aberrant immune response against a substance that does not normally elicit an immune response in a healthy subject. Examples of autoimmune diseases that may be treated with a compound, pharmaceutical composition, or method described herein include acne vulgaris, acute disseminated encephalomyelitis, acute necrotizing hemorrhagic leukoencephalitis, Addison's disease, agammaglobulinemia, Aicardi-Goutières syndrome (AGS), alopecia areata, alopecia totalis, amyloidosis, ankylosing spondylitis, anti-GBM/anti-TBM nephritis, antiphospholipid syndrome, autoimmune angioedema, autoimmune aplastic anemia, autoimmune dysautonomia, autoimmune hepatitis, autoimmune hyperlipidemia, autoimmune immunodeficiency, autoimmune inner ear disease, autoimmune myocarditis, autoimmune oophoritis, autoimmune pancreatitis, autoimmune retinopathy, autoimmune thrombocytopenic purpura, autoimmune thyroid disease, autoimmune urticaria, axonal or neuronal neuropathies, balo disease, Behcet's disease, bullous pemphigoid, cardiomyopathy, Castleman disease, celiac disease, Chagas disease, chronic atypical neutrophilic dermatosis with lipodystrophy and elevated temperature (CANDLE), chronic active hepatitis, chronic fatigue syndrome, chronic inflammatory demyelinating polyneuropathy, chronic recurrent multifocal ostomyelitis, Churg-Strauss syndrome, cicatricial pemphigoid/benign mucosal pemphigoid, Crohn's disease, Cogans syndrome, cold agglutinin disease, congenital heart block, coxsackie myocarditis, CREST disease, Cushing's disease, demyelinating neuropathies, depression, dermatitis herpetiformis, dermatomyositis, Devic's disease (neuromyelitis optica), discoid lupus, Dressler's syndrome, dry eye syndrome DES (keratoconjunctivitis sicca), endometriosis, eosinophilic esophagitis, eosinophilic fasciitis, erythema nodosum, essential mixed cryoglobulinemia, experimental allergic encephalomyelitis, Evans syndrome, fibromyalgia, fibrosing alveolitis, giant cell arteritis (temporal arteritis), giant cell myocarditis, glomerulonephritis, Goodpasture's syndrome, granulomatosis with polyangiitis, graft-versus-host disease (GVDH), Graves' disease, Guillain-Barre syndrome, Hashimoto's encephalitis, Hashimoto's thyroiditis, hemolytic anemia, Henoch-Schonlein purpura, herpes gestationis, hidradenitis suppurativa, hypogammaglobulinemia, idiopathic thrombocytopenic purpura, IgA nephropathy, IgG4-related sclerosing disease, inflammatory bowel disease (IBD), immunoregulatory lipoproteins, inclusion body myositis, interstitial cystitis, juvenile arthritis, juvenile diabetes (Type 1 diabetes), juvenile dermatomyositis (JDM), juvenile myositis, Kawasaki syndrome, Lambert-Eaton syndrome, leukocytoclastic vasculitis, lichen planus, lichen sclerosus, ligneous conjunctivitis, linear IgA disease, lupus, lyme disease, chronic, Meniere's disease, microscopic polyangiitis, mixed connective tissue disease, Mooren's ulcer, Mucha-Habermann disease, multiple sclerosis (MS), myasthenia gravis, myositis, narcolepsy, neuromyelitis optica, neutropenia, ocular cicatricial pemphigoid, optic neuritis, palindromic rheumatism, pediatric autoimmune neuropsychiatric disorders associated with streptococcus, paraneoplastic cerebellar degeneration, paroxysmal nocturnal hemoglobinuria p, Parry Romberg syndrome, Parsonnage-Turner syndrome, Pars planitis (peripheral uveitis), pemphigus, peripheral neuropathy, perivenous encephalomyelitis, pernicious anemia, POEMS syndrome, polyarteritis nodosa, polycystic ovary syndrome (PCOS), Type I, II, & III autoimmune polyglandular syndromes, polymyalgia rheumatica, polymyositis, postmyocardial infarction syndrome, postpericardiotomy syndrome, progesterone dermatitis, primary biliary cirrhosis, primary sclerosing cholangitis, psoriasis, psoriatic arthritis, plaque psoriasis, idiopathic pulmonary fibrosis, pyoderma gangrenosum, pure red cell aplasia, Raynauds phenomenon, reactive Arthritis, reflex sympathetic dystrophy, Reiter's syndrome, relapsing polychondritis, restless legs syndrome, retroperitoneal fibrosis, rheumatic fever, rheumatoid arthritis, sarcoidosis, Schmidt syndrome, scleritis, scleroderma, Sjogren's syndrome, sperm & testicular autoimmunity, stiff person syndrome, stimulator of interferon genes (STING)-associated vasculopathy with onset during infancy (SAVI), subacute bacterial endocarditis, Susac's syndrome, sympathetic ophthalmia, systemic lupus erythematosus (SLE), Takayasu's arteritis, temporal arteritis/Giant cell arteritis, thrombocytopenic purpura, Tolosa-Hunt syndrome, transplant rejection (allograft transplant rejection), transverse myelitis, Type 1 diabetes, ulcerative colitis, undifferentiated connective tissue disease, uveitis, vasculitis, vesiculobullous dermatosis, vitiligo, or Wegener's granulomatosis.

The term "immune-mediated disease" refers to chronic inflammatory diseases perpetuated by antibodies and cellular immunity. Immune-mediated diseases include, for example, but not limited to, asthma, allergies, arthritis (e.g., rheumatoid arthritis, psoriatic arthritis, and ankylosing spondylitis), juvenile arthritis, inflammatory bowel diseases (e.g., ulcerative colitis and Crohn's disease), endocrinopathies (e.g., type 1 diabetes and Graves' disease), neurodegenerative diseases (e.g., multiple sclerosis (MS)), autistic spectrum disorder, depression, Alzheimer's disease, Guillain-Barre syndrome, obsessive-compulsive disorder, optic neuritis, retinal degeneration, dry eye syndrome DES, Sjogren's syndrome, amyotrophic lateral sclerosis (ALS), Parkinson's disease, Huntington's Disease, Guillain-Barre syndrome, myasthenia gravis, and chronic idiopathic demyelinating disease (CID)), vascular diseases (e.g., autoimmune hearing loss, systemic vasculitis, and atherosclerosis), and skin diseases (e.g., acne vulgaris dermatomyositis, pemphigus, systemic lupus erythematosus (SLE), discoid lupus erthematosus, scleroderma, psoriasis, plaque psoriasis, vasculitics, vitiligo and alopecias). Hashimoto's thyroiditis, pernicious anemia, Cushing's disease, Addison's disease, chronic active hepatitis, polycystic ovary syndrome (PCOS), celiac disease, pemphigus, transplant rejection (allograft transplant rejection), graft-versus-host disease (GVDH).

The term "cancer" as used herein refers to all types of cancer, neoplasm or malignant tumors found in mammals, e.g., humans, including hematological cancers leukemia, and lymphomas, T-ALL, large B-cell lymphoma, solid cancers such as carcinomas and sarcomas. Exemplary cancers include blood cancer, brain cancer, glioma, glioblastoma, neuroblastoma, prostate cancer, colorectal cancer, pancreatic cancer, cervical cancer, gastric cancer, ovarian cancer, lung cancer, and cancer of the head. Exemplary cancers include cancer of the thyroid, endocrine system, brain, breast, cervix, colon, head & neck, liver, kidney, lung, non-small cell lung, melanoma, mesothelioma, ovary, sarcoma, stomach, uterus, medulloblastoma, colorectal cancer, pancreatic cancer. Additional examples include penile, skin - non-melanoma, anal, hepatobiliary, esophagogastric, uterine sarcoma, gastrointestinal stromal tumor, salivary gland, peripheral nervous system, soft tissue sarcoma, bone, renal, myeloproliferative neoplasms, thyroid carcinoma, cholangiocarcinoma, pancreatic adenocarcinoma, skin cutaneous melanoma, colon adenocarcinoma, rectum adenocarcinoma, stomach adenocarcinoma, esophageal carcinoma, head and neck squamous cell carcinoma, breast invasive carcinoma, lung adenocarcinoma, lung squamous cell carcinoma, Hodgkin's Disease, Non-Hodgkin's Lymphoma, multiple myeloma, neuroblastoma, glioma, glioblastoma multiforme, ovarian cancer, rhabdomyosarcoma, primary thrombocytosis, primary macroglobulinemia, primary brain tumors, cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, endometrial cancer, adrenal cortical cancer, neoplasms of the endocrine or exocrine pancreas, medullary thyroid cancer, medullary thyroid carcinoma, melanoma, colorectal cancer, papillary thyroid cancer, hepatocellular carcinoma, metastatic leiomyosarcoma, synovial sarcoma, undifferentiated pleomorphic sarcoma, round cell liposarcoma or prostate cancer.

In certain embodiments of the use, the disease or disorder is selected from: inflammatory bowel disease, psoriasis, psoriatic arthritis, alopecia areata, eczema, ankylosing spondylitis (AS), vitiligo, atopic dermatitis, discoid lupus erythematosus (DLE), subacute cutaneous lupus erythematosus (SCLE), systemic lupus erythematosus (SLE), Sjogren's syndrome, scleroderma, Crohn's Disease (CD), rheumatoid arthritis (RA), T-cell acute lymphoblastic leukemia (T-ALL), cutaneous T-cell lymphomas (CTCL), multiple sclerosis (MS), Alzheimer's Disease (AD), Parkinson's Disease (PD), type I diabetes, asthma, kidney fibrosis, diabetic nephropathy, polycystic kidney disease, HIV-associated nephropathy, chronic myelogenous leukemia (CML), essential thrombocythemia (ET), polycythemia vera (PV), myelofibrosis (MF), breast cancer and ovarian cancer.

Isotopically-labeled compounds are also within the scope of the present disclosure. As used herein, an "isotopically-labeled compound" refers to a presently disclosed compound including pharmaceutical salts and prodrugs thereof, each as described herein, in which one or more atoms are replaced by an atom having an atomic mass or mass number different from the atomic mass or mass number usually found in nature. Examples of isotopes that can be incorporated into compounds presently disclosed include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

By isotopically-labeling the presently disclosed compounds, the compounds may be useful in drug and/or substrate tissue distribution assays. Tritiated (³H) and carbon-14 (¹⁴C) labeled compounds are particularly preferred for their ease of preparation and detectability. Further, substitution with heavier isotopes such as deuterium (²H) can afford certain therapeutic advantages resulting from greater metabolic stability, for example increased *in vivo* half-life or reduced dosage requirements and, hence, may be preferred in some circumstances. Isotopically labeled compounds presently disclosed, including pharmaceutical salts, esters, and prodrugs thereof, can be prepared by any means known in the art.

Further, substitution of normally abundant hydrogen (¹H) with heavier isotopes such as deuterium can afford certain therapeutic advantages, *e.g*., resulting from improved absorption, distribution, metabolism and/or excretion (ADME) properties, creating drugs with improved efficacy, safety, and/or tolerability. Benefits may also be obtained from replacement of normally abundant ¹²C with ¹³C. (See, WO 2007/005643, WO 2007/005644, WO 2007/016361, and WO 2007/016431.)

Stereoisomers (*e.g.,* cis and trans isomers) and all optical isomers of a presently disclosed compound (*e.g*., R and S enantiomers), as well as racemic, diastereomeric and other mixtures of such isomers are within the scope of the present disclosure.

Compounds of the present invention are, subsequent to their preparation, preferably isolated and purified to obtain a composition containing an amount by weight equal to or greater than 95% ("substantially pure"), which is then used or formulated as described herein. In certain embodiments, the compounds of the present invention are more than 99% pure. Solvates and polymorphs of the compounds of the invention are also contemplated herein. Solvates of the compounds of the present invention include, for example, hydrates.

Any appropriate route of administration can be employed, for example, parenteral, intravenous, subcutaneous, intramuscular, intraventricular, intracorporeal, intraperitoneal, rectal, or oral administration. Most suitable means of administration for a particular patient will depend on the nature and severity of the disease or condition being treated or the nature of the therapy being used and on the nature of the active compound.

Solid dosage forms for oral administration include capsules, tablets, pills, powders, and granules. In such solid dosage forms, the compounds described herein or derivatives thereof are admixed with at least one inert customary excipient (or carrier) such as sodium citrate or dicalcium phosphate or (i) fillers or extenders, as for example, starches, lactose, sucrose, glucose, mannitol, and silicic acid, (ii) binders, as for example, carboxymethylcellulose, alignates, gelatin, polyvinylpyrrolidone, sucrose, and acacia, (iii) humectants, as for example, glycerol, (iv) disintegrating agents, as for example, agar-agar, calcium carbonate, potato or tapioca starch, alginic acid, certain complex silicates, and sodium carbonate, (v) solution retarders, as for example, paraffin, (vi) absorption accelerators, as for example, quaternary ammonium compounds, (vii) wetting agents, as for example, cetyl alcohol, and glycerol monostearate, (viii) adsorbents, as for example, kaolin and bentonite, and (ix) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents. Solid compositions of a similar type may also be employed as fillers in soft and hard- filled gelatin capsules using such excipients as lactose or milk sugar as well as high molecular weight polyethyleneglycols, and the like. Solid dosage forms such as tablets, dragees, capsules, pills, and granules can be prepared with coatings and shells, such as enteric coatings and others known in the art.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents, and emulsifiers, such as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil, sesame oil, glycerol, tetrahydrofurfuryl alcohol, polyethyleneglycols, and fatty acid esters of sorbitan, or mixtures of these substances, and the like. Besides such inert diluents, the composition can also include additional agents, such as wetting, emulsifying, suspending, sweetening, flavoring, or perfuming agents.

Materials, compositions, and components disclosed herein can be used for, can be used in conjunction with, can be used in preparation for, or are products of the disclosed methods and compositions. It is understood that when combinations, subsets, interactions, groups, *etc.* of these materials are disclosed that while specific reference of each various individual and collective combinations and permutations of these compounds may not be explicitly disclosed, each is specifically contemplated and described herein. For example, if a method is disclosed and discussed and a number of modifications that can be made to a number of molecules including in the method are discussed, each and every combination and permutation of the method, and the modifications that are possible are specifically contemplated unless specifically indicated to the contrary. Likewise, any subset or combination of these is also specifically contemplated and disclosed. This concept applies to all aspects of this disclosure including, but not limited to, steps in methods using the disclosed compositions. Thus, if there are a variety of additional steps that can be performed, it is understood that each of these additional steps can be performed with any specific method steps or combination of method steps of the disclosed methods, and that each such combination or subset of combinations is specifically contemplated and should be considered disclosed.

The following examples are meant to be illustrative of the practice of the invention and not limiting in any way.

### Examples

**Abbreviations**

| | |
|---|---|
| Methanol: | MeOH |
| Dichloromethane: | DCM |
| Petroleum ether: | PE |
| Ethyl acetate: | EtOAc or EA |
| Acetonitrile: | ACN or MeCN |
| Isopropanol: | IPA |
| Triethylamine: | TEA |
| Sodium hydroxide: | NaOH |
| Propylphosphonic Acid Anhydride: | T₃P |
| Nitrogen: | N₂ |
| *N,N*-Diisopropylethylamine: | DIPEA |
| *N,N-*Dimethylformamide*:* | DMF |
| 4-Methylbenzenesulfonic acid: | pTSA or TsOH |
| Diphenyl phosphorazidate: | DPPA |
| *N*-Iodosuccinimide: | NIS |
| Tetrabutylammonium fluoride: | TBAF |
| Methanesulfonyl chloride: | MsCl |
| Methyl 2,2-difluoro-2- (fluorosulfonyl)acetate: | MDFA |
| Tosylmethyl isocyanide: | TosMIC |
| Diethylaminosulphur trifluoride: | DAST |
| Thin-Layer Chromatography: | TLC |
| High Performance Liquid Chromatography: | HPLC |
| Room temperature: | r.t. |
| Hours: | h |
| Diethylamine: | DEA |

Representative methods of *prep*-HPLC: Flow rate and gradient may change.

Exemplary methods for *prep-*HPLC are provided below.

Method A: NH₄HCO₃: Column: Gilson2-Xbridge C18 19*150 mm, 5 µm; mobile phase: CH₃CN in water (0.1% NH₄HCO₃) from 20% to 60%, Flow rate: 15 mL/min.

Method B: TFA: Column: Waters-Xbridge C18 10*190 mm, 5 µm; mobile phase: CH₃CN in water (0.1% TFA) from 15% to 40%, Flow rate: 15 mL/min.

Method C: HCOOH: Column: Waters-Xbridge C18 10*190 mm, 5 µm; mobile phase: CH₃CN in water (0.1% formic acid) from 15% to 40%, Flow rate: 15 mL/min.

Method D: HCOOH:_Column: Waters SunFire ^{®} Prep C18 OBDTM (5 micron, 19*150 mm); mobile phase: CH₃CN in water (0.1% formic acid) from 18% to 38%, Flow rate: 20 mL/min.

Method E: NH₄HCO₃: Column: Waters Xbridge ^{®} Prep C18 OBD^{™} (5 micron, 19*150 mm); mobile phase: CH₃CN in water (10 mM NH₄HCO₃) from 20% to 60%, Flow rate: 20 mL/min.

### Representative method of chiral prep-HPLC:

Method F: Gilson 281, Daicel Chiralpak IE, 10 µm, 30*250 mm; Mobile phase: Hexane/EtOH/Diethylamine = 70/30/0.3, Flow rate: 25 mL/min.

Method G: Gilson 281, Daicel Chiralpak IG, 10 µm, 30*250 mm; Mobile phase: Hexane/EtOH = 70/30, Flow rate: 25 mL/min.

Method H: Gilson 281, Daicel Chiralpak IB N, 10 µm, 30*250 mm; Mobile phase: Hexane/IPA/Diethylamine = 80/20/0.3, Flow rate: 30 mL/min.

Method I: Gilson 281, Daicel Chiralpak IA, 10 µm, 30*250 mm; Mobile phase: Hexane/EtOH/Diethylamine = 30/70/0.3, Flow rate: 25 mL/min.

Method J: Gilson 281, Daicel Chiralpak IB N-5, 10 µm, 30*250 mm; Mobile phase: Hexane\IPA\Diethylamine = 80\20\0.3; Flow rate: 25 mL/min.

Method K: Gilson 281, Daicel Chiralpak IE, 10 µm 50*250 mm; Mobile phase: Hexane/EtOH=60/40; Flow rate: 60 mL/min.

Method L: Gilson 281, Daicel Chiralpak IC, 10 µm 30*250 mm; Mobile phase: Hex/IPA/DEA = 60/40/0.3; Flow rate: 25 mL/min.

Method M: Gilson 281, Daicel Chiralpak IH, 10 µm 30*250 mm; Mobile phase: Hex/EtOH = 90/10; Flow rate: 25 mL/min

Method N: Gilson 281, Daicel Chiralpak IK, 10 µm 50*250 mm; Mobile phase: Hex/IPA = 70/30; Flow rate: 60 mL/min.

Method O: Gilson 281, Daicel Chiralpak ID, 10 µm 30*250 mm; Mobile phase: Hex/IPA/DEA = 80/20/0.2; Flow rate: 25 mL/min.

### Representative methods of analytical-HPLC

Method 1: Analysis was performed on an Agilent 1200_series HPLC-6120MS. UHPLC Long Gradient Equivalent 5% to 95% acetonitrile in water (containing 0.02% NH₄OAc) run time of 6.5 minutes with a flow rate of 1.5 mL/min. A Waters Xbridge C18 column (18.5 micron, 4.6*50 mm) was used at a temperature of 40 °C.

Method 2: Analysis was performed on an Agilent 1200series HPLC-6120MS. UHPLC Long Gradient Equivalent 5% to 95% acetonitrile in water (containing 0.1% trifluoroacetic acid) run time of 6.5 minutes with a flow rate of 1.5 mL/min. A Waters Xbridge C18 column (18.5 micron, 4.6*50 mm) was used at a temperature of 40 °C.

Method 3: Analysis was performed on an Agilent 1260_series HPLC-6120MS. UHPLC Long Gradient Equivalent 5% to 95% acetonitrile in water (containing 0.02% NH₄OAc) run time of 2.5 minutes with a flow rate of 0.5 mL/min. A Diamonsil Plus C18 column (18.5 micron, 4.6*30 mm) was used at a temperature of 40 °C.

Method 4: Analysis was performed on an Agilent 1260_series HPLC-6125C MS or Agilent 1290 Infinity II HPLC-6125C MS. HPLC Long Gradient Equivalent 20% to 100% acetonitrile in water (containing 0.1% FA) run time of 1.3 to 4.5 minutes with a flow rate of 0.7 mL/min or 1 mL/min. Agilent ZORBAX SB-C18 column (1.8 micron, 2.1*50 mm) or Agilent Poroshell120 SB-C18 column (1.9 micron, 2.1*50 mm) were used at a temperature of 40 °C.

Method 5: Analysis was performed on a SHIMADZU 20A HPLC. HPLC Long Gradient Equivalent Hexane/EtOH/DEA (70/30/0.2) run time of 20 minutes with a flow rate of 1 mL/min. CHIRALPAK IE (5 µm, 4.6*250 mm) was used at a temperature of 30 °C.

Method 6: Analysis was performed on a SHIMADZU 20A HPLC. HPLC Long Gradient Equivalent Hexane/EtOH/DEA (30/70/0.2) run time of 30 minutes with a flow rate of 1 mL/min. CHIRALPAK IA (5 µm, 4.6*250 mm) was used at a temperature of 30 °C.

Method 7: Analysis was performed on a SHIMADZU 20A HPLC. HPLC Long Gradient Equivalent Hexane/IPA/DEA (80/20/0.2) run time of 30 minutes with a flow rate of 1 mL/min. CHIRALPAK IB N-5 (5 µm, 4.6*250 mm) was used at a temperature of 30 °C.

Method 8: Analysis was performed on a SHIMADZU 20A HPLC. HPLC Long Gradient Equivalent Hexane/EtOH (70/30) run time of 30 minutes with a flow rate of 1 mL/min. CHIRALPAK IG (5 µm, 4.6*250 mm) was used at a temperature of 30 °C.

Method 9: Analysis was performed on a Shimadzu LC-20A HPLC. HPLC Long Gradient Equivalent Hex\EtOH = 70\30 run time of 7 minutes with a flow rate of 1 mL/min. CHIRALPAK IE (5 µm, 4.6*150 mm) was used at a temperature of 35 °C.

Method 10: Analysis was performed on a Shimadzu LC-20A HPLC. HPLC Long Gradient Equivalent Hex\EtOH = 60\40 run time of 15 minutes with a flow rate of 1 mL/min. CHIRALPAK IG (5 µm, 4.6*150 mm) was used at a temperature of 30 °C.

Method 11: Analysis was performed on a SHIMADZU 20A HPLC. HPLC Long Gradient Equivalent Hexane/EtOH (90/10) run time of 30 minutes with a flow rate of 1 mL/min. CHIRALPAK IH (5 µm, 4.6*250 mm) was used at a temperature of 30 °C.

Method 12: Analysis was performed on a SHIMADZU 20A HPLC. HPLC Long Gradient Equivalent Hex/IPA/DEA (50/50/0.2) run time of 25 minutes with a flow rate of 1 mL/min. CHIRALPAK ID (5 µm, 4.6*250 mm) was used at a temperature of 30 °C.

Method 13: Analysis was performed on a SHIMADZU 20AT HPLC. HPLC Long Gradient Equivalent Hexane/IPA (70/30) run time of 30 minutes with a flow rate of 1 mL/min. CHIRALPAK IK (5 µm, 4.6*250 mm) was used at a temperature of 35 °C.

Method 14: Analysis was performed on a SHIMADZU 20A HPLC. HPLC Long Gradient Equivalent Hex/IPA (98/2) run time of 25 minutes with a flow rate of 1 mL/min. CHIRALPAK IB N (5 µm, 4.6*250 mm) was used at a temperature of 30 °C.

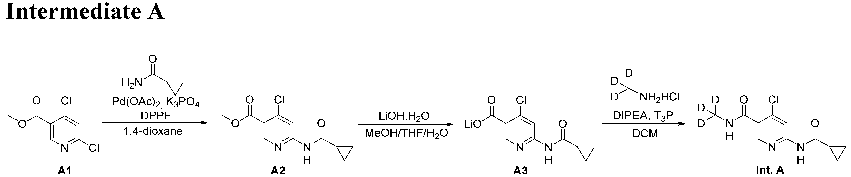

### Step 1. Methyl 4-chloro-6-(cyclopropanecarboxamido) nicotinate (A2)

A mixture of **A1** (2.0 g, 9.71 mmol), cyclopropanecarboxamide (826 mg, 9.71 mmol), Pd(OAc)₂ (109 mg, 0.49 mmol), dppf (538 mg, 0.97 mmol), K₃PO₄ (4.12 g, 19.42 mmol) in dioxane (30 mL) was stirred at 90 °C for 4 h under N₂. The mixture was diluted with H₂O (100 mL), extracted with EtOAc (30 mL*3), washed with brine (30 mL), dried over Na₂SO₄, concentrated and purified by flash chromagraphy (PE/EA = 10/1 to 1/1) to get compound **A2** (1.8 g, 73% yield) as a white solid. LC-MS (ESI, Method 4) t_{R} = 3.33 min, m/z (M+H)⁺ = 255.0.

### Step 2. Lithium 4-chloro-6-(cyclopropanecarboxamido)nicotinate (A3)

To a solution of **A2** (500 mg, 1.96 mmol) in a co-solvent of MeOH (2 mL), THF (2 mL) and water (1 mL) was added LiOH.H₂O (165 mg, 3.93 mmol). Then the mixture was stirred at r.t. overnight. The mixture was concentrated to dryness to give compound **A3** (480 mg, 99% yield) as a white solid. LC-MS (ESI, Method 4) t_{R} = 3.81 min, m/z (M+H)⁺ = 241.1.

### Step 3. 4-Chloro-6-(cyclopropanecarboxamido)-N-(methyl-d₃)nicotinamide (Int. A)

To a solution of **A3** (480 mg, 1.95 mmol) in DCM (15 mL) was sequentially added methan-*d₃*-amine hydrochloride (275 mg, 3.89 mmol), DIPEA (1.51 g, 11.68 mmol) and T₃P (1.86 g, 2.92 mmol, 50% in EtOAc) at 0 °C. The resulting mixture was stirred at r.t. overnight. The mixture was diluted with H₂O (30 mL) and extracted with DCM (30 mL*3). The organic layer was washed with brine (50 mL), dried over Na₂SO₄ and filtered. The filtrate was concentrated to dryness to give **Int. A** (300 mg, 60% yield) as a white solid. LC-MS (ESI, Method 4) t_{R} = 2.25 min, m/z (M+H)⁺= 257.1.

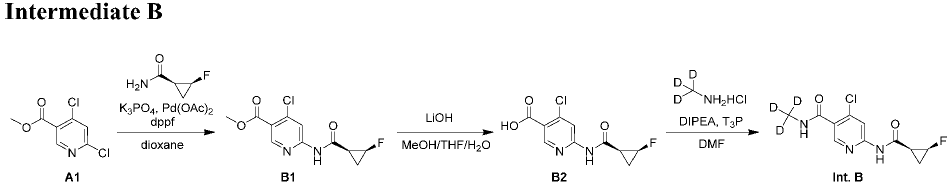

### Step 1. Methyl 4-chloro-6-((1S,2S)-2-fluorocyclopropane-1-carboxamido)nicotinate (B1)

A mixture of **A1** (1.0 g, 4.85 mmol), (1*S*,2*S*)-2-fluorocyclopropane-1-carboxamide (751 mg, 7.28 mmol), K₃PO₄ (2.04 g, 9.67 mmol), dppf (269 mg, 0.49 mmol) and Pd(OAc)₂ (109 mg, 0.49 mmol) in dioxane (8 mL) was stirred at 90 °C for 12 h under N₂. After cooling to r.t., the mixture was filtered and the filtrate was concentrated. The residue was purified by flash chromatography on silica gel (DCM/MeOH = 30/1) to afford **B1** (220 mg, 17% yield) as a yellow solid. LC-MS (ESI, Method 3) t_{R} = 1.16 min, m/z (M+H)⁺ = 273.6.

### Step 2. 4-Chloro-6-((1S,2S)-2-fluorocyclopropane-1-carboxamido)nicotinic acid (B2)

To a solution of **B1** (900 mg, 3.30 mmol) in MeOH/THF/H₂O (15 mL, v/v/v = 2/2/1) was added LiOH.2H₂O (990 mg, 16.50 mmol) and stirred at 25 °C for 12 h. The reaction mixture was concentrated to dryness and acidified with 1 *N* HCl to pH = 2. The formed solid was filtered and the filter cake was dried to afford **B2** (700 mg, 82% yield) as a yellow solid. LC-MS (ESI, Method 3) t_{R} = 1.00 min, m/z (M+H)⁺ = 258.9.

### Step 3. 4-Chloro-6-((1S,2S)-2-fluorocyclopropane-1-carboxamido)-N-(methyl-d₃)nicotinamide (Int. B)

A mixture of **B2** (700 mg, 2.71 mmol), DIPEA (2.10 g, 16.24 mmol, 2.83 mL), T₃P (1.72 g, 5.41 mmol, 3.45 mL, 50% wt. in DMF) and methan-*d₃*-amine hydrochloride (379 mg, 5.41 mmol) in DMF (4 mL) was stirred at 30 °C for 48 h. The mixture was diluted with H₂O (10 mL), extracted with DCM (10*3 mL). The organic layer was washed with brine (2 mL), dried over Na₂SO₄, filtered and concentrated. The residue was purified by flash chromatography on silical gel (DCM/MeOH = 20/1) to afford **Int. B** (500 mg, 67% yield) as a white solid. ¹H NMR (400 MHz, DMSO-*d*₆) δ 11.24 (s, 1H), 8.43 (s, 1H), 8.29 (s, 1H), 8.19 (s, 1H), 5.04-4.86 (m, 1H), 2.49-2.40 (m, 1H), 1.69-1.62 (m, 1H), 1.24-1.18 (m, 1H). LC-MS (ESI, Method 3) t_{R} = 0.91 min, m/z (M+H)⁺ = 275.0.

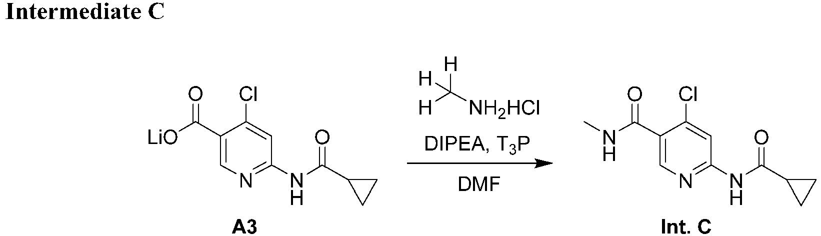

### Step 1. 4-Chloro-6-(cyclopropanecarboxamido)-N-methylnicotinamide (Int. C)

A mixture of **A3** (338 mg, 1.37 mmol), DIPEA (1.06 g, 8.23 mmol), methylamine hydrochloride (184 mg, 2.75 mmol) and T₃P (1.75 g, 2.74 mmol, 50% wt. in DMF) in DMF (2 mL) was stirred at 50 °C for 24 h. The reaction mixture was poured into water (5 mL) and extracted with EtOAc (20 mL*3). The separated organic layer was washed with water (5 mL) and concentrated under reduced pressure. The residue was purified by flash chromatography on silica gel (DCM/MeOH = 10/1) to afford **Int. C** (120 mg, 34% yield) as a white solid. ¹H NMR (400 MHz, DMSO-*d*₆) δ 11.19 (s, 1H), 8.46 (s, 1H), 8.37 (s, 1H), 8.19 (s, 1H), 2.75 (d, *J =* 5.2 Hz, 3H), 2.02-1.99 (m, 1H), 0.85-0.82 (m, 4H). LC-MS (ESI, Method 3) t_{R} = 0.94 min, m/z (M+H)⁺ = 254.2.

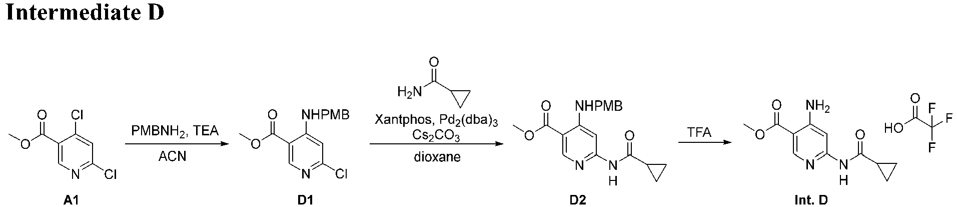

### Step 1. Methyl 6-chloro-4-((4-methoxybenzyl)amino)nicotinate (D1)

To a solution of **A1** (5 g, 24.27 mmol) in ACN (8 mL) was added (4-methoxyphenyl)methanamine (3.33 g, 24.27 mmol, 3.2 mL) and TEA (4.91 g, 48.54 mmol, 6.8 mL). Then the mixture was stirred at r.t. for 24 h. The mixture was diluted with H₂O (100 mL), extracted with EA (50 mL*3), washed with brine, dried over Na₂SO₄, concentrated and purified by flash chromtography (PE/EA = 20/1 to 5/1) to get compound **D1** (6.5 g, 87% yield) as an off-white solid. LC-MS (ESI, Method 4) t_{R} = 4.18 min, m/z (M+H)⁺= 307.1.

### Step 2. Methyl 6-(cyclopropanecarboxamido)-4-((4-methoxybenzyl)amino) nicotinate (D2)

A mixture of **D1** (2 g, 6.52 mmol), cyclopropanecarboxamide (1.11 g, 13.04 mmol), XantPhos (754 mg, 1.30 mmol), Pd₂(dba)₃ (597 mg, 0.65 mmol), Cs₂CO₃ (5.31 g, 16.30 mmol) in 1,4-dioxane (30 mL) was stirred at 110 °C for 2 h. Then the mixture was diluted with H₂O (100 mL), extracted with EA (60 mL*3), washed with brine, dried over Na₂SO₄ and concentrated to get compound **D2** (2.3 g, 99% yield) as a yellow solid. LC-MS (ESI, Method4) t_{R} = 2.91 min, m/z (M+H)⁺ = 356.2.

### Step 3. Methyl 4-amino-6-(cyclopropanecarboxamido)nicotinate 2,2,2-trifluoroacetate (Int. D)

A solution of **D2** (2.1 g, 5.91 mmol) in TFA (10 mL) was stirred at 80 °C for 16 h. Then the mixture was concentrated and diluted with EA (10 mL), filtered and wash with EA (5 mL*2). Then the solid was dried to get compound **Int. D** (1.8 g, 87% yield, TFA salt) as an off-white solid. LC-MS (ESI, Method 4) t_{R} = 1.28 min, m/z (M+H)⁺= 236.2.

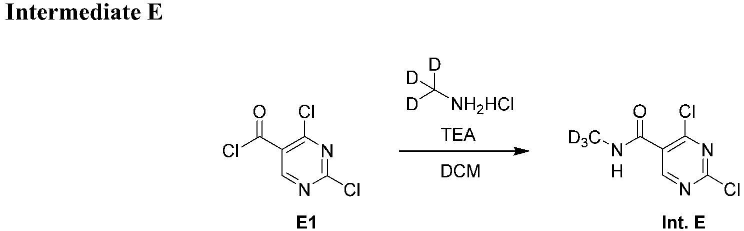

### Step 1. 2,4-Dichloro-N-(methyl-d₃)pyrimidine-5-carboxamide (Int. E)

To a mixture of methan-*d*₃-amine hydrochloride (1.40 g, 19.86 mmol) in DCM (200 mL) was added **E1** (3.5 g, 16.55 mmol) slowly followed by TEA (1.68 g, 16.55 mmol, 2.31 mL) at -78 °C. After stirring for 1 h at this temperature, the reaction was quenched with water (30 mL). The organic layer was separated and concentrated. The residue was purified by flash chromatography on silica gel (PE/EA = 5/1) to afford **Int. E** (1.38 g, 35% yield) as a white solid. ¹H NMR (400 MHz, DMSO-*d*₆) δ 8.88 (s, 1H), 8.85 (s, 1H). LC-MS (ESI, Method 3) t_{R} = 0.80 min, m/z (M+H)⁺ = 208.9.

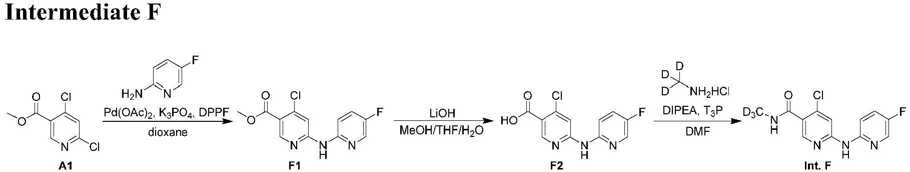

### Step 1. Methyl 4-chloro-6-((5-fluoropyridin-2-yl)amino)nicotinate (F1)

A mixture of **A1** (2.0 g, 9.71 mmol), 5-fluoropyridin-2-amine (1.31 g, 11.65 mmol), K₃PO₄ (4.12 g, 19.42 mmol), DPPF (807 mg, 1.46 mmol) and Pd(OAc)₂ (327 mg, 1.46 mmol) in anhydrous dioxane (20 mL) was stirred at 90 °C for 12 h. After cooling to r.t., the mixture was filtered and the filtrate was concentrated. The residue was purified by flash chromatography on silica gel (PE/EA = 7/1) to afford **F1** (2.0 g, 73% yield) as a yellow solid. LC-MS (ESI, Method 3) t_{R} = 1.11 min, m/z (M+H)⁺= 282.4.

### Step 2. 4-Chloro-6-((5-fluoropyridin-2-yl)amino)nicotinic acid (F2)

To a solution of **F1** (2.0 g, 7.10 mmol) in MeOH/THF/H₂O (20 mL, v/v/v = 2/2/1) was added LiOH.H₂O (1.49 g, 35.50 mmol). After stirring at r.t. for 12 h, the reaction mixture was concentrated to dryness and acidified with 1 N HCl to pH = 2. The formed solid was collected by filtering and the filter cake was dried to give compound **F2** (1.8 g, 95% yield) as a yellow solid. LC-MS (ESI, Method 3) t_{R} = 0.88 min, m/z (M+H)⁺ = 268.2.

### Step 3. 4-Chloro-6-((5-fluoropyridin-2-yl)amino)-N-(methyl-d₃)nicotinamide (Int. F)

A mixture of **F2** (400 mg, 1.50 mmol), methan-*d₃*-amine hydrochloride (529 mg, 7.50 mmol) and DIPEA (1.16 g, 9.00 mmol) in T₃P (2 mL, 50% wt in DMF) was stirred at 50 °C for 16 h. After cooling to r.t., the reaction mixture was poured into water (10 mL) and the formed solid was collected by filtering. The filter cake was slurried with MeOH (5 mL) for 30 min. The solid was filtered and dried to afford **Int. F** (380 mg, 90% yield) as a yellow solid. LC-MS (ESI, Method 3) t_{R} = 1.10 min, m/z (M+H)⁺= 284.1.

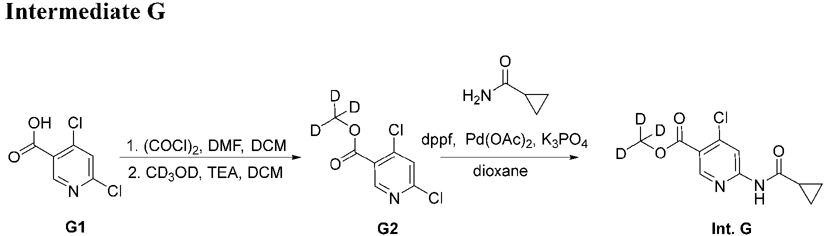

### Step 1. Methyl-d₃ 4,6-dichloronicotinate (G2)

To a solution of **G1** (2.00 g, 11 mmol) in DCM (20 mL) was added (COCl)₂ (3.96 g, 31 mmol) and DMF (0.08 g, 1 mmol, 0.08 mL) at 0 °C. Then the mixture was stirred at 25 °C for 2 h, and it was concentrated to give a residue. To the DCM (20 mL) solution of residue was added CD₃OD (0.38 g, 0.011 mol, 0.43 mL) and TEA (2.10 g, 0.021 mol, 2.9 mL) at 0 °C. The mixture was stirred at 25 °C for 1 h. The reaction mixture was diluted with water (20 mL), then extracted with DCM (30 mL*3). The combined organic layer was washed with brine (10 mL*2), dried over anhydrous Na₂SO₄, filtered and concentrated. The residue was purified by silica gel column (EtOAc in PE is 2-20%) to give **G2** (1.5 g, 68% yield) as a white solid. LC-MS (ESI, Method 4) t_{R} = 0.25 min, m/z [M+H]⁺ = 208.9.

### Step 2. Methyl-d₃ 4-chloro-6-(cyclopropanecarboxamido) nicotinate (Int. G)

To a solution of **G2** (1.5 g, 7 mmol) in dioxane (20 mL) was added cyclopropanecarboxamide (0.61 g, 7 mmol), Pd(OAc)₂ (0.16 g, 0.7 mmol), dppf (1.20 g, 1.4 mmol) and K₃PO₄ (3.06 g, 14 mmol) under N₂ atmosphere. The mixture was stirred at 75 °C for 16 h. The reaction mixture was diluted with water (30 mL), then extracted with EA (30 mL*3). The combined organic layer was washed with brine (10 mL*2), dried over anhydrous Na₂SO₄, filtered and concentrated. The residue purified by Prep-TLC (PE/EA = 5/1) to give **Int. G** (0.80 g, 44% yield) as a white solid. LC-MS (ESI, Method 3) t_{R} = 1.92 min, m/z [M+H]⁺ = 257.7.

### Example 1

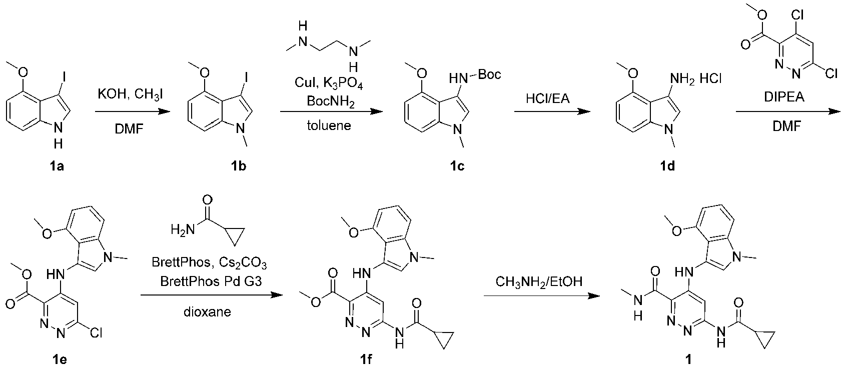

### Step 1. 3-Iodo-4-methoxy-1-methyl-1H-indole (1b)

To a solution of **1a** (2.78 g, 10.18 mmol) in DMF (15 mL) was added KOH (2.28 g, 40.72 mmol) and CH₃I (4.34 g, 30.54 mmol) at 0 °C. The mixture was stirred at r.t. for 1 h. The reaction mixture was poured into ice-water (50 mL) and the formed solid was filtered. The solid was dried to give compound **1b** (2.8 g, 96% yield) as a brown solid. ¹H NMR (400 MHz, DMSO-*d*₆) δ 7.37 (s, 1H), 7.11-7.05 (m, 2H), 6.55 (dd, *J* = 7.6, 1.2 Hz, 1H), 3.84 (s, 3H), 3.74 (s, 3H).

### Step 2. Tert-butyl (4-methoxy-1-methyl-1H-indol-3-yl)carbamate (1c)

A mixture of **1b** (500 mg, 1.74 mmol), K₃PO₄ (738 mg, 3.48 mmol), BocNH₂ (612 mg, 5.22 mmol), CuI (100 mg, 0.52 mmol) and N,N-dimethylethane-1,2-diamine (46 mg, 0.52 mmol) in toluene (5 mL) was stirred at 110 °C overnight. The mixture was concentrated and the residue was purified by flash chromatography on silica gel (PE/EA = 4/1) to give compound **1c** (240 mg, 50% yield) as a white solid. ¹H NMR (300 MHz, DMSO-*d*₆) δ 7.76 (s, 1H), 7.34 (s, 1H), 7.10-6.97 (m, 2H), 6.52 (d, *J* = 10.4 Hz, 1H), 3.90 (s, 3H), 3.71 (s, 3H), 1.50 (s, 9H).

### Step 3. 4-Methoxy-1-methyl-1H-indol-3-amine hydrochloride (1d)

To a solution of **1c** (300 mg, 1.09 mmol) in EtOAc (2 mL) was added HCl/EA (2 mL, 6 M) at r.t. The mixture was stirred at r.t. overnight. The formed solid was filtered and the filter cake was dried to give compound **1d** (158 mg, 68% yield) as a white solid. ¹H NMR (400 MHz, DMSO-*d*₆) δ 9.99 (brs, 3H), 7.37 (s, 1H), 7.17 (t*, J =* 8.0 Hz, 1H), 7.09 (d, *J =* 8.4 Hz, 1H), 6.64 (d, *J =* 8.0 Hz, 1H), 3.94 (s, 3H), 3.76 (s, 3H).

### Step 4. Methyl 6-chloro-4-((4-methoxy-1-methyl-1H-indol-3-yl)amino)pyridazine-3-carboxylate (1e)

A mixture of **1d** (90 mg, 0.42 mmol), methyl 4,6-dichloropyridazine-3-carboxylate (88 mg, 0.42 mmol) and DIPEA (273 mg, 2.12 mmol) in DMF (1 mL) was stirred at 80 °C for 10 h. The reaction was concentrated and the residue was purified by flash chromatography on silica gel (PE/EA = 4/1) to give compounc **1e** (80 mg, 55% yield) as a yellow solid. LC-MS (ESI, Method 3) t_{R} = 1.45 min, m/z (M+H)⁺= 347.1.

### Step 5. Methyl 6-(cyclopropanecarboxamido)-4-((4-methoxy-1-methyl-1H-indol-3-yl)amino)pyridazine-3-carboxylate (1f)

A mixture of **1e** (98 mg, 0.28 mmol), cyclopropanecarboxamide (48 mg, 0.57 mmol), Cs₂CO₃ (276 mg, 0.85 mmol), BrettPhos (26 mg, 0.028 mmol) and BrettPhos Pd G3 (15 mg, 28 mmol) in anhydrous 1,4-dioxane (1 mL) was stirred at 80 °C for 6 h. The mixture was concentrated and the residue was purified by flash chromatography on silica gel (DCM/MeOH = 20/1) to give compound **1f** (65 mg, 58% yield) as a yellow solid. LC-MS (ESI, Method 3) t_{R} = 0.49 min, m/z (M+H)⁺= 396.2.

### Step 6. 6-(Cyclopropanecarboxamido)-4-((4-methoxy-1-methyl-1H-indol-3-yl)amino)-N-methylpyridazine-3-carboxamide (1)

A mixture of **1f** (50 mg, 0.13 mmol) in methylamine (1.5 mL, 30% alcohol solution) was stirred at 80 °C for 48 h. The mixture was concentrated. The residue was purified by *prep-*HPLC (Method C) to give compound **1** (4 mg, 8% yield) as a yellow solid. ¹H NMR (400 MHz, DMSO-*d*₆) δ 11.18 (s, 1H), 11.02 (s, 1H), 9.02-8.99 (m, 1H), 7.98 (s, 1H), 7.23 (s, 1H), 7.12 (t, *J* = 8.0 Hz, 1H), 7.04 (d, *J* = 8.0 Hz, 1H), 6.53 (d, *J=* 7.6 Hz, 1H), 3.81 (s, 3H), 3.76 (s, 3H), 2.86 (d, *J =* 4.8 Hz, 3H), 2.10-2.06 (m, 1H), 0.83-0.81 (m, 4H). LC-MS (ESI, Method 2) t_{R} = 2.43 min, m/z (M+H)⁺ = 395.2.

### Example 2

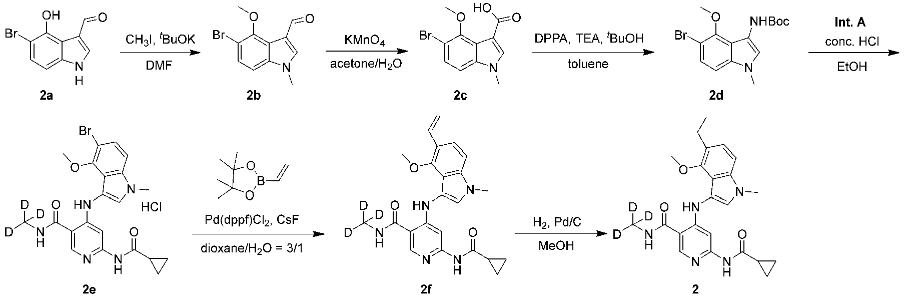

### Step 1. 5-Bromo-4-methoxy-1-methyl-1H-indole-3-carbaldehyde (2b)

A mixture of **2a** (1.5 g, 6.3 mmol) and *^{t}*BuOK (1.7 g, 15.6 mmol) in DMF (15 mL) was stirred for 10 min at 0 °C. Then iodomethane (3.5 g, 24.9 mmol) was added to the reaction mixture. After stirring at r.t. for 18 h, the mixture was diluted with water (30 mL) and extracted with EtOAc (30 mL*3). The combined organic phase was washed with brine (40 mL), concentrated and the residue was purified by flash chromatography on silica gel (PE/DCM = 1/1) to afford **2b** (1.6 g, 96% yield) as a yellow solid. ¹H NMR (400 MHz, DMSO-*d*₆) δ 10.12 (s, 1H), 8.25 (s, 1H), 7.48 (d, *J=* 8.8 Hz, 1H), 7.36 (d, *J=* 8.8 Hz, 1H), 3.88 (s, 3H), 3.85 (s, 3H).

### Step 2. 5-Bromo-4-methoxy-1-methyl-1H-indole-3-carboxylic acid (2c)

A mixture of **2b** (1.2 g, 4.5 mmol) and KMnO₄ (1.4 g, 8.9 mmol) in acetone/H₂O (80 mL, v/v = 1/1) was stirred at r.t. for 10 h. The mixture was filtered and the organic solvent was removed under reduced pressure. The aqueous layer was basified with 1 M NaOH to pH >10 and washed with EtOAc (20 mL). The separated aqueous layer was acidified with 2 M HCl to pH = 5 and extracted with DCM/MeOH (40 mL*3, v/v = 10/1). The combined organic phase was washed with brine (40 mL), concentrated to afford the crude **2c** (0.60 g, 50% yield) as a yellow solid. LC-MS (ESI, Method 3) t_{R} = 1.11 min, m/z (⁷⁹Br, M+H)⁺ = 284.0.

### Step 3. Tert-butyl (5-bromo-4-methoxy-1-methyl-1H-indol-3-yl) carbamate (2d)

A mixture of **2c** (417 mg, 1.5 mmol), DPPA (444 mg, 1.6 mmol), Et₃N (444 mg, 4.40 mmol) in toluene (4 mL) was stirred at 110 °C for 1 h. Then *^{t}*BuOH (217 mg, 2.9 mmol) was added into the mixture. After stirring at 110 °C for 12 h, the reaction mixture was cooled, concentrated and the residue was purified by flash chromatography on silica gel (PE/EA = 20/1) to afford **2d** (60 mg, 12% yield) as a yellow solid. LC-MS (ESI, Method 3) t_{R} = 1.47 min, m/z (⁷⁹Bᵣ, M+H)⁺ = 355.2.

### Step 4. 4-((5-Bromo-4-methoxy-1-methyl-1H-indol-3-yl) amino)-6-(cyclopropanecarboxamido)-N-(methyl-d₃) nicotinamide hydrochloride (2e)

A mixture of **2d** (55 mg, 0.15 mmol), **Int. A** (40 mg, 0.14 mmol), cat. *conc.* HCl in EtOH (1 mL) was stirred at 90 °C for 18 h. The reaction mixture was cooled, concentrated and purified by *prep-TLC* (DCM/MeOH = 30/1) to afford **2e** (46 mg, 60% yield) as a yellow solid. LC-MS (ESI, Method 3) t_{R} = 1.30 min, m/z (⁷⁹Br, M+H)⁺ = 475.3.

### Step 5. 6-(Cyclopropanecarboxamido)-4-((4-methoxy-1-methyl-5-vinyl-1H-indol-3-yl)amino)-N-(methyl-d₃)nicotinamide (2f)

A mixture of **2e** (56 mg, 0.11 mmol), 4,4,5,5-tetramethyl-2-vinyl-1,3,2-dioxaborolane (19 mg, 0.13 mmol), CsF (48 mg, 0.315 mmol) and Pd(dppf)Cl₂ (8 mg, 0.01 mmol) in dioxane/H₂O (1.2 mL, v/v=3/1) was stirred at 150 °C for 5 h under microwave under N₂. After cooling to r.t., the mixture was filtered and the residue was concentrated to afford the crude **2f** (40 mg, 90% yield) as a black solid. LC-MS (ESI, Method 3) t_{R} = 1.26 min, m/z (M+H)⁺ = 423.4.

### Step 6. 6-(Cyclopropanecarboxamido)-4-((5-ethyl-4-methoxy-1-methyl-1H-indol-3-yl)amino)-N-(methyl-d₃)nicotinamide (2)

A mixture of **2f** (40 mg, 0.095 mmol) and Pd/C (4 mg, 0.036 mmol, wetted with ca. 50% water) in MeOH was stirred at r.t under H₂ (0.1 MPa) for 5 h. The mixture was filtered and purified by *prep-HPLC* (Method A) to afford **2** (11.4 mg, 28% yield) as a yellow solid. ¹H NMR (400 MHz, DMSO-*d*₆) δ 10.57 (s, 1H), 10.33 (s, 1H), 8.44-8.46 (m, 2H), 7.74 (s, 1H), 7.27 (s, 1H), 7.16 (d, *J =* 8.4 Hz, 1H), 7.02 (d, *J* = 8.4 Hz, 1H), 3.74 (s, 3H), 3.61 (s, 3H), 2.63 (q, *J =* 7.6 Hz, 2H), 1.97-1.91 (m, 1H), 1.16 (t, *J =* 7.6 Hz, 3H), 0.73-0.69 (m, 4H). LC-MS (ESI, Method 2) t_{R} = 2.58 min, m/z (M+H)⁺= 425.2.

### Example 3

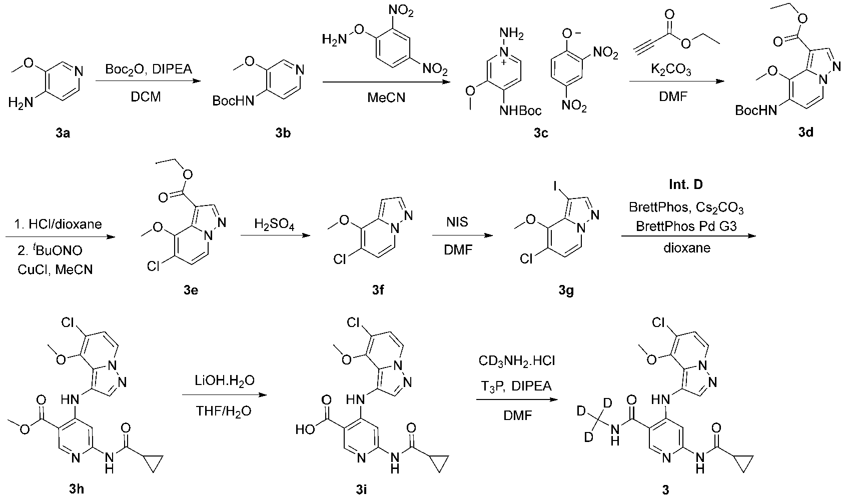

### Step 1. Tert-butyl (3-methoxypyridin-4-yl)carbamate (3b)

A solution **of 3a** (800 mg, 6.44 mmol), Boc₂O (1.83 g, 8.38 mmol, 1.9 mL) and DIPEA (1.67 g, 12.89 mmol, 2.24 mL) in DCM (15 mL) was stirred at 20 °C for 12 h. A yellow solution was formed. The reaction mixture was concentrated and purified by flash chromatography (EA in PE is 10-50%) to give compound 3b (1.45 g, yield given) as a white solid. LC-MS (ESI, Method 4) t_{R} = 1.92 min, m/z (M+H)⁺ = 225.1.

### Step 2. 1-Amino-4-((tert-butoxycarbonyl)amino)-3-methoxypyridin-1-ium 2,4-dinitrophenolate (3c)

A mixture of **3b** (1.45 g, 6.47 mmol) and O-(2,4-dinitrophenyl) hydroxylamine (1.42 g, 7.11 mmol) in MeCN (50 mL) was stirred at 50 °C for 16 h. A yellow solution was formed. The reaction was concentrated to give **3c** (2.73 g, crude) as a yellow solid, which was used for the next step directly without further purification. LC-MS (ESI, Method 4) t_{R} = 1.66 min, m/z M⁺ = 240.1.

### Step 3. Ethyl 5-((tert-butoxycarbonyl)amino)-4-methoxypyrazolo[1,5-a]pyridine-3-carboxylate (3d)

A mixture of ethyl propiolate (685 mg, 6.98 mmol, 0.71 mL), **3c** (1.29 g, 5.37 mmol) and K₂CO₃ (1.48 g, 10.74 mmol) in DMF (15 mL) was stirred at 20 °C for 2 h. A black suspension was formed. The reaction mixture was concentrated in vacuo and diluted with water (50 mL), then extracted with EtOAc (50 mL*2). The combined organic layer was washed with water (50 mL*2), brine (50 mL*2), dried over anhydrous Na₂SO₄, filtered and concentrated. The residue was purified by flash chromatography (EA in PE is 10-30%) to give **3d** (428 mg, 22% yield) as a yellow solid. LC-MS (ESI, Method 4) t_{R} = 4.16 min, m/z (M+H)⁺ = 336.1.

### Step 4. Ethyl 5-chloro-4-methoxy-pyrazolo[1,5-a]pyridine-3-carboxylate (3e)

To a mixture of **3d** (330 mg, 0.98 mmol) in DCM (2 mL) was added HCl/dioxane (4 M, 2 mL) at 20 °C. The resulting mixture was stirred at 20 °C for 1 h. A yellow solution was formed. The rection mixture was concentrated to give ethyl 5-amino-4-methoxy-pyrazolo[1,5-*a*]pyridine-3-carboxylate (231 mg, HCl salt, crude) as a yellow solid. LC-MS (ESI, Method 4) t_{R} = 0.48 min, m/z (M+H)⁺ = 236.1.

To a mixture of ethyl 5-amino-4-methoxy-pyrazolo[1,5-*a*]pyridine-3-carboxylate (231 mg, HCl salt, crude) in MeCN (5 mL), was added *tert-*butyl nitrite (152 mg, 1.47 mmol, 0.18 mL) at 0 °C and stirred for 10 min. Then CuCl (145.8 mg, 1.47 mmol) was added into the above mixture. The resulting mixture was stirred at 80 °C for 12 h. A yellow solution was formed. The reaction mixture was concentrated and purified by flash chromatograph (EA in PE is 10-30%) to give **3e** (100 mg, 40% yield) as a yellow solid. LC-MS (ESI, Method 4) t_{R} = 2.44 min, m/z (M+H)⁺ = 255.2.

### Step 5. 5-Chloro-4-methoxy-pyrazolo[1,5-a]pyridine (3f)

Heat a well-stirred solution of **3e** (100 mg, 0.39 mmol) in 2 mL of 50% H₂SO₄ at 110°C for 3 h. A yellow solution was formed. Cool down the solution to room temperature. Neutralize the solution with aq. NaOH (1.0 M). Add 40 mL of water to the above solution. Extract the solution by EtOAc (30 mL*3). Combine the organic layer. Dry the organic layer over anhydrous Na₂SO₄. Filter the organic layer through a pad of Celite. Evaporate the organic layer in vacuum to give **3f** (33 mg, 46% yield) as a white solid. ¹H NMR (400 MHz, CDCl₃) *δ* 8.20 (dd, *J* = 7.6 Hz, 1.2 Hz, 1H), 7.92 (d, *J* = 2.4 Hz, 1H), 6.72 (d, *J* = 7.6 Hz, 1H), 6.63 (dd, *J* = 2.4 Hz, 1.2 Hz, 1H), 4.06 (s, 3H). LC-MS (ESI, Method 4) t_{R} = 2.46 min, m/z (M+H)⁺ = 183.1.

### Step 6. 5-Chloro-3-iodo-4-methoxy-pyrazolo[1,5-a]pyridine (3g)

A mixture of **3f** (31 mg, 0.17 mmol) and NIS (38.2 mg, 0.17 mmol) in DMF (2 mL) was stirred at 20 °C for 12 h. A yellow suspension was formed. The reaction mixture was quenched with water (50 mL) and extracted with EtOAc (30 mL*3). The combined organic layer was washed with brine (40 mL*2), dried over anhydrous Na₂SO₄, filtered and concentrated. The residue was purified by flash chromatograph (EA in PE is 10-30%) to give 3g (50 mg, 95% yield) as a yellow solid. LC-MS (ESI, Method 4) t_{R} = 3.12 min, m/z (M+H)⁺ = 308.9.

### Step 7. Methyl 4-((5-chloro-4-methoxypyrazolo[1,5-a]pyridin-3-yl)amino)-6-(cyclopropanecarboxamido)nicotinate (3h)

A mixture of **3g** (50 mg, 0.16 mmol), **Int. D** (68 mg, 0.19 mmol, TFA salt), BrettPhos (17.4 mg, 0.032 mmol), Cs₂CO₃ (132 mg, 0.41 mmol) and BrettPhos Pd G3 (14.7 mg, 0.016 mmol) in dioxane (1 mL) was degassed and purged with nitrogen for 3 times. The resulting mixture was stirred at 100 °C under N₂ atmosphere for 24 h. A yellow suspension was formed. The reaction mixture was concentrated and purified by *prep-*TLC (PE/EA = 1/2) to give 3h (60 mg, 89% yield) as a yellow solid. ¹H NMR (400 MHz, DMSO-*d*₆) *δ* 10.85 (s, 1H), 9.58 (s, 1H), 8.67 (s, 1H), 8.54 (d, *J =* 7.2 Hz, 1 H), 8.15 (s, 1H), 7.64 (s, 1H), 6.98 (d, *J =* 7.2 Hz, 1H), 3.89 (s, 3H), 3.77 (s, 3H), 1.98-1.90 (m, 1H), 0.78-0.70 (m, 4H). LC-MS (ESI, Method 4) t_{R} = 2.42 min, m/z (M+H)⁺ = 416.2.

### Step 8. 4-((5-Chloro-4-methoxypyrazolo[1,5-a]pyridin-3-yl)amino)-6-(cyclopropanecarboxamido)nicotinic acid (3i)

A mixture of **3h** (60 mg, 0.144 mmol) and LiOH.H₂O (18 mg, 0.433 mmol) in co-solvent of THF (3 mL) and water (1 mL) was stirred at 40 °C for 12 h. A yellow solution was formed. The reaction mixture was concentrated and dried in vacuo to give **3i** (58 mg, crude) as a yellow solid, which was used for the next step directly without further purification. LC-MS (ESI, Method 4) t_{R} = 1.01 min, m/z (M+H)⁺ = 402.2.

### Step 9 4-((5-Chloro-4-methoxypyrazolo[1,5-a]pyridin-3-yl)amino)-6-(cyclopropanecarboxamido)-N-(methyl-d₃)nicotinamide (3)

A mixture of CD₃NH₂.HCl (30.5 mg, 0.433 mmol), **3i** (58 mg, crude), DIPEA (93.3 mg, 0.721 mmol, 0.13 mL) and T₃P (275.6 mg, 0.433 mmol, 50% purity in EtOAc) in DMF (2 mL) was stirred at 20 °C for 12 h. A yellow solution was formed. The rection mixture was filtered and purified by *Prep-*HPLC (Method E) to give **3** (3.6 mg, 6% yield) as a white solid. ¹H NMR (400 MHz, DMSO-*d*₆) *δ* 10.69 (s, 1H), 10.35 (s, 1H), 8.55 (s, 1H), 8.51-8.43 (m, 2H), 8.11 (s, 1H), 7.65 (s, 1H), 6.94 (d, *J =* 7.2 Hz, 1H), 3.79 (s, 3H), 1.98-1.90 (m, 1H), 0.76-0.71 (m, 4H). LC-MS (ESI, Method 4) t_{R} = 1.97 min, m/z (M+H)⁺ = 418.2.

### Example 4

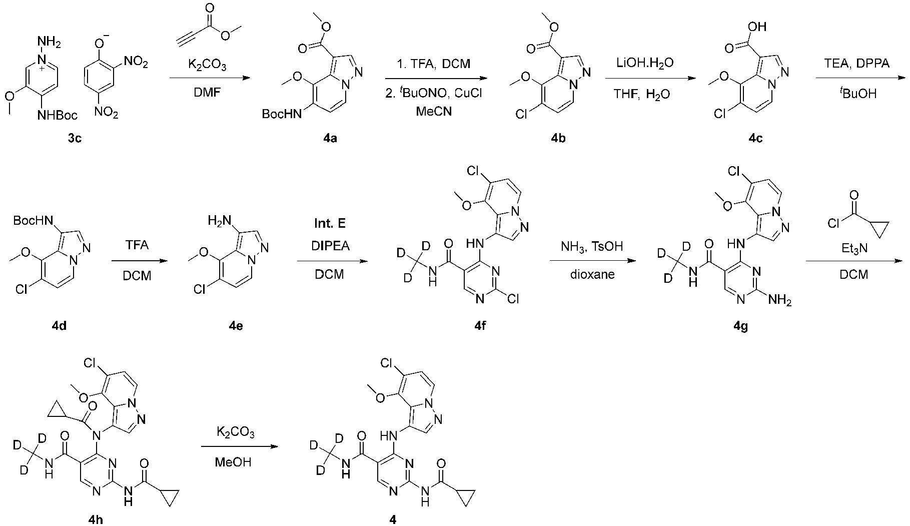

### Step 1. Methyl 5-((tert-butoxycarbonyl)amino)-4-methoxypyrazolo[1,5-a] pyridine-3-carboxylate (4a)

A mixture of methyl propiolate (749 mg, 8.91 mmol, 7.93 mL), **3c** (1.89 g, 4.45 mmol) and K₂CO₃ (1.23 g, 8.91 mmol) in DMF (5 mL) was stirred at 20 °C for 2 h. A black suspension was formed. The reaction mixture was concentrated and diluted with water (50 mL), then extracted with EtOAc (50 mL*2). The combined organic layer was washed with water (50 mL*2), brine (50 mL*2), dried over anhydrous Na₂SO₄, filtered and concentrated. The residue was purified by flash chromatography (EA in PE is 10-30%) to give **4a** (350 mg, 24% yield) as a yellow solid. ¹H NMR (400 MHz, CDCl₃) *δ* 8.35 (s, 1H), 8.28 (d, *J =* 7.6 Hz, 1H), 8.01 (d, *J =* 7.6 Hz, 1H), 7.32 (brs, 1H), 3.89 (s, 3H), 3.88 (s, 3H), 1.55 (s, 9H). LC-MS (ESI, Method 4) t_{R} = 4.13 min, m/z (M+H)⁺ = 322.1.

### Step 2. Methyl 5-chloro-4-methoxy-pyrazolo[1,5-a]pyridine-3-carboxylate (4b)

To a mixture of **4a** (1.25 g, 3.89 mmol) in DCM (5 mL), was added TFA (2 mL) at 20 °C. The resulting mixture was stirred at 20 °C for 1 h. A yellow solution was formed. The rection mixture was concentrated to give methyl 5-amino-4-methoxy-pyrazolo[1,5-*a*]pyridine-3-carboxylate (800 mg, TFA salt, crude) as a yellow solid. LC-MS (ESI, Method 4) t_{R} = 0.46 min, m/z (M+H)⁺ = 222.1.

To a mixture of methyl 5-amino-4-methoxy-pyrazolo[1,5-*a*]pyridine-3-carboxylate (800 mg, TFA salt, crude) in MeCN (10 mL), was added *tert-*butyl nitrite (559 mg, 5.42 mmol, 0.65 mL) at 0 °C, and stirred for 10 min. Then CuCl (716 mg, 7.23 mmol) was added into the above mixture, the resulting mixture was stirred at 80 °C for 2 h. A yellow solution was formed. The reaction mixture was concentrated and purified by flash chromatography (EA in PE is 10-30%) to give **4b** (600 mg, 69% yield) as a yellow solid. LC-MS (ESI, Method 4) t_{R} = 2.31 min, m/z (M+H)⁺ = 241.2.

### Step 3. 5-Chloro-4-methoxypyrazolo[1,5-a]pyridine-3-carboxylic acid (4c)

A mixture of **4b** (300 mg, 1.25 mmol) and LiOH.H₂O (157 mg, 3.74 mmol) in co-solvent of THF (6 mL) and water (2 mL) was stirred at 70 °C for 12 h. A yellow solution was formed. The reaction mixture was diluted with water (30 mL) and extracted with EtOAc (20 mL). The aqueous layer was adjusted to pH = 3 with aq. HCl (2 M) and extracted with EtOAc (20 mL*3). The organic layer was concentrated and dried in vacuo to give **4c** (180 mg, 64% yield) as a white solid. LC-MS (ESI, Method 4) t_{R} = 1.79 min, m/z (M+H)⁺ = 227.0.

### Step 4. Tert-butyl (5-chloro-4-methoxypyrazolo[1,5-a]pyridin-3-yl)carbamate (4d)

A mixture of **4c** (180 mg, 0.79 mmol), N,N-diethylethanamine (161 mg, 1.59 mmol, 0.22 mL) and DPPA (284 mg, 1.03 mmol, 0.22 mL) in tBuOH (3 mL) was stirred at 110 °C for 6 h. A yellow suspension was formed. The reaction mixture was diluted with water (50 mL) and extracted with EtOAc (50 mL*3). The combined organic layer was washed with water (100 mL*3), brine (100 mL*2), dried over anhydrous Na₂SO₄, filtered and concentrated. The residue was purified by flash chromatography (EA in PE is 5-30%) to give **4d** (100 mg, 42% yield) as a yellow solid. LC-MS (ESI, Method 4) t_{R} = 2.31 min, m/z (M+H)⁺ = 298.1.

### Step 5. 5-Chloro-4-methoxypyrazolo[1,5-a]pyridin-3-amine (4e)

To a mixture of **4d** (100 mg, 0.34 mmol) in DCM (3 mL) was added TFA (1 mL) at 20 °C. The resulting mixture was stirred at 20 °C for 1 h. A yellow solution was formed. The reaction mixture was quenched with *sat. aq.* NaHCO₃ (50 mL) and extracted with EtOAc (30 mL*3). The combined organic layer was washed with water (60 mL*3), brine (60 mL*2), dried over anhydrous Na₂SO₄, filtered and concentrated. The residue was purified by flash chromatography (EA in PE is 10-60%) to give **4e** (40 mg, 60% yield) as a yellow solid. LC-MS (ESI, Method 4) t_{R} = 0.65 min, m/z (M+H)⁺ = 198.1.

### Step 6. 2-Chloro-4-((5-chloro-4-methoxypyrazolo[1,5-a]pyridin-3-yl)amino)-N-(methyl-d₃)pyrimidine-5-carboxamide (4f)

To a mixture of **4e** (40 mg, 0.20 mmol) and **Int. E** (42 mg, 0.20 mmol) in DCM (5 mL), was added DIPEA (52 mg, 0.40 mmol, 0.07 mL) at 0 °C. The resulting mixture was stirred at 20 °C for 12 h. A yellow solution was formed. The reaction mixture was quenched with water (50 mL) and extracted with EtOAc (30 mL*3). The combined organic layer was washed with water (60 mL*3), brine (60 mL*2), dried over anhydrous Na₂SO₄, filtered and concentrated to give **4f** (74 mg, 98% yield) as a white solid. LC-MS (ESI, Method 4) t_{R} = 2.88 min, m/z (M+H)⁺ = 370.1.

### Step 7. 2-Amino-4-((5-chloro-4-methoxypyrazolo[1,5-a]pyridin-3-yl)amino)-N-(methyl-d₃)pyrimidine-5-carboxamide (4g)

A mixture of **4f** (20 mg, 0.054 mmol) and TsOH (19 mg, 0.11 mmol) in NH₃/dioxane solution (3 mL, 0.4 M) was stirred at 80 °C for 72 h. A white suspension was formed. The reaction mixture was diluted with water (40 mL) and extracted with DCM (40 mL*3). The combined organic layer was washed with water (40 mL*3), brine (40 mL*2), dried over anhydrous Na₂SO₄, filtered and concentrated. The residue was purified by *prep-*TLC (DCM/MeOH = 10/1) to give **4g** (12 mg, 63% yield) as a yellow solid. LC-MS (ESI, Method 4) t_{R} = 1.87 min, m/z (M+H)⁺ = 351.2.

### Step 8. 4-(N-(5-chloro-4-methoxypyrazolo[1,5-a]pyridin-3-yl)cyclopropane carboxamido)-2-(cyclopropanecarboxamido)-N-(methyl-d₃)pyrimidine-5-carboxamide (4h)

A mixture of **4g** (12 mg, 0.034 mmol), cyclopropane carbonyl chloride (11 mg, 0.103 mmol) and DIPEA (17 mg, 0.171 mmol, 0.024 mL) in DCM (2 mL) was stirred at 20 °C for 12 h. A yellow solution was formed. The reaction mixture was concentrated and dried in vacuo to give **4h** (16 mg, crude) as a yellow solid, which was used for the next step directly without further purification. LC-MS (ESI, Method 4) t_{R} = 2.94 min, m/z (M+H)⁺ = 487.3.

### Step 9. 4-((5-Chloro-4-methoxypyrazolo[1,5-a]pyridin-3-yl)amino)-2-(cyclopropanecarboxamido )-N-(methyl-d₃)pyrimidine-5-carboxamide (4)

A mixture of **4h** (16 mg, crude) and K₂CO₃ (9 mg, 0.066 mmol) in MeOH (3 mL) was stirred at 20 °C for 12 h. A yellow solution was formed. The rection mixture was filtered and purified by *prep-*HPLC (Method D) further lyophilizate to give **4** (2.3 mg, 16.7% yield) as a yellow solid. ¹H NMR (400 MHz, DMSO-*d*₆) *δ* 11.82 (s, 1H), 10.98 (s, 1H), 9.62 (s, 1H), 8.72 (s, 1H), 8.62 (s, 1H), 8.39 (d, *J =* 7.2 Hz, 1H), 6.83 (d, *J* = 7.2 Hz, 1H), 4.09 (s, 3H), 2.15-2.06 (m, 1H), 0.93-0.80 (m, 4H). LC-MS (ESI, Method 4) t_{R} = 2.17 min, m/z (M+H)⁺ = 419.2.

### Example 5

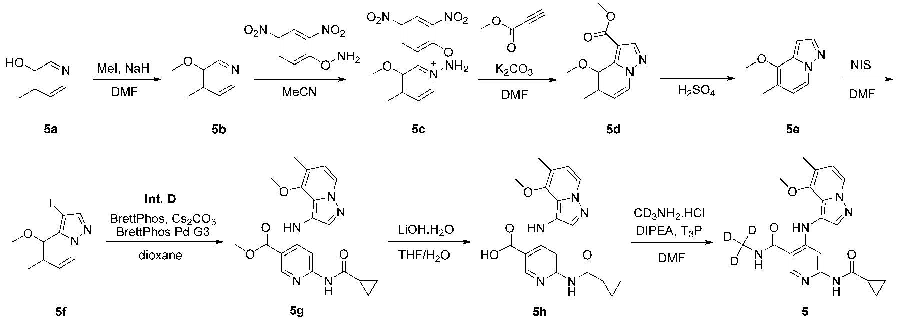

### Step 1. 3-Methoxy-4-methylpyridine (5b)

To a mixture of **5a** (2.50 g, 22.91 mmol) in DMF (20 mL), was added NaH (1.37 g, 34.36 mmol, 60% in mineral oil) and CH₃I (3.58 g, 25.20 mmol, 1.57 mL) at 0 °C. The resulting mixture was stirred at 20 °C for 2 h. A black suspension was formed. The reaction mixture was diluted with water (100 mL), then extracted with EtOAc (50 mL*2). The combined organic layer was washed with water (50 mL*2), brine (50 mL*2), dried over anhydrous Na₂SO₄, filtered and concentrated. The residue was purified by flash chromatography (EA in PE is 10-30%) to give **5b** (230 mg, 8% yield) as a yellow solid. ¹H NMR (400 MHz, CDCl₃) *δ* 8.15 (s, 1H), 8.10 (d, *J =* 4.8 Hz, 1H), 6.72 (m, 1H), 3.90 (s, 3H), 2.21 (s, 3H). LC-MS (ESI, Method 4) t_{R} = 0.56 min, m/z (M+H)⁺ = 124.0.

### Step 2. 1-Amino-3-methoxy-4-methylpyridin-1-ium 2,4-dinitrophenolate (5c)

A mixture of **5b** (230 mg, 1.87 mmol) and O-(2,4-dinitrophenyl) hydroxylamine (409 mg, 2.05 mmol) in MeCN (3 mL) was stirred at 50 °C for 16 h. A yellow solution was formed. The reaction was concentrated to give **5c** (260 mg, crude) as a yellow solid, which was used for the next step directly without further purification. LC-MS (ESI, Method 4) t_{R} = 1.16 min, m/z M⁺ = 140.1.

### Step 3. Methyl 4-methoxy-5-methylpyrazolo[1,5-a]pyridine-3-carboxylate (5d)

A mixture of methyl propiolate (236 mg, 2.80 mmol, 0.25 mL), **5c** (260 mg, 1.87 mmol) and K₂CO₃ (516 mg, 3.74 mmol) in DMF (3 mL) was stirred at 20 °C for 2 h. A black suspension was formed. The reaction mixture was concentrated in vacuo and diluted with water (50 mL), then extracted with EtOAc (50 mL*2). The combined organic layer was washed with water (50 mL*2), brine (50 mL*2), dried over anhydrous Na₂SO₄, filtered and concentrated. The residue was purified by flash chromatography (EA in PE is 10-30%) to give 5d (140 mg, 34% yield) as a yellow solid. LC-MS (ESI, Method 4) t_{R} = 2.60 min, m/z (M+H)⁺ = 221.0.

### Step 4. 4-Methoxy-5-methylpyrazolo[1,5-a]pyridine (5e)

A well-stirred solution of **5d** (140 mg, 0.636 mmol) in 2 mL of 50% H₂SO₄ was heated at 110°C for 3 h. A yellow solution was formed. Cool down the solution to room temperature. Neutralize the solution with *aq.* NaOH (1.0 M) using litmus paper as the indicator. Add 40 mL of water to the above solution. Extract the solution by EtOAc (30 mL*3). The combined organic layer was dried over anhydrous Na₂SO₄, filtered through a pad of celite and evaporated to give **5e** (50 mg, 48% yield) as a light-yellow solid. ¹H NMR (400 MHz, CDCl₃) *δ* 8.18 (d, *J =* 7.2 Hz, 1H), 7.87 (d, *J=* 2.4 Hz, 1H), 6.54 (d, *J =* 7.2 Hz, 1H), 6.51 (d, *J =* 2.4 Hz, 1H), 3.95 (s, 3H), 2.28 (s, 3H). LC-MS (ESI, Method 4) t_{R} = 2.29 min, m/z (M+H)⁺ = 163.1.

### Step 5. 3-Iodo-4-methoxy-5-methylpyrazolo[1,5-a]pyridine (5f)

A mixture of **5e** (50 mg, 0.308 mmol) and NIS (73 mg, 0.324 mmol) in DMF (1 mL) was stirred at 20 °C for 24 h. A yellow suspension was formed. The reaction mixture was quenched with water (50 mL) and extracted with EtOAc (30 mL*3). The combined organic layer was washed with brine (40 mL*2), dried over anhydrous Na₂SO₄, filtered and concentrated. The residue was purified by flash chromatography (EA in PE is 10-30%) to give **5f** (70 mg, 79% yield) as a yellow solid. LCMS (ESI, Method 4) t_{R} = 2.93 min, m/z (M+H)⁺ = 289.0. ¹H NMR (400 MHz, CDCl₃) *δ* 8.20 (d, *J* = 6.8 Hz, 1H), 7.85 (s, 1H), 6.59 (d, *J* = 6.8 Hz, 1H), 3.88 (s, 3H), 2.32 (s, 3H).

### Step 6. Methyl 6-(cyclopropanecarboxamido)-4-((4-methoxy-5-methylpyrazolo [1,5-a]pyridin-3-yl)amino)nicotinate (5g)

A mixture of **5f** (70 mg, 0.243 mmol), **Int. D** (102 mg, 0.29 mmol, TFA salt), BrettPhos (26 mg, 0.049 mmol), Cs₂CO₃ (198 mg, 0.607 mmol) and BrettPhos Pd G3 (22 mg, 0.024 mmol) in dioxane (3 mL) was degassed and purged with nitrogen for 3 times. The resulting mixture was stirred at 100 °C under N₂ atmosphere for 72 h. A yellow suspension was formed. The reaction mixture was concentrated and purified by *prep-*TLC (DCM/MeOH = 10/1) to give **5g** (60 mg, 62% yield) as a yellow solid. LC-MS (ESI, Method 4) t_{R} = 2.18 min, m/z (M+H)⁺ = 396.3.

### Step 7. 6-(Cyclopropanecarboxamido)-4-((4-methoxy-5-methylpyrazolo[1,5-a]pyridin-3-yl)amino)nicotinic acid (5h)

A mixture of **5g** (60 mg, 0.152 mmol) and LiOH.H₂O (19 mg, 0.455 mmol) in THF (3 mL) and water (1 mL) was stirred at 40 °C for 12 h. A yellow solution was formed. The reaction mixture was concentrated and dried in vacuo to give **5h** (58 mg, crude) as a yellow solid, which was used for the next step directly without further purification. LC-MS (ESI, Method 4) t_{R} = 1.78 min, m/z (M+H)⁺ = 382.2.

### Step 8. 6-(Cyclopropanecarboxamido)-4-((4-methoxy-5-methylpyrazolo[1,5-a]pyridin-3-yl)amino)-N-(methyl-d₃)nicotinamide (5)

A mixture of CD₃NH₂.HCl (32 mg, 0.456 mmol), **5h** (58 mg, crude), DIPEA (118 mg, 0.912 mmol, 0.158 mL) and T₃P (290 mg, 0.456 mmol, 50% purity in EtOAc) in DMF (2 mL) was stirred at 20 °C for 12 h. A yellow solution was formed. The rection mixture was filtered and purified by *prep-*HPLC (Method D) to give **5** (5.2 mg, 9% yield) as a light-yellow solid. ¹H NMR (400 MHz, DMSO-*d*₆) *δ* 10.65 (s, 1H), 10.28 (s, 1H), 8.51 (s, 1H), 8.48 (s, 1H), 8.35 (d, *J* = 6.8 Hz, 1H), 7.99 (s, 1H), 7.64 (s, 1H), 6.73 (d, *J =* 6.8 Hz, 1H), 3.66 (s, 3H), 2.22 (s, 3H), 1.98-1.90 (m, 1H), 0.74-0.70 (m, 4H). LC-MS (ESI, Method 4) t_{R} = 1.82 min, m/z (M+H)⁺ = 398.3.

### Example 6

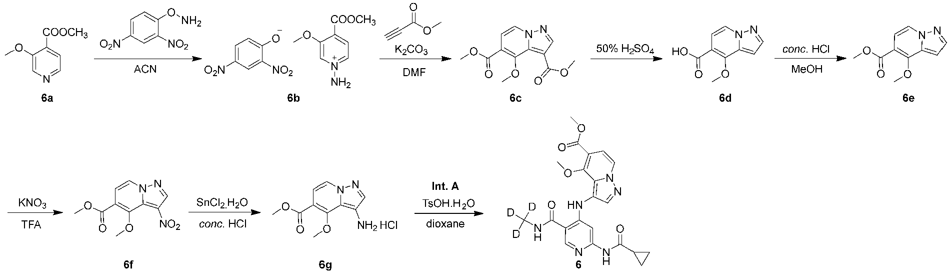

### Step 1. 1-Amino-3-methoxy-4-(methoxycarbonyl)pyridin-1-ium 2,4-dinitrophenolate (6b)

A mixture of **6a** (6.8 g, 40.68 mmol) and O-(2,4-dinitrophenyl)hydroxylamine (9.72 g, 48.82 mmol) in ACN (60 mL) was stirred at 50 °C overnight. The mixture was concentrated to afford **6b** (14.9 g, crude) as a yellow solid, which was used for the next step directly without further purification. LC-MS (ESI, Method 3) t_{R} = 0.46 min, m/z M⁺= 183.0.

### Step 2. Dimethyl 4-methoxypyrazolo[1,5-a]pyridine-3,5-dicarboxylate (6c)

A mixture of **6b** (14.9 g, 40.68 mmol), methyl prop-2-ynoate (3.53 g, 42.03 mmol) and K₂CO₃ (10.56 g, 76.43 mmol) in DMF (60 mL) was stirred at 30 °C for 2 h. The reaction mixture was poured into ice-water (200 mL) and extracted with EtOAc (200 mL*2). The combined organic phase was washed with brine (100 mL*2) and concentrated. The residue was purified by flash chromatography on silica gel (PE/EtOAc = 5/1) to afford **6c** (6.62 g, 62% yield) as a yellow solid. LC-MS (ESI, Method 3) t_{R} = 1.10 min, m/z (M+H)⁺= 265.2.

### Step 3. 4-Methoxypyrazolo[1,5-a]pyridine-5-carboxylic acid (6d)

A mixture of **6c** (7 g, 26.49 mmol) in *aq.* H₂SO₄ (130 mL, 50% wt in water) was stirred at 85 °C for 4 h. After cooling to r.t., the mixture was diluted with water (200 mL), neutralized with *aq.* NaOH (1 N) to pH = 3. The mixture was extracted with EtOAc (200 mL*3). The compound orgainc layer was washed with brine (100 mL) and concentrated to afford **6d** (4.4 g, 86% yield) as a brown solid. LC-MS (ESI, Method 3) t_{R} = 0.94 min, m/z (M+H)⁺ = 193.1.

### Step 4. Methyl 4-methoxypyrazolo[1,5-a]pyridine-5-carboxylate (6e)

To a solution of **6d** (4.4 g, 22.90 mmol) in MeOH (250 mL) was added *conc.* HCl (3.75 mL, 46 mmol). After stirring for 18 h at 80 °C, the mixture was concentrated and the residue was diluted with DCM (20 mL). The solution was washed with *sat.* NaHCO₃ (20 mL) and the aqeous layer was extracted with DCM (20 mL*2). The combined organic layer was concentrated and the residue was purified by flash chromatography on silica gel (PE/EA = 4/1) to afford **6e** (2.76 g, 58% yield) as a yellow solid. LC-MS (ESI, Method 3) t_{R} = 1.14 min, m/z (M+H)⁺ = 207.1.

### Step 5. Methyl 4-methoxy-3-nitropyrazolo[1,5-a]pyridine-5-carboxylate (6f)

To a solution of **6e** (500 mg, 2.42 mmol) in TFA (5 mL) was added KNO₃ (245 mg, 2.42 mmol) at r.t. and the mixture was stirred at 35 °C for 3 h. The solvent was removed by pumping through N₂. The mixture was basified with *sat.* Na₂CO₃ to pH > 8 and extracted with EtOAc (5 mL*3). The organic layer was concentrated and purified by flash chromatography (PE/EA = 3/1) to afford the crude product. The crude product was triturated with EtOAc (3 mL). The formed soid was filtered and dried to afford **6f** (300 mg, 49% yield) as a yellow solid. LC-MS (ESI, Method 3) t_{R} = 1.06 min, m/z (M+H)⁺ = 252.2.

### Step 6. Methyl 3-amino-4-methoxypyrazolo[1,5-a]pyridine-5-carboxylate hydrochloride (6g)

To a solution of **6f** (200 mg, 0.79 mmol) in *conc.* HCl (4 mL) was added SnCl₂·2H₂O (359 mg, 1.59 mmol) at 0 °C and the mixture was stirred at 0 °C for 2 h. The formed soild was filtered and dried to afford **6g** (150 mg, 73% yield) as a yellow solid. LC-MS (ESI, Method 3) t_{R} = 0.53 min, m/z (M+H)⁺ = 222.0.

### Step 7. Methyl 3-((2-(cyclopropanecarboxamido)-5-((methyl-d₃)carbamoyl)pyridin-4-yl)amino)-4-methoxypyrazolo[1,5-a]pyridine-5-carboxylate (6)

To a solution of **6g** (150 mg, 0.58 mmol) and **Int. A** (149 mg, 0.58 mmol) in dioxane (2 mL) was added TsOH.H₂O (22 mg, 0.12 mmol) and stirred at 100 °C for 12 h. The reaction mixture was concentrated and purified by flash chromatography on silical gel (DCM/MeOH = 10/1) to afford **6** (110 mg, 43% yield) as a yellow solid. ¹H NMR (400 MHz, DMSO-*d*₆) *δ* 10.76 (s, 1H), 10.68 (s, 1H), 8.56 (s, 1H), 8.51 (s, 1H), 8.44 (d, *J* = 7.2 Hz, 1H), 8.18 (s, 1H), 7.87 (s, 1H), 7.07 (d, *J =* 7.2 Hz, 1H), 3.87 (s, 3H), 3.87 (s, 3H), 2.03-1.92 (m, 1H), 0.80-0.70 (m, 4H). LC-MS (ESI, Method 3) t_{R} = 1.05 min, m/z (M+H)⁺ = 442.1.

### Example 7

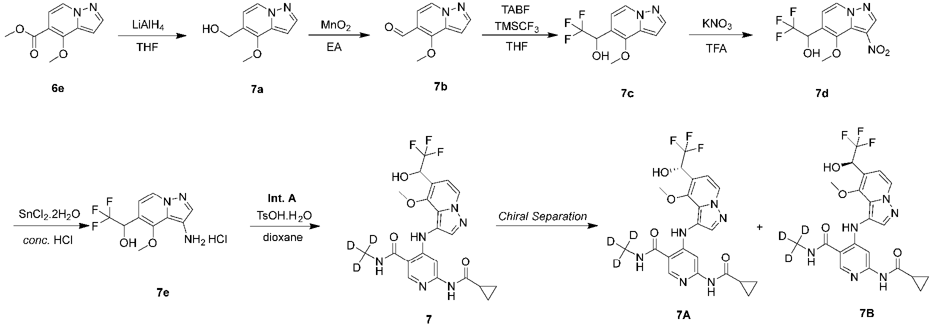

### Step 1. (4-Methoxypyrazolo[1,5-a]pyridin-5-yl)methanol (7a)

To a solution of **6e** (1.3 g, 6.30 mmol) in THF (15 mL) was added LiAlH₄ (455 mg, 11.98 mmol) at 0 °C. After stirring at 0 °C for 1 h, the reaction was quenched with aqueous solution of seignette salt (10 mL) and extracted with EtOAc (15 mL*2). The combined organic layer was concentrated, and the residue was purified by flash chromatography on silica gel (PE/EA = 2/1) to afford **7a** (1.07 g, 95% yield) as a white solid. LC-MS (ESI, Method 3) t_{R} = 0.94 min, m/z (M+H)⁺ = 179.0.

### Step 2. 4-Methoxypyrazolo[1,5-a]pyridine-5-carbaldehyde (7b)

To a solution of **7a** (1.0 g, 5.62 mmol) in EtOAc (10 mL) was added MnO₂ (2.92 g, 33.68 mmol) at r.t. After stirring for 4 h at 80 °C, the mixture was filtered, and the filtrate was concentrated to afford **7b** (800 mg, 81% yield) as a yellow solid. LC-MS (ESI, Method 3) t_{R} = 1.02 min, m/z (M+H)⁺ = 177.3.

### Step 3. 2,2,2-Trifluoro-1-(4-methoxypyrazolo[1,5-a]pyridin-5-yl)ethan-1-ol (7c)

To a solution of **7b** (620 mg, 3.52 mmol) and trimethyl(trifluoromethyl)silane (1.10 g, 7.74 mmol) in THF (10 mL) was added TBAF (0.2 mL, 0.2 mmol, 1.0 M in THF) at 0 °C. The mixture was stirred at 0 °C for 1 h and at 25 °C for 16 h. Then 1 M HCl solution was added, and the reaction was stirred at 25 °C for 2 h. The mixture was adjusted to pH = 8 with *aq.* NaOH (1 M). The mixture was extracted with EtOAc (5 mL*3). The organic layer was concentrated, and the residue was purified by flash chromatography (PE/EA = 3/1) to afford **7c** (834 mg, 96% yield) as a yellow oil. ¹H NMR (400 MHz, DMSO-*d*₆) δ 8.52 (d, *J =* 6.8 Hz, 1H), 8.04 (d, *J* = 2.4 Hz, 1H), 6.97-6.88 (m, 3H), 5.48-5.45 (m, 1H), 4.03 (s, 3H). LC-MS (ESI, Method 3) t_{R} = 1.09 min, m/z (M+H)⁺ = 247.2.

### Step 4. 2,2,2-Trifluoro-1-(4-methoxy-3-nitropyrazolo[1,5-a]pyridin-5-yl)ethan-1-ol (7d)

A mixture **of 7c** (210 mg, 0.85 mmol) and KNO₃ (95 mg, 0.94 mmol) in TFA (3 mL) was stirred at 30 °C for 2 h. The solvent was removed with pumping through N₂. The residue was dissolved in EtOAc (5 mL) and basified with *sat.* Na₂CO₃ solution (10 mL) to pH = 9. The mixture was extracted with EtOAc (10 mL*2). The combined organic phase was concentrated, and the residue was purified by flash chromatography on silica gel (DCM/MeOH = 19/1) to afford **7d** (150 mg, 60% yield) as a brown solid. ¹H NMR (400 MHz, DMSO-*d*₆) δ 8.96 (s, 1H), 8.86 (d, *J =* 6.8 Hz, 1H), 7.35 (d, *J =* 7.2 Hz, 1H), 7.31 (d, *J =* 6.0 Hz, 1H), 5.62-5.55 (m, 1H), 3.80 (s, 3H). LC-MS (ESI, Method 3) t_{R} = 1.12 min, m/z (M+H)⁺ = 292.1.

### Step 5. 1-(3-Amino-4-methoxypyrazolo[1,5-a]pyridin-5-yl)-2,2,2-trifluoroethan-1-ol hydrochloride (7e)

To a mixture **of 7d** (200 mg, 0.69 mmol) in *conc.* HCl (4 mL) was added SnCl₂·2H₂O (620 mg, 2.75 mmol) at 0 °C. After stirring at 10 °C for 1 h, the reaction mixture was filtered. The filter cake was dried under vacuum to afford **7e** (150 mg, 66% yield) as a yellow solid. LC-MS (ESI, Method 3) t_{R} = 0.85 min, m/z (M+H)⁺ = 262.0.

### Step 6. 6-(Cyclopropanecarboxamido)-4-((4-methoxy-5-(2,2,2-trifluoro-1-hydroxyethyl)pyrazolo[1,5-a]pyridin-3-yl)amino)-N-(methyl-d₃)nicotinamide (7)

A mixture of **7e** (193 mg, 0.59 mmol), **Int. A** (151 mg, 0.59 mmol) and TsOH.H₂O (45 mg, 0.24 mmol) in 1,4-dioxane (3 mL) was stirred at 100 °C for 16 h in a sealed tube. The reaction mixture was concentrated, and the residue was purified by flash chromatography on silica gel (DCM/MeOH = 9/1) to afford the crude product. The crude product was purified by *prep-HPLC* (Method A) to afford **7** (250 mg, 88% yield) as a white solid. ¹H NMR (400 MHz, DMSO-*d*₆) δ 10.68 (s, 1H), 10.36 (s, 1H), 8.55 (s, 1H), 8.51-8.49 (m, 2H), 8.11 (s, 1H), 7.68 (s, 1H), 7.02 (d, *J* = 5.6 Hz, 1H), 6.90 (d, *J =* 7.6 Hz, 1H), 5.43-5.36 (m, 1H), 3.72 (s, 3H), 1.99-1.90 (m, 1H), 0.76-0.67 (m, 4H). LC-MS (ESI, Method 2) t_{R} = 3.15 min, m/z (M+H)⁺ = 482.0. **Step 7. (*S*)-6-(cyclopropanecarboxamido)-4-((4-methoxy-5-(2,2,2-trifluoro-1-hydroxyethyl)pyrazolo[1,5-*a*]pyridin-3-yl)amino)-*N-*(methyl-*d*₃)nicotinamide (7A) and (*R*)-6-(cyclopropanecarboxamido)-4-((4-methoxy-5-(2,2,2-trifluoro-1-hydroxyethyl)pyrazolo[1,5-*a*]pyridin-3-yl)amino)-*N-*(methyl-*d*₃)nicotinamide (7B)**

Compound **7** (520 mg, 1.08 mmol) was separated by chiral *prep*-HPLC (Method F) to obtain **7A** (204.2 mg, 39% yield) as a white solid and **7B** (206.1 mg, 40% yield) as a white solid.

**7A:** ¹H NMR (400 MHz, DMSO-*d*₆) δ 10.67 (s, 1H), 10.36 (s, 1H), 8.54 (s, 1H), 8.51-8.49 (m, 2H), 8.11 (s, 1H), 7.68 (s, 1H), 7.01 (d, *J* = 5.6 Hz, 1H), 6.90 (d, *J* = 7.2 Hz, 1H), 5.45-5.32 (m, 1H), 3.72 (s, 3H), 1.97-1.90 (m, 1H), 0.74-0.71 (m, 4H). LC-MS (ESI, Method 2) t_{R} = 2.39 min, m/z (M+H)⁺ = 482.1. HPLC (Method 5) t_{R} = 10.03 min.

7B: ¹H NMR (400 MHz, DMSO-*d*₆) δ 10.67 (s, 1H), 10.36 (s, 1H), 8.54 (s, 1H), 8.51-8.49 (m, 2H), 8.11 (s, 1H), 7.68 (s, 1H), 7.01 (d, *J =* 6.0 Hz, 1H), 6.89 (d, *J* = 7.2 Hz, 1H), 5.41-5.30 (m, 1H), 3.72 (s, 3H), 1.97-1.90 (m, 1H), 0.74-0.71 (m, 4H). LC-MS (ESI, Method 2) t_{R} = 2.40 min, m/z (M+H)⁺ = 482.1. HPLC (Method 5) t_{R} = 12.27 min.

### Example 8

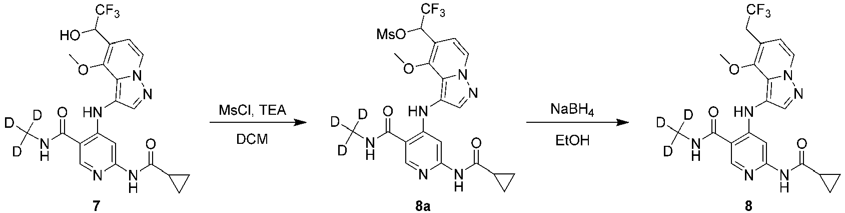

### Step 1. 1-(3-((2-(Cyclopropanecarboxamido)-5-((methyl-d₃)carbamoyl)pyridin-4-yl)amino)-4-methoxypyrazolo[1,5-a]pyridin-5-yl)-2,2,2-trifluoroethyl methanesulfonate (8a)

To a solution of **7** (80 mg, 0.17 mmol) and TEA (100 mg, 1.00 mmol, 0.14 mL) in DCM (1 mL) was added MsCl (29 mg, 0.50 mmol) at 0 °C. Then the mixture was stirred at 14 °C for 30 min. The reaction was quenched with ice-water (3 mL), extracted with DCM (5 mL*3). The combined organic layer was dried over Na₂SO₄, filtered and the filtrate was concentrated to afford **8a** (90 mg, crude) as a yellow oil, which was used for the next step directly without further purification. LC-MS (ESI, Method 3) t_{R} = 1.12 min, m/z (M+H)⁺ = 560.2.

### Step 2. 6-(Cyclopropanecarboxamido)-4-((4-methoxy-5-(2,2,2-trifluoroethyl)pyrazolo[1,5-a]pyridin-3-yl)amino)-N-(methyl-d₃)nicotinamide (8)

To a solution of **8a** (90 mg, 0.16 mmol) in EtOH (1 mL) was added NaBH₄ (9 mg, 0.24 mmol) at 0 °C, then the mixture was stirred at 15 °C for 18 h. The reaction mixture was quenched with ice-water (3 mL) and extracted with EtOAc (5 mL*2). The combined organic layer was concentrated and the residue was purified by *prep-HPLC* (Method A) to afford **8** (3.5 mg, 5% yield) as a white solid. ¹H NMR (400 MHz, DMSO-*d*₆) δ 10.68 (s, 1H), 10.35 (s, 1H), 8.55 (s, 1H), 8.51-8.48 (m, 2H), 8.10 (s, 1H), 7.69 (s, 1H), 6.83 (d, *J* = 6.8 Hz, 1H), 3.71 (s, 3H), 3.74-3.65 (m, 2H), 1.96-1.93 (m, 1H), 0.75-0.72 (m, 4H). LC-MS (ESI, Method 2) t_{R} = 2.91 min, m/z (M+H)⁺ = 466.1.

### Example 9

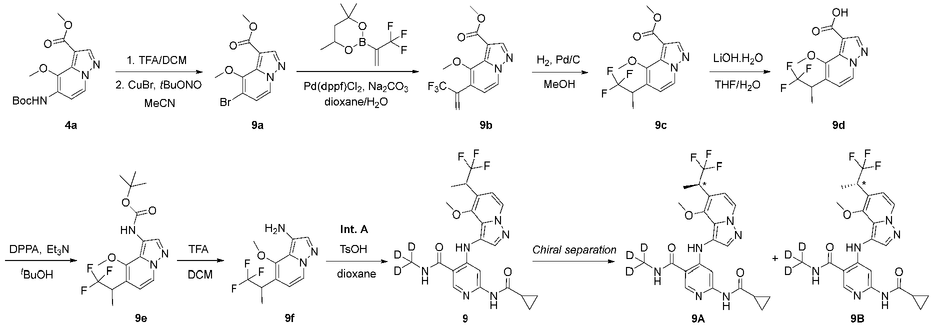

### Step 1. Methyl 5-bromo-4-methoxy-pyrazolo[1,5-a]pyridine-3-carboxylate (9a)

To a mixture of **4a** (2.20 g, 6.85 mmol) in DCM (10 mL), was added TFA (10 mL) at 20 °C. The resulting mixture was stirred at 20 °C for 1 h. A yellow solution was formed. The rection mixture was concentrated to give methyl 5-amino-4-methoxy-pyrazolo[1,5-*a*]pyridine-3-carboxylate (1.51 g, TFA salt, crude) as a yellow solid. LC-MS (ESI, Method 4) t_{R} = 1.38 min, m/z (M+H)⁺ = 222.1.

To a mixture of methyl 5-amino-4-methoxy-pyrazolo[1,5-*a*]pyridine-3-carboxylate (1.51 mg, TFA salt, crude) in MeCN (10 mL), was added *tert-*butyl nitrite (1.06 g, 10.24 mmol, 1.22 mL) at 0 °C, and stirred for 10 min. Then CuBr (1.96 g, 13.65 mmol) was added into the above mixture, the resulting mixture was stirred at 80 °C for 2 h. A yellow solution was formed. The reaction mixture was concentrated and purified by flash chromatography (EA in PE is 10-30%) to give **9a** (300 mg, 15% yield) as a yellow solid. LC-MS (ESI, Method 4) t_{R} = 2.53 min, m/z (⁷⁹Br, M+H)⁺ = 285.0.

### Step 2. Methyl 4-methoxy-5-(3,3,3-trifluoroprop-1-en-2-yl)pyrazolo[1,5-a]pyridine-3-carboxylate (9b)

A mixture of **9a** (80 mg, 0.281 mmol), 4,4,6-trimethyl-2-(3,3,3-trifluoroprop-1-en-2-yl)-1,3,2-dioxaborinane (86 mg, 0.365 mmol), Na₂CO₃ (89 mg, 0.842 mmol) and Pd(dppf)Cl₂ (46 mg, 0.056 mmol) in dioxane (2 mL) and water (0.2 mL) was degassed and purged with nitrogen for 3 times. The resulting mixture was stirred at 100 °C under N₂ atmosphere for 1 h. A yellow suspension was formed. The reaction mixture was diluted with water (30 mL) and extracted with EtOAc (30 mL*3). The combined organic layer was washed with water (50 mL*3), brine (50 mL*2), dried over anhydrous Na₂SO₄, filtered and concentrated. The residue was purified by flash chromatography (EA in PE is 0-20%) to give **9b** (84 mg, 99% yield) as a white solid. LC-MS (ESI, Method 4) t_{R} = 2.91 min, m/z (M+H)⁺ = 301.1.

### Step 3. Methyl 4-methoxy-5-(1,1,1-trifluoropropan-2-yl)pyrazolo[1,5-a]pyridine-3-carboxylate (9c)

To a solution of **9b** (84 mg, 0.28 mmol) in MeOH (5 mL), was added Pd/C (8 mg, 10% wt%, 10% Pd (dry basis), wetted with 55% H₂O). The reaction mixture was degassed and purged with hydrogen for 3 times. The resulting mixture was stirred at 40 °C under H₂ atmosphere for 24 h. A black suspension was formed. The reaction mixture was filtered and concentrated to give **9c** (80 mg, 99% yield) as a white solid. LC-MS (ESI, Method 4) t_{R} = 2.93 min, m/z (M+H)⁺ = 303.2.

### Step 4. 4-Methoxy-5-(1,1,1-trifluoropropan-2-yl)pyrazolo[1,5-a]pyridine-3-carboxylic acid (9d)

A mixture of **9c** (80 mg, 0.26 mmol) and LiOH.H₂O (89 mg, 2.12 mmol) in co-solvent of THF (3 mL) and water (1 mL) was stirred at 70 °C for 24 h. A yellow solution was formed. The reaction mixture was diluted with water (30 mL), extracted with EtOAc (20 mL) and the organic layer was discarded. The aqueous layer was adjusted to pH = 3 with *aq.* HCl (2 M), then extracted with EtOAc (20 mL*3). The organic layer was concentrated and dried in vacuo to give **9d** (76 mg, 99% yield) as a white solid. LC-MS (ESI, Method 4) t_{R} = 2.35 min, m/z (M+H)⁺ = 289.1.

### Step 5. Tert-butyl (4-methoxy-5-(1,1,1-trifluoropropan-2-yl)pyrazolo[1,5-a]pyridin-3-yl)carbamate (9e)

A mixture of **9d** (80 mg, 0.28 mmol), N,N-diethylethanamine (56 mg, 0.56 mmol, 0.08 mL) and DPPA (99 mg, 0.36 mmol, 0.08 mL) in tBuOH (1 mL) was stirred at 110 °C for 6 h. A yellow suspension was formed. The reaction mixture was diluted with water (30 mL) and extracted with EtOAc (30 mL*3). The combined organic layer was washed with water (50 mL*3), brine (50 mL*2), dried over anhydrous Na₂SO₄, filtered and concentrated. The residue was purified by flash chromatography (EA in PE is 5-30%) to give **9e** (20 mg, 20% yield) as a yellow solid. LC-MS (ESI, Method 4) t_{R} = 3.20 min, m/z (M+H)⁺ = 360.2.

### Step 6. 4-Methoxy-5-(1,1,1-trifluoropropan-2-yl)pyrazolo[1,5-a]pyridin-3-amine (9f)

To a mixture of **9e** (20 mg, 0.056 mmol) in DCM (3 mL), was added TFA (1 mL) at 20 °C. The resulting mixture was stirred at 20 °C for 1 h. A yellow solution was formed. The reaction mixture was quenched with *sat. aq.* NaHCO₃ (50 mL) and extracted with EtOAc (30 mL*3). The combined organic layer was washed with water (60 mL*3), brine (60 mL*2), dried over anhydrous Na₂SO₄, filtered and concentrated. The residue was purified by flash chromatography (EA in PE is 10-60%) to give **9f** (12 mg, 83% yield) as a yellow solid. LC-MS (ESI, Method 4) t_{R} = 1.87 min, m/z (M+H)⁺ = 260.2.

### Step 7. 6-(Cyclopropanecarboxamido)-4-((4-methoxy-5-(1,1,1-trifluoropropan-2-yl)pyrazolo[1,5-a]pyridin-3-yl)amino)-N-(methyl-d₃)nicotinamide (9)

A mixture of **9f** (10 mg, 0.039 mmol), **Int. A** (10 mg, 0.039 mmol) and TsOH (7 mg, 0.041 mmol) in dioxane (1 mL) was stirred at 100 °C for 2 h. A yellow solution was formed. The rection mixture was filtered and purified by *prep-HPLC* (Method E) to give **9** (3.7 mg, 20% yield) as an off-white solid. ¹H NMR (400 MHz, DMSO-*d*₆) *δ* 10.68 (s, 1H), 10.36 (s, 1H), 8.55 (s, 1H), 8.52-8.49 (m, 2H), 8.10 (s, 1H), 7.69 (s, 1H), 6.86 (d, *J* = 7.2 Hz, 1H), 4.16-4.07 (m, 1H), 3.68 (s, 3H), 1.98-1.90 (m, 1H), 1.44 (d, *J =* 7.2 Hz, 3H), 0.74-0.70 (m, 4H). LC-MS (ESI, Method 4) t_{R} = 2.27 min, m/z (M+H)⁺ = 480.3.

### Step 8. (R*) -6-(cyclopropanecarboxamido)-4-((4-methoxy-5-(1,1,1-trifluoropropan-2-yl)pyrazolo[1,5-a]pyridin-3-yl)amino)-N-(methyl-d₃)nicotinamide (9A) and (S*)-6-(cyclopropanecarboxamido)-4-((4-methoxy-5-(1,1,1-trifluoropropan-2-yl)pyrazolo[1,5-a]pyridin-3-yl)amino)-N-(methyl-d₃)nicotinamide (9B)

Compound **9** (24 mg, 0.05 mmol) was separated by chiral *prep-HPLC* (Method I) to obtain **9A** (9.5 mg, 40% yield) as a white solid and **9B** (9.1 mg, 38% yield) as a white solid.

**9A:** LC-MS (ESI, Method 4) t_{R} = 2.26 min, m/z (M+H)⁺ = 480.2. HPLC (Method 6) t_{R} = 6.42 min.

**9B:** LC-MS (ESI, Method 4) t_{R} = 2.27 min, m/z (M+H)⁺ = 480.3. HPLC (Method 6) t_{R} = 11.56 min.

### Example 10

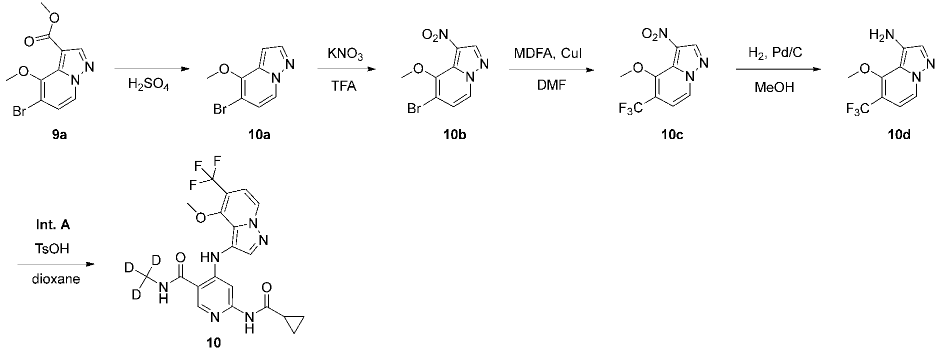

### Step 1. 5-Bromo-4-methoxy-pyrazolo[1,5-a]pyridine (10a)

Heat a well-stirred solution of **9a** (285 mg, 1.00 mmol) in 5 mL of 50% H₂SO₄ at 110°C for 3 h. A yellow solution was formed. Cool down the solution to room temperature. Neutralize the solution with *aq.* NaOH (1.0 M) using litmus paper as the indicator. Add 40 mL of water to the above solution. Extract the solution by EtOAc (30 mL*3). Combine the organic layer. Dry the organic layer over anhydrous Na₂SO₄. Filter the organic layer through a pad of celite. Evaporate the solvent in vacuum to give **10a** (220 mg, 97% yield) as a white solid. ¹H NMR (400 MHz, CDCl₃) *δ* 8.15 (dd, *J=* 7.2 Hz, 1.2 Hz, 1H), 7.91 (d, *J =* 2.4 Hz, 1H), 6.84 (d, *J =* 7.6 Hz, 1H), 6.63 (dd, *J* = 2.4 Hz, 0.8 Hz, 1H), 4.05 (s, 3H). LC-MS (ESI, Method 4) t_{R} = 2.54 min, m/z (⁷⁹Br, M+H)⁺ = 227.0.

### Step 2. 5-Bromo-4-methoxy-3-nitro-pyrazolo[1,5-a]pyridine (10b)

A mixture of **10a** (100 mg, 0.44 mmol) and KNO₃ (40 mg, 0.40 mmol) in TFA (3 mL) was stirred at 30 °C for 2 h. A yellow solution was formed. The reaction mixture was diluted with EtOAc (30 mL), quenched with *aq.* NaHCO₃ (50 mL), separated and extracted with EtOAc (30 mL*3). The combined organic layer was washed with water (50 mL*3), brine (50 mL*2), dried over anhydrous Na₂SO₄, filtered and concentrated. The residue was purified by flash chromatography (EA in PE is 5-30%) to give **10b** (100 mg, 83% yield) as a yellow solid. LC-MS (ESI, Method 4) t_{R} = 2.53 min, m/z (⁷⁹Br, M+H)⁺ = 272.0.

### Step 3. 4-Methoxy-3-nitro-5-(trifluoromethyl)pyrazolo[1,5-a]pyridine (10c)

A mixture of **10b** (80 mg, 0.29 mmol), CuI (67 mg, 0.35 mmol) and MDFA (85 mg, 0.44 mmol) in DMF (1 mL) was stirred at 100 °C for 12 h. A yellow suspension was formed. The reaction mixture was diluted with water (30 mL) and extracted with EtOAc (30 mL*3). The combined organic layer was washed with water (50 mL*3), brine (50 mL*2), dried over anhydrous Na₂SO₄, filtered and concentrated. The residue was purified by flash chromatography (EA in PE is 5-30%) to give **10c** (60 mg, 78% yield) as a yellow solid. ¹H NMR (400 MHz, CDCl₃) *δ* 8.74 (s, 1H), 8.47 (d, *J* = 7.2 Hz, 1H), 7.26 (d, *J =* 7.2 Hz, 1H), 4.01 (s, 3H). LC-MS (ESI, Method 4) t_{R} = 2.75 min, m/z (M+H)⁺ = 262.1.

### Step 4. 4-Methoxy-5-(trifluoromethyl)pyrazolo[1,5-a]pyridin-3-amine (10d)

To a solution of **10c** (60 mg, 0.23 mmol) in MeOH (5 mL), was added Pd/C (6 mg, 10% wt%, 10% Pd (dry basis), wetted with 55% H₂O). The equipment was degassed and purged with hydrogen for 3 times. The resulting mixture was stirred at 40 °C under H₂ atmosphere for 24 h. A black suspension was formed. The reaction mixture was filtered and concentrated to give **10d** (27 mg, 51% yield) as a yellow solid. LC-MS (ESI, Method 4) t_{R} = 1.92 min, m/z (M+H)⁺ = 232.1.

### Step 5. 6-(Cyclopropanecarboxamido)-4-((4-methoxy-5-(trifluoromethyl)pyrazolo[1,5-a]pyridin-3-yl)amino)-N-(methyl-d₃)nicotinamide (10)

A mixture of **10d** (27 mg, 0.12 mmol), **Int. A** (29 mg, 0.12 mmol) and TsOH (24 mg, 0.14 mmol) in dioxane (1 mL) was stirred at 100 °C for 2 h. A yellow solution was formed. The rection mixture was filtered and purified by flash chromatography (MeOH in DCM is 0-10%) and further triturated with MeCN to give **10** (9.7 mg, 18% yield) as an off-white solid. ¹H NMR (400 MHz, DMSO-*d*₆) *δ* 10.74 (s, 1H), 10.48 (s, 1H), 8.63-8.59 (m, 2H), 8.52 (s, 1H), 8.25 (s, 1H), 7.73 (s, 1H), 7.01 (d, *J* = 7.6 Hz, 1H), 3.81 (s, 3H), 1.98-1.90 (m, 1H), 0.76-0.71 (m, 4H). LC-MS (ESI, Method 4) t_{R} = 2.15 min, m/z (M+H)⁺ = 452.3.

### Example 11

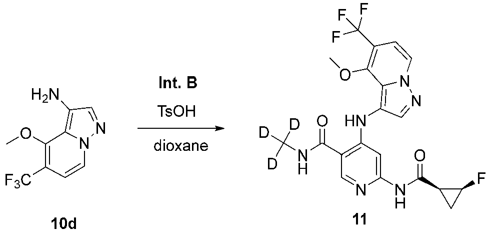

### Step 1. 6-((1S,2S)-2-fluorocyclopropane-1-carboxamido)-4-((4-methoxy-5-(trifluoromethyl)pyrazolo[1,5-a]pyridin-3-yl)amino)-N-(methyl-d₃)nicotinamide (11)

A mixture **of 10d** (16 mg, 0.068 mmol), **Int. B** (15 mg, 0.055 mmol) and TsOH (14 mg, 0.082 mmol) in dioxane (1 mL) was stirred at 100 °C for 6 h. A yellow suspension was formed. The reaction mixture was diluted with *aq.* NaHCO₃ (30 mL) and extracted with EtOAc (30 mL*3). The combined organic layer was washed with water (60 mL*2), brine (60 mL*2), dried over anhydrous Na₂SO₄, filtered and concentrated. The residue was purified by flash chromatography (MeOH in DCM is 0-10%) and triturated in MeCN to give **11** (17.4 mg, 66% yield) as a light-yellow solid. ¹H NMR (400 MHz, DMSO-*d*₆) *δ* 10.74 (s, 1H), 10.46 (s, 1H), 8.59-8.55 (m, 2H), 8.49 (s, 1H), 8.23 (s, 1H), 7.69 (s, 1H), 6.97 (d, *J* = 7.2 Hz, 1H), 4.93-4.72 (m, 1H), 3.78 (s, 3H), 2.14-2.06 (m, 1H), 1.56-1.43 (m, 1H), 1.11-1.02 (m, 1H). LC-MS (ESI, Method 4) t_{R} = 2.22 min, m/z (M+H)⁺ = 470.2.

### Example 12

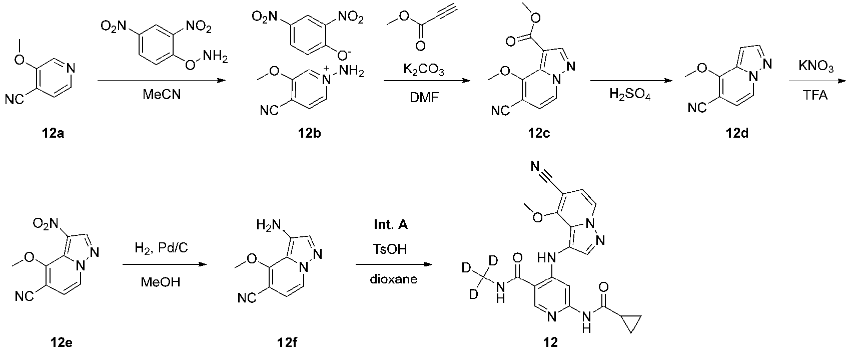

### Step 1. 1-Amino-4-cyano-3-methoxypyridin-1-ium 2,4-dinitrophenolate (12b)

A mixture of **12a** (500 mg, 3.73 mmol) and O-(2,4-dinitrophenyl) hydroxylamine (816 mg, 4.10 mmol) in MeCN (10 mL) was stirred at 50 °C for 16 h. A yellow solution was formed. The reaction was concentrated to give **12b** (559 mg, crude) as a yellow solid, which was used for the next step directly without further purification. LC-MS (ESI, Method 4) t_{R} = 0.64 min, m/z M⁺ = 150.1.

### Step 2. Methyl 5-cyano-4-methoxypyrazolo[1,5-a]pyridine-3-carboxylate (12c)

A mixture of methyl propiolate (626 mg, 7.45 mmol, 0.66 mL), **12b** (559 mg, 3.72 mmol) and K₂CO₃ (1.03 g, 7.45 mmol) in DMF (10 mL) was stirred at 20 °C for 2 h. A black suspension was formed. The reaction mixture was concentrated in vacuo and diluted with water (50 mL), then extracted with EtOAc (50 mL*2). The combined organic layer was washed with water (50 mL*2), brine (50 mL*2), dried over anhydrous Na₂SO₄, filtered and concentrated. The residue was purified by flash chromatography (EA in PE is 10-30%) to give **12c** (400 mg, 46% yield) as a yellow solid. ¹H NMR (400 MHz, CDCl₃) *δ* 8.45 (s, 1H), 8.32 (d, *J=* 7.2 Hz, 1H), 6.94 (d, *J* = 7.2 Hz, 1H), 4.29 (s, 3H), 3.92 (s, 3H). LC-MS (ESI, Method 4) t_{R} = 2.18 min, m/z (M+H)⁺ = 232.1.

### Step 3. 4-Methoxypyrazolo[1,5-a]pyridine-5-carbonitrile (12d)

Heat a well-stirred solution of **12c** (50 mg, 0.216 mmol) in 1 mL of 50% H₂SO₄ at 100°C for 1 h. A brown solution was formed. Cool down the solution to room temperature. Neutralize the solution with *aq.* NaOH (1.0 M) using litmus paper as the indicator. Add 40 mL of water to the above solution. Extract the solution by EtOAc (30 mL*3). Combine the organic layer. Dry the organic layer over anhydrous Na₂SO₄. Filter the organic layer through a pad of celite. Evaporate the organic layer in vacuum to give **12d** (20 mg, 53% yield) as a white solid. ¹H NMR (400 MHz, CDCl₃) *δ* 8.18 (dd, *J* = 7.2 Hz, 0.8 Hz, 1H), 7.98 (d, *J =* 2.4 Hz, 1H), 6.88 (dd, *J =* 2.4 Hz, 0.8 Hz, 1H), 6.72 (d, *J* = 7.2 Hz, 1H), 4.40 (s, 3H). LC-MS (ESI, Method 4) t_{R} = 2.07 min, m/z (M+H)⁺ = 174.2.

### Step 4. 4-Methoxy-3-nitro-pyrazolo[1,5-a]pyridine-5-carbonitrile (12e)

A mixture of **12d** (20 mg, 0.115 mmol) in TFA (1 mL) was added KNO₃ (11 mg, 0.11 mmol) at 20 °C. The resulting mixture was stirred at 30 °C for 2 h. A yellow solution was formed. The reaction mixture was quenched with *aq.* NaHCO₃ (50 mL) and extracted with EtOAc (30 mL*3). The combined organic layer was washed with brine (40 mL*2), dried over anhydrous Na₂SO₄, filtered and concentrated. The residue was purified by flash chromatography (EA in PE is 10-30%) to give **12e** (20 mg, 79% yield) as a yellow solid. LC-MS (ESI, Method 4) t_{R} = 2.19 min, m/z (M+H)⁺ = 219.1.

### Step 5. 3-Amino-4-methoxy-pyrazolo[1,5-a]pyridine-5-carbonitrile (12f)

A mixture of **12e** (20 mg, 0.092 mmol) and Pd/C (5 mg, 10% Pd (dry basis), wetted with 55% H₂O) in MeOH (3 mL) was degassed and purged with hydrogen for 3 times. The resulting mixture was stirred at 40 °C under H₂ atmosphere for 12 h. A black suspension was formed. The reaction mixture was filtered and concentrated to give **12f** (12 mg, 70% yield) as a yellow solid. LC-MS (ESI, Method 4) t_{R} = 0.62 min, m/z (M+H)⁺ = 189.1. ¹H NMR (400 MHz, CDCl₃) *δ* 7.83 (d, *J=* 7.2 Hz, 1H), 7.50 (s, 1H), 6.40 (d, *J=* 7.2 Hz, 1H), 4.41 (s, 3H).

### Step 6. 4-((5-Cyano-4-methoxypyrazolo[1,5-a]pyridin-3-yl)amino)-6-(cyclopropanecarboxamido)-N-(methyl-d₃)nicotinamide (12)

A mixture of **12f** (12 mg, 0.064 mmol), **Int. A** (13 mg, 0.051 mmol) and TsOH (13 mg, 0.076 mmol) in dioxane (2 mL) was stirred at 100 °C for 6 h. A yellow suspension was formed. The reaction mixture was diluted with *aq.* NaHCO₃ (30 mL) and extracted with EtOAc (30 mL*3). The combined organic layer was washed with water (60 mL*2), brine (50 mL*2), dried over anhydrous Na₂SO₄, filtered and concentrated. The residue was purified by flash chromatography (MeOH in DCM is 0-10%) and triturated in MeOH to give 12 (9.0 mg, 34% yield) as a yellow solid. ¹H NMR (400 MHz, DMSO-*d*₆) *δ* 10.89 (s, 1H), 10.81 (s, 1H), 8.58 (s, 1H), 8.52 (s, 1H), 8.41 (d, *J* = 7.2 Hz, 1H), 8.20 (s, 1H), 7.94 (s, 1H), 6.94 (d, *J* = 7.2 Hz, 1H), 4.25 (s, 3H), 2.01-1.94 (m, 1H), 0.80-0.77 (m, 4H). LC-MS (ESI, Method 4) t_{R} = 1.87 min, m/z (M+H)⁺ = 409.3.

### Example 13

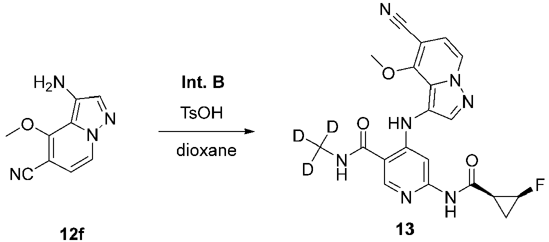

### Step 1. 4-((5-Cyano-4-methoxypyrazolo[1,5-a]pyridin-3-yl)amino)-6-((1S,2S)-2-fluorocyclopropane-1-carboxamido)-N-(methyl-d₃)nicotinamide (13)

A mixture of **12f** (18 mg, 0.096 mmol), **Int. B** (21 mg, 0.076 mmol) and TsOH (20 mg, 0.115 mmol) in dioxane (2 mL) was stirred at 100 °C for 6 h. A yellow suspension was formed. The reaction mixture was diluted with *aq.* NaHCO₃ (30 mL) and extracted with EtOAc (30 mL*3). The combined organic layer was washed with water (60 mL*2), brine (100 mL*2), dried over anhydrous Na₂SO₄, filtered and concentrated. The residue was purified by flash chromatography (MeOH in DCM is 0-10%) and triturated in MeCN to give **13** (23.4 mg, 72% yield) as a yellow solid. ¹H NMR (400 MHz, DMSO-*d*₆) *δ* 10.90 (s, 1H), 10.85 (s, 1H), 8.60 (s, 1H), 8.53 (s, 1H), 8.42 (d, *J* = 7.2 Hz, 1H), 8.22 (s, 1H), 7.94 (s, 1H), 6.95 (d, *J =* 7.2 Hz, 1H), 5.00-4.79 (m, 1H), 4.25 (s, 3H), 2.22-2.14 (m, 1H), 1.66-1.55 (m, 1H), 1.18-1.09 (m, 1H). LC-MS (ESI, Method 4) t_{R} = 1.85 min, m/z (M+H)⁺ = 427.2.

### Example 14

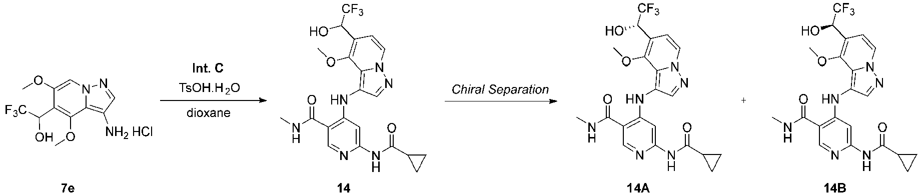

### Step 1. 6-(Cyclopropanecarboxamido)-4-((4-methoxy-3-(2,2,2-trifluoro-1-hydroxyethyl)pyrazolo[1,5-a]pyridin-5-yl)amino)-N-methylnicotinamide (14)

A solution of **7e** (150 mg, 0.46 mmol), **Int.** C (116 mg, 0.46 mmol) and TsOH.H₂O (38 mg, 0.20 mmol) in dioxane (3 mL) was stirred at 100 °C for 16 h. After cooling to r.t., the mixture was concentrated and the residue was purified by chromatography column on silica gel (DCM/MeOH = 20/1) to afford **14** (136 mg, 62% yield) as a white solid. LC-MS (ESI, Method 3) t_{R} = 1.08 min, m/z (M+H)⁺ = 479.2.

### Step 2. (S)-6-(cyclopropanecarboxamido)-4-((4-methoxy-3-(2,2,2-trifluoro-1-hydroxyethyl)pyrazolo[1,5-a]pyridin-5-yl)amino)-N-methylnicotinamide (14A) and (R)-6-(cyclopropanecarboxamido)-4-((4-methoxy-3-(2,2,2-trifluoro-1-hydroxyethyl)pyrazolo[1,5-a]pyridin-5-yl)amino)-N-methylnicotinamide (14B)

Compound **14** (136 mg, 0.28 mmol) was separated by chiral *prep-HPLC* (Method F) to afford **14A** (43 mg, 32% yield) as a white solid and **14B** (39 mg, 29% yield) as a white solid.

**14A:** ¹H NMR (400 MHz, DMSO-*d*₆) δ 10.66 (s, 1H), 10.34 (s, 1H), 7.56 (d, *J* = 4.4 Hz, 1H), 8.51-8.49 (m, 2H), 8.11 (s, 1H), 7.68 (s, 1H), 7.02 (d, *J =* 5.6 Hz, 1H), 6.89 (d, *J* = 7.2 Hz, 1H), 5.41-5.37 (m, 1H), 3.72 (s, 3H), 2.80 (d, *J* = 4.4 Hz, 3H), 1.94-1.92 (m, 1H), 0.73-0.71 (m, 4H). LC-MS (ESI, Method 2) t_{R} = 2.39 min, m/z (M+H)⁺ = 479.2. HPLC (Method 5) t_{R} = 8.98 min.

**14B:** ¹H NMR (400 MHz, DMSO-*d*₆) δ 10.66 (s, 1H), 10.35 (s, 1H), 7.56 (d, *J* = 4.4 Hz, 1H), 8.51-8.49 (m, 2H), 8.11 (s, 1H), 7.68 (s, 1H), 7.03 (d, *J* = 5.6 Hz, 1H), 6.90 (d, *J* = 7.2 Hz, 1H), 5.41-5.38 (m, 1H), 3.72 (s, 3H), 2.80 (d, *J* = 4.4 Hz, 3H), 1.95-1.92 (m, 1H), 0.74-0.72 (m, 4H). LC-MS (ESI, Method 2) t_{R} = 2.39 min, m/z (M+H)⁺ = 479.2. HPLC (Method 5) t_{R} = 10.89 min.

### Example 15

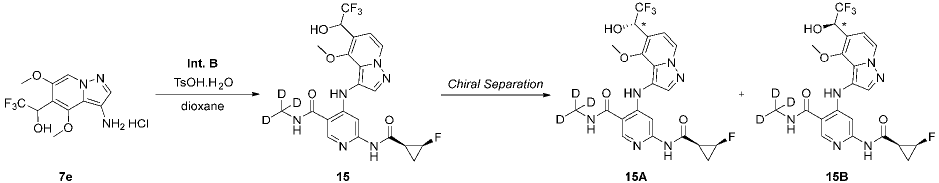

### Step 1. 6-((1S,2S)-2-fluorocyclopropane-1-carboxamido)-4-((4-methoxy-5-(2,2,2-trifluoro-1-hydroxyethyl)pyrazolo[1,5-a]pyridin-3-yl)amino)-N-(methyl-d₃)nicotinamide (15)

A mixture of **7e** (252 mg, 0.77 mmol), **Int. B** (210 mg, 0.77 mmol) and TsOH.H₂O (58 mg, 0.31 mmol) in 1,4-dioxane (2 mL) was stirred at 100 °C for 12 h. After cooling to r.t., the mixture was concentrated and the residue was purified by *prep-HPLC* (Method A) to afford **15** (170 mg, 45% yield) as a white solid. LC-MS (ESI, Method 3) t_{R} = 1.06 min, m/z (M+H)⁺ = 500.2.

### Step 2. 6-((1S,2S)-2-fluorocyclopropane-1-carboxamido)-4-((4-methoxy-5-((S*)-2,2,2-trifluoro-1-hydroxyethyl)pyrazolo[1,5-a]pyridin-3-yl)amino)-N-(methyl-d₃)nicotinamide (15A) and 6-((1S,2S)-2-fluorocyclopropane-1-carboxamido)-4-((4-methoxy-5-((R*)-2,2,2-trifluoro-1-hydroxyethyl)pyrazolo[1,5-a]pyridin-3-yl)amino)-N-(methyl-d₃)nicotinamide (15B)

Compound **15** (170 mg, 0.34 mmol) was separated by chiral *prep-HPLC* (Method F) to afford **15A** (66 mg, 17% yield) as a white solid and **15B** (73 mg, 19% yield) as a white solid.

**15A:** ¹H NMR (400 MHz, DMSO-*d*₆) δ 10.71 (s, 1H), 10.37 (s, 1H), 8.55 (s, 1H), 8.52-8.50 (m, 2H), 8.13 (s, 1H), 7.68 (s, 1H), 7.04 (d, *J* = 5.6 Hz, 1H), 6.91 (d, *J* = 7.2 Hz, 1H), 5.42-5.38 (m, 1H), 4.96-4.75 (m, 1H), 3.73 (s, 3H), 2.16-2.12 (m, 1H), 1.58-1.51 (m, 1H), 1.12-1.06 (m, 1H). LC-MS (ESI, Method 2) t_{R} = 2.36 min, m/z (M+H)⁺ = 500.2. HPLC (Method 5) t_{R} = 9.59 min.

**15B:** ¹H NMR (400 MHz, DMSO-*d*₆) δ 10.71 (s, 1H), 10.38 (s, 1H), 8.55 (s, 1H), 8.52-8.50 (m, 2H), 8.12 (s, 1H), 7.69 (s, 1H), 7.01 (d, *J* = 5.6 Hz, 1H), 6.90 (d, *J =* 7.2 Hz, 1H), 5.42-5.38 (m, 1H), 4.96-4.76 (m, 1H), 3.72 (s, 3H), 2.17-2.10 (m, 1H), 1.57-1.49 (m, 1H), 1.13-1.05 (m, 1H). LC-MS (ESI, Method 2) t_{R} = 2.34 min, m/z (M+H)⁺ = 500.2. HPLC (Method 5) t_{R} = 11.56 min.

### Example 16

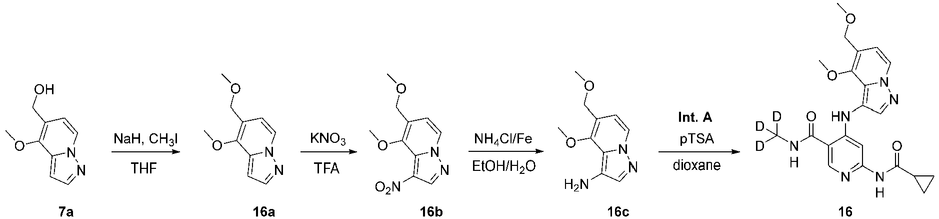

### Step 1. 4-Methoxy-5-(methoxymethyl)pyrazolo[1,5-a]pyridine (16a)

To a solution of **7a** (200 mg, 1.12 mmol) in THF (2 mL) was added NaH (67 mg, 1.68 mmol, 60% purity in mineral oil) at 0 °C, then the mixture was stirred at 0 °C for 30 min. Iodomethane (191 mg, 1.35 mmol) was added into the mixture and stirred at r.t. for 5 h. The mixture was diluted with H₂O (30 mL), extracted with EtOAc (15 mL*3), washed with brine (20 mL), dried over Na₂SO₄ and concnetrated to get compound **16a** (181 mg, 84% yield) as a yellow oil. LC-MS (ESI, Method 4) t_{R} = 2.08 min, m/z (M+H)⁺ = 193.1.

### Step 2. 4-Methoxy-5-(methoxymethyl)-3-nitropyrazolo[1,5-a]pyridine (16b)

To a solution of **16a** (180 mg, 0.94 mmol) in TFA (2 mL) was added KNO₃ (95 mg, 0.94 mmol) at 0 °C, then the mixture was stirred at r.t. for 4 h. The mixture was diluted with H₂O (20 mL), extracted with EtOAc (10 mL*3). The combined organic layer was washed with *aq.* Na₂CO₃ and brine, dried over Na₂SO₄ and concentrated to get compound **16b** (150 mg, 67% yield) as a yellow oil. LC-MS (ESI, Method 4) t_{R} = 2.16 min, m/z (M+H)⁺ = 238.1.

### Step 3. 4-Methoxy-5-(methoxymethyl)pyrazolo[1,5-a]pyridin-3-amine (16c)

To a solution of **16b** (150 mg, 0.63 mmol) in EtOH (3 mL) and H₂O (1 mL) was added Fe powder (177 mg, 3.16 mmol) and NH₄Cl (169 mg, 3.16 mmol). Then the mixture was stirred at 80 °C for 2 h. The mixture was filtered and the filter cake was washed with EtOAc (10 mL). The filtrate was concentrated and diluted with H₂O (10 mL), extracted with EtOAc (10 mL*3), washed with brine (10 mL), dried over Na₂SO₄, concentrated and purified by flash chromatography (DCM/MeOH = 50/1 to 5/1) to get compound **16c** (75 mg, 57% yield) as a yellow oil. LC-MS (ESI, Method 4) t_{R} = 0.44 min, m/z (M+H)⁺ = 208.1.

### Step 4. 6-(Cyclopropanecarboxamido)-4-((4-methoxy-5-(methoxymethyl)pyrazolo[1,5-a]pyridin-3-yl)amino)-N-(methyl-d₃)nicotinamide (16)

A mixture of **16c** (20 mg, 0.96 mmol), **Int. A** (25 mg, 0.096 mmol) and pTSA (17 mg, 0.096 mmol) in dioxane (1 mL) was stirred at 100 °C for 2 h. then the mixture was concentrated and purified by *prep-*HPLC (Method E) to get compound **16** (11.4 mg, 28% yield) as a pale-yellow solid. ¹H NMR (400 MHz, CDCl₃) δ 10.34 (s, 1H), 8.38 (s, 1H), 8.31 (s, 1H), 8.16 (d, *J* = 6.8 Hz, 1H), 8.05 (s, 1H), 7.87 (s, 1H), 6.77 (d, *J =* 7.2 Hz, 1H), 6.39 (s, 1H), 4.51 (s, 2H), 3.80 (s, 3H), 3.39 (s, 3H), 1.52-1.47 (m, 1H), 1.05-1.01 (m, 2H), 0.89-82 (m, 2H). LC-MS (ESI, Method 4) t_{R} = 1.72 min, m/z (M+H)⁺ = 428.4.

### Example 17

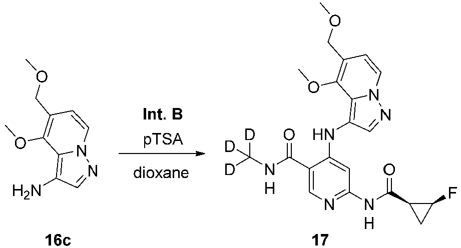

### Step 1. 6-((1S,2S)-2-fluorocyclopropane-1-carboxamido)-4-((4-methoxy-5-(methoxymethyl)pyrazolo[1,5-a]pyridin-3-yl)amino)-N-(methyl-d₃)nicotinamide (17)

A mixture of **16c** (15 mg, 0.72 mmol), **Int. B** (16 mg, 0.058 mmol) and pTSA (12 mg, 0.072 mmol) in dioxane (1 mL) was stirred at 100 °C for 2 h. Then the mixture was concentrated and purified by *prep-HPLC* (Method E) to get compound **17** (10.7 mg, 33% yield) as a pale-yellow solid. ¹H NMR (400 MHz, DMSO-*d*₆) δ 10.68 (s, 1H), 10.33 (s, 1H), 8.51 (s, 1H), 8.46 (s, 1H), 8.39 (d, *J =* 6.8 Hz, 1H), 8.03 (s, 1H), 7.64 (s, 1H), 6.79 (d, *J =* 7.2 Hz, 1H), 4.92-4.72 (m, 1H), 4.41 (s, 2H), 3.67 (s, 3H), 3.26 (s, 3H), 2.11-2.08 (m, 1H), 1.55-1.45 (m, 1H), 1.08-1.03 (m, 1H). LC-MS (ESI, Method 4) t_{R} = 1.68 min, m/z (M+H)⁺ = 446.3.

### Example 18

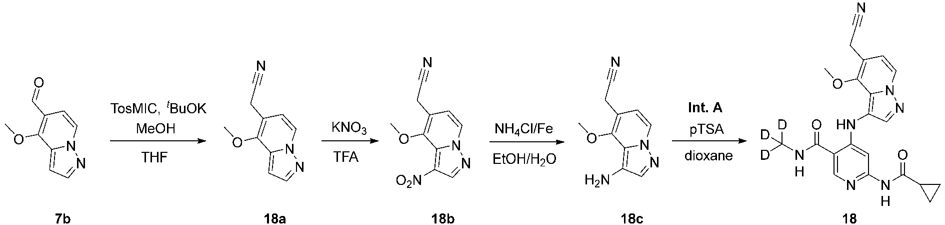

### Step 1. 2-(4-Methoxypyrazolo[1,5-a]pyridin-5-yl)acetonitrile (18a)

To a suspension of t-BuOK (127 mg, 1.14 mmol) in THF (2 mL) was added TosMIC (111 mg, 0.57 mmol) in THF(2 mL) at - 60 °C, then the mixture was stirred at - 60 °C for 15 min. A solution of **7b** (50 mg, 0.28 mmol) in THF(1 mL) was added into the mixture dropwise at - 60 °C and stirred for another 1.5 h at -60 °C. MeOH (5 mL) was added into the mixture and the mixture was stirred at 70 °C for 20 min. The mixture was then concentrated and diluted with H₂O (20 mL), extracted with EtOAc (10 mL*3), wash with brine (15 mL), dried over Na₂SO₄, concentrated and purified by flash chromatography (PE/EA = 10/1 to 1/2) to get compound **18a** (10 mg, 19 % yield) as a yellow solid. LC-MS (ESI, Method 4) t_{R} = 1.82 min, m/z (M+H)⁺ = 188.1.

### Step 2. 2-(4-Methoxy-3-nitropyrazolo[1,5-a]pyridin-5-yl)acetonitrile (18b)

To a solution of **18a** (10 mg, 0.053 mmol) in TFA (1 mL) was added KNO₃ (5 mg, 0.053 mmol) at 0 °C, then the mixture was stirred at r.t. for 4 h. The mixture was diluted with H₂O (20 mL), extracted with EtOAc (10 mL*3). The combined organic layer was washed with *aq.* Na₂CO₃ and brine, dried over Na₂SO₄, concentrated to get compound **18b** (10 mg, 81% yield) as a yellow solid. LC-MS (ESI, Method 4) t_{R} = 1.94 min, m/z (M+H)⁺ = 233.1.

### Step 3. 2-(3-Amino-4-methoxypyrazolo[1,5-a]pyridin-5-yl)acetonitrile (18c)

To a solution of **18b** (10 mg, 0.043 mmol) in EtOH (1 mL) and H₂O (0.2 mL) was added Fe powder (12 mg, 0.22 mmol) and NH₄Cl (12 mg, 0.22 mmol). Then the mixture was stirred at 80 °C for 2 h. The mixture was filtered and the filter cake was washed with EtOAc (10 mL). The filtrate was concentrated and diluted with H₂O (10 mL), extracted with EtOAc (10 mL*3), washed with brine (10 mL), dried over Na₂SO₄, concentrated to get compound **18c** (5 mg, 57% yield) as a yellow solid. LC-MS (ESI, Method 4) t_{R} = 0.45 min, m/z (M+H)⁺ = 203.2.

### Step 4. 4-((5-(Cyanomethyl)-4-methoxypyrazolo[1,5-a]pyridin-3-yl)amino)-6-(cyclopropanecarboxamido)-N-(methyl-d₃)nicotinamide (18)

A solution of **18c** (5 mg, 0.025 mmol), **Int. A** (6 mg, 0.025 mmol) and pTSA (4 mg, 0.025 mmol) in dioxane (0.5 mL) was stirred at 100 °C for 2 h. Then the mixture was concentrated and purified by *prep-HPLC* (Method E) to get compound **18** (2 mg, 19% yield) as a pale-yellow solid. ¹H NMR (400 MHz, DMSO-*d*₆) δ 11.64 (s, 1H), 10.31 (s, 1H), 8.51 (s, 1H), 8.48-8.46 (m, 2H), 8.06 (s, 1H), 7.62 (s, 1H), 6.83 (d, *J =* 7.2 Hz, 1H), 3.98 (s, 2H), 3.70 (s, 3H), 1.93-1.87 (m, 1H), 0.70-0.68 (m, 4H). LC-MS (ESI, Method 4) t_{R} = 1.48 min, m/z (M+H)⁺ = 423.3.

### Example 19

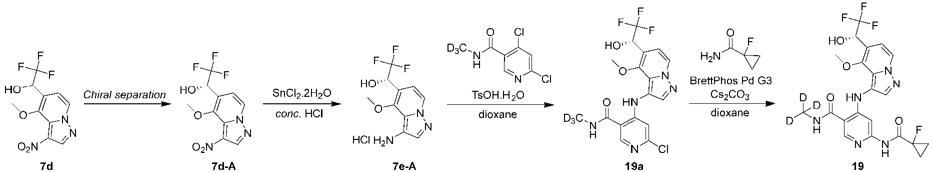

### Step 1. (S)-2,2,2-trifluoro-1-(4-methoxy-3-nitropyrazolo[1,5-a]pyridin-5-yl)ethan-1-ol (7d-A)

**7d** (5.9 g, 20 mmol) was separated by chiral *prep-HPLC* (Method G) to afford **7d-A** (2.6 g, 44% yield) as a green solid.

¹H NMR (400 MHz, CDCl₃) δ 8.68 (s, 1H), 8.42 (d, *J* = 6.8 Hz, 1H), 7.33 (d, *J* = 6.8 Hz, 1H), 5.70-7.67 (m, 1H), 3.92 (s, 3H). HPLC (Method 8) t_{R} = 6.06 min.

### Step 2. (S)-1-(3-amino-4-methoxypyrazolo[1,5-a]pyridin-5-yl)-2,2,2-trifluoroethan-1-ol hydrochloride (7e-A)

To a mixture of **7d-A** (1.1 g, 3.78 mmol) in *conc.* HCl (15 mL) was added SnCl₂·2H₂O (1.7 g, 7.56 mmol) at 0 °C. After stirring at 10 °C for 1 h, the reaction mixture was filtered. The filter cake was dried under vacuum to afford **7e-A** (1 g, 89% yield) as a yellow solid. LC-MS (ESI, Method 3) t_{R} = 0.81 min, m/z (M+H)⁺ = 262.0.

### stii cum o fost asta? Step 3. (S)-6-chloro-4-((4-methoxy-5-(2,2,2-trifluoro-1-hydroxyethyl)pyrazolo[1,5-a]pyridin-3-yl)amino)-N-(methyl-d₃)nicotinamide (19a)

A mixture of **7e-A** (55 mg, 0.21 mmol), 4,6-dichloro-*N*-(methyl-*d*₃)nicotinamide (48 mg, 0.23 mmol) and TsOH.H₂O (16 mg, 0.084 mmol) in 1,4-dioxane (0.3 mL) was stirred at 100 °C for 18 h. After cooling to r.t., the mixture was concentrated. And the residue was purified by flash chromatography on silica gel (DCM/MeOH = 19/1) to afford **19a** (50 mg, 55% yield) as a yellow solid. LC-MS (ESI, Method 3) t_{R} = 1.16 min, m/z (M+H)⁺ = 433.0.

### Step 4. (S)-6-(1-fluorocyclopropane-1-carboxamido)-4-((4-methoxy-5-(2,2,2-trifluoro-1-hydroxyethyl)pyrazolo[1,5-a]pyridin-3-yl)amino)-N-(methyl-d₃)nicotinamide (19)

A mixture of **19a** (50.0 mg, 0.12 mmol), 1-fluorocyclopropane-1-carboxamide (60.0 mg, 0.58 mmol), Cs₂CO₃ (113 mg, 0.35 mmol) and Brettphos Pd G3 (21 mg, 0.023 mmol) in 1,4-dioxane (0.5 mL) was stirred at 90 °C for 12 h under N₂ atmosphere. The reaction mixture was concentrated, and the residue was purified by *prep-*HPLC (Method A) to afford **19** (5.2 mg, 9% yield) as a white solid. ¹H NMR (400 MHz, DMSO-*d*₆) δ 10.37 (s, 1H), 9.97 (s, 1H), 8.63 (s, 1H), 8.61-8.51 (m, 2H), 8.15 (s, 1H), 7.61 (s, 1H), 7.03 (d, *J* = 4.4 Hz, 1H), 6.91 (d, *J =* 6.4 Hz, 1H), 5.43-5.38 (m, 1H), 3.73 (s, 3H), 1.47-1.35 (m, 4H). LC-MS (ESI, Method 2) t_{R} = 2.48 min, m/z (M+H)⁺ = 500.1.

### Example 20

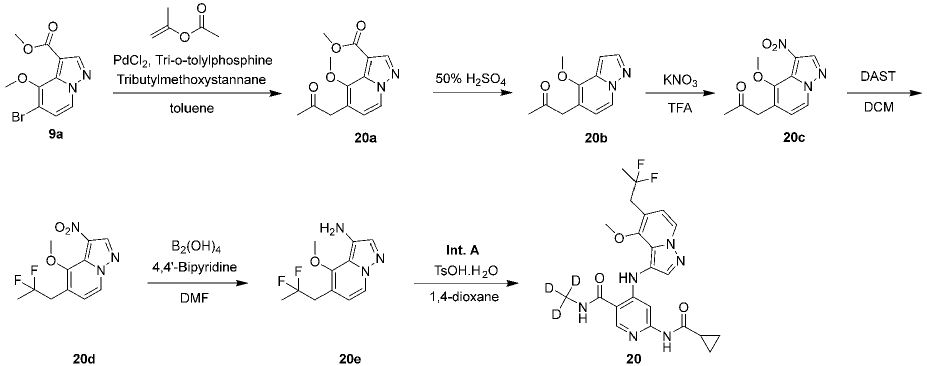

### Step 1. Methyl 4-methoxy-5-(2-oxopropyl)pyrazolo[1,5-a]pyridine-3-carboxylate (20a)

A mixture of **9a** (500 mg, 1.7 mmol), prop-1-en-2-yl acetate (263 mg, 2.6 mmol), tri-o-tolylphosphine (32 mg, 0.105 mmol), tributylmethoxystannane (844 mg, 2.6 mmol) in toluene (5 mL) was stirred at 100 °C for 15 min. To the mixture was added PdCl₂ (9 mg, 0.052 mmol) and stirred at 100 °C for 3 h. The mixture was concentrated. The residue was purified by flash chromatography on silica gel (PE/EA = 3/1) to afford compound **20a** (130 mg, 28% yield) as a yellow solid. ¹H NMR (400 MHz, DMSO-*d*₆) δ 8.60 (d, *J* = 7.2 Hz, 1H), 8.42 (s, 1H), 6.96 (d, *J* = 7.2 Hz, 1H), 3.96 (s, 2H), 3.79 (s, 3H), 3.68 (s, 3H), 2.24 (s, 3H). LC-MS (ESI, Method 3) t_{R} = 1.36 min, m/z (M+H)⁺ =263.1.

### Step 2. 1-(4-Methoxypyrazolo[1,5-a]pyridin-5-yl)propan-2-one (20b)

A mixture of **20a** (270 mg, 1 mmol) in 50% H₂SO₄ (3 mL) was stirred at 85 °C for 4 h. After cooling down, the reaction mixture was diluted with ice-water (5 mL), basified with 1 N NaOH to pH = 3. The mixture was extracted with EtOAc (5 mL*3). The organic layer was washed with brine (5 mL), dried over Na₂SO₄ and filtered. The filtrate was concentrated. The residue was purified by flash chromatography on silica gel (PE/EA = 3/1) to afford **20b** (200 mg, 95% yield) as a yellow oil. LC-MS (ESI, Method 3) t_{R} = 1.29 min, m/z (M+H)⁺ =205.2.

### Step 3. 1-(4-Methoxy-3-nitropyrazolo[1,5-a]pyridin-5-yl)propan-2-one (20c)

To a solution of **20b** (200 mg, 0.98 mmol) in TFA (2 mL) was added KNO₃ (99 mg, 0.98 mmol) at 0 °C. The mixture was stirred at 30 °C for 2 h. The solvent was removed by pumping through N₂. The residue was dissolved in EtOAc (15 mL) and basified with *sat.* Na₂CO₃ to pH = 9. The mixture was extracted with EtOAc (20 mL*2). The combined organic phase was concentrated and the residue was purified by flash chromatography on silica gel (PE/EA= 3/1) to afford **20c** (153 mg, 62% yield) as a yellow solid. LC-MS (ESI, Method 3) t_{R} = 1.34 min, m/z (M+H)⁺ =250.1.

### Step 4. 5-(2,2-Difluoropropyl)-4-methoxy-3-nitropyrazolo[1,5-a]pyridine (20d)

To a solution of **20c** (110 mg, 0.44 mmol) in DCM (2 mL) was added DAST (355 mg, 2 mmol) at 0 °C. Then the mixture was stirred at 25 °C for 10 h. The mixture was diluted with DCM (5 mL), washed with *sat.* NaHCO₃ (5 mL). The organic layer was concentrated and the residue was purified by flash chromatography on silica gel (PE/EA = 3/1) to afford **20d** (84 mg, 70% yield) as a yellow solid. LC-MS (ESI, Method 3) t_{R} = 1.55 min, m/z (M+H)⁺ =272.2.

### Step 5. 5-(2,2-Difluoropropyl)-4-methoxypyrazolo[1,5-a]pyridin-3-amine (20e)

To a solution of **20d** (74 mg, 0.27 mmol), 4,4'-Bipyridine (2 mg, 0.013 mmol) in DMF (1 mL) was added tetrahydroxydiboron (73 mg, 0.82 mmol) at 0 °C. After stirred at 10 °C for 10 min, the mixture was concentrated and the residue was purified by *prep-*HPLC (Method A) to afford **20e** (50 mg, 75% yield) as a yellow solid. LC-MS (ESI, Method 3) t_{R} = 1.42 min, m/z (M+H)⁺ =242.2.

### Step 6. 6-(Cyclopropanecarboxamido)-4-((5-(2,2-difluoropropyl)-4-methoxypyrazolo[1,5-a]pyridin-3-yl)amino)-N-(methyl-d₃)nicotinamide (20)

A mixture of **20e** (40 mg, 0.16 mmol), **Int. A** (46 mg, 0.18 mmol), TsOH.H₂O (15 mg, 0.08 mmol) in 1,4-dioxane (1 mL) was stirred at 90 °C for 7 h. The mixture was concentrated and the residue was purified by *prep-HPLC* (Method A) to afford **20** (63 mg, 82% yield) as a white solid. ¹H NMR (400 MHz, DMSO-*d*₆) δ 10.67 (s, 1H), 10.31 (s, 1H), 8.54 (s, 1H), 8.49 (s, 1H), 8.43 (d, *J* = 7.2 Hz, 1H), 8.05 (s, 1H), 7.65 (s, 1H), 6.76 *(d, J =* 7.2 Hz, 1H), 3.66 (s, 3H), 3.26 (t*, J* = 16.4 Hz, 2H), 1.94-1.91 (m, 1H), 1.61 (t, *J =* 18.8 Hz, 3H), 0.73-0.70 (m, 4H). LC-MS (ESI, Method 2) t_{R} = 2.94 min, m/z (M+H)⁺ = 462.1.

### Example 21

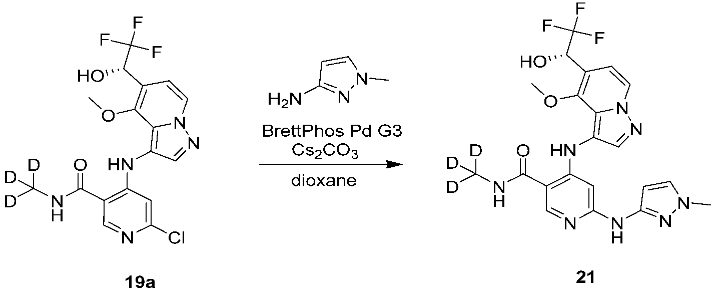

### Step 1. (S)-4-((4-methoxy-5-(2,2,2-trifluoro-1-hydroxyethyl)pyrazolo[1,5-a]pyridin-3-yl)amino)-N-(methyl-d₃)-6-((1-methyl-1H-pyrazol-3-yl)amino)nicotinamide (21)

A mixture of **19a** (44 mg, 0.10 mmol), 1-methyl-1*H*-pyrazol-3-amine (49 mg, 0.51 mmol), Cs₂CO₃ (66 mg, 0.20 mmol) and Brettphos Pd G3 (18 mg, 0.02 mmol) in 1,4-dioxane (0.5 mL) was stirred at 90 °C for 12 h under N₂ atmosphere. The reaction mixture was concentrated and the residue was purified by *prep-*HPLC (Method A) to afford **21** (23 mg, 47% yield) as a white solid. ¹H NMR (400 MHz, DMSO-*d*₆) δ 10.38 (s, 1H), 9.14 (s, 1H), 8.51 (d, *J=* 7.2 Hz, 1H), 8.40 (s, 1H), 8.34 (s, 1H), 8.22 (s, 1H), 7.45 (s, 1H), 7.03-7.01 (m, 2H), 6.89 (d, *J* = 6.8 Hz, 1H), 6.08 (s, 1H), 5.44-5.39 (m, 1H), 3.77 (s, 3H), 3.63 (s, 3H). LC-MS (ESI, Method 2) t_{R} = 0.94 min, m/z (M+H)⁺ = 494.1.

### Example 22

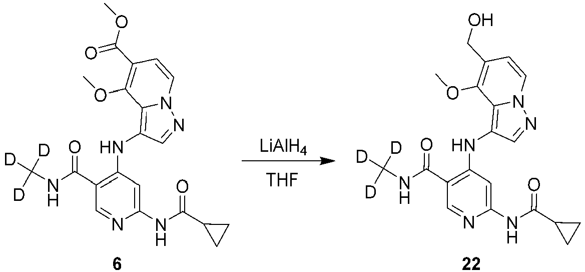

### Step 1. 6-(Cyclopropanecarboxamido)-4-((5-(hydroxymethyl)-4-methoxypyrazolo[1,5-a]pyridin-3-yl)amino)-N-(methyl-d₃)nicotinamide (22)

To a solution of **6** (110 mg, 0.25 mmol) in THF (5 mL) was slowly added LiAlH₄ (51 mg, 1.50 mmol) at 0 °C and the mixture was stirred at 0 °C for 2 h. The mixture was quenched with H₂O (0.05 mL), 15% *aq.* NaOH (0.05 mL) and H₂O (0.1 mL) at 0 °C sequentially. Then the mixture was stirred at r.t. for 15 min, dried over Na₂SO₄ and filtered. The filtrate was concentrated and purified by flash chromatography on silica gel (DCM/MeOH = 8/1) to afford **22** (70 mg, 68% yield) as a yellow solid. ¹H NMR (400 MHz, DMSO-*d*₆) δ 10.64 (s, 1H), 11.31 (s, 1H), 8.50 (s, 1H), 8.48 (s, 1H), 8.42 (d, *J* = 7.2 Hz, 1H), 8.02 (s, 1H), 7.66 (s, 1H), 6.91 (d, *J* = 7.2 Hz, 1H), 5.29-5.26 (m, 1H), 4.53 (d, *J* = 6.0 Hz, 2H), 3.69 (s, 3H), 1.97-1.91 (m, 1H), 0.73-0.72 (m, 4H). LC-MS (ESI, Method 2) t_{R} = 2.73 min, m/z (M+H)⁺ = 414.0.

### Example 23

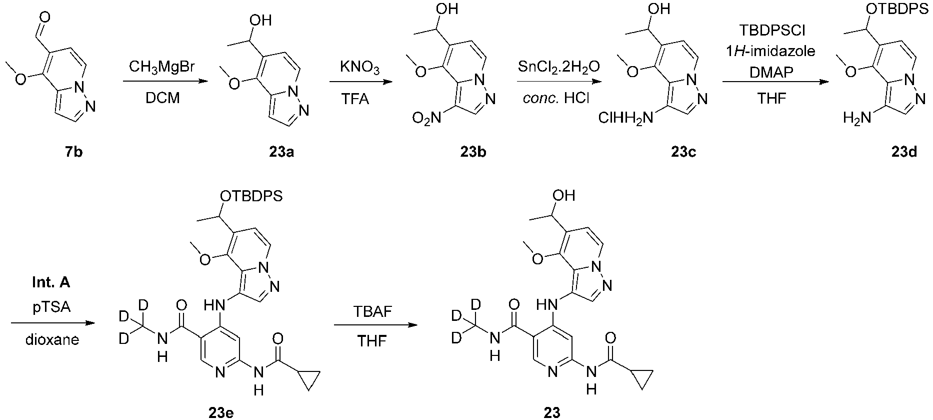

### Step 1. 1-(4-Methoxypyrazolo[1,5-a]pyridin-5-yl)ethan-1-ol (23a)

To a stirred solution of **7b** (755 mg, 4.29 mmol) in anhydrous DCM (10 mL) was added methylmagnesium bromide (2.89 mL, 8.67mmol, 3 M in Et₂O) dropwise at 0 °C. Then the reaction mixture was stirred at 0 °C for 0.5 h. Then it was quenched with *sat.* NH₄Cl (5 mL) and extracted with DCM (10 mL*3). The combined organic layer was washed with water (15 mL), brine (10 mL) and dried over anhydrous Na₂SO₄. After filtration, the filtrate was concentrated under vacuum and the residue was purified by flash chromatography (MeOH/DCM = 3/100) to afford compound **23a** (787 mg, 96% yield) as a light-yellow oil. LC-MS (ESI, Method 4) t_{R} = 1.49 min, m/z (M+H)⁺ = 193.1.

### Step 2. 1-(4-Methoxy-3-nitropyrazolo[1,5-a]pyridin-5-yl)ethan-1-ol (23b)

To a stirred solution of **23a** (740 mg, 3.85 mmol) in TFA (7 mL) was added KNO₃ (385 mg, 3.77 mmol) at 0 °C. Then it was stirred at 25 °C for 0.5 h. The solvent was evaporated under reduced pressure to give a residue, which was purified by flash chromatography (PE/EA = 2/1) to give compound **23b** (830 mg, 91% yield) as a yellow solid. LC-MS (ESI, Method 4) t_{R} = 1.75 min, m/z (M+H)⁺ = 238.0.

### Step 3. 1-(3-Amino-4-methoxypyrazolo[1,5-a]pyridin-5-yl)ethan-1-ol hydrochloride (23c)

To a suspension of **23b** (830 mg, 3.50 mmol) in *conc.* HCl (10 mL) was added SnCl₂.H₂O (3.28 g, 12.83 mmol) at 0 °C. Then it was stirred at 25 °C for 0.5 h. After filtration, the solid was dried under vacuum to give **23c** (802 mg, 94% yield) as a yellow solid. LC-MS (ESI, Method 4) t_{R} = 0.34 min, m/z (M+H)⁺ = 208.2.

### Step 4. 5-(1-((Tert-butyldiphenylsilyl)oxy)ethyl)-4-methoxypyrazolo[1,5-a]pyridin-3-amine (23d)

To a solution of **23c** (710 mg, 2.91 mmol), imidazole (992 mg, 14.57 mmol), DMAP (36 mg, 0.29 mmol) in THF (10 mL) was added *tert*-butylchlorodiphenylsilane (1.04 g, 3.79 mmol). Then the mixture was stirred at 50 °C for 2 h. The mixture was diluted with H₂O (20 mL), extracted with EtOAc (15 mL*3), washed with brine (20 mL), dried over Na₂SO₄, filtered, concentrated and purified by flash chromatography (DCM/MeOH = 100/1 to 10/1) to get compound **23d** (70 mg, 5.4% yield) as a yellow oil. LC-MS (ESI, Method 4) t_{R} = 4.10 min, m/z (M+H)⁺ = 446.3.

### Step 5. 4-((5-(1-((Tert-butyldiphenylsilyl)oxy)ethyl)-4-methoxypyrazolo[1,5-a]pyridin-3-yl)amino)-6-(cyclopropanecarboxamido)-N-(methyl-d₃)nicotinamide (23e)

A mixture of **23d** (60 mg, 0.13 mmol), **Int. A** (21 mg, 0.081 mmol), pTSA (23 mg, 0.13 mmol) in dioxane (1 mL) was stirred at 70 °C for 6 h. The mixture was diluted with H₂O (10 mL), extracted with EtOAc (10 mL*3), washed with brine (20 mL), dried over Na₂SO₄, filtered, concentrated and purified by flash chromatography (DCM/MeOH = 100/1 to 20/1) to get compound **23e** (20 mg, 22% yield) as a yellow oil. LC-MS (ESI, Method 4) t_{R} = 3.87 min, m/z (M+H)⁺ = 666.5.

### Step 6. 6-(Cyclopropanecarboxamido)-4-((5-(1-hydroxyethyl)-4-methoxypyrazolo[1,5-a]pyridin-3-yl)amino)-N-(methyl-d₃)nicotinamide (23)

Compound **23e** (20 mg, 0.03 mmol) was dissolved in TBAF solution (1 M in THF, 0.5 mL), then the mixture was stirred at r.t. for 2 h. The mixture was diluted with H₂O (10 mL), extracted with EtOAc (10 mL*3), washed with *aq.* NH₄Cl (10 mL) and brine (10 mL), dried over Na₂SO₄, concentrated and purified by flash chromatography (DCM/MeOH = 50/1 to 10/1) to get the crude product. Then the crude was purified by *prep-HPLC* (Method E) to get compound **23** (4.0 mg, 35% yield) as a pale-yellow solid. ¹H NMR (400 MHz, DMSO-*d*₆) δ 10.66 (s, 1H), 10.29 (s, 1H), 8.52 (s, 1H), 8.48 (s, 1H), 8.43 (d, *J =* 7.2 Hz, 1H), 8.02 (s, 1H), 7.66 (s, 1H), 6.93 (d*, J =* 7.2 Hz, 1H), 5.26 (d, J = 4.4 Hz, 1H), 5.10-5.04 (m, 1H), 3.68 (s, 3H), 1.96-1.90 (m, 1H), 1.31 (d, *J=* 6.4 Hz, 3H), 0.74-0.71 (m, 4H). LC-MS (ESI, Method 4) t_{R} = 1.00 min, m/z (M+H)⁺ = 428.3.

### Example 24

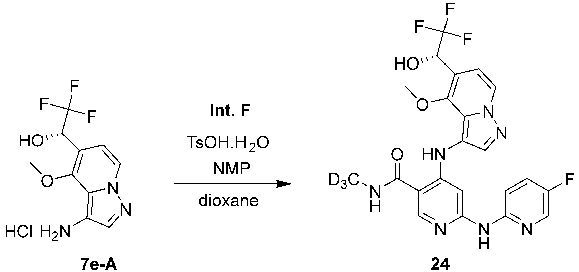

### Step 1. (S)-6-((5-fluoropyridin-2-yl)amino)-4-((4-methoxy-5-(2,2,2-trifluoro-1-hydroxyethyl)pyrazolo[1,5-a]pyridin-3-yl)amino)-N-(methyl-d₃)nicotinamide (24)

A mixture of **7e-A** (35 mg, 0.12 mmol), **Int. F** (33 mg, 0.12 mmol) and TsOH.H₂O (2 mg, 0.012 mmol) in NMP (0.1 mL) and 1,4-dioxane (0.3 mL) was stirred at 100 °C for 12 h. After cooling to r.t., the mixture was concentrated. And the residue was purified by *prep-*HPLC (Method A) to afford **24** (14 mg, 23% yield) as a white solid. ¹H NMR (400 MHz, DMSO-*d*₆) δ 10.34 (s, 1H), 9.64 (s, 1H), 8.52 (d, *J =* 7.2 Hz, 1H), 8.47-8.44 (m, 2H), 8.20 (s, 1H), 8.00 (s, 1H), 7.82-7.79 (m, 1H), 7.62-7.58 (m, 1H), 7.17 (s, 1H), 7.04 (d*, J =* 5.2 Hz, 1H), 6.91 (d*, J* = 7.2 Hz, 1H), 5.43-5.39 (m, 1H), 3.77 (s, 3H). LC-MS (ESI, Method 2) t_{R} = 2.87 min, m/z (M+H)⁺ = 509.0.

### Example 25

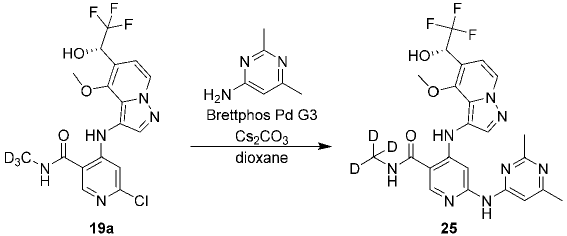

### Step 1. (S)-6-((2,6-Dimethylpyrimidin-4-yl)amino)-4-((4-methoxy-5-(2,2,2-trifluoro-1-hydroxyethyl)pyrazolo[1,5-a]pyridin-3-yl)amino)-N-(methyl-d₃)nicotinamide (25)

To a mixture of **19a** (50 mg, 0.11 mmol) and 2,6-dimethylpyrimidin-4-amine (28 mg, 0.23 mmol) in dioxane (2 mL) was added BrettPhos Pd G3 (41 mg, 0.05 mmol) and Cs₂CO₃ (75 mg, 0.23 mmol). The mixture was stirred at 100 °C for 3 h under N₂ atmosphere. The mixture was concentrated and the residue was purified by *prep-*HPLC (Method A) to afford **25** (7.3 mg, 12% yield) as a yellow solid. ¹H NMR (400 MHz, DMSO-*d*₆) δ 10.36 (s, 1H), 9.90 (s, 1H), 8.54-8.49 (m, 3H), 8.24 (s, 1H), 7.73 (s, 1H), 7.06-7.02 (m, 2H), 6.90 (d, *J =* 6.8 Hz, 1H), 5.42-5.39 (m, 1H), 3.76 (s, 3H), 2.25 (s, 3H), 2.19 (s, 3H). LC-MS (ESI, Method 2) t_{R} = 0.88 min, m/z (M+H)⁺ = 520.2.

### Example 26

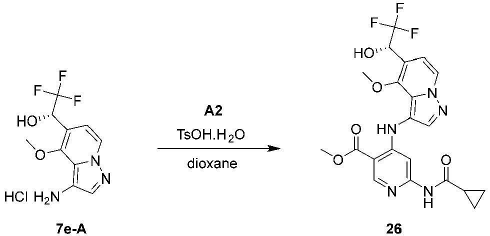

### Step 1. Methyl (5)-6-(cyclopropanecarboxamido)-4-((4-methoxy-5-(2,2,2-trifluoro-1-hydroxyethyl)pyrazolo[1,5-a]pyridin-3-yl)amino)nicotinate (26)

A mixture of **7e-A** (30 mg, 0.10 mmol), **A2** (23 mg, 0.090 mmol) and TsOH.H₂O (7.0 mg, 0.036 mmol) in dioxane (0.5 mL) was stirred at 100 °C for 18 h. The mixture was concentrated and the residue was purified by *prep-HPLC* (Method A) to afford **26** (12.2 mg, 25% yield) as a white solid. ¹H NMR (400 MHz, DMSO-*d*₆) δ 10.82 (s, 1H), δ 9.57 (s, 1H) , 8.67 (s, 1H), 8.54 (d, *J* = 6.8 Hz, 1H), 8.15 (s, 1H), 7.66 (s, 1H), 7.04 (d, *J* = 4.4 Hz, 1H), 6.93 (d, *J* = 6.8 Hz, 1H), 5.46-5.33 (m, 1H), 3.89 (s, 3H), 3.70 (s, 3H), 2.03-1.86 (m, 1H), 0.79-0.65 (m, 4H). LC-MS (ESI, Method 2) t_{R} = 2.64 min, m/z (M+H)⁺ = 480.1.

### Example 27

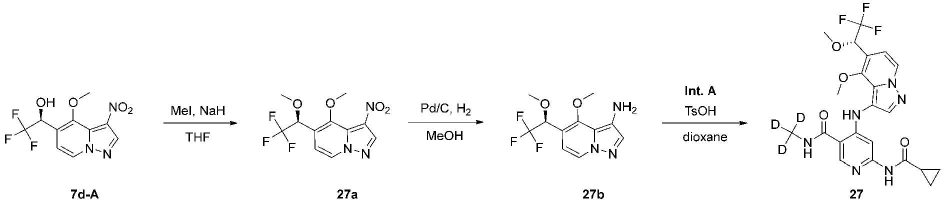

### Step 1. (S)-4-methoxy-3-nitro-5-(2,2,2-trifluoro-1-methoxyethyl)pyrazolo[1,5-a]pyridine (27a)

To a solution of **7d-A** (100 mg, 0.34 mmol) in THF (5 mL) was added NaH (21 mg, 0.52 mmol, 60% in mineral oil) at 0 °C. The mixture was stirred for 20 min, then CH₃I (73 mg, 0.52 mmol, 0.032 mL) was added slowly. The mixture was moved to room temperature and stirred for 4 h. The mixture was quenched with *sat.* NH₄Cl (20mL) and extracted with EtOAc (30 mL*2). The combined organic layer was washed with brine (20 mL*2), dried over anhydrous sodium sulfate and concentrated under vacuum. The residue was purified by flash chromatography (EA in PE is 25%) to give **27a** (52 mg, 49% yield) as a yellow solid. ¹H NMR (400 MHz, CDCl₃) δ 8.69 (s, 1H), 8.42 (d, *J =* 7.2 Hz, 1H), 7.24 (d, *J =* 7.2 Hz, 1H), 5.23 (q, *J=* 6.4 Hz, 1H), 3.92 (s, 3H), 3.45 (s, 3H). LC-MS (ESI, Method 4) t_{R} = 2.94 min, m/z (M+H)⁺ = 306.1.

### Step 2. (S)-4-methoxy-5-(2,2,2-trifluoro-1-methoxyethyl)pyrazolo[1,5-a]pyridin-3-amine (27b)

To a solution of **27a** (52 mg, 0.17 mmol) in MeOH (2 mL) was added Pd/C (5 mg, 0.05 mmol, 10% on charcoal) under H₂ atmosphere, the mixture was stirred at 25 °C for 3 h. The reaction mixture was filtered and the filtrate was concentrated to give **27b** (45 mg, 96% yield) as a brown solid, which was used for the next step directly without further purification. LC-MS (ESI, Method 4) t_{R} = 1.92 min, m/z (M+H)⁺ = 276.1.

### Step 3. (S)-6-(cyclopropanecarboxamido)-4-((4-methoxy-5-(2,2,2-trifluoro-1-methoxyethyl)pyrazolo[1,5-a]pyridin-3-yl)amino)-N-(methyl-d₃)nicotinamide (27)

To a solution of **27b** (45 mg, 0.16 mmol) and **Int. A** (34 mg, 0.13 mmol) in dioxane (2 mL) was added TsOH (31 mg, 0.18 mmol). The mixture was stirred at 85 °C for 3 h. The mixture was concentrated under vacuum and the residue was purified by *prep-HPLC* (Method E) to give 27 (18 mg, 22% yield) as a white solid. ¹H NMR (400 MHz, DMSO-*d*₆) *δ* 10.66 (s, 1H), 10.37 (s, 1H), 8.53 (s, 1H), 8.49 (d, *J=* 7.2 Hz, 1H), 8.47 (s, 1H), 8.11 (s, 1H), 7.68 (s, 1H), 6.73 (d, *J* = 7.2 Hz, 1H), 5.24 *(q, J=* 6.8 Hz, 1H), 3.69 (s, 3H), 3.30 (s, 3H), 1.97-1.82 (m, 1H), 0.73-0.61 (m, 4H). LC-MS (ESI, Method 4) t_{R} = 2.29 min, m/z (M+H)⁺ = 496.4.

### Example 28

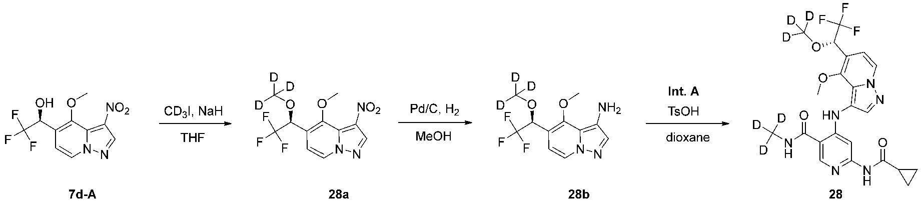

### Step 1. (S)-4-methoxy-3-nitro-5-(2,2,2-trifluoro-1-(methoxy-d₃)ethyl)pyrazolo[1,5-a]pyridine (28a)

To a solution of **7d-A** (100 mg, 0.34 mmol) in THF (5 mL) was added NaH (21 mg, 0.52 mmol, 60% in mineral oil) at 0 °C. The mixture was stirred for 20 min, then CD₃I (75 mg, 0.52 mmol, 0.032 mL) was added slowly. The mixture was moved to room temperature and stirred for 4 h. The mixture was quenched with *sat.* NH₄Cl (20mL) and extracted with EtOAc (30 mL*2). The combined organic layer was washed with brine (20 mL*2), dried over anhydrous sodium sulfate and concentrated under vacuum. The residue was purified by flash chromatography (EA in PE is 25%) to give **28a** (55 mg, 52% yield) as a yellow solid. LC-MS (ESI, Method 4) t_{R} = 2.91 min, m/z (M+H)⁺ = 309.1.

### Step 2. (S)-4-methoxy-5-(2,2,2-trifluoro-1-(methoxy-d₃)ethyl)pyrazolo[1,5-a]pyridin-3-amine (28b)

To a solution of **28a** (55 mg, 0.17 mmol) in MeOH (2 mL) was added Pd/C (5 mg, 0.05 mmol, 10% on charcoal) under H₂ atmosphere. The mixture was stirred at 25 °C for 3 h. The reaction mixture was filtered and the filtrate was concentrated to give **28b** (45mg, 91% yield) as a brown solid, which was used for the next step directly without further purification. LC-MS (ESI, Method 4) t_{R} = 1.89 min, m/z (M+H)⁺ = 279.1.

### Step 3. (S)-6-(cyclopropanecarboxamido)-4-((4-methoxy-5-(2,2,2-trifluoro-1-(methoxy-d₃)ethyl)pyrazolo[1,5-a]pyridin-3-yl)amino)-N-(methyl-d₃)nicotinamide (28)

To a solution of **28b** (45 mg, 0.16 mmol) and **Int. A** (34 mg, 0.13 mmol) in dioxane (2 mL) was added TsOH (31 mg, 0.18 mmol), the mixture was stirred at 85 °C for 3 h. The mixture was concentrated under vacuum and the residue was purified by *prep-HPLC* (Method E) to give **28** (23 mg, 28% yield) as a white solid. ¹H NMR (400 MHz, DMSO-*d*₆) *δ* 10.66 (s, 1H), 10.37 (s, 1H), 8.53 (s, 1H), 8.49 (d, *J* = 7.2 Hz, 1H), 8.47 (s, 1H), 8.11 (s, 1H), 7.68 (s, 1H), 6.73 (d, *J* = 7.2 Hz, 1H), 5.24 (q*, J =* 6.8 Hz, 1H), 3.69 (s, 3H), 1.95-1.85 (m, 1H), 0.74-0.61 (m, 4H). LC-MS (ESI, Method 4) t_{R} = 2.27 min, m/z (M+H)⁺ = 499.4.

### Example 29

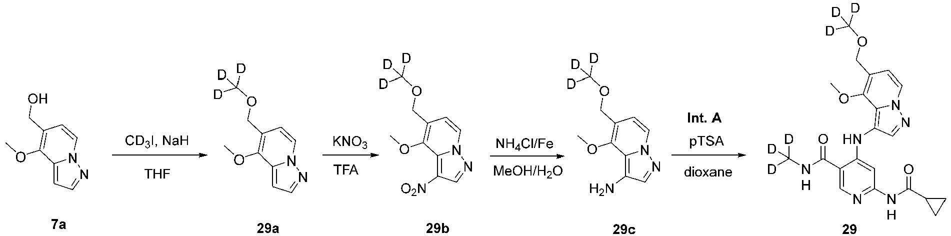

### Step 1. 4-Methoxy-5-((methoxy-d₃)methyl)pyrazolo[1,5-a]pyridine (29a)

To a solution of **7a** (80 mg, 0.45 mmol) in THF (1 mL) was added NaH (23 mg, 0.58 mmol, 60% purity in mineral oil) at 0 °C, then the mixture was stirred at 0 °C for 30 min. Iodomethane-*d*₃ (78 mg, 0.54 mmol) was added into the mixture, then the mixture was stirred at r.t. for 1 h. The mixture was diluted with H₂O (30 mL), extracted with EtOAc (15 mL*3), washed with brine (20 mL), dried over Na₂SO₄ and concentrated to get compound **29a** (80 mg, 91% yield) as a yellow oil. LC-MS (ESI, Method 4) t_{R} = 2.05 min, m/z (M+H)⁺ = 196.1.

### Step 2. 4-Methoxy-5-((methoxy-d₃)methyl)-3-nitropyrazolo[1,5-a]pyridine (29b)

To a solution of **29a** (80 mg, 0.41 mmol) in TFA (1 mL) was added KNO₃ (41 mg, 0.41 mmol) at 0 °C, then the mixture was stirred at r.t. for 4 h. The mixture was diluted with H₂O (20 mL), extracted with EtOAc (10 mL*3). The combined organic layer was washed with *aq.* Na₂CO₃ and *sat.* brine, dried over Na₂SO₄ and concentrated to get compound **29b** (90 mg, 91% yield) as a yellow oil. LC-MS (ESI, Method 4) t_{R} = 2.16 min, m/z (M+H)⁺ = 241.1.

### Step 3. 4-Methoxy-5-((methoxy-d₃)methyl)pyrazolo[1,5-a]pyridin-3-amine (29c)

To a solution of **29b** (90 mg, 0.37 mmol) in MeOH (2 mL) and H₂O (0.5 mL) was added Fe powder (105 mg, 1.87 mmol) and NH₄Cl (100 mg, 1.87 mmol). Then the mixture was stirred at 70 °C for 2 h. The mixture was filtered and washed with EtOAc (10 mL). The filtrate was concentrated and diluted with H₂O (10 mL), extracted with EtOAc (10 mL*3), washed with brine (10 mL), dried over Na₂SO₄, concentrated and purified by flash chromatography (DCM/MeOH = 50/1 to 5/1) to get compound **29c** (70 mg, 89% yield) as a yellow oil. LC-MS (ESI, Method 4) t_{R} = 0.45 min, m/z (M+H)⁺ = 211.3.

### Step 4. 6-(Cyclopropanecarboxamido)-4-((4-methoxy-5-((methoxy-d₃)methyl)pyrazolo[1,5-a]pyridin-3-yl)amino)-N-(methyl-d₃)nicotinamide (29)

A mixture of **29c** (33 mg, 0.16 mmol), **Int. A** (40 mg, 0.16 mmol) and pTSA (27 mg, 0.16 mmol) in dioxane (1 mL) was stirred at 100 °C for 2 h. then the mixture was concentrated and purified by *prep-HPLC* (Method E) to get compound **29** (18.4 mg, 27% yield) as a pale-yellow solid. ¹H NMR (400 MHz, DMSO-*d*₆) δ 10.67 (s, 1H), 10.34 (s, 1H), 8.52 (s, 1H), 8.48 (s, 1H), 8.42 (d, *J* = 7.2 Hz, 1H), 8.05 (s, 1H), 7.68 (s, 1H), 6.82 (d, *J =* 7.2 Hz, 1H), 4.44 (s, 2H), 3.70 (s, 3H), 1.96-1.90 (m, 1H), 0.74-0.71 (m, 4H). LC-MS (ESI, Method 4) t_{R} = 1.68 min, m/z (M+H)⁺ = 431.4.

### Example 30

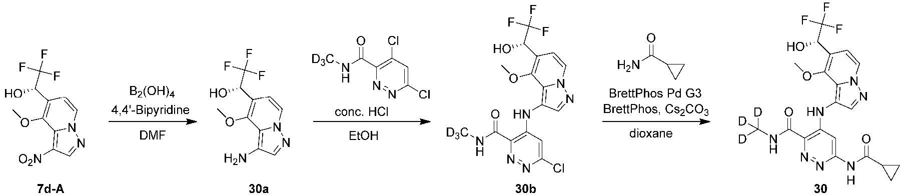

### Step 1. (S)-1-(3-amino-4-methoxypyrazolo[1,5-a]pyridin-5-yl)-2,2,2-trifluoroethan-1-ol (30a)

To a solution of **7d-A** (100 mg, 0.34 mmol), 4,4'-Bipyridine (2 mg, 0.013 mmol) in DMF (1 mL) was added tetrahydroxydiboron (92 mg, 1.032 mmol) at 0 °C. After stirred at 10 °C for 10 min, the mixture was concentrated and the residue was purified by *prep-HPLC* (Method A) to afford **30a** (45 mg, 50% yield) as a green solid. ¹H NMR (400 MHz, DMSO-*d*₆) δ 8.16 (d, *J =* 7.2 Hz, 1H), 7.49 (s, 1H), 6.83 (d, *J =* 6.0 Hz, 1H), 6.58 (d, *J =* 7.6 Hz, 1H), 5.36-5.29 (m, 1H), 4.31 (s, 2H), 3.87 (3, 3H).

### Step 2. (S)-6-chloro-4-((4-methoxy-5-(2,2,2-trifluoro-1-hydroxyethyl)pyrazolo[1,5-a]pyridin-3-yl)amino)-N-(methyl-d₃)pyridazine-3-carboxamide (30b)

To a solution of **30a** (45 mg, 0.17 mmol) and 4,6-dichloro-*N*-(methyl-*d*₃)pyridazine-3-carboxamide (47 mg, 0.22 mmol) in EtOH (0.5 mL) was added *conc.* HCl (7 mg, 0.17 mmol) and stirred at 80 °C for 12 h. The reaction mixture was purified by *prep-HPLC* (Method A) to afford **30b** (60 mg, 80% yield) as a yellow solid. LC-MS (ESI, Method 3) t_{R} = 1.14 min, m/z (M+H)⁺ = 434.1.

### Step 3. (S)-6-(cyclopropanecarboxamido)-4-((4-methoxy-5-(2,2,2-trifluoro-1-hydroxyethyl)pyrazolo[1,5-a]pyridin-3-yl)amino)-N-(methyl-d₃)pyridazine-3-carboxamide (30)

A mixture of **30b** (60 mg, 0.14 mmol), cyclopropanecarboxamide (35 mg, 0.41 mmol), BrettPhos (15 mg, 0.03 mmol) and BrettPhos Pd G3 (25.08 mg, 0.03 mmol) in dioxane (0.5 mL) was stirred at 100 °C under N₂ for 12 h. The reaction mixture was concentrated to dryness and purified by flash chromatography on silica gel (DCM/MeOH = 10/1) to give compound **30** (15 mg, 22% yield) as a yellow solid. ¹H NMR (400 MHz, DMSO-*d*₆) δ 11.24 (s, 1H), 10.55 (s, 1H), 9.09 (s, 1H), 8.54 (d, *J* = 7.2 Hz, 1H), 8.12 (s, 1H), 7.79 (s, 1H), 7.04 (d, *J =* 5.6Hz, 1H), 6.93 (d, *J =* 7.2 Hz, 1H), 5.40-5.37 (m, 1H), 3.72 (s, 3H), 2.01-1.99 (m, 1H), 0.79-0.75 (m, 4H). LC-MS (ESI, Method 2) t_{R} = 0.98 min, m/z (M+H)⁺ = 483.1.

### Example 31

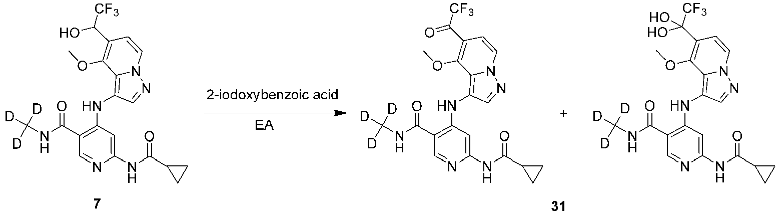

### 6-(Cyclopropanecarboxamido)-4-((4-methoxy-5-(2,2,2-trifluoroacetyl)pyrazolo[1,5-a]pyridin-3-yl)amino)-N-(methyl-d₃)nicotinamide and 6-(Cyclopropanecarboxamido)-4-((4-methoxy-5-(2,2,2-trifluoro-1,1-dihydroxyethyl)pyrazolo[1,5-a]pyridin-3-yl)amino)-N-(methyl-d₃)nicotinamide (31)

To a solution of **7** (50 mg, 0.10 mmol) in EtOAc (0.5 mL) was added 2-iodoxybenzoic acid (116 mg, 0.40 mmol). After stirring at 95 °C for 12 h, the reaction mixture was concentrated to dryness and purified by flash chromatography (DCM/MeOH = 10/1) to afford **31** as a brown solid as a mixture. ¹H NMR (400 MHz, DMSO-*d*₆) δ 10.81 (s, 0.2H), 10.79 (s, 0.2H), 10.69 (s, 0.8H), 10.37 (s, 0.8H), 8.62 (s, 0.2H), 8.56 (s, 0.2H), 8.55 (s, 0.8H), 8.54 (d, *J =* 8.0 Hz, 0.2H), 8.53 (s, 0.8H), 8.42 (d, *J =* 7.6 Hz, 0.8H), 8.28 (s, 0.2H), 8.10 (s, 0.8H), 7.93 (s, 0.2H), 7.75 (s, 0.8H), 7.71 (s, 1.6H), 7.04 (d, *J =* 7.2 Hz, 0.2H), 6.98 (d, *J =* 7.6 Hz, 0.8H), 3.90 (s, 0.6H), 3.70 (s, 2.4H), 1.97-1.93 (m, 1H), 0.77-0.73 (m, 4H). LC-MS (ESI, Method 2) t_{R} = 1.99 min, m/z (M+H)⁺ = 480.2, 498.2.

### Example 32

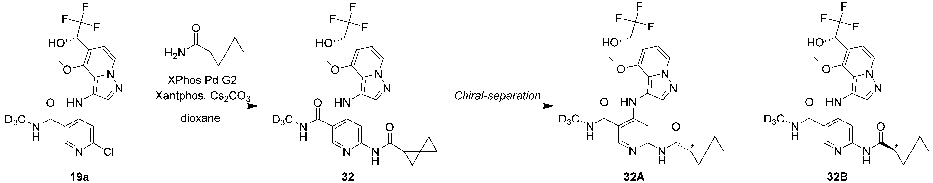

### Step 1. 4-((4-Methoxy-5-((S)-2,2,2-trifluoro-1-hydroxyethyl)pyrazolo[1,5-a]pyridin-3-yl)amino)-N-(methyl-d₃)-6-(spiro[2.2]pentane-1-carboxamido)nicotinamide (32)

A mixture of **19a** (30.0 mg, 0.07 mmol) and spiro[2.2]pentane-1-carboxamide (39.0 mg, 0.35 mmol), Xantphos (8 mg, 0.014 mmol), XPhos Pd G2 (11 mg, 0.014 mmol) and Cs₂CO₃ (45 mg, 0.14 mmol) in 1,4-dioxane (0.4 mL) was stirred at 100 °C for 12 h under N₂ atmosphere. The reaction mixture was concentrated and the residue was purified by *prep-HPLC* (Method A) to afford **32** (7 mg, 20% yield) as a white solid. ¹H NMR (400 MHz, DMSO-*d*₆) δ 10.49 (s, 1H), 10.40 (d, *J =* 6.4 Hz, 1H), 8.56-8.49 (m, 3H), 8.14 (s, 1H), 7.74-7.71 (m, 1H), 7.04 (d, *J* = 5.6 Hz, 1H), 6.90 (d, *J =* 7.2 Hz, 1H), 5.42-5.39 (m, 1H), 3.72 (s, 3H), 2.31-2.28 (m, 1H), 1.35-1.30 (m, 1H), 1.26-1.23 (m, 1H), 0.85-0.79 (m, 3H), 0.68-0.67 (m, 1H). LC-MS (ESI, Method 2) t_{R} = 1.11 min, m/z (M+H)⁺ = 508.0.

### Step 2. 4-((4-Methoxy-5-((S)-2,2,2-trifluoro-1-hydroxyethyl)pyrazolo[1,5-a]pyridin-3-yl)amino)-N-(methyl-d₃)-6-((S*)-spiro[2.2]pentane-1-carboxamido)nicotinamide (32A) and 4-((4-Methoxy-5-((S)-2,2,2-trifluoro-1-hydroxyethyl)pyrazolo[1,5-a]pyridin-3-yl)amino)-N-(methyl-d₃)-6-((R*)-spiro[2.2]pentane-1-carboxamido)nicotinamide (32B)

Compound **32** (23 mg, 0.05 mmol) was separated by chiral *prep-HPLC* (Method H) to obtain **32A** (5 mg, 22% yield) as a white solid and **32B** (6.5 mg, 28% yield) as a white solid. **32A:** ¹H NMR (400 MHz, DMSO-*d*₆) δ 10.47 (s, 1H), 10.40 (s, 1H), 8.54-8.49 (m, 3H), 8.14 (s, 1H), 7.74 (s, 1H), 7.04 (d, *J* = 4.4 Hz, 1H), 6.91 (d, *J* = 6.8 Hz, 1H), 5.42-5.38 (m, 1H), 3.73 (s, 3H), 2.31-2.28 (m, 1H), 1.30-1.26 (m, 2H), 0.83-0.82 (m, 3H), 0.68-0.65 (m, 1H). LC-MS (ESI, Method 2) t_{R} = 1.11 min, m/z (M+H)⁺ = 508.0. HPLC (Method 7) t_{R} = 8.14 min.

**32B:** ¹H NMR (400 MHz, DMSO-*d*₆) δ 10.47 (s, 1H), 10.38 (s, 1H), 8.56-8.49 (m, 3H), 8.14 (s, 1H), 7.71 (s, 1H), 7.04 (d, *J* = 5.2 Hz, 1H), 6.91 (d, *J =* 7.2 Hz, 1H), 5.42-5.39 (m, 1H), 3.73 (s, 3H), 2.31-2.28 (m, 1H), 1.30-1.25 (m, 2H), 0.84-0.81 (m, 3H), 0.69-0.66 (m, 1H). LC-MS (ESI, Method 2) t_{R} = 1.11 min, m/z (M+H)⁺ = 508.0. HPLC (Method 7) t_{R} = 10.48 min.

### Example 33

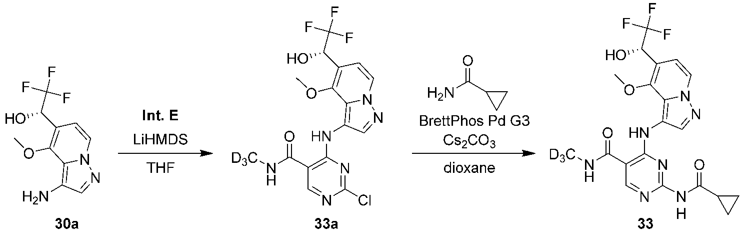

### Step 1. (S)-2-chloro-4-((4-methoxy-5-(2,2,2-trifluoro-1-hydroxyethyl)pyrazolo[1,5-a]pyridin-3-yl)amino)-N-(methyl-d₃)pyrimidine-5-carboxamide (33a)

To a solution of **30a** (80 mg, 0.31 mmol) and **Int. E** (77 mg, 0.37 mmol) in THF (0.8 mL) was added LiHMDS (1.2 mL, 1.2 mmol, 1 M in THF) at -40 °C. The reaction was stirred at -40 °C to r.t. for 1 h and quenched with H₂O (2 mL). The organic solvent was removed under reduced pressure. The formed solid was collected by filtering and purified by flash chromatography (DCM/MeOH = 20/1) to afford **33a** (32 mg, 24% yield) as a yellow solid. LC-MS (ESI, Method 3) t_{R} = 1.18 min, m/z (M+H)⁺ = 434.2.

### Step 2. (S)-2-(cyclopropanecarboxamido)-4-((4-methoxy-5-(2,2,2-trifluoro-1-hydroxyethyl)pyrazolo[1,5-a]pyridin-3-yl)amino)-N-(methyl-d₃)pyrimidine-5-carboxamide (33)

A mixture of **33a** (60 mg, 0.07 mmol), cyclopropanecarboxamide (31 mg, 0.37 mmol), BrettPhos Pd G3 (13 mg, 0.015 mmol) and Cs₂CO₃ (48 mg, 0.15 mmol) in dioxane (0.4 mL) was stirred at 90 °C for 12 h under N₂ atmosphere. The reaction mixture was concentrated and the residue was purified by *prep-HPLC* (Method A) to afford **33** (8 mg, 22% yield) as a white solid. ¹H NMR (400 MHz, DMSO-*d*₆) δ 11.73 (s, 1H), 10.96 (s, 1H), 9.61 (s, 1H), 8.74 (s, 1H), 8.63 (s, 1H), 8.43 (d, *J =* 7.2 Hz, 1H), 7.04 (d, *J =* 5.6 Hz, 1H), 6.84 (d, *J* = 7.2 Hz, 1H), 5.47-5.44 (m, 1H), 3.95 (s, 3H), 2.14-2.11 (m, 1H), 0.93-0.86 (m, 4H). LC-MS (ESI, Method 2) t_{R} = 1.11 min, m/z (M+H)⁺ = 483.0.

### Example 34

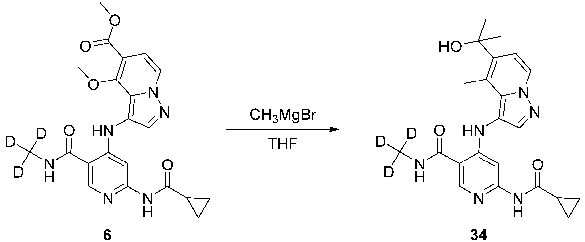

### Step 1. 6-(Cyclopropanecarboxamido)-4-((5-(2-hydroxypropan-2-yl)-4-methylpyrazolo[1,5-a]pyridin-3-yl)amino)-N-(methyl-d₃)nicotinamide (34)

To a solution of **6** (5 mg, 0.01 mmol) in THF (1 mL) was added CH₃MgBr (0.041 mL, 0.12 mmol, 3 M in Et₂O) slowly at 0 °C under N₂ atmosphere. The mixture was stirred for 10 min, then heated to 50 °C and stirred for 12 h. The mixture was quenched with *sat.* NH₄Cl (20mL) and extracted with DCM (20 mL*2). The combined organic layer was washed with brine (20 mL*2), dried and condensed by vacuum. The residue was purified by *prep-*HPLC (Method A) to give **34** (1.8 mg, 37% yield) as a white solid. ¹H NMR (400 MHz, DMSO-*d*₆) *δ* 10.58 (s, 1H), 9.96 (s, 1H), 8.48 (s, 1H), 8.44 (s, 1H), 8.37 (d, *J =* 7.2 Hz, 1H), 7.87 (s, 1H), 7.24 (s, 1H), 6.99 (d, *J =* 7.6 Hz, 1H), 5.14 (s, 1H), 2.55 (s, 3H), 1.91-1.82 (m, 1H), 1.47 (s, 6H), 0.69-0.59 (m, 4H). LC-MS (ESI, Method 4) t_{R} = 1.46 min, m/z (M+H)⁺ = 426.3.

### Example 35

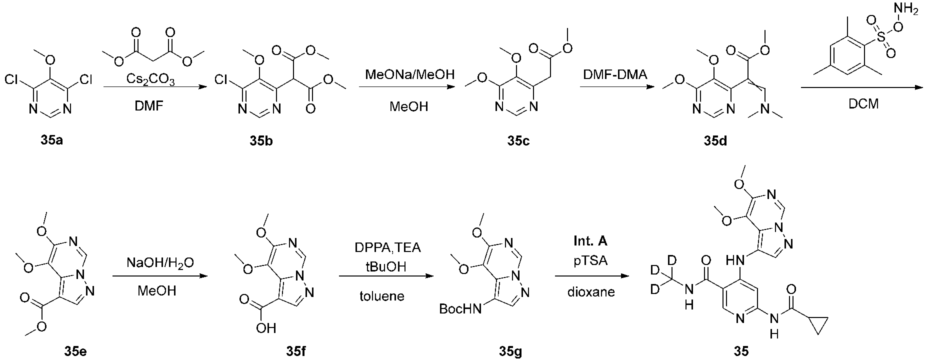

### Step 1. Dimethyl 2-(6-chloro-5-methoxypyrimidin-4-yl)malonate (35b)

To a solution of dimethyl malonate (7.38 g, 55.86 mmol, 6.36 mL) in DMF (100 mL) was added Cs₂CO₃ (36.40 g, 111.73 mmol) and 4,6-dichloro-5-methoxy-pyrimidine (10 g, 55.86 mmol), then the mixture was stirred at 100 °C for 2 h. The mixture was concentrated in vacuo, diluted with H₂O (100 mL), adjusted pH to 2 with 2 N HCl, extracted with EtOAc (100 mL*3), washed with brine (100 mL*2), dried over Na₂SO₄, concentrated to get the crude compound **35b** (15 g, 98% yield) as a yellow solid. LC-MS (ESI, Method 4) t_{R} = 2.23 min, m/z (M+H)⁺ = 275.1.

### Step 2. Methyl 2-(5,6-dimethoxypyrimidin-4-yl)acetate (35c)

To a solution of **35b** (12 g, 43.69 mmol) in MeOH (50 mL) was added NaOMe (16.2 mL, 87.38 mmol, 5.4 M in MeOH), then the mixture was stirred at 60 °C for 2 h. The mixture was concentrated and diluted with H₂O (100 mL), adjusted pH to 2 with 2 N HCl, extracted with EtOAc (100 mL*3), washed with brine (100 mL), dried over Na₂SO₄, concentrated and purified by flash chromatography (PE/EA = 20/1 to 1/1) to get compound **35c** (9 g, 97% yield) as a white solid. LC-MS (ESI, Method 4) t_{R} = 1.79 min, m/z (M+H)⁺ = 213.1.

### Step 3. Methyl 2-(5,6-dimethoxypyrimidin-4-yl)-3-(dimethylamino)acrylate (35d)

A solution of **35c** (1 g, 4.71 mmol) in DMF-DMA (1 mL) was stirred at 120 °C for 6 h. The mixture was concentrated to get compound **35d** (1.26 g, crude) as a yellow oil. LC-MS (ESI, Method 4) t_{R} = 1.23 min, m/z (M+H)⁺ = 268.1.

### Step 4. Methyl 4,5-dimethoxypyrazolo[1,5-c]pyrimidine-3-carboxylate (35e)

To a solution of **35d** (1.26 g, 4.71 mmol) in DCM (10 mL) was added *O-*(mesitylsulfonyl)hydroxylamine (2.90 g, 9.43 mmol) at 0 °C, then the mixture was stirred at r.t. for 2 h. Then the mixture was concentrated and the solid was triturated with H₂O (10 mL), filtered, and the filter cake was washed with DCM (2 mL*2). The solid was dried in vacuo to get compound **35e** (600 mg, 54% yield) as a white solid. ¹H NMR (400 MHz, DMSO-*d*₆) δ 9.42 (s, 1H), 8.45 (s, 1H), 3.99 (s, 3H), 3.78 (s, 3H), 3.75 (s, 3H). LC-MS (ESI, Method 4) t_{R} = 2.04 min, m/z (M+H)⁺ = 238.1.

### Step 5. 4,5-Dimethoxypyrazolo[1,5-c]pyrimidine-3-carboxylic acid (35f)

To a suspension of **35e** (550 mg, 2.32 mmol) in MeOH (11 mL) was added a solution of NaOH (4.64 mL, 4.64 mmol, 1 M in H₂O) slowly, then the mixture was stirred at 40 °C for 16 h. The mixture was concentrated and then treated with H₂O (5 mL), adjusted pH to 1 with 2 N HCl. Then the mixture was filtered and the filter cake was washed with Et₂O (2 mL). The solid was dried in vacuo to get compound **35f** (90 mg, 17% yield) as a white solid. LC-MS (ESI, Method 4) t_{R} = 1.20 min, m/z (M+H)⁺ = 224.1.

### Step 6. Tert-butyl (4,5-dimethoxypyrazolo[1,5-c]pyrimidin-3-yl)carbamate (35g)

To a solution of 35f (30 mg, 0.134 mmol) in toluene (1 mL) was added DPPA (74 mg, 0.27 mmol) and TEA (54 mg, 0.54 mmol), then the mixture was stirred at 80 °C for 1 h and cooled to r.t., then *^{t}*BuOH (0.5 mL) was added into the mixture. The mixture was stirred at 110 °C for 4 h, concentrated and purified by flash chromatography (DCM/MeOH = 50/1 to 15/1) to get compound **35g** (25 mg, 63% yield) as a yellow solid. LC-MS (ESI, Method 4) t_{R} = 2.64 min, m/z (M+H)⁺ = 295.2.

### Step 7. 6-(Cyclopropanecarboxamido)-4-((4,5-dimethoxypyrazolo[1,5-c]pyrimidin-3-yl)amino)-N-(methyl-d₃)nicotinamide (35)

A mixture of **35g** (25 mg, 0.085 mmol), **Int. A** (22 mg, 0.085 mmol) and pTSA (15 mg, 0.085 mmol) in dioxane (1 mL) was stirred at 100 °C for 2 h. Then the mixture was concentrated and purified by *prep-*HPLC (Method E) to get compound **35** (10.7 mg, 30% yield) as a pale-yellow solid. ¹H NMR (400 MHz, DMSO-*d*₆) δ 10.63 (s, 1H), 10.13(s, 1H), 9.22 (s, 1H), 8.48 (s, 1H), 8.44 (s, 1H), 8.13 (s, 1H), 7.49 (s, 1H), 3.92 (s, 3H), 3.67 (s, 3H), 1.92-1.86 (m, 1H), 0.70-0.67 (m, 4H). LC-MS (ESI, Method 4) t_{R} = 1.71 min, m/z (M+H)⁺ = 415.3.

### Example 36

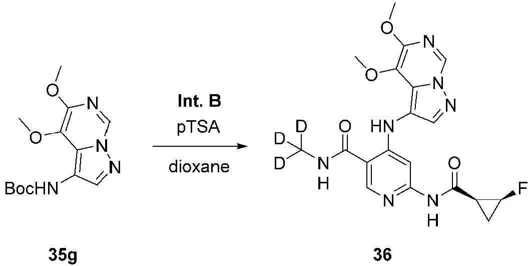

### Step 1. 4-((4,5-Dimethoxypyrazolo[1,5-c]pyrimidin-3-yl)amino)-6-((1S,2S)-2-fluorocyclopropane-1-carboxamido)-N-(methyl-d₃)nicotinamide (36)

A mixture of **35g** (18 mg, 0.061 mmol), **Int.** B (17 mg, 0.061 mmol) and pTSA (11 mg, 0.061 mmol) in dioxane (1 mL) was stirred at 100 °C for 2 h. Then the mixture was concentrated and purified by *prep-*HPLC (Method E) to get compound **36** (6.5 mg, 24% yield) as a pale-yellow solid. ¹H NMR (400 MHz, CDCl₃) δ 10.00 (s, 1H), 8.79 (s, 1H), 8.25 (s, 1H), 8.23 (s, 1H), 8.04 (s, 1H), 7.64 (s, 1H), 6.22 (s, 1H), 4.86-4.65 (m, 1H), 4.02 (s, 3H), 3.84 (s, 3H), 1.85-1.82 (m, 1H), 1.64-1.60 (m, 1H), 1.21-1.13 (m, 1H). LC-MS (ESI, Method 4) t_{R} = 1.65 min, m/z (M+H)⁺ = 433.3.

### Example 37

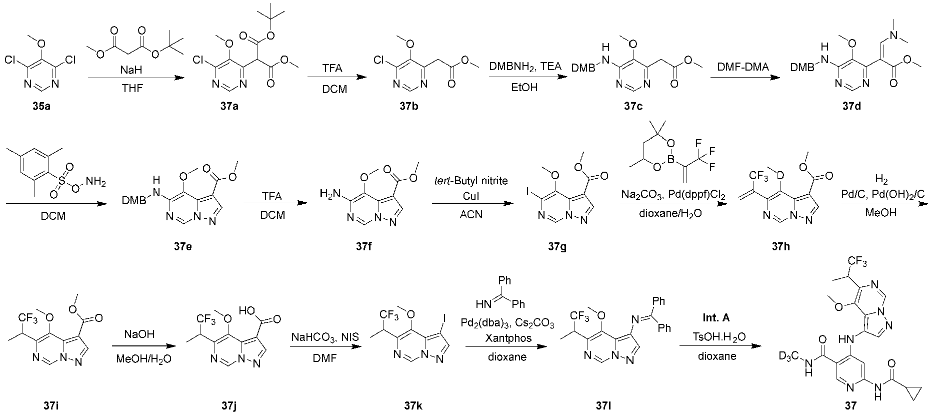

### Step 1. 1-Tert-butyl 3-methyl 2-(6-chloro-5-methoxypyrimidin-4-yl)malonate (37a)

To a solution of *tert-*butyl methyl malonate (13.08 g, 75.08 mmol) in THF (200 mL) was added NaH (6.01 g, 150.16 mmol, 60% in mineral oil) at 0 °C. The mixture was stirred at 0 °C for 30 min. Then a solution of **35a** (11.2 g, 62.57 mmol) in THF (20 mL) was added to the mixture at 0 °C. The mixture was stirred at 80 °C for 3 h and poured into ice-water (150 mL). The mixture was acidified with 2 N HCl to pH = 2 and extracted with EtOAc (300 mL*2). The combined organic layer was washed with brine (100 mL), dried over Na₂SO₄, filtered and concentrated to afford **37a** (19.8 g, crude) as a yellow oil. LC-MS (ESI, Method 3) t_{R} = 1.26 min, m/z (M+H-56)⁺ = 261.2.

### Step 2. Methyl 2-(6-chloro-5-methoxypyrimidin-4-yl)acetate (37b)

To a mixture of **37a** (18.0 g, 56.83 mmol) in DCM (150 mL) was added TFA (75 mL) at 0 °C. After stirring at r.t. for 4 h, the reaction mixture was concentrated. The residue was diluted with EtOAc (500 mL) and washed with *sat.* NaHCO₃ (150 mL) and brine (100 mL). The organic layer was concentrated. The residue was purified by flash chromatography on silica gel (PE/EA = 10/1) to afford **37b** (10.5 g, 85% yield) as a yellow oil. ¹H NMR (400 MHz, CDCl₃) δ 8.67 (s, 1H), 3.95 (s, 3H), 3.91 (s, 2H), 3.75 (s, 3H).

### Step 3. Methyl 2-(6-((2,4-dimethoxybenzyl)amino)-5-methoxypyrimidin-4-yl)acetate (37c)

A mixture of **37b** (1.0 g, 4.62 mmol), TEA (934 mg, 9.23 mmol) and DMBNH₂ (1.00 g, 6.00 mmol) in EtOH (10 mL) was stirred at 80 °C for 5 h. The mixture was diluted with H₂O (20 mL), extracted with EtOAc (40 mL*2). The organic layer was concentrated and the residue was purified by flash chromatography on silica gel (PE/EA = 1/1) to afford **37c** (1.33 g, 83% yield) as a white solid. LC-MS (ESI, Method 3) t_{R} = 1.07 min, m/z (M+H)⁺ = 348.2.

### Step 4. (Z)-Methyl 2-(6-((2,4-dimethoxybenzyl)amino)-5-methoxypyrimidin-4-yl)-3-(dimethylamino)acrylate (37d)

A mixture of **37c** (1.33 g, 3.83 mmol) in DMF-DMA (10 mL) was stirred at 120 °C for 18 h. The mixture was concentrated to afford **37d** (1.54 g, crude) as a brown oil. LC-MS (ESI, Method 3) t_{R} = 1.07 min, m/z (M+H)⁺ = 403.3.

### Step 5. Methyl 5-((2,4-dimethoxybenzyl)amino)-4-methoxypyrazolo[1,5-c]pyrimidine-3-carboxylate (37e)

To a mixture of **37d** (1.61 g, 4.00 mmol) in DCM (30 mL) was added dropwise a solution of *O*-(mesitylsulfonyl)hydroxylamine (1.03 g, 4.80 mmol) in DCM (5 mL) at 0 °C. The mixture was stirred at 0 °C for 2 h. Another batch of a solution of *O-*(mesitylsulfonyl)hydroxylamine (515 mg, 2.40 mmol) in DCM (5 mL) was added into the mixture at 0 °C. The mixture was stirred at 0 °C for 1 h. The mixture was concentrated and purified by flash chromatography on silica gel (DCM/MeOH = 50/1) to afford **37e** (788 mg, 53% yield) as a yellow solid. ¹H NMR (400 MHz, CDCl₃) δ 8.84 (s, 1H), 8.27 (s, 1H), 7.21 (d, *J* = 8.4 Hz, 1H), 6.46 (d, *J* = 2.0 Hz, 1H), 6.41 (dd, *J=* 8.0 Hz, 2.4 Hz, 1H), 5.64 (t, *J =* 6.0 Hz, 1H), 4.64 (d, *J* = 6.0 Hz, 2H), 3.86 (s, 3H), 3.85 (s, 3H), 3.80 (s, 3H), 3.79 (s, 3H).

### Step 6. Methyl 5-amino-4-methoxypyrazolo[1,5-c]pyrimidine-3-carboxylate (37f)

To a mixture of **37e** (4.26 g, 11.44 mmol) in DCM (15 mL) was added TFA (15 mL) dropwise at r.t. The reaction mixture was stirred at r.t. for 1 h and concentrated under vacuum at 30 °C. The residue was dissolved in DCM (80 mL) and the solution was basified with 1 M NaOH to pH = 12. The mixture was extracted with DCM (80 mL). The organic layer was concentrated and the residue was purified by flash chromatography on silica gel (DCM/MeOH = 10/1) to afford **37f** (1.94 g, 76% yield) as a yellow solid. ¹H NMR (400 MHz, DMSO-*d*₆) δ 9.09 (s, 1H), 8.27 (s, 1H), 6.68 (brs, 2H), 3.77 (s, 3H), 3.71 (s, 3H).

### Step 7. Methyl 5-iodo-4-methoxypyrazolo[1,5-c]pyrimidine-3-carboxylate (37g)

To a mixture of **37f** (1.1 g, 4.95 mmol) inACN (12 mL) was added *tert-*Butyl nitrite (766 mg, 7.43 mmol, 0.88 mL) at 0 °C. The mixture was stirred at 15 °C for 10 min. CuI (1.41 g, 7.43 mmol) was added to the mixture and stirred at 70 °C for 2 h. After cooling to r.t., the reaction was quenched with 40 mL of 25% *aq*.NH₃.H₂O and extracted with EtOAc (60 mL*2). The combined organic layer was concentrated and the residue was purified by flash chromatography (PE/EA = 2/1) to afford **37g** (410 mg, 25% yield) as a yellow solid. LC-MS (ESI, Method 3) t_{R} = 1.24 min, m/z (M+H)⁺ = 333.9.

### Step 8. Methyl 4-methoxy-5-(3,3,3-trifluoroprop-1-en-2-yl)pyrazolo[1,5-c]pyrimidine-3-carboxylate (37h)

A mixture of **37g** (450 mg, 1.35 mmol), 4,4,6-trimethyl-2-(3,3,3-trifluoroprop-1-en-2-yl)-1,3,2-dioxaborinane (600 mg, 2.70 mmol), Na₂CO₃ (287 mg, 2.70 mmol) and Pd(dppf)Cl₂ (99 mg, 0.14 mmol) in 1,4-dioxane/H₂O (4.5 mL/1.5 mL) was stirred at 90 °C for 3 h. After cooled to r.t., the reaction mixture was diluted with EtOAc (15 mL), washed with water (7 mL) and brine (7mL). The organic layer was concentrated to afford **37h** (400 mg, crude) as a yellow oil, which was used for the next step directly without further purification. LC-MS (ESI, Method 3) t_{R} = 1.25 min, m/z (M+H)⁺ = 302.0.

### Step 9. Methyl 4-methoxy-5-(1,1,1-trifluoropropan-2-yl)pyrazolo[1,5-c]pyrimidine-3-carboxylate (37i)

A mixture of **37h** (400 mg, 1.33 mmol), Pd(OH)₂/C (200 mg, Pd 20% on carbon, wetted with water) and Pd/C (200 mg, 10% wt. wetted with water) in MeOH (4 mL) was stirred at 50 °C under H₂ (50 psi) atmosphere for 18 h. The reaction mixture was filtered and the filtrate was concentrated to afford **37i** (400 mg, crude) as a yellow solid, which was used for the next step directly without further purification. LC-MS (ESI, Method 3) t_{R} = 1.27 min, m/z (M+H)⁺ = 304.2.

### Step 10. 4-Methoxy-5-(1,1,1-trifluoropropan-2-yl)pyrazolo[1,5-c]pyrimidine-3-carboxylic acid (37j)

A mixture of **37i** (400 mg, 1.32 mmol) and NaOH (106 mg, 2.64 mmol) in MeOH (8 mL) and H₂O (4 mL) was stirred at 40 °C for 10 h. The reaction mixture was concentrated and added into water (8 mL). The aqueous solution was acidified with 2 *N* HCl to pH = 1. The resultant mixture was extracted with EtOAc (20 mL*2) and the combined organic phase was concentrated. The residue was purified by *prep-HPLC* (Method A) to afford **37j** (180 mg, 51 % yield) as a white solid. LC-MS (ESI, Method 3) t_{R} = 0.47 min, m/z (M+H)⁺ = 290.1.

### Step 11. 3-Iodo-4-methoxy-5-(1,1,1-trifluoropropan-2-yl)pyrazolo[1,5-c]pyrimidine (37k)

To a mixture of **37j** (92 mg, 0.32 mmol) and NaHCO₃ (80 mg, 0.95 mmol) in DMF (2 mL) was added NIS (215 mg, 0.95 mmol) at 0 °C. The mixture was stirred at 40 °C for 10 h. After cooling to r.t., the reaction mixture was diluted with *sat.* Na₂S₂O₃ (5 mL) at 0 °C. Then the mixture was extracted with EtOAc (8 mL*2). The combined organic layer was washed with brine (5 mL*2), dried over Na₂SO₄, filtered and the filtrate was concentrated to afford **37k** (115 mg, crude) as a yellow solid, which was used for the next step directly without further purification. LC-MS (ESI, Method 3) t_{R} = 1.38 min, m/z (M+H)⁺ = 371.9.

### Step 12. N-(4-methoxy-5-(1,1,1-trifluoropropan-2-yl)pyrazolo[1,5-c]pyrimidin-3-yl)-1,1-diphenylmethanimine (37l)

A mixture of **37k** (115 mg, 0.31 mmol), diphenylmethanimine (112 mg, 0.62 mmol, 0.1 mL), Xantphos (18 mg, 0.031 mmol), Pd₂(dba)₃ (28 mg, 0.031 mmol) and Cs₂CO₃ (202 mg, 0.62 mmol) in 1,4-dioxane (1 mL) was stirred at 90 °C for 16 h under N₂. After cooling to r.t., the mixture was diluted with water (5 mL), extracted with EtOAc (8 mL*3). The combined organic layer was concentrated and the residue was purified by flash chromatography (PE/EA = 5/1) to afford **37l** (20 mg, 15% yield) as a yellow oil. LC-MS (ESI, Method 3) t_{R} = 1.62 min, m/z (M+H)⁺ = 425.2.

### Step 13. 6-(Cyclopropanecarboxamido)-4-((4-methoxy-5-(1,1,1-trifluoropropan-2-yl)pyrazolo[1,5-c]pyrimidin-3-yl)amino)-N-(methyl-d₃)nicotinamide (37)

A mixture of **37l** (20 mg, 0.047 mmol), **Int. A** (12 mg, 0.047 mmol) and TsOH.H₂O (2 mg, 0.01 mmol) in 1,4-dioxane (0.5 mL) was stirred at 60 °C for 16 h. The mixture was concentrated and the residue was purified by *prep-HPLC* (Method A) to afford **37** (7.2 mg, 32% yield) as a white solid. ¹H NMR (400 MHz, DMSO-*d*₆) δ 10.69 (s, 1H), 10.36 (s, 1H), 9.37 (s, 1H), 8.56 (s, 1H), 8.52 (s, 1H), 8.33 (s, 1H), 7.63 (s, 1H), 4.20-4.03 (m, 1H), 3.74 (s, 3H), 2.02-1.87 (m, 1H), 1.45 (d, *J=* 7.2 Hz, 3H), 0.80-0.66 (m, 4H). LC-MS (ESI, Method 2) t_{R} = 2.75 min, m/z (M+H)⁺ = 481.2.

### Example 38

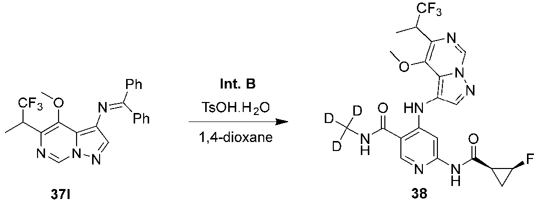

### Step 1. 6-((1S,2S)-2-fluorocyclopropane-1-carboxamido)-4-((4-methoxy-5-(1,1,1-trifluoropropan-2-yl)pyrazolo[1,5-c]pyrimidin-3-yl)amino)-N-(methyl-d₃)nicotinamide (38)

A mixture of **37l** (20 mg, 0.047 mmol), **Int. B** (13 mg, 0.047 mmol) and TsOH.H₂O (3 mg, 0.016 mmol) in 1,4-dioxane (0.5 mL) was stirred at 80 °C for 8 h. After cooling to r.t., the mixture was diluted with EtOAc (6 mL), washed with water (2 mL) and brine (2 mL). The organic layer was concentrated and the residue was purified by *prep-HPLC* (Method A) to afford **38** (10.2 mg, 43% yield) as a white solid. ¹H NMR (400 MHz, DMSO-*d*₆) δ 10.76 (s, 1H), 10.40-10.38 (m, 1H), 9.38 (s, 1H), 8.59 (s, 1H), 8.53 (s, 1H), 8.35 (s, 1H), 7.64-7.63 (m, 1H), 4.94-4.77 (m, 1H), 4.13-4.11 (m, 1H), 3.75-3.74 (m, 3H), 2.14-2.12 (m, 1H), 1.56-1.44 (m, 4H), 1.13-1.10 (m, 1H). LC-MS (ESI, Method 2) t_{R} = 2.73 min, m/z (M+H)⁺ = 499.2.

### Example 39

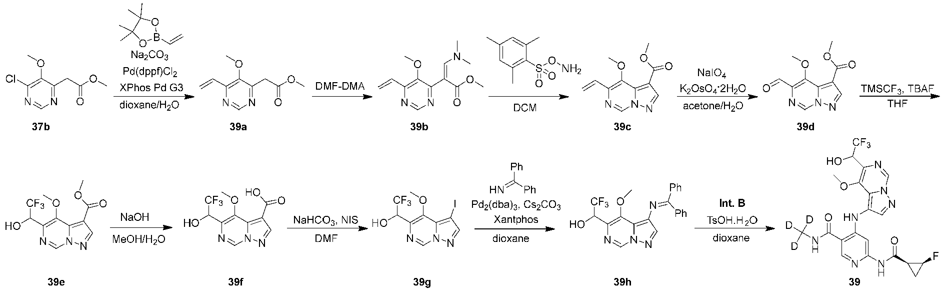

### Step 1. Methyl 2-(5-methoxy-6-vinylpyrimidin-4-yl)acetate (39a)

To a mixture of **37b** (12.1 g, 55.86 mmol) in dioxane (64 mL) and water (16 mL) was added Pd(dppf)Cl₂ (2.0 g, 2.79 mmol), XPhos Pd G3 (472 mg, 0.56 mmol) and Na₂CO₃ (11.8 g, 111.72 mmol). The reaction mixture was stirred at 90 °C for 28 h under N₂ atmosphere. After cooling to r.t., the mixture was diluted with water (20 mL) and extracted with EtOAc (70 mL*3). The organic layer was washed by brine (30 mL), dried over Na₂SO₄, filtered and concentrated. The residue was purified by flash chromatography on silica gel (PE/EA = 3/1) to afford **39a** (7 g, 60% yield) as a yellow oil. LC-MS (ESI, Method 3) t_{R} = 1.02 min, m/z (M+H)⁺ = 209.3.

### Step 2. Methyl 3-(dimethylamino)-2-(5-methoxy-6-vinylpyrimidin-4-yl)acrylate (39b)

A mixture of **39a** (7 g, 33.61 mmol) in DMF-DMA (13.4 mL, 100.83 mmol) was stirred at 60 °C for 4 h. The mixture was concentrated to afford **39b** (8.7 g, crude) as a yellow oil, which was used for the next step directly without further purification. LC-MS (ESI, Method 3) t_{R} = 0.94 min, m/z (M+H)⁺ = 264.3.

### Step 3. Methyl 4-methoxy-5-vinylpyrazolo[1,5-c]pyrimidine-3-carboxylate (39c)

To a mixture **39b** (8.7 g, 33.04 mmol) in DCM (30 mL) was dropped a solution of amino 2,4,6-trimethylbenzenesulfonate (14.23 g, 66.09 mmol) in DCM (30 mL) at 0 °C. The reaction was stirred at 0 °C for 1 h. The mixture was basified to pH = 9 with *sat.* Na₂CO₃. The separated aqueous layer was extracted with DCM (200 mL*3). The combined organic layer was concentrated and the residue was purified by flash chromatography on silica gel (PE/EA = 1/1) to afford **39c** (2.1 g, 27% yield) as a yellow solid. LC-MS (ESI, Method 3) t_{R} = 1.17 min, m/z (M+H)⁺ = 234.2.

### Step 4. Methyl 5-formyl-4-methoxypyrazolo[1,5-c]pyrimidine-3-carboxylate (39d)

To a mixture **39c** (2.0 g, 8.58 mmol) in acetone (10 mL) was added K₂O_{S}O₄.2H₂O (158 mg, 0.43 mmol). A solution of NaIO₄ (3.67 g, 17.15 mmol) in water (10 mL) was added to the mixture at 0 °C. The mixture was stirred at 25 °C for 1 h and filtered. The filtrate was concentrated and purified by flash chromatography on silica gel (PE/EA = 1/1) to afford **39d** (1.2 g, 59% yield) as a yellow solid. LC-MS (ESI, Method 3) t_{R} = 0.94 min, m/z (M+H)⁺ = 236.0.

### Step 5. Methyl 4-methoxy-5-(2,2,2-trifluoro-1-hydroxyethyl)pyrazolo[1,5-c]pyrimidine-3-carboxylate (39e)

To a mixture of **39d** (1.20 g, 5.10 mmol) in THF (10 mL) was added TMSCF₃ (1.60 g, 11.22 mmol) dropwise followed by TBAF (1.0 M in THF, 0.25 mL). After stirring at 0 °C for 10 min, the reaction mixture was diluted with THF (10 mL) and *aq.* HCl (10 mL, 1 M). The mixture was stirred at 25 °C for 1 h. The mixture was basified with *aq.* NaOH (1 M) to pH = 8 and extracted with EtOAc (50 mL*3). The combined organic layer was concentrated to afford **39e** (1 g, 64% yield) as a brown oil, which was used for the next step directly without further purification. LC-MS (ESI, Method 3) t_{R} = 1.12 min, m/z (M+H)⁺ = 306.0.

### Step 6. 4-Methoxy-5-(2,2,2-trifluoro-1-hydroxyethyl)pyrazolo[1,5-c]pyrimidine-3-carboxylic acid (39f)

To a mixture of **39e** (600 mg, 1.97 mmol) in methanol (4 mL) and water (2 mL) was added NaOH (157.3 mg, 3.93 mmol) and the mixture was stirred at 45 °C for 8 h. The mixture was adjusted with *aq.* HCl (2 M) to pH = 1 and extracted with EtOAc (20 mL*3). The organic layer was concentrated and the residue was purified by *prep-HPLC* (Method A) to afford **39f** (130 mg, 23% yield) as a yellow solid. LC-MS (ESI, Method 3) t_{R} = 0.92 min, m/z (M+H)⁺ = 292.0.

### Step 7. 2,2,2-Trifluoro-1-(3-iodo-4-methoxypyrazolo[1,5-c]pyrimidin-5-yl)ethan-1-ol (39g)

To a solution of 39f (110 mg, 0.38 mmol) and NaHCO₃ (95 mg, 1.13 mmol) in DMF (1.2 mL) was added NIS (255 mg, 1.13 mmol) at 0 °C. The mixture was stirred at 15 °C for 18 h. The reaction was quenched with *sat.* Na₂S₂O₃ (10 mL) under ice-water bath, and extracted with EtOAc (15 mL*2). The combined organic layer was washed with brine (5 mL), dried over Na₂SO₄, filtered and concentrated. The residue was purified by *prep-HPLC* (Method A) to afford **39g** (16 mg, 11 % yield) as a yellow solid. LC-MS (ESI, Method 3) t_{R} = 1.19 min, m/z (M+H)⁺ = 373.9.

### Step 8. 1-(3-((Diphenylmethylene)amino)-4-methoxypyrazolo[1,5-c]pyrimidin-5-yl)-2,2,2-trifluoroethan-1-ol (39h)

A mixture of **39g** (17 mg, 0.045 mmol), diphenylmethanimine (17 mg, 0.091 mmol), Xantphos (3 mg, 0.0046 mmol), Pd₂(dba)₃ (4 mg, 0.0046 mmol) and Cs₂CO₃ (30 mg, 0.091 mmol) in 1,4-dioxane (0.5 mL) was stirred at 90 °C for 8 h under N₂ atmosphere. The mixture was diluted with water (3 mL), extracted with EtOAc (5 mL*3). The combined organic layer was concentrated and the residue was purified by *prep-TLC* (PE/EA = 5/1) to afford **39h** (8 mg, 41% yield) as a yellow solid. LC-MS (ESI, Method 3) t_{R} = 1.44 min, m/z (M+H)⁺ = 427.2.

### Step 9. 6-((18,25)-2-fluorocyclopropane-1-carboxamido)-4-((4-methoxy-5-(2,2,2-trifluoro-1-hydroxyethyl)pyrazolo[1,5-c]pyrimidin-3-yl)amino)-N-(methyl-d₃)nicotinamide (39)

A mixture of **39h** (16 mg, 0.038 mmol), **Int. B** (12 mg, 0.045 mmol) and TsOH.H₂O (3 mg, 0.015 mmol) in 1,4-dioxane (0.5 mL) was stirred at 80 °C for 8 h. The mixture was filtered and washed with DCM (2 mL). The filter cake was purified by *prep-*TLC (DCM/MeOH = 10/1) to afford **39** (5.8 mg, 31% yield) as a white solid. ¹H NMR (400 MHz, DMSO-*d*₆) δ 10.78 (s, 1H), 10.43 (s, 1H), 9.38 (s, 1H), 8.60 (s, 1H), 8.54 (s, 1H), 8.38 (s, 1H), 7.64 (s, 1H), 5.39-5.27 (m, 1H), 4.95-4.74 (m, 1H), 3.78 (s, 3H), 2.20-2.07 (m, 1H), 2.05-1.94 (m, 1H), 1.59-1.41 (m, 1H), 1.15-1.03 (m, 1H). LC-MS (ESI, Method 2) t_{R} = 2.45 min, m/z (M+H)⁺ = 501.0*.*

### Example 40

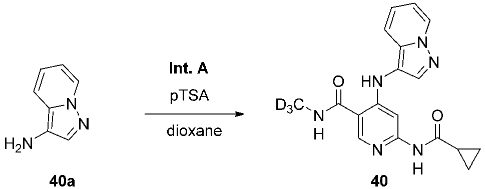

### Step 1. 6-(Cyclopropanecarboxamido)-N-(methyl-d₃)-4-(pyrazolo[1,5-a]pyridin-3-ylamino)nicotinamide (40)

To a solution of **Int. A** (20 mg, 0.078 mmol), pTSA (13 mg, 0.078mmol) in dioxane (7 mL) was added **40a** (21 mg, 0.16 mmol) at r.t. The mixture was stirred at 100 °C for 3 h. It was added into H₂O (10 mL) and extracted by EA (20 mL). The combined organic layer was washed by brine (20 mL*2), dried over anhydrous Na₂SO₄, filtered and concentrated in vacuo. The residue was purified by *prep-HPLC* (Method E) to afford **40** (16.5 mg, 60% yield) as a white solid. ¹H NMR (400 MHz, DMSO-*d*₆) δ 10.63 (s, 1H), 10.06 (s, 1H), 8.68-8.65 (m, 1H), 8.54 (s, 1H), 8.49 (s, 1H), 8.04 (s, 1H), 7.42-7.39 (m, 1H), 7.38 (s, 1H), 7.23-7.18 (m, 1H), 6.95-6.90 (m, 1H), 1.95-1.86 (m, 1H), 0.72-0.63 (m, 4H). LC-MS (ESI, Method 4) t_{R} = 4.44 min, m/z (M+H)⁺ = 354.3.

### Example 41

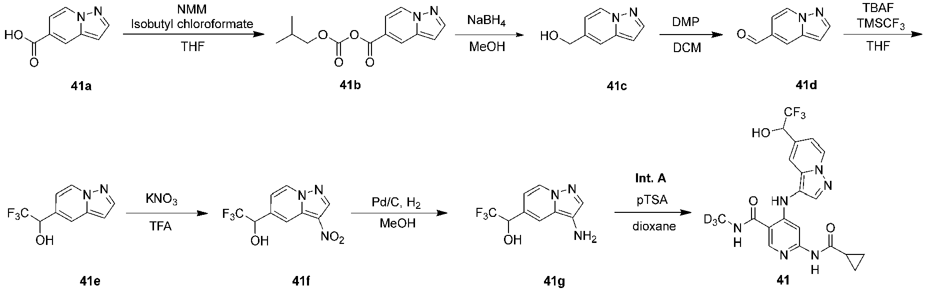

### Step 1. (Isobutyl carbonic) pyrazolo[1,5-a]pyridine-5-carboxylic anhydride (41b)

To a solution of **41a** (1 g, 6.17 mmol) in THF (20 mL) was added NMM (749 mg, 7.40 mmol) at 0 °C under nitrogen. Isobutyl chloroformate (1.01 g, 7.40 mmol) was dropped 5 minutes later. The reaction was stirred at 0 °C to r.t. for 2 h. The resulting solution was added into H₂O (30 mL) and extracted by EA (50 mL). The combined organic layer was washed by brine (30 mL*2), dried over Na₂SO₄ and filtered. The filtrate was concentrated in vacuo to afford **41b** (1.8 g, crude) as a yellow oil, which was used for the next step directly without further purification. LC-MS (ESI, Method 4) t_{R} = 3.14 min, m/z (M+H)⁺ = 263.1.

### Step 2. Pyrazolo[1,5-a]pyridin-5-ylmethanol (41c)

To a solution of **41b** (1.8 g, crude) in methanol (20 mL) was added NaBH₄ (662 mg, 17.5 mmol) at 0 °C. The reaction was stirred at 0 °C for 2 h. The resulting solution was added into NH₄Cl solution (30 mL) and extracted by EA (40 mL). The combined organic layer was washed by brine (30 mL*2), dried over Na₂SO₄, filtered and concentrated in vacuo to afford **41c** (1 g, crude) as a yellow oil, which was used for the next step directly without further purification. LC-MS (ESI, Method 4) t_{R} = 0.72 min, m/z (M+H)⁺ = 149.1.

### Step 3. Pyrazolo[1,5-a]pyridine-5-carbaldehyde (41d)

To a solution of **41c** (1 g, 5.40 mmol) in DCM (15 mL) was added Dess-Martin Periodinane (3.44 g, 8.10 mmol) at r.t. The reaction was stirred at 25 °C for 1 h. The resulting solution was filtered and the combined organic layer was concentrated in vacuo. The crude was purified by flash chromatography (EA in PE is 10-40%) to afford **41d** (600 mg, 76% yield) as a white solid. LC-MS (ESI, Method 4) t_{R} = 1.34 min, m/z (M+H)⁺ = 147.1.

### Step 4. 2,2,2-Trifluoro-1-(pyrazolo[1,5-a]pyridin-5-yl)ethan-1-ol (41e)

To a solution of **41d** (300 mg, 2.05 mmol) and TMSCF₃ (409 mg, 2.87 mmol) in THF (15 mL) was added TBAF (1.0 M in THF, 107 mg, 0.41 mmol) at 0 °C. The mixture was stirred at 0 °C for 1 h and at 25 °C for 12 h. Then 1 M HCl solution was added, and the reaction was stirred at 25 °C for 2 h. The mixture was adjusted to pH = 8 with *aq.* NaOH (1 M). The mixture was extracted with EA (15 mL*3). The organic layer was concentrated and the residue was purified by flash chromatography (PE/EA = 2/1) to afford **41e** (238 mg, 54% yield) as a white solid. LC-MS (ESI, Method 4) t_{R} = 2.00 min, m/z (M+H)⁺ = 217.1.

### Step 5. 2,2,2-Trifluoro-1-(3-nitropyrazolo[1,5-a]pyridin-5-yl)ethan-1-ol (41f)

To a solution of **41e** (100 mg, 0.46 mmol) in TFA (5 mL) was added KNO₃ (56 mg, 0.56 mmol) at r.t. The reaction was stirred at 25 °C for 2 h. The resulting solution was added into H₂O (10 mL) and the pH was adjusted to 8-9 with *sat.* Na₂CO₃ solution. The mixture was extracted with EA (20 mL*2). The combined organic phase was concentrated and the residue was purified by flash chromatography (PE/EA = 1/1) to afford 41f (100 mg, 83% yield) as a yellow oil. LC-MS (ESI, Method 4) t_{R} = 2.33 min, m/z (M+H)⁺ = 262.0.

### Step 6. 1-(3-Aminopyrazolo[1,5-a]pyridin-5-yl)-2,2,2-trifluoroethan-1-ol (41g)

To a solution of **41f** (100 mg, 0.38 mmol) in methanol (10 mL) was added Pd/C (10% on carbon, wetted with ca. 55% water) (8 mg) under H₂ atmosphere. The mixture was stirred at 25 °C for 3 h. The catalyst was filtered off and the filtrate was condensed by vacuum to give **41g** (100 mg, crude) as a brown solid, which was used in the next step without further purification. LC-MS (ESI, Method 4) t_{R} = 0.38 min, m/z (M+H)⁺ = 232.0.

### Step 7. 6-(Cyclopropanecarboxamido)-N-(methyl-d₃)-4-((5-(2,2,2-trifluoro-1-hydroxyethyl)pyrazolo[1,5-a]pyridin-3-yl)amino)nicotinamide (41)

To a solution of **Int. A** (15 mg, 0.058 mmol), 4-methylbenzenesulfonic acid (10 mg, 0.058 mmol) in dioxane (4 mL) was added **41g** (27 mg, 0.12 mmol) at r.t. The mixture was stirred at 100 °C for 3 h. The resulting solution was added into H₂O (20 mL) and extracted by EA (30 mL). The combined organic layer was washed by brine (20 mL*2), dried over Na₂SO₄, filtered and concentrated in vacuo. The crude was purified by flash chromatography and then *prep-HPLC* (Method E) to afford **41** (6.4 mg, 24% yield) as a light pink solid. ¹H NMR (400 MHz, DMSO-*d*₆) δ 10.64 (s, 1H), 10.09 (s, 1H), 8.71 (d, *J=* 7.2 Hz, 1H), 8.56 (s, 1H), 8.50 (s, 1H), 8.09 (s, 1H), 7.56 (d, *J* = 1.6 Hz, 1H), 7.43 (s, 1H), 7.06 (d, *J =* 6.0 Hz, 1H), 6.99 (d, *J=* 7.2 Hz, 1H), 5.36-5.27 (m, 1H), 1.94-1.85 (m, 1H), 0.73-0.62 (m, 4H). LC-MS (ESI, Method 4) t_{R} = 1.47 min, m/z (M+H)⁺ = 452.2.

### Example 42

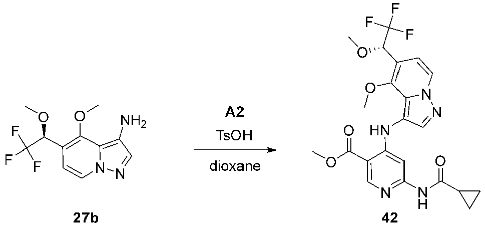

### Step 1. Methyl (S)-6-(cyclopropanecarboxamido)-4-((4-methoxy-5-(2,2,2-trifluoro-1-methoxyethyl)pyrazolo[1,5-a]pyridin-3-yl)amino)nicotinate (42)

To a solution of **27b** (70 mg, 0.25 mmol) and **A2** (45 mg, 0.18 mmol) in dioxane (2 mL) was added TsOH (48 mg, 0.28 mmol), the mixture was stirred at 85 °C for 3 h. The mixture was condensed by vacuum and the residue was purified by *prep-HPLC* (Method E) to give **42** (30 mg, 24% yield) as an off-white solid. ¹H NMR (400 MHz, DMSO-*d*₆) *δ* 10.81 (s, 1H), 9.57 (s, 1H), 8.64 (s, 1H), 8.53 (d, *J =* 7.2 Hz, 1H), 8.14 (s, 1H), 7.64 (s, 1H), 6.76 (d, *J* = 7.2 Hz, 1H), 5.25 (q, *J=* 6.8 Hz, 1H), 3.85 (s, 3H), 3.67 (s, 3H), 3.30 (s, 3H), 1.95-1.84 (m, 1H), 0.75-0.57 (m, 4H). LC-MS (ESI, Method 4) t_{R} = 2.82 min, m/z (M+H)⁺ = 494.3.

### Example 43

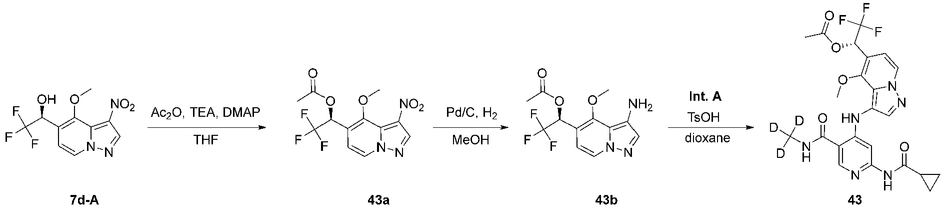

### Step 1. (S)-2,2,2-trifluoro-1-(4-methoxy-3-nitropyrazolo[1,5-a]pyridin-5-yl)ethyl acetate (43a)

To a solution of **7d-A** (150 mg, 0.52 mmol), TEA (261 mg, 2.58 mmol, 0.36 mL) and DMAP (6 mg, 0.052 mmol) in THF (5 mL) was added Ac₂O (526 mg, 5.15 mmol, 0.49 mL), the mixture was stirred at 55 °C for 2 h. The mixture was quenched with *sat.* NaHCO₃ (20 mL) and extracted with EtOAc (30 mL*2). The combined organic layer was washed with brine (20 mL*2), dried and condensed by vacuum. The residue was purified by flash chromatography (EtOAc in PE is 20%) to give **43a** (149 mg, 87%yield) as a yellow solid. LC-MS (ESI, Method 4) t_{R} = 2.95 min, m/z (M+H)⁺ = 334.1.

### Step 2. (S)-1-(3-amino-4-methoxypyrazolo[1,5-a]pyridin-5-yl)-2,2,2-trifluoroethyl acetate (43b)

To a solution of **43a** (90 mg, 0.27 mmol) in MeOH (4 mL) was added Pd/C (9 mg, 0.081 mmol, 10% on carbon, wetted with ca. 55% water) under H₂ atmosphere. The mixture was stirred at 25 °C for 3 h. The reaction mixture was filtered and the filtrate was concentrated to give **43b** (70 mg, 85% yield) as a brown oil, which was used for the next step directly without further purification. LC-MS (ESI, Method 4) t_{R} = 2.18 min, m/z (M+H)⁺ = 304.1.

### Step 3. (S)-1-(3-((2-(cyclopropanecarboxamido)-5-((methyl-d₃)carbamoyl)pyridin-4-yl)amino)-4-methoxypyrazolo[1,5-a]pyridin-5-yl)-2,2,2-trifluoroethyl acetate (43)

To a solution of **43b** (70 mg, 0.23 mmol) and **Int. A** (41 mg, 0.16 mmol) in dioxane (4 mL) was added TsOH (44 mg, 0.25 mmol). The mixture was stirred at 85 °C for 3 h. The mixture was condensed by vacuum and the residue was purified by *prep-HPLC* (Method E) to give **43** (45 mg, 37% yield) as an off-white solid. ¹H NMR (400 MHz, DMSO-*d*₆) *δ* 10.71 (s, 1H), 10.41 (s, 1H), 8.58 (s, 1H), 8.55 (d, *J=* 7.2 Hz, 1H), 8.52 (s, 1H), 8.17 (s, 1H), 7.71 (s, 1H), 6.83 (d, *J* = 7.2 Hz, 1H), 6.59 (q, *J =* 7.2 Hz, 1H), 3.78 (s, 3H), 2.20 (s, 3H), 1.97-1.89 (m, 1H), 0.78-0.66 (m, 4H). LC-MS (ESI, Method 4) t_{R} = 2.34 min, m/z (M+H)⁺ = 524.4.

### Example 44

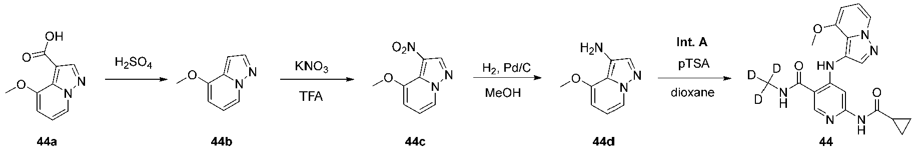

### Step 1. 4-Methoxypyrazolo[1,5-a]pyridine (44b)

Heat a well-stirred solution of **44a** (200 mg, 1.04 mmol) in 1 mL of 50% H₂SO₄ at 100 °C for 1 h. A brown solution was formed. Cool down the solution to room temperature. Neutralize the solution with aq. NaOH (1.0 M) using litmus paper as the indicator. Add 40 mL of water to the above solution. Extract the solution by EtOAc (30 mL*3). Combine the organic layer. Dry the organic layer over anhydrous Na₂SO₄. Filter the organic layer through a pad of celite. Evaporate the organic layer in vacuum to give **44b** (45 mg, 94% yield) as a colorless oil. LC-MS (ESI, Method 4) t_{R} = 2.01 min, m/z (M+H)⁺ = 149.1.

### Step 2. 4-Methoxy-3-nitro-pyrazolo[1,5-a]pyridine (44c)

A mixture of **44b** (145 mg, 0.98 mmol) in TFA (2 mL) was added into KNO₃ (89 mg, 0.88 mmol) at 20 °C. The resulting mixture was stirred at 30 °C for 1 h. A yellow solution was formed. The reaction mixture was quenched with aq. NaHCO₃ (50 mL) and extracted with EtOAc (30 mL*3). The combined organic layer was washed with brine (40 mL*2), dried over anhydrous Na₂SO₄, filtered and concentrated. The residue was purified by flash chromatography (EA in PE is 20-60%) to give **44c** (80 mg, 42% yield) as a yellow solid. LC-MS (ESI, Method 4) t_{R} = 1.78 min, m/z (M+H)⁺ = 194.1.

### Step 3. 4-Methoxypyrazolo[1,5-a]pyridin-3-amine (44d)

A mixture of **44c** (50 mg, 0.26 mmol) and Pd/C (5 mg, 10% Pd (dry basis), wetted with 55% H₂O) in MeOH (3 mL) was degassed and purged with hydrogen for 3 times. The resulting mixture was stirred at 40 °C under H₂ atmosphere for 12 h. A black suspension was formed. The reaction mixture was filtered and concentrated. The residue was purified by *prep-*TLC (DCM/MeOH = 10/1) to give **44d** (20 mg, 47% yield) as a red solid. ¹H NMR (400 MHz, CDCl₃) *δ* 7.85 (d, *J* = 7.2 Hz, 1H), 7.46 (s, 1H), 6.43 (t, *J=* 7.2 Hz, 1H), 6.40 (d, *J=* 7.6 Hz, 1H), 3.92 (s, 3H), 3.56 (brs, 2H). LC-MS (ESI, Method 4) t_{R} = 0.43 min, m/z (M+H)⁺ = 164.1.

### Step 4. 6-(Cyclopropanecarboxamido)-4-((4-methoxypyrazolo[1,5-a]pyridin-3-yl)amino)-N-(methyl-d₃)nicotinamide (44)

A mixture of **44d** (20 mg, 0.12 mmol), **Int. A** (31 mg, 0.12 mmol) and pTSA (42 mg, 0.24 mmol) in dioxane (2 mL) was stirred at 100 °C for 12 h. A yellow suspension was formed. The reaction mixture was diluted with aq. NaHCO₃ (30 mL) and extracted with EtOAc (30 mL*3). The combined organic layer was washed with water (60 mL*2) and brine (50 mL*2), dried over anhydrous Na₂SO₄, filtered and concentrated. The residue was purified by flash chromatography (MeOH in DCM is 0-10%) and triturated in MeCN to give **44** (26.5 mg, 56% yield) as a light-yellow solid. ¹H NMR (400 MHz, DMSO-*d*₆) *δ* 10.69 (s, 1H), 10.58 (s, 1H), 8.49 (s, 1H), 8.46 (s, 1H), 8.19 (d, *J=* 6.8 Hz, 1H), 7.99 (s, 1H), 7.84 (s, 1H), 6.78 (t, *J=* 7.2 Hz, 1H), 6.53 (d, *J =* 7.6 Hz, 1H), 3.86 (s, 3H), 2.00-1.93 (m, 1H), 0.78-0.73 (m, 4H). LC-MS (ESI, Method 4) t_{R} = 4.92 min, m/z (M+H)⁺ = 384.3.

### Example 45

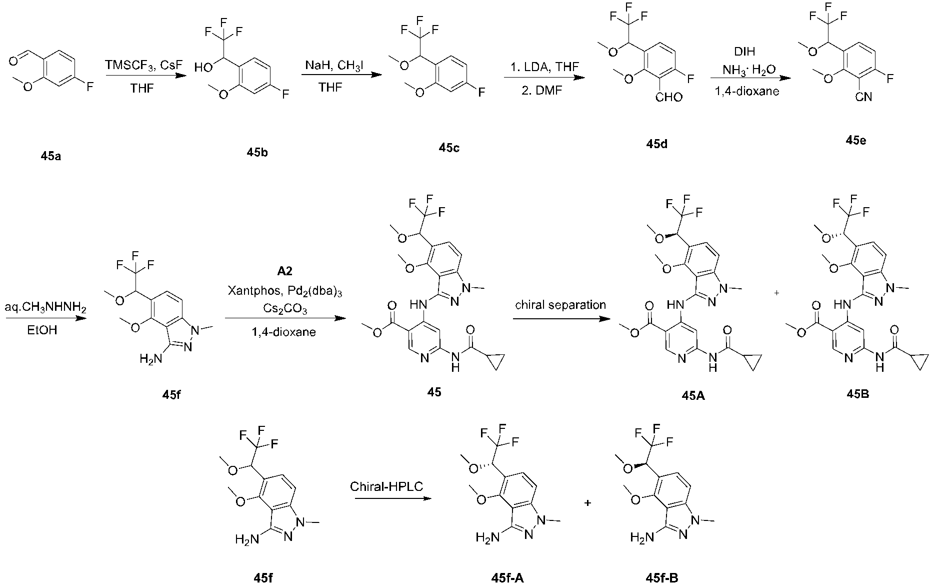

### Step 1. 2,2,2-Trifluoro-1-(4-fluoro-2-methoxyphenyl)ethan-1-ol (45b)

To a solution of **45a** (5 g, 32.44 mmol) and CsF (98 mg, 0.65 mmol) in THF (50 mL) was slowly added TMSCF₃ (9.23 g, 64.88 mmol) at 0 °C. After stirring at 30 °C for 3 h, the reaction was quenched with *aq.* HCl (1 mL, 2 M) and stirred at 20 °C for 1 h. The mixture was diluted with *sat.* NaHCO₃ (10 mL), extracted with EtOAc (10 mL). The organic layer was washed with brine (10 mL), dried over anhydrous Na₂SO₄ and filtered. The filtrate was concentrated. The residue was purified by flash chromatography on silica gel (PE/EtOAc = 20/1) to give **45b** (7.2 g, 99% yield) as a yellow oil. ¹H NMR (400 MHz, DMSO-*d*₆) δ 7.50 (t, *J=* 8.0 Hz, 1H), 6.97 (dd, *J=* 11.6, 2.8 Hz, 1H), 6.87-6.82 (m, 1H), 6.72 (d, *J=* 6.0 Hz, 1H), 5.37-5.32 (m, 1H), 3.82 (s, 3H).

### Step 2. 4-Fluoro-2-methoxy-1-(2,2,2-trifluoro-1-methoxyethyl)benzene (45c)

To a solution of **45b** (4.8 g, 21.41 mmol) in THF (48 mL) was added NaH (1.03 g, 25.70 mmol, 60% in mineral oil) at 0 °C. After stirring at 0 °C for 30 min, CH₃I (9.12 g, 64.24 mmol) was added and the reaction was stirred at 0 °C for 1 h, then 30 °C for 3 h. The solution was poured into ice-water (30 mL) and was extracted with EtOAc (40 mL*2). The combined organic phase was washed with brine (15 mL), dried with anhydrous Na₂SO₄ and concentrated. The residue was purified by flash chromatography on silica gel (PE/EtOAc = 30/1) to give **45c** (4.5 g, 88% yield) as a yellow oil. ¹H NMR (400 MHz, DMSO-*d*₆) δ 7.39 (t, *J* = 7.6 Hz, 1H), 6.97 (dd, *J =* 11.6, 2.4 Hz, 1H), 6.91-6.85 (m, 1H), 5.18-5.15 (m, 1H), 3.85 (s, 3H), 3.31 (s, 3H).

### Step 3. 6-Fluoro-2-methoxy-3-(2,2,2-trifluoro-1-methoxyethyl)benzaldehyde (45d)

To a solution of **45c** (1.28 g, 5.37 mmol) in THF (13 mL) was added LDA (4.03 mL, 2 M in THF) at -50 °C dropwise. The mixture was stirred at -50 °C for 1 h. Then DMF (8.06 mmol, 0.62 mL) was added to the mixture at -50 °C and the reaction was stirred at -50 °C for 1 h. The reaction was quenched with *sat.* NH₄Cl (25 mL) and extracted with EtOAc (50 mL*3). The combined organic layer was dried with anhydrous Na₂SO₄ and concentrated under reduced pressure to give **45d** (1.43 g, crude) as a yellow oil, which was used for the next step directly without further purification. ¹H NMR (400 MHz, DMSO-*d*₆) δ 10.27 (s, 1H), 7.77-7.73 (m, 1H), 7.31-7.26 (m, 1H), 5.26-5.23(m, 1H), 3.85 (s, 3H), 3.31 (s, 3H).

### Step 4. 6-Fluoro-2-methoxy-3-(2,2,2-trifluoro-1-methoxyethyl)benzonitrile (45e)

To a solution of **45d** (1.43 g, 5.37 mmol) in NH₃.H₂O (7 mL) and 1,4-dioxane (7 mL) was added 1,3-diiodo-5,5-dimethylimidazolidine-2,4-dione (2.04 g, 5.37 mmol) at 0 °C. Then the mixture was stirred at 33 °C for 16 h. TLC showed the starting material was disappeared. The mixture was diluted with water (15 mL) and extracted with EtOAc (20 mL*2). The organic layer was dried with anhydrous Na₂SO₄ and concentrated under reduced pressure to give **45e** (1.4 g, crude) as a yellow solid, which was used for the next step directly without further purification.

### Step 5. 4-Methoxy-1-methyl-5-(2,2,2-trifluoro-1-methoxyethyl)-1H-indazol-3-amine (45f)

A mixture of **45e** (1.2 g, 4.56 mmol) and 40% *aq.* methylhydrazine (7 mL) in EtOH (5 mL) was stirred at 90 °C for 4 h. After cooling to r.t., the mixture was diluted with water (25 mL) and extracted with EtOAc (30 mL*2). The combined organic layer was dried with anhydrous Na₂SO₄ and concentrated. The residue was purified by flash chromatography on silica gel (PE/EtOAc = 5/1) to give **45f** (390 mg, 36% yield) as a yellow solid. LC-MS (ESI, Method 3) t_{R} = 1.19 min, m/z (M+H)⁺ = 290.4.

### Step 6. Methyl 6-(cyclopropanecarboxamido)-4-((4-methoxy-1-methyl-5-(2,2,2-trifluoro-1-methoxyethyl)-1H-indazol-3-yl)amino)nicotinate (45)

A mixture of **45f** (200 mg, 0.69 mmol), **A2** (194 mg, 0.76 mmol), Pd₂(dba)₃ (127 mg, 0.14 mmol), Xantphos (80 mg, 0.14 mmol) and Cs₂CO₃ (451 mg, 1.38 mmol) in 1,4-dioxane (2 mL) was stirred at 90 °C for 5 h under N₂. The reaction mixture was concentrated, and the residue was purified by flash chromatography on silica gel (DCM/MeOH = 49/1) to give **45** (298 mg, 85% yield) as a yellow solid. LC-MS (ESI, Method 3) t_{R} = 1.29 min, m/z (M+H)⁺ = 508.5.

### Step 7. Methyl (R)-6-(cyclopropanecarboxamido)-4-((4-methoxy-1-methyl-5-(2,2,2-trifluoro-1-methoxyethyl)-1H-indazol-3-yl)amino)nicotinate (45A) and Methyl (S)-6-(cyclopropanecarboxamido)-4-((4-methoxy-1-methyl-5-(2,2,2-trifluoro-1-methoxyethyl)-1H-indazol-3-yl)amino)nicotinate (45B)

Compound **45** (290 mg, 0.57 mmol) was separated by chiral *prep*-HPLC (Method J) to give **45A** (77.5 mg, 27% yield) as a white solid and **45B** (77.5 mg, 27% yield) as a white solid.

**45A:** LC-MS (ESI, Method 2) t_{R} = 0.98 min, m/z (M+H)⁺ = 508.1. ¹H NMR (400 MHz, DMSO-*d*₆) δ 10.90 (s, 1H), 10.89 (s, 1H), 9.17 (s, 1H), 8.77 (s, 1H), 7.47 (d, *J* = 9.2 Hz, 1H), 7.41 (d, *J= 8.8* Hz, 1H), 5.29 (q, *J=* 6.8 Hz, 1H), 3.98 (s, 3H), 3.92 (s, 3H), 3.89 (s, 3H), 3.32 (s, 3H), 2.06-2.01 (m, 1H), 0.84-0.80 (m, 4H). Chiral-HPLC (Method 7) t_{R} = 13.97 min.

**45B:** LC-MS (ESI, Method 2) t_{R} = 0.98 min, m/z (M+H)⁺ = 508.1. ¹H NMR (400 MHz, DMSO-*d*₆) δ 10.90 (s, 1H), 10.89 (s, 1H), 9.17 (s, 1H), 8.77 (s, 1H), 7.47 *(d, J=* 8.8 Hz, 1H), 7.41 (d, *J =* 9.2 Hz, 1H), 5.29 (q, *J =* 7.2 Hz, 1H), 3.98 (s, 3H), 3.92 (s, 3H), 3.89 (s, 3H), 3.31 (s, 3H), 2.07-2.01 (m, 1H), 0.86-0.79 (m, 4H). Chiral-HPLC (Method 7) t_{R} = 17.26 min.

### Step 8: (S)-4-Methoxy-1-methyl-5-(2,2,2-trifluoro-1-methoxyethyl)-1H-indazol-3-amine (45f-A) and (R)-4-Methoxy-1-methyl-5-(2,2,2-trifluoro-1-methoxyethyl)-1H-indazol-3-amine (45f-B)

45f (5.8 g, 20.05 mmol) was separated by chiral *prep-HPLC* (Method G, Hex/EtOH = 90/10) to afford **45f-A** (1.84 g, 32% yield) as a white solid, Chiral HPLC (Method 8, Hex/EtOH = 90/10) t_{R} = 8.64 min and **45f-B** (2.23 g, 38% yield) as a white solid, Chiral HPLC (Method 8, Hex/EtOH = 90/10) t_{R} = 10.30 min.

### Example 46

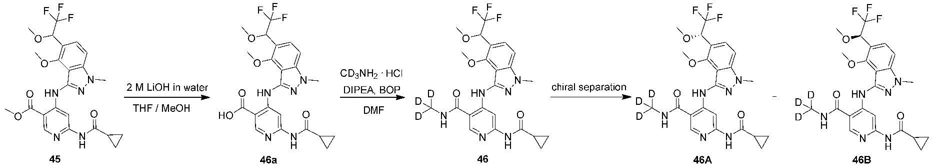

### Step 1. 6-(Cyclopropanecarboxamido)-4-((4-methoxy-1-methyl-5-(2,2,2-trifluoro-1-methoxyethyl)-1H-indazol-3-yl)amino)nicotinic acid (46a)

To a solution of **45** (80 mg, 0.16 mmol) in THF (6 mL) and MeOH (2 mL) was added *aq.* LiOH (0.5 mL, 2 M in water) at 10 °C. Then the mixture was stirred at 30 °C for 3 h. The mixture was adjusted to pH < 7 with 2 N HCl and extracted with EtOAc (8 mL*2). The combined organic layer was dried over anhydrous Na₂SO₄, filtered and concentrated to give **46a** (77 mg, crude) as a yellow oil, which was used for the next step directly without further purification. LC-MS (ESI, Method 3) t_{R} = 1.21 min, m/z (M+H)⁺ = 494.4.

### Step 2. 6-(Cyclopropanecarboxamido)-4-((4-methoxy-1-methyl-5-(2,2,2-trifluoro-1-methoxyethyl)-1H-indazol-3-yl)amino)-N-(methyl-d₃)nicotinamide (46)

To a solution of **46a** (70 mg, 0.14 mmol), methyl-*d*₃-amine hydrochloride (20 mg, 0.28 mmol) and DIPEA (73 mg, 0.57 mmol) in DMF (0.5 mL) was added BOP (125 mg, 0.28 mmol) at 0 °C. Then the mixture was stirred at 0 °C for 2 h. The mixture was quenched with ice-water (3 mL) and extracted with EtOAc (6 mL*3). The combined organic layer was dried over anhydrous Na₂SO₄, filtered and concentrated. The residue was purified by *Prep-HPLC* (Method C) to give **46** (8.8 mg, 12% yield) as a white solid. LC-MS (ESI, Method 2) t_{R} = 0.93 min, m/z (M+H)⁺ = 510.2. ¹H NMR (400 MHz, DMSO-*d*₆) δ 11.60 (s, 1H), 10.70 (s, 1H), 9.12 (s, 1H), 8.62 (s, 1H), 8.57 (s, 1H), 7.43 (d, *J =* 8.8 Hz, 1H), 7.38 (d, *J =* 8.8 Hz, 1H), 5.32-5.24 (m, 1H), 3.95 (s, 3H), 3.89 (s, 3H), 3.31 (s, 3H), 2.08-2.00 (m, 1H), 0.85-0.77 (m, 4H).

### Step 3. (S)-6-(cyclopropanecarboxamido)-4-((4-methoxy-1-methyl-5-(2,2,2-trifluoro-1-methoxyethyl)-1H-indazol-3-yl)amino)-N-(methyl-d₃)nicotinamide (46A) and (R)-6-(cyclopropanecarboxamido)-4-((4-methoxy-1-methyl-5-(2,2,2-trifluoro-1-methoxyethyl)-1H-indazol-3-yl)amino)-N-(methyl-d₃)nicotinamide (46B)

**46** (220 mg, 0.41 mmol) was separated by chiral *prep-HPLC* (Method K) to give **46A** (102 mg, 46% yield) as a white solid and **46B** (102 mg, 46% yield) as a white solid.

**46A:** LC-MS (ESI, Method 2) t_{R} = 1.09 min, m/z (M+H)⁺ = 510.0. ¹H NMR (400 MHz, DMSO-*d*₆) δ 11.62 (s, 1H), 10.73 (s, 1H), 9.14 (s, 1H), 8.65 (s, 1H), 8.58 (s, 1H), 7.45-7.38 (m, 2H), 5.32-5.26 (m, 1H), 3.97 (s, 3H), 3.90 (s, 3H), 3.32 (s, 3H), 2.05-2.00 (m, 1H), 0.85-0.78 (m, 4H). Chiral HPLC (Method 9) t_{R} = 5.39 min.

**46B:** LC-MS (ESI, Method 2) t_{R} = 1.09 min, m/z (M+H)⁺ = 510.0. ¹H NMR (400 MHz, DMSO-*d*₆) δ 11.62 (s, 1H), 10.72 (s, 1H), 9.14 (s, 1H), 8.64 (s, 1H), 8.58 (s, 1H), 7.45-7.38 (m, 2H), 5.31-5.26 (m, 1H), 3.96 (s, 3H), 3.89 (s, 3H), 3.31 (s, 3H), 2.04-1.99 (m, 1H), 0.87-0.79 (m, 4H). Chiral HPLC (Method 9) t_{R} = 5.94 min.

### Example 47

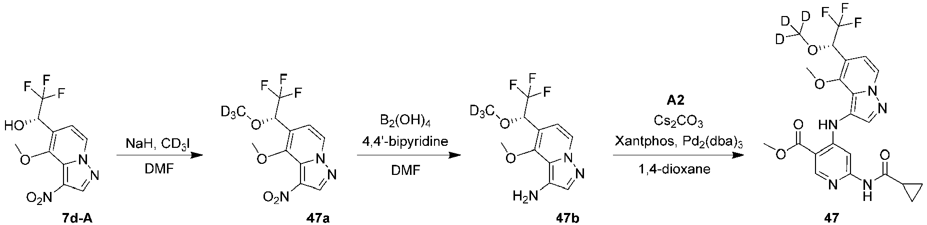

### Step 1. (S)-4-Methoxy-3-nitro-5-(2,2,2-trifluoro-1-(methoxy-d₃)ethyl)pyrazolo[1,5-a]pyridine (47a)

To a solution of **7d-A** (200 mg, 0.69 mmol) and CD₃I (498 mg, 3.43 mmol) in anhydrous DMF (2 mL) was slowly added NaH (33 mg, 0.82 mmol, 60% in mineral oil) at 0 °C and stirred at 0 °C for 30 min. The solution was poured into ice-water (5 mL) and extracted with EtOAc (5 mL*2). The combined organic phase was dried over anhydrous Na₂SO₄, filtered and concentrated. The residue was purified by flash chromatography on silica gel (PE/EtOAc = 20/1) to give **47a** (200 mg, 94% yield) as a yellow solid. LC-MS (ESI, Method 3) t_{R} = 1.27 min, m/z (M+H)⁺ = 309.3.

### Step 2. (S)-4-Methoxy-5-(2,2,2-trifluoro-1-(methoxy-d₃)ethyl)pyrazolo[1,5-a]pyridin-3-amine (47b)

To a solution of **47a** (200 mg, 0.65 mmol) and 4,4'-bipyridine (4.05 mg, 0.026 mmol) in DMF (2 mL) was slowly added B₂(OH)₄ (175 mg, 1.95 mmol) at 0 °C and the reaction was stirred at 0 °C for 30 min. The reaction mixture was purified by *Prep-HPLC* (Method A) directly to give **47b** (130 mg, 72% yield) as a brown solid. LC-MS (ESI, Method 3) t_{R} = 1.01 min, m/z (M+H)⁺ = 279.2.

### Step 3. Methyl (S)-6-(cyclopropanecarboxamido)-4-((4-methoxy-5-(2,2,2-trifluoro-1-(methoxy-d₃)ethyl)pyrazolo[1,5-a]pyridin-3-yl)amino)nicotinate (47)

A mixture of **47b** (30 mg, 0.11 mmol), **A2** (33 mg, 0.13 mmol), Cs₂CO₃ (70 mg, 0.22 mmol), Xantphos (12 mg, 0.022 mmol) and Pd₂(dba)₃ (20 mg, 0.022 mmol) in dioxane (0.4 mL) was stirred at 100 °C under N₂ for 5 h. The reaction mixture was cooled, concentrated and purified by flash chromatography on silica gel (PE/EtOAc = 3/1) to give **47** (21.5 mg, 40% yield) as a yellow solid. LC-MS (ESI, Method 2) t_{R} = 0.90 min, m/z (M+H)⁺ = 497.1. ¹H NMR (400 MHz, DMSO-*d*₆) δ 10.83 (s, 1H), 9.61 (s, 1H), 8.68 (s, 1H), 8.56 (d, *J* = 7.6 Hz, 1H), 8.17 (s, 1H), 7.67(s, 1H), 6.81 *(d, J=* 7.2 Hz, 1H), 5.29-5.26 (m, 1H), 3.89 (s, 3H), 3.71 (s, 3H), 1.95-1.92 (m, 1H), 0.75-0.67 (m, 4H).

### Example 48

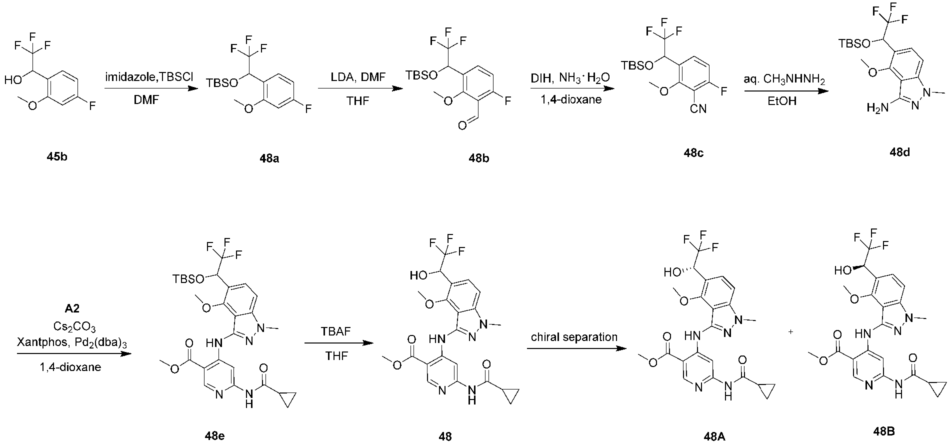

### Step 1. Tert-butyldimethyl(2,2,2-trifluoro-1-(4-fluoro-2-methoxyphenyl)ethoxy)silane (48a)

To a solution of **45b** (3 g, 13.38 mmol) and imidazole (1.37 g, 20.08 mmol) in DMF (3 mL) was added TBSCl (3.03 g, 20.08 mmol) at r.t.. After stirring at 30 °C for 3 h, the reaction was diluted with 0.2 M HCl (20 mL) and extracted with EtOAc (20 mL*2). The organic layer was washed with brine (5 mL), dried over Na₂SO₄ and filtered. The filtrate was concentrated and purified by flash chromatography on silica gel (PE/EtOAc = 50/1) to give **48a** (3.5 g, 77% yield) as a colorless liquid. ¹H NMR (400 MHz, DMSO-*d*₆) δ 7.54 (d, *J* = 8.0 Hz, 1H), 7.08 (dd, *J* = 11.2, 2.4 Hz, 1H), 7.08 (td, *J =* 8.4, 2.4 Hz, 1H), 5.53 (q, *J =* 6.8 Hz, 1H), 3.91 (s, 3H), 0.91 (s, 9H), 0.14 (s, 3H), 0.07 (s, 3H).

### Step 2. 3-(1-((Tert-butyldimethylsilyl)oxy)-2,2,2-trifluoroethyl)-6-fluoro-2-methoxybenzaldehyde (48b)

To a solution of **48a** (1 g, 2.95 mmol) in THF (10 mL) was added LDA (6 mL, 12 mmol, 2 M in THF) dropwise over 30 min at -60 °C. The reaction mixture was stirred for 1 h at the same temperature. Then DMF (0.65 g, 8.86 mmol) was added to the solution dropwise at - 60 °C. The reaction was stirred for another 2 h at -60 °C and quenched with aqueous HCl solution (10 mL, 2 M). The mixture was extracted with EtOAc (10 mL*2) and the combined organic layer was washed with brine (5 mL), dried over anhydrous Na₂SO₄, filtered and concentrated in vacuo to give **48b** (1 g, 92% yield) as a yellow oil which was used for the next step directly without further purification.

### Step 3. 3-(1-((Tert-butyldimethylsilyl)oxy)-2,2,2-trifluoroethyl)-6-fluoro-2-methoxybenzonitrile (48c)

To a solution of **48b** (200 mg, 0.55 mol) in 1,4-dioxane (1 mL) and NH₃.H₂O (1 mL) was added 1,3-diiodo-5,5-dimethylimidazolidine-2,4-dione (207 mg, 0.55 mmol) at ice-bath. Then the mixture was stirred at r.t. for 16 h. The mixture was diluted with water (4 mL) and extracted with EtOAc (8 mL). The separated organic layer was dried over anhydrous Na₂SO₄, filtered and concentrated under reduced pressure at 35 °C to give **48c** (198 mg, crude) as a yellow oil which was used for the next step directly without further purification.

### Step 4. 5-(1-((Tert-butyldimethylsilyl)oxy)-2,2,2-trifluoroethyl)-4-methoxy-1-methyl-1H-indazol-3-amine (48d)

A mixture of **48c** (198 mg, 0.55 mmol), 40% *aq.* methylhydrazine (2 mL) and EtOH (2 mL) was stirred at 90 °C for 5 h. After cooling to r.t., the reaction mixture was diluted with water (8 mL) and extracted with EtOAc (10 mL*2). The combined organic layer was dried over anhydrous Na₂SO₄, filtered and concentrated. The residue was purified by flash chromatography on silica gel (PE/EtOAc = 5/1 to 2/1) to give **48d** (70 mg, 33% yield) as a yellow oil. LC-MS (ESI, Method 3) t_{R} = 1.57 min, m/z (M+H)⁺ = 390.5.

### Step 5. Methyl 4-((5-(1-((tert-butyldimethylsilyl)oxy)-2,2,2-trifluoroethyl)-4-methoxy-1-methyl-1H-indazol-3-yl)amino)-6-(cyclopropanecarboxamido)nicotinate (48e)

A mixture of **48d** (75 mg, 0.19 mmol), **A2** (54 mg, 0.21 mmol), Pd₂(dba)₃ (35 mg, 0.039 mmol), Xantphos (22 mg, 0.039 mol) and Cs₂CO₃ (125 mg, 0.39 mmol) in 1,4-dioxane (0.5 mL) was stirred at 90 °C for 3 h under N₂. The reaction mixture was concentrated, and the residue was purified by flash chromatography on silica gel (DCM/MeOH = 20/1) to give **48e** (85 mg, 73% yield) as a yellow solid. LC-MS (ESI, Method 3) t_{R} = 1.56 min, m/z (M+H)⁺ = 608.7.

### Step 6. Methyl 6-(cyclopropanecarboxamido)-4-((4-methoxy-1-methyl-5-(2,2,2-trifluoro-1-hydroxyethyl)-1H-indazol-3-yl)amino)nicotinate (48)

To a solution of **48e** (70 mg, 0.12 mmol) in THF (1 mL) was added TBAF (1 mL, 1 M in THF) at 0 °C. Then the mixture was stirred at 30 °C for 1 h. The reaction mixture was diluted with water (3 mL) and extracted with EtOAc (6 mL*2). The combined organic layer was dried over anhydrous Na₂SO₄, filtered and concentrated. The residue was purified by flash chromatography on silica gel (DCM/MeOH = 97/3) to give **48** (40 mg, 70% yield) as a yellow solid. LC-MS (ESI, Method 3) t_{R} = 1.17 min, m/z (M+H)⁺ = 494.4. ¹H NMR (400 MHz, DMSO-*d*₆) δ 10.91 (s, 1H), 10.88 (s, 1H), 9.20 (s, 1H), 8.77 (s, 1H), 7.54 (d, *J= 8.8* Hz, 1H), 7.43 (d, *J=* 9.2 Hz, 1H), 6.87 (brs, 1H), 5.48-5.42 (m, 1H), 3.97 (s, 3H), 3.92 (s, 3H), 3.87 (s, 3H), 2.10-1.96 (m, 1H), 0.93-0.76 (m, 4H).

### Step 7. Methyl (S)-6-(cyclopropanecarboxamido)-4-((4-methoxy-1-methyl-5-(2,2,2-trifluoro-1-hydroxyethyl)-1H-indazol-3-yl)amino)nicotinate (48A) and Methyl (R)-6-(cyclopropanecarboxamido)-4-((4-methoxy-1-methyl-5-(2,2,2-trifluoro-1-hydroxyethyl)-1H-indazol-3-yl)amino)nicotinate (48B)

**48** (15 mg, 0.03 mmol) was separated by chiral *prep-HPLC* (Method G, Hex/EtOH = 60/40) to afford **48A** (5.3 mg, 36% yield) as a white solid and **48B** (5.6 mg, 38% yield) as a white solid.

**48A:** LC-MS (ESI, Method 2) t_{R} = 0.89 min, m/z (M+H)⁺ = 494.1. ¹H NMR (400 MHz, DMSO-*d*₆) δ 10.91 (s, 1H), 10.88 (s, 1H), 9.20 (s, 1H), 8.77 (s, 1H), 7.54 (d, *J=* 8.8 Hz, 1H), 7.43 (d, *J= 8.8* Hz, 1H), 6.84 (d, *J=* 5.2 Hz, 1H), 5.52-5.39 (m, 1H), 3.97 (s, 3H), 3.92 (s, 3H), 3.87 (s, 3H), 2.07-2.01 (m, 1H), 0.87-0.79 (m, 4H). Chiral HPLC (Method 8, Hex\EtOH = 60\40) t_{R} = 5.08 min.

**48B:** LC-MS (ESI, Method 2) t_{R} = 0.88 min, m/z (M+H)⁺ = 494.1. ¹H NMR (400 MHz, DMSO-*d*₆) δ 10.91 (s, 1H), 10.88 (s, 1H), 9.20 (s, 1H), 8.77 (s, 1H), 7.54 *(d, J=* 8.8 Hz, 1H), 7.43 (d, *J= 8.8* Hz, 1H), 6.84 (d, *J=* 5.6 Hz, 1H), 5.51-5.38 (m, 1H), 3.97 (s, 3H), 3.92 (s, 3H), 3.87 (s, 3H), 2.07-2.01 (m, 1H), 0.87-0.79 (m, 4H). Chiral HPLC (Method 8, Hex\EtOH = 60\40) t_{R} = 6.87 min.

### Example 49

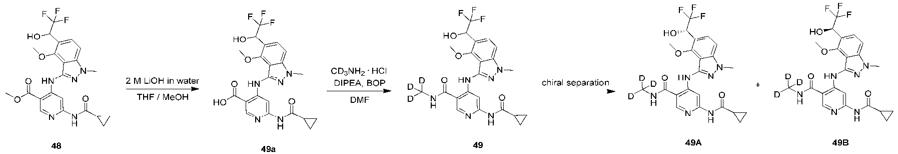

### Step 1. 6-(Cyclopropanecarboxamido)-4-((4-methoxy-1-methyl-5-(2,2,2-trifluoro-1-hydroxyethyl)-1H-indazol-3-yl)amino)nicotinic acid (49a)

To a solution of **48** (38 mg, 0.077 mmol) in THF (0.5 mL) and MeOH (0.5 mL) was added LiOH in water (0.25 mL, 2 M) under ice-bath. Then the mixture was stirred at 30 °C for 2 h. The mixture was adjusted to pH < 7 with 2 N HCl and extracted with EtOAc (8 mL*2). The combined organic layer was dried over anhydrous Na₂SO₄, filtered and concentrated to give **49a** (36 mg, crude) as a yellow solid which was used for the next step directly without further purification. LC-MS (ESI, Method 3) t_{R} = 1.12 min, m/z (M+H)⁺ = 480.4.

### Step 2. 6-(Cyclopropanecarboxamido)-4-((4-methoxy-1-methyl-5-(2,2,2-tritluoro-1-hydroxyethyl)-1H-indazol-3-yl)amino)-N-(methyl-d₃)nicotinamide (49)

To a solution of **49a** (35 mg, 0.073 mmol), trideuteriomethanamine hydrochloride (10 mg, 0.15 mmol) and DIPEA (38 mg, 0.29 mmol) in DMF (0.5 mL) was added BOP (65 mg, 0.15 mmol) at 0 °C. Then the mixture was stirred at 0 °C for 2 h. The mixture was quenched with ice-water (4 mL) and extracted with EtOAc (7 mL*3). The combined organic layer was dried over anhydrous Na₂SO₄, filtered and concentrated. The residue was purified by *Prep-HPLC* (Method A) to give **49** (7.3 mg, 20% yield) as a white solid. LC-MS (ESI, Method 2) t_{R} = 0.84 min, m/z (M+H)⁺ = 496.1. ¹H NMR (400 MHz, DMSO-*d*₆) δ 11.58 (s, 1H), 10.69 (s, 1H), 9.14 (s, 1H), 8.61 (s, 1H), 8.56 (s, 1H), 7.51 (d, *J* = 8.8 Hz, 1H), 7.39 (d, *J* = 8.8 Hz, 1H), 6.81 (d, *J=* 6.0 Hz, 1H), 5.46-5.41 (m, 1H), 3.94 (s, 3H), 3.87 (s, 3H), 2.06-1.95 (m, 1H), 0.92-0.69 (m, 4H).

### Step 3. (S)-6-(cyclopropanecarboxamido)-4-((4-methoxy-1-methyl-5-(2,2,2-trifluoro-1-hydroxyethyl)-1H-indazol-3-yl)amino)-N-(methyl-d₃)nicotinamide (49A) and (R)-6-(cyclopropanecarboxamido)-4-((4-methoxy-1-methyl-5-(2,2,2-trifluoro-1-hydroxyethyl)-1H-indazol-3-yl)amino)-N-(methyl-d₃)nicotinamide (49B)

**49** (135 mg, 0.27 mol) was separated by chiral *prep-HPLC* (Method I, Hex/IPA/DEA = 60/40/0.3) to afford **49A** (40 mg, 30% yield) as a white solid and **49B** (45.4 mg, 34% yield) as a white solid.

**49A:** LC-MS (Method 2) t_{R} = 0.83 min, m/z (M+H)⁺ = 496.1. ¹H NMR (400 MHz, DMSO-*d*₆) δ 11.58 (s, 1H), 10.69 (s, 1H), 9.14 (s, 1H), 8.61 (s, 1H), 8.56 (s, 1H), 7.51 *(d, J=* 8.8 Hz, 1H), 7.39 (d, *J= 8.8* Hz, 1H), 6.83 (d, *J=* 5.2 Hz, 1H), 5.46-5.41 (m, 1H), 3.94 (s, 3H), 3.87 (s, 3H), 2.07-1.97 (m, 1H), 0.89-0.75 (m, 4H). Chiral HPLC (Method 6, Hex/IPA/DEA = 60/40/0.2) t_{R} = 9.08 min.

**49B:** LC-MS (Method 2) t_{R} = 0.84 min, m/z (M+H)⁺ = 496.1. ¹H NMR (400 MHz, DMSO-*d*₆) δ 11.58 (s, 1H), 10.69 (s, 1H), 9.14 (s, 1H), 8.61 (s, 1H), 8.56 (s, 1H), 7.52 *(d, J=* 7.6 Hz, 1H), 7.39 (d, *J* = 8.0 Hz, 1H), 6.83 (d, *J* = 5.2 Hz, 1H), 5.57-5.31 (m, 1H), 3.95 (s, 3H), 3.87 (s, 3H), 2.12-1.91 (m, 1H), 0.99-0.72 (m, 4H). Chiral HPLC (Method 6, Hex/IPA/DEA = 60/40/0.2) t_{R} = 12.84 min.

### Example 50

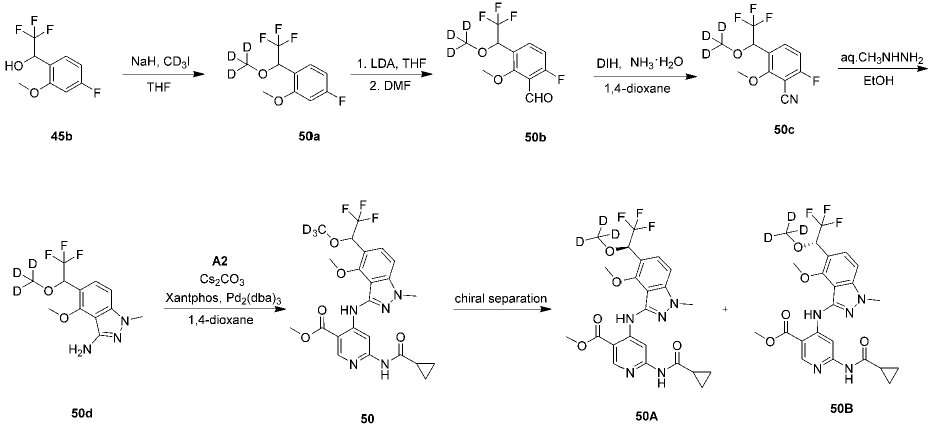

### Step 1. 4-Fluoro-2-methoxy-1-(2,2,2-trifluoro-1-(methoxy-d₃)ethyl)benzene (50a)

To a solution of **45b** (5 g, 22.31 mmol) in THF (50 mL) was added NaH (1.07 g, 26.77 mmol, 60% in mineral oil) at 0 °C. After stirring at 0 °C for 30 min, CD₃I (9.70 g, 66.92 mmol) was added to it. The reaction was stirred at 0 °C for 1 h and stirred at 30 °C for 3 h. The solution was poured into ice-water (30 mL) and extracted with EtOAc (40 mL*2). The combined organic phase was washed with brine (15 mL), dried with anhydrous Na₂SO₄, filtered and concentrated. The residue was purified by flash chromatography on silica gel (PE/EtOAc = 30/1) to give **50a** (5 g, 93% yield) as a yellow oil. ¹H NMR (400 MHz, CDCl₃) δ 7.50-7.46 (m, 1H), 6.7-6.73 (m, 1H), 6.69-6.65 (m, 1H), 5.12-5.07 (m, 1H), 3.86 (s, 3H).

### Step 2. 4-Fluoro-2-methoxy-1-(2,2,2-trifluoro-1-(methoxy-d₃)ethyl)benzene (50b)

To a solution of **50a** (2.5 g, 10.36 mmol) in THF (20 mL) was added LDA (15 mL, 30 mmol, 2 M in THF) dropwise at -50 °C. The mixture was stirred at -50 °C for 1 h. Then DMF (2.41 mL, 31.09 mmol) was added to the mixture at -50 °C and stirred at -50 °C for 1 h. The reaction was quenched with *sat.* NH₄Cl (25 mL) and extracted with EtOAc (50 mL*3). The combined organic layer was dried over anhydrous Na₂SO₄, filtered and concentrated under reduced pressure at 35 °C to give **50b** (2 g, crude) as a yellow oil, which was used for the next step directly without further purification.

### Step 3. 6-Fluoro-2-methoxy-3-(2,2,2-trifluoro-1-(methoxy-d₃)ethyl)benzonitrile (50c)

To a solution of **50b** (400 mg, 1.49 mmol) in NH₃.H₂O (2 mL) and 1,4-dioxane (2 mL) was added 1,3-diiodo-5,5-dimethylimidazolidine-2,4-dione (567 mg, 1.49 mmol) at ice-bath. Then the mixture was stirred at 30 °C for 12 h. TLC showed the starting material was disappeared. The mixture was diluted with water (3 mL), extracted with EtOAc (10 mL*2). The organic layer was dried over anhydrous Na₂SO₄, filtered and concentrated under reduced pressure at 35 °C to give **50c** (1.6 g, crude) as a yellow liquid, which was used for the next step directly without further purification.

### Step 4. 4-Methoxy-1-methyl-5-(2,2,2-trifluoro-1-(methoxy-d₃)ethyl)-1H-indazol-3-amine (50d)

A mixture of **50c** (300 mg, 1.13 mmol) and 40% *aq.* methylhydrazine (1.5 mL) in EtOH (1.5 mL) was stirred at 90 °C for 12 h. After cooling to r.t., the mixture was diluted with water (3 mL) and extracted with EtOAc (10 mL*2). The combined organic layer was dried over anhydrous Na₂SO₄, filtered and concentrated. The residue was purified by flash chromatography on silica gel (PE/EtOAc = 5/1) to give **50d** (150 mg, 46% yield) as a yellow solid. LC-MS (ESI, Method 3) t_{R} = 1.20 min, m/z (M+H)⁺ = 293.4.

### Step 5. Methyl 6-(cyclopropanecarboxamido)-4-((4-methoxy-1-methyl-5-(2,2,2-trifluoro-1-(methoxy-d₃)ethyl)-1H-indazol-3-yl)amino)nicotinate (50)

A mixture of **50d** (150 mg, 0.51 mmol), **A2** (131 mg, 0.51 mmol), Pd₂(dba)₃ (94 mg, 0.10 mmol), Xantphos (60 mg, 0.10 mmol) and Cs₂CO₃ (335 mg, 1.03 mmol) in 1,4-dioxane (2 mL) was stirred at 90 °C for 12 h under N₂. The reaction mixture was concentrated, and the residue was purified by flash chromatography on silica gel (PE/EtOAc = 2/1) to give **50** (70 mg, 27% yield) as a yellow solid. LC-MS (ESI, Method 3) t_{R} = 1.28 min, m/z (M+H)⁺ = 511.5.

### Step 6. Methyl (R)-6-(cyclopropanecarboxamido)-4-((4-methoxy-1-methyl-5-(2,2,2-trifluoro-1-(methoxy-d3)ethyl)-1H-indazol-3-yl)amino)nicotinate (50A) and Methyl (S)-6-(cyclopropanecarboxamido)-4-((4-methoxy-1-methyl-5-(2,2,2-trifluoro-1-(methoxy-d3)ethyl)-1H-indazol-3-yl)amino)nicotinate (50B)

**50** (70 mg, 0.14 mmol) was separated by chiral *prep-HPLC* (Method G, Hex\EtOH = 80\20) to give **50A** (26 mg, 37% yield) as a white solid and **50B** (25 mg, 36% yield) as a white solid.

**50A:** LC-MS (ESI, Method 2) t_{R} = 1.13 min, m/z (M+H)⁺ = 511.0. ¹H NMR (400 MHz, DMSO-*d*₆) δ 10.92 (s, 1H), 10.91 (s, 1H), 9.18 (s, 1H), 8.77 (s, 1H), 7.48 *(d, J=* 8.8 Hz, 1H), 7.41 (d, *J= 8.8* Hz, 1H), 5.30-5.27 (m, 1H), 3.98 (s, 3H), 3.92 (s, 3H), 3.89 (s, 3H), 2.05-1.98 (m, 1H), 0.84-0.80 (m, 4H). Chiral HPLC (Method 8, Hex/IPA = 80/20) t_{R} = 12.51 min.

**50B:** LC-MS (ESI, Method 2) t_{R} = 1.13 min, m/z (M+H)⁺ = 511.0 . ¹H NMR (400 MHz, DMSO-*d*₆) δ 10.92 (s, 1H), 10.91 (s, 1H), 9.18 (s, 1H), 8.77 (s, 1H), 7.48 (d, *J=* 8.8 Hz, 1H), 7.41 (d, *J= 8.8* Hz, 1H), 5.30-5.27 (m, 1H), 3.98 (s, 3H), 3.92 (s, 3H), 3.88 (s, 3H), 2.05-2.02 (m, 1H), 0.84-0.80 (m, 4H). Chiral HPLC (Method 8, Hex/IPA = 80/20) t_{R} = 16.16 min.

### Example 51

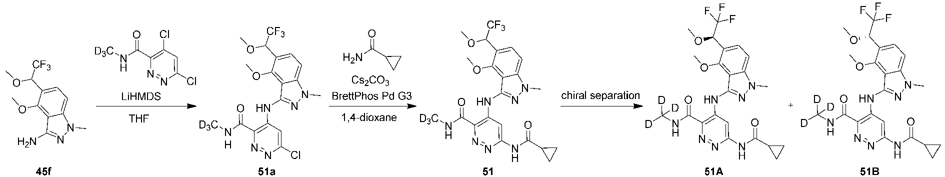

### Step 1. 6-Chloro-4-((4-methoxy-1-methyl-5-(2,2,2-trifluoro-1-methoxyethyl)-1H-indazol-3-yl)amino)-N-(methyl-d3)pyridazine-3-carboxamide (51a)

To a solution of **45f** (100 mg, 0.35 mmol) and 4,6-dichloro-*N*-(methyl-*d*₃)pyridazine-3-carboxamide (87 mg, 0.41 mmol) in THF (0.8 mL) was added LiHMDS (1.4 mL, 1.40 mmol, 1 M in THF) at -40 °C. The reaction was stirred at -40 °C to 25 °C for 2 h. The mixture was quenched with H₂O (2 mL) and the organic solvent was evaporated. The formed solid was filtered and the filter cake was dried to give **51a** (100 mg, 63% yield) as a white solid. LC-MS (ESI, Method 3) t_{R} = 1.48 min, m/z (M+H)⁺ = 462.3.

### Step 2. 6-(Cyclopropanecarboxamido)-4-((4-methoxy-1-methyl-5-(2,2,2-tritluoro-1-methoxyethyl)-1H-indazol-3-yl)amino)-N-(methyl-d3)pyridazine-3-carboxamide (51)

A mixture of **51a** (100 mg, 0.22 mmol), cyclopropanecarboxamide (91 mg, 1.08 mmol), BrettPhos Pd G3 (39 mg, 0.043 mmol) and Cs₂CO₃ (141 mg, 0.43 mol) in 1,4-dioxane (1.2 mL) was stirred at 90 °C for 5 h under N₂ atmosphere. The reaction mixture was concentrated, and the residue was purified by flash chromatography on silica gel (DCM/MeOH = 20/1) to afford **51** (80 mg, 72% yield) as a white solid. LC-MS (ESI, Method 3) t_{R} = 1.31 min, m/z (M+H)⁺ = 511.5.

### Step 3. (R)-6-(cyclopropanecarboxamido)-4-((4-methoxy-1-methyl-5-(2,2,2-trifluoro-1-methoxyethyl)-1H-indazol-3-yl)amino)-N-(methyl-d3)pyridazine-3-carboxamide (51A) and (S)-6-(cyclopropanecarboxamido)-4-((4-methoxy-1-methyl-5-(2,2,2-trifluoro-1-methoxyethyl)-1H-indazol-3-yl)amino)-N-(methyl-d3)pyridazine-3-carboxamide (51B)

**51** (80 mg, 0.16 mmol) was separated by chiral prep-HPLC (Method L) to afford **51A** (20 mg, 25% yield) as a white solid and **51B** (12 mg, 15% yield) as a white solid.

**51A:** LC-MS (ESI, Method 2) t_{R} = 1.16 min, m/z (M+H)⁺ = 511.0. ¹H NMR (400 MHz, DMSO-*d*₆) δ 12.04 (s, 1H), 11.30 (s, 1H), 9.44 (s, 1H), 9.20 (s, 1H), 7.48-7.41 (m, 2H), 5.32-5.29 (m, 1H), 3.98 (s, 3H), 3.94 (s, 3H), 3.32 (s, 3H), 2.14-2.10 (m, 1H), 0.90-0.87 (m, 4H). Chiral HPLC (Method 8, Hex\EtOH = 90\10) t_{R} = 8.65 min.

**51B:** LC-MS (ESI, Method 2) t_{R} = 1.00 min, m/z (M+H)⁺ = 511.2. ¹H NMR (400 MHz, DMSO-*d*₆) δ 12.04 (s, 1H), 11.30 (s, 1H), 9.44 (s, 1H), 9.21 (s, 1H), 7.48-7.41 (m, 2H), 5.33-5.27 (m, 1H), 3.98 (s, 3H), 3.94 (s, 3H), 3.34 (s, 3H), 2.17-2.11 (m, 1H), 0.92-0.84 (m, 4H). Chiral HPLC (Method 8, Hex\EtOH = 90\10) t_{R} = 10.33 min.

### Example 52

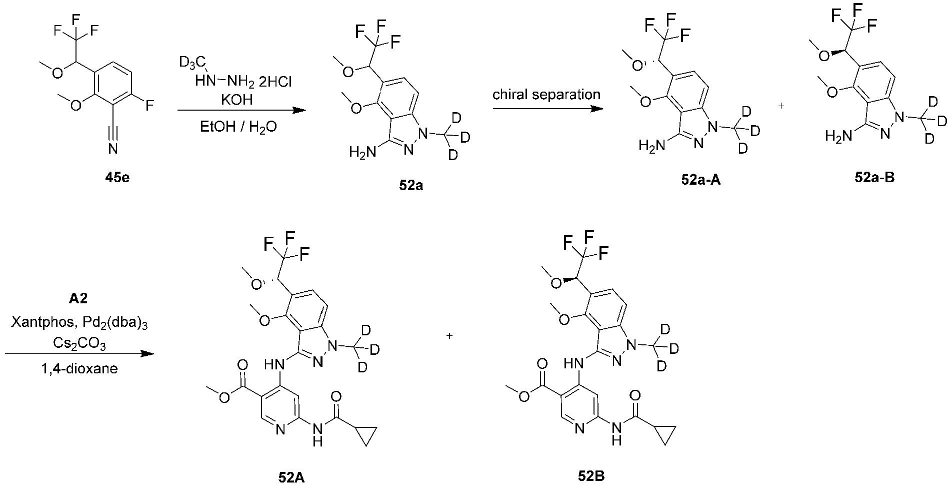

### Step 1. 4-Methoxy-1-(methyl-d3)-5-(2,2,2-trifluoro-1-methoxyethyl)-1H-indazol-3-amine (52a)

To a mixture of methyl-*d*₃-hydrazine hydrochloride (1:2) (1.50 g, 12.30 mmol) in EtOH/H₂O (0.5 mL/0.1 mL) was added KOH (1.34 g, 23.94 mmol) at ice-bath. The reaction was stirred at 30 °C for 30 min. The solid was filtered off. The filtrate was added into **45e** (175 mg, 0.66 mmol) and was stirred at 90 °C for 2 h in a sealed tube. After cooling to r.t., the mixture was purified by *Prep-HPLC* (Method C, HCOOH) to give compound **52a** (58 mg, 29.8% yield) as a yellow solid. ¹H NMR (400 MHz, DMSO-*d*₆) δ 7.23 (d, *J=* 8.8 Hz, 1H), 7.19 (d, *J=* 8.8 Hz, 1H), 5.22 (s, 2H), 5.21-5.15 (m, 1H), 3.87 (s, 3H), 3.28 (s, 3H).

### Step 2. (S)-4-methoxy-1-(methyl-d3)-5-(2,2,2-trifluoro-1-methoxyethyl)-1H-indazol-3-amine (52a-A) and (R)-4-methoxy-1-(methyl-d3)-5-(2,2,2-trifluoro-1-methoxyethyl)-1H-indazol-3-amine (52a-B)

**52a** (150 mg, 0.51 mmol) was separated by chiral *prep-HPLC* (Method G, Hex/EtOH = 90/10, 20 mL/min) to afford **52a-A** (64 mg, 43% yield) as a white solid and **52a-B** (65 mg, 43% yield) as a white solid.

**52a-A:** Chiral HPLC (Method 8, Hex\EtOH = 90\10) t_{R} = 8.63 min.

**52a-B:** Chiral HPLC (Method 8, Hex\EtOH = 90\10) t_{R} = 10.32 min.

### Step 3. Methyl (S)-6-(cyclopropanecarboxamido)-4-((4-methoxy-1-(methyl-d3)-5-(2,2,2-trifluoro-1-methoxyethyl)-1H-indazol-3-yl)amino)nicotinate (52A) and Methyl (R)-6-(cyclopropanecarboxamido)-4-((4-methoxy-1-(methyl-d3)-5-(2,2,2-trifluoro-1-methoxyethyl)-1H-indazol-3-yl)amino)nicotinate (52B)

A mixture of **52a-A** (30 mg, 0.10 mmol), **A2** (26 mg, 0.10 mmol), Pd₂(dba)₃ (19 mg, 0.021 mmol), Xantphos (12 mg, 0.021 mmol) and Cs₂CO₃ (67 mg, 0.21 mmol) in 1,4-dioxane (0.5 mL) was stirred at 90 °C for 4 h under N₂. The reaction mixture was concentrated, and the residue was purified by flash chromatography on silica gel (DCM/MeOH = 20/1) to afford the crude product. Then the crude compound was re-purified by *Prep-HPLC* (Method A) to afford **52A** (24.2 mg, 46% yield) as a white solid. LC-MS (ESI, Method 2) t_{R} = 0.97 min, m/z (M+H)⁺ = 511.1. ¹H NMR (400 MHz, DMSO-*d*₆) δ 10.91 (s, 1H), 10.89 (s, 1H), 9.17 (s, 1H), 8.78 (s, 1H), 7.49-7.40 (m, 2H), 5.32-5.27 (m, 1H), 3.93 (s, 3H), 3.89 (s, 3H), 3.31 (s, 3H), 2.09-1.99 (m, 1H), 0.88-0.76 (m, 4H). Chiral HPLC (Method 7, Hex\IPA = 85\15 run time of 40 minutes) t_{R} = 21.74 min.

A mixture of **52a-B** (30 mg, 0.10 mmol), **A2** (29 mg, 0.11 mmol), Pd₂(dba)₃ (19 mg, 0.021 mmol), Xantphos (12 mg, 0.021 mmol) and Cs₂CO₃ (67 mg, 0.21 mmol) in 1,4-dioxane (0.5 mL) was stirred at 90 °C for 4 h under N₂. The reaction mixture was concentrated and the residue was purified by flash chromatography on silica gel (DCM/MeOH = 20/1) to afford the crude product. Then the crude compound was re-purified by *Prep-HPLC* (Method A) to afford **52B** (13.5 mg, 26% yield) as a white solid. LC-MS (ESI, Method 2) t_{R} = 0.97 min, m/z (M+H)⁺ = 511.1. ¹H NMR (400 MHz, DMSO-*d*₆) δ 10.91 (s, 1H), 10.90 (s, 1H), 9.18 (s, 1H), 8.78 (s, 1H), 7.49-7.40 (m, 2H), 5.33-5.27 (m, 1H), 3.93 (s, 3H), 3.89 (s, 3H), 3.33 (s, 3H), 2.04-1.98 (m, 1H), 0.83-0.76 (m, 4H). Chiral HPLC (Method 7, Hex\IPA = 85\15 run time of 40 minutes) t_{R} = 16.91 min.

### Example 53

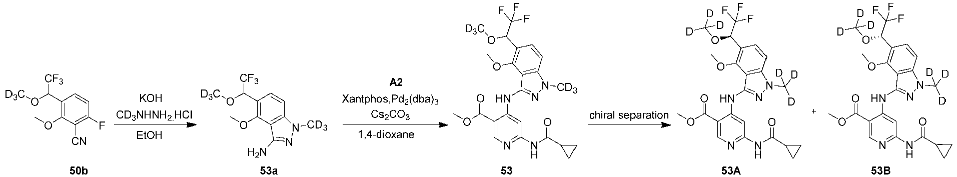

### Step 1. 4-(Methoxy-d3)-1-methyl-5-(2,2,2-trifluoro-1-(methoxy-d3))-1H-indazol-3-amine 4-Fluoro-2-methoxy-1-(2,2,2-trifluoro-1-(methoxy-d3)ethyl)benzene (53a)

To a solution of CD₃NHNH₂.HCl (700 mg, 5.74 mmol) in EtOH/H₂O (0.5 mL/0.1 mL) was added KOH (600 mg, 10.69 mmol) under ice-bath and the solution was stirred at 30 °C for 30 min. The formed solid was filtered off. The filtrate was added into **50b** (500 mg, 1.88 mmol). The reaction mixture was stirred at 90 °C for 6 h in a sealed tube. The mixture was concentrated, and the residue was purified by flash chromatography on silica gel (PE/EtOAc = 2/1) to give the crude compound. The crude compound was re-purified by *Prep-HPLC* (Method C) to give **53a** (80 mg, 15% yield) as a yellow oil. ¹H NMR (400 MHz, CDCl₃) δ 7.46 (d, *J* = 8.8 Hz, 1H), 7.03 (d, *J= 8.8* Hz, 1H), 5.13-5.08 (m, 1H), 3.99 (s, 3H).

### Step 2. Methyl 6-(cyclopropanecarboxamido)-4-((4-(methoxy-d3)-1-methyl-5-(2,2,2-trifluoro-1-(methoxy-d3))-1H-indazol-3-yl)amino)nicotinate (53)

A mixture of **53a** (35 mg, 0.12 mmol), **A2** (36 mg, 0.14 mmol), Pd₂(dba)₃ (22 mg, 0.02 mmol), Xantphos (14 mg, 0.02 mmol) and Cs₂CO₃ (77 mg, 0.23 mmol) in 1,4-dioxane (0.5 mL) was stirred at 90 °C for 4 h under N₂. The reaction mixture was concentrated, and the residue was purified by flash chromatography on silica gel (DCM/MeOH = 19/1) to give the crude product. The crude product was re-purified by *Prep-HPLC* (Method C) to give **53** (39 mg, 57% yield) as a white solid. LC-MS (ESI, Method 3) t_{R} = 1.30 min, m/z (M+H)⁺ = 514.4.

### Step 3. Methyl (R)-6-(cyclopropanecarboxamido)-4-((4-methoxy-1-(methyl-d3)-5-(2,2,2-trifluoro-1-(methoxy-d3)ethyl)-1H-indazol-3-yl)amino)nicotinate (53A) and Methyl (S)-6-(cyclopropanecarboxamido)-4-((4-methoxy-1-(methyl-d3)-5-(2,2,2-trifluoro-1-(methoxy-d3)ethyl)-1H-indazol-3-yl)amino)nicotinate (53B)

**53** (39 mg, 0.07 mmol) was separated by chiral *prep-HPLC* (Method G, Hex/IPA = 80/20) to give **53A** (13.2 mg, 34% yield) as a white solid and **53B** (11.6 mg, 30% yield) as a white solid.

**53A:** LC-MS (ESI, Method 2) t_{R} = 0.96 min, m/z (M+H)⁺ = 514.2. ¹H NMR (400 MHz, DMSO-*d*₆) δ 10.90 (s, 1H), 10.89 (s, 1H), 9.17 (s, 1H), 8.77 (s, 1H), 7.47 (d, *J=* 8.8 Hz, 1H), 7.41 (d, *J= 8.8* Hz, 1H), 5.31-5.26 (m, 1H), 3.92 (s, 3H), 3.89 (s, 3H), 2.08-1.99 (m, 1H), 0.87-0.79 (m, 4H). Chiral HPLC (Method 8, Hex/IPA = 80/20) t_{R} = 12.46 min.

**53B:** LC-MS (ESI, Method 2) t_{R} = 0.96 min, m/z (M+H)⁺ = 514.2. ¹H NMR (400 MHz, DMSO-*d*₆) δ 10.99 (s, 1H), 10.94 (s, 1H), 9.12 (s, 1H), 8.77 (s, 1H), 7.48 *(d, J=* 8.8 Hz, 1H), 7.42 *(d, J=* 8.8 Hz, 1H), 5.31-5.26 (m, 1H), 3.92 (s, 3H), 3.89 (s, 3H), 2.05-1.99 (m, 1H), 0.84-0.78 (m, 4H). Chiral HPLC (Method 8, Hex/IPA = 80/20) t_{R} = 16.09 min.

### Example 54

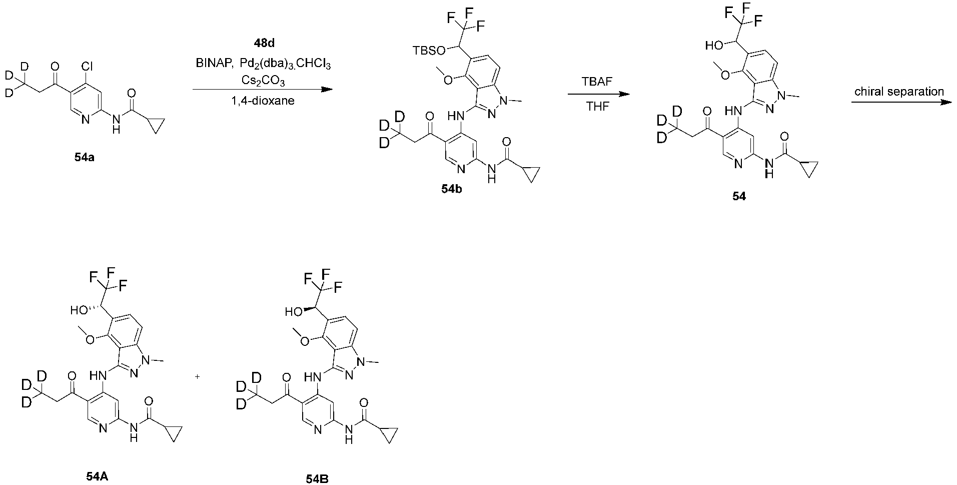

### Step 1. N-(4-((5-(1-((tert-butyldimethylsilyl)oxy)-2,2,2-trifluoroethyl)-4-methoxy-1-methyl-1H-indazol-3-yl)amino)-5-(propanoyl-3,3,3-d3)pyridin-2-yl)cyclopropanecarboxamide (54b)

A mixture of **54a** (60 mg, 0.235 mmol, WO2020086616, P246), **48d** (110 mg, 0.281 mmol), Cs₂CO₃ (153 mg, 0.469 mmol), Pd₂(dba)₃.CHCl₃ (24 mg, 0.023 mmol) and BINAP (15 mg, 0.0241 mmol) in 1,4-dioxane (1 mL) was stirred for 4 h at 110 °C under N₂. The mixture was concentrated and the crude product was purified by flash chromatography on silica gel (PE/EtOAc = 3/1) to afford **54b** (80 mg, 56% yield) as a yellow solid. LC-MS (ESI, Method 3) t_{R} = 1.66 min, m/z (M+H)⁺ = 609.6.

### Step 2. N-(4-((4-methoxy-1-methyl-5-(2,2,2-trifluoro-1-hydroxyethyl)-1H-indazol-3-yl)amino)-5-(propanoyl-3,3,3-d3)pyridin-2-yl)cyclopropanecarboxamide (54)

To a solution of **54b** (80 mg, 0.13 mmol) in THF (1 mL) was added TBAF (0.1 mL, 1.0 M in THF) at 0 °C. After stirring at 25 °C for 1 h, the reaction mixture was concentrated and the residue was purified by *Prep-HPLC* (Method C) to afford **54** (30 mg, 46% yield) as a green solid. LC-MS (ESI, Method 2) t_{R} = 0.90 min, m/z (M+H)⁺ = 495.1. ¹H NMR (400 MHz, DMSO-*d*₆) δ 11.98 (s, 1H), 10.87 (s,1H), 9.21 (s, 1H), 8.92 (s, 1H), 7.54 (d, *J=* 8.8 Hz, 1H), 7.42 (d, *J=* 8.8 Hz, 1H), 6.83 (brs, 1H), 5.49-5.43 (m, 1H), 3.96 (s, 3H), 3.87 (s, 3H), 3.13 (s, 2H), 2.09-2.03 (m, 1H), 0.87-0.79 (m, 4H).

### Step 3. (S)-N-(4-((4-methoxy-1-methyl-5-(2,2,2-trifluoro-1-hydroxyethyl)-1H-indazol-3-yl)amino)-5-(propanoyl-3,3,3-d3)pyridin-2-yl)cyclopropanecarboxamide (54A) and (R)-N-(4-((4-methoxy-1-methyl-5-(2,2,2-trifluoro-1-hydroxyethyl)-1H-indazol-3-yl)amino)-5-(propanoyl-3,3,3-d3)pyridin-2-yl)cyclopropanecarboxamide (54B)

**54** (28 mg, 0.05 mmol) was separated by chiral *prep-HPLC* (Method G) to afford **54A** (12.0 mg, 43% yield) as a green solid and **54B** (12.0 mg, 43% yield) as a green solid.

**54A:** LC-MS (ESI, Method 2) t_{R} = 0.91 min, m/z (M+H)⁺ = 495.1. ¹H NMR (400 MHz, DMSO-*d*₆) δ 11.98 (s, 1H), 10.87 (s, 1H), 9.21 (s, 1H), 8.92 (s, 1H), 7.54 *(d, J=* 8.8 Hz, 1H), 7.42 (d, *J* = 8.8 Hz, 1H), 6.84 (d, *J* = 6.0 Hz, 1H), 5.48-5.44 (m, 1H), 3.96 (s, 3H), 3.87 (s, 3H), 3.13 (s, 2H), 2.08-2.04 (m, 1H), 0.85-0.81 (m, 4H). Chiral HPLC (Method 8) t_{R} = 8.35 min.

**54B:** LC-MS (ESI, Method 2) t_{R} = 0.90 min, m/z (M+H)⁺ = 495.1. ¹H NMR (400 MHz, DMSO-*d*₆) δ 11.99 (s, 1H), 10.87 (s,1H), 9.21 (s, 1H), 8.92 (s, 1H), 7.54 (d, *J=* 8.8 Hz, 1H), 7.42 (d, *J= 8.8* Hz, 1H), 6.84 (d, *J=* 6.0 Hz, 1H), 5.48-5.44 (m, 1H), 3.96 (s, 3H), 3.87 (s, 3H), 3.13 (s, 2H), 2.08-2.04 (m, 1H), 0.85-0.81 (m, 4H). Chiral HPLC (Method 8) t_{R} = 12.07 min.

### Example 55

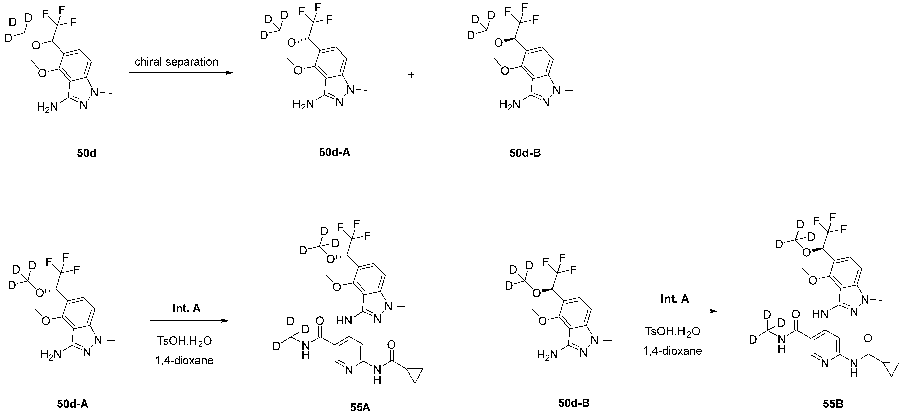

### Step 1. (S)-4-methoxy-1-methyl-5-(2,2,2-trifluoro-1-(methyl-d3))-1H-indazol-3-amine (50d-A) and (R)-4-methoxy-1-methyl-5-(2,2,2-trifluoro-1-(methyl-d3))-1H-indazol-3-amine (50d-B)

**50d** (320 mg, 1.09 mmol) was separated by chiral *prep-HPLC* (Method G, Hex/EtOH = 90/10) to afford **50d-A** (130 mg, 41% yield) as a white solid, Chiral HPLC (Method 8, Hex\EtOH = 90\10) t_{R} = 8.69 min; and **50d-B** (130 mg, 41% yield) as a white solid, Chiral HPLC (Method 8, Hex\EtOH = 90\10) t_{R} = 10.39 min.

### Step 2. (S)-6-(cyclopropanecarboxamido)-4-((4-methoxy-1-methyl-5-(2,2,2-trifluoro-1-(methoxy-d3)ethyl)-1H-indazol-3-yl)amino)-N-(methyl-d3)nicotinamide (55A)

A mixture of **50d-A** (120 mg, 0.41 mmol), **Int. A** (137 mg, 0.53 mmol) and TsOH.H₂O (8 mg, 0.041 mmol) in 1,4-dioxane (1.5 mL) was stirred at 100 °C for 72 h under N₂. The reaction mixture was concentrated, and the residue was purified by flash chromatography on silica gel (DCM/MeOH = 20/1) to afford **55A** (24.2 mg, 11.5% yield) as a yellow solid. LC-MS (ESI, Method 1) t_{R} = 1.29 min, m/z (M+H)⁺ = 513.1. ¹H NMR (400 MHz, DMSO-*d*₆) δ 11.69 (s, 1H), 10.97 (s, 1H), 9.02 (s, 1H), 8.75 (s, 1H), 8.56 (s, 1H), 7.46 (d, *J= 8.8* Hz, 1H), 7.40 (d, *J=* 8.8 Hz, 1H), 5.31-5.26 (m, 1H), 3.97 (s, 3H), 3.90 (s, 3H), 2.05-1.99 (m, 1H), 0.85-0.80 (m, 4H). Chiral HPLC (Method 5, Hex/EtOH/DEA = 60/40/0.2) t_{R} = 7.45 min.

### Step 3. (R)-6-(cyclopropanecarboxamido)-4-((4-methoxy-1-methyl-5-(2,2,2-trifluoro-1-(methoxy-d3)ethyl)-1H-indazol-3-yl)amino)-N-(methyl-d3)nicotinamide (55B)

A mixture of **50d-B** (120 mg, 0.41 mmol), **Int. A** (137 mg, 0.53 mmol) and TsOH.H₂O (8 mg, 0.041 mmol) in 1,4-dioxane (3 mL) was stirred at 100 °C for 96 h under N₂. The reaction mixture was concentrated, and the residue was purified by flash chromatography on silica gel (DCM/MeOH = 20/1) to afford **55B** (28.5 mg, 13.6% yield) as a yellow solid. LC-MS (ESI, Method 1) t_{R} = 1.29 min, m/z (M+H)⁺ = 513.1. ¹H NMR (400 MHz, DMSO-*d*₆) δ 11.71 (s, 1H), 10.98 (s, 1H), 9.02 (s, 1H), 8.75 (s, 1H), 8.56 (s, 1H), 7.46 (d, *J=* 8.8 Hz, 1H), 7.40 (d, *J= 8.8* Hz, 1H), 5.31-5.26 (m, 1H), 3.98 (s, 3H), 3.90 (s, 3H), 2.05-1.99 (m, 1H), 0.85-0.80 (m, 4H). Chiral HPLC (Method 5, Hex/EtOH/DEA = 60/40/0.2) t_{R} = 8.07 min.

### Example 56

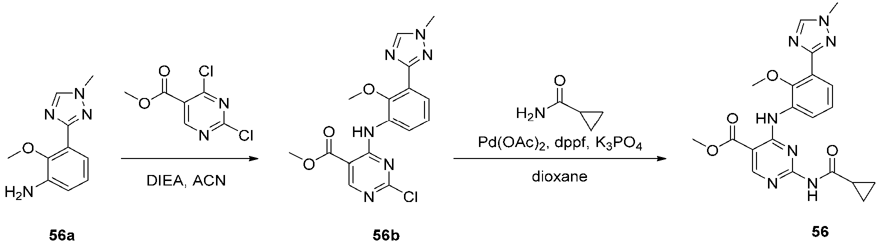

### Step 1. Methyl 2-chloro-4-((2-methoxy-3-(1-methyl-1H-1,2,4-triazol-3-yl)phenyl)amino)pyrimidine-5-carboxylate (56b)

To a solution of methyl 2,4-dichloropyrimidine-5-carboxylate (264 mg, 1.27 mmol) and **56a** (200 mg, 0.98 mmol) in ACN (8 mL) was added DIEA (190 mg, 1.47 mmol, 0.26 mL), the mixture was stirred at 45 °C for 12 h. The reaction mixture was diluted with water (50 mL), then extracted with EtOAc (50 mL*2). The combined organic layer was washed with brine (30 mL*2), dried over anhydrous Na₂SO₄, filtered and concentrated. The residue was purified by flash chromatography with silica gel (MeOH in DCM is 5%) to give **56b** (186 mg, 0.50 mmol, 51% yield) as a yellow solid. ¹H NMR (400 MHz, DMSO-*d*₆) δ 11.00 (s, 1H), 8.86 (s, 1H), 8.58 (s, 1H), 8.47 (dd, *J* = 8.0, 1.6 Hz, 1H), 7.65 (dd, *J =* 8.0, 1.6 Hz, 1H), 7.30 (t, *J* = 8.0 Hz, 1H), 3.96 (s, 3H), 3.94 (s, 3H), 3.81 (s, 3H). LC-MS (ESI, Method 4) t_{R} = 2.69 min, m/z [M+H]⁺ = 375.2.

### Step 2. Methyl 2-(cyclopropanecarboxamido)-4-((2-methoxy-3-(1-methyl-1H-1,2,4-triazol-3-yl)phenyl)amino)pyrimidine-5-carboxylate (56)

To a solution of **56b** (70 mg, 0.19 mmol) and cyclopropanecarboxamide (64 mg, 0.75 mmol) in dioxane (1 mL) was added Pd(OAc)₂ (8 mg, 0.037 mmol), dppf (41 mg, 0.075 mmol) and K₃PO₄ (119 mg, 0.56 mmol) under N₂ atmosphere, the mixture was stirred at 100 °C for 2 h. The reaction mixture was diluted with water (20 mL), then extracted with EtOAc (20 mL*2). The combined organic layer was washed with brine (20 mL*2), dried over anhydrous Na₂SO₄, filtered and concentrated. The residue was slurried in MeOH (10 mL) and filtered to give **56** (34 mg, 0.081 mmol, 44% yield) as an off-white solid. ¹H NMR (400 MHz, DMSO-*d*6) *δ* 11.14 (s, 1H), 11.06 (s, 1H), 9.34 (dd, *J =* 8.4, 1.6 Hz, 1H), 8.87 (s, 1H), 8.57 (s, 1H), 7.55 (dd, *J =* 7.6, 1.6 Hz, 1H), 7.19 (t, *J* = 8.0 Hz, 1H), 3.95 (s, 3H), 3.91 (s, 3H), 3.82 (s, 3H), 2.20-2.11 (m, 1H), 0.93-0.82 (m, 4H). LC-MS (ESI, Method 4) t_{R} = 2.09 min, m/z [M+H]⁺ = 424.3.

### Example 57

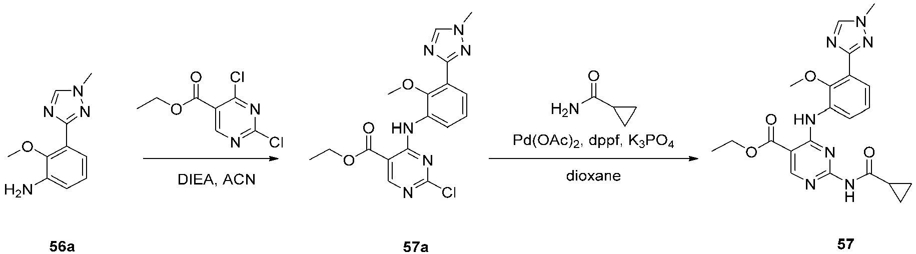

### Step 1. Ethyl 2-chloro-4-((2-methoxy-3-(1-methyl-1H-1,2,4-triazol-3-yl)phenyl)amino)pyrimidine-5-carboxylate (57a)

To a solution of ethyl 2,4-dichloropyrimidine-5-carboxylate (211 mg, 0.95 mmol) and **56a** (150 mg, 0.73 mmol) in ACN (8 mL) was added DIEA (142 mg, 1.10 mmol, 0.19 mL), the mixture was stirred at 45 °C for 12 h. The reaction mixture was diluted with water (50 mL), then extracted with EtOAc (50 mL*2). The combined organic layer was washed with brine (30 mL*2), dried over anhydrous Na₂SO₄, filtered and concentrated. The residue was purified by flash chromatography with silica gel (MeOH in DCM is 5%) to give **57a** (273 mg, 0.70 mmol, 95% yield) as a white solid. ¹H NMR (400 MHz, CDCl₃) *δ* 11.22 (s, 1H), 8.85 (s, 1H), 8.67 (dd, *J=* 8.0, 1.6 Hz, 1H), 8.18 (s, 1H), 7.76 (dd, *J=* 8.0, 1.6 Hz, 1H), 7.27 (t, *J=* 8.0 Hz, 1H), 4.44 (q, *J=* 7.2 Hz, 2H), 4.03 (s, 3H), 3.86 (s, 3H), 1.42 (t, *J=* 7.2 Hz, 3H). LC-MS (ESI, Method 4) t_{R} = 2.96 min, m/z [M+H]⁺ = 389.2.

### Step 2. Ethyl 2-(cyclopropanecarboxamido)-4-((2-methoxy-3-(1-methyl-1H-1,2,4-triazol-3-yl)phenyl)amino)pyrimidine-5-carboxylate (57)

To a solution of **57a** (80 mg, 0.21 mmol) and cyclopropanecarboxamide (70 mg, 0.82 mmol) in dioxane (5 mL) was added Pd(OAc)₂ (9 mg, 0.041 mmol), dppf (46 mg, 0.082 mmol) and K₃PO₄ (131 mg, 0.62 mmol) under N₂ atmosphere, the mixture was stirred at 100 °C for 1 h. The reaction mixture was diluted with water (20 mL), then extracted with EtOAc (20 mL*2). The combined organic layer was washed with brine (20 mL*2), dried over anhydrous Na₂SO₄, filtered and concentrated. The residue was slurried in MeOH (10 mL) and filtered to give **57** (43 mg, 0.098 mmol, 48% yield) as a white solid. ¹H NMR (400 MHz, DMSO-*d*₆) δ 11.15 (s, 1H), 11.08 (s, 1H), 9.34 (dd, *J=* 8.4, 1.6 Hz, 1H), 8.87 (s, 1H), 8.57 (s, 1H), 7.54 (dd, *J=* 8.0, 1.6 Hz, 1H), 7.18 (t, *J =* 8.0 Hz, 1H), 4.38 (q, *J =* 7.2 Hz, 2H), 3.95 (s, 3H), 3.81 (s, 3H), 2.21-2.11 (m, 1H), 1.36 (t, *J =* 7.2 Hz, 3H), 0.95-0.80 (m, 4H). LC-MS (ESI, Method 4) t_{R} = 2.31 min, m/z [M+H]⁺ = 438.3.

### Example 58

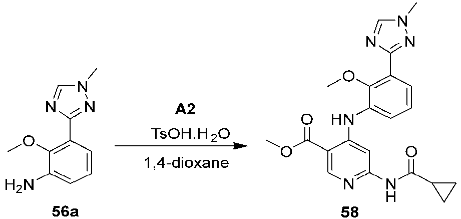

### Step 1. Methyl 6-(cyclopropanecarboxamido)-4-((2-methoxy-3-(1-methyl-1H-1,2,4-triazol-3-yl)phenyl)amino)nicotinate (58)

A mixture of **A2** (30 mg, 0.12 mmol), **56a** (24 mg, 0.12 mmol) and TsOH·H₂O (9 mg, 0.05 mmol) in dioxane (0.5 mL) was stirred at 100 °C for 16 h. After cooling to room temperature, the mixture was concentrated and the residue was purified by *Prep-HPLC* (Method A) to give **58** (13 mg, 20% yield) as a white solid. ¹H NMR (400 MHz, DMSO-*d*₆) δ 10.91 (s, 1H), 9.93 (s, 1H), 8.72 (s, 1H), 8.56 (s, 1H), 8.02 (s, 1H), 7.65 (dd, *J* = 7.6, 1.6 Hz, 1H), 7.52 (dd, *J=* 8.0, 1.6 Hz, 1H), 7.26 (t, *J=* 8.0 Hz, 1H), 3.94 (s, 3H), 3.89 (s, 3H), 3.71 (s, 3H), 2.00-1.97 (m, 1H), 0.79-0.77 (m, 4H). LC-MS (Method 2) t_{R} = 0.76 min, m/z (M+H)⁺ = 423.1.

### Example 59

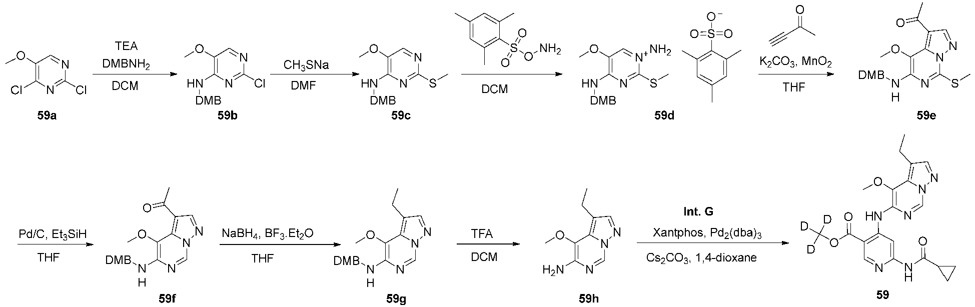

### Step 1. 2-Chloro-N-(2,4-dimethoxybenzyl)-5-methoxypyrimidin-4-amine (59b)

To a solution of **59a** (25.00 g, 139.66 mmol) in DCM (250 mL) was added TEA (28.26 g, 279.32 mmol) and (2,4-dimethoxyphenyl) methanamine (22.18 g, 132.68 mmol) at 0 °C. Then the mixture was stirred at room temperature for 1 h, diluted with water (100 mL) and extracted with DCM (150 mL*2). The combined organic layer was concentrated, and the residue was purified by flash chromatography on silica gel (PE/EA = 2/1) to afford **59b** (32.06 g, 74% yield) as a white solid. ¹H NMR (400 MHz, DMSO-*d*₆) δ 7.69 (s, 1H), 7.65 (t, *J =* 6.0 Hz, 1H), 6.99 (d, *J=* 8.0 Hz, 1H), 6.55 (d, *J* = 2.4 Hz, 1H), 6.47 (dd, *J* = 8.4 Hz, 2.4 Hz, 1H), 4.42 (d, *J* = 6.0 Hz, 2H), 3.86 (s, 3H), 3.80 (s, 3H), 3.73 (s, 3H).

### Step 2. N-(2,4-dimethoxybenzyl)-5-methoxy-2-(methylthio)pyrimidin-4-amine (59c)

A mixture of **59b** (12.00 g, 38.74 mmol) and MeSNa (4.07 g, 58.11 mmol) in DMF (50 mL) was stirred at 80 °C for 5 h. After cooling to room temperature, the reaction mixture was diluted with water (80 mL) and extracted with EtOAc (100 mL*3). The combined organic layer was concentrated, and the residue was purified by flash chromatography on silica gel (PE/EA = 5/1) to afford **59c** (4.3 g, 35% yield) as a yellow oil. LC-MS (Method 3) t_{R} = 0.28 min, m/z (M+H)⁺ = 322.3.

### Step 3. 1-Amino-4-((2,4-dimethoxybenzyl)amino)-5-methoxy-2-(methylthio)pyrimidin-1-ium 2,4,6-trimethylbenzenesulfonate (59d)

To a solution of **59c** (5.56 g, 17.30 mmol) in DCM (13 mL) was added *O-*(mesitylsulfonyl) hydroxylamine (3.72 g, 17.30 mmol) at 0 °C. Then the mixture was stirred at 0 °C for 3 h. The suspension was filtered, and the filter cake was dried to afford **59d** (2.2 g, 38% yield) as a white solid. LC-MS (Method 3) t_{R} = 0.97 min, m/z M⁺ = 337.3.

### Step 4. 1-(5-((2,4-Dimethoxybenzyl)amino)-4-methoxy-7-(methylthio)pyrazolo[1,5-c]pyrimidin-3-yl)ethanone (59e)

To a solution of **59d** (1.5 g, 4.45 mmol) and but-3-yn-2-one (363 mg, 5.33 mmol) in THF (15 mL) was added MnO₂ (580 mg, 6.67 mmol) and K₂CO₃ (922 mg, 6.67 mmol) at 0 °C. After stirring at 35 °C for 4 h, the reaction mixture was filtered. The filtrate was diluted with water (25 mL) and extracted with EtOAc (50 mL*2). The combined organic layer was concentrated, and the residue was purified by flash chromatography on silica gel (PE/EA = 2/1) to afford **59e** (330 mg, 18% yield) as a yellow solid. LC-MS (Method 3) t_{R} = 1.24 min, m/z (M+H)⁺ = 403.3.

### Step 5. 1-(5-((2,4-Dimethoxybenzyl)amino)-4-methoxypyrazolo[1,5-c]pyrimidin-3-yl)ethanone (59f)

A mixture of **59e** (320 mg, 0.79 mmol) and Pd/C (45 mg, 10% wt wetted in 50% water) in Et₃SiH (3 mL) and THF (3 mL) was stirred at 80 °C for 6 h. After cooling to room temperature, the reaction mixture was diluted with water (5 mL) and extracted with EtOAc (8 mL*2). The combined organic layer was dried over Na₂SO₄, filtered and concentrated. The residue was purified by flash chromatography on silica gel (DCM/EtOAc = 3/1) to afford **59f** (97 mg, 34% yield) as a yellow solid. LC-MS (Method 3) t_{R} = 1.16 min, m/z (M+H)⁺ = 357.3.

### Step 6. N-(2,4-dimethoxybenzyl)-3-ethyl-4-methoxypyrazolo[1,5-c]pyrimidin-5-amine (59g)

To a solution of **59f** (95 mg, 0.27 mmol) in THF (1 mL) was added NaBH₄ (10 mg, 0.27 mmol) and BF₃.Et₂O (76 mg, 0.53 mmol) under ice-water bath. The mixture was stirred at room temperature for 1 h, then poured into ice-water (2 mL) and extracted with EtOAc (5 mL*2). The combined organic layer was concentrated, and the residue was purified by flash chromatography on silica gel (DCM/MeOH = 20/1) to afford **59g** (44 mg, 48% yield) as a yellow oil. LC-MS (Method 3) t_{R} = 1.30 min, m/z (M+H)⁺ = 343.4.

### Step 7. 3-Ethyl-4-methoxypyrazolo[1,5-c]pyrimidin-5-amine (59h)

A mixture of **59g** (42 mg, 0.12 mmol) in TFA/DCM (0.4 mL/1.2 mL) was stirred at 8 °C for 30 min. The solvent was removed. The residue was diluted with DCM (1 mL), adjusted to pH > 7 with NH₃/1,4-dioxane (2 mL, 2 M). Then the mixture was concentrated, and the residue was purified by flash chromatography on silica gel (DCM/MeOH = 10/1) to afford **59h** (14 mg, 59% yield) as a yellow oil. LC-MS (Method 3) t_{R} = 1.09 min, m/z (M+H)⁺ = 193.0.

### Step 8. Methyl-d₃ 6-(cyclopropanecarboxamido)-4-((3-ethyl-4-methoxypyrazolo[1,5-c]pyrimidin-5-yl)amino)nicotinate (59)

A mixture of **59h** (10 mg, 0.05 mmol), **Int G** (16 mg, 0.06 mmol), Pd₂(dba)₃ (10 mg, 0.01 mmol), Xantphos (6 mg, 0.01 mol) and Cs₂CO₃ (34 mg, 0.10 mmol) in 1,4-dioxane (0.3 mL) was stirred at 90 °C for 3 h under N₂ atmosphere. After the reaction mixture was cooled, it was concentrated and purified by flash chromatography on silica gel (DCM/MeOH = 20/1) to give the crude product. The crude product was purified by *Prep-HPLC* (Method A) to afford **59** (3 mg, 14% yield) as a white solid. ¹H NMR (400 MHz, DMSO-*d*₆) δ 10.90 (s, 1H), 10.61 (s, 1H), 9.27 (s, 1H), 8.87 (s, 1H), 8.75 (s, 1H), 8.08 (s, 1H), 3.88 (s, 3H), 2.82 (q, *J =* 7.6 Hz, 2H), 2.04-2.01 (m, 1H), 1.28 (t, *J* = 7.2 Hz, 3H), 0.82-0.80 (m, 4H). LC-MS (ESI, Method 2) t_{R} = 0.89 min, m/z (M+H)⁺ = 414.1.

### Example 60

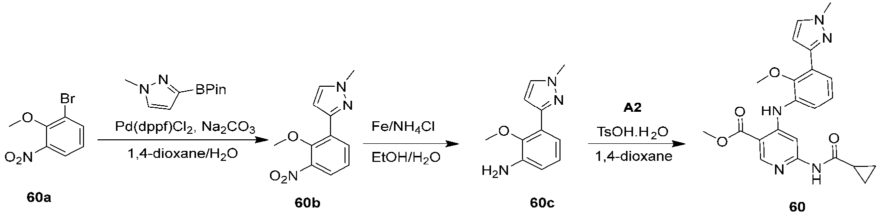

### Step 1. 3-(2-methoxy-3-nitrophenyl)-1-methyl-1H-pyrazole (60b)

A mixture of **60a** (223 mg, 0.96 mmol), 1-methyl-3-(4,4,5,5-tetramethyl-1,3,2-dioxaborolan-2-yl)-1H-pyrazole (200 mg, 0.96 mmol), Pd(dppf)Cl₂ (70.3 mg, 96.12 µmol) and Na₂CO₃ (204 mg, 1.92 mmol) in 1,4-dioxane (4 mL) and H₂O (1 mL) in a 10 mL sealed tube was stirred at 80 °C for 4 h under N₂ atmosphere. After cooling to room temperature, the mixture was diluted with EtOAc (20 mL), washed with water (5 mL) and brine (5 mL) and concentrated. The residue was purified by flash chromatography on silica gel (PE/EtOAc = 10/1 to 5/1) to give **60b** (200 mg, 89% yield) as a yellow solid. LC-MS (ESI, Method 3) t_{R} = 1.18 min, m/z (M+H)⁺ = 234.2.

### Step 2. 2-Methoxy-3-(1-methyl-1H-pyrazol-3-yl)aniline (60c)

A mixture of **60b** (200 mg, 0.858 mmol), iron powder (239 mg, 4.29 mmol) and NH₄Cl (459 mg, 8.58 mmol) in EtOH (4 mL) and H₂O (2 mL) was stirred at 80 °C for 1 h. After the reaction was completed, the solid was filtered off when it is warm and washed with EtOH (10 mL). The combined filtrate was concentrated and purified by flash chromatography on silica gel (PE/EtOAc = 1/1) to give **60c** (130 mg, 75% yield) as a white solid. LC-MS (ESI, Method 3) t_{R} = 0.93 min, m/z (M+H)⁺ = 204.2.

### Step 3. Methyl 6-(cyclopropanecarboxamido)-4-((2-methoxy-3-(1-methyl-1H-pyrazol-3-yl)phenyl)amino)nicotinate (60)

A mixture of **A2** (50 mg, 196.3 µmol), **60c** (43.9 mg, 215.97 µmol) and TsOH·H₂O (14.9 mg, 78.53 µmol) in dioxane (0.5 mL) was stirred at 100 °C for 16 h. The mixture was concentrated, and the residue was purified by *Prep-HPLC* (Method A) to give **60** (35 mg, 42% yield) as a white solid. ¹H NMR (400 MHz, DMSO-*d*₆) δ 10.89 (s, 1H), 9.93 (s, 1H), 8.71 (s, 1H), 8.03 (s, 1H), 7.77 *(d, J=* 2.0 Hz, 1H), 8.45 (dd *J* = 6.8, 7.6 Hz, 1H), 7.39 *(d, J=* 7.2 Hz, 1H), 7.21 (t, *J=* 7.6 Hz, 1H), 6.72 (d, *J* = 2.0 Hz, 1H), 3.91 (s, 3H), 3.89 (s, 3H), 3.58 (s, 3H), 2.00-1.97 (m, 1H), 0.79-0.77 (m, 4H). LC-MS (ESI, Method 2) t_{R} = 0.84 min, m/z (M+H)⁺ = 422.1.

### Example 61

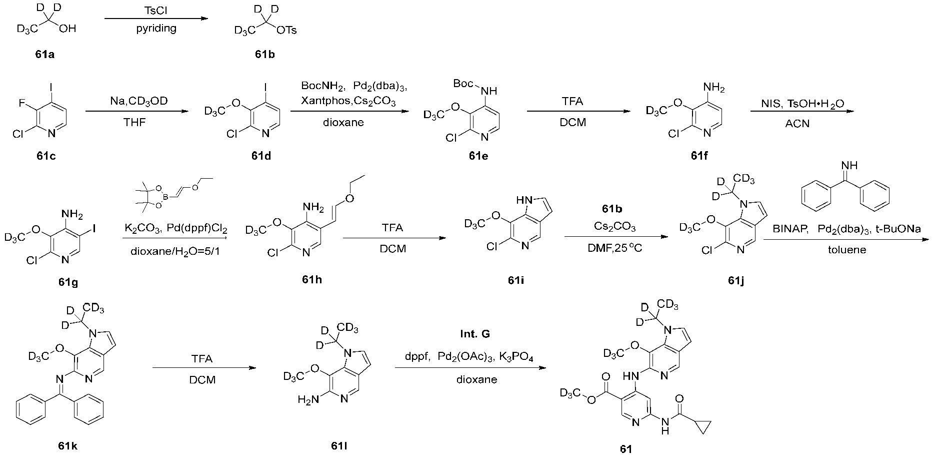

### Step 1. Ethyl-d₅ 4-methylbenzenesulfonate (61b)

A solution of **61a** (3.00 g, 59 mmol), TsCl (11.19 g, 59 mmol) and TEA (11.88 g, 117 mmol, 16.3 mL) in DCM (30 mL) was stirred at 25 °C for 16 h. The reaction mixture was diluted with water (50 mL), then extracted with DCM (30 mL*3). The combined organic layer was washed with brine (10 mL*2), dried over anhydrous Na₂SO₄, filtered and concentrated. The residue was purified by silica gel column (EtOAc in PE is 2-20%) to give 61b (3.00 g, 25% yield) as a colorless oil. ¹H NMR (400 MHz, CDCl₃) δ 7.80 (d, *J =* 8.0 Hz, 2H), 7.34 (d, *J =* 8.0 Hz, 2H), 2.45 (s, 3H).

### Step 2. 2-Chloro-4-iodo-3-(methoxy-d₃) pyridine (61d)

The solution of Na (0.89 g, 0.039 mol) in CD₃OD (10 mL) was stirred at 25 °C for 3 h. To a solution of **61c** (10 g, 0.039 mol) in THF (100 mL) was added the CD₃ONa solution. The mixture was stirred at 45 °C for 3 h. The reaction mixture was diluted with water (50 mL), then extracted with EA (50 mL*3). The combined organic layer was washed with brine (10 mL*2), dried over anhydrous Na₂SO₄, filtered and concentrated to give **61d** (10.0 g, crude). It was used for the next step directly without further purification. ¹H NMR (400 MHz, DMSO *d*₆) *δ* 7.91 (d, *J* = 4.8 Hz, 1H), 7.86 (d, *J* = 4.8 Hz, 1H). LC-MS (ESI, Method 3) t_{R} = 1.98 min, m/z [M+H]⁺ =273.0.

### Step 3. Tert-butyl (2-chloro-3-(methoxy-d₃) pyridin-4-yl) carbamate (61e)

A solution of **61d** (10.00 g, 37 mmol), BocNH₂ (6.45 g, 55 mmol), Xantphos (2.12 g, 4 mmol), Pd₂(dba)₃ (3.66 g, 4 mmol) and Cs₂CO₃ (35.87 g, 111 mmol) in dioxane (100 mL) was stirred at 80 °C for 6 h under N₂ atmosphere. The reaction mixture was diluted with water (100 mL), then extracted with EA (100 mL*3). The combined organic layer was washed with brine (50 mL*2), dried over anhydrous Na₂SO₄, filtered and concentrated to give **61e** (5 g, crude). It was used for the next step directly without further purification. LC-MS (ESI, Method 3) t_{R} = 2.75 min, m/z [M+H]⁺ = 262.1.

### Step 4. 2-Chloro-3-(methoxy-d₃) pyridin-4-amine (61f)

To a solution of **61e** (10.00 g, 37 mmol) in DCM (80 mL) was added TFA (30 mL) at 0 °C. The mixture was stirred at 25 °C for 3 h. The mixture was concentrated to give a residue. The residue was adjusted to pH = 7 with sat. aq. NaHCO₃, extracted with DCM (50 mL*3), dried over Na₂SO₄ and concentrated. The residue was purified by silica gel column (EtOAc in PE is 2-20%) to give **61f** (3.90 g, 63% yield) as a yellow solid. ¹H NMR (400 MHz, DMSO *d₆*) δ *7.60 (d, J* = 5.6 Hz, 1H), 6.59 (d, *J* = 5.6 Hz, 1H), 6.30 (s, 2H). LC-MS (ESI, Method 3) t_{R} = 1.27 min, m/z [M+H]⁺ = 161.6.

### Step 5. 2-Chloro-5-iodo-3-(methoxy-d₃) pyridin-4-amine (61g)

A solution of **61f** (3.00 g, 19 mmol), TsOH·H₂O (0.35 g, 2 mmol) and NIS (6.28 g, 28 mmol) in ACN (30 mL) was stirred at 70 °C for 16 h. The reaction mixture was diluted with water (50 mL), then extracted with EA (50 mL*3). The combined organic layer was washed with brine (50 mL), dried over anhydrous Na₂SO₄, filtered and concentrated. The residue was purified by silica gel column (EtOAc in PE is 1-20%) to give 61g (3.00 g, 56% yield) as a yellow solid. ¹H NMR (400 MHz, DMSO *d₆*) δ 8.02 (s, 1H), 6.34 (s, 2H). LC-MS (ESI, Method 3) t_{R} = 2.47 min, m/z [M+H]⁺ = 288.0.

### Step 6. (E)-2-chloro-5-(2-ethoxyvinyl)-3-(methoxy-d₃) pyridin-4-amine (61h)

To a solution of **61g** (3.00 g, 11 mmol) in dioxane/H₂O = 5/1 (30 mL) was added 2-[(*E*)-2-ethoxyethenyl]-4,4,5,5-tetramethyl-1,3,2-dioxaborolane (2.47 g, 13 mmol), K₂CO₃ (4.31 g, 31 mmol) and Pd(dppf)Cl₂ (0.76 g, 1 mmol). The mixture was stirred for 16 h at 60 °C under N₂ atmosphere. The mixture was quenched with water (50 mL), extracted with EA (50 mL*3), dried over Na₂SO₄ and concentrated to give a residue. The residue was purified by silica gel column (EtOAc in PE is 10-20%) to give **61h** (1.4 g, 58% yield) as a yellow oil. ¹H NMR (400 MHz, DMSO *d₆*) δ 7.69 (s, 1H), 6.96 (d, *J=* 12.4 Hz, 1H), 6.09 (s, 2H), 5.76 (d, *J=* 12.8 Hz, 1H), 3.94-3.89 (m, 2H), 1.25 (t, 3H). LC-MS (ESI, Method 3) t_{R} = 1.56 min, m/z [M+H]⁺ = 232.1.

### Step 7. 6-Chloro-7-(methoxy-d₃)-1H-pyrrolo [3,2-c] pyridine (61i)

To a solution of **61h** (1.40 g, 6 mmol) in DCM (16 mL) was added TFA (4 mL) at 0 °C. The mixture was stirred at 25 °C for 2 h. The mixture was concentrated to give a residue. The residue was adjusted to pH = 7 with sat. aq. NaHCO₃, extracted with DCM (30 mL*3), dried over Na₂SO₄ and concentrated to give **61i** (1.10 g, crude) as a yellow solid. it was used for the next step directly without further purification. ¹H NMR (400 MHz, DMSO *d*₆) δ 12.07 (s, 1H), 8.40 (s, 1H), 7.49 (d, *J=* 3.2 Hz, 1H), 6.65 (d, *J =* 4.0 Hz, 1H). LC-MS (ESI, Method 3) t_{R} = 1.57 min, m/z [M+H]⁺ = 185.6.

### Step 8. 6-Chloro-1-(ethyl-d₅)-7-(methoxy-d₃)-1H-pyrrolo[3,2-c] pyridine (61j)

A solution of **61i** (1.20 g, 7 mmol), **61b** (2.00 g, 10 mmol) and Cs₂CO₃ (4.24 g, 13 mmol) in DMF (15 mL) was stirred at 70 °C for 16 h. The reaction mixture was diluted with water (30 mL), then extracted with EA (30 mL*3). The combined organic layer was washed with brine (10 mL*2), dried over anhydrous Na₂SO₄, filtered and concentrated. The residue was purified by silica gel column (EtOAc in PE is 2-20%) to give **61j** (1.00 g, 71 % yield) as a yellow solid. ¹H NMR (400 MHz, DMSO *d*₆) δ 8.40 (s, 1H), 7.53 (d, *J* = 4.0 Hz, 1H), 6.65 (d, *J* = 3.2 Hz, 1H). LC-MS (ESI, Method 3) t_{R} = 1.98 min, m/z [M+H]⁺ = 219.1.

### Step 9. N-(1-(ethyl-d₅)-7-(methoxy-d₃)-1H-pyrrolo[3,2-c] pyridin-6-yl)-1,1-diphenylmethanimine (61k)

To a solution of **61j** (1.00 g, 5 mmol), diphenylmethanimine (2.50 g, 14 mmol) and BINAP (1.15 g, 2 mmol) in toluene (15 mL) was added Pd(dba)₃ (0.84 g, 1 mmol), t-BuONa (1.33 g, 14 mmol). The reaction mixture was stirred at 110 °C for 16 h under N₂ atmosphere. The reaction mixture was diluted with water (40 mL), then extracted with DCM (30 mL*3). The combined organic layer was washed with brine (10 mL*2), dried over anhydrous Na₂SO₄, filtered and concentrated. The residue was purified by Prep-TLC (DCM/MeOH = 20/1) to give **61k** (0.9 g, 54% yield) as a yellow solid. ¹H NMR (400 MHz, DMSO *d*₆) δ 8.16 (s, 1H), 7.74-7.72 (m, 2H), 7.57-7.48 (m, 3H), 7.25-7.21 (m, 4H), 7.13-7.10 (m, 2H), 6.65 *(d, J=* 3.2 Hz, 1H). LC-MS (ESI, Method 3) t_{R} = 2.76 min, m/z [M+H]⁺ = 363.9.

### Step 10. 1-(Ethyl-d₅)-7-(methoxy-d₃)-1H-pyrrolo[3,2-c] pyridin-6-amine (61l)

To a solution of **61k** (850 mg, 2.34 mmol) in DCM (15 mL) was added TFA (5 mL). The mixture was stirred at 25 °C for 16 h. The mixture was concentrated to give a residue. The residue was adjusted to pH = 7 with sat. aq. NaHCO₃, extracted with DCM (30 mL*3), dried over Na₂SO₄ and concentrated. The residue purified by Prep-TLC (DCM/MeOH = 20/1) to give **611** (200 mg, 43% yield) as a red solid. ¹H NMR (400 MHz, DMSO *d*₆) δ 8.02 (s, 1H), 7.09 (d, *J* = 3.2 Hz, 1H), 6.34 (d, *J* = 3.2 Hz, 1H), 5.22 (s, 2H). LC-MS (ESI, Method 3) t_{R} = 1.80 min, m/z [M+H]⁺ = 199.7.

### Step 11. Methyl-d₃ 6-(cyclopropanecarboxamido)-4-((1-(ethyl-d₅)-7-(methoxy-d₃)-1H-pyrrolo[3,2-c] pyridin-6-yl) amino) nicotinate (61)

To a solution of **61l** (100 mg, 0.502 mmol), **Int G** (129 mg, 0.502 mmol) and Pd(OAc)₂ (11 mg, 0.051 mmol) in dioxane (10 mL) was added dppf (110 mg, 0.151 mmol), K₃PO₄ (213 mg, 1.004 mmol). The reaction mixture was stirred at 80 °C for 12 h. The reaction mixture was diluted with water (10 mL), then extracted with DCM (10 mL*3). The combined organic layer was washed with brine (10 mL*2), dried over anhydrous Na₂SO₄, filtered and concentrated. The residue was purified by Prep-HPLC (Method A) to give **61** (14 mg, 7% yield) as a yellow solid. ¹H NMR (400 MHz, DMSO *d*₆) δ 10.81 (s, 1H), 10.67 (s, 1H), 9.12 (s, 1H), 8.73 (s, 1H), 8.41 (s, 1H), 7.44 (d, *J=* 3.2 Hz, 1H), 6.59 (d, *J=* 3.2 Hz, 1H), 2.07-2.06 (m, 1H), 0.80-0.78 (m, 4H). LC-MS (ESI, Method 3) t_{R} = 1.75 min, m/z [M+H]⁺ = 421.2.

### Example 62

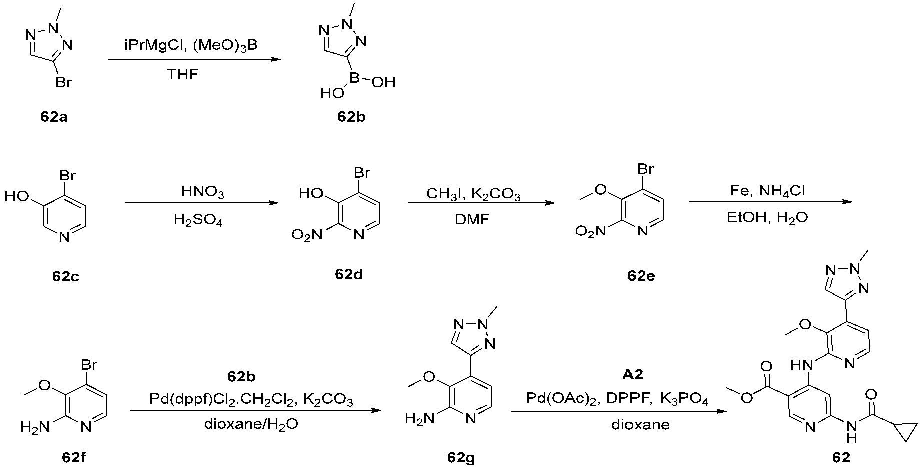

### Step 1. (2-Methyl-2H-1,2,3-triazol-4-yl)boronic acid (62b)

To a solution of **62a** (1.5 g, 9.26 mmol) in THF (25 mL) was dropped iPrMgCl (2 M in THF, 4.7 mL) at 0 °C under nitrogen atmosphere. The mixture was stirred at 0 °C for 2 h. Then the temperature was decreased to -20°C and trimethyl borate (1.06 g, 10.19 mmol) was dropped to the solution slowly. The mixture was stirred at -20°C for 1 h. The solution was added to H₂O (30 mL), and the pH was adjusted to 5~6. The aqueous solution was extracted by EA (40 mL*2), the combined organic layer was washed by brine (15 mL*2), dried over Na₂SO₄ and filtered. The filtrate was concentrated in vacuo to afford **62b** (700 mg, 60% yield) as a white solid. LC-MS (ESI, Method 4) t_{R} = 0.35 min, m/z (M+H)⁺ = 128.0.

### Step 2. 4-Bromo-2-nitropyridin-3-ol (62d)

The solution of **62c** (3 g, 17.24 mmol) in H₂SO₄ (30 mL) was stirred at -10 °C under nitrogen atmosphere for 5 min, HNO₃ (7.05 g, 111.88 mmol) was added dropwise over 10 min. The mixture was stirred at -10 °C to room temperature for 12 h. The solution was poured into a crushed ice (100 g). After completely quenched, the mixture was extracted by EA (30 mL*2). The combined organic layer was washed by brine (30 mL*2), dried over Na₂SO₄ and filtered. The filtrate was concentrated in vacuo to afford **62d** (2 g, 48% yield, 90% purity) as a yellow solid without further purification. LC-MS (ESI, Method 4) t_{R} = 1.62 min, m/z (M+H)⁺ = 218.9.

### Step 3. 4-Bromo-3-methoxy-2-nitropyridine (62e)

To a solution of **62d** (2 g, 8.22 mmol, 90% purity) in DMF (40 mL) was added K₂CO₃ (3.41 g, 24.66 mmol) at room temperature under nitrogen atmosphere for 5 min. Iodomethane (2.33 g, 16.44 mmol) was added and the mixture was stirred at 60 °C for 3 h. The solution was added to H₂O (30 mL) and extracted by EA (50 mL), the combined organic layer was washed by brine (20 mL*2), dried over Na₂SO₄ and filtered. The filtrate was concentrated in vacuo. The crude was purified by silica gel column (EA/PE = 1/2) to afford **62e** (1.26 g, 66% yield) as a white solid. LC-MS (ESI, Method 4) t_{R} = 2.49 min, m/z (M+H)⁺ = 233.0.

### Step 4. 4-Bromo-3-methoxypyridin-2-amine (62f)

To a solution of **62e** (1.26 g, 5.41 mmol), Fe (1.51 g, 27.04 mmol) in EtOH (16 mL) and H₂O (4 mL) was added NH₄Cl (2.89 g, 54.07 mmol) at room temperature. The mixture was stirred at 80 °C for 2 h under nitrogen atmosphere. The resulting solution was filtered, and the filtrate was added with H₂O (10 mL). The solution was extracted by EA (30 mL*2), the combined organic layer was dried over Na₂SO₄ and filtered. The filtrate was concentrated in vacuo to afford **62f** (1 g, 91% yield) as a light-yellow solid without further purification. LC-MS (ESI, Method 4) t_{R} = 0.48 min, m/z (M+H)⁺ = 203.0.

### Step 5. 3-Methoxy-4-(2-methyl-2H-1,2,3-triazol-4-yl)pyridin-2-amine (62g)

To a solution of **62f** (1 g, 4.93 mmol), Pd(dppf)Cl₂.CH₂Cl₂ (603 mg, 0.74 mmol), K₂CO₃ (1.70 g, 12.31 mmol) in H₂O (2 mL) and dioxane (10 mL) was added **62b** (688 mg, 5.42 mmol) at room temperature. The mixture was stirred at 100 °C for 2 h under nitrogen atmosphere. The resulting solution was added to H₂O (20 mL) and extracted by EA (30 mL*2), the combined organic layer was dried over Na₂SO₄, filtered and concentrated in vacuo. The crude was purified by silica gel column (EA/PE = 1/1) to afford **62g** (740 mg, 73% yield) as an off-white solid. ¹H NMR (400 MHz, DMSO-*d*₆) δ 8.15 (s, 1H), 7.72 (d, *J =* 5.2 Hz, 1H), 6.96 (d, *J* = 5.2 Hz, 1H), 6.06 (s, 2H), 4.22 (s, 3H), 3.63 (s, 3H). LC-MS (ESI, Method 4) t_{R} = 0.43 min, m/z (M+H)⁺ = 206.1.

### Step 6. Methyl 6-(cyclopropanecarboxamido)-4-((3-methoxy-4-(2-methyl-2H-1,2,3-triazol-4-yl)pyridin-2-yl)amino)nicotinate (62)

To a solution of **A2** (161 mg, 0.63 mmol), Pd(OAc)₂ (21 mg, 0.095 mmol), dppf (105 mg, 0.19 mmol), K₃PO₄ (336 mg, 1.58 mmol) in dioxane (9 mL) was added **62g** (130 mg, 0.63 mmol) at room temperature. The mixture was stirred at 100 °C for 3 h under nitrogen atmosphere. The resulting solution was added to H₂O (20 mL) and extracted by EA (40 mL), the combined organic layer was washed by brine (15 mL*2), dried over Na₂SO₄, filtered and concentrated in vacuo. The crude was purified by *Prep*-HPLC (Method E) to afford **62** (21 mg, 8% yield) as a white solid. ¹H NMR (400 MHz, Chloroform-d) δ 11.54 (s, 1H), 9.89 (s, 1H), 8.88-8.81 (brs, 1H), 8.82 (s, 1H), 8.29 (d, *J* = 5.2 Hz, 1H), 8.18 (s, 1H), 7.48 (d, *J =* 5.2 Hz, 1H), 4.29 (s, 3H), 3.98 (s, 3H), 3.83 (s, 3H), 1.67-1.59 (m, 1H), 1.21-1.15 (m, 2H), 0.96-0.89 (m, 2H). LC-MS (ESI, Method 4) t_{R} = 5.57 min, m/z (M+H)⁺ = 424.3.

### Example 63

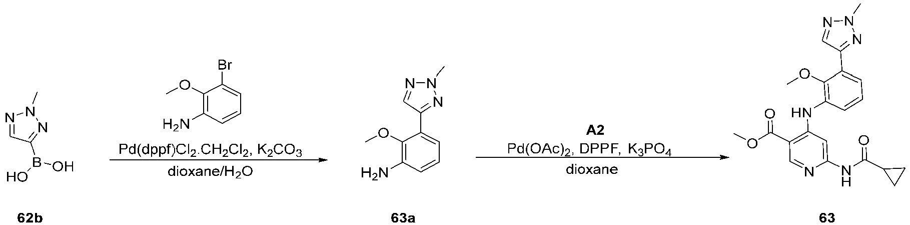

### Step 1. 2-Methoxy-3-(2-methyl-2H-1,2,3-triazol-4-yl)aniline (63a)

To a solution of ·3-bromo-2-methoxyaniline (300 mg, 1.48 mmol), Pd(dppf)Cl₂·CH₂Cl₂ (121 mg, 0.15 mmol), K₂CO₃ (513 mg, 3.71 mmol) in H₂O (2 mL) and dioxane (10 mL) was added **62b** (207 mg, 1.63 mmol), The mixture was stirred at 100 °C for 2 h under nitrogen atmosphere. The resulting solution was added to H₂O (15 mL) and extracted by EA (20 mL*2), the combined organic layer was dried over Na₂SO₄, filtered and concentrated in vacuo. The crude was purified by silica gel column to afford **63a** (230 mg, 76% yield) as a light-yellow solid. LC-MS (ESI, Method 4) t_{R} = 1.47 min, m/z (M+H)⁺ = 205.1.

### Step 2. Methyl 6-(cyclopropanecarboxamido)-4-((2-methoxy-3-(2-methyl-2H-1,2,3-triazol-4-yl)phenyl)amino)nicotinate (63)

To a solution of **A2** (100 mg, 0.39 mmol), Pd(OAc)₂ (18 mg, 0.078 mmol), dppf (87 mg, 0.16 mmol), K₃PO₄ (208 mg, 0.98 mmol) in dioxane (10 mL) was added **63a** (80 mg, 0.39 mmol). The mixture was stirred at 100 °C for 3 h under nitrogen atmosphere. The resulting solution was added to H₂O (20 mL) and extracted by EA (30 mL), the combined organic layer was washed by brine (15 mL*2), dried over Na₂SO₄, filtered and concentrated in vacuo. The crude was purified by silica gel column (EA/PE = 1/2) to afford **63** (97 mg, 59% yield) as a white solid. ¹H NMR (400 MHz, DMSO-*d*₆) δ 10.94 (s, 1H), 9.94 (s, 1H), 8.72 (s, 1H), 8.12 (s, 1H), 8.01 (s, 1H), 7.70 (dd, *J =* 8.0, 1.6 Hz, 1H), 7.47 (dd, *J =* 8.0, 1.6 Hz, 1H), 7.29 (t, *J =* 8.0 Hz, 1H), 4.23 (s, 3H), 3.89 (s, 3H), 3.64 (s, 3H), 2.02-1.93 (m, 1H), 0.81-0.74 (m, 4H). LC-MS (ESI, Method 4) t_{R} = 5.47 min, m/z (M+H)⁺ = 423.4.

### Example 64

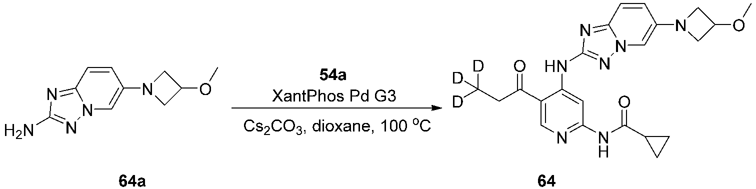

### Step 1. N-[4-[[6-(3-methoxyazetidin-1-yl)-[1,2,4]triazolo[1,5-a]pyridin-2-yl]amino]-5-(3,3,3-trideuteriopropanoyl)-2-pyridyl]cyclopropanecarboxamide (64)

A mixture of **54a** (20 mg, 0.08 mmol), **64a** (19 mg, 0.08 mmol, WO2021170046A1, P28-29), XantPhos Pd G3 (8 mg, 0.008 mmol), Cs₂CO₃ (76 mg, 0.24 mmol) in dioxane (3 mL) was stirred at 100 °C for 12 h under N₂ atmosphere. The reaction mixture was diluted with EtOAc (50 mL) and filtered through a pad of celite. The filtrate was washed with water (50 mL) and brine (50 mL), dried over anhydrous Na₂SO₄, filtered and concentrated. The residue was purified by flash chromatography on silical gel (MeOH in DCM is 0-8%) and further Prep-HPLC (Method E) to give **64** (7.1 mg, 21% yield) as a white soild. ¹H NMR (400 MHz, DMSO-*d*₆) *δ* 11.92 (s, 1H), 10.96 (s, 1H), 9.17 (s, 1H), 8.93 (s, 1H), 7.91 (d, *J* = 2.4 Hz, 1H), 7.56 (d, *J =* 10.0 Hz, 1H), 7.11 (dd, *J* = 9.6 Hz, 2.4 Hz, 1H), 4.36-4.30 (m, 1H), 4.11 (dd, *J =* 7.2 Hz, 6.0 Hz, 2H), 3.67 (dd, *J* = 8.0 Hz, 4.4 Hz, 2H), 3.35 (s, 3H), 3.12 (s, 2H), 2.11-2.05 (m, 1H), 0.91-0.81 (m, 4H). LC-MS (ESI, Method 4) t_{R} = 5.13 min, m/z (M+H)⁺ = 439.2.

### Example 65

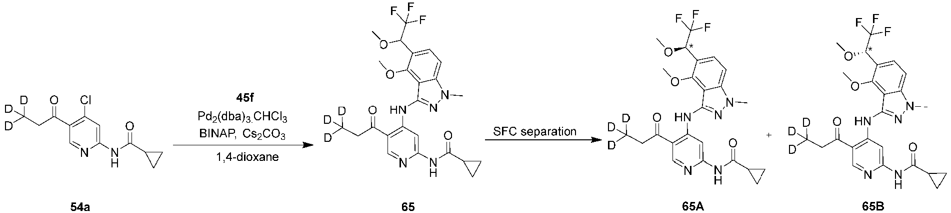

### Step 1. N-(4-((4-methoxy-1-methyl-5-(2,2,2-trifluoro-1-methoxyethyl)-1H-indazol-3-yl)amino)-5-(propanoyl-3,3,3-d₃)pyridin-2-yl)cyclopropanecarboxamide (65)

A mixture of **54a** (60 mg, 0.235 mmol), **45f** (61 mg, 0.211 mmol), Cs₂CO₃ (153 mg, 0.469 mmol), Pd₂(dba)₃·CHCl₃ (24.3 mg, 0.0235 mmol) and BINAP (14.6 mg, 0.0235 mmol) in 1,4-dioxane (0.6 mL) was stirred at 110 °C for 6 h under N₂ atmosphere. The mixture was cooled and concentrated. The residue was purified by flash chromatography on silica gel (DCM/MeOH = 20/1) to afford the crude product and repurified by *Prep*-HPLC (Method A) to afford **65** (22.5 mg, 19% yield) as a white solid. ¹H NMR (400 MHz, DMSO-*d*₆) δ 11.98 (s, 1H), 10.87 (s, 1H), 9.19 (s, 1H), 8.93 (s, 1H), 7.47 (d, *J =* 8.4 Hz, 1H), 7.41 (d, *J =* 8.8 Hz, 1H), 5.32-5.28 (m, 1H), 3.98 (s, 3H), 3.89 (s, 3H), 3.32 (s, 3H), 3.14 (s, 2H), 2.07-2.06 (m, 1H), 0.83-0.82 (m, 4H). LCMS (ESI, Method 2): t_{R} = 0.98 min, m/z (M+H)⁺ = 509.2.

### Step 2. (R*)-N-(4-((4-methoxy-1-methyl-5-(2,2,2-trifluoro-1-methoxyethyl)-1H-indazol-3-yl)amino)-5-(propanoyl-3,3,3-d₃)pyridin-2-yl)cyclopropanecarboxamide (65A) and (S*)-N-(4-((4-methoxy-1-methyl-5-(2,2,2-trifluoro-1-methoxyethyl)-1H-indazol-3-yl)amino)-5-(propanoyl-3,3,3-d₃)pyridin-2-yl)cyclopropanecarboxamide (65B)

**65** (20 mg, 0.04 mmol) was separated by chiral *prep*-HPLC (Method J, Hex/EtOH/DEA= 80/20/0.2) to afford **65A** (5.5 mg, 28% yield) as a white solid and **65B** (7.2 mg, 36% yield) as a white solid.

**65A:** LC-MS (ESI, Method 2): t_{R} = 1.12 min, m/z (M+H)⁺ = 509.0. ¹H NMR (400 MHz, DMSO-*d*₆) δ 11.99 (s, 1H), 10.88 (s, 1H), 9.19 (s, 1H), 8.92 (s, 1H), 7.47 (d, *J =* 8.8 Hz, 1H), 7.41 (d, *J* = 8.8 Hz, 1H), 5.31-5.28 (m, 1H), 3.98 (s, 3H), 3.89 (s, 3H), 3.32 (s, 3H), 3.14 (s, 2H), 2.09-2.04 (m, 1H), 0.84-0.82 (m, 4H). Chiral-HPLC (Method 7, Hex\EtOH\DEA = 80\20\0.2 run time of 15 minutes) t_{R} = 8.92 min.

**65B:** LC-MS (ESI, Method 2): t_{R} = 1.12 min, m/z (M+H)⁺ = 509.0. ¹H NMR (400 MHz, DMSO-*d*₆) δ 11.99 (s, 1H), 10.88 (s, 1H), 9.19 (s, 1H), 8.93 (s, 1H), 7.47 (d, *J =* 8.8 Hz, 1H), 7.41 (d, *J =* 8.8 Hz, 1H), 5.32-5.29 (m, 1H), 3.98 (s, 3H), 3.89 (s, 3H), 3.32 (s, 3H), 3.14 (s, 2H), 2.09-2.05 (m, 1H), 0.84-0.82 (m, 4H). Chiral-HPLC (Method 7, Hex\EtOH\DEA = 80\20\0.2 run time of 15 minutes) t_{R} = 10.51 min.

### Example 66

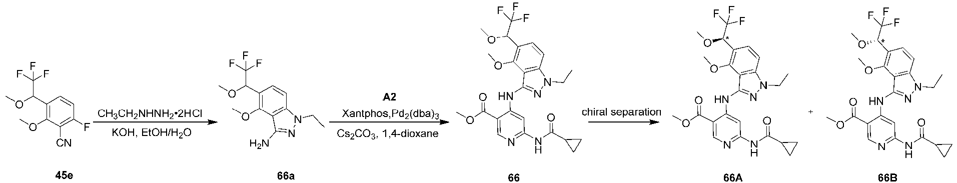

### Step 1. 1-Ethyl-4-methoxy-5-(2,2,2-trifluoro-1-methoxyethyl)-1H-indazol-3-amine (66a)

To a solution of C₂H₅NHNH₂·2HCl (1.99 g, 15.20 mmol) in EtOH/H₂O (7.2 mL, v/v =5/1) was added KOH (1.71 g, 30.40 mmol) at ice-bath. Then the mixture was stirred at 30 °C for 30 min. and it was filtered, the filtrate was added with **45e** (1 g, 3.80 mmol). The mixture was stirred at 90 °C for 4 h in sealed tube. Then it was diluted with EtOAc (25 mL) and washed with water (15 mL). The organic layer was concentrated and the residue was purified by column chromatography (PE/EtOAc = 2/1) to give **66a** (110 mg, 10% yield) as a yellow solid. LC-MS (ESI, Method 3): t_{R} = 1.21 min, m/z (M+H)⁺ = 304.2.

### Step 2. Methyl 6-(cyclopropanecarboxamido)-4-((1-ethyl-4-methoxy-5-(2,2,2-trifluoro-1-methoxyethyl)-1H-indazol-3-yl)amino)nicotinate (66)

A mixture of **66a** (50 mg, 0.165 mmol), **A2** (46.2 mg, 0.181 mmol), Pd₂(dba)₃ (30.2 mg, 0.033 mmol), Xantphos (19.1 mg, 0.033 mmol) and Cs₂CO₃ (107.4 mg, 0.329 mmol) in 1,4-dioxane (0.5 mL) was stirred at 90 °C for 4 h under N₂ atmosphere in a sealed tube. The reaction mixture was concentrated and the residue was purified by flash chromatography on silica gel (DCM/MeOH = 98/2) to give the crude product. The crude compound was purified by *Prep-*HPLC (Method A) to afford **66** (40 mg, 47% yield) as a white solid. LC-MS (ESI, Method 3): t_{R} = 1.33 min, m/z (M+H)⁺ = 522.5.

### Step 3. Methyl (R*)-6-(cyclopropanecarboxamido)-4-((1-ethyl-4-methoxy-5-(2,2,2-trifluoro-1-methoxyethyl)-1H-indazol-3-yl)amino)nicotinate (66A) and Methyl (S*)-6-(cyclopropanecarboxamido)-4-((1-ethyl-4-methoxy-5-(2,2,2-trifluoro-1-methoxyethyl)-1H-indazol-3-yl)amino)nicotinate (66B)

**66** (40 mg, 0.077mol) was separated by chiral *prep*-HPLC (Method G, Hex/IPA/DEA = 70/30/0.2) to afford **66A** (12.3 mg, 31% yield) as a white solid and **66B** (14.6 mg, 37% yield) as a white solid.

**66A:** LC-MS (ESI, Method 2): t_{R} = 1.01 min, m/z (M+H)⁺ = 522.1. ¹H NMR (400 MHz, DMSO-*d*₆) δ 10.91 (s, 1H), 10.88 (s, 1H), 9.27 (s, 1H), 8.77 (s, 1H), 7.51 (d, *J* = 9.2 Hz, 1H), 7.40 (d, *J =* 8.8 Hz, 1H), 5.32-5.25 (m, 1H), 4.34 (q, *J =* 7.2 Hz, 2H), 3.93 (s, 3H), 3.89 (s, 3H), 3.32 (s, 3H), 2.07-2.00 (m, 1H), 1.49 (t, *J =* 7.2 Hz, 3H), 0.85-0.77 (m, 4H). Chiral-HPLC (Method 8, Hex/IPA/DEA = 70/30/0.2) t_{R} = 7.50 min.

**66B:** LC-MS (ESI, Method 2): t_{R} = 1.01 min, m/z (M+H)⁺ = 522.1. ¹H NMR (400 MHz, DMSO-*d*₆) δ 10.91 (s, 1H), 10.88 (s, 1H), 9.27 (s, 1H), 8.77 (s, 1H), 7.51 (d, *J* = 9.2 Hz, 1H), 7.40 (d, *J* = 8.8 Hz, 1H), 5.28 (q, *J =* 6.8 Hz 1H), 4.34 (q, *J =* 7.2 Hz, 2H), 3.93 (s, 3H), 3.89 (s, 3H), 3.32 (s, 3H), 2.09-1.99 (m, 1H), 1.49 (t, *J =* 6.8 Hz, 3H), 0.86-0.78 (m, 4H). Chiral-HPLC (Method 8, Hex/IPA/DEA = 70/30/0.2) t_{R} = 10.15 min.

### Example 67

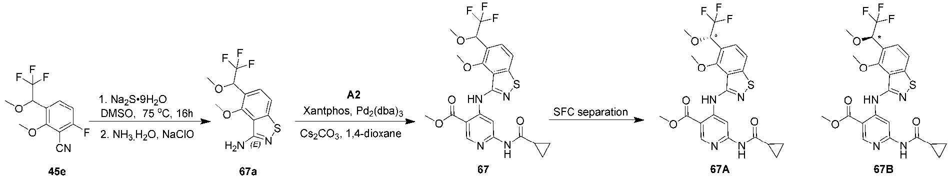

### Step 1. 4-Methoxy-5-(2,2,2-trifluoro-1-methoxyethyl)benzo[d]isothiazol-3-amine (67a)

A mixture of **45e** (250 mg, 0.95 mmol), Na₂S.9H₂O (296 mg, 1.23 mmol) and DMSO (4 mL) in a sealed tube was stirred at 75 °C for 16 h. The reaction was cooled to room temperature. Sodium hypochlorite solution (2 mL) was added to ammonium hydroxide (2 mL) under ice-water bath. To it was added the above reaction solution dropwise. The mixture was stirred for 15 min under ice-water bath and stirred for 1 h at room temperature. The solution was concentrated and purified by *Prep*-HPLC (Mehod A) to afford the crude product. The crude product was re-purified by flash chromatography on silica gel (PE/EA = 10/1 to 4/1) to afford compound **67a** (80 mg, 29% yield) as a white solid. ¹H NMR (300 MHz, CDCl₃) δ 7.52 (d, *J =* 9.0 Hz, 2H), 5.36 (s, 2H), 5.03-5.00 (m, 1H), 4.01 (s, 3H), 3.31 (s, 3H).

### Step 2. Methyl 6-(cyclopropanecarboxamido)-4-((4-methoxy-5-(2,2,2-trifluoro-1-methoxyethyl)benzo[d]isothiazol-3-yl)amino)nicotinate (67)

A mixture of **67a** (30 mg, 0.102 mmol), **A2** (28.8 mg, 0.113 mmol), Pd₂(dba)₃ (18.8 mg, 0.021 mmol), Xantphos (11.9 mg, 0.021 mmol) and Cs₂CO₃ (66.9 mg, 0.205 mmol) in 1,4-dioxane (0.5 mL) was stirred at 90 °C for 3 h under N₂ atmosphere. The reaction mixture was concentrated and purified by *Prep*-HPLC (Method A) to give **67** (16 mg, 31% yield) as a white solid. ¹H NMR (400 MHz, DMSO-*d*₆) δ 11.86 (s, 1H), 11.02 (s, 1H), 9.84 (s, 1H), 8.85 (s, 1H), 8.05 (d, *J* = 8.4 Hz, 1H), 7.66 (d, *J =* 8.4 Hz, 1H), 5.48-5.43 (m, 1H), 3.95 (s, 3H), 3.92 (s, 3H), 3.40 (s, 3H), 2.11-2.00 (m, 1H), 0.95-0.79 (m, 4H). LC-MS (ESI, Method 2): t_{R} = 1.00 min, m/z (M+H)⁺ = 511.1.

### Step 3. Methyl (S*)-6-(cyclopropanecarboxamido)-4-((4-methoxy-5-(2,2,2-trifluoro-1-methoxyethyl)benzo[d]isothiazol-3-yl)amino)nicotinate (67A) and Methyl (R*)-6-(cyclopropanecarboxamido)-4-((4-methoxy-5-(2,2,2-trifluoro-1-methoxyethyl)benzo[d]isothiazol-3-yl)amino)nicotinate (67B)

**67** (16 mg, 0.0313 mmol) was separated by Chiral-HPLC (Method M) to give **67A** (1.9 mg, 12% yield) as a white solid and **67B** (3.2 mg, 20% yield) as a white solid.

**67A:** ¹H NMR (400 MHz, DMSO-*d*₆) δ 11.87 (s, 1H), 11.05 (s, 1H), 9.85 (s, 1H), 8.85 (s, 1H), 8.05 (d, *J* = 8.4 Hz, 1H), 7.65 (d, *J =* 8.4 Hz, 1H), 5.48-5.43 (m, 1H), 3.95 (s, 3H), 3.93 (s, 3H), 3.39 (s, 3H), 2.07-2.00 (m, 1H), 0.90-0.82 (m, 4H). LC-MS (ESI, Method 2): t_{R} = 1.05 min, m/z (M+H)⁺ = 511.0. Chiral HPLC (Method 11): t_{R} = 15.07 min.

**67B:** ¹H NMR (400 MHz, DMSO-*d*₆) δ 11.88 (s, 1H), 11.06 (s, 1H), 9.86 (s, 1H), 8.86 (s, 1H), 8.06 (d, *J* = 8.4 Hz, 1H), 7.66 (d, *J =* 8.4 Hz, 1H), 5.49-5.43 (m, 1H), 3.94 (s, 3H), 3.91 (s, 3H), 3.40 (s, 3H), 2.10-2.01 (m, 1H), 0.91-0.82 (m, 4H). LC-MS (ESI, Method 2): t_{R} = 1.05 min, m/z (M+H)⁺ = 511.0. Chiral HPLC (Method 11): t_{R} = 19.39 min.

### Example 68

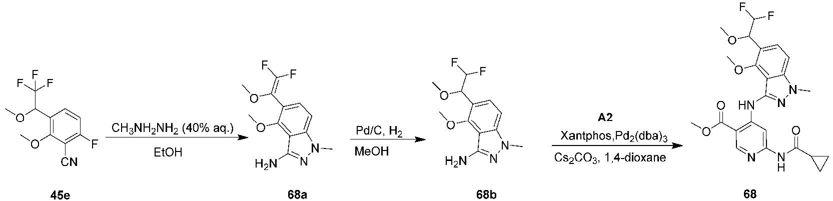

### Step 1. 5-(2,2-Difluoro-1-methoxyvinyl)-4-methoxy-1-methyl-1H-indazol-3-amine (68a)

A mixture of **45e** (2.0 g, 7.6 mmol) and methylhydrazine solution (14 mL, 40% in water) and EtOH (7 mL) was stirred at 90 °C for 4 h. After cooling to room temperature, the mixture was diluted with water (18 mL) and extracted with EtOAc (25 mL*2). The combined organic layer was concentrated and purified by flash chromatography on silica gel (PE/EtOAc = 5/1) to give the crude compound **68a** (230 mg, ~20-30% purity) as a yellow oil. LC-MS (ESI, Method 3): t_{R} = 1.16 min, m/z (M+H)⁺ = 270.4.

### Step 2. 5-(2,2-Difluoro-1-methoxyethyl)-4-methoxy-1-methyl-1H-indazol-3-amine (68b)

A mixture of **68a** (230 mg, 0.85 mmol), Pd/C (23 mg, 10% Palladium on Carbon wetted with 50% water) in MeOH (10 mL) was stirred at 60 °C for 16 h under H₂ atmosphere (1 atm). After cooling to room temperature, the mixture was filtered and concentrated. The residue was purified by *Prep*-HPLC (Method A, NH₄HCO₃) to give **68b** (20 mg, 9% yield) as a white solid. LC-MS (ESI, Method 3): t_{R} = 1.11 min, m/z (M+H)⁺ = 272.2.

### Step 3. Methyl 6-(cyclopropanecarboxamido)-4-((5-(2,2-difluoro-1-methoxyethyl)-4-methoxy-1-methyl-1H-indazol-3-yl)amino)nicotinate (68)

A mixture of **68b** (28.1 mg, 103.7 µmol), **A2** (29.1 mg, 114.1 mmol), Cs₂CO₃ (67.6 mg, 207.2 µmol), Xantphos (12.0 mg, 20.74 µmol) and Pd₂(dba)₃ (19.0 mg, 20.74 µmol) in 1,4-dioxane (0.5 mL) was stirred at 90 °C for 4 h under N₂ atmosphere in sealed tube. The mixture was concentrated and purified by *Prep*-HPLC (Method A, NH₄HCO₃) to give **68** (5.0 mg, 10%yield) as a white solid. ¹H NMR (400 MHz, DMSO-*d*₆) δ 10.91 (s, 1H), 10.88 (s, 1H), 9.20 (s, 1H), 8.77 (s, 1H), 7.44 (d, *J =* 8.8 Hz, 1H), 7.41 (d, *J* = 8.8 Hz, 1H), 6.29 (td, *J =* 55.2, 4.4 Hz,1H), 4.92-4.82 (m, 1H), 3.97 (s, 3H), 3.95 (s, 3H), 3.87 (s, 3H), 3.23 (s, 3H), 2.07-2.01 (m, 1H), 0.86-0.79 (m, 4H). LC-MS (ESI, Method 2): t_{R} = 0.92 min, m/z (M+H)⁺ = 490.1.

### Example 69

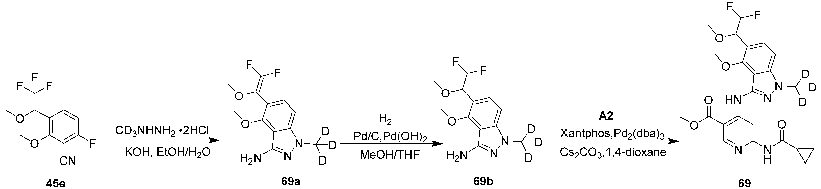

### Step 1. 5-(2,2-Difluoro-1-methoxyvinyl)-4-methoxy-1-(methyl-d₃)-1H-indazol-3-amine (69a)

To a mixture of hydrazine, methyl-*d*₃-, hydrochloride (1:2) (1.50 g, 12.30 mmol, CAS: 20165-35-9) in EtOH/H₂O (0.5 mL/0.1 mL) was added KOH (1.34 g, 23.94 mmol) at ice-bath and stirred at 30 °C for 30 min. The solid was filtered off and the filtrate was added **45e** (175 mg, 0.66 mmol mmol) and stirred at 90 °C for 2 h in sealed tube. After cooling to room temperature, the mixture was purified by *Prep*-HPLC (Method C) to give compound **69a** (15 mg, 8% yield) as a yellow oil. LC-MS (ESI, Method 3): t_{R} = 1.16 min, m/z (M+H)⁺ = 273.4.

### Step 2. 5-(2,2-Difluoro-1-methoxyethyl)-4-methoxy-1-(methyl-d₃)-1H-indazol-3-amine (69b)

A mixture of **69a** (15 mg, 55.05 µmol), Pd/C (5 mg, 10% Palladium on Carbon wetted with 50% water) and Pd(OH)₂ (5 mg) in MeOH (0.5 mL) and THF (1 mL) was stirred at 40 °C for 16 h under H₂ atmosphere (1 atm). After cooling to room temperature, the mixture was filtered and concentrated. The residue was purified by *Prep*-HPLC (Method A) to give **69b** (10 mg, 67% yield) as a yellow oil. LC-MS (ESI, Method 3): t_{R} = 1.11 min, m/z (M+H)⁺ = 275.3.

### Step 3. Methyl 6-(cyclopropanecarboxamido)-4-((5-(2,2-difluoro-1-methoxyethyl)-4-methoxy-1-(methyl-d₃)-1H-indazol-3-yl)amino)nicotinate (69)

A mixture of **69b** (25 mg, 0.0912 mmol), **A2** (25.5 mg, 0.100 mmol), Cs₂CO₃ (59.4 mg, 0.182 mmol), Xantphos (10.6 mg, 0.0182 mmol) and Pd₂(dba)₃ (16.7 mg, 0.0182 mmol) in 1,4-dioxane (0.5 mL) was stirred at 90 °C for 4 h under N₂ atmosphere in a sealed tube. The mixture was concentrated and purified by *Prep*-TLC (DCM/MeOH = 20/1) to give the crude product. Then the crude compound was re-purified by *Prep*-HPLC (Method A) to afford **69** (14.9 mg, 33% yield) as a white solid. ¹H NMR (400 MHz, DMSO-*d*₆) δ 10.91 (s, 1H), 10.88 (s, 1H), 9.20 (s, 1H), 8.77 (s, 1H), 7.44 (d, *J =* 8.8 Hz, 1H), 7.41 (d, *J* = 8.8 Hz, 1H), 6.29 (td, *J =* 55.2, 4.4 Hz,1H), 4.92-4.82 (m, 1H), 3.92 (s, 3H), 3.87 (s, 3H), 3.23 (s, 3H), 2.08-1.98 (m, 1H), 0.89-0.76 (m, 4H). LC-MS (ESI, Method 2): t_{R} = 0.93 min, m/z (M+H)⁺ = 493.1.

### Example 70

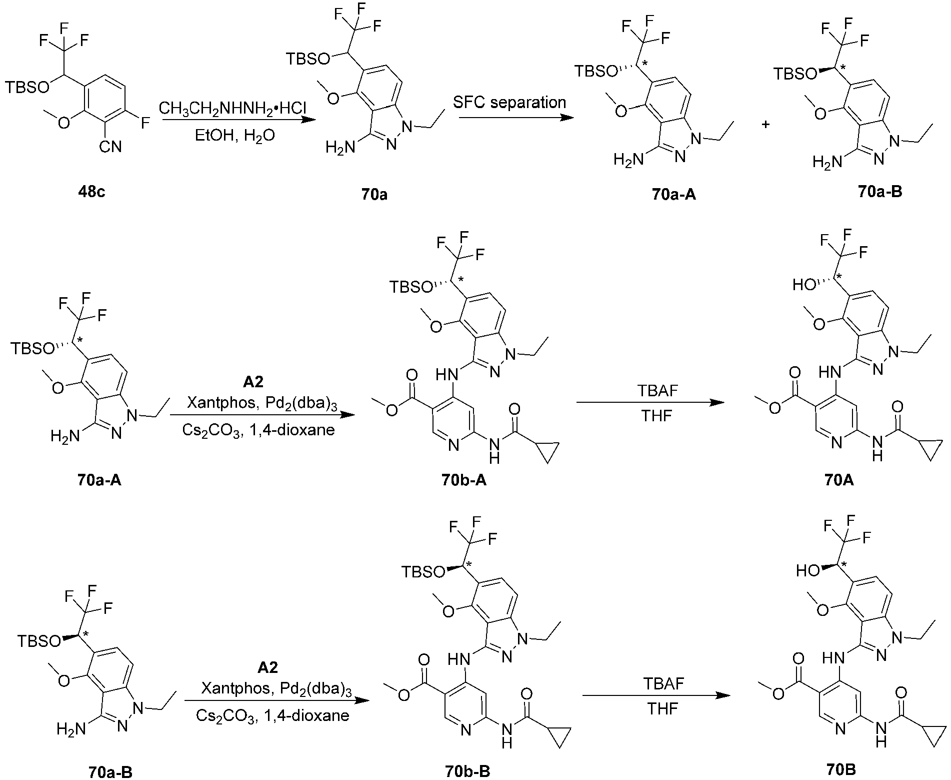

### Step 1. 5-(1-((Tert-butyldimethylsilyl)oxy)-2,2,2-trifluoroethyl)-1-ethyl-4-methoxy-1H-indazol-3-amine (70a)

To a solution of CH₃CH₂NHNH₂·HCl (5.86 g, 44.03 mmol) in EtOH/H₂O (15/5 mL) was added KOH (4.94 g, 88.05 mmol) at 0 °C and stirred at 25 °C for 0.5 h. The reaction mixture was filtered, to the filtrate was added **48c** (2 g, 5.50 mmol). The reaction mixture was stirred at 70 °C for 4 h. The mixture was extracted with EtOAc (10 mL*2), The combined organic layer was concentrated and purified by flash chromatography on silica gel (PE/EtOAc = 3/1) to give **70a** (230 mg, 10% yield) as a yellow oil. ¹H NMR (400 MHz, DMSO-*d*₆) δ 7.39 (d, *J =* 8.8 Hz, 1H), 7.29 (d, *J* = 9.2 Hz, 1H), 5.97-5.80 (m, 1H), 5.29 (s, 2H), 4.18 (q, *J* = 7.2 Hz, 2H), 3.96 (s, 3H), 1.37 (t, *J =* 7.2 Hz, 3H), 0.91 (s, 9H), 0.18 (s, 3H), 0.00 (s, 3H). LC-MS (ESI, Method 3): t_{R} = 1.61 min, m/z (M+H)⁺ = 404.3.

### Step 2. (S*)-5-(1-((tert-butyldimethylsilyl)oxy)-2,2,2-trifluoroethyl)-1-ethyl-4-methoxy-1H-indazol-3-amine (70a-A) and (R*)-5-(1-((tert-butyldimethylsilyl)oxy)-2,2,2-trifluoroethyl)-1-ethyl-4-methoxy-1H-indazol-3-amine (70a-B)

**70a** (230 mg, 0.14 mmol) was separated by Chiral-HPLC (Method H, Hex/IPA = 98/2, Flow rate: 25 mL/min) to give **70a-A** (80 mg, 35% yield) as a white solid and **70a-B** (80 mg, 35% yield) as a white solid. **70a-A** Chiral HPLC (Method 7, Hex/IPA = 98/2): t_{R} = 8.68 min. **70a-B** Chiral HPLC (Method 7, Hex/IPA = 98/2): t_{R} = 9.68 min.

### Step 3. Methyl (S*)-4-((5-(1-((tert-butyldimethylsilyl)oxy)-2,2,2-trifluoroethyl)-1-ethyl-4-methoxy-1H-indazol-3-yl)amino)-6-(cyclopropanecarboxamido)nicotinate (70b-A) and Methyl (R*)-4-((5-(1-((tert-butyldimethylsilyl)oxy)-2,2,2-trifluoroethyl)-1-ethyl-4-methoxy-1H-indazol-3-yl)amino)-6-(cyclopropanecarboxamido)nicotinate (70b-B)

A mixture of **70a-A** (30.0 mg, 74.35 µmol) or **70a-B** (30.0 mg, 74.35 µmol), **A2** (22.7 mg, 89.22 µmol), Cs₂CO₃ (48.5 mg, 148.69 µmol), Xantphos (8.60 mg, 14.87 µmol) and Pd₂(dba)₃ (13.6 mg, 14.87 µmol) in 1,4-dioxane (0.4 mL) was stirred at 90 °C under N₂ atmosphere for 6 h. The reaction mixture was concentrated and purified by flash chromatography on silica gel (PE/EtOAc = 3/1) to give **70b-A** (35 mg, 76% yield) or **70b-B** (40 mg, 87% yield) as a yellow solid. **70b-A:** LC-MS (ESI, Method 3): t_{R} = 1.69 min, m/z (M+H)⁺ = 622.5. **70b-B:** LC-MS (ESI, Method 3): t_{R} = 1.68 min, m/z (M+H)⁺ = 622.5.

### Step 4. Methyl (S*)-6-(cyclopropanecarboxamido)-4-((1-ethyl-4-methoxy-5-(2,2,2-trifluoro-1-hydroxyethyl)-1H-indazol-3-yl)amino)nicotinate (70A) and Methyl (R*)-6-(cyclopropanecarboxamido)-4-((1-ethyl-4-methoxy-5-(2,2,2-trifluoro-1-hydroxyethyl)-1H-indazol-3-yl)amino)nicotinate (70B)

To a solution of **70b-A** (35.0 mg, 56.30 µmol) in THF (0.5 mL) was added TBAF (0.5 mL, 0.5 mmol, 1.0 M in THF) at 0 °C and the mixture was stirred at 25 °C for 1 h. The reaction mixture was diluted into H₂O (1 mL) and extracted with EtOAc (1 mL*2). The combined organic layer was concentrated and purified by flash chromatography on silica gel (DCM/MeOH = 20/1) to give **70A** (19.5 mg, 68% yield) as a yellow solid. ¹H NMR (400 MHz, DMSO-*d*₆): δ 10.91 (s, 1H), 10.89 (s, 1H), 9.29 (s, 1H), 8.77 (s, 1H), 7.53 (d, *J =* 8.8 Hz, 1H), 7.47 (d, *J* = 9.2 Hz, 1H), 6.84(d, *J =* 5.6 Hz, 1H), 5.46-5.42 (m, 1H), 4.33 (q, *J =* 7.2 Hz, 2H), 3.92 (s, 3H), 3.87 (s, 3H), 2.05-2.02 (m, 1H), 1.48 (t, *J =* 7.2 Hz, 3H), 0.83-0.81 (m, 4H). LC-MS (ESI, Method 2): t_{R} = 0.92 min, m/z (M+H)⁺ = 508.1. Chiral HPLC (Method 6, Hex/EtOH/DEA = 80/20/0.2): t_{R} = 7.67 min.

To a solution of **70b-B** (40.0 mg, 64.34 µmol) in THF (0.3 mL) was added TBAF (0.3 mL, 1.0 M in THF, 0.3 mmol) at 0 °C and the mixture was stirred at 25 °C for 1 h. The reaction mixture was diluted into H₂O (1 mL) and extracted with EtOAc (1 mL*2). The combined organic layer was concentrated and purified by flash chromatography (DCM/MeOH = 20/1) to give **70B** (20.5 mg, 63% yield) as a white solid. ¹H NMR (400 MHz, DMSO-*d*₆): δ 10.91 (s, 1H), 10.88 (s, 1H), 9.29 (s, 1H), 8.77 (s, 1H), 7.53 (d, *J =* 8.8 Hz, 1H), 7.47 (d, *J =* 8.8 Hz, 1H), 6.83 (d, *J* = 5.6 Hz, 1H), 5.46-5.42 (m, 1H), 4.33 (q, *J =* 7.2 Hz, 2H), 3.92 (s, 3H), 3.87(s, 3H), 2.06-2.02 (m, 1H), 1.48 (t, *J =* 7.2 Hz, 3H), 0.83-0.81 (m, 4H). LC-MS (ESI, Method 2): t_{R} = 0.93 min, m/z (M+H)⁺ = 508.1. Chiral HPLC (Method 6, Hex/EtOH/DEA = 80/20/0.2): t_{R} = 9.14 min.

### Example 71

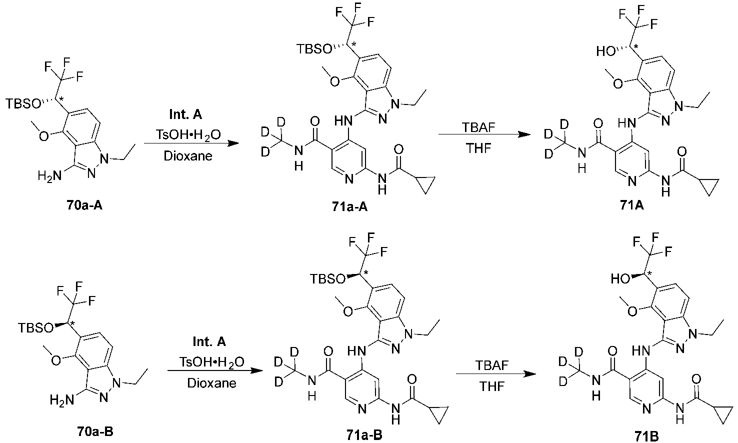

### Step 1. (S*)-4-((5-(1-((tert-butyldimethylsilyl)oxy)-2,2,2-trifluoroethyl)-1-ethyl-4-methoxy-1H-indazol-3-yl)amino)-6-(cyclopropanecarboxamido)-N-(methyl-d₃)nicotinamide (71a-A) and (R*)-4-((5-(1-((tert-butyldimethylsilyl)oxy)-2,2,2-trifluoroethyl)-1-ethyl-4-methoxy-1H-indazol-3-yl)amino)-6-(cyclopropanecarboxamido)-N-(methyl-d₃)nicotinamide (71a-B)

To a solution of **70a-A** (45 mg, 0.112 mmol) and **Int. A** (34.4 mg, 0.134 mmol) in 1,4-dioxane (0.5 mL) was added TsOH·H₂O (2.1 mg, 11.2 µmol) and stirred at 110 °C for 72 h. The reaction mixture was concentrated and purified by flash chromatography on silica gel (DCM/MeOH = 20/1) to give **71a-A** (50 mg, 72% yield) as a yellow solid. LC-MS (ESI, Method 3): t_{R} = 1.77 min, m/z (M+H)⁺ = 624.5.

To a solution of **70a-B** (50 mg, 123.9 µmol) and **Int. A** (38.2 mg, 0.148 mmol) in 1,4-dioxane (0.5 mL) was added TsOH·H₂O (2.4 mg, 12.39 µmol) and the mixture was stirred at 110 °C for 48 h. The reaction mixture was concentrated and purified by flash chromatography (DCM/MeOH = 20/1) to give **71a-B** (60 mg, 78% yield) as a yellow solid. LC-MS (ESI, Method 3): t_{R} = 1.77 min, m/z (M+H)⁺ = 624.3.

### Step 2. (S*)-6-(cyclopropanecarboxamido)-4-((1-ethyl-4-methoxy-5-(2,2,2-trifluoro-1-hydroxyethyl)-1H-indazol-3-yl)amino)-N-(methyl-d₃)nicotinamide (71A) and (R*)-6-(cyclopropanecarboxamido)-4-((1-ethyl-4-methoxy-5-(2,2,2-trifluoro-1-hydroxyethyl)-1H-indazol-3-yl)amino)-N-(methyl-d₃)nicotinamide (71B)

To a solution of **71a-A** (50 mg, 80.16 µmol) in THF (0.5 mL) was added TBAF (0.5 mL, 0.5 mmol, 1.0 M in THF) at 0 °C and the mixture was stirred at 25 °C for 1 h. The reaction mixture was diluted into H₂O (1 mL), extracted with EtOAc (1 mL*2). The organic layer was concentrated and purified by flash chromatography on silica gel (DCM/MeOH = 20/1) to give **71A** (18.1 mg, 44% yield) as a white solid. ¹H NMR (400 MHz, DMSO-*d*₆) δ 11.61 (s, 1H), 10.73 (s, 1H), 9.24 (s, 1H), 8.64 (s, 1H), 8.57 (s, 1H), 7.50 (d, *J* = 8.8 Hz, 1H), 7.43 (d, *J* = 8.8 Hz, 1H), 6.82 (s, 1H), 5.45-5.43 (m, 1H), 4.30 (q, *J =* 7.2 Hz, 2H), 3.87 (s, 3H), 2.02-2.00 (m, 1H), 1.46 (t, *J =* 7.2 Hz, 3H), 0.81-0.79 (m, 4H). LC-MS (ESI, Method 2): t_{R} = 0.87 min, m/z (M+H)⁺ = 510.2. Chiral HPLC (Method 7, Hex/EtOH/DEA = 70/30/0.2): t_{R} = 5.33 min.

To a solution of **71a-B** (60 mg, 96.19 µmol) in THF (0.5 mL) was added TBAF (0.3 mL, 1.0 M in THF, 0.3 mmol) at 0 °C and the mixture was stirred at 25 °C for 2 h. The reaction mixture was diluted into H₂O (3 mL), extracted with EtOAc (6 mL*2). The organic layer was concentrated and purified by flash chromatography (DCM/MeOH = 20/1) to give **71B** (27.1 mg, 55% yield) as a white solid. ¹H NMR (400 MHz, DMSO-*d*₆) δ 11.59 (s, 1H), 10.70 (s, 1H), 9.23 (s, 1H), 8.63 (s, 1H), 8.57 (s, 1H), 8.39 (s, 1H), 7.51 (d, *J =* 8.8 Hz, 1H), 7.43 (d, *J =* 8.8 Hz, 1H), 5.47-5.42 (m, 1H), 4.31 (q, *J =* 7.2 Hz, 2H), 3.88 (s, 3H), 2.04-2.01 (m, 1H), 1.47 (t, *J =* 7.2 Hz, 3H), 0.81-0.79 (m, 4H). LC-MS (ESI, Method 2): t_{R} = 0.87 min, m/z (M+H)⁺ = 510.2. Chiral HPLC (Method 7, Hex/EtOH/DEA = 70/30/0.2): t_{R} = 7.02 min.

### Example 72

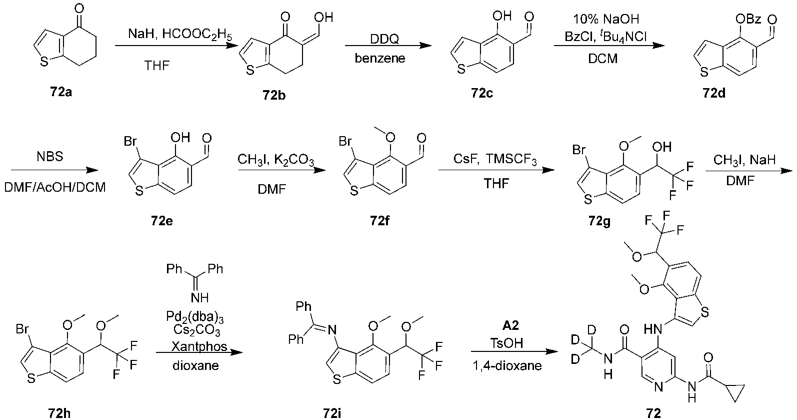

### Step 1. 6,7-Dihydrobenzo[b]thiophen-5(4H)-ylidene-methanol (72b)

To a suspension of NaH (7.1 g, 295.6 mmol, 60% dispersion in mineral oil) in THF (630 mL) was added HCOOC₂H₅ (21.88 g, 295.6 mmol) at 0 °C. The mixture was stirred at 0 °C and then a solution of **72a** (9 g, 59.1 mmol) in THF (630 mL) was added to the mixture at 0 °C. The mixture was stirred at room temperature for 24 h. The mixture was quenched with 1 mol/L HCl to adjust to pH = 2, and then the mixture was extracted with EtOAc (50 mL*2). The combined organic layer was concentrated and purified by flash chromatography on silica gel (PE/EtOAc = 4/1) to give **72b** (9.5 g, 89% yield) as a yellow oil. ¹H NMR (300 MHz, CDCl₃) δ 13.97 (s, 1H), 7.53 (s, 1H), 7.46 (d, *J =* 5.1 Hz, 1H), 7.16 (d, *J =* 5.1 Hz, 1H), 3.04 (t, *J =* 6.6 Hz, 2H), 2.71 (d, *J =* 6.9 Hz, 2H).

### Step 2. 4-Hydroxybenzo[b]thiophene-5-carbaldehyde (72c)

A mixture of **72b** (5 g, 27.7 mmol) and DDQ (7.56 g, 33.29 mmol) in benzene (60 mL) was stirred at 90 °C for 3 h. The solid was filtered off and the filtrate was concentrated and diluted with EtOAc (10 mL). The organic layer was washed with 10 mL *sat*. NaHCO₃ and concentrated. The residue was purified by flash chromatography on silica gel (PE/EtOAc = 20/1) to give **72c** (3.2 g, 64% yield) as a yellow solid. ¹H NMR (300 MHz, DMSO-*d*₆) δ 11.62 (s, 1H), 10.26 (s, 1H), 7.80 (d, *J =* 5.7 Hz, 1H), 7.74 (d, *J =* 5.4 Hz, 1H), 7.67 (d, *J =* 8.7 Hz, 1H), 7.64 (d, *J =* 8.7 Hz, 1H).

### Step 3. 5-Formylbenzo[b]thiophen-4-yl benzoate (72d)

To a solution of 72c (2.1 g, 11.78 mmol), 10% aq. NaOH (3.7 mL, 11.7 mmol) and tetrabutylammonium chloride (327.5 mg, 1.18 mmol) in DCM (5 mL) was added benzoyl chloride (2.48 g, 17.68 mmol) at 0 °C. The mixture was stirred at 0 °C for 30 min. The reaction was diluted with H₂O (5 mL) and extracted with DCM (30 mL). The organic layer was concentrated and purified by flash chromatography on silica gel (PE/EtOAc=10/1) to afford **72d** (3 g, 90% yield) as a yellow solid. ¹H NMR (400 MHz, CDCl₃) δ 10.22 (s, 1H), 8.25-8.23 (m, 2H), 8.04 (d, *J* = 8.4 Hz, 1H), 8.02 (d, *J* = 8.4 Hz, 1H), 7.67-7.63 (m, 1H), 7.54-7.51 (m, 2H), 7.45 (d, *J* = 5.6 Hz, 1H), 7.29 (d, *J=* 5.6 Hz, 1H).

### Step 4. 3-Bromo-4-hydroxybenzo[b]thiophene-5-carbaldehyde (72e)

A mixture of **72d** (380.0 mg, 1.35 mmol) and NBS (838.5 mg, 4.71 mmol) in DMF/AcOH/DCM (3 mL, v/v/v = 1/1/1) was stirred at 75 °C for 16 h. After cooling to room temperature, the mixture was diluted with H₂O (10 mL) and extracted with EtOAc (10 mL*2). The combined organic layer was concentrated and purified by flash chromatography on silica gel (PE/EtOAc = 5/1) to afford **72e** (300 mg, 87% yield) as a yellow solid. ¹H NMR (400 MHz, CDCl₃) δ 12.53 (s, 1H), 9.95 (s, 1H), 7.47 (d, *J =* 8.4 Hz, 1H), 7.42 (d, *J =* 8.4 Hz, 1H), 7.37 (s, 1H).

### Step 5. 3-Bromo-4-methoxybenzo[b]thiophene-5-carbaldehyde (72f)

To a mixture of **72e** (300.0 mg, 1.17 mmol) and K₂CO₃ (322.5 mg, 2.33 mmol) in DMF (0.5 mL) was added CH₃I (329.0 mg, 2.33 mmol) at 0 °C. The mixture was stirred at 25 °C for 2 h, then it was diluted with water (5 mL) and extracted with EtOAc (5 mL*3). The combined organic layer was concentrated and purified by flash chromatography on silica gel (PE/EtOAc = 20/1) to afford **72f** (100 mg, 32% yield) as a yellow solid. ¹H NMR (300 MHz, CDCl₃) δ 10.57 (s, 1H), 7.91 (d, *J* = 8.4 Hz, 1H), 7.72 (d, *J =* 8.4 Hz, 1H), 7.52 (s, 1H), 4.13 (s, 3H).

### Step 6. 1-(3-Bromo-4-methoxybenzo[b]thiophen-5-yl)-2,2,2-trifluoroethan-1-ol (72g)

To a solution of **72f** (100 mg, 0.369 mmol) and TMSCF₃ (104.8 mg, 0.738 mmol) in THF (2 mL) was added CsF (56 mg, 0.368 mmol) at 0 °C. The mixture was stirred at 30 °C for 1 h. 1 mol/L HCl (2 mL) was added into the mixture and the mixture was stirred at 30 °C for 1 h. The pH was adjusted to 8 with sat. NaHCO₃ and extracted with EtOAc (20 ml*2). The combined organic layer was concentrated and purified by flash chromatography on silica gel (PE/EtOAc = 10/1) to afford **72g** (95.0 mg, 86% yield) as a yellow solid. ¹H NMR (400 MHz, CDCl₃) δ 7.60 (d, *J =* 8.8 Hz, 1H), 7.47 (d, *J =* 8.8 Hz, 1H), 7.18 (s, 1H), 5.57-5.49 (m, 1H), 3.88 (s, 3H).

### Step 7. 3-Bromo-4-methoxy-5-(2,2,2-trifluoro-1-methoxyethyl) benzo[b]thiophene (72h)

To a solution of **72g** (95 mg, 0.278 mmol) and CH₃I (79.1 mg, 0.557mmol) in DMF (1 mL) was slowly added NaH (22.3 mg, 0.556 mmol) at 0 °C. The mixture was stirred at 25 °C for 1 h and poured into ice-water (5 mL). The mixture was extracted with EtOAc (10 mL*2), and the combined organic phase was washed with brine (3 mL), dried over Na₂SO₄, filtered and concentrated. The residue was purified by flash chromatography on silica gel (PE/EtOAc = 10/1) to afford **72h** (80.0 mg, 81% yield) as a yellow solid. ¹H NMR (400 MHz, CDCl₃) δ 7.70 (d, *J =* 8.4 Hz, 1H), 7.56 (d, *J* = 8.4 Hz, 1H), 7.46 (s, 1H), 5.21-5.19 (m, 1H), 3.95 (s, 3H), 3.39 (s, 3H).

### Step 8. N-(4-methoxy-5-(2,2,2-trifluoro-1-methoxyethyl)benzo[b]thiophen-3-yl)-1,1-diphenylmethanimine (72i)

A mixture of **72h** (80.0 mg, 0.225 mmol), diphenylmethanimine (122.5 mg, 0.677 mmol), Xantphos (13.0 mg, 0.0225 mmol), Cs₂CO₃ (146.8 mg, 0.450 mmol) and Pd₂(dba)₃ (20.6 mg, 0.0225 mmol) in 1,4 -dioxane (0.5 mL) was stirred at 100 °C for 16 h under N₂ atmosphere. The mixture was concentrated and purified by flash chromatography on silica gel (PE/EtOAc = 5/1) to afford **72i** (100.0 mg, 98% yield) as a green oil. LC-MS (ESI, Method 3): t_{R} = 1.71 min, m/z (M+H)⁺ = 456.2.

### Step 9. 6-(Cyclopropanecarboxamido)-4-((4-methoxy-5-(2,2,2-trifluoro-1-methoxyethyl)benzo[b]thiophen-3-yl)amino)-N-(methyl-d₃)nicotinamide (72)

A mixture of **72i** (75.0 mg, 0.165 mmol), **Int. A** (42.3 mg, 0.165 mol) and TsOH (2.8 mg, 0.0165 mmol) in 1,4-dioxane (2 mL) was stirred at 90 °C for 16 h. The mixture was concentrated and purified by *Prep*-HPLC (Method A) to afford **72** (10 mg, 12% yield) as a white solid. ¹H NMR (400 MHz, DMSO-*d*₆) δ 10.97 (s, 1H), 10.76 (s, 1H), 8.58 (s, 1H), 8.53 (s, 1H), 8.19 (s, 1H), 7.89 (d, *J =* 8.4 Hz, 1H), 7.53 (s, 1H), 7.42 (d, *J =* 8.4 Hz, 1H), 5.34-5.32 (m, 1H), 3.72 (s, 3H), 3.29 (s, 3H), 1.98-1.95 (m, 1H), 0.77-0.75 (m, 4H). LC-MS (ESI, Method 1): t_{R} = 1.35 min, m/z (M+H)⁺ = 512.1.

### Example 73

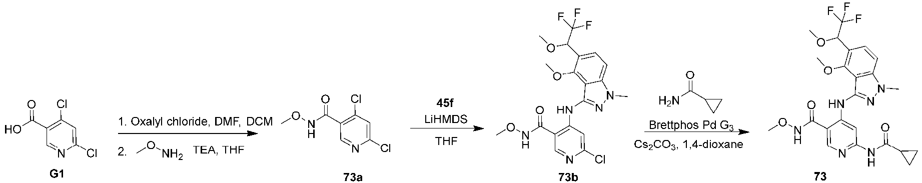

### Step 1. 4,6-Dichloro-N-methoxynicotinamide (73a)

To a solution of **G1** (500 mg, 2.60 mmol) and DMF (9.5 mg, 0.13 mmol) in DCM (2 mL) was added oxalyl chloride (727 mg, 5.73 mmol) at 0 °C. The mixture was stirred at 0 °C for 30 min and concentrated to give a crude. A mixture of *O*-methylhydroxylamine hydrochloride (435 mg, 5.21 mmol) and KOH (292 mg, 5.21 mmol) in THF (2 mL) was stirred at room temperature for 1 h. The solid was filtered off and filtrate was cooled to 0 °C. To the solution was added TEA (1.05 g, 10.42 mmol, 1.45 mL), followed by a solution of the previous crude in 2 mL THF. The reaction was stirred at 0 °C for 1.5 h. The reaction mixture was diluted with water (5 mL) and extracted with DCM (5 mL*3). The combined organic layer was concentrated and purified by flash chromatography on silica gel (DCM/MeOH= 40/1) to give **73a** (404 mg, 70% yield) as a yellow solid. ¹H NMR (400 MHz, DMSO-*d*₆) δ 11.85 (s, 1H), 8.53 (s, 1H), 7.96 (s, 1H),3.74 (s, 3H). LC-MS (ESI, Method 3): t_{R} = 0.92 min, m/z (M+H)⁺ = 221.0.

### Step 2. 6-Chloro-N-methoxy-4-((4-methoxy-1-methyl-5-(2,2,2-trifluoro-1-methoxyethyl)-1H-indazol-3-yl)amino)nicotinamide (73b)

To a solution of **45f** (50 mg, 0.173 mmol) and **73a** (38.2 mg, 0.173 mmol) in THF (0.4 mL) was added LiHMDS (0.7 mL, 0.7 mmol, 1 M in THF) at -60 °C. The reaction mixture was stirred at -40 °C to 25 °C for 4 h and quenched with H₂O (2 mL). The organic layer was evaporated, the formed solid was collected by filtering. The filter cake was dried to give **73b** (70 mg, 92% yield) as a brown solid. LC-MS (ESI, Method 2): t_{R} = 1.39 min, m/z (M+H)⁺ = 474.2.

### Step 3. 6-(Cyclopropanecarboxamido)-N-methoxy-4-((4-methoxy-1-methyl-5-(2,2,2-trifluoro-1-methoxyethyl)-1H-indazol-3-yl)amino)nicotinamide (73)

A mixture of **73b** (75 mg, 0.158 mmol), cyclopropanecarboxamide (67.4 mg, 0.791 mmol), Cs₂CO₃ (103.1 mg, 0.317 mmol) and BrettPhos Pd G3 (28.7 mg, 0.0317 mmol) in 1,4-dioxane (1 mL) was stirred at 90 °C for 4 h under N₂ atmosphere. The mixture was concentrated and purified by flash chromatography on silica gel (DCM/MeOH = 20/1) to give the crude product. The crude product was purified by *Prep*-HPLC (Method A) to give **73** (10.5 mg, 13% yield) as a white solid. 1H NMR (400 MHz, DMSO-*d*₆) δ 11.93 (s, 1H), 11.47 (s, 1H), 10.67 (s, 1H), 9.05 (s, 1H), 8.47 (s, 1H), 7.43 (d, *J =* 8.8 Hz, 1H), 7.38 (d, *J =* 8.8 Hz, 1H), 5.29-5.25 (m, 1H), 3.94 (s, 3H), 3.82 (s, 3H), 3.72 (s, 3H), 3.23 (s, 3H), 2.03-1.99 (m, 1H), 0.86-0.80 (m, 4H). LC-MS (ESI, Method 2): t_{R} = 0.90 min, m/z (M+H)⁺ = 523.1.

### Example 74

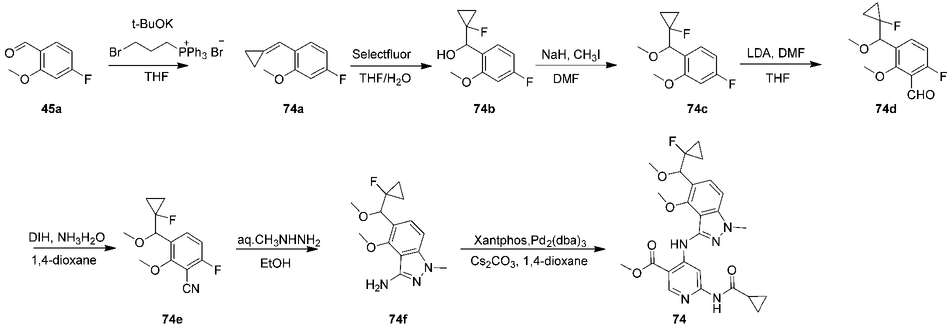

### Step 1. 1-(Cyclopropylidenemethyl)-4-fluoro-2-methoxybenzene (74a)

To a solution of (3-Bromopropyl)triphenylphosphonium bromide (9.94 g, 21.41 mmol) in THF (42 mL) was dropwise added a solution of ^{t}BuOK (4.80 g, 42.82 mmol) in THF (42 mL) at 0 °C. Then the mixture was stirred at 70 °C for 2 h. a solution of **45a** (3.0 g, 19.46 mmol) in THF (30 mL) was added into the reaction mixture at 10 °C. The mixture was stirred at 70 °C for 12 h. After cooling to room temperature, the suspension was filtered and concentrated. The residue was purified by flash chromatography on silica gel (PE) to give **74a** (1.32 g, 38% yield) as a yellow oil. ¹H NMR (400 MHz, DMSO-*d*₆) δ 7.67 (dd, *J =* 8.8, 6.8 Hz, 1H), 6.93-6.89 (m, 2H), 6.78-6.74 (m, 1H), 3.82 (s, 3H), 1.41-1.32 (m, 2H), 1.17-1.06 (m, 2H).

### Step 2. (4-Fluoro-2-methoxyphenyl)(1-fluorocyclopropyl)methanol (74b)

To a solution of **74a** (1.32 g, 7.41 mmol) in THF (11 mL) and H₂O (3 mL) was added selectfluor (5.25 g, 14.81 mmol) at 0 °C. Then the mixture was stirred at 15 °C for 16 h. The mixture was diluted with DCM (30 mL) and washed with H₂O (12 mL). The organic layer was concentrated at 35 °C under reduced pressure and the residue was purified by flash chromatography on silica gel (PE/EtOAc = 4/1) to give **74b** (1.3 g. 82% yield) as a yellow oil. ¹H NMR (400 MHz, DMSO-*d*₆) δ 7.47-7.43 (m, 1H), 6.89-6.85 (m, 1H), 6.78-6.73 (m, 1H), 5.58 (d, *J* = 5.2 Hz, 1H), 5.01 (dd, *J =* 18.0, 5.2 Hz, 1H), 3.79 (s, 3H), 1.01-0.77 (m, 2H), 0.76-0.65 (m, 1H), 0.64-0.52 (m, 1H).

### Step 3. 4-Fluoro-1-((1-fluorocyclopropyl)(methoxy)methyl)-2-methoxybenzene (74c)

To a solution of **74b** (1.4 g, 6.54 mmol) in DMF (14 mL) was added NaH (314 mg, 7.84 mmol, 60% in mineral oil) at 0 °C and stirred at 0 °C for 30 min. Then CH₃I (928 mg, 6.54 mmol) was added into the mixture and stirred at 0 °C for 1 h. The mixture was added into ice-water (30 mL) and extracted with EtOAc (50 mL*2). The combined organic layer was concentrated at 35 °C under reduced pressure and the residue was purified by flash chromatography on silica gel (PE/EtOAc = 20/1) to give **74c** (1.2 g, 80% yield) as a yellow oil. ¹H NMR (400 MHz, DMSO-*d*₆) δ 7.31-7.29 (m, 1H), 6.96-6.92 (m, 1H), 6.81-6.76 (m, 1H), 4.70 (d, *J* = 19.2 Hz, 1H), 3.81 (s, 3H), 3.23 (s, 3H), 1.07-0.94 (m, 1H), 0.92-0.78 (m, 1H), 0.73-0.63 (m, 1H), 0.62-0.53 (m, 1H).

### Step 4. 6-Fluoro-3-((1-fluorocyclopropyl)(methoxy)methyl)-2-methoxybenzaldehyde (74d)

To a solution of **74c** (200 mg, 0.876 mmol) in THF (2 mL) was added a solution of LDA (1.10 mL, 2.2 mmol, 2 M in THF) at -50 °C. The mixture was stirred at -50 °C for 30 min. Then DMF (128.1 mg, 1.75 mmol) was added into the reaction mixture and stirred at -50 °C for 2 h and stirred at 0 °C for 30 min. The mixture was quenched with water (6 mL) and extracted with EtOAc (10 mL*2). The combine organic layer was concentrated to give **74d** (224 mg, crude) as a yellow oil which was used for the next step directly without further purification.

### Step 5. 6-Fluoro-3-((1-fluorocyclopropyl)(methoxy)methyl)-2-methoxybenzonitrile (74e)

To a solution of **74d** (220 mg, 0.859 mmol) in 1,4-dioxane (1 mL) and NH₃.H₂O (1 mL) was added 1,3-diiodo-5,5-dimethylimidazolidine-2,4-dione (326.2 mg, 0.859 mmol) under ice-water bath. Then the mixture was stirred at 20 °C for 16 h. Then the mixture was diluted with water (8 mL) and extracted with EtOAc (20 mL*2). The combined organic layer was concentrated to give **74e** (217 mg, crude) as a yellow oil which was used for the next step directly without further purification.

### Step 6. 5-((1-Fluorocyclopropyl)(methoxy)methyl)-4-methoxy-1-methyl-1H-indazol-3-amine (74f)

To a solution of **74e** (217 mg, 0.859 mmol) in EtOH (1 mL) was added CH₃NHNH₂ (2 mL, 40% in water) at 10 °C. Then the mixture was stirred at 70 °C for 4 h in a sealed tube. After cooling to room temperature, the reaction mixture was diluted with water (6 mL) and extracted with EtOAc (12 mL*2). The combined organic layer was concentrated and purified by flash chromatography on silica gel (PE/EtOAc = 2/1) to give **74f** (45 mg, 19% yield) as a yellow oil. ¹H NMR (400 MHz, CDCl₃) δ 7.38 (d, *J =* 8.8 Hz, 1H), 6.91 (d, *J =* 8.8 Hz, 1H), 5.20 (d, *J =* 17.2 Hz, 1H), 3.89 (s, 3H), 3.74 (s, 3H), 3.25 (s, 3H), 1.08-0.81 (m, 2H), 0.70-0.69 (m, 1H), 0.59-0.57 (m, 1H). LC-MS (ESI, Method 3): t_{R} = 1.12 min, m/z (M+H)⁺ = 280.2.

### Step 7. Methyl 6-(cyclopropanecarboxamido)-4-((5-((1-fluorocyclopropyl) (methoxy)methyl)-4-methoxy-1-methyl-1H-indazol-3-yl)amino)nicotinate (74)

A mixture of **74f** (43 mg, 0.154 mmol), **A2** (43.1 mg, 0.169 mmol), Pd₂(dba)₃ (28.2 mg, 30.79 µmol), Cs₂CO₃ (100 mg, 0.308 mmol) and Xantphos (17.8 mg, 0.031 mmol) in 1,4-dioxane (0.5 mL) was stirred at 90 °C for 4 h under N₂ atmosphere in a sealed tube. The mixture was concentrated and purified by flash chromatography on silica gel (DCM/MeOH = 19/1) to give the crude product. The crude compound was re-purified by *Prep*-HPLC (Method A) to give **74** (42 mg, 55% yield) as a white solid. ¹H NMR (400 MHz, DMSO-*d*₆) δ 10.89 (s, 2H), 9.17 (s, 1H), 8.76 (s, 1H), 7.43 (d, *J =* 8.4 Hz, 1H), 7.38 (d, *J* = 8.8 Hz, 1H), 4.84 (d, *J =* 17.2 Hz, 1H), 3.96 (s, 3H), 3.92 (s, 3H), 3.86 (s, 3H), 3.23 (s, 3H), 2.07-1.99 (m, 1H), 1.09-0.87 (m, 2H), 0.86-0.75 (m, 6H). LC-MS (ESI, Method 2): t_{R} = 1.22 min, m/z (M+H)⁺ = 498.1.

### Example 75

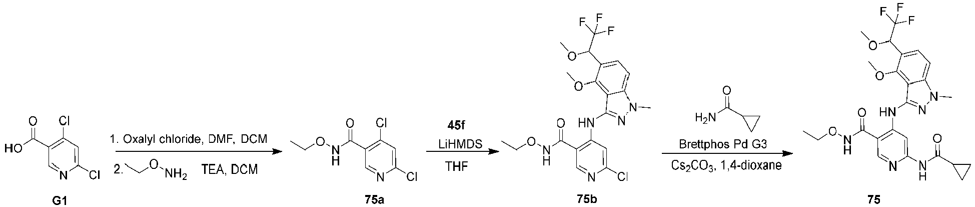

### Step 1. 4,6-Dichloro-N-ethoxynicotinamide (75a)

To a solution of **G1**(700 mg, 3.65 mmol) and DMF (13.32 mg, 0.182 mmol) in DCM (5 mL) was added oxalyl chloride (1.02 g, 8.02 mmol) at 0 °C. The mixture was stirred at 0 °C for 30 min and concentrated. The residue was dissolved in 2 mL of dry DCM and dropwise added to the mixture of *O*-ethylhydroxylamine (711 mg, 7.29 mmol) and TEA (1.48 g, 14.58 mmol) in DCM (5 mL). The mixture was stirred at 25 °C for 2 h and diluted with water. The mixture was extracted with EtOAc (10 mL*3) and concentrated. The residue was purified by flash chromatography on silica gel (DCM/MeOH= 20/1) to give **75a** (620 mg, 72% yield) as a white solid. ¹H NMR (400 MHz, DMSO-*d*₆) δ 11.71 (s, 1H), 8.53 (s, 1H), 7.96 (s, 1H), 3.96 (q, *J =* 6.8 Hz, 2H), 1.22 (t, *J =* 6.8 Hz, 3H). LC-MS (ESI, Method 3): t_{R} = 0.97 min, m/z (M+H)⁺ = 235.0.

### Step 2. 6-Chloro-N-ethoxy-4-((4-methoxy-1-methyl-5-(2,2,2-trifluoro-1-methoxyethyl)-1H-indazol-3-yl)amino)nicotinamide (75b)

To a solution of **45f** (120 mg, 0.415 mmol) and **75a** (97.5 mg, 0.415 mmol) in THF (0.4 mL) was added LiHMDS (1.66 mL, 1.66 mmol, 1 M in THF) at -40 °C. The reaction was stirred at -40 °C to 15 °C for 1 h and quenched with H₂O (6 mL). The mixture was extracted with EtOAc (15 mL*3) and dried over Na₂SO₄, filtered and concentrated to give **75b** (202 mg, crude) as a yellow oil which was used for the next step directly without further purification.LC-MS (ESI, Method 3): t_{R} = 1.12 min, m/z (M+H)⁺ = 488.2.

### Step 3. 6-(Cyclopropanecarboxamido)-N-ethoxy-4-((4-methoxy-1-methyl-5-(2,2,2-trifluoro-1-methoxyethyl)-1H-indazol-3-yl)amino)nicotinamide (75)

A mixture of **75b** (200 mg, 0.41 mmol), cyclopropanecarboxamide (174.4 mg, 2.05 mmol), Cs₂CO₃ (267.1 mg, 0.82 mmol) and BrettPhos Pd G3 (74.3 mg, 0.082 mmol) in 1,4-dioxane (2 mL) was stirred at 90 °C for 8 h under N₂ atmosphere. The mixture was concentrated and purified by flash chromatography on silica gel (DCM/MeOH = 20/1) to give the crude product. The crude product was purified by *Prep*-HPLC (Method A) to give 75 (16.6 mg, 8% yield) as a white solid. ¹H NMR (400 MHz, DMSO-*d*₆) δ 11.80 (s, 1H), 11.46 (s, 1H), 10.66 (s, 1H), 9.02 (s, 1H), 8.47 (s, 1H), 7.43 (d, *J =* 8.8 Hz, 1H), 7.38 (d, *J =* 8.8 Hz, 1H), 5.28 (q, *J* = 8.8 Hz, 1H), 3.97-3.92 (m, 5H), 3. 88 (s, 3H), 3.30 (s, 3H), 2.04-1.97 (m, 1H), 1.22 (t, *J =* 7.2 Hz, 3H), 0.84-0.74 (m, 4H). LC-MS (ESI, Method 2): t_{R} = 1.22 min, m/z (M+H)⁺ = 537.0.

### Example 76

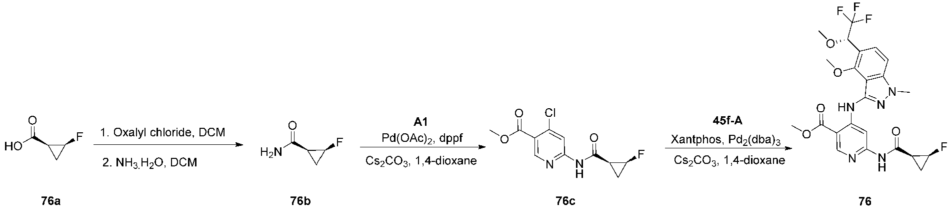

### Step 1. (1S,2S)-2-Fluorocyclopropane-1-carboxamide (76b)

To a solution of **76a** (2 g, 19.22 mmol, CAS: 127199-14-8) in DCM (20 mL) was added oxalyl chloride (3.66 g, 28.82 mmol, 2.51 mL) at 0 °C. The mixture was stirred at 0 °C for 2 h. Then the solvent was removed under reduced pressure and the residue was dissolved in DCM (5 mL). To the solvent was added NH₃·H₂O (3 mL, 33% in water) dropwise at 0 °C. The mixture was stirred for 2 h and concentrated to give **76b** (1.5 g, 76% yield) as a white solid. ¹H NMR (400 MHz, DMSO-*d*₆) δ 7.54 (s, 1H), 6.96 (s, 1H), 4.88-4.68 (m, 1H), 1.75-1.68 (m, 1H), 1.52-1.42 (m, 1H), 1.01-0.93 (m, 1H).

### Step 2. Methyl 4-chloro-6-((1S,2S)-2-fluorocyclopropane-1-carboxamido)nicotinate (76c)

To a solution of **76b** (100 mg, 0.970 mmol), **A1** (400 mg, 1.94 mmol), Pd(OAc)₂(21.8 mg, 0.097 mmol), dppf (53.7 mg, 0.097 mmol) in 1,4-dioxane (1 mL) was added Cs₂CO₃ (948 mg, 2.91 mmol). The mixture was heated to 90 °C for 16 h under N₂ atmosphere. The solvent was removed, and the residue was purified by flash chromatography on silica gel (PE/EtOAc = 4/1) to give **76c** (100 mg, 38% yield) as a yellow solid. ¹H NMR (400 MHz, DMSO-*d*₆) δ 11.47 (s, 1H), 8.82 (s, 1H), 8.28 (s, 1H), 5.07-4.86 (m, 1H), 3.89 (s, 3H), 2.49-1.99 (m, 1H), 1.72-1.62 (m, 1H), 1.27-1.17 (m, 1H). LC-MS (ESI, Method 2) t_{R} = 1.06 min, m/z (M)⁺ = 272.8.

### Step 3. Methyl 6-((1S,2S)-2-fluorocyclopropane-1-carboxamido)-4-((4-methoxy-1-methyl-5-((S)-2,2,2-trifluoro-1-methoxyethyl)-1H-indazol-3-yl)amino)nicotinate (76)

To a solution of **76c** (100 mg, 0.367 mmol), **45f-A** (60 mg, 0.207 mmol), Pd₂(dba)₃ (33.6 mg, 36.68 µmol), Xantphos (21.2 mg, 36.68 µmol) in dioxane (2 mL) was added Cs₂CO₃ (239 mg, 0.734 mmol). The mixture was stirred at 90 °C for 16 h under N₂ atmosphere. After the reaction mixture was cooled to room temperature, it was concentrated and purified by flash chromatography on silica gel (PE/EtOAc = 2/3) to give the crude compound which was purified by *Prep*-HPLC (Method A) to give **76** (56 mg, 29% yield) as a yellow solid. ¹H NMR (400 MHz, DMSO-*d*₆) δ 10.96 (s, 1H), 10.94 (s, 1H), 9.19 (s, 1H), 8.79 (s, 1H), 7.49 (d, *J =* 8.8 Hz, 1H), 7.42 (d, *J =* 8.8 Hz, 1H), 5.30 (q, *J =* 6.8 Hz, 1H), 5.03-4.82 (m, 1H), 4.00 (s, 3H), 3.93 (s, 3H), 3.90 (s, 3H), 3.31 (s, 3H), 2.27-2.20 (m, 1H), 1.71-1.61 (m, 1H), 1.23-1.13 (m, 1H). LC-MS (ESI, Method 2) t_{R} = 1.25 min, m/z (M+H)⁺ =526.1.

### Example 77

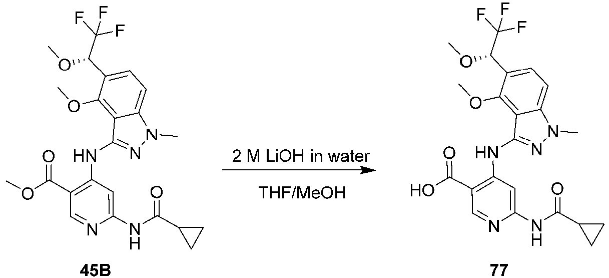

### (S)-6-(Cyclopropanecarboxamido)-4-((4-methoxy-1-methyl-5-(2,2,2-trifluoro-1-methoxyethyl)-1H-indazol-3-yl)amino)nicotinic acid (77)

To a solution of **45B** (53 mg, 104.4 µmol) in THF (1 mL) and MeOH (1 mL) was added *aq.* LiOH (0.5 mL, 2 M in water) at ice-bath. After stirring at 30 °C for 2 h, the mixture was adjusted to pH < 3 with 1 mol/L HCl. The mixture was extracted with EtOAc (10 mL*2) and concentrated. The residue was purified by *Prep*-HPLC (Method A) to give 77 (20 mg, 39% yield) as a white solid. ¹H NMR (400 MHz, DMSO-*d*₆): δ 11.36 (s, 1H), 10.84 (s, 1H), 9.16 (s, 1H), 8.74 (s, 1H), 7.46 (d, *J =* 8.4 Hz, 1H), 7.40 (d, *J =* 8.4 Hz, 1H), 5.30-5.24 (m, 1H), 3.98 (s, 3H), 3.89 (s, 3H), 3.35 (s, 3H), 2.05-2.01 (m, 1H), 0.83-0.80 (m, 4H). LC-MS (ESI, Method 2) t_{R} = 1.17 min, m/z (M+H)⁺ = 494.0.

### Example 78

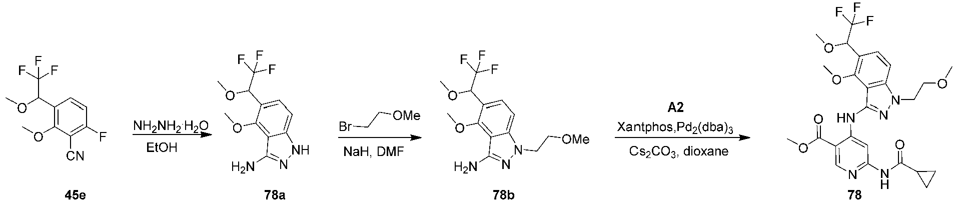

### Step 1. 4-Methoxy-5-(2,2,2-trifluoro-1-methoxyethyl)-1H-indazol-3-amine (78a)

A solution of **45e** (1.1 g, 4.18 mmol) and NH₂NH₂·H₂O (3 mL, 4.18 mmol) in EtOH (7 mL) was stirred at 70 °C for 4 h. After cooling to room temperature, the reaction mixture was poured into water (2 mL) and extracted with EtOAc (3 mL*2). The combined organic phase was concentrated and purified by flash chromatography (PE/EtOAc = 1/1) to give **78a** (700 mg, 61% yield) as a yellow solid. ¹H NMR (400 MHz, DMSO-*d*₆): δ 11.78 (s, 1H), 7.20 (d, *J =* 8.4 Hz, 1H), 7.10 (d, *J =* 8.4 Hz, 1H), 5.19-5.16 (m, 1H), 5.14 (s, 2H), 3.88 (s, 3H), 3.42 (s, 3H). LC-MS (ESI, Method 3): t_{R} = 1.12 min, m/z (M+H)⁺ = 276.2.

### Step 2. 4-Methoxy-5-(2,2,2-trifluoro-1-methoxyethyl)-1H-indazol-3-amine (78b)

To a solution of **78a** (500 mg, 1.82 mmol) in DMF (5 mL) was added NaH (145.5 mg, 3.63 mmol, 60% dispersion in mineral oil) at 0 °C. The mixture was stirred at 0 °C for 30 min. Then a solution of 1-bromo-2-methoxyethane (278 mg, 2.00 mmol) in DMF (0.5 mL) was added into the reaction mixture and stirred at 20 °C for 1 h. The reaction mixture was quenched with ice-water (10 mL) and extracted with EtOAc (15 mL). The organic layer was concentrated and the residue was purified by flash chromatography on silica gel (PE/EtOAc = 4/1) to give **78b** (380 mg, 63% yield) as a yellow solid. LC-MS (ESI, Method 3): t_{R} = 1.36 min, m/z (M+H)⁺ = 333.8.

### Step 3. Methyl 6-(cyclopropanecarboxamido)-4-((4-methoxy-1-(2-methoxyethyl)-5-(2,2,2-trifluoro-1-methoxyethyl)-1H-indazol-3-yl)amino)nicotinate (78)

A mixture of **78b** (40 mg, 0.120 mmol), **A2** (30.6 mg, 0.120 mmol), Cs₂CO₃ (78.2 mg, 0.240 mmol), Xantphos (6.9 mg, 0.012 mmol) and Pd₂(dba)₃ (11 mg, 0.012 mmol) in 1,4-dioxane (1 mL) was stirred at 90 °C for 5 h under N₂ atmosphere. After the reaction mixture was cooled to room temperature, it was concentrated and purified by flash chromatography on silica gel (DCM/MeOH = 20/1) to give the crude product. The crude product was purified by *Prep-*HPLC (Method A, NH₄HCO₃) to give **78** (21.5 mg, 32% yield) as a white solid. ¹H NMR (400 MHz, MeOH-*d*₄) δ 9.38 (s, 1H), 8.82 (s, 1H), 7.50 (d, *J =* 9.2 Hz, 1H), 7.39 (d, *J =* 8.8 Hz, 1H), 5.23-5.21 (m, 1H), 4.53 (t, *J =* 5.2 Hz, 2H), 4.02-3.99 (m, 5H), 3.99 (s, 3H), 3.38 (s, 3H), 3.34 (s, 3H), 1.94-1.92 (m, 1H), 1.04-0.92 (m, 4H). LC-MS (ESI, Method 2): t_{R} = 1.44 min, m/z (M+H)⁺ = 552.2.

### Example 79

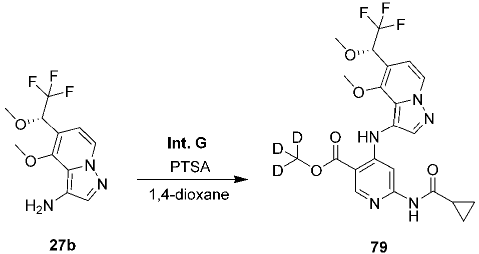

### Step 1. Methyl-d₃ (S)-6-(cyclopropanecarboxamido)-4-((4-methoxy-5-(2,2,2-trifluoro-1-methoxyethyl)pyrazolo[1,5-a]pyridin-3-yl)amino)nicotinate (79)

A mixture **of Int. G** (30 mg, 0.12 mmol), **27b** (32 mg, 0.12 mmol) and PTSA (9 mg, 0.05 mmol) in dioxane (0.6 mL) was stirred at 100 °C for 16 h. After the mixture was cooled to room temperature, it was concentrated and purified by *Prep*-HPLC(Method A) to give **79** (25 mg, 43% yield) as a yellow solid. ¹H NMR (400 MHz, DMSO-*d*₆) δ 10.82 (s, 1H), 9.61 (s, 1H), 8.68 (s, 1H), 8.56 (d, *J =* 7.2 Hz, 1H), 8.17 (s, 1H), 7.68 (s, 1H), 6.80 (d, *J =* 7.2 Hz, 1H), 5.28-5.25 (m, 1H), 3.71 (s, 3H), 3.33 (s, 3H), 1.94-1.92 (m, 1H), 0.75-0.67 (m, 4H). LC-MS (ESI, Method 2) t_{R} = 0.91 min, m/z (M+H)⁺ = 497.1.

### Example 80

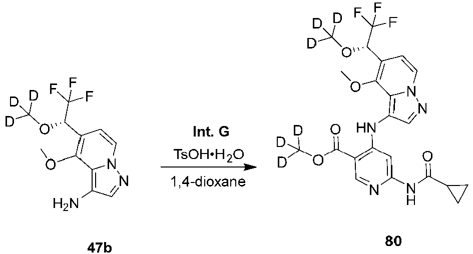

### Step 1. Methyl-d₃ (S)-6-(cyclopropanecarboxamido)-4-((4-methoxy-5-(2,2,2-trifluoro-1-(methoxy-d₃)ethyl)pyrazolo[1,5-a]pyridin-3-yl)amino)nicotinate (80)

A mixture of **Int. G** (25 mg, 0.097 mmol), **47b** (27 mg, 0.097 mmol) and TsOH·H₂O (8 mg, 0.04 mmol) in dioxane (0.5 mL) was stirred at 100 °C for 16 h. After the mixture was cooled to room temperature, it was concentrated and purified by *Prep*-HPLC (Method A) to give **80** (30 mg, 62% yield) as a white solid. ¹H NMR (400 MHz, DMSO-*d*₆): δ 10.82 (s, 1H), 9.60 (s, 1H), 8.68 (s, 1H), 8.56 (d, *J =* 7.2 Hz, 1H), 8.17 (s, 1H), 7.67 (s, 1H), 6.80 (d, *J =* 7.6 Hz, 1H), 5.28-5.24 (m, 1H), 3.71 (s, 3H), 1.94-1.92 (m, 1H), 0.74-0.67 (m, 4H). LC-MS (Method 2): t_{R} = 0.90 min, m/z (M+H)⁺ = 500.2.

### Example 81

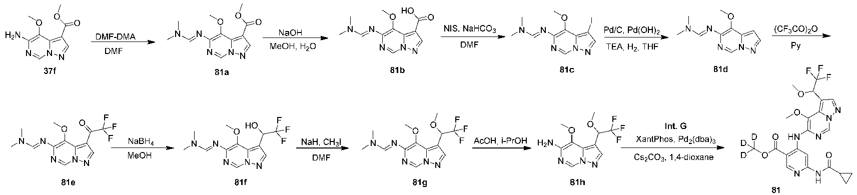

### Step 1. Methyl 5-(((dimethylamino)methylene)amino)-4-methoxypyrazolo[1,5-c]pyrimidine-3-carboxylate (81a)

A mixture of **37f** (4 g, 18.00 mmol), DMF-DMA (4.29 g, 36.00 mmol, 4.78 mL) in DMF (20 mL) was stirred at 50 °C for 4 h. The mixture was concentrated to afford **81a** (4.99 g, 100% yield) as a yellow oil. LC-MS (ESI, Method 3): t_{R} = 0.70 min, m/z (M+H)⁺ = 278.3.

### Step 2. 5-(((Dimethylamino)methylene)amino)-4-methoxypyrazolo[1,5-c]pyrimidine-3-carboxylic acid (81b)

To a mixture of **81a** (4.99 g, 18.01 mmol) in MeOH (30 mL) and H₂O (15 mL) was added NaOH (1.44 g, 36.06 mmol), the mixture was stirred at 45 °C for 4 h. The mixture was concentrated and acidized with 2M HCl to pH = 1. The formed solid was filtered and dried to afford **81b** (4.0 g, 84% yield) as a yellow solid. LC-MS (ESI, Method 3): t_{R} = 0.17 min, m/z (M+H)⁺ = 264.2.

### Step 3. N'-(3-iodo-4-methoxypyrazolo[1,5-c]pyrimidin-5-yl)-N,N-dimethylformimidamide (81c)

To a solution **of 81b** (4 g, 15.19 mmol) and NaHCO₃ (2.55 g, 30.39 mmol) in DMF (40 mL) was added NIS (6.84 g, 30.39 mmol) at 0 °C. Then the reaction was warmed to 20 °C and stirred for 1 h. The reaction mixture was diluted with EtOAc (50 mL). The separated organic layer was washed with *sat.* Na₂S₂O₃ (20 mL*2), *sat.* NaHCO₃ (20 mL*2) and brine (20 mL), then it was dried over Na₂SO₄, filtered and concentrated to afford the crude product. Then the crude product was purified by *Prep*-HPLC (Method A) to afford **81c** (550 mg, 10% yield)) as a grown solid. LC-MS (ESI, Method 3): t_{R} = 0.90 min, m/z (M+H)⁺ = 345.8.

### Step 4. N'-(4-methoxypyrazolo[1,5-c]pyrimidin-5-yl)-N,N-dimethylformimidamide (81d)

To a solution of **81c** (500 mg, 1.45 mmol) and triethylamine (146.6 mg, 1.45 mmol, 201.92 µL) in THF (5 mL) was added Pd/C (10% on Carbon, 50 mg) and Pd(OH)₂ (50 mg) at 10 °C and the mixture was stirred at 30 °C under H₂ (1 atm) atmosphere for 16 h. The catalyst was filtered off and filtrate was concentrated. The residue was purified by flash chromatography on silica gel (PE:EtOAc = 1:2) to afford **81d** (280 mg, 88% yield) as a yellow solid. ¹H NMR (400 MHz, DMSO-*d*₆) δ 9.09 (d, *J* = 2.0 Hz, 1H), 8.53 (s, 1H), 7.99 (d, *J =* 2.0 Hz, 1H), 6.45 (s, 1H), 3.99 (s, 3H), 3.10 (s, 3H), 3.04 (s, 3H).

### Step 5. N'-(4-methoxy-3-(2,2,2-trifluoroacetyl)pyrazolo[1,5-c]pyrimidin-5-yl)-N,N-dimethylformimidamide (81e)

To a solution of **81d** (270 mg, 1.23 mmol) in Py (2 mL) was slowly added trifluoroacetic anhydride (388 mg, 1.85 mmol) at 0 °C. After stirring at 0 °C for 2 h, the reaction mixture was purified by *Prep*-HPLC (Method A) to afford **81e** (300 mg, 77% yield) as a yellow solid. LC-MS (ESI, Method 3): t_{R} = 0.90 min, m/z (M+H)⁺ = 316.2.

### Step 6. N'-(4-methoxy-3-(2,2,2-trifluoro-1-hydroxyethyl)pyrazolo[1,5-c]pyrimidin-5-yl)-N,N-dimethylformimidamide (81f)

To a solution of **81e** (150 mg, 0.476 mmol) in MeOH (3 mL) was added NaBH₄ (36 mg, 0.952 mmol) at -5 °C. After stirring at -5 °C for 0.5 h, the reaction mixture was diluted with H₂O (0.5 mL) and concentrated to afford **81f** (140 mg, 93% yield) as a yellow solid. LC-MS (ESI, Method 3): t_{R} = 0.90 min, m/z (M+H)⁺ = 318.1.

### Step 7. N'-(4-methoxy-3-(2,2,2-trifluoro-1-methoxyethyl)pyrazolo[1,5-c]pyrimidin-5-yl)-N,N-dimethylformimidamide (81g)

To a solution of **81f** (240 mg, 0.756 mmol) and CH₃I (64.4 mg, 0.454 mmol) in DMF (2 mL) was added NaH (61 mg, 1.51 mmol, 60% dispersion in mineral oil) at 0 °C. Then the mixture was stirred at 0 °C for 1 h and diluted with water (6 mL). The mixture was extracted with EtOAc (8 mL*3), and the combined organic layer was concentrated. The residue was purified by *Prep*-HPLC (Method A) to afford **81g** (57 mg, 23% yield) as a yellow solid. LC-MS (ESI, Method 3): t_{R} = 1.02 min, m/z (M+H)⁺ = 332.2.

### Step 8. 4-Methoxy-3-(2,2,2-trifluoro-1-methoxyethyl)pyrazolo[1,5-c]pyrimidin-5-amine (81h)

A mixture of **81g** (75 mg, 0.226 mmol) in AcOH and i-PrOH (0.5 mL and 0.5 mL) was stirred at 60 °C for 6 h. The mixture was concentrated and purified by *Prep*-TLC (PE:EtOAc = 1:1) to afford **81h** (34 mg, 54% yield) as a white solid. LC-MS (ESI, Method 3): t_{R} = 1.08 min, m/z (M+H)⁺ = 277.0.

### Step 9. Methyl-d3 6-(cyclopropanecarboxamido)-4-((4-methoxy-3-(2,2,2-trifluoro-1-methoxyethyl)pyrazolo[1,5-c]pyrimidin-5-yl)amino)nicotinate (81)

A mixture of **81h** (28 mg, 0.101 mmol), **Int. G** (28.7 mg, 0.112 mmol), Pd₂(dba)₃ (18.6 mg, 0.020 mmol), XantPhos (11.7 mg, 0.020 mmol) and Cs₂CO₃ (66.1 mg, 0.203 mmol) in 1,4-dioxane (0.5 mL) was stirred at 90 °C for 3 h under N₂ atmosphere. The reaction was cooled to 15 °C and concentrated. The residue was purified by chromatography column (DCM:MeOH = 20:1) to give the crude product. The crude product was re-purified by *Prep*-HPLC (Method A) to afford **81** (12.3 mg, 24% yield) as a white solid. LC-MS (ESI, Method 2): t_{R} = 1.23 min, m/z

(M+H)⁺ = 498.1. ¹H NMR (300 MHz, DMSO-*d*₆) δ 11.00 (s, 1H), 10.77 (s, 1H), 9.47 (s, 1H), 8.98 (s, 1H), 8.80 (s, 1H), 8.25 (s, 1H), 5.30 (q, *J =* 6.9 Hz, 1H), 3.93 (s, 3H), 3.39 (s, 3H), 2.10-2.00 (m, 1H), 0.88-.079 (m, 4H).

### Example 82

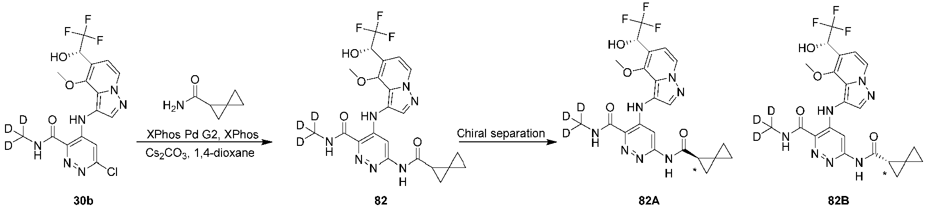

### Step 1. 4-((4-Methoxy-5-((S)-2,2,2-trifluoro-1-hydroxyethyl)pyrazolo[1,5-a]pyridin-3-yl)amino)-N-(methyl-d₃)-6-(spiro[2.2]pentane-1-carboxamido)pyridazine-3-carboxamide (82)

A mixture of **30b** (130.0 mg, 0.30 mmol), spiro[2.2]pentane-1-carboxamide (99.9 mg, 0.90 mmol), Xantphos (35 mg, 0.060 mmol), XPhos Pd G2 (47 mg, 0.060 mmol) and Cs₂CO₃ (195 mg, 0.60 mmol) in 1,4-dioxane (1.2 mL) was stirred at 100 °C for 12 h under N₂ atmosphere. After the reaction mixture was cooled, it was concentrated and purified by flash chromatography on silica gel (DCM/MeOH = 20/1) to afford **82** (90 mg, 59% yield) as a white solid. LC-MS (ESI, Method 3) t_{R} = 1.17 min, m/z (M+H)⁺ = 509.4.

### Step 2. 4-((4-Methoxy-5-((S)-2,2,2-trifluoro-1-hydroxyethyl)pyrazolo[1,5-a]pyridin-3-yl)amino)-N-(methyl-d₃)-6-((R*)-spiro[2.2]pentane-1-carboxamido)pyridazine-3-carboxamide (82A) and 4-((4-Methoxy-5-((S)-2,2,2-trifluoro-1-hydroxyethyl)pyrazolo[1,5-a]pyridin-3-yl)amino)-N-(methyl-d₃)-6-((S*)-spiro[2.2]pentane-1-carboxamido)pyridazine-3-carboxamide (82B)

**82** (40 mg, 0.08 mmol) was separated by Chiral-HPLC (Method J, Hex\EtOH\DEA = 70\30\0.2, 15 mL/min) to afford **82A** (6 mg, 8% yield) as a white solid and **82B** (6.5 mg, 8% yield) as a white solid.

**82A**: ¹H NMR (400 MHz, DMSO-*d₆*) δ 11.06 (s, 1H), 10.58 (s, 1H), 9.09 (s, 1H), 8.55 (d, *J =* 7.6 Hz, 1H), 8.19 (s, 1H), 7.84 (s, 1H), 6.94 (d, *J =* 7.2 Hz, 1H), 5.42-5.38 (m, 1H), 3.72 (s, 3H), 2.40-2.38 (m, 1H), 1.32-1.30 (m, 2H), 0.87-0.82 (m, 3H), 0.70-0.67 (m, 1H). LC-MS (ESI, Method 2) t_{R} = 1.01 min, m/z (M+H)⁺ = 509.0. Chiral HPLC (Method 7, Hex\EtOH\DEA = 70\30\0.2): t_{R} = 5.74 min.

**82B:** ¹H NMR (400 MHz, DMSO-*d₆*) δ 11.05 (s, 1H), 10.56 (s, 1H), 9.09 (s, 1H), 8.55 (d, *J =* 6.8 Hz, 1H), 8.18 (s, 1H), 7.81 (s, 1H), 6.95 (d, *J =* 7.6 Hz, 1H), 5.43-5.38 (m, 1H), 3.72 (s, 3H), 2.40-2.37 (m, 1H), 1.32-1.28 (m, 2H), 0.87-0.82 (m, 3H), 0.69-0.67 (m, 1H). LC-MS (ESI, Method 2) t_{R} = 1.01min, m/z (M+H)⁺ = 509.0. Chiral HPLC (Method 7, Hex\EtOH\DEA = 70\30\0.2): t_{R} = 6.88 min.

### Example 83

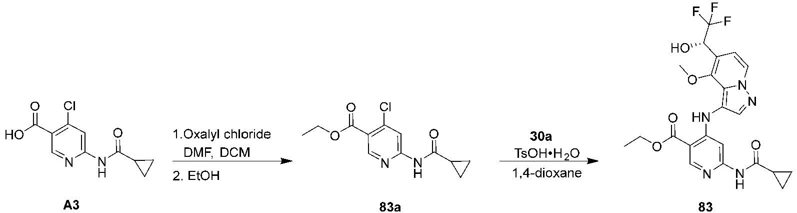

### Step 1. Ethyl 4-chloro-6-(cyclopropanecarboxamido)nicotinate (83a)

To a solution of **A3** (500 mg, 2.08 mmol) and DMF (7.59 mg, 0.10 mmol) in DCM (5 mL) was added oxalyl chloride (580 mg, 4.57 mmol, 397.40 µL) at 0 °C. The reaction was stirred at 25°C for 2 h and quenched with ethanol (192 mg, 4.16 mmol, 242.64 µL). After stirring for 30 min at 25 °C, the reaction solution was diluted with *sat.* NaHCO₃ (10 mL) and extracted with DCM (15 mL*3).The combined organic layer was washed with brine (10 mL), dried over Na₂SO₄ and fitlered. The filtrate was concentrated to give **83a** (410 mg, 73% yield) as a white solid. ¹H NMR (400 MHz, DMSO-*d*₆): δ 11.10 (s, 1H), 8.79 (s, 1H), 8.27 (s, 1H), 4.32 (q, *J* = 7.2 Hz, 2H), 2.05-2.02 (m, 1H), 1.29 (t, *J =* 7.2 Hz, 3H), 0.89-0.86 (m, 4H).

### Step 2. (S)-ethyl 6-(cyclopropanecarboxamido)-4-((4-methoxy-5-(2,2,2-trifluoro-1-hydroxyethyl)pyrazolo[1,5-a]pyridin-3-yl)amino)nicotinate (83)

A mixture of **83a** (40 mg, 0.15 mmol), **30a** (39 mg, 0.15 mmol) and TsOH·H₂O (11 mg, 0.06 mmol) in 1,4-dioxane (0.5 mL) was stirred at 100 °C for 16 h. The reaction solution was concentrated and purified by *Prep*-HPLC (Method A) to give **83** (50 mg, 68% yield) as a white solid. ¹H NMR (400 MHz, DMSO-*d*₆) δ 11.17 (s, 1H), 9.84 (s, 1H), 8.67 (s, 1H), 8.57 (d, *J* = 7.2 Hz, 1H), 8.17 (s, 1H), 7.27 (s, 1H), 6.97 (d, *J =* 7.2 Hz, 1H), 5.41-5.38 (m, 1H), 4.38 (q, *J =* 7.2 Hz, 2H), 3.71 (s, 3H), 1.88-1.85 (m, 1H), 1.36 (t, *J =* 7.2 Hz, 3H), 0.83-0.76 (m, 4H). LC-MS (ESI, Method 2): t_{R} = 1.01 min, m/z (M+H)⁺ = 494.0.

### Example 84

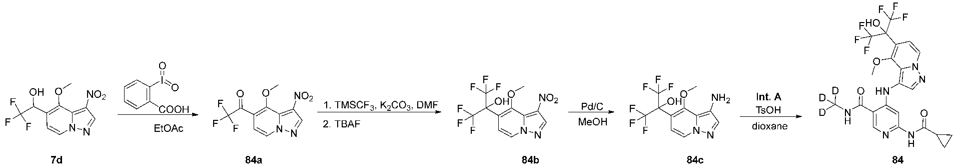

### Step 1. 2,2,2-Trifluoro-1-(4-methoxy-3-nitropyrazolo[1,5-a]pyridin-5-yl)ethanone (84a)

To a solution of **7d** (30 g, 103 mmol) in EtOAc (400 mL) was added 1-Hydroxy-1,2-benziodoxol-3(1H)-one 1-oxide (86.55 g, 309 mmol, CAS: 61717-82-6). The reaction was stirred at 85 °C for 48 h. After the reaction is cooled to room temperature, it was filtered. The filtrate was concentrated. and purified by flash chromatography on silica gel (PE/EtOAc = 3/1) to give **84a** (27.4 g, 92% yield) as a yellow solid. ¹H NMR (400 MHz, DMSO-*d*₆) δ 8.95 (s, 1H), 8.78 (d, *J =* 7.2 Hz, 1H), 7.47 (d, *J =* 7.2 Hz, 1H), 3.76 (s, 3H).

### Step 2. 1,1,1,3,3,3-Hexafluoro-2-(4-methoxy-3-nitropyrazolo[1,5-a]pyridin-5-yl)propan-2-ol (84b)

To a solution of **84a** (200 mg, 0.69 mmol) and K₂CO₃ (10 mg, 0.069 mmol) in DMF (4 mL) was added TMSCF₃ (148 mg, 1.04 mmol, 0.16 mL), the mixture was stirred at 25 °C for 4 h. TBAF (2.0 mL, 2.07 mmol, 1 M in THF) was added to the solution and the mixture was stirred for 12 h. The reaction mixture was diluted with water (50 mL) and extracted with EtOAc (50 mL*2). The combined organic layer was washed with brine (30 mL*2), dried over anhydrous Na₂SO₄, filtered and concentrated. The residue was purified by column chromatography (EtOAc in PE is 30%) to give **84b** (166 mg, 0.46 mmol, 67% yield) as an off-white solid. LC-MS (ESI, Method 4) t_{R} = 2.96 min, m/z (M+H)⁺ = 360.1.

### Step 3. 2-(3-Amino-4-methoxypyrazolo[1,5-a]pyridin-5-yl)-1,1,1,3,3,3-hexafluoropropan-2-ol (84c)

To a solution of **84b** (65 mg, 0.18 mmol) in MeOH (4 mL) was added Pd/C (6 mg, 0.054 mmol, 10% on charcoal) under H₂ atmosphere. The mixture was stirred at 25 °C for 2 h. Then the reaction mixture was filtered and concentrated to give **84c** (50 mg, 0.15 mmol) as a brown solid, which was used for the next step directly without further purification. LC-MS (ESI, Method 4) t_{R} = 2.45 min, m/z (M+H)⁺ = 330.2.

### Step 4. 6-(Cyclopropanecarboxamido)-4-((5-(1,1,1,3,3,3-hexafluoro-2-hydroxypropan-2-yl)-4-methoxypyrazolo[1,5-a]pyridin-3-yl)amino)-N-(methyl-d₃)nicotinamide (84)

To a solution of **84c** (50 mg, 0.15 mmol) and **Int. A** (27 mg, 0.11 mmol) in dioxane (2 mL) was added TsOH (26 mg, 0.15 mmol), the mixture was stirred at 85 °C for 3 h. The reaction mixture was diluted with water (30 mL) and extracted with EtOAc (30 mL*2). The combined organic layer was washed with brine (20 mL*2), dried over anhydrous Na₂SO₄, filtered and concentrated. The residue was purified by *Prep*-HPLC (Method E, NH₄HCO₃) to give **84** (29 mg, 0.053 mmol, 35% yield) as a white solid. ¹H NMR (400 MHz, DMSO-*d*₆) *δ* 10.69 (s, 1H), 10.32 (s, 1H), 8.81 (s, 1H), 8.58 (s, 1H), 8.54 (d, *J =* 7.6 Hz, 1H), 8.51 (s, 1H), 8.16 (s, 1H), 7.68 (s, 1H), 7.05 (d, *J* = 7.6 Hz, 1H), 3.69 (s, 3H), 1.97-1.88 (m, 1H), 0.75-0.68 (m, 4H). LC-MS (ESI, Method 4) t_{R} = 2.34 min, m/z (M+H)⁺ = 550.3.

### Example 85

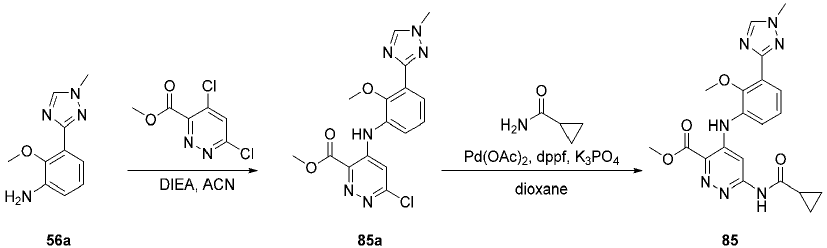

### Step 1. Methyl 6-chloro-4-((2-methoxy-3-(1-methyl-1H-1,2,4-triazol-3-yl)phenyl)amino)pyridazine-3-carboxylate (85a)

To a solution of methyl 4,6-dichloropyridazine-3-carboxylate (304 mg, 1.47 mmol) and 56a (200 mg, 0.98 mmol) in ACN (3 mL) was added DIEA (316 mg, 2.45 mmol, 0.43 mL), the mixture was stirred at 80 °C for 12 h. The reaction mixture was diluted with water (50 mL) and extracted with EtOAc (50 mL*2). The combined organic layer was washed with brine (30 mL*2), dried over anhydrous Na₂SO₄, filtered and concentrated. The residue was purified by column chromatography (MeOH in DCM is 5%) to give **85a** (162 mg, 44% yield) as a yellow solid. ¹H NMR (400 MHz, DMSO-*d*₆) *δ* 9.80 (s, 1H), 8.57 (s, 1H), 7.78 (dd, *J =* 8.0, 1.6 Hz, 1H), 7.56 (dd, *J =* 8.0, 1.6 Hz, 1H), 7.31 (t, *J =* 8.0 Hz, 1H), 7.07 (s, 1H), 3.99 (s, 3H), 3.95 (s, 3H), 3.70 (s, 3H). LC-MS (ESI, Method 4) t_{R} = 2.08 min, m/z (M+H)⁺ = 375.2.

### Step 2. Methyl 6-(cyclopropanecarboxamido)-4-((2-methoxy-3-(1-methyl-1H-1,2,4-triazol-3-yl)phenyl)amino)pyridazine-3-carboxylate (85)

To a solution of **85a** (50 mg, 0.13 mmol) and cyclopropanecarboxamide (57 mg, 0.67 mmol) in dioxane (2 mL) was added Pd(OAc)₂ (6 mg, 0.027 mmol), dppf (30 mg, 0.053 mmol) and K₃PO₄ (85 mg, 0.40 mmol) under N₂ atmosphere, the mixture was stirred at 100 °C for 1 h. The reaction mixture was diluted with water (20 mL) and extracted with EtOAc (20 mL*2). The combined organic layer was washed with brine (20 mL*2), dried over anhydrous Na₂SO₄, filtered and concentrated. The residue was purified by *Prep*-HPLC (Method E) to give 85 (2 mg, 4% yield) as a white solid. ¹H NMR (400 MHz, DMSO-*d*₆) *δ* 11.53 (s, 1H), 9.74 (s, 1H), 8.57 (s, 1H), 8.06 (s, 1H), 7.71 (dd, *J =* 8.0, 1.6 Hz, 1H), 7.50 (dd, *J =* 8.0, 1.6 Hz, 1H), 7.29 (t, *J =* 8.0 Hz, 1H), 3.97 (s, 3H), 3.94 (s, 3H), 3.71 (s, 3H), 2.10-2.01 (m, 1H), 0.85-0.76 (m, 4H). LC-MS (ESI, Method 4) t_{R} = 2.13 min, m/z (M+H)⁺ = 424.3.

### Example 86

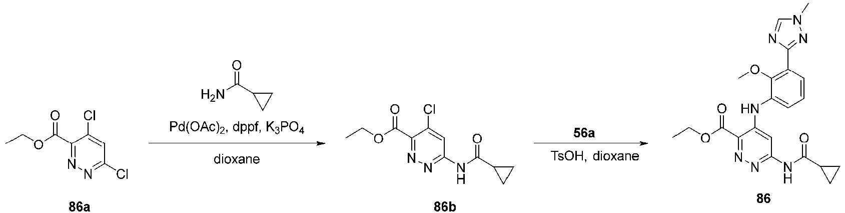

### Step 1. Ethyl 4-chloro-6-(cyclopropanecarboxamido)pyridazine-3-carboxylate (86b)

To a solution of **86a** (200 mg, 0.90 mmol) and cyclopropanecarboxamide (77 mg, 0.90 mmol) in dioxane (15 mL) was added Pd(OAc)₂ (41 mg, 0.18 mmol), dppf (201 mg, 0.36 mmol) and K₃PO₄ (576 mg, 2.71 mmol) under N₂ atmosphere, the mixture was stirred at 85 °C for 2 h. The reaction mixture was diluted with water (50 mL) and extracted with EtOAc (50 mL*2). The combined organic layer was washed with brine (30 mL*2), dried over anhydrous Na₂SO₄, filtered and concentrated. The residue was purified by column chromatography (EtOAc in PE is 25%) to give **86b** (143 mg, 59% yield) as a white solid. ¹H NMR (400 MHz, DMSO-*d*₆) *δ* 12.01 (s, 1H), 8.56 (s, 1H), 4.43 (q, *J =* 7.2 Hz, 2H), 2.15-2.05 (m, 1H), 1.35 (t, *J =* 7.2 Hz, 3H), 0.97-0.85 (m, 4H). LC-MS (ESI, Method 4) t_{R} = 2.34 min, m/z (M+H)⁺ = 270.1.

### Step 2. Ethyl 6-(cyclopropanecarboxamido)-4-((2-methoxy-3-(1-methyl-1H-1,2,4-triazol-3-yl)phenyl)amino)pyridazine-3-carboxylate (86)

To a solution of **86b** (50 mg, 0.19 mmol) and **56a** (38 mg, 0.19 mmol) in dioxane (3 mL) was added TsOH (35 mg, 0.20 mmol), the mixture was stirred at 85 °C for 4 h. The reaction mixture was diluted with water (30 mL) and extracted with EtOAc (30 mL*2). The combined organic layer was washed with brine (20 mL*2), dried over anhydrous Na₂SO₄, filtered and concentrated. The residue was slurried in MeOH (10 mL) and filtered to give **86** (44 mg, 54% yield) as an off-white solid. ¹H NMR (400 MHz, DMSO-*d*₆) *δ* 11.50 (s, 1H), 9.76 (s, 1H), 8.57 (s, 1H), 8.08 (s, 1H), 7.71 (dd, *J =* 8.0, 1.6 Hz, 1H), 7.50 (dd, *J =* 8.0, 1.6 Hz, 1H), 7.29 (t, *J =* 8.0 Hz, 1H), 4.45 (q, *J* = 7.2 Hz, 2H), 3.94 (s, 3H), 3.71 (s, 3H), 2.13-2.01 (m, 1H), 1.39 (t, *J =* 7.2 Hz, 3H), 0.87-0.75 (m, 4H). LC-MS (ESI, Method 4) t_{R} = 2.27 min, m/z (M+H)⁺ = 438.3.

### Example 87

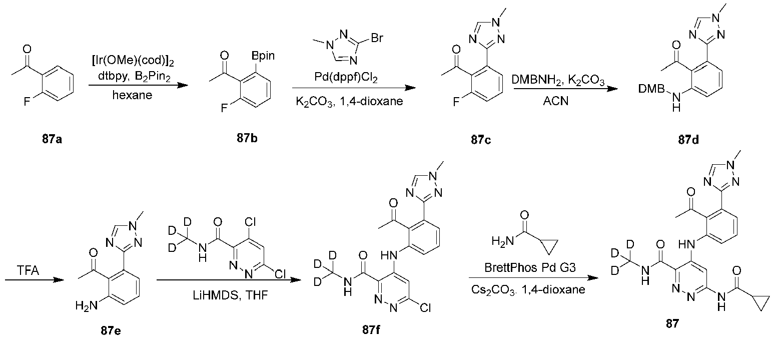

### Step 1. 1-(2-Fluoro-6-(4,4,5,5-tetramethyl-1,3,2-dioxaborolan-2-yl)phenyl)ethan-1-one (87b)

A mixture of **87a** (2 g, 14.48 mmol), B₂Pin₂ (3.31 g,13.03 mmol), [Ir(OMe)(cod)]₂ (28 mg, 43.4 µmol) and 4,4'-ditertbutylbipyridine (23 mg, 86.87 µmol) in hexane (16 mL) was stirred at 55 °C for 16 h under N₂ atmosphere. After cooling to room temperature, the mixture was diluted with EtOAc (20 mL) and washed with brine (20 mL). The organic layer was dried over Na₂SO₄ and filtered. The filtrate was concentrated and purified by flash chromatography on silica gel (PE/EtOAc = 20/1) to give **87b** (1.9 g, 50% yield) as a yellow oil.

### Step 2. 1-(2-Fluoro-6-(1-methyl-1H-1,2,4-triazol-3-yl)phenyl)ethan-1-one (87c)

A mixture of **87b** (500 mg, 1.89 mmol), 3-bromo-1-methyl-1,2,4-triazole (337.35 mg, 2.08 mmol), Pd(dppf)Cl₂ (69.26 mg, 94.66 µmol) and K₂CO₃ (523.33 mg, 3.79 mmol) in 1,4-dioxane (5 mL) and H₂O (1 mL) was stirred at 90°C for 3 h. After cooling to room temperature, the mixture was diluted with EtOAc (10 mL) and washed with brine (10 mL*2). The organic layer was dried over Na₂SO₄ and filtered. The filtrate was concentrated and purified by flash chromatography on silica gel (PE/EtOAc = 1/1 to 0/1) to give **87c** (56 mg, 13% yield) as a yellow solid. LC-MS (ESI, Method 3) t_{R} = 1.01 min, m/z (M+H)⁺ = 220.4.

### Step 3. 1-(2-((2,4-Dimethoxybenzyl)amino)-6-(1-methyl-1H-1,2,4-triazol-3-yl)phenyl)ethan-1-one (87d)

A mixture of **87c** (150 mg, 0.68 mmol), K₂CO₃ (189 mg, 1.37 mmol) and (2,4-dimethoxyphenyl)methanamine (206 mg, 1.23 mmol) in ACN (1.5 mL) was stirred at 100 °C for 16 h. After the mixture was cooled to room temperature, it was concentrated and purified by flash chromatography on silica gel (PE/EtOAc = 1/1) to give **87d** (170 mg, 68% yield) as a yellow solid. LC-MS (ESI, Method 3) t_{R} = 1.01 min, m/z (M+H)⁺ = 367.3.

### Step 4. 1-(2-Amino-6-(1-methyl-1H-1,2,4-triazol-3-yl)phenyl)ethan-1-one (87e)

A solution of **87d** (170 mg, 0.46 mmol) and TFA (1.5 mL) was stirred at 0 °C to room temperature for 1 h. The solvent was removed by pumping with N₂. And the residue was purified by *Prep*-HPLC (Method A) to give **87e** (60 mg, 60% yield) as a yellow solid. LC-MS (ESI, Method 3) t_{R} = 1.07 min, m/z (M+H)⁺ = 217.1.

### Step 5. 4-((2-Acetyl-3-(1-methyl-1H-1,2,4-triazol-3-yl)phenyl)amino)-6-chloro-N-(methyl-d₃)pyridazine-3-carboxamide (87f)

To a solution of **87e** (40 mg, 0.18 mmol) and 4,6-dichloro-*N*-(methyl-*d*₃)pyridazine-3-carboxamide (77 mg, 0.37 mmol) in THF (0.4 mL) was added LiHMDS (0.74 mL, 0.74 mmol, 1 M in THF) at -40 °C. The reaction was stirred at -40 °C to room temperature for 2 h. The reaction was quenched with H₂O (2 mL) and the organic phase was concentrated. The residue was extracted with EtOAc (5 mL*3) and the combined organic layer was dried over Na₂SO₄ and filtered. The filtrate was concentrated and purified by flash chromatography on silica gel (DCM/MeOH = 20/1) to give **87f** (25 mg, 35% yield) as a brown solid. LC-MS (ESI, Method 3) t_{R} = 1.03 min, m/z (M+H)⁺ = 389.3.

### Step 6. 4-((2-Acetyl-3-(1-methyl-1H-1,2,4-triazol-3-yl)phenyl)amino)-6-(cyclopropanecarboxamido)-N-(methyl-d₃)pyridazine-3-carboxamide (87)

A mixture of **87f** (25 mg, 0.06 mmol), cyclopropanecarboxamide (27 mg, 0.32 mmol), BrettPhos Pd G3 (12 mg, 0.013 mmol) and Cs₂CO₃ (42 mg, 0.13 mmol) in dioxane (0.4 mL) was stirred at 90 °C for 12 h under N₂ atmosphere. The reaction mixture was cooled to room temperature, concentrated and purified by flash chromatography on silica gel (DCM/MeOH = 20/1) to give the crude product. The crude product was purified by *Prep*-HPLC (Method A) to give **87** (3 mg, 11% yield) as a white solid. ¹H NMR (400 MHz, DMSO-*d₆*) δ 11.11 (s, 1H), 11.04 (s, 1H), 8.93 (s, 1H), 8.50 (s, 1H), 8.14 (d, *J =* 7.2 Hz, 1H), 7.84 (d, *J =* 5.6 Hz, 1H), 7.54-7.50 (m, 1H), 7.31 (s, 1H), 3.82 (s, 3H), 2.44 (s, 3H), 1.99-1.98 (m, 1H), 0.77-0.72 (m, 4H). LC-MS (ESI, Method 2) t_{R} = 0.56 min, m/z (M+H)⁺ = 438.1.

### Example 88

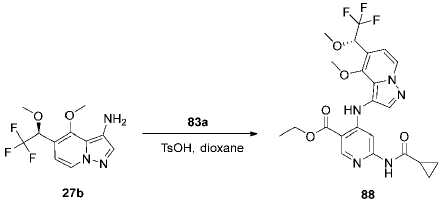

### Step 1. Ethyl (S)-6-(cyclopropanecarboxamido)-4-((4-methoxy-5-(2,2,2-trifluoro-1-methoxyethyl)pyrazolo[1,5-a]pyridin-3-yl)amino)nicotinate (88)

To a solution of **83a** (19 mg, 0.071 mmol) and 27b (15 mg, 0.055 mmol) in dioxane (1 mL) was added TsOH (9 mg, 0.055 mmol), the mixture was stirred at 85 °C for 2 h. The reaction mixture was diluted with water (30 mL) and extracted with EtOAc (30 mL*2). The combined organic layer was washed with brine (20 mL*2), dried over anhydrous Na₂SO₄, filtered and concentrated. The residue was purified by *Prep*-TLC (MeOH in DCM is 4%) to give **88** (12 mg, 43% yield) as a white solid. ¹H NMR (400 MHz, DMSO-*d*₆) *δ* 10.86 (s, 1H), 9.65 (s, 1H), 8.68 (s, 1H), 8.57 (d, *J* = 7.2 Hz, 1H), 8.18 (s, 1H), 7.70 (s, 1H), 6.80 (d, *J* = 7.2 Hz, 1H), 5.28 (q, *J =* 6.8 Hz, 1H), 4.36 (q, *J* = 7.2 Hz, 2H), 3.71 (s, 3H), 3.33 (s, 3H), 1.99-1.88 (m, 1H), 1.36 (t, *J =* 7.2 Hz, 3H), 0.76-0.64 (m, 4H). LC-MS (ESI, Method 4) t_{R} = 3.03 min, m/z (M+H)⁺ = 508.4.

### Example 89

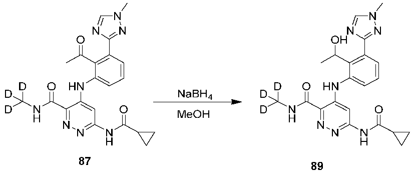

### Step 1. 6-(Cyclopropanecarboxamido)-4-((2-(1-hydroxyethyl)-3-(1-methyl-1H-1,2,4-triazol-3-yl)phenyl)amino)-N-(methyl-d₃)pyridazine-3-carboxamide (89)

To a solution of **87** (50 mg, 0.11 mmol) in MeOH (0.5 mL) was added NaBH₄ (8.65 mg, 0.23 mmol) at 0 °C. The reaction was stirred at 0 °C to room temperature for 1 h. The mixture was concentrated and purified by *Prep*-HPLC (Method A) to give **89** (15 mg, 30% yield) as a white solid. ¹H NMR (400 MHz, DMSO-*d*₆) δ 11.01-10.99 (m, 1H), 10.41-10.38 (m, 1H), 8.95-8.93 (m, 1H), 8.37 (s, 1H), 7.92-7.88 (m, 1H), 7.73-7.68 (m, 1H), 7.53-7.46 (m, 1H), 7.07-7.00 (m, 1H), 5.22-5.17 (m, 1H), 4.92-4.73 (m, 1H), 3.73 (s, 3H), 1.99-1.94 (m, 1H), 1.31-1.27 (m, 3H), 0.75-0.68 (m, 4H). LC-MS (ESI, Method 2) t_{R} = 0.70 min, m/z (M+H)⁺ = 440.1.

### Example 90

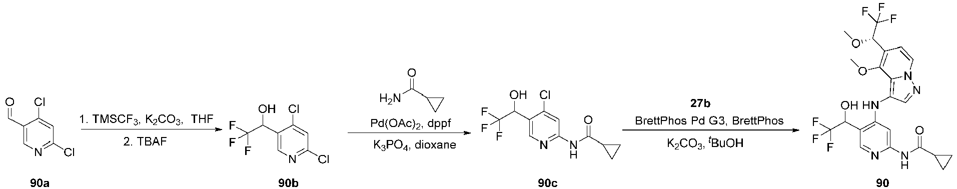

### Step 1. 1-(4,6-Dichloropyridin-3-yl)-2,2,2-trifluoroethan-1-ol (90b)

To a solution of **90a** (200 mg, 1.14 mmol) and K₂CO₃ (16 mg, 0.11 mmol) in THF (6 mL) was added TMSCF₃ (242 mg, 1.70 mmol, 0.27 mL), the mixture was stirred at 25 °C for 4 h. TBAF (1.7 mL, 1.70 mmol, 1 M in THF) was added to the solution and the mixture was stirred for 12 h. The reaction mixture was diluted with water (50 mL) and extracted with EtOAc (50 mL*2). The combined organic layer was washed with brine (30 mL*2), dried over anhydrous Na₂SO₄, filtered and concentrated. The residue was purified by column chromatography (EtOAc in PE is 25%) to give **90b** (195 mg, 70% yield) as a colorless oil. LC-MS (ESI, Method) t_{R} = 2.66 min, m/z (M-H)⁻ = 243.9.

### Step 2. N-(4-chloro-5-(2,2,2-trifluoro-1-hydroxyethyl)pyridin-2-yl) cyclopropanecarboxamide (90c)

To a solution of **90b** (185 mg, 0.75 mmol) and cyclopropanecarboxamide (64 mg, 0.75 mmol) in dioxane (10 mL) was added Pd(OAc)₂ (34 mg, 0.15 mmol), dppf (167 mg, 0.30 mmol) and K₃PO₄ (479 mg, 2.26 mmol) under N₂ atmosphere, the mixture was stirred at 85 °C for 2 h. The reaction mixture was diluted with water (20 mL) and extracted with EtOAc (20 mL*2). The combined organic layer was washed with brine (20 mL*2), dried over anhydrous Na₂SO₄, filtered and concentrated. The residue was purified by column chromatography (EtOAc in PE is 20%) to give **90c** (130 mg, 59% yield) as an off-white solid. ¹H NMR (400 MHz, CDCl₃) *δ* 8.51 (s, 1H), 8.47 (s, 1H), 8.33 (s, 1H), 5.55 (q, *J =* 6.4 Hz, 1H), 3.17 (s, 1H), 1.65-1.50 (m, 1H), 1.17-1.06 (m, 2H), 0.98-0.91 (m, 2H). LC-MS (ESI, Method 4) t_{R} = 2.54 min, m/z (M+H)⁺ = 295.1.

### Step 3. N-(4-((4-methoxy-5-((S)-2,2,2-trifluoro-1-methoxyethyl)pyrazolo[1,5-a]pyridin-3-yl)amino)-5-(2,2,2-trifluoro-1-hydroxyethyl)pyridin-2-yl)cyclopropanecarboxamide (90)

To a solution of **90c** (29 mg, 0.098 mmol), **27b** (27 mg, 0.098 mmol) and K₂CO₃ (41 mg, 0.29 mmol) in ^{t}BuOH (2.5 mL) was added BrettPhos Pd G3 (18 mg, 0.020 mmol) and BrettPhos (21 mg, 0.039 mmol) under N₂ atmosphere, the mixture was stirred at 110 °C under microwave radiation for 3.5 h. The reaction mixture was diluted with water (20 mL) and extracted with EtOAc (20 mL*2). The combined organic layer was washed with brine (20 mL*2), dried over anhydrous Na₂SO₄, filtered and concentrated. The residue was purified by *Prep*-HPLC (Method E) to give **90** (8 mg, 15% yield) as a white solid. ¹H NMR (400 MHz, DMSO-*d*₆) *δ* 10.49 (s, 1H), 8.54 (d, *J =* 7.2 Hz, 1H), 8.13-8.08 (m, 2H), 8.07-8.03 (m, 1H), 7.46-7.39 (m, 1H), 7.20-7.12 (m, 1H), 6.77 (d, *J =* 7.2 Hz, 1H), 5.58 (q, *J =* 6.8 Hz, 1H), 5.31-5.21 (m, 1H), 3.67 (s, 3H), 3.31 (s, 3H), 1.96-1.81 (m, 1H), 0.71-0.56 (m, 4H). LC-MS (ESI, Method 4) t_{R} = 2.47 min, m/z (M+H)⁺ = 534.4.

### Example 91

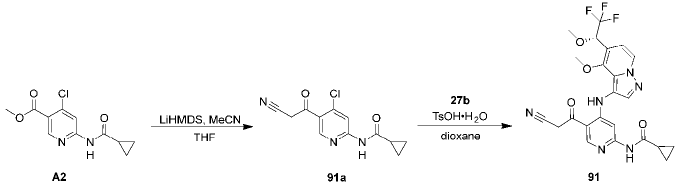

### Step 1. N-(4-chloro-5-(2-cyanoacetyl)pyridin-2-yl)cyclopropanecarboxamide (91a)

To a solution of MeCN (403 mg, 0.51 mL, 9.82 mmol) in THF (2.5 mL) was slowly added LiHMDS (5.89 mL, 5.89 mmol, 1 M in THF) at -60°C and the solution was stirred at - 60°C for 30 min. Then a solution of **A2** (500 mg, 1.96 mmol) in THF (2.5 mL) was added to the mixture at -60°C and the mixture was stirred at -60°C for 1 h. The reaction was quenched with ice-water (2 mL) and extracted with EtOAc (3 mL*2). The organic layer was concentrated and purified by flash chromatography on silica gel (DCM/MeOH = 20/1) to give the **91a** (350 mg, 68% yield) as a yellow solid. LC-MS (ESI, Method 3): t_{R} = 1.11 min, m/z (M+H)⁺ = 264.1.

### Step 2. (S)-N-(5-(2-cyanoacetyl)-4-((4-methoxy-5-(2,2,2-trifluoro-1-methoxyethyl)pyrazolo[1,5-a]pyridin-3-yl)amino)pyridin-2-yl)cyclopropanecarboxamide (91)

To a solution of **91a** (50 mg, 0.19 mmol) and **27b** (52 mg, 0.19 mmol) in dioxane (0.6 mL) was added TsOH·H₂O (4 mg, 0.019 mmol) and the mixture was stirred at 100°C for 1.5 h. The formed solid was filtered and purified by flash chromatography (DCM/MeOH = 20/1) to afford **91** (40 mg, 42% yield). ¹H NMR (400 MHz, DMSO-*d*₆): δ 10.92(s, 1H), 10.37 (s, 1H), 8.68 (s, 1H), 8.57 (d, *J =* 7.2 Hz, 1H), 8.18 (s, 1H), 7.69 (s, 1H), 6.81 (d, *J =* 7.6 Hz, 1H), 5.29-5.26 (m, 1H), 4.84 (s, 2H), 3.70 (s, 3H), 3.42 (s, 3H), 1.98-1.95 (m, 1H), 0.76-0.69 (m, 4H). LC-MS (ESI, Method 2): t_{R} = 0.93 min, m/z (M+H)⁺ = 503.1.

### Example 92

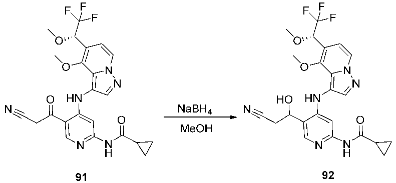

### Step 1. N-(5-(2-cyano-1-hydroxyethyl)-4-((4-methoxy-5-((S)-2,2,2-trifluoro-1-methoxyethyl)pyrazolo[1,5-a]pyridin-3-yl)amino)pyridin-2-yl)cyclopropanecarboxamide (92)

To a solution of **91** (15 mg, 0.0299 mmol) in MeOH (0.5 mL) was added NaBH₄ (6 mg, 0.149 mmol) at 0 °C and the mixture was stirred at 0 °C for 0.5 h. The reaction mixture was purified by *Prep*-HPLC (Method B) to give 92 (7.4 mg, 49% yield) as a yellow solid. ¹H NMR (400 MHz, DMSO-*d*₆): δ 10.40 (s, 1H), 8.53 (d, *J=* 7.2 Hz, 1H), 8.06 (d, *J =* 1.6 Hz, 1H), 8.05 (d, *J* = 2.8 Hz, 1H), 7.91 (s, 1H), 7.42 (s, 1H), 6.77 (d, *J =* 7.2 Hz, 1H), 6.28 (s, 1H), 5.27-5.24 (m, 1H), 5.20 (s, 1H), 3.69 (s, 3H), 3.51 (s, 3H), 2.99-2.97 (m, 2H), 1.89-1.86 (m, 1H), 0.67-0.61 (m, 4H). LC-MS (ESI, Method 2): t_{R} = 0.84 min, m/z (M+H)⁺ = 505.1.

### Example 93

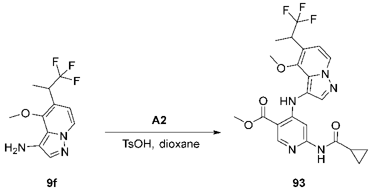

### Step 1. Methyl 6-(cyclopropanecarbonylamino)-4-[[4-methoxy-5-(2,2,2-trifluoro-1-methylethyl)pyrazolo[1,5-a]pyridin-3-yl]amino]pyridine-3-carboxylate (93)

A mixture of **9f** (44 mg, 0.17 mmol), **A2** (35 mg, 0.14 mmol) and TsOH (29 mg, 0.17 mmol) in dioxane (3 mL) was stirred at 100 °C for 12 h. A yellow solution was formed. The reaction mixture was diluted with EtOAc (100 mL), then washed with aq. NaHCO₃ (50 mL*3) and brine (50 mL*2). The organic phase was dried over anhydrous Na₂SO₄, filtered and concentrated. The residue was purified by column chromatograph (EA in PE is 5-50%) to give **93** (63.5 mg, 78% yield) as a yellow solid. ¹H NMR (400 MHz, CDCl₃): *δ* 9.90 (s, 1H), 8.73 (s, 1H), 8.25 (brs, 1H), 8.20 (d, *J =* 7.2 Hz, 1H), 8.08 (s, 1H), 7.88 (s, 1H), 6.72 (d, *J =* 7.2 Hz, 1H), 4.13-4.04 (m, 1H), 3.95 (s, 3H), 3.74 (s, 3H), 1.52-1.42 (m, 1H), 1.44 (d, *J* = 7.2 Hz, 3H), 1.05-1.00 (m, 2H), 0.88-0.80 (m, 2H). LCMS (ESI, Method 4) t_{R} = 2.84 min, m/z (M+H)⁺ = 478.3.

### Example 94

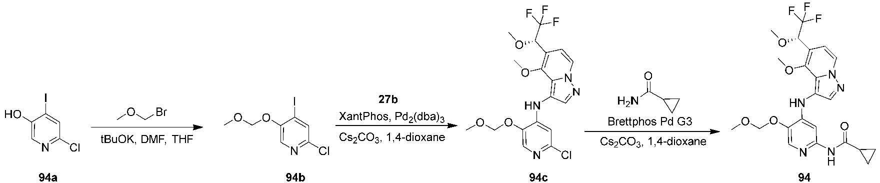

### Step 1. 2-Chloro-4-iodo-5-(methoxymethoxy)pyridine (94b)

To a solution of **94a** (200 mg, 783 mmol) and Potassium tert-butoxide (176 mg, 1.57 mmol) in DMF (2 mL) and THF (1 mL) was added bromo(methoxy)methane (147 mg, 1.17 mmol) at 0 °C. The mixture was stirred at 0 °C for 2 h, then it was diluted with water (5 mL) and extracted with EtOAc (10 mL*3). The combined organic layer was washed with brine (5 mL*3) and concentrated. The residue was purified with column chromatography (PE/EtOAc = 3/1) to give **94b** (195 mg, 83% yield) as a white solid. ¹H NMR (400 MHz, DMSO-*d*₆) δ 8.09 (s, 1H), 8.00 (s, 1H), 8.36 (s, 2H), 3.42 (s, 3H).

### Step 2. (S)-N-(2-chloro-5-(methoxymethoxy)pyridin-4-yl)-4-methoxy-5-(2,2,2-trifluoro-1-methoxyethyl)pyrazolo[1,5-a]pyridin-3-amine (94c)

A mixture of **27b** (50 mg, 0.18 mmol), **94b** (70.3 mg, 0.235 mmol), Pd₂(dba)₃ (33.0 mg, 0.036 mmol), XantPhos (20.8 mg, 0.036 mmol) and Cs₂CO₃ (117.5 mg, 0.36 mmol) in 1,4-dioxane (1 mL) was stirred at 90 °C for 5 h under N₂ atmosphere. The mixture was concentrated and purified by flash chromatography (DCM/MeOH = 20/1) to give **94c** (70 mg, 87% yield) as a yellow solid. LC-MS (ESI, Method 3) t_{R} = 1.26 min, m/z (M+H)⁺ = 447.4.

### Step 3. (S)-N-(4-((4-methoxy-5-(2,2,2-trifluoro-1-methoxyethyl)pyrazolo[1,5-a]pyridin-3-yl)amino)-5-(methoxymethoxy)pyridin-2-yl)cyclopropanecarboxamide (94)

A mixture of **94c** (70 mg, 0.162 mmol), cyclopropanecarboxamide (68.8 mg, 0.809 mmol), BrettPhos Pd G3 (29.3 mg, 0.0324 mmol) and Cs₂CO₃ (105.4 mg, 0.323 mmol) in dioxane (1 mL) was stirred at 90 °C for 5 h under N₂ atmosphere. The reaction mixture was concentrated and purified by column chromatography (DCM/MeOH = 20/1) to give **94** (42.5 mg, 53% yield) as a white solid. ¹H NMR (400 MHz, DMSO-*d*₆) δ 10.29 (s, 1H), 8.53 (d, *J =* 6.8 Hz, 1H), 8.06 (s, 1H), 7.82 (s, 1H), 7.77 (s, 1H), 7.34 (s, 1H), 6.77 (d, *J =* 6.8 Hz, 1H), 5.26-5.24 (m, 1H), 5.22 (s, 2H), 3.70 (s, 3H), 3.51 (s, 3H), 3.35 (s, 3H), 1.86-1.83 (m, 1H), 0.64-0.61 (m, 4H). LC-MS (ESI, Method 2) t_{R} = 0.87 min, m/z (M+H)⁺ = 496.1.

### Example 95

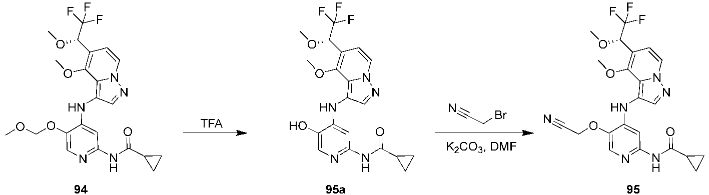

### Step 1. (S)-N-(5-hydroxy-4-((4-methoxy-5-(2,2,2-trifluoro-1-methoxyethyl)pyrazolo[1,5-a]pyridin-3-yl)amino)pyridin-2-yl)cyclopropanecarboxamide (95a)

A solution of **94** (40 mg, 0.0807 mmol) and TFA (1 mL) was stirred at 25 °C for 3 h. The solvents was removed by pumping wiht N₂ gas to give **95a** (30 mg, 66% yield TFA salt) as brown oil. The mixture was used for the next step directly without further purification. LC-MS (ESI, Method 3) t_{R} = 1.13 min, m/z (M+H)⁺ = 452.4.

### Step 2. (S)-N-(5-(cyanomethoxy)-4-((4-methoxy-5-(2,2,2-trifluoro-1-methoxyethyl)pyrazolo[1,5-a]pyridin-3-yl)amino)pyridin-2-yl)cyclopropanecarboxamide (95)

A mixture of **95a** (30 mg, 0.0665 mmol), 2-bromoacetonitrile (23.9 mg, 0.199 mmol) and K₂CO₃ (36.7 mg, 0.239 mmol) in DMF (0.3 mL) was stirred at 25 °C for 12 h. The solid was filtered off and the filtrate was concentrated. The residue was purified by *Prep*-HPLC (Method A) to give **95** (8 mg, 25% yield) as a white solid. ¹H NMR (400 MHz, DMSO-*d*₆) δ 10.34 (s, 1H), 8.53 (d, *J =* 6.4 Hz, 1H), 8.05 (s, 1H), 7.99 (s, 1H), 7.93 (s, 1H), 7.32 (s, 1H), 6.79 (d, *J =* 6.4 Hz, 1H), 5.27-5.24 (m, 1H), 5.21 (s, 2H), 3.69 (s, 3H), 3.32 (s, 3H), 1.86-1.83 (m, 1H), 0.66-0.60 (m, 4H). LC-MS (ESI, Method 2) t_{R} = 0.84 min, m/z (M+H)⁺ = 491.1.

### Example 96

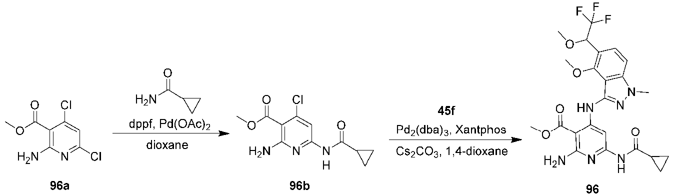

### Step 1. Methyl 2-amino-4-chloro-6-(cyclopropanecarboxamido) nicotinate (96b)

To a mixture of cyclopropanecarboxamide (288.8 mg, 3.39 mmol), **96a** (500 mg, 2.26 mmol) and K₃PO₄ (720.2 mg, 3.39 mmol) in dioxane (5 mL) was added dppf (62.7 mg, 113.10 µmol) and Pd(OAc)₂ (25.4 mg, 113.10 µmol). The mixture was stirred at 90 °C for 3 h under N₂ atmosphere. The reaction mixture was cooled to room temperature and concentrated. The residue was purified by flash chromatography on silica gel (PE/EtOAc = 10/1) to give **96b** (557 mg, 99% yield) as a white solid. LC-MS (ESI, Method 3): t_{R} = 1.17 min, m/z (M+H)⁺ = 270.3.

### Step 2. Methyl 2-amino-6-(cyclopropanecarboxamido)-4-((4-methoxy-1-methyl-5-(2,2,2-trifluoro-1-methoxyethyl)-1H-indazol-3-yl) amino) nicotinate (96)

A mixture of **96b** (51.3 mg, 190.14 µmol), **45f** (50.00 mg, 172.86 µmol), Pd₂(dba)₃ (31.7 mg, 34.57 µmol), Xantphos (20.0 mg, 34.57 µmol) and Cs₂CO₃ (112.6 mg, 345.72 µmol) in dioxane (1 mL) was stirred at 90°C for 16 h under N₂. The reaction mixture was cooled to room temperature and concentrated. The residue was purified by flash chromatography (DCM/MeOH = 20/1) to afford the crude product. Then the crude product was purified by *Prep-*HPLC (Method A) to give **96** (2.4 mg, 3% yield) as a white solid. ¹H NMR (400 MHz, DMSO-*d*₆): δ 10.93 (s, 1H), 10.19 (s, 1H), 8.26 (s, 1H), 7.45 (d, *J =* 8.8 Hz, 1H), 7.39 (d, *J =* 8.8 Hz, 1H), 6.71 (s, 2H), 5.28-5.24 (m, 1H), 3.96 (s, 3H), 3.91 (s, 3H), 3.82 (s, 3H), 3.30 (s, 3H), 2.03-1.98 (m, 1H), 0.76-0.43 (m, 4H). LC-MS (ESI, Method 2): t_{R} = 0.94 min, m/z (M+H)⁺ = 523.1.

### Example 97

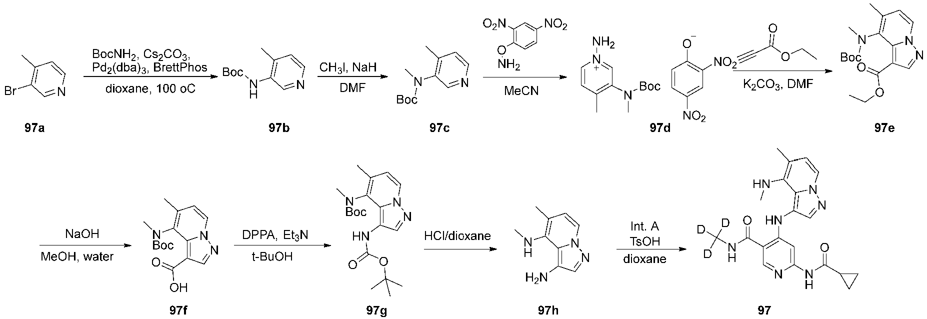

### Step 1. Tert-butyl N-(4-methyl-3-pyridyl)carbamate (97b)

A mixture of **97a** (2.00 g, 11.63 mmol), BocNH₂ (2.04 g, 17.44 mmol), Cs₂CO₃ (11.36 g, 34.88 mmol), BrettPhos (624 mg, 1.16 mmol) and Pd₂(dba)₃ (532mg, 0.58 mmol) in dioxane (20 mL) was stirred at 100 °C under N₂ atmosphere for 12 h. A yellow suspension was formed. The reaction mixture was diluted with EtOAc (100 mL) and filtered through a pad of celite. The filtrate was concentrated and purified by column chromatograph (EA in PE is 10-50%) to give **97b** (2.20 g, 91% yield) as a yellow solid. LC-MS (ESI, Method 4) t_{R} = 0.78 min, m/z (M+H)⁺ = 209.1

### Step 2. Tert-butyl N-methyl-N-(4-methyl-3-pyridyl)carbamate (97c)

A mixture of **97b** (700 mg, 3.36 mmol), NaH (403 mg, 10.08 mmol, 60% purity in mineral oil) and CH₃I (763 mg, 5.38 mmol, 334 µL) in DMF (10 mL) was stirred at 0-10 °C for 2 h. A brown suspension was formed. The reaction mixture was diluted with water (50 mL) and extracted with EtOAc (50 mL*3). The combined organic layer was washed with water(50 mL*3) and brine(50 mL*2), then it was dried over anhydrous Na₂SO₄, filtered and concentrated. The residue was purified by column chromatography (EtOAc in PE is 10-40%) to give **97c** (500 mg, 67% yield) as a yellow oil. ¹H NMR (400 MHz, CDCl₃): *δ* 8.38 (d, *J =* 4.8 Hz, 1H), 8.35 (d, *J =* 5.2 Hz, 1H), 7.16 (d, *J =* 5.2 Hz, 1H), 3.20 (s, 3H), 2.24 (s, 3H), 1.55-1.30 (m, 9H). LC-MS (ESI, Method 4) t_{R} = 1.50 min, m/z (M+H)⁺ = 223.1.

### Step 3. Tert-butyl N-(1-amino-4-methyl-pyridin-1-ium-3-yl)-N-methyl-carbamate (97d)

A mixture of **97c** (500 mg, 2.25 mmol) and O-(2,4-dinitrophenyl) hydroxylamine (493 mg, 2.47 mmol) in MeCN (5 mL) was stirred at 50 °C for 12 h. A yellow suspension was formed. The reaction was concentrated to give **97d** (536 mg, crude) as a yellow solid, which was used for the next step directly without further purification. LC-MS (ESI, Method 4) t_{R} = 0.64 min, m/z (M)⁺ = 238.2

### Step 4. Ethyl 4-[tert-butoxycarbonyl(methyl)amino]-5-methyl-pyrazolo[1,5-a]pyridine-3-carboxylate (97e)

A mixture of ethyl prop-2-ynoate (286 mg, 2.92 mmol, 300 µL), **97d** (536 mg, 2.25 mmol) and K₂CO₃ (622 mg, 4.50 mmol) in DMF (5 mL) was stirred at 20 °C for 2 h. A black suspension was formed. The reaction mixture was diluted with water (50 mL) and extracted with EtOAc (50 mL*2). The combined organic layer was washed with water (50 mL*2) and brine (50 mL*2), then it was dried over anhydrous Na₂SO₄, filtered and concentrated. The residue was purified by column chromatography (EtOAc in PE is 10-50%) to give to **97e** (300 mg, 40% yield) as a yellow solid. ¹H NMR (400 MHz, CDCl₃): *δ* 8.42 (s, 1H), 8.35 (d, *J* = 7.2 Hz, 1H), 6.79 (d, *J* = 7.2 Hz, 1H), 4.29 (q, *J =* 7.2 Hz, 2H), 3.14 (s, 3H), 2.32 (s, 3H), 1.36 (t, *J =* 7.2 Hz, 3H), 1.24 (s, 9H). LC-MS (ESI, Method 4) t_{R} = 3.04 min, m/z (M+H)⁺ = 334.1.

### Step 5. 4-[Tert-butoxycarbonyl(methyl)amino]-5-methyl-pyrazolo[1,5-a]pyridine-3-carboxylic acid (97f)

A well-stirred solution of **97e** (300 mg, 0.90 mmol) and NaOH(108 mg, 2.70 mmol) in MeOH (4 mL) and water (1 mL) was stirred at 70°C for 12 h. A yellow solution was formed. The solution was colled to room temperature and diluted with water (30 mL). And it was neutralized with aq. HCl (1.0 M) until pH = 5-6. The solution was extracted by EtOAc (30 mL*3) and dried with anhydrous Na₂SO₄. Then it was filtered through a pad of celite and evaporated the organic layers in vacuum to give**97f** (260 mg, 975% yield) as a light yellow solid. LC-MS (ESI, Method 4) t_{R} = 2.25 min, m/z (M+H)⁺ = 306.2.

### Step 6. Tert-butyl N-[3-(tert-butoxycarbonylamino)-5-methyl-pyrazolo[1,5-a]pyridin-4-yl]-N-methyl-carbamate (97g)

A mixture of **97f** (160 mg, 0.52 mmol) and DIEA (159 mg, 1.57 mmol, 220 µL) in toluene (5 mL) and *^{t}*BuOH (1 mL) was stirred at 80 °C for 1 h. A yellow solution was formed. Then DPPA (216 mg, 0.79 mmol, 170 µL) was added to the solution. The resulting mixture was stirred at 100 °C for 2 h. After it was cooled to room temperature, the mixture was diluted with EtOAc (30 mL) and quenched with aq. NaHCO₃ (50 mL). Then it was extracted with EtOAc (30 mL*3), washed with water (50 mL*3) and brine (50 mL*2). And it was dried over anhydrous Na₂SO₄, filtered and concentrated. The residue was purified by column chromatograph (EA in PE is 5-30%) to give 97g (30 mg, 15% yield) as a yellow solid. LC-MS (ESI, Method 4) t_{R} = 3.08 min, m/z (M+H)⁺ = 377.3.

### Step 7. N⁴,5-dimethylpyrazolo[1,5-a]pyridine-3,4-diamine (97h)

A solution of **97g** (20 mg, 0.05 mmol) in HCl/MeOH (2 mL, 4 mol/L) was stirred at 20 °C for 1 h. A yellow solution was formed. The reaction mixture was concentrated in vacuo to give 97h (9 mg, HCl salt) as a yellow solid. LC-MS (ESI, Method 4) t_{R} = 0.55 min, m/z (M+H)⁺ = 177.1

### Step 8. 6-(Cyclopropanecarbonylamino)-4-[[5-methyl-4-(methylamino)pyrazolo [1,5-a]pyridin-3-yl]amino]-N-(trideuteriomethyl)pyridine-3-carboxamide (97)

A mixture of **97h** (9 mg, 0.05 mmol), **Int. A** (10 mg, 0.04 mmol) and TsOH (9 mg, 0.05 mmol) in dioxane (1 mL) was stirred at 100 °C for 12 h. A yellow solution was formed. The reaction mixture was diluted with EtOAc (100 mL) and washed with aq. NaHCO₃ (50 mL*3), brine (50 mL*2). Then it was dried over anhydrous Na₂SO₄, filtered and concentrated. The residue was purified by column chromatograph (MeOH in DCM is 0-10%) and further purified by *Prep*-HPLC (Method E) to give **97** (2.9 mg, 14% yield) as a yellow solid. ¹H NMR (400 MHz, CDCl₃): δ 10.59 (s, 1H), 10.04 (s, 1H), 8.47 (s, 1H), 8.42 (s, 1H), 8.08 (d, *J* = 6.8 Hz, 1H), 8.83 (s, 1H), 7.52 (s, 1H), 6.59 (d, *J =* 7.2 Hz, 1H), 4.34 (q, *J =* 6.0 Hz, 1H), δ 3.51 (s, 1H), 2.57 (d, *J =* 6.0 Hz, 3H), 2.17 (s, 3H), 1.92-1.83 (m, 1H), 0.72-0.59 (m, 4H). LC-MS (ESI, Method 4) t_{R} = 1.738 min, m/z (M+H)⁺ = 397.3.

### Example 98

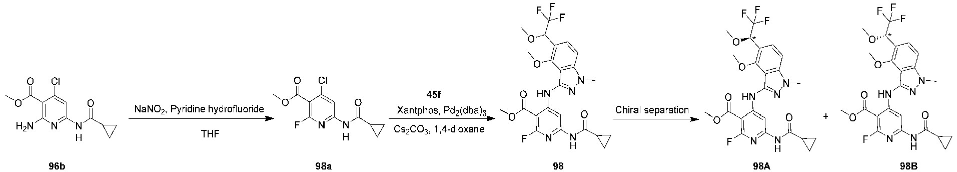

### Step 1. Methyl 4-chloro-6-(cyclopropanecarboxamido)-2-fluoronicotinate (98a)

**96b** (130 mg, 0.482 mmol) and NaNO₂ (133 mg, 1.93 mmol) was dissolved in THF (1.3 mL) and the mixture was cooled to 0 °C. Then it was added pyridine hydro fluoride (238.9 mg, 2.41 mmol). The reaction mixture was stirred at 0 °C for 40 min. The reaction was diluted with EtOAc (5 mL) and *sat.* sodium bicarbonate (5 mL). The mixture was filtered and extracted with EtOAc (5 mL*2) Then the combined organic layer was dried over Na₂SO₄, filtered and concentrated. The residue was purified by column chromatography on silica gle (PE/EtOAc = 5/1) to give **98a** (50 mg, 38% yield) as a white solid. LC-MS (ESI, Method 3): t_{R} = 1.28 min, m/z (M+H)⁺= 273.1.

### Step 2. Methyl 6-(cyclopropanecarboxamido)-2-fluoro-4-((4-methoxy-1-methyl-5-(2,2,2-trifluoro-1-methoxyethyl)-1H-indazol-3-yl)amino)nicotinate (98)

A mixture of **98a** (40 mg, 0.147 mmol), **45f** (46.6 mg, 0.161 mmol), Cs₂CO₃ (95.6 mg, 0.293 mmol), Pd₂(dba)₃ (26.9 mg, 0.029 mmol) and Xantphos (17 mg, 0.029 mmol) in 1,4-dioxane (0.5 mL) was stirred at 90 °C for 3 h under N₂ atmosphere. The reaction mixture was concentrated and purified by column chromatography (PE/EtOAc = 4/1) to give the crude product. The crude compound was re-purified by *Prep*-HPLC (Method A) to give **98** (42 mg, 55% yield) as a white solid. LC-MS (ESI, Method 3): t_{R} = 1.44 min, m/z (M+H)⁺= 526.4.

### Step 3. Methyl (R*)-6-(cyclopropanecarboxamido)-2-fluoro-4-((4-methoxy-1-methyl-5-(2,2,2-trifluoro-1-methoxyethyl)-1H-indazol-3-yl)amino)nicotinate (98A) and Methyl (S*)-6-(cyclopropanecarboxamido)-2-fluoro-4-((4-methoxy-1-methyl-5-(2,2,2-trifluoro-1-methoxyethyl)-1H-indazol-3-yl)amino)nicotinate (98B)

**98** (40 mg, 0.076 mmol) was separated by Chiral-HPLC (Method J, Hex/IPA = 85/15) to give **98A** (16 mg, 40% yield) as a white solid and **98B** (15.5 mg, 39% yield) as a white solid.

**98A:** ¹H NMR (400 MHz, DMSO-*d*₆) δ 11.06 (s, 1H), 10.95 (s, 1H), 8.96 (s, 1H), 7.49 (d, *J* = 8.8 Hz, 1H), 7.42 (d, *J =* 8.8 Hz, 1H), 5.29 (q, *J =* 6.8 Hz, 1H), 3.99 (s, 3H), 3.91 (s, 3H), 3.86 (s, 3H), 3.31 (s, 3H), 2.00-1.93 (m, 1H), 0.86-0.78 (m, 4H). LC-MS (ESI, Method 2): t_{R} = 1.26 min, m/z (M+H)⁺= 525.9. Chiral HPLC (Method 7, Hex/IPA = 85/15): t_{R} = 15.00 min.

**98B:** ¹H NMR (400 MHz, DMSO-*d*₆) δ 11.07 (s, 1H), 10.95 (s, 1H), 8.96 (s, 1H), 7.49 (d, *J* = 8.8 Hz, 1H), 7.42 (d, *J =* 8.8 Hz, 1H), 5.27 (q, *J =* 6.8 Hz, 1H), 3.99 (s, 3H), 3.91 (s, 3H), 3.86 (s, 3H), 3.32 (s, 3H), 2.00-1.94 (m, 1H), 0.84-0.80 (m, 4H). LC-MS (ESI, Method 2): t_{R} = 1.25 min, m/z (M+H)⁺= 526.2. Chiral HPLC (Method 7, Hex/IPA = 85/15): t_{R} = 19.12 min.

### Example 99

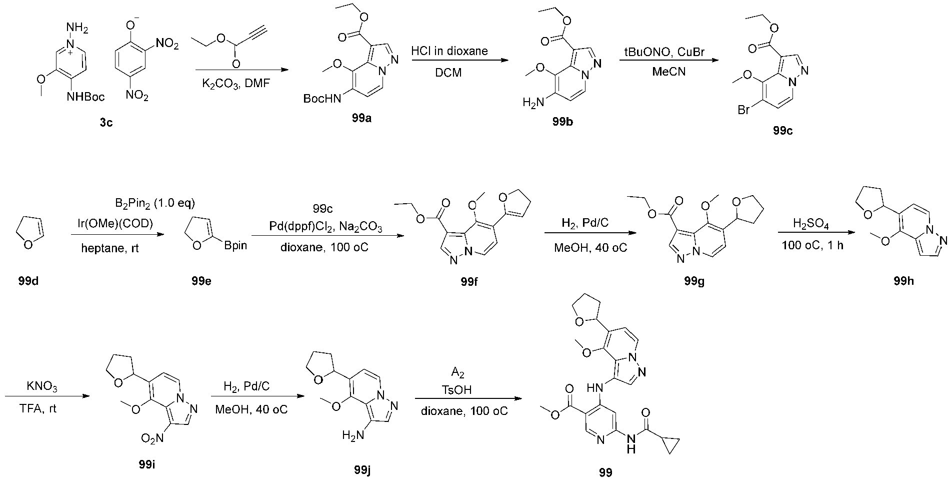

### Step 1. Ethyl 5-((tert-butoxycarbonyl)amino)-4-methoxypyrazolo[1,5-a]pyridine-3-carboxylate (99a)

A mixture of ethyl propiolate (685 mg, 6.98 mmol, 707 µL), **3c** (1.29 g, 5.37 mmol) and K₂CO₃ (1.48 g, 10.74 mmol) in DMF (15 mL) was stirred at 20 °C for 2 h. A black suspension was formed. The reaction mixture was concentrated in vacuo and diluted with water (50 mL), then it was extracted with EtOAc (50 mL*2). The combined organic layer was washed with water (50 mL*2) and brine (50 mL*2), then it was dried over anhydrous Na₂SO₄, filtered and concentrated. The residue was purified by column chromatography (EtOAc in PE is 10-30%) to give to **99a** (428 mg, 22% yield) as a yellow solid. ¹H NMR (400 MHz, CDCl₃): *δ* 8.36 (s, 1H), 8.28 (d, *J =* 7.6 Hz, 1H), 8.00 (d, *J* = 7.6 Hz, 1H), 7.32 (brs, 1H), 4.35 (q, *J* = 7.2 Hz, 1H), 3.89 (s, 3H), 1.55 (s, 9H), 1.39 (t, *J =* 7.2 Hz, 1H). LC-MS (Method 4) t_{R} = 4.16 min, m/z (M+H) = 336.1.

### Step 2. Ethyl 5-amino-4-methoxypyrazolo[1,5-a]pyridine-3-carboxylate (99b)

To a mixture of **99a** (330 mg, 0.98 mmol) in DCM (2 mL) was added HCl in dioxane (4 M, 2 mL) at 20 °C. The resulting mixture was stirred at 20 °C for 1 h. A yellow solution was formed. The rection mixture was concentrated to give **99b** (231 mg, HCl salt, crude) as a yellow solid. LC-MS (Method 4) t_{R} = 0.48 min, m/z (M+H)⁺ = 236.1.

### Step 3. Ethyl 5-bromo-4-methoxy-pyrazolo[1,5-a]pyridine-3-carboxylate (99c)

To a mixture of **99b** (231 mg, HCl salt, crude) in MeCN (5 mL), was added *tert*-butyl nitrite (152 mg, 1.47 mmol, 175 µL) at 0 °C, then the mixture was stirred for 10 min. CuBr (212 mg, 1.47 mmol) was added into the above mixture, and the resulting mixture was stirred at 80 °C for 12 h. A yellow solution was formed. The reaction mixture was concentrated and purified by column chromatograph (EA in PE is 10-30%) to give **99c** (100 mg, 40% yield) as a yellow solid. LC-MS (Method 4) t_{R} = 2.48 min, m/z (M+H)⁺ = 299.2.

### Step 4. 2-(2,3-Dihydrofuran-5-yl)-4,4,5,5-tetramethyl-1,3,2-dioxaborolane (99e)

A mixture of **99d** (4.14 g, 59.07 mmol), B₂Pin₂ (5.00 g, 19.69 mmol), Ir(OMe)(COD) (258 mg, 0.39 mmol) and dtbpy (211 mg, 0.78 mmol) in heptane (60 mL) was stirred at 80 °C under N₂ atmosphere for 12 h. A black suspension was formed. The reaction mixture was diluted with EtOAc (60 mL). The combined organic layer was washed with water (50 mL*3) and brine (50 mL*2), then it was filtered, concentrated and purified by column chromatograph (EA in PE is 0-10%) to give **99e** (2.30 g, 60% yield) as a white solid. ¹H NMR (400 MHz, CDCl₃): *δ 6.84* (t, *J =* 2.0 Hz, 1H), 4.35 (t, *J =* 9.6 Hz, 2H), 2.67 (td, *J =* 9.6, 2.0 Hz, 2H), 1.26 (s, 12H). LC-MS (ESI, Method 4) t_{R} = 2.75 min, m/z (M+H)⁺ = 197.1.

### Step 5. Ethyl 5-(2,3-dihydrofuran-5-yl)-4-methoxy-pyrazolo[1,5-a]pyridine-3-carboxylate (99f)

A mixture of **99e** (350 mg, 1.79 mmol), **99c** (427 mg, 1.43 mmol), Na₂CO₃ (568 mg, 5.36 mmol) and Pd(dppf)Cl₂ (146 mg, 0.18 mmol) in dioxane (10 mL) and water (2 mL) was stirred at 100 °C under N₂ atmosphere for 2 h. A yellow suspension was formed. The reaction mixture was diluted with water (30 mL) and extracted with EtOAc (30 mL*3). The combined organic layer was washed with water (50 mL*3) and brine (50 mL*2), then it was dried over anhydrous Na₂SO₄, filtered and concentrated. The residue was purified by column chromatograph (EA in PE is 0-20%) to give **99f** (280 mg, 68% yield) as a yellow oil. ¹H NMR (400 MHz, CDCl₃): *δ* 8.38 (s, 1H), 8.26 (d, *J* = 7.2 Hz, 1H), 7.49 (t, *J =* 2.0 Hz, 1H), 6.71 (d, *J* = 7.2 Hz, 1H), 4.53 (t, *J* = 9.6 Hz, 2H), 4.37 (q, *J* = 7.2 Hz, 2H), 3.82 (s, 3H), 3.08 (td, *J* = 9.6, 2.0 Hz, 2H), 1.40 (q, *J* = 7.2 Hz, 3H). LC-MS (ESI, Method 4) t_{R} = 2.82 min, m/z (M+H)⁺ = 289.1.

### Step 6. Ethyl 4-methoxy-5-tetrahydrofuran-2-yl-pyrazolo[1,5-a]pyridine-3-carboxylate (99g)

To a solution of **99f** (280 mg, 0.97 mmol) in MeOH (5 mL), was added Pd/C (30 mg, 10% wt%, 10% Pd (dry basis), wetted with 55% H₂O). The resulting mixture was stirred at 40 °C under H₂ atmosphere for 12 h. A black suspension was formed. The reaction mixture was filtered and concentrated to give **99g** (280 mg, crude) as a colorless oil. LC-MS (ESI, Method 4) t_{R} = 2.48 min, m/z (M+H)⁺ = 291.2.

### Step 7. 4-Methoxy-5-tetrahydrofuran-2-yl-pyrazolo[1,5-a]pyridine (99h)

A solution of **99g** (280 mg, 0.96 mmol) in 5 mL of 50% H₂SO₄ was stirred at 110°C for 1 h. A yellow solution was formed. The mixture was cooled to room temperature and neutralized with aq. NaOH (1.0 M). The above solution was added 40 mL of water and extracted with EtOAc (30 mL*3). The combined organic layer was dried over anhydrous Na₂SO₄, then it was filtered and concentrated to give **99h** (50 mg, 24% yield) as a yellow oil. ¹H NMR (400 MHz, CDCl₃): *δ* 8.31 (d, *J* = 7.2 Hz, 1H), 7.91 (d, *J =* 2.4 Hz, 1H), 6.71 (d, *J =* 7.2 Hz, 1H), 6.56 (d, *J* = 2.4 Hz, 1H), 4.14-4.06 (m, 2H), 3.99 (s, 3H), 3.95-3.88 (m, 2H), 3.78-3.72 (m, 1H), 2.42-2.33 (m, 1H), 2.01-1.91 (m, 1H). LC-MS (ESI, Method 4) t_{R} = 2.02 min, m/z (M+H)⁺ = 219.1.

### Step 8. 4-Methoxy-3-nitro-5-tetrahydrofuran-2-yl-pyrazolo[1,5-a]pyridine (99i)

A mixture of **99h** (50 mg, 0.23 mmol) and KNO₃ (18 mg, 0.18 mmol) in TFA (3 mL) was stirred at 20 °C for 2 h. A yellow solution was formed. The reaction mixture was diluted with EtOAc (30 mL) and quenched with aq. NaHCO₃ (50 mL). the solution was extracted with EtOAc (30 mL*3) and the combined organic layer was washed with water (50 mL*3) and brine (50 mL*2) then it was dried over anhydrous Na₂SO₄, filtered and concentrated. The residue was purified by column chromatograph (EA in PE is 5-50%) to give **99i** (35 mg, 58% yield) as a yellow solid. ¹H NMR (400 MHz, CDCl₃): *δ* 8.64 (s, 1H), 8.35 (d, *J =* 7.2 Hz, 1H), 7.10 (d, *J =* 7.2 Hz, 1H), 4.19-4.12 (m, 1H), 4.11-4.03 (m, 2H), 3.95-3.88 (m, 1H), 3.90 (s, 3H), 3.86-3.77 (m, 1H), 2.53-2.45 (m, 1H), 1.99-1.90 (m, 1H). LC-MS (ESI, Method 4) t_{R} = 2.19 min, m/z (M+H)⁺ = 264.1.

### Step 9. 4-Methoxy-5-tetrahydrofuran-2-yl-pyrazolo[1,5-a]pyridin-3-amine (99j)

To a solution of **99i** (55 mg, 0.21 mmol) in MeOH (3 mL) was added Pd/C (10 mg, 10% wt%, 10% Pd (dry basis), wetted with 55% H₂O). The mixture was stirred at 40 °C under H₂ atmosphere for 12 h. A black suspension was formed. The reaction mixture was filtered, concentrated and purified by column chromatograph (MeOH in DCM is 0-10%) to give **99j** (28 mg, 57% yield) as a yellow solid. LC-MS (ESI, Method 4) t_{R} = 0.47 min, m/z (M+H)⁺ = 234.1.

### Step 10. Methyl 6-(cyclopropanecarboxamido)-4-((4-methoxy-5-(tetrahydrofuran-2-yl)pyrazolo[1,5-a]pyridin-3-yl)amino)nicotinate (99)

A mixture of **99j** (28 mg, 0.12 mmol), **A2** (25 mg, 0.10 mmol) and TsOH (21 mg, 0.12 mmol) in dioxane (3 mL) was stirred at 100 °C for 12 h. A yellow solution was formed. The reaction mixture was diluted with EtOAc (100 mL) and washed with aq. NaHCO₃ (50 mL*3), brine (50 mL*2). Then it was dried over anhydrous Na₂SO₄, filtered and concentrated. The residue was purified by column chromatograph (MeOH in DCM is 0-10%) and prep-HPLC (Method E) to give **99** (23.1 mg, 53% yield) as a yellow solid. ¹H NMR (400 MHz, DMSO-*d₆*): *δ* 10.82 (s, 1H), 9.56 (s, 1H), 8.66 (s, 1H), 8.46 (d, *J =* 7.2 Hz, 1H), 8.06 (s, 1H), 7.66 (s, 1H), 6.83 (d, *J* = 7.2 Hz, 1H), 4.04-3.92 (m, 2H), 3.88 (s, 3H), 3.82-3.70 (m, 2H), 3.65 (s, 3H), 3.61-3.55 (m, 1H), 2.34-2.23 (m, 1H), 1.98-1.84 (m, 2H), 0.77-0.68 (m, 4H). LCMS (ESI, Method 4) t_{R} = 2.192 min, m/z (M+H)⁺ = 452.3.

### Example 100

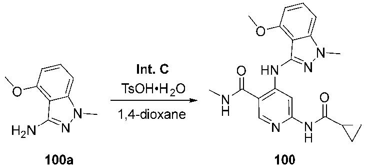

### 6-(Cyclopropanecarboxamido)-4-((4-methoxy-1-methyl-1H-indazol-3-yl) amino)-N-methylnicotinamide (100)

A mixture of 100a (50 mg, 0.282 mmol), **Int. C** (82.3 mg, 0.324 mmol) and TsOH·H₂O (5.37 mg, 0.028 mmol) in 1,4-dioxane (0.5 mL) was stirred at 110 °C for 16 h. After cooling to room temperature, the mixture was basified with NH₃·H₂O (0.3 mL) and concentrated. The residue was purified by *Prep*-HPLC (Method A) to afford **100** (65 mg, 58% yield) as a yellow solid. LC-MS (ESI, Method 2): t_{R} = 1.13 min, m/z (M+H)⁺= 395.1. ¹H NMR (400 MHz, DMSO-*d*₆) δ 11.69 (s, 1H), 10.67 (s, 1H), 9.24 (s, 1H), 8.55 (d, *J=* 4.8 Hz, 1H), 8.52 (s, 1H), 7.29 (t, *J=* 8.0 Hz, 1H), 7.04 (d, *J =* 8.4 Hz, 1H), 6.49 (d, *J =* 7.6 Hz, 1H), 3.96 (s, 3H), 3.89 (s, 3H), 2.83 (d, *J* = 4.0 Hz, 3H), 2.07-2.01 (m, 1H), 0.85-0.78 (m, 4H).

### Example 101

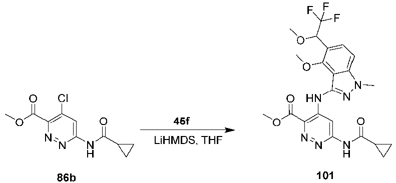

### Step 1. Methyl 6-(cyclopropanecarboxamido)-4-((4-methoxy-1-methyl-5-(2,2,2-trifluoro-1-methoxyethyl)-1H-indazol-3-yl)amino)pyridazine-3-carboxylate (101)

To a solution of **45f** (30 mg, 0.10 mmol) and **86b** (32 mg, 0.12 mmol) in THF (0.3 mL) was added LiHMDS (0.4 mL, 0.4 mmol, 1 M in THF) at -40 °C. The solution was stirred for 2 h at -40 °C, then it was quenched with H₂O (2 mL) and evaporated the organic solvent. The mixture was extracted with EtOAc (5 mL*3) and the organic layer was dried over Na₂SO₄, filtered and concentrated. The residue was purified by *Prep-HPLC* (Method A) to give 101 (1.1 mg, 2.1% yield) as a white solid. ¹H NMR (400 MHz, CD₃OD) δ 8.27 (s, 1H), 7.52 (d, *J* = 8.4 Hz, 1H), 7.40 (d, *J =* 8.4 Hz, 1H), 5.24-5.19 (m, 1H), 4.06 (s, 3H), 4.04 (s, 3H), 3.98 (s, 3H), 3.35 (s, 3H), 2.01-1.96 (m, 1H), 1.06-0.96 (m, 4H LC-MS (ESI, Method 2): t_{R} = 1.08 min, m/z (M+H)⁺= 508.7.

### Example 102

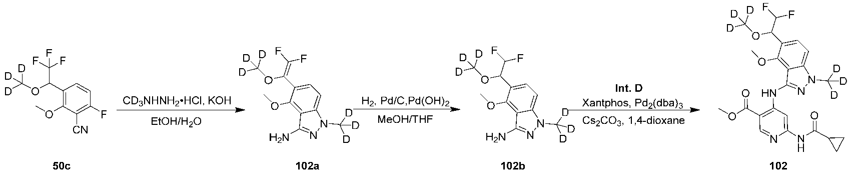

### Step 1. 5-(2,2-Difluoro-1-(methoxy-d₃)vinyl)-4-methoxy-1-(methoxy-d₃)-1H-indazol-3-amine (102a)

To a solution of CD₃NHNH₂.HCl (700 mg, 5.74 mmol, CAS: 2303489-12-3) in EtOH/H₂O (0.5 mL/0.1 mL) was added KOH (600 mg, 10.69 mmol) under ice-bath and the solution was stirred at 30 °C for 30 min. The formed solid was filtered off and the filtrate was added with **50c** (500 mg, 1.88 mmol). The reaction mixture was stirred at 90 °C for 6 h in a sealed tube. The mixture was concentrated and purified by column chromatography on silica gel (PE/EtOAc = 2/1) to give the crude compound 102a (80 mg, 15% yield) as a yellow oil. LC-MS (ESI, Method 3): t_{R} = 1.17 min, m/z (M+H)⁺= 276.4.

### Step 2 5-(2,2-Dilluoro-1-(methoxy-d₃)ethyl)-4-methoxy-1-(methyl-d₃)-1H-indazol-3-amine (102b)

A mixture of 102a (80 mg, 0.291 mmol), Pd/C (20 mg, 10% Palladium on Carbon wetted with 50% water) and Pd(OH)₂ (10 mg) in MeOH (2 mL) and THF (0.5 mL) was stirred at 40 °C for 16 h under H₂ (1 atm) atmosphere. After cooling to r.t., the mixture was filtered and concentrated. The residue was purified by *Prep*-HPLC (Method A) to give **102b** (22 mg, 27%yield) as a yellow solid. LC-MS (ESI, Method 3): t_{R} = 1.11 min, m/z (M+H)⁺= 278.3.

### Step 3. Methyl 6-(cyclopropanecarboxamido)-4-((5-(2,2-difluoro-1-(methoxy-d₃)ethyl)-4-methoxy-1-(methyl-d₃)-1H-indazol-3-yl)amino)nicotinate (102)

A mixture of **102b** (22 mg, 0.0793 mmol), **A2** (24.3 mg, 0.0952 mmol), Cs₂CO₃ (51.7 mg, 0.159 mol), Xantphos (9.2 mg, 0.0159 mmol) and Pd₂(dba)₃ (14.5 mg, 0.0159 mmol) in 1,4-dioxane (0.5 mL) was stirred at 90 °C for 4 h under N₂ atmosphere in sealed tube. The reaction mixture was concentrated and purified by column chromatography on silica gel (DCM/MeOH = 5/1) to give crude product. The crude compound was purified by *Prep*-HPLC (Method A) to give **102** (13.5 mg, 34%yield) as a white solid. ¹H NMR (400 MHz, DMSO-*d*₆) δ 10.91 (s, 1H), 10.88 (s, 1H), 9.20 (s, 1H), 8.77 (s, 1H), 7.44 (d, *J* = 8.8 Hz, 1H), 7.41 (d, *J* = 8.8 Hz, 1H), 6.29 (td,*J* = 55.6, 4.8 Hz, 1H), 4.90-4.84 (m, 1H), 3.92 (s, 3H), 3.87 (s, 3H), 2.08-1.99 (m, 1H), 0.88-0.76 (m, 4H). LC-MS (ESI, Method 2): t_{R} = 0.92 min, m/z (M+H)⁺= 496.1.

### Example 103

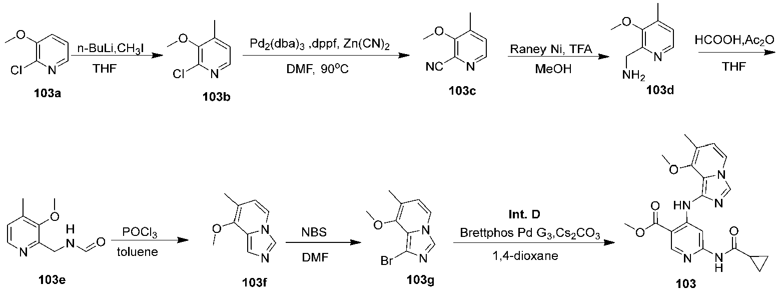

### Step 1. 2-Chloro-3-methoxy-4-methylpyridine (103b)

To a solution of **103a** (1 g, 6.97 mmol) in THF (10 mL) was added n-BuLi (5.3 mL, 8.48 mmol, 1.6 M in THF) at -60 °C. The reaction mixture was stirred for 1 h at -60 °C. Then CH₃I (1.48 g, 10.45 mmol) was added to the solution at -60 °C. The reaction mixture was stirred for another 2 h at the same temperature. The reaction was quenched with H₂O (10 mL) and extracted with EtOAc (10 mL*2). The combined organic layer was concentrated and purified by flash chromatography (PE/EtOAc = 20/1) to give **103b** (300 mg, 27% yield) as a colorless liquid. LC-MS (ESI, Method 3) t_{R} = 1.09 min, m/z (M+H)⁺= 158.0.

### Step 2. 3-Methoxy-4-methylpicolinonitrile (103c)

A mixture **of 103b** (1.5 g, 9.52 mmol), Zn(CN)₂ (2.24 g, 19.04 mmol), dppf (1.06 g, 1.90 mmol) and Pd₂(dba)₃ (872 mg, 0.952 mmol) in DMF (15 mL) was stirred at 90 °C under N₂ atmosphere for 12 h. The solid was filtered off and the filtrate was concentrated. The residue was purified by flash chromatography (PE/EtOAc = 5/1) to give **103c** (1 g, 71% yield) as a yellow solid. ¹H NMR (400 MHz, DMSO-*d*₆): δ 8.36 (d, *J =* 4.8 Hz, 1H), 7.63 (d, *J =* 4.8 Hz, 1H), 3.98 (s, 3H), 2.34 (s, 3H). LC-MS (ESI, Method 3) t_{R} = 1.05 min, m/z (M+H)⁺= 149.3.

### Step 3. (3-Methoxy-4-methylpyridin-2-yl)methanamine (103d)

To a mixture of **103c** (1.5 g, 10.12 mmol) and Raney Ni (1.0 g) in MeOH (20 mL) was added TFA (1.15 g, 10.12 mmol). The reaction mixture was stirred at 50 °C unde H₂ (50 Psi) atmosphere for 12 h. The catalyst was filtered off and the filtrate was concentrated. The residue was purified by *Prep*-HPLC (Method A) to give **103d** (1 g, 65% yield) as green oil. LC-MS (ESI, Method 3) t_{R} = 0.41 min, m/z (M+H)⁺= 153.4.

### Step 4. N-((3-Methoxy-4-methylpyridin-2-yl)methyl)formamide (103e)

A mixture of HCOOH (3 mL) and Ac₂O (9 mL) was stirred at 50 °C for 1 h. The reaction mixture was cooled to 0 °C. A solution of **103d** (1 g, 6.57 mmol) in THF (3 mL) was added into the reaction mixture at 0 °C. After stirring for 2 h at 0 °C, the reaction mixture was diluted with ice-water (5 mL) and extracted with EtOAc (5 mL*2). The combined organic phase was washed with brine (1 mL) and concentrated. The residue was purified by flash chromatography (DCM/MeOH = 20/1) to give **103e** (900 mg, 76% yield) as a colorless oil. LC-MS (ESI, Method 3) t_{R} = 0.30 min, m/z (M+H)⁺= 181.3.

### Step 5. 8-Methoxy-7-methylimidazo[1,5-a]pyridine (103f)

To a solution of **103e** (900 mg, 4.99 mmol) in toluene (15 mL) was added POCl₃ (3.06 g, 19.98 mmol, 1.86 mL) at 0 °C. After stirring at 100 °C for 2 h. The reaction mixture was concentrated and purified by flash chromatography (DCM/MeOH = 20/1) to give **103f** (650 mg, 80% yield) as a yellow oil. ¹H NMR (400 MHz, DMSO-*d*₆): δ 8.31 (s, 1H), 8.04 (d, *J =* 6.4 Hz, 1H), 7.34 (s, 1H), 8.49 (d, *J =* 6.8 Hz, 1H), 3.92 (s, 3H), 2.12 (s, 3H). LC-MS (ESI, Method 3) t_{R} = 0.70 min, m/z (M+H)⁺= 163.1.

### Step 6. 1-Bromo-8-methoxy-7-methylimidazo[1,5-a]pyridine (103g)

To a solution of **103f** (300 mg, 1.85 mmol) in DMF (3 mL) was added NBS (280 mg, 1.57 mmol) portionwise at -60 °C. The reaction was stirred at -60 °C for 1 h and diluted with H₂O (3 mL). The mixture was extracted with EtOAc (3 mL*2). The combined organic layer was concentrated and purified by flash chromatography (DCM/MeOH = 50/1) to give **103g** (280 mg, 63% yield) as a yellow oil. ¹H NMR (400 MHz, DMSO-*d*₆): δ 8.37 (s, 1H), 8.13 (d, *J* = 6.9 Hz, 1H), 8.60 (d, *J* = 7.2 Hz, 1H), 3.80 (s, 3H), 2.18 (s, 3H). LC-MS (ESI, Method 3) t_{R} = 1.15 min, m/z (M+H)⁺= 243.0.

### Step 7. Methyl 6-(cyclopropanecarboxamido)-4-((8-methoxy-7-methylimidazo[1,5-a]pyridin-1-yl)amino)nicotinate (103)

A mixture of **103g** (80 mg, 0.332 mmol), **Int. D** (78 mg, 0.332 mmol), BrettPhos Pd G3 (60.2 mg, 66.37 µmol) and Cs₂CO₃ (216 mg, 0.664 mmol) in dioxane (0.5 mL) was stirred at 160 °C for 6 h under N₂ atmosphere. The reaction mixture was concentrated and purified by flash chromatography (DCM/MeOH = 20/1) to give the crude product. The crude product was purified by *Prep*-HPLC (Method A) to give **103** (5 mg, 4% yield) as a yellow solid. ¹H NMR (400 MHz, MeOD-*d*₄) δ 8.70 (s, 1H), 8.19 (s, 1H), 8.00 (s, 1H), 7.88 (d, *J* = 7.2 Hz, 1H), 6.49 (d, *J* = 7.2 Hz, 1H), 3.95 (s, 3H), 3.72 (s, 3H), 2.19 (s, 3H), 1.83-1.80 (m, 1H), 0.88-0.82 (m, 4H). LC-MS (ESI, Method 2) t_{R} = 0.83 min, m/z (M+H)⁺= 396.1.

### Example 104

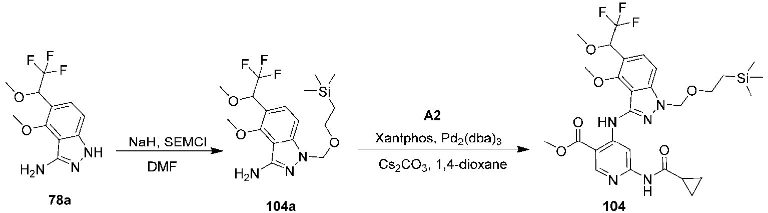

### Step 1. 4-Methoxy-5-(2,2,2-trifluoro-1-methoxyethyl)-1-((2-(trimethylsilyl)ethoxy)methyl)-1H-indazol-3-amine (104a)

To a solution of **78a** (350 mg, 1.27 mmol) in DMF (4 mL) was added NaH (76 mg, 1.91 mmol, 60% purity in mineral oil) slowly at 0 °C and the mixture was stirred for 30 min. Then SEMCl (255 mg, 1.53 mmol) was added to the solution at 0 °C. After stirring at 0 °C for 1 h, the reaction mixture was poured into ice-water (2 mL) and extracted with EtOAc (2 mL*2). The combined organic phase was concentrated and purified by column chromatography on silica gel (PE/EtOAc = 5/1) to give the **104a** (120 mg, 23% yield) as a yellow oil. LC-MS (ESI, Method 3): t_{R} = 1.47 min, m/z (M+H)⁺= 406.3.

### Step 2. Methyl 6-(cyclopropanecarboxamido)-4-((4-methoxy-5-(2,2,2-trifluoro-1-methoxyethyl)-1-((2-(trimethylsilyl)ethoxy)methyl)-1H-indazol-3-yl)amino)nicotinate (104)

A mixture of **104a** (120 mg, 0.295 mmol), **A2** (90.4 mg, 0.355 mmol), Cs₂CO₃ (192.8 mg, 0.592 mmol), Xantphos (34.2 mg, 0.059 mmol) and Pd₂(dba)₃ (54.2 mg, 0.059 mmol) in 1,4-dioxane (1 mL) was stirred at 90 °C under N₂ atmosphere for 12 h. The reaction mixture was concentrated and purified by column chromatography on silica gel (PE/EtOAc = 3/1) to give the **104** (150 mg, 81% yield) as a yellow solid. ¹H NMR (400 MHz, DMSO-*d*₆): δ 11.15 (s, 1H), 11.03 (s, 1H), 9.49 (s, 1H), 8.92 (s, 1H), 7.70 (d, *J =* 8.8 Hz, 1H), 7.59 (d, *J =* 8.8 Hz, 1H), 5.80 (s, 2H), 5.46-5.43 (m, 1H), 4.06 (s, 3H), 4.05 (s, 3H), 3.77 (t, *J =* 8.0 Hz, 2H), 3.46 (s, 3H), 2.17-2.15 (m, 1H), 0.97-0.93 (m, 6H), 0.01 (s, 9H). LC-MS (ESI, Method 2): t_{R} = 1.12 min, m/z (M+H)⁺= 624.2.

### Example 105

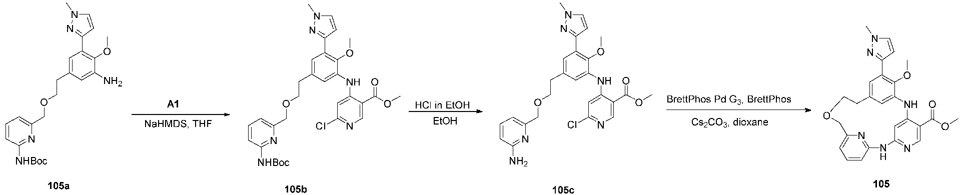

### Step 1. Methyl 4-((5-(2-((6-((tert-butoxycarbonyl)amino)pyridin-2-yl)methoxy)ethyl)-2-methoxy-3-(1-methyl-1H-pyrazol-3-yl)phenyl)amino)-6-chloronicotinate (105b)

To a solution of **105a** (253 mg, 0.56 mmol, WO2022213980A1, page 36-37) and **A1** (210 mg, 1.02 mmol) in dry THF (5 ml) was added NaHMDS (0.8 mL, 1.6 mmol) at 0 °C under N₂ atmosphere. After stirring at 0 °C for 10 minutes, the reaction mixture was stirred at room temperature for 30 minutes. Then it was quenched with MeOH (2 mL) and concentrated. The residue was purified by chromatography column (MeOH:DCM = 6:94) to afford the compound **105b** (138 mg, 40% yield) as a brown solid. LC-MS (ESI, Method 4) t_{R} = 1.07 min, m/z (M+H)⁺ = 623.2.

### Step 2. Methyl 4-((5-(2-((6-aminopyridin-2-yl)methoxy)ethyl)-2-methoxy-3-(1-methyl-1H-pyrazol-3-yl)phenyl)amino)-6-chloronicotinate (105c)

A mixture of **105b** (138 mg, 0.22 mmol) and HCl/EtOH (0.5 mL, 5 mmol) in EtOH (0.5 mL) was stirred at 40 °C for 1 h. Then the solvent was removed, and the residue was purified by reversed-phase chromatograph column (ACN:Water = 1:1, 0.05% NaHCO₃ was contained in water) to afford the compound **105c** (48 mg, 41% yield) as a light brown solid. LC-MS (ESI, Method 4) t_{R} = 0.68 min, m/z (M+H)⁺= 523.2.

### Step 3. Methyl 10-methoxy-11-(1-methyl-1H-pyrazol-3-yl)-16-oxa-2,4,8,22-tetraazatetracyclo[16.3.1.1^{3,7}.1^{9,13}]tetracosa-1(22),3,5,7(24),9(23),10,12,18,20-nonaene-6-carboxylate (105)

To a stirred mixture of **105c** (28 mg, 0.054 mmol), Cs₂CO₃ (42 mg, 0.13 mmol) and BrettPhos (3.2 mg, 0.006 mmol) in dioxane (2 mL) was added BrettPhos Pd G3 (3.8 mg, 0.004 mmol). And the resulting reaction mixture was stirred under N₂ atmosphere at 110 °C for 3.5 h. Then the reaction mixture was cooled down to room temperature and filtered. The filtrate was concentrated and purified by *Prep*-TLC (MeOH:DCM = 1:40) to afford a crude product which was further purified by *Prep*-HPLC (Method E) to afford the compound 105 (2.0 mg, 7.7% yield) as a white solid. LC-MS (ESI, Method 4) t_{R} = 0.83 min, m/z (M+H)⁺= 487.2. ¹H NMR (400 MHz, CDCl₃) δ 10.60 (s, 1H), 8.90 (s, 1H), 8.74 (s, 1H), 7.90 (s, 1H), 7.74 (d, *J =* 2.0 Hz, 1H), 7.55-7.48 (m, 2H), 7.41 (d, *J* = 2.0 Hz, 1H), 6.87 (d, *J* = 2.4 Hz, 1H), 6.78 (d, *J =* 7.2 Hz, 1H), 6.66 (d, *J =* 8.4 Hz, 1H), 4.50 (s, 2H), 3.98 (s, 3H), 3.93 (s, 3H), 3.90-3.86 (m, 2H), 3.68 (s, 3H), 2.96-2.92 (m, 2H).

### Example 106

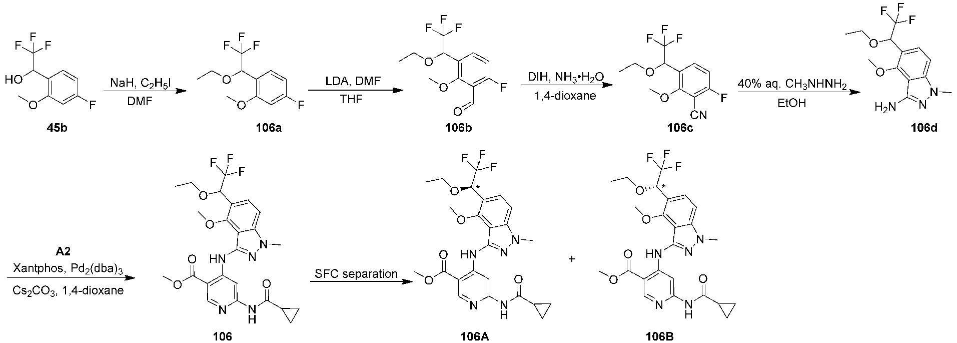

### Step 1. 1-(1-Ethoxy-2,2,2-trifluoroethyl)-4-fluoro-2-methoxybenzene (106a)

To a solution of **45b** (5 g, 22.31 mmol) in THF (50 mL) was slowly added NaH (1.07 g, 26.77 mmol, 60% purity in mineral oil) and the mixture was stirred for 30 min at 0 °C. Then CH₃CH₂I (10.44 g, 66.92 mmol) was added to the reaction at 0 °C and the mixture was stirred at 35 °C for 12 h. The reaction mixture was poured into ice-water (20 mL) and extracted with EtOAc (20 mL*2). The combined organic phase was concentrated and purified by column chromatography (PE/EtOAc = 30/1) to give the **106a** (3.45 g, 61% yield) as a yellow liquid. ¹H NMR (400 MHz, CDCl₃) δ 7.40 (t, *J =* 7.6 Hz, 1H), 6.64-6.59 (m, 1H), 6.53 (dd, *J* = 10.8, 2.4 Hz, 1H), 5.07(q, *J =* 6.4 Hz, 1H), 3.49 (s, 3H), 3.73-3.40 (m, 2H), 1.12 (t, *J* = 7.2 Hz, 3H).

### Step 2. 3-(1-Ethoxy-2,2,2-trifluoroethyl)-6-fluoro-2-methoxybenzaldehyde (106b)

To a solution of **106a** (3.45 g, 13.68 mmol) in THF (30 mL) was added LDA (20.5 mL, 41 mmol, 2 M in THF) dropwise over 30 min at -50 °C. The reaction mixture was stirred for 1 h at the same temperature. Then DMF (3.00 g, 3.18 mL, 41.04 mmol) was added to the solution dropwise at -50 °C. The reaction mixture was stirred for another 1 h at the same temperature. The reaction mixture was quenched with H₂O (20 mL) and extracted with ethyl acetate (20 mL*2). The combined organic phase was washed with brine (30 mL), dried over Na₂SO₄, filtered and concentrated to give **106b** (3 g, 78% yield) as a yellow liquid which was used for the next step directly without further purification.

### Step 3. 5-(1-Ethoxy-2,2,2-trifluoroethyl)-4-methoxy-1-methyl-1H-indazol-3-amine (106c)

To a solution of **106b** (3 g, 10.71 mmol) in NH₃·H₂O (15 mL) and 1,4-dioxane (15 mL) was slowly added 1,3-diiodo-5,5-dimethylimidazolidine-2,4-dione (1.38 g, 10.71 mmol) at 0 °C and the mixture was stirred at 25 °C for 12 h. The reaction mixture was diluted with H₂O (10 mL) and extracted with EtOAc (20 mL*2). The combined organic layer was concentrated to give **106c** (2.1 g, 71% yield) as a yellow liquid which was used for the next step directly without further purification.

### Step 4. 3-(1-Ethoxy-2,2,2-trifluoroethyl)-6-fluoro-2-methoxybenzonitrile (106d)

To a solution of **106c** (500 mg, 1.80 mmol) in EtOH (4 mL) was added 40% *aq.* methylhydrazine (1 mL) and the reaction mixture was stirred at 70°C for 4 h. The reaction mixture was diluted with H₂O (3 mL) and extracted with EtOAc (4 mL*2). The combined organic layer was concentrated and purified by flash chromatography (PE/EtOAc = 3/1) to give **106d** (300 mg, 55% yield) as a yellow solid. ¹H NMR (400 MHz, DMSO-*d*₆) δ 7.26 (d, *J* = 8.8 Hz, 1H), 7.18 (d, *J* = 8.8 Hz, 1H), 5.27-5.23 (m, 1H), 5.21 (s, 2H), 3.87 (s, 3H), 3.73 (s, 3H), 3.54-3.50 (m, 1H), 3.42-3.40 (m, 1H), 1.12 (t, *J* = 7.2 Hz, 3H). LC-MS (ESI, Method 3): t_{R} = 1.25 min, m/z (M+H)⁺= 304.3.

### Step 5. Methyl 6-(cyclopropanecarboxamido)-4-((5-(1-ethoxy-2,2,2-trifluoroethyl)-4-methoxy-1-methyl-1H-indazol-3-yl)amino)nicotinate (106)

A mixture of **106d** (80 mg, 0.264 mmol), **A2** (80 mg, 0.317 mmol), Pd₂(dba)₃ (48.3 mg, 52.7 µmol), Xantphos (30.5 mg, 52.7 µmol) and Cs₂CO₃ (172 mg, 0.527 mmol) in 1,4-dioxane (1 mL) was stirred at 90 °C for 12 h under N₂ atmosphere. The reaction mixture was concentrated and purified by column chromatography (PE/EtOAc = 2/1) to give **106** (100 mg, 73% yield) as a yellow solid. ¹H NMR (400 MHz, DMSO-*d*₆) δ 10.88 (s, 2H), 9.15 (s, 1H), 8.77 (s, 1H), 7.46 (d, *J* = 8.8 Hz, 1H), 7.45 (d, *J =* 9.2Hz, 1H), 5.37-5.35 (m, 1H), 3.98 (s, 3H), 3.92 (s, 3H), 3.88 (s, 3H), 3.60-3.56 (m, 1H), 3.46-3.42 (m, 1H), 2.05-2.01 (m, 1H), 1.14 (t, *J =* 6.8 Hz, 3H), 0.83-0.81 (m, 4H). LC-MS (ESI, Method 2): t_{R} = 1.02min, m/z (M+H)⁺= 522.1.

### Step 6. Methyl (R*)-6-(cyclopropanecarboxamido)-4-((5-(1-ethoxy-2,2,2-trifluoroethyl)-4-methoxy-1-methyl-1H-indazol-3-yl)amino)nicotinate (106A) and Methyl (S*)-6-(cyclopropanecarboxamido)-4-((5-(1-ethoxy-2,2,2-trifluoroethyl)-4-methoxy-1-methyl-1H-indazol-3-yl)amino)nicotinate (106B)

**106** (100 mg, 0.14 mmol) was separated by chiral *prep*-HPLC (Method G, Hex/IPA/DEA = 70/30/0.3) to give **106A** (34.2 mg, 34% yield) as a white solid and **106B** (39.1 mg, 39% yield,) as a white solid.

**106A:** ¹H NMR (400 MHz, DMSO-*d*₆) δ 10.90 (s, 1H), 10.89 (s, 1H), 9.16 (s, 1H), 8.77 (s, 1H), 7.47 (d, *J =* 8.8 Hz, 1H), 7.46 (d, *J =* 8.8 Hz, 1H), 5.37-5.34 (m, 1H), 3.98 (s, 3H), 3.92 (s, 3H), 3.88 (s, 3H), 3.60-3.56 (m, 1H), 3.46-3.42 (m, 1H), 2.05-2.01 (m, 1H), 1.14 (t, *J* = 6.8 Hz, 3H), 0.83-0.81 (m, 4H). LC-MS (ESI, Method 2): t_{R} = 1.01min, m/z (M+H)⁺= 522.1. Chiral HPLC (Method 8, Hex/IPA/DEA = 80/20/0.2): t_{R} = 10.15 min.

**106B:** ¹H NMR (400 MHz, DMSO-*d*₆) δ 10.91 (s, 1H), 10.89 (s, 1H), 9.16 (s, 1H), 8.77 (s, 1H), 7.47 (d, *J* = 8.8 Hz, 1H), 7.46 (d, *J =* 8.8 Hz, 1H), 5.37-5.35 (m, 1H), 3.98 (s, 3H), 3.92 (s, 3H), 3.88 (s, 3H), 3.60-3.56 (m, 1H), 3.46-3.42 (m, 1H), 2.05-2.01 (m, 1H), 1.14 (t, *J* = 6.8 Hz, 3H), 0.83-0.81 (m, 4H). LC-MS (ESI, Method 2): t_{R} = 1.01 min, m/z (M+H)⁺= 522.1. Chiral HPLC (Method 8, Hex/IPA/DEA = 80/20/0.2): t_{R} = 13.81 min.

### Example 107

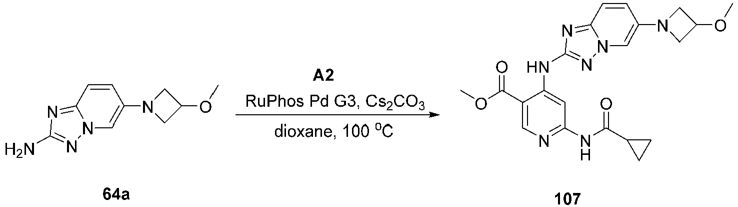

### Step 1. Methyl 6-(cyclopropanecarbonylamino)-4-[[6-(3-methoxyazetidin-1-yl)-[1,2,4]triazolo[1,5-a]pyridin-2-yl]amino]pyridine-3-carboxylate (107)

A mixture of **A2** (50 mg, 0.20 mmol), **64a** (43 mg, 0.20 mmol, WO2021170046A1 P29), RuPhos Pd G3 (16 mg, 0.02 mmol), Cs₂CO₃ (192 mg, 0.60 mmol) in dioxane (5 mL) was stirred at 100 °C for 1 h under N₂ atmosphere. The reaction mixture was diluted with EtOAc (50 mL) and filtered through a pad of celite. The filtrate was washed with water (50 mL) and brine (50 mL), then it was dried over anhydrous Na₂SO₄, filtered and concentrated. The residue was purified by column chromatography (MeOH in DCM is 0-8%) and *prep-HPLC* (Method E) to give **107** (51.3 mg, 60% yield) as a white soild. ¹H NMR (400 MHz, DMSO-*d*₆) *δ* 10.98 (s, 1H), 9.40 (s, 1H), 8.81 (brs, 1H), 8.78 (s, 1H), 7.71 (d, *J* = 2.0 Hz, 1H), 7.46 (d, *J =* 10.0 Hz, 1H), 6.90 (dd, *J =* 9.6 Hz, 2.4 Hz, 1H), 4.41-4.33 (m, 1H), 4.14 (dd, *J* = 7.2 Hz, 4.0 Hz, 2H), 3.95 (s, 3H), 3.73 (dd, *J =* 7.2 Hz, 4.0 Hz, 2H), 3.36 (s, 3H), 1.67-1.58 (m, 1H), 1.22-1.16 (m, 2H), 0.96-0.88 (m, 2H). LC-MS (Method 4) t_{R} = 5.11 min, m/z (M+H)⁺= 438.3.

### Example 108

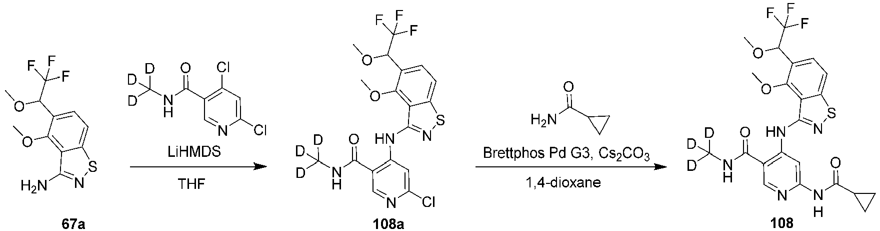

### Step 1. 6-Chloro-4-((4-methoxy-5-(2,2,2-trifluoro-1-methoxyethyl)benzo[d]isothiazol-3-yl)amino)-N-(methyl-d₃)nicotinamide (108b)

To a solution of **67a** (80 mg, 0.273 mmol) and 4,6-dichloro-*N*-(methyl-*d*₃)nicotinamide (68.3 mg, 0.328 mmol) in THF (0.3 mL) was added LiHMDS (1.1 mL, 1.1 mmol, 1 M in THF) at -40 °C. After stirring for 4 h at -40 °C, the reaction was quenched with H₂O (2 mL) and organic solvent was evaporated. The formed solid was filtered and purified by flash chromatography (DCM/MeOH = 20/1) to give **108a** (20 mg, 16% yield) as a white solid. LC-MS (ESI, Method 3): t_{R} = 1.53 min, m/z (M+H)⁺= 464.2.

### Step 2. 6-(Cyclopropanecarboxamido)-4-((4-methoxy-5-(2,2,2-trifluoro-1-methoxyethyl)benzo[d]isothiazol-3-yl)amino)-N-(methyl-d₃)nicotinamide (108)

A mixture of **108a** (20 mg, 0.043 mmol), cyclopropanecarboxamide (18.3 mg, 0.216 mmol), Brettphos Pd G3 (7.8 mg, 0.0086 mmol) and Cs₂CO₃ (28.1 mg, 0.086 mmol) in 1,4-dioxane (0.5 mL) was stirred at 90 °C for 5 h under N₂ atmosphere. The reaction mixture was concentrated and purified by *Prep-HPLC* (Method A) to give **108** (12 mg, 54% yield) as a white solid. ¹H NMR (400 MHz, CD₃OD) δ 9.78 (s, 1H), 8.52 (s, 1H), 7.84 (d, *J=* 8.4 Hz, 1H), 7.00 (d, *J* = 8.4 Hz, 1H), 5.37-5.32 (m, 1H), 4.03 (s, 3H), 3.45 (s, 3H), 1.96-1.91 (m, 1H), 1.07-1.03 (m, 2H), 0.95-0.91 (m, 2H). LC-MS (ESI, Method 2): t_{R} = 0.95 min, m/z (M+H)⁺= 513.1.

### Example 109

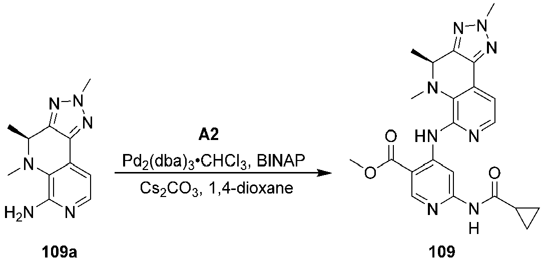

### Step 1. Methyl (S)-6-(cyclopropanecarboxamido)-4-((2,4,5-trimethyl-4,5-dihydro-2H-[1,2,3]triazolo[4,5-c][1,7]naphthyridin-6-yl)amino)nicotinate (109)

A mixture of **109a** (30 mg, 0.130 mmol, WO 2022175747, P130), **A2** (49.8 mg, 0.195 mmol), Cs₂CO₃ (127.4 mg, 0.391 mmol), Pd₂(dba)₃·CHCl₃ (13.3 mg, 13.03 µmol) and BINAP (8.1 mg, 13.03 µmol) in 1,4-dioxane (0.5 mL) was stirred at 110 °C under N₂ atmosphere for 5 h. The reaction mixture was concentrated and purified by column chromatography (PE/EtOAc = 1/1) to give the crude product. The crude product was purified by *Prep-HPLC* (Method A) to give **109** (17.2 mg, 29% yield) as a white solid. ¹H NMR (400 MHz, DMSO-*d*₆) δ 11.27 (s, 1H), 10.89 (s, 1H), 8.67 (s, 1H), 8.79 (s, 1H), 7.16 (d, *J =* 4.8 Hz, 1H), 7.28 (d, *J =* 4.8 Hz, 1H), 4.59-4.54 (m, 1H), 4.24 (s, 3H), 3.93 (s, 3H), 2.67 (s, 3H), 2.06-2.03 (m, 1H), 1.16 (d, *J* = 7.2 Hz, 3H), 0.86-0.82 (m, 4H). LC-MS (ESI, Method 2): t_{R} = 1.17 min, m/z (M+H)⁺= 449.1. Chiral HPLC (Method 12): t_{R} = 9.06 min.

### Example 110

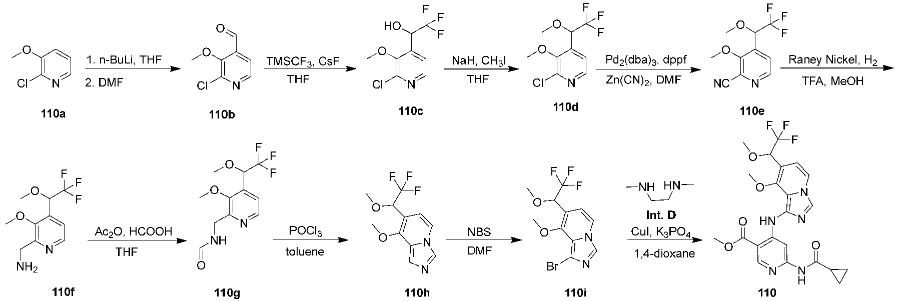

### Step 1. 2-Chloro-3-methoxyisonicotinaldehyde (110b)

To a solution of **110a** (15 g, 104.48 mmol) in THF (100 mL) was added n-BuLi (46 mL, 114.93 mmol, 2.5 M in THF) dropwise over 30 min at -60 °C. The reaction mixture was stirred for 1 h at the same temperature. Then DMF (11.45 g, 156.72 mmol) was added to the solution dropwise at -60 °C. The reaction was stirred for another 2 h at the same temperature. The reaction mixture was quenched with H₂O (30 mL) and extracted with EtOAc (30 mL*2). The combined organic layer was concentrated and purified by flash chromatography (PE/EtOAc = 10/1) to give **110b** (14 g, 78% yield) as a yellow solid. LC-MS (ESI, Method 3): t_{R} = 0.92 min, m/z (M+H)⁺= 172.0.

### Step 2. 1-(2-Chloro-3-methoxypyridin-4-yl)-2,2,2-trifluoroethan-1-ol (110c)

To a solution of **110b** (18 g, 62.94 mmol) and TMSCF₃ (17.88 g, 125.89 mmol) in THF (200 mL) was added CsF (191.22 mg, 1.26 mmol) at 0 °C. The mixture was stirred at 30 °C for 1 h. After 1 M HCl (20 mL) was added to the reaction, the mixture was stirred at 30 °C for 2 h. The pH was adjusted to 8 with *sat.* NaHCO₃ and then it was extracted with EtOAc (30 mL*2). The combined organic layer was concentrated and purified by flash chromatography on silica gel (PE/EtOAc = 4/1) to give **110c** (12.5 g, 82% yield) as a yellow solid. ¹H NMR (400 MHz, DMSO-*d*₆) δ 8.30 (d, *J* = 5.2 Hz, 1H), 7.58 (d, *J* = 5.2 Hz, 1H), 7.24 (d, *J =* 6.0 Hz, 1H), 5.48-5.44 (m, 1H), 3.89 (s, 3H).

### Step 3. 2-Chloro-3-methoxy-4-(2,2,2-trifluoro-1-methoxyethyl)pyridine (110d)

To a solution of **110c** (6 g, 24.83 mmol) and CH₃I (3.88 g, 27.32 mmol) in THF (60 mL) was slowly added NaH (1.19 g, 29.80 mmol, 60% in mineral oil) at 0°C. After stirring at 25°C for 1 h, the reaction mixture was poured into ice-water (20 mL) and extracted with EtOAc (20 mL*2). The combined organic phase was washed with brine (15 mL), dried with Na₂SO₄, filtered and concentrated. The residue was purified by flash chromatography (PE/EtOAc = 10/1) to give **110d** (4.20 g, 66% yield) as a yellow solid. ¹H NMR (400 MHz, CDCl₃) δ 8.26 (d, *J* = 4.8 Hz, 1H), 7.43 (d, *J* = 4.8 Hz, 1H), 5.10-5.06 (m, 1H), 3.98 (s, 3H), 3.45 (s, 3H).

### Step 4. 3-Methoxy-4-(2,2,2-trifluoro-1-methoxyethyl)picolinonitrile (110e)

A mixture of **110d** (3.80 g, 14.87 mmol), Zn(CN)₂ (3.48 g, 29.73 mmol), dppf (824.1 mg, 1.49 mmol) and Pd₂(dba)₃ (680.6 mg, 0.74 mmol) in DMF (30 mL) was stirred at 85°C under N₂ atmosphere for 12 h. The reaction mixture was concentrated and purified by flash chromatography (PE/EA=10/1) to give 110e (3.40 g, 92% yield) as brown liquid. LC-MS (ESI, Method 3): t_{R} = 1.21 min, m/z (M+H)⁺= 247.1.

### Step 5. (3-Methoxy-4-(2,2,2-trifluoro-1-methoxyethyl)pyridin-2-yl)methanamine (110f)

A mixture of 110e (1.5 g, 6.09 mmol), Raney Nickel (5 g, 6.09 mmol) and TFA (694.7 mg, 6.09 mmol) in MeOH (15 mL) was stirred at 60 °C for 16 h under H₂ atmosphere (1 atm). The mixture was filtered and concentrated. And the residue was purified by *Prep*-HPLC (Method A) to give 110f (600 mg, 39% yield) as a yellow solid. LC-MS (ESI, Method 3): t_{R} = 0.88 min, m/z (M+H)⁺= 251.1.

### Step 6. N-((3-methoxy-4-(2,2,2-trifluoro-1-methoxyethyl)pyridin-2-yl)methyl)formamide (110g)

A mixture of HCOOH (5 mL) and Ac₂O (6 mL) was stirred at 50°C for 1 h. And a solution of **110f** (600.0 mg, 2.40 mmol) in THF (4 mL) was added into the reaction mixture at 0°C. After stirring at 0°C for 2 h, the reaction mixture was diluted with ice-water (10 mL) and extracted with EtOAc (20 mL*2). The combined organic phase was washed with brine (5 mL), concentrated and purified by flash chromatography (DCM/MeOH=20/1) to give **110g** (520 mg, 78% yield) as a yellow oil. LC-MS (ESI, Method 3): t_{R} = 1.02 min, m/z (M+H)⁺ = 279.3.

### Step 7. 8-Methoxy-7-(2,2,2-trifluoro-1-methoxyethyl)imidazo[1,5-a]pyridine (110h)

To a solution of **110g** (470 mg, 1.69 mmol) in toluene (4 mL) was added POCl₃ (1.30 g, 8.45 mmol) at 0°C and the reaction mixture was stirred at 100°C for 2 h. The reaction mixture was concentrated and basified with Na₂CO₃ solution (10 mL) to adjust to pH > 8. Then the mixture was extracted with EtOAc (15 mL*2). The combined organic layer was concentrated and purified by flash chromatography (DCM/MeOH = 20/1) to give **110h** (420.0 mg, 1.61 mmol) as a yellow solid. LC-MS (ESI, Method 3): t_{R} = 1.00 min, m/z (M+H)⁺= 261.3.

### Step 8. 1-Bromo-8-methoxy-7-(2,2,2-trifluoro-1-methoxyethyl)imidazo[1,5-a]pyridine (110i)

To a solution of **110h** (300.0 mg, 1.15 mmol) in DMF (5 mL) was added NBS (174.4 mg, 0.979 mmol) at -60 °C. The mixture was stirred at -60 °C for 2 h. The mixture was diluted with H₂O (5 mL) and extracted with EtOAc (10 mL*2). The combined organic layer was concentrated and purified by flash chromatography (PE/EtOAc = 5/1) to give **110i** (200 mg, 51% yield) as a white solid. LC-MS (ESI, Method 3): t_{R} = 1.03 min, m/z (M+H)⁺= 341.0.

### Step 9. Methyl 6-(cyclopropanecarboxamido)-4-((8-methoxy-7-(2,2,2-trifluoro-1-methoxyethyl)imidazo[1,5-a]pyridin-1-yl)amino)nicotinate (110)

A mixture of **110i** (70.0 mg, 0.206 mmol), **Int. D** (72.8 mg, 309.64 mmol), *N,N-*dimethylethane-1,2-diamine (9.1 mg, 0.103 mmol), CuI (19.7 mg, 0.103 mmol) and K₃PO₄ (87.6 mg, 0.413 mmol) in 1,4-dioxane (0.7 mL) was stirred at 100 °C for 12 h under N₂ atmosphere. The reaction was cooled to room temperature and concentrated. The residue was purified by flash chromatography (DCM/MeOH = 20/1) and prep-TLC (DCM/MeOH = 20/1) to give the crude product which was purified by *Prep-HPLC* (Method A) to give **110** (2.5 mg, 2% yield) as a white solid. ¹H NMR (400 MHz, DMSO-d₆) δ 10.82 (s, 1H), 10.29 (s, 1H), 8.70 (s, 1H), 8.52 (s, 1H), 8.46 (s, 1H), 8.13 (d, *J* = 7.6 Hz, 1H), 6.55 (d, *J* = 7.2 Hz, 1H), 5.20-5.18 (m, 1H), 3.89 (s, 3H), 3.77 (s, 3H), 3.29 (s, 3H), 2.02-1.94 (m, 1H), 0.81-0.70 (m, 4H). LC-MS (ESI, Method 2): t_{R} = 0.913 min, m/z (M+H)⁺ = 494.1.

### Example 111

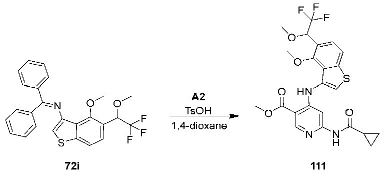

### Methyl 6-(cyclopropanecarboxamido)-4-((4-methoxy-5-(2,2,2-trifluoro-1-methoxyethyl)benzo[b]thiophen-3-yl)amino)nicotinate (111)

A mixture of **72i** (50.0 mg, 0.110 mmol), **A2** (28.0 mg, 0.111 mmol) and TsOH (1.9 mg, 0.011 mmol) in 1,4-dioxane (0.5 mL) was stirred at 90 °C for 16 h. The mixture was concentrated and purified by *Prep-HPLC* (Method A) to afford **111** (4.2 mg, 7.5% yield) as a white solid. ¹H NMR (400 MHz, DMSO-*d*₆) δ 10.93 (s, 1H), 10.37 (s, 1H), 8.74 (s, 1H), 8.17 (s, 1H), 7.93 (d, *J =* 8.0 Hz, 1H), 7.67 (s, 1H), 7.45 (d, *J =* 8.4 Hz, 1H), 5.34-5.32 (m, 1H), 3.91 (s, 3H), 3.69 (s, 3H), 3.34 (s, 3H), 2.00-1.97 (m, 1H), 0.79-0.76 (m, 4H). LC-MS (ESI, Method 2): t_{R} = 1.00 min, m/z (M+H)⁺= 510.1.

### Example 112

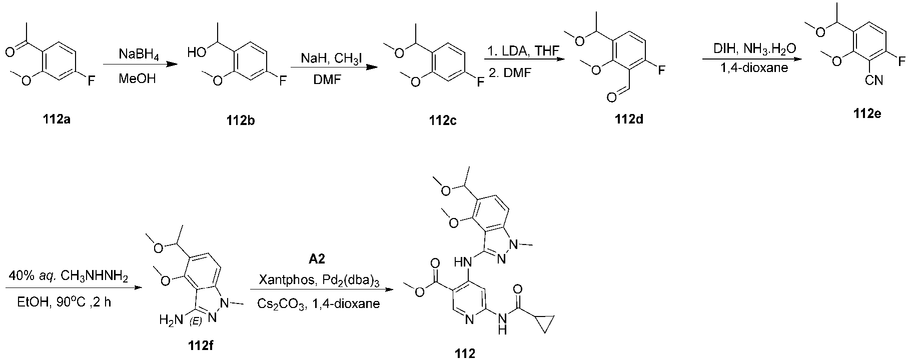

### Step 1. 1-(4-Fluoro-2-methoxyphenyl)ethan-1-ol (112b)

To a solution of **112a** (4 g, 23.79 mmol) in MeOH (20 mL) was added NaBH₄ (1.35 g, 35.68 mmol) at 0 °C. The reaction mixture was warmed to room temperature and stirred for another 2 h. The mixture was concentrated and purified by flash chromatography (PE/EA = 3/1) to give **112b** (4 g, 99% yield) as a yellow oil. ¹H NMR (400 MHz, DMSO-*d*₆) δ 7.43-7.39 (m, 1H), 6.84-6.80 (m, 1H), 6.75-6.70 (m, 1H), 5.01 (d, *J* = 4.4 Hz, 1H), 4.96-4.90 (m, 1H), 3.78 (s, 3H), 1.23 (d, *J =* 6.4 Hz, 3H).

### Step 2. 4-Fluoro-2-methoxy-1-(1-methoxyethyl)benzene (112c)

To a solution of **112b** (5 g, 29.38 mmol) in DMF (20 mL) was added NaH (846.2 mg, 35.26 mmol, 60% dispersion in mineral oil) at 0 °C and the mixture was stirred for 30 min. And then the reaction mixture was added CH₃I (6.26 g, 44.10 mmol) and stirred for 3 h at 0 °C. It was quenched with ice-water (50 mL) and extracted with EtOAc (20 mL*3). The combined organic layer was concentrated and purified by flash chromatography (PE/EtOAc = 5/1) to give **112c** (3.7 g, 68% yield) as a yellow oil. ¹H NMR (400 MHz, CDCl₃) δ 7.36-7.32 (m, 1H), 6.71-6.67 (m, 1H), 6.64-6.59 (m, 1H), 4.70 (q, *J =* 6.8 Hz, 1H), 3.83 (s, 3H), 3.26 (s, 3H), 1.38 (d, *J =* 6.4 Hz, 3H).

### Step 3. 6-Fluoro-2-methoxy-3-(1-methoxyethyl)benzaldehyde (112d)

To a solution of **112c** (700 mg, 3.80 mmol) in THF (10 mL) was added LDA (6 mL 12 mmol, 2 M in THF) dropwise over 30 min at -60 °C. The reaction mixture was stirred for 1 h at the same temperature. Then DMF (833 mg, 11.40 mmol, 882.67 µL) was added to the solution dropwise at -60 °C. The reaction mixture was stirred for another 1 h at the same temperature. The mixture was quenched with H₂O (20 mL) and extracted with EtOAc (20 mL *2). The combined organic layer was washed with brine (30 mL), dried over Na₂SO₄, filtered and concentrated to give **112d** (600 mg, 74% yield) as a yellow liquid which was used for the next step directly without further purification.

### Step 4. 6-Fluoro-2-methoxy-3-(1-methoxyethyl)benzonitrile (112e)

To a solution of **112d** (600 mg, 2.83 mmol) in dioxane (5 mL) and NH₃.H₂O (5 mL) was slowly added 1,3-diiodo-5,5-dimethylimidazolidine-2,4-dione (1.07 g, 2.83 mmol) at 0 °C and the mixture was stirred at 25 °C for 12 h. The reaction mixture was diluted with H₂O (10 mL) and extracted with EtOAc (20 mL*2). The combined organic phase was concentrated and purified by column chromatography (PE/EtOAc = 10/1) to give the crude **112e** (500 mg, 85% yield) as a yellow liquid. The crude product was used for the next step directly without further purification.

### Step 5. 4-Methoxy-5-(1-methoxyethyl)-1-methyl-1H-indazol-3-amine (112f)

A solution of **112e** (380 mg, 1.82 mmol), *aq.* Methylhydrazine (1 mL, 40% in water) and EtOH (3 mL) in a sealed tube of 10 mL was stirred at 90 °C for 2 h. After cooling to room temperature, the mixture was concentrated and purified by column chromatography on silica gel (PE/EtOAc = 3/1 to 1/1) to give **112f** (30 mg, 7% yield) as a yellow solid. LC-MS (ESI, Method 3): t_{R} = 1.01 min, m/z (M+H)⁺= 236.0.

### Step 6. Methyl 6-(cyclopropanecarboxamido)-4-((4-methoxy-5-(1-methoxyethyl)-1-methyl-1H-indazol-3-yl)amino)nicotinate (112)

A mixture of **112f** (20 mg, 85.00 µmol), **A2** (32.4 mg, 127.51 µmol), Pd₂(dba)₃ (7.8 mg, 8.5 µmol), Xantphos (9.8 mg, 17.00 µmol), Cs₂CO₃ (55.4 mg, 170.01 µmol) in 1,4-dioxane (0.5 mL) was stirred at 90°C for 8 h under N₂ atmosphere. The solvent was removed after the mixture is cooled to room temperature. And the residue was purified by column chromatography (DCM/MeOH= 20/1) to give the crude product which was purified by *Prep-HPLC* (Method A) to give **112** (30 mg, 78% yield) as a white solid. ¹H NMR (400 MHz, DMSO-*d*₆) δ 10.95 (s, 1H), 10.88 (s, 1H), 9.24 (s, 1H), 8.77 (s, 1H), 7.39-7.38 (m, 2H), 4.78 (q, *J =* 6.4 Hz, 1H), 3.95 (s, 3H), 3.92 (s, 3H), 3.86 (s, 3H), 3.08 (s, 3H), 2.05-2.01 (m, 1H), 1.40 (d, *J =* 6.4 Hz, 3H), 0.85-0.79 (m, 4H). LC-MS (ESI, Method 2): t_{R} = 1.19 min, m/z (M+H)⁺ = 454.1.

### Example 113

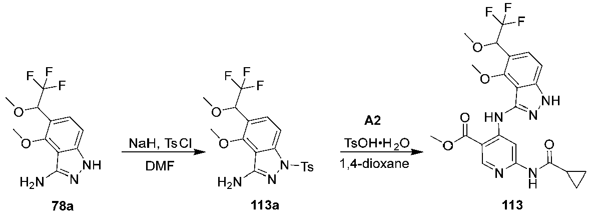

### Step 1. 4-Methoxy-1-tosyl-5-(2,2,2-trifluoro-1-methoxyethyl)-1H-indazol-3-amine (113a)

To a solution of **78a** (190 mg, 0.69 mmol) in DMF (2 mL) was slowly added NaH (42 mg, 1.04 mmol, 60% in mineral oil) at 0°C. After stirring at 0°C for 30 min, the reaction was added with tosyl chloride (71.6 mg, 0.828 mmol). The mixture was stirred at 0°C for 1 h and poured into ice-water (2 mL). The mixture was extracted with EtOAc (2 mL*2). The combined organic phase was concentrated and purified by flash chromatography (PE/EtOAc = 5/1) to give **113a** (180 mg, 61% yield) as a yellow solid. LC-MS (ESI, Method 3): t_{R} = 1.36 min, m/z (M+H)⁺= 430.3.

### Step 2. Methyl 6-(cyclopropanecarboxamido)-4-((4-methoxy-5-(2,2,2-trifluoro-1-methoxyethyl)-1H-indazol-3-yl)amino)nicotinate (113)

To a solution of **113a** (130 mg, 0.303 mmol) and **A2** (100 mg, 0.394 mmol) in 1,4-dioxane (1 mL) was added TsOH·H₂O (5.8 mg, 30.27 µmol) and the mixture was stirred at 110 °C for 96 h. The reaction mixture was concentrated and purified by flash chromatography (DCM/MeOH = 10/1) to give **113** (10 mg, 7% yield) as a yellow solid. ¹H NMR (400 MHz, DMSO-*d*₆) δ 13.00 (s, 1H), 10.95 (s, 1H), 10.93 (s, 1H), 9.27 (s, 1H), 8.78 (s, 1H), 7.38 (d, *J =* 8.8 Hz, 1H), 7.33 (d, *J =* 8.8 Hz, 1H), 5.32-5.27 (m, 1H), 3.93 (s, 3H), 3.90 (s, 3H), 3.32 (s, 3H), 2.07-2.01 (m, 1H), 0.88-0.81 (m, 4H). LC-MS (ESI, Method 2): t_{R} = 1.24 min, m/z (M+H)⁺= 494.1.

### Example 114

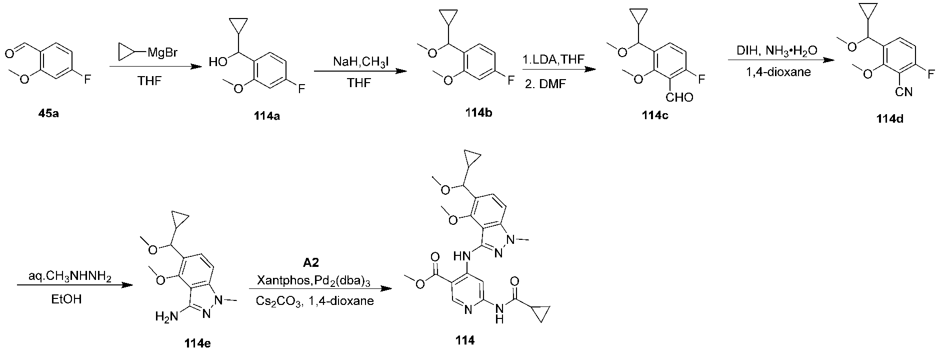

### Step 1. Cyclopropyl(4-fluoro-2-methoxyphenyl)methanol (114a)

To a solution of **45a** (1 g, 6.49 mmol) in THF (10 mL) was slowly added cyclopropylmagnesium bromide (19.5 mL, 9.7 mmol, 0.5 M in THF) at -30°C and the mixture was stirred at -30°C for 1 h. After the mixture was stirred at 25 °C for 1 h. it was diluted with H₂O (10 mL) and extracted with EtOAc (10 mL*2). The combined organic layer was concentrated and purified by flash chromatography (PE/EtOAc = 40/1) to afford **114a** (900 mg, 71% yield) as a yellow liquid. ¹H NMR (400 MHz, CDCl₃) δ 7.32-7.28 (m, 1H), 6.61-6.52 (m, 2H), 4.13 (d, *J=* 8.4 Hz, 1H), 3.76 (s, 3H), 2.54 (brs, 1H), 1.24-1.29 (m, 1H), 0.58-0.54 (m, 1H), 0.43-0.36 (m, 2H), 0.32-0.25 (m, 1H).

### Step 2. 1-(Cyclopropyl(methoxy)methyl)-4-fluoro-2-methoxybenzene (114b)

To a solution of **114a** (900 mg, 4.59 mmol) in THF (9 mL) was added NaH (220 mg, 5.50 mmol, 60% in mineral oil) at 0 °C. After stirring at 0 °C for 30 min, it was added with CH₃I (1.95 g, 13.76 mmol) and stirred at 0 °C for 1 h. And then the solution was stirred at 35 °C for 12 h. it was poured into ice-water (5 mL) and extracted with EtOAc (5 mL*2). The combined organic phase was concentrated and purified by column chromatography (PE/EtOAc = 30/1) to give **114b** (800 mg, 83% yield) as a yellow liquid. ¹H NMR (400 MHz, DMSO-*d*₆) δ 7.27 (dd, *J* = 8.4, 7.2 Hz, 1H), 6.63-6.59 (m, 1H), 6.53 (dd, *J =* 11.2, 2.4 Hz, 1H), 4.03 (d, *J =* 7.2 Hz, 1H), 3.73 (s, 3H), 3.15 (s, 3H), 1.08-1.05 (m, 1H), 0.54-0.46 (m, 1H), 0.32-0.25 (m, 3H).

### Step 3. 3-(Cyclopropyl(methoxy)methyl)-6-fluoro-2-methoxybenzaldehyde (114c)

To a solution of **114b** (800 mg, 4.08 mmol) in THF (8 mL) was dropwise added LDA (6.2 mL, 12.4 mmol, 2 M in THF) at -50 °C. The mixture was stirred at -50 °C for 1 h. Then DMF (0.95 mL, 12.23 mmol) was added to the mixture at -50 °C and the mixture was stirred at - 50 °C for 1 h. The reaction was quenched with *sat.* NH₄Cl (10 mL) and extracted with EtOAc (10 mL*3). The combined organic layer was concentrated to give **114c** (700 mg, crude) as a yellow oil which was used for the next step directly without further purification.

### Step 4. 3-(Cyclopropyl(methoxy)methyl)-6-fluoro-2-methoxybenzonitrile (114d)

To a solution of **114c** (700 mg, 3.12 mmol) in NH₃·H₂O (4 mL) and 1,4-dioxane (4 mL) was added 1,3-diiodo-5,5-dimethylimidazolidine-2,4-dione (1.19 g, 3.12 mmol) at ice-bath. Then the mixture was stirred at 25 °C for 12 h. The mixture was diluted with water (5 mL) and extracted with EtOAc (5 mL*2). The combined organic layer was concentrated to give **114d** (500 mg, crude) as a yellow liquid which was used for the next step directly without further purification.

### Step 5. 5-(Cyclopropyl(methoxy)methyl)-4-methoxy-1-methyl-1H-indazol-3-amine (114e)

A mixture of **114d** (500 mg, 2.13 mmol) and 40% *aq.* methylhydrazine (2 mL) in EtOH (4 mL) was stirred at 70 °C for 4 h. After cooling to rom temperature, the mixture was diluted with water (2 mL) and extracted with EtOAc (4 mL*2). The combined organic layer was concentrated and purified by column chromatography (PE/EtOAc = 3/1) to give **114e** (20 mg, 4.6% yield) as a yellow solid. LC-MS (ESI, Method 3): t_{R} = 1.13min, m/z (M+H)⁺= 262.2. Step **6. Methyl 6-(cyclopropanecarboxamido)-4-((5-(cyclopropyl(methoxy)methyl)-4-methoxy-1-methyl-1*H*-indazol-3-yl)amino)nicotinate (114)**

A mixture of **114e** (20 mg, 0.0765 mmol), **A2** (23.4 mg, 0.918 mmol), Pd₂(dba)₃ (14.1 mg, 0.0153 mmol), Xantphos (8.9 mg, 0.0153 mmol) and Cs₂CO₃ (49.9 mg, 0.153 mmol) in 1,4-dioxane (0.2 mL) was stirred at 90 °C for 12 h under N₂ atmosphere. The reaction mixture was concentrated and purified by column chromatography (DCM/MeOH = 20/1) to give **114** (5 mg, 14% yield) as a white solid. ¹H NMR (400 MHz, DMSO-*d*₆) δ 10.89 (s, 1H), 10.86 (s, 1H), 9.21 (s, 1H), 8.76 (s, 1H), 7.45 (d, *J* = 8.8 Hz, 1H), 7.37 (d, *J* = 8.8 Hz, 1H), 4.09 (d, *J* = 7.6 Hz, 1H), 3.96 (s, 3H), 3.92 (s, 3H), 3.84 (s, 3H), 3.14 (s, 3H), 2.06-2.01 (m, 1H), 1.23-1.15 (m, 1H), 0.86-0.81 (m, 4H), 0.61-0.48 (m, 2H), 0.36-0.34 (m, 1H), 0.33-0.31 (m, 1H). LC-MS (ESI, Method 2): t_{R} = 1.30 min, m/z (M+H)⁺= 480.2.

### Example 115

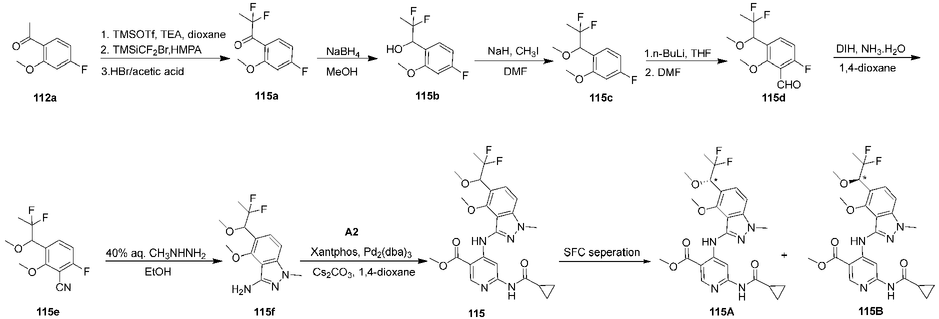

### Step 1. 2,2-Difluoro-1-(4-fluoro-2-methoxyphenyl)propan-1-one (115a)

To a solution of **112a** (1 g, 5.95 mmol) and TEA (842 mg, 8.33 mmol) in 1,4-dioxane (10 mL) was added TMSOTf (1.59 g, 7.14 mmol) at 0 °C. Then the cooling bath was removed, and the solution was stirred at 20 °C for 40 min. The mixture was cooled to 10 °C, it was added TMSCF₂Br (4.23 g, 20.81 mmol) followed by HMPA (3.20 g, 17.84 mmol). The mixture was stirred at 20 °C for 2 h and 30 °C for 1 h. The reaction mixture became cloudy firstly, and finally became a two-phase mixture. The mixture was evaporated under reduced pressure. Then the residue was added HBr in acetic acid (16.04 g, 65.41 mmol, 33% purity) followed by H₂O (1.18g, 65.41 mmol). The reaction mixture was stirred at 80 °C for 1 h. After cooling to room temperature, the mixture was quenched with saturated aqueous Na₂CO₃ (65 mL) and diluted with water (15 mL). The solid was filtered off and the filtrate was extracted with EtOAc (80 mL*2). The combined organic layer was concentrated and purified by flash chromatography (PE/EtOAc = 19/1) to give **115a** (1.09 g, 84% yield) as a yellow oil. ¹H NMR (400 MHz, CDCl₃) δ 7.54-7.50 (m, 1H), 6.62-6.55 (m, 2H), 3.70 (s, 3H), 1.73 (t, *J =* 19.2 Hz, 3H).

### Step 2. 2,2-Difluoro-1-(4-fluoro-2-methoxyphenyl)propan-1-ol (115b)

To a solution of **115a** (1.09 g, 5.00 mmol) in MeOH (11 mL) was added NaBH₄ (378.00 mg, 9.99 mmol) at 0 °C. After stirring at 20 °C for 2 h, the reaction was quenched with water (10 mL) and extracted with EtOAc (15 mL*3). The combined organic layer was dried over Na₂SO₄ and filtered. The filtrate was concentrated to give **115b** (1.09 g, 99% yield) as a yellow oil. ¹H NMR (400 MHz, CDCl₃) δ 7.45-7.41 (m, 1H), 6.75-6.64 (m, 2H), 5.20-5.13 (m, 1H), 3.85 (s, 3H), 3.01 (d, *J* = 6.0 Hz, 1H), 1.59 (t, *J =* 18.8 Hz, 3H).

### Step 3. 1-(2,2-Difluoro-1-methoxypropyl)-4-fluoro-2-methoxybenzene (115c)

To a solution of **115b** (485 mg, 2.20 mmol) in DMF (5 mL) was added NaH (106 mg, 2.64 mmol, 60% in mineral oil) at 0 °C. The mixture was stirred at 0 °C for 30 min. Then CH₃I (469 mg, 3.30 mmol) was added to the mixture and the mixture was stirred at 0 °C for 15 min. The mixture was poured into ice-water (5 mL) and extracted with EtOAc (15 mL*2). The combined organic layer was concentrated and purified by flash chromatography (PE/EtOAc = 10/1) to give **115c** (410 mg, 79% yield) as a yellow oil. ¹H NMR (400 MHz, CDCl₃) δ 7.42-7.38 (m, 1H), 6.73-6.88 (m, 1H), 6.65-6.61 (m, 1H), 4.85 (t, *J =* 10.0 Hz, 1H), 3.82 (s, 3H), 3.32 (s, 3H), 1.56 (t, *J* = 19.2 Hz, 3H).

### Step 4. 3-(2,2-Difluoro-1-methoxypropyl)-6-fluoro-2-methoxybenzaldehyde (115d)

To a solution of **115c** (95 mg, 0.406 mmol) in THF (1 mL) was added n-BuLi (0.51 mL, 0.81 mmol, 1.6 M in hexanes) at -50 ° C. Then the mixture was stirred at -50° C for 30 min. DMF (89 mg, 1.22 mmol) was added to the mixture and the mixture was stirred at -50 °C for 1 h. The mixture was quenched with water (6 mL) and extracted with EtOAc (10 mL*2). The combine organic layer was concentrated to give **115d** (100 mg, crude) as a yellow oil which was used for the next step directly without further purification.

### Step 5. 3-(2,2-Difluoro-1-methoxypropyl)-6-fluoro-2-methoxybenzonitrile (115e)

To a solution of **115d** (100 mg, 0.381 mmol) in 1,4-dioxane (0.6 mL) and NH₃·H₂O (0.6 mL) was added 1,3-diiodo-5,5-dimethylimidazolidine-2,4-dione (144.9 mg, 0.381 mmol) at ice-bath. Then the mixture was stirred at 20 °C for 16 h. The mixture was diluted with water (5 mL) and extracted with EtOAc (8 mL*2). The combined organic layer was concentrated to give **115e** (98 mg, crude) as a yellow oil which was used for the next step directly without further purification.

### Step 6. 5-(2,2-Difluoro-1-methoxypropyl)-4-methoxy-1-methyl-1H-indazol-3-amine (115f)

A mixture of **115e** (98 mg, 0.378 mmol) and *aq.* methylhydrazine (1 mL, 40% in water) in EtOH (1 mL) was stirred at 70 °C for 4 h. After cooling to room temperature, the mixture was diluted with H₂O (5 mL) and extracted with EtOAc (8 mL*2). The combined organic layer was concentrated and purified by flash chromatography on silica gel (DCM/EtOAc = 5/1) to give **115f** (17 mg, 16% yield) as a yellow solid. LC-MS (ESI, Method 3): t_{R} = 1.18 min, m/z (M+H)⁺ = 285.8.

### Step 7. Methyl 6-(cyclopropanecarboxamido)-4-((5-(2,2-difluoro-1-methoxypropyl)-4-methoxy-1-methyl-1H-indazol-3-yl)amino)nicotinate (115)

A mixture of **115f** (17 mg, 59.60 µmol), **A2** (16.7 mg, 65.55 µmol), Pd₂(dba)₃ (10.9 mg, 11.92 µmol), Cs₂CO₃ (38.8 mg, 0.119 mmol) and Xantphos (6.9 mg, 11.92 µmol) in 1,4-dioxane (0.5 mL) was stirred at 90 °C for 4 h under N₂ atmosphere in a sealed tube. The mixture was concentrated and purified by flash chromatography (DCM/MeOH = 19/1) to give the crude product. The crude product was purified by *Prep-HPLC* (Method A) to give **115** (9.8 mg, 33% yield) as a white solid. ¹H NMR (400 MHz, DMSO-*d*₆) δ 10.89 (s, 1H), 10.88 (s, 1H), 9.17 (s, 1H), 8.77 (s, 1H), 7.42 (d, *J =* 9.6 Hz, 1H), 7.40 (d, *J = 9.6* Hz, 1H), 4.93-4.87 (m, 1H), 3.97 (s, 3H), 3.92 (s, 3H), 3.86 (s, 3H), 3.23 (s, 3H), 2.07-2.01(m, 1H), 1.66 (t, *J =* 19.2 Hz, 3H), 0.86-0.80 (m, 4H). LC-MS (ESI, Method 2): t_{R} = 3.76 min, m/z (M+H)⁺= 504.2.

### Step 8. Methyl (S*)-6-(cyclopropanecarboxamido)-4-((5-(2,2-difluoro-1-methoxypropyl)-4-methoxy-1-methyl-1H-indazol-3-yl)amino)nicotinate (115A) and Methyl (R*)-6-(cyclopropanecarboxamido)-4-((5-(2,2-difluoro-1-methoxypropyl)-4-methoxy-1-methyl-1H-indazol-3-yl)amino)nicotinate (115B)

**115** (7.7 mg, 15.29 µmol) was separated by chiral *prep*-HPLC (Method N) to afford **115A** (1.7 mg, 22% yield) as a white solid and **115B** (2.2 mg, 29% yield) as a white solid.

**115A:** LC-MS (ESI, Method 2): t_{R} = 1.23 min, m/z (M+H)⁺= 504.1. Chiral-HPLC (Method 13) t_{R} = 14.32 min.

**115B:** LC-MS (ESI, Method 2): t_{R} = 4.17 min, m/z (M+H)⁺= 504.1. Chiral-HPLC (Method 13) t_{R} = 15.82 min.

### Example 116

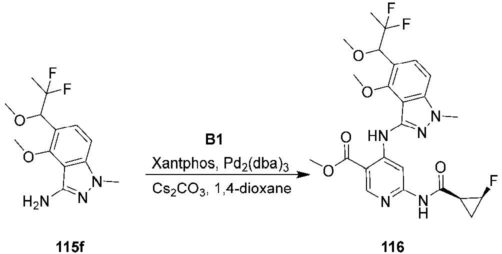

### Methyl 4-((5-(2,2-difluoro-1-methoxypropyl)-4-methoxy-1-methyl-1H-indazol-3-yl)amino)-6-((1S,2S)-2-fluorocyclopropane-1-carboxamido)nicotinate (116)

A mixture of **115f** (20 mg, 70.1 µ mol), **B1** (21.0 mg, 77.1 µ mol), Pd₂(dba)₃ (12.8 mg, 14.02 µmol), Xantphos (8.1 mg, 14.02 µmol) and Cs₂CO₃ (45.7 mg, 140.2 µmol) in 1,4-dioxane (0.5 mL) was stirred at 90 °C for 3 h under N₂ atmosphere in a sealed tube. The mixture was concentrated and purified by flash chromatography on silica gel (DCM/MeOH = 19/1) to give the crude product. The crude product was purified by *Prep-HPLC* (Method A) to afford **116** (9.3 mg, 25% yield) as a white solid. 1H NMR (400 MHz, DMSO-*d*₆) δ 10.92 (s, 1H), 10.90 (s, 1H), 9.18 (d, *J =* 4.8 Hz, 1H), 8.77 (s, 1H), 7.43 (d, *J* = 8.8 Hz, 1H), 7.40 (d, *J =* 8.8 Hz, 1H), 5.05-4.79 (m, 2H), 3.99 (s, 3H), 3.93 (s, 3H), 3.87 (s, 3H), 3.23 (s, 3H), 2.41-2.17 (m, 1H), 1.66 (t, *J =* 19.2 Hz, 3H), 1.12-0.85 (m, 2H). LC-MS (ESI, Method 2): t_{R} = 1.23 min, m/z (M+H)⁺= 522.1.

### Example 117

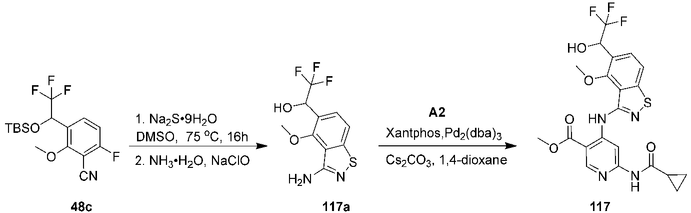

### Step 1. 1-(3-Amino-4-methoxybenzo[d]isothiazol-5-yl)-2,2,2-trifluoroethan-1-ol (117a)

A mixture of **48c** (200 mg, 0.55 mmol), Na₂S·9H₂O (264 mg, 1.10 mmol) and DMSO (3 mL) in seal tube was stirred at 75 °C for 16 h. The reaction was cooled to room temperature. Sodium hypochlorite solution (3 mL) was added to ammonium hydroxide (3 mL) under ice-water bath. To it was added the above reaction solution dropwise. The mixture was stirred for 15 min at this temperature and stirred for 1 h at room temperature. The solution was concentrated and purified by *Prep*-HPLC (Method A) to give compound **117a** (50 mg, 58% yield) as a yellow solid. ¹H NMR (300 MHz, DMSO-*d*₆) δ 7.81 (d, *J* = 8.0 Hz, 1H), 7.84 (d, *J =* 8.0 Hz, 1H), 7.20 (s, 1H), 6.58 (s, 2H), 5.50-5.48 (m, 1H), 3.89 (s, 3H).

### Step 2. Methyl 6-(cyclopropanecarboxamido)-4-((4-methoxy-5-(2,2,2-trifluoro-1-hydroxyethyl)benzo[d]isothiazol-3-yl)amino)nicotinate (117)

A mixture of **117a** (28 mg, 100.6 µmol), **A2** (28.2 mg, 100.6 µmol), Pd₂(dba)₃ (18.4 mg, 20.13 µmol), Xantphos (11.7 mg, 20.13 µmol) and Cs₂CO₃ (65.6 mg, 201.3 µmol) in 1,4-dioxane (0.5 mL) was stirred at 90 °C for 8 h under N₂ atmosphere in a sealed tube. The mixture was concentrated and purified by column chromatography (DCM/MeOH = 19/1) to give the crude product. The crude product was purified by *Prep*-HPLC (Method C) to give 117 (8.7 mg, 17% yield) as a white solid. ¹H NMR (400 MHz, CD₃OD) δ 8.62 (s, 1H), 7.92 (s, 1H), 7.69 (d, *J* = 8.4 Hz, 1H), 7.62 (d, *J =* 8.8 Hz, 1H), 6.43 (q, *J =* 6.0 Hz, 1H), 4.08 (s, 3H), 3.92 (s, 3H), 1.83-1.79 (m, 1H), 1.00-0.83 (m, 4H). LC-MS (ESI, Method 2): t_{R} = 1.27 min, m/z (M+H)⁺= 497.0.

### Example 118

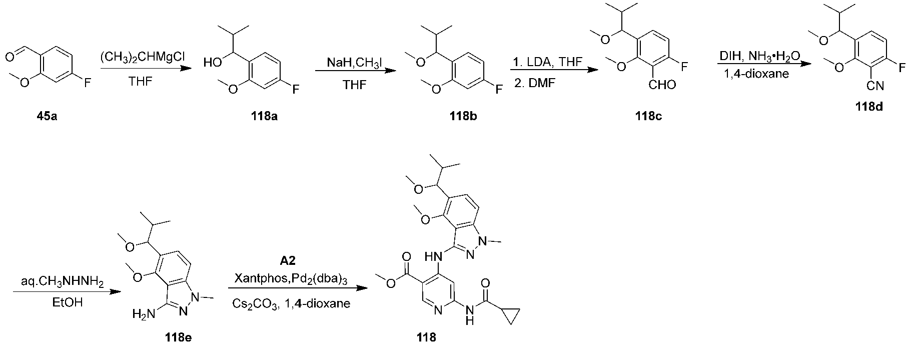

### Step 1. 1-(4-Fluoro-2-methoxyphenyl)-2-methylpropan-1-ol (118a)

To a solution of **45a** (2 g, 12.98 mmol) in THF (20 mL) was slowly added (isopropyl)magnesium chloride (9.8 mL, 19.5 mmol, 2 M in THF) at -40°C and the mixture was stirred at -40°C for 1 h. After the mixture was stirred at 25°C for 1 h. it was diluted with H₂O (20 mL) and extracted with EtOAc (20 mL*2). The organic layer was concentrated and purified by flash chromatography (PE/EtOAc = 40/1) to afford **118a** (1.8 g, 70% yield) as a yellow liquid. ¹H NMR (400 MHz, CDCl₃) δ 7.24-7.20 (m, 1H), 6.86-6.58 (m, 2H), 4.50 (d, *J =* 9.2 Hz, 1H), 3.81 (s, 3H), 2.07-1.97 (m, 2H), 1.00 (d, *J =* 6.8 Hz, 3H), 0.78 (d, *J =* 6.8 Hz, 3H).

### Step 2. 4-Fluoro-2-methoxy-1-(1-methoxy-2-methylpropyl)benzene (118b)

To a solution of **118a** (1.8 g, 9.08 mmol) in THF (15 mL) was added NaH (545 mg, 13.62 mmol, 60% in mineral oil) at 0 °C. After stirring at 0 °C for 30 min, the mixture was added with CH₃I (2.58 g, 18.16 mmol) and stirred at 0 °C for 1 h. After it was stirred at 35 °C for 12 h. The solution was poured into ice-water (10 mL) and extracted with EtOAc (10 mL*2). The combined organic phase was purified by flash chromatography (PE/EtOAc = 30/1) to give **118b** (1.17 g, 61% yield) as a yellow liquid. ¹H NMR (400 MHz, CDCl₃) δ 7.16 (d, *J* = 7.2 Hz, 1H), 6.62-6.56 (m, 1H), 6.51 (dd, *J =* 11.2, 2.4 Hz, 1H), 4.20 (d, *J =* 6.8 Hz, 1H), 3.71 (s, 3H), 3.10 (s, 3H), 1.84-1.79 (m, 1H), 0.85 (d, *J =* 6.8 Hz, 3H), 0.72 (d, *J =* 6.8 Hz, 3H).

### Step 3. 6-Fluoro-2-methoxy-3-(1-methoxy-2-methylpropyl)benzaldehyde (118c)

To a solution of **118b** (1.17 g, 5.51 mmol) in THF (10 mL) was dropwise added LDA (8.3 mL, 16.54 mmol, 2 M in THF) at -50 °C. The mixture was stirred at -50 °C for 1 h. Then DMF (1.28 mL, 16.54 mmol) was added to the mixture at -50 °C and the mixture was stirred at - 50 °C for 1 h. The reaction was quenched with *sat.* NH₄Cl (10 mL) and extracted with EtOAc (10 mL*3). The combined organic layer was concentrated to give **118c** (800 mg, crude) as a yellow oil which was used for the next step directly without further purification.

### Step 4. 6-Fluoro-2-methoxy-3-(1-methoxy-2-methylpropyl)benzonitrile (118d)

To a solution of **118c** (800 mg, 3.33 mmol) in NH₃·H₂O (4 mL) and 1,4-dioxane (4 mL) was added 1,3-diiodo-5,5-dimethylimidazolidine-2,4-dione (1.27 g, 3.33 mmol) at ice-bath. Then the mixture was stirred at 25 °C for 12 h. The mixture was diluted with water (5 mL) and extracted with EtOAc (5 mL*2). The organic layer was concentrated to give **118d** (600 mg, crude) as a yellow liquid which was used for the next step directly without further purification.

### Step 5. 4-Methoxy-5-(1-methoxy-2-methylpropyl)-1-methyl-1H-indazol-3-amine (118e)

A mixture of **118d** (600 mg, 2.13 mmol) and 40% *aq.* methylhydrazine (2 mL) in EtOH (4 mL) was stirred at 70 °C for 4 h. After cooling to room temperature, the mixture was diluted with water (2 mL) and extracted with EtOAc (4 mL*2). The combined organic layer was concentrated and purified by column chromatography (PE/EtOAc = 3/1) to give **118e** (45 mg, 6.8% yield) as a yellow solid. LC-MS (ESI, Method 3): t_{R} = 1.25 min, m/z (M+H)⁺= 263.8.

### Step 6. Methyl 6-(cyclopropanecarboxamido)-4-((4-methoxy-5-(1-methoxy-2-methylpropyl)-1-methyl-1H-indazol-3-yl)amino)nicotinate (118)

A mixture of **118e** (45 mg, 170.8 µmol), **A2** (50.2 mg, 205.1 µmol), Pd₂(dba)₃ (31.3 mg, 34.1 µmol), Xantphos (19.8 mg, 34.1 µmol) and Cs₂CO₃ (111.4 mg, 341.8 µmol) in 1,4-dioxane (0.5 mL) was stirred at 90 °C for 4 h under N₂ atmosphere. The reaction mixture was concentrated and purified by flash chromatography on silica gel (DCM/MeOH = 30/1) to give crude product. The crude product was purified by *Prep-HPLC* (Method C) to give **118** (40 mg, 49% yield) as a white solid. ¹H NMR (400 MHz, DMSO-*d*₆) δ 10.85 (s, 2H), 9.16 (s, 1H), 8.76 (s, 1H), 7.37 (d, *J =* 8.8 Hz, 1H), 7.31 (d, *J =* 8.8 Hz, 1H), 4.25 (d, *J =* 7.6 Hz, 1H), 3.96 (s, 3H), 3.92 (s, 3H), 3.84 (s, 3H), 3.19 (s, 3H), 2.04-2.01 (m, 1H), 1.94-1.89 (m, 1H), 1.01 (d, *J =* 6.8 Hz, 3H), 0.85-0.81 (m, 4H), 0.79 (d, *J* = 6.8 Hz, 3H). LC-MS (ESI, Method 2): t_{R} = 1.28 min, m/z (M+H)⁺= 482.1.

### Example 119

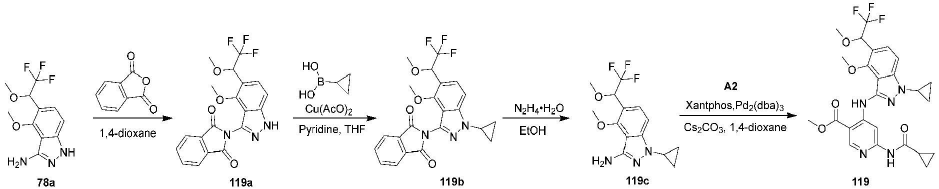

### Step 1. 2-(4-Methoxy-5-(2,2,2-trifluoro-1-methoxyethyl)-1H-indazol-3-yl)isoindoline-1,3-dione (119a)

A mixture of **78a** (500 mg, 1.82 mmol) and isobenzofuran-1,3-dione (1.08 g, 7.27 mmol) in 1,4-dioxane (10 mL) was stirred for 14 h at 105°C. After cooling to room temperature, the mixture was concentrated and purified by flash chromatography on silica gel (PE/EtOAc = 3/1) to give **119a** (300 mg, 41% yield) as a yellow solid. LC-MS (ESI, Method 3): t_{R} = 1.36 min, m/z (M+H)⁺= 405.8.

### Step 2. 2-(1-Cyclopropyl-4-methoxy-5-(2,2,2-trifluoro-1-methoxyethyl)-1H-indazol-3-yl) isoindoline-1,3-dione (119b)

A mixture of **119a** (260 mg, 0.64 mmol), cyclopropylboronic acid (165.3 mg, 1.92 mmol), pyridine (50.7 mg, 0.64 mmol, 51.67 µL) and Cu(OAc)₂ (174.8 mg, 0.96 mmol) in THF (6 mL) was stirred at 70°C for 14 h. After cooling to room temperature, the reaction was poured into ice-water (5 mL) and extracted with DCM (10 mL*3). Then it was dried over Na₂SO₄, filtered and concentrated to give **119b** (320 mg, 90% yield, 80% purity) as a yellow solid. LC-MS (ESI, Method 3): t_{R} = 1.39 min, m/z (M+H)⁺= 445.9.

### Step 3. 1-Cyclopropyl-4-methoxy-5-(2,2,2-trifluoro-1-methoxyethyl)-1H-indazol-3-amine (119c)

To a solution of **119b** (300 mg, 0.674 mmol) in EtOH (3 mL) was added hydrazinium hydroxide (3 mL, 98% purity) and stirred at 25°C for 1 h. The solvent was removed, then the residue was extracted with DCM (5 mL*3), dried over Na₂SO₄, filtered and concentrated. The residue was purified by flash chromatography (PE/EtOAc = 1/1) to give **119c** (100 mg, 47% yield) as a white solid. ¹H NMR (400 MHz, DMSO-*d*₆): δ 7.27 (s, 1H), 5.26 (s, 2H), 5.16 (q, *J =* 8.8 Hz, 1H), 3.87 (s, 3H), 3.42-3.39 (m, 1H), 3.32 (s, 3H), 1.00-0.98 (m, 4H). LC-MS (ESI, Method 3): t_{R} = 0.91 min, m/z (M+H)⁺= 316.2.

### Step 4. Methyl 6-(cyclopropanecarboxamido)-4-((1-cyclopropyl-4-methoxy-5-(2,2,2-trifluoro-1-methoxyethyl)-1H-indazol-3-yl)amino)nicotinate (119)

A mixture of **119c** (40 mg, 0.127 mmol), **A2** (35.5 mg, 0.147 mmol), Xantphos (14.7 mg, 25.37 µmol), Pd₂(dba)₃ (23.2 mg, 25.37 µmol mmol) and Cs₂CO₃ (82.7 mg, 0.254 mmol) in 1,4-dioxane (0.5 mL) was stirred at 90 °C for 2 h under N₂ atomsphere. The solvent was removed. The residue was purified by column chromatography (PE/EtOAc = 1/1) to give **119** (35 mg, 59% yield) as a white solid. ¹H NMR (400 MHz, DMSO-*d*₆): δ 10.90 (s, 2H), 9.29 (s, 1H), 8.77 (s, 1H), 8.61 (d, *J =* 8.8 Hz, 1H), 8.45 (d, *J* = 8.8 Hz, 1H), 5.30-5.28 (m, 1H), 3.92 (s, 3H), 3.89 (s, 3H), 3.79-3.76 (m, 1H), 3.32 (s, 3H), 2.06-2.03 (m, 1H), 1.30-1.29 (m, 2H), 1.07-1.05 (m, 2H), 0.84-0.82 (m, 4H). LC-MS (ESI, Method 2): t_{R} = 1.30 min, m/z (M+H)⁺= 534.1.

### Example 120

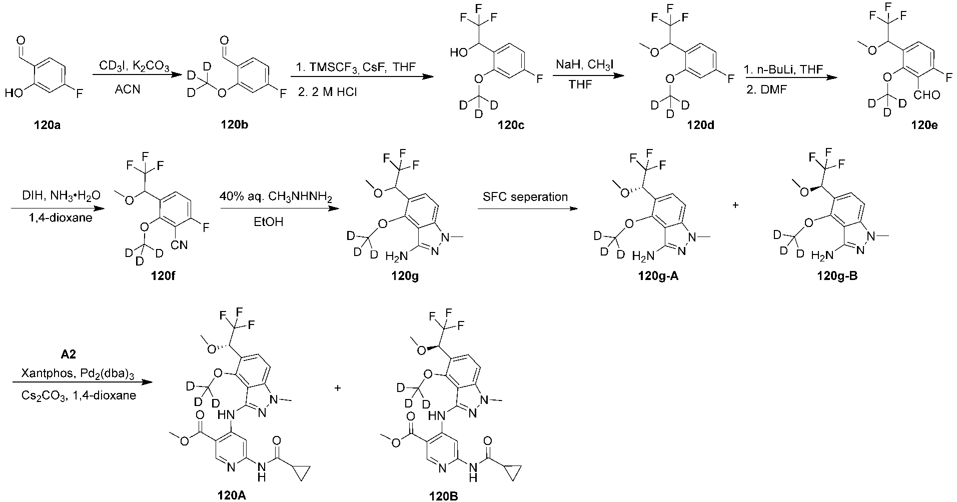

### Step 1. 4-Fluoro-2-(methoxy-d₃)benzaldehyde (120b)

To a solution of **120a** (5 g, 35.69 mmol) and K₂CO₃ (9.86 g, 71.37 mmol) in ACN (50 mL) was added CD₃I (15.52 g, 107.06 mmol) and the mixture was stirred at 35°C for 12 h. The reaction mixture was concentrated and purified by flash chromatography (PE/EtOAc = 50/1) to give **120b** (5 g, 89% yield) as a white solid. ¹H NMR (400 MHz, CDCl₃) δ 10.29 (s, 1H), 7.78 (dd, *J =* 8.4, 7.2 Hz, 1H), 6.67-6.59 (m, 2H).

### Step 2. 2,2,2-Trifluoro-1-(4-fluoro-2-(methoxy-d₃)phenyl)ethan-1-ol (120c)

To a solution of 120b (5 g, 31.82 mmol) and TMSCF3 (9.05 g, 63.63 mmol) in THF (50 mL) was slowly added CsF (96.7 mg, 0.636 mmol) at 0°C and the mixture was stirred at 20°C for 1 h. Then the mixture was added with *aq.* HCl (2 M, 20 mL) and stirred at 35°C for 8 h. The mixture was extracted with EtOAc (20 mL*2). The organic layer was concentrated and purified by flash chromatography (PE/EtOAc = 20/1) to give **120c** (5.3 g, 73% yield) as a yellow liquid. ¹H NMR (400 MHz, CDCl₃) δ 7.40 (t, *J =* 8.0 Hz, 1H), 6.74-6.69 (m, 1H), 6.65 (dd, *J =* 10.4 Hz, 2.4 Hz, 1H), 5.30 (q, *J =* 6.8 Hz, 1H), 3.33 (s, 1H).

### Step 3. 4-Fluoro-2-(methoxy-d₃)-1-(2,2,2-trifluoro-1-methoxyethyl)benzene (120d)

To a solution of **120c** (5.3 g, 23.33 mmol) in THF (50 mL) was slowly added NaH (1.40 g, 35.00 mmol, 60% in mineral oil) at 0°C and the mixture was stirred at 0°C for 30 min. The mixture was added with CH₃I (9.94 g, 69.99 mmol) at 0°C and stirred at 35°C for 3 h. Then it was poured into ice-water (20 mL) and extracted with EtOAc (20 mL*2). The combined organic phase was concentrated and purified by flash chromatography (PE/EtOAc = 30/1) to give **120d** (5.3 g, 94% yield) as a yellow liquid. ¹H NMR (400 MHz, CDCl₃) δ 7.46 (t, *J =* 7.6 Hz, 1H), 6.75-6.70 (m, 1H), 6.63 (dd, *J =* 10.8 Hz, 2.4 Hz, 1H), 5.08 (q, *J =* 6.4 Hz, 1H), 3.38 (s, 3H).

### Step 4. 6-Fluoro-2-(methoxy-d₃)-3-(2,2,2-trifluoro-1-methoxyethyl)benzaldehyde (120e)

To a solution of **120d** (1 g, 4.15 mmol) in THF (10 mL) was added n-BuLi (5.2 mL, 8.32 mmol, 1.6 M in hexane) dropwise over 30 min at -60 °C. The reaction mixture was stirred for 1 h at the same temperature. Then DMF (0.96 mL, 12.44 mmol) was added to the solution dropwise at -60 °C. The reaction was stirred for another 1 h at the same temperature and quenched with 4 M HCl (5 mL). The mixture was extracted with EtOAc (10 mL*2). The combined organic layer was washed with brine (3 mL), dried over Na₂SO₄, filtered and concentrated to give **120e** (900 mg, 81% yield) as a yellow liquid.

### Step 5. 6-Fluoro-2-(methoxy-d₃)-3-(2,2,2-trifluoro-1-methoxyethyl)benzonitrile (120f)

To a solution of **120e** (900 mg, 3.35 mmol) in NH₃·H₂O (6 mL) and 1,4-dioxane (3 mL) was slowly added 1,3-diiodo-5,5-dimethylimidazolidine-2,4-dione (1.27 g, 3.34 mmol) at 0 °C and the mixture was stirred at 25°C for 12 h. The reaction mixture was diluted with H₂O (5 mL) and extracted with EtOAc (10 mL*2). The combined organic phase was concentrated to give **120f** (800 mg, 90% yield) as a yellow liquid.

### Step 6. 4-(Methoxy-d₃)-1-methyl-5-(2,2,2-trifluoro-1-methoxyethyl)-1H-indazol-3-amine (120g)

To a solution of **120f** (800 mg, 3.01 mmol) in EtOH (3 mL) was added *aq.* methylhydrazine (5 mL, 40% in water). The reaction was stirred at 70°C for 3 h. Then it was concentrated and purified by flash chromatography (PE/EtOAc = 2/1) to give **120g** (420 mg, 48% yield) as a white solid. ¹H NMR (400 MHz, DMSO-*d*₆) δ 7.23 (d, *J =* 8.8 Hz, 1H), 7.19 (d, *J =* 8.8 Hz, 1H), 5.22 (s, 2H), 5.21-5.17 (m, 1H), 3.73 (s, 3H), 3.28 (s, 3H). LC-MS (ESI, Method 3): t_{R} = 1.16 min, m/z (M+H)⁺= 293.3.

### Step 7. (S)-4-(methoxy-d₃)-1-methyl-5-(2,2,2-trifluoro-1-methoxyethyl)-1H-indazol-3-amine (120g-A) and (R)-4-(methoxy-d₃)-1-methyl-5-(2,2,2-trifluoro-1-methoxyethyl)-1H-indazol-3-amine (120g-B)

**120g** (420 mg, 1.44 mmol) was separated by chiral *prep-HPLC* (Method G, Hex/EtOH = 80/20) to give **120g-A** (180 mg, 43% yield) as a white solid and **120g-B** (182 mg, 43% yield) as a white solid.

**120g-A:** Chiral-HPLC (Method 8, Hex\EtOH = 80\20, Column Size 4.6mm*150mm 3um) t_{R} = 6.22 min.

**120g-B:** Chiral-HPLC (Method 8, Hex\EtOH = 80\20, Column Size 4.6mm*150mm 3um) t_{R} = 7.16 min.

### Step 8. Methyl (S)-6-(cyclopropanecarboxamido)-4-((4-(methoxy-d₃)-1-methyl-5-(2,2,2-trifluoro-1-methoxyethyl)-1H-indazol-3-yl)amino)nicotinate (120A) and Methyl (R)-6-(cyclopropanecarboxamido)-4-((4-(methoxy-d₃)-1-methyl-5-(2,2,2-trifluoro-1-methoxyethyl)-1H-indazol-3-yl)amino)nicotinate (120B)

A mixture of **120g-A** (37 mg, 0.127 mmol), **A2** (38.7 mg, 0.152 mmol), Cs₂CO₃ (82.5 mg, 0.253 mmol), Xantphos (14.7 mg, 0.0253 mmol) and Pd₂(dba)₃ (23.2 mg, 0.0253 mmol) in 1,4-dioxane (0.5 mL) was stirred at 90 °C under N₂ atmosphere for 6 h. The reaction mixture was concentrated and purified by column chromatography (DCM/MeOH = 20/1) to give **120A** (27 mg, 42% yield) as a white solid. ¹H NMR (400 MHz, DMSO-*d*₆) δ 10.91 (s, 1H), 10.89 (s, 1H), 9.17 (s, 1H), 8.77 (s, 1H), 7.47 (d, *J =* 8.8 Hz, 1H), 7.41 (d, *J* = 8.8 Hz, 1H), 5.30-5.26 (m, 1H), 3.98 (s, 3H), 3.92 (s, 3H), 3.36 (s, 3H), 2.05-2.02 (m, 1H), 0.84-0.81 (m, 4H). LC-MS (ESI, Method 2): t_{R} = 1.24 min, m/z (M+H)⁺= 511.1. Chiral-HPLC (Method 8, Hex\EtOH\DEA = 90\10\0.2): t_{R} = 19.63 min.

A mixture of **120g-B** (36.0 mg, 123 µmol), **A2** (37.6 mg, 148 µmol), Cs₂CO₃ (80.3 mg, 246 µmol), Xantphos (7.1 mg, 12.32 µmol) and Pd₂(dba)₃ (11.3 mg, 12.32 µmol) in 1,4-dioxane (0.5 mL) was stirred at 90 °C under N₂ atmosphere for 5 h. The mixture was concentrated and purified by Prep-HPLC (Method A) to give the **120B** (23 mg, 37% yield) as a white solid. ¹H NMR (400 MHz, DMSO-*d*₆) δ 10.91 (s, 1H), 10.89 (s, 1H), 9.17 (s, 1H), 8.77 (s, 1H), 7.47 (d, *J* = 8.8 Hz, 1H), 7.41 (d, *J =* 8.8 Hz, 1H), 5.30-5.26 (m, 1H), 3.98 (s, 3H), 3.92 (s, 3H), 3.36 (s, 3H), 2.05-2.02 (m, 1H), 0.84-0.81 (m, 4H). LC-MS (ESI, Method 2): t_{R} = 1.24 min, m/z (M+H)⁺ = 511.1. Chiral-HPLC (Method 8, Hex\EtOH\DEA = 90\10\0.2): t_{R} = 18.54 min.

### Example 121

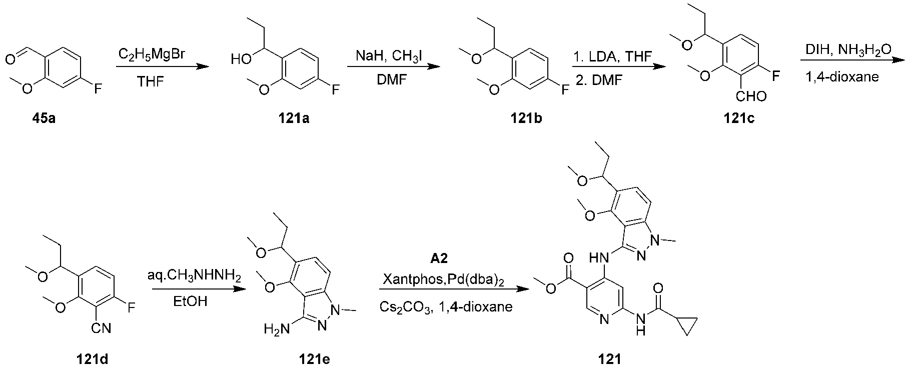

### Step 1. 1-(4-Fluoro-2-methoxyphenyl)propan-1-ol (121a)

To a solution of **45a** (2 g, 12.98 mmol) in THF (10 mL) was added ethylmagnesium bromide (13 mL, 26 mmol, 2 M in THF) at -60 °C. The reaction was stirred for 2 h at -60 °C and quenched with *sat.* NH₄Cl (30 mL). The mixture was extracted with EtOAc (30 mL*2). The combined organic phase was washed with brine (20 mL), dried over anhydrous Na₂SO₄, filtered and concentrated to give **121a** (2.1 g, 88% yield) as a yellow liquid. ¹H NMR (400 MHz, DMSO-*d*₆) δ 7.37 (d, *J =* 8.0 Hz, 1H), 6.84-6.80 (m, 1H), 6.75-6.71 (m, 1H), 4.95 (d, *J =* 4.8 Hz, 1H), 4.76-4.72 (m, 1H), 3.78 (s, 3H), 1.62-1.55 (m, 1H), 1.49-1.42 (m, 1H), 0.82 (t, *J* =7.6 Hz, 3H).

### Step 2. 4-Fluoro-2-methoxy-1-(1-methoxypropyl)benzene (121b)

To a solution of **121a** (2.1 g, 11.40 mmol) in DMF (20 mL) was added NaH (410.4 mg, 17.10 mmol, 60% in mineral oil) at 0 °C. The mixture was stirred at 0 °C for 30 min. Then the mixture was added with CH₃I (4.85 g, 34.20 mmol) and stirred at 20 °C for 12 h. It was quenched with *sat.* NH₄Cl (30 mL) at 0 °C. The mixture was extracted with EtOAc (30.0 mL*2). The combined organic phase was washed with brine (20 mL), dried over Na₂SO₄, filtered and concentrated to give **121b** (2.1 g, 93% yield) as a yellow liquid. ¹H NMR (400 MHz, CDCl₃) δ 7.22-7.18 (m, 1H), 6.62-6.58 (m, 1H), 6.54-6.50 (m, 1H), 4.41 (t, *J =* 6.0 Hz, 1H), 3.73 (s, 3H), 3.15 (s, 3H), 1.64-1.56 (m, 2H), 0.81 (t, *J* =7.2 Hz, 3H).

### Step 3. 6-Fluoro-2-methoxy-3-(1-methoxypropyl)benzaldehyde (121c)

To a solution of **121b** (1 g, 5.04 mmol) in THF (10 mL) was added LDA (7.5 mL, 15 mmol, 2 M in THF) dropwise at -60 °C under N₂ atomsphere. The mixture was stirred for 1h at - 60 °C. Then the mixture was added with DMF (1.11 g, 15.13 mmol, 1.17 mL) dropwise at - 60 °C and stirred at -60 °C for 2 h. It was quenched with *sat.* NH₄Cl (20 mL). The mixture was extracted with EtOAc (10 mL*3), washed with brine (20 mL), dried over Na₂SO₄, filtered and concentrated to give **121c** (1 g, crude) as a yellow oil which was used in the next step without further purification.

### Step 4. 6-Fluoro-2-methoxy-3-(1-methoxypropyl)benzonitrile (121d)

To a solution of **121c** (1 g, 4.42 mmol) in dioxane (5 mL) and NH₃·H₂O (5 mL) was added 1,3-diiodo-5,5-dimethylimidazolidine-2,4-dione (1.68 g, 4.42 mmol) at 0°C. The mixture was stirred at 35°C for 16 h. The reaction was cooled to room temperature and added with NH₄Cl (20 mL). Then it was extracted with EtOAc (10 mL*3). The combine organic layer was washed with brine (20 mL), dried over Na₂SO₄, filtered and concentrated to give **121d** (800 mg, 81 % yield) as a yellow oil which was used for the next step directly without further purification.

### Step 5. 4-Methoxy-5-(1-methoxypropyl)-1-methyl-1H-indazol-3-amine (121e)

To a solution of **121d** (800 mg, 3.58 mmol) in EtOH (2 mL) was added *aq.* methylhydrazine (4 mL, 40% in water). The mixture was stirred at 80 °C for 4 h. The solvent was cooled to room temperature and concentrated. The residue was purified by flash chromatography (PE/EtOAc = 3/1to1/1) to give **121e** (140 mg, 25% yield) as a yellow solid. LC-MS (ESI, Method 3): t_{R} = 1.16 min, m/z (M+H)⁺= 249.8.

### Step 6. Methyl 6-(cyclopropanecarboxamido)-4-((4-methoxy-5-(1-methoxypropyl)-1-methyl-1H-indazol-3-yl)amino)nicotinate (121)

A solution of **121e** (40 mg, 160.4 µmol), **A2** (81.7 mg, 320.9 µmol), Pd(dba)₂ (18.5 mg, 32.1 µmol), Xantphos (18.6 mg, 32.1 µmol), Cs₂CO₃ (209.1 mg, 641.8 µmol) in dioxane (2 mL) was stirred at 90 °C for 16 h under N₂ atomsphere. After the solvent was cooled to room temperature, it was concentrated and purified by flash chromatography (PE/EtOAc = 3/1 to 1/1) to give the crude compound which was purified by *Prep*-HPLC (Method A) to give **121** (20 mg, 67% yield) as a white solid. ¹H NMR (400 MHz, DMSO-*d*₆) δ 10.91 (s, 1H), 10.87 (s, 1H), 9.21 (s, 1H), 8.76 (s, 1H), 7.37 (d, *J =* 8.8 Hz, 1H), 7.34 (d, *J =* 8.8 Hz, 1H), 4.53 (t, *J =* 6.8 Hz, 1H), 3.96 (s, 3H), 3.93 (s, 3H), 3.85 (s, 3H), 3.10 (s, 3H), 2.05-2.02 (m, 1H), 1.86-1.79 (m, 1H), 1.66-1.59 (m, 1H), 0.89-0.81 (m, 7H). LC-MS (ESI, Method 2): t_{R} = 1.24 min, m/z (M+H)⁺= 468.1.

### Example 122

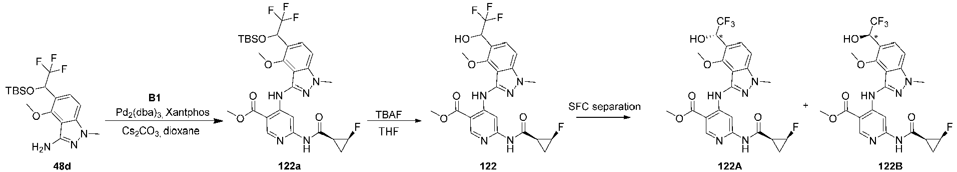

### Step 1. Methyl 4-((5-(1-((tert-butyldimethylsilyl) oxy)-2,2,2-trifluoroethyl)-4-methoxy-1-methyl-1H-indazol-3-yl) amino)-6-((1S,2S)-2-fluorocyclopropane-1-carboxamido) nicotinate (122a)

A mixture of **48d** (100 mg, 256.8 µmol), **B1** (77.0 mg, 282.4 µmol), Pd₂(dba)₃ (47.0 mg, 51.35 µmol), Xantphos (29.7 mg, 51.35 µmol) and Cs₂CO₃ (167.3 mg, 513.5 µmol) in dioxane (1 mL) was stirred at 90°C for 4 h under N₂ atmosphere. After cooling to room temperature, the mixture was diluted with water (10 mL) and extracted with EtOAc (20 mL*3). The combined organic layer was concentrated and purified by flash chromatography (DCM/MeOH = 10/1) to give **122a** (141 mg, 225.3 µmol, 88% yield) as a yellow oil. LC-MS (ESI, Method 3): t_{R} = 1.57 min, m/z (M+H)⁺= 625.9.

### Step 2. Methyl 6-((1S,2S)-2-fluorocyclopropane-1-carboxamido)-4-((4-methoxy-1-methyl-5-(2,2,2-trifluoro-1-hydroxyethyl)-1H-indazol-3-yl) amino) nicotinate (122)

A mixture of **122a** (140 mg, 223.8 µmol) in TBAF (1 mL, 1 M in THF) and THF (1 mL) was stirred at 15°C for 2 h. The mixture was diluted with ice water (2 mL) and extracted with EtOAc (5 mL*2). The organic layer was concentrated and purified by flash chromatography (DCM/MeOH = 20/1) to give **122** (40 mg, 35% yield) as a yellow oil. ¹H NMR (400 MHz, DMSO-*d*₆) δ 10.94 (s, 1H), 10.93 (s, 1H), 9.22 (s, 1H), 8.78 (s, 1H), 7.55 (d, *J* = 8.8 Hz, 1H), 7.44 (d, *J =* 8.8 Hz, 1H), 6.84 (d, *J =* 5.6 Hz, 1H), 5.47-5.43 (m, 1H), 5.02-4.84 (m, 1H), 3.98 (s, 3H), 3.93 (s, 3H), 3.88 (s, 3H), 2.24-2.22 (m, 1H), 1.70-1.63 (m, 1H), 1.20-1.15 (m, 1H). LC-MS (ESI, Method 3): t_{R} = 1.19 min, m/z (M+H)⁺= 512.1.

### Step 3. Methyl 6-((1S,2S)-2-fluorocyclopropane-1-carboxamido)-4-((4-methoxy-1-methyl-5-((S*)-2,2,2-trifluoro-1-hydroxyethyl)-1H-indazol-3-yl) amino) nicotinate (122A) and Methyl 6-((1S,2S)-2-fluorocyclopropane-1-carboxamido)-4-((4-methoxy-1-methyl-5-((R*)-2,2,2-trifluoro-1-hydroxyethyl)-1H-indazol-3-yl) amino) nicotinate (122B)

Compound **122** (30 mg, 58.66 µmol) was separated by chiral *prep*-HPLC (Method G, Hex/IPA = 70/30) to afford **122A** (9 mg, 30% yield) as a white solid and **122B** (17 mg, 57% yield) as a white solid.

**122A:** ¹H NMR (400 MHz, DMSO-*d*₆) δ 10.94 (s, 1H), 10.93 (s, 1H), 9.22 (s, 1H), 8.78 (s, 1H), 7.55 (d, *J =* 8.8 Hz, 1H), 7.44 (d, *J =* 8.8 Hz, 1H), 6.84 (d, *J =* 5.6 Hz, 1H), 5.47-5.43 (m, 1H), 5.02-4.84 (m, 1H), 3.98 (s, 3H), 3.93 (s, 3H), 3.88 (s, 3H), 2.24-2.22 (m, 1H), 1.70-1.63 (m, 1H), 1.20-1.15 (m, 1H). LC-MS (ESI, Method 2): t_{R} = 1.17 min, m/z (M+H)⁺= 512.1. Chiral-HPLC (Method 8) t_{R} = 7.26 min.

**122B:** ¹H NMR (400 MHz, DMSO-*d*₆) δ 10.94 (s, 1H), 10.93 (s, 1H), 9.22 (s, 1H), 8.78 (s, 1H), 7.55 (d, *J =* 8.8 Hz, 1H), 7.44 (d, *J =* 8.8 Hz, 1H), 6.84 (d, *J =* 5.6 Hz, 1H), 5.47-5.43 (m, 1H), 5.02-4.84 (m, 1H), 3.98 (s, 3H), 3.93 (s, 3H), 3.88 (s, 3H), 2.24-2.22 (m, 1H), 1.70-1.63 (m, 1H), 1.20-1.15 (m, 1H). LC-MS (ESI, Method 2): t_{R} = 1.16 min, m/z (M+H)⁺= 512.0. Chiral-HPLC (Method 8) t_{R} = 12.00 min.

### Example 123

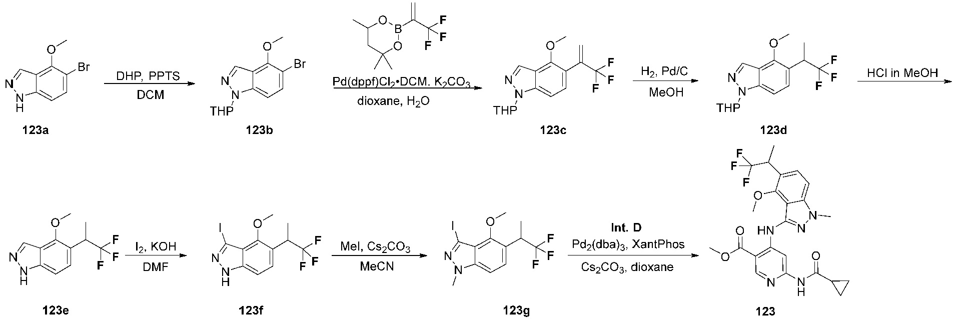

### Step 1. 5-Bromo-4-methoxy-1-(tetrahydro-2H-pyran-2-yl)-1H-indazole (123b)

To a stirred mixture of **123a** (2.47 g, 10.88 mmol) and PPTS (273 mg, 1.09 mmol) in DCM (25 mL) was added DHP (1.83 g, 21.75 mmol). The mixture was stirred at 50°C for 4 h. Then the solvent was removed and the residue was purified by column chromatography (PE/EA = 7/3) to afford the compound **123b** (2.90 g, 86% yield) as an off-white oil. LC-MS (ESI, Method 4) t_{R} = 3.23 min, m/z (M+H)⁺ = 311.1.

### Step 2. 4-Methoxy-1-(tetrahydro-2H-pyran-2-yl)-5-(3,3,3-trifluoroprop-1-en-2-yl)-1H-indazole (123c)

To a stirred mixture of **123b** (2.90 g, 9.31 mmol), 4,4,6-trimethyl-2-(3,3,3-trifluoroprop-1-en-2-yl)-1,3,2-dioxaborinane (2.5 g, 11.26 mmol) and K₂CO₃ (2.4 g, 17.37 mmol) in dioxane (25 mL) and H₂O (5 mL) was added Pd(dppf)Cl₂·DCM (360 mg, 0.44 mmol). The mixture was refluxed under N₂ atmosphere at 100 °C for 16 h. Then it was poured into water (30 mL) and extracted with EtOAc (20 mL*3). The combined organic phase was washed with brine (15 mL) and dried over anhydrous Na₂SO₄. Then it was filtered and concentrated, the residue was purified by column chromatography (MeOH/DCM = 1/10) to afford the compound **123c** (2.0 g, 66% yield) as a light yellow oil. LC-MS (ESI, Method 4) t_{R} = 3.45 min, m/z (M+H)⁺ = 327.2.

### Step 3. 4-Methoxy-1-(tetrahydro-2H-pyran-2-yl)-5-(1,1,1-trifluoropropan-2-yl)-1H-indazole (123d)

A mixture of **123c** (1.0 g, 3.06 mmol) and Pd/C (10%, 100 mg) in MeOH (10 mL) was stirred at room temperature under H₂ atmosphere for 16 h. Then it was filtered and concentrated to give **123d** (1.01 g, 99% yield) as a yellow oil. LC-MS (ESI, Method 4) t_{R} = 3.51 min, m/z (M+H)⁺= 329.2.

### Step 4. 4-Methoxy-5-(1,1,1-trifluoropropan-2-yl)-1H-indazole (123e)

A solution of **123d** (1.01 g, 3.07 mmol) in HCl/MeOH (4 M, 10 mL) was stirred at room temperature for 3 h. Then the solvent was removed to give **123e** (0.74 g, 98% yield) as a white solid. LC-MS (ESI, Method 4) t_{R} = 3.81 min, m/z (M+H)⁺= 245.1.

### Step 5. 3-Iodo-4-methoxy-5-(1,1,1-trifluoropropan-2-yl)-1H-indazole (123f)

To a solution of **123e** (0.74 g, 3.03 mmol) and I₂ (750 mg, 2.95 mmol) in DMF (10mL) was added KOH (580 mg, 10.33 mmol), and the reaction mixture was stirred at room temperature for 2 h. Then it was quenched with water (20 mL) and extracted with EtOAc (20 mL*3). The combined organic phase was washed with brine (15 mL) and dried over anhydrous Na₂SO₄, then it was filtered and concentrated. The residue was purified by column chromatography (EA/PE = 1/10) to afford the compound **123f** (0.96 g, 86% yield) as a white solid. LC-MS (ESI, Method 4) t_{R} = 3.10 min, m/z (M+H)⁺= 371.1.

### Step 6. 3-Iodo-4-methoxy-1-methyl-5-(1,1,1-trifluoropropan-2-yl)-1H-indazole (123g)

To a mixture of **123f** (0.74 g, 2.0 mmol) and Cs₂CO₃ (1.03 g, 3.16 mmol) in CH₃CN (10mL) was added CH₃I (319 mg, 2.25 mmol), and the reaction mixture was stirred at 75 °C for 2 h. Then it was quenched with water (20 mL) and extracted with EA (20 mL*3). The combined organic phase was washed with brine (15 mL) and dried over anhydrous Na₂SO₄, then it was filtered and concentrated. The residue was purified by column chromatography (EA/PE = 1/25) to afford the compound **123g** (542 mg, 71% yield) as a white solid. LC-MS (ESI, Method 4) t_{R} = 3.45 min, m/z (M+H)⁺= 385.1.

### Step 7. Methyl 6-(cyclopropanecarboxamido)-4-((4-methoxy-1-methyl-5-(1,1,1-trifluoropropan-2-yl)-1H-indazol-3-yl)amino)nicotinate (123)

To a stirred mixture of **123g** (149 mg, 0.39 mmol) and **Int. D** (112 mg, 0.47 mmol) in dioxane (5 mL) was added Pd₂(dba)₃ (38 mg, 0.041 mmol), XantPhos (43 mg, 0.074 mmol) and Cs₂CO₃ (258 mg, 0.79 mmol). The reaction mixture was stirred under N₂ atmosphere at 100 °C for 16 h. Then it was poured into water (10 mL) and extracted with EA (15 mL*3). The combined organic phase was washed with brine (15 mL) and dried over anhydrous Na₂SO₄, then it was filtered and concentrated. The residue was purified by column chromatography (MeOH/DCM = 1/20) to give a crude product, which was further purified by *Prep*-HPLC (Method D) to afford the compound **123** (20.0 mg, 11% yield) as a white solid. ¹H NMR (400 MHz, DMSO-*d*₆) δ 10.96 (s, 1H), 10.95 (s, 1H), 9.20 (s, 1H), 8.77 (s, 1H), 7.47-7.41 (m, 2H), 4.18-4.11 (m, 1H), 3.97 (s, 3H), 3.92 (s, 3H), 3.84 (s, 3H), 2.07-2.02 (m, 1H), 1.48 (d, *J =* 7.2 Hz, 3H), 0.86-0.81 (m, 4H). LC-MS (ESI, Method 4) t_{R} = 3.28 min, m/z (M+H)⁺= 492.3.

### Example 124

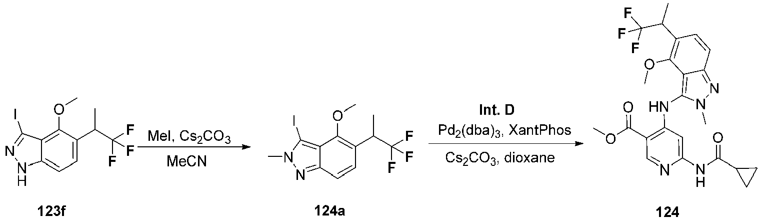

### Step 1. 3-Iodo-4-methoxy-2-methyl-5-(1,1,1-trifluoropropan-2-yl)-2H-indazole (124a)

To a mixture of **123f** (0.74 g, 2.0 mmol) and Cs₂CO₃ (1.03 g, 3.16 mmol) in MeCN (10mL) was added MeI (319 mg, 2.25 mmol), and the reaction mixture was stirred at 75 °C for 2 h. Then it was quenched with water (20 mL) and extracted with EA (20 mL * 3). The combined organic phase was washed with brine (15 mL) and dried over anhydrous Na₂SO₄, then it was filtered and concentrated. The residue was purified by column chromatography (EA/PE = 1/25) to afford the compound **124a** (66 mg, 8% yield) as a white solid. LC-MS (ESI, Method 4) t_{R} = 3.27 min, m/z (M+H)⁺ = 385.1.

### Step 2. Methyl 6-(cyclopropanecarboxamido)-4-((4-methoxy-2-methyl-5-(1,1,1-trifluoropropan-2-yl)-2H-indazol-3-yl)amino)nicotinate (124)

To a stirred mixture of **124a** (18.5 mg, 0.048 mmol) and **Int. D** (20 mg, 0.085 mmol) in dioxane (1 mL) was added Pd₂(dba)₃ (4.8 mg, 0.005 mmol), XantPhos (6.7 mg, 0.011 mmol) and Cs₂CO₃ (38 mg, 0.12 mmol). The reaction mixture was stirred under N₂ atmosphere at 100 °C for 16 h. Then it was concentrated and the residue was purified by *Prep-TLC* (MeOH/DCM = 1/40) to give a crude product, which was further purified by *Prep-HPLC* (Method D) to afford the compound **124** (6.0 mg, 25% yield) as a white solid. ¹H NMR (400 MHz, DMSO-*d*₆) δ 10.90 (s, 1H), 9.72 (s, 1H), 8.74 (s, 1H), 7.42-7.34 (m, 1H), 7.24 (d, *J* = 8.8 Hz, 1H), 7.06-7.04 (m, 1H), 4.09-4.03 (m, 1H), 3.91 (s, 3H), 3.90 (s, 3H), 3.61 (s, 3H), 1.92-1.86 (m, 1H), 1.41 (t, *J* = 7.2 Hz, 3H), 0.71-0.63 (m, 4H). LC-MS (ESI, Method 4) t_{R} = 3.06 min, m/z (M+H)⁺ = 492.3.

### Example 125

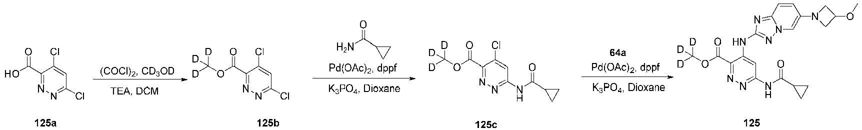

### Step 1. Methyl-d3 4,6-dichloropyridazine-3-carboxylate (125b)

To a solution **of 125a** (2.00 g,10.10 mmol) and TEA (3.09 g, 30.3 mmol) in DCM (20 mL) was added (COCl)₂ (3.85 g, 30.30 mmol) at 0 °C. The solution was stirred at 0 °C for 2 h. Then the solution was added with CD₃OD (1.09 g, 30.3 mmol), it was stirred at 25 °C for 1 h. The reaction mixture was poured into water (50 mL) and extracted with EA (50 mL*3). The combined organic layer was washed with brine (50 mL), dried over Na₂SO₄ and concentrated to give crude product. The crude product was purified by column chromatography (EA in PE is 5%~20%) to give **125b** (1.5 g, 71% yield) as a yellow solid. ¹H NMR (400 MHz, DMSO-*d*₆): δ 8.58 (s, 1H). LC-MS (ESI, Method 2), t_{R} = 1.46 min, m/z (M+H)⁺ = 209.9.

### Step 2. Methyl-d3 4-chloro-6-(cyclopropanecarboxamido) pyridazine-3-carboxylate (125c)

A solution **of 125b** (1.00 g, 4.80 mmol), cyclopropanecarboxamide (0.41 g, 4.80 mmol), palladium diacetate (0.020 g, 0.096 mmol), dppf (0.11 g, 0.19 mmol) and K₃PO₄ (3.06 g, 14.40 mmol) in dioxane (10 mL) was stirred at 100 °C for 6 h under N₂ atmosphere. The reaction mixture was poured into water (50 mL) and extracted with EA (50 mL*3). The combined organic layer was washed with brine, dried over Na₂SO₄ and concentrated to give the crude product. The crude product was purified by column chromatography (EA in PE is 5%~20%) to give **125c** (0.3 g, 26% yield) as a yellow solid. ¹H NMR (400 MHz, DMSO-*d*₆): δ 12.03 (s, 1H), 8.56 (s, 1H), 2.06-2.12 (m, 1H), 0.88-0.96 (m, 4H). LC-MS (ESI, Method 2) t_{R} = 1.97 min, m/z (M+H)⁺ = 258.7.

### Step 3. Methyl-d3 6-(cyclopropanecarboxamido)-4-((6-(3-methoxyazetidin-1-yl)-[1,2,4]triazolo[1,5-a]pyridin-2-yl)amino)pyridazine-3-carboxylate (125)

A solution of **125c** (150 mg, 0.58 mmol), **64a** (127 mg, 0.58 mmol), palladium diacetate (3 mg, 0.012 mmol), dppf (13 mg, 0.023 mmol) and K₃PO₄ (369 mg, 1.74 mmol) in dioxane (10 mL) was stirred at 100 °C for 6 h under N₂ atmosphere. The reaction mixture was concentrated and purified by *prep-HPLC* (Method A) to give **125** (10 mg, 4% yield) as a yellow solid. ¹H NMR (400 MHz, DMSO-*d*₆): δ 11.59 (s, 1H), 10.59 (s, 1H), 9.39 (s, 1H), 7.94 (s, 1H), 7.59 (d, *J* = 9.6 Hz, 1H), 7.14 (d, *J* = 9.6 Hz, 1H), 4.33 (m, 1H), 4.12 (t, *J* = 7.2 Hz, 2H), 3.67-3.69 (m, 2H), 3.25 (s, 3H), 2.13 (m, 1H), 0.89-0.91 (m, 4H). LC-MS (ESI, Method 1) t_{R} = 1.75 min, m/z (M+H)⁺ = 442.2.

### Example 126

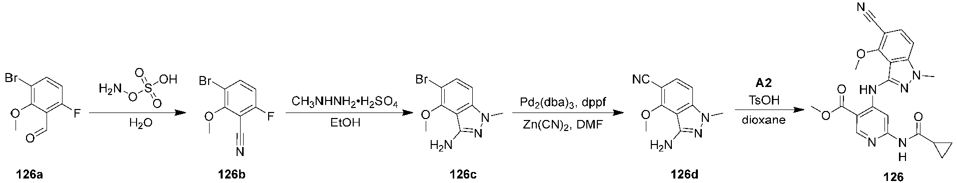

### Step 1. 3-Bromo-6-fluoro-2-methoxybenzonitrile (126b)

To a mixture of **126a** (4.10 g, 17.59 mmol) in H₂O (41 mL), was added NH₂OSO₃H (3.48 g, 30.79 mmol). The resulting mixture was stirred at 50 °C for 16 h, then it was extracted with DCM (150 mL*3). The combined organic layer was washed with water (100 mL*2) and brine (100 mL*2), then it was dried over anhydrous Na₂SO₄, filtered and concentrated to give **126b** (4.00 g, crude) as a yellow solid, which was used for the next step directly without further purification. LC-MS (ESI, Method 4) t_{R} = 2.92 min, m/z (M+H)⁺ = 229.9.

### Step 2. 5-Bromo-4-methoxy-1-methyl-1H-indazol-3-amine (126c)

A mixture of **126b** (4.00 g, 17.39 mmol) and CH₃NHNH₂·H₂SO₄ (12.53 g, 69.56 mmol) in EtOH (40 mL) was stirred at 80 °C for 16 h. The reaction mixture was concentrated and diluted with water (50 mL), then it was extracted with EtOAc (50 mL*2). The combined organic layer was washed with water (50 mL*2) and brine (50 mL*2), then it was dried over anhydrous Na₂SO₄, filtered and concentrated. The residue was purified by column chromatography (EtOAc in PE is 0-40%) to give **126c** (1.31 g, 29% yield) as a yellow solid. LC-MS (ESI, Method 4) t_{R} = 2.39 min, m/z (M+H)⁺ = 256.0.

### Step 3. 3-Amino-4-methoxy-1-methyl-1H-indazole-5-carbonitrile (126d)

To a mixture of **126c** (300 mg, 1.17 mmol) in DMF (5 mL) was added Zn(CN)₂ (206 mg, 1.76 mmol), dppf (162 mg, 0.29 mmol) and Pd₂(dba)₃ (107 mg, 0.12 mmol). The resulting mixture was stirred at 100 °C for 16 h under N₂ atmosphere. The reaction mixture was concentrated and diluted with water (50 mL), then it was extracted with EtOAc (50 mL*2). The combined organic layer was washed with water (50 mL*2) and brine (50 mL*2), then it was dried over anhydrous Na₂SO₄, filtered and concentrated. The residue was purified by column chromatography (EtOAc in PE is 0-60%) to give **126d** (42 mg, 18% yield) as a yellow solid. LC-MS (ESI, Method 4) t_{R} = 1.78 min, m/z (M+H)⁺ = 203.0.

### Step 4. Methyl 4-((5-cyano-4-methoxy-1-methyl-1H-indazol-3-yl)amino)-6-(cyclopropanecarboxamido)nicotinate (126)

To a solution of 126d (20 mg, 0.10 mmol) in dioxane (0.5 mL) was added A2 (25 mg, 0.10 mmol) and TsOH (17. mg, 0.10 mmol). The reaction mixture was stirred at 100 °C for 16 h under N₂ atmosphere. The reaction mixture was concentrated and diluted with water (10 mL), then it was extracted with EtOAc (10 mL*2). The combined organic layer was washed with water (10 mL*2) and brine (10 mL*2), then it was dried over anhydrous Na₂SO₄, filtered and concentrated. The residue was purified by column chromatography (EtOAc in PE is 0-82%) to give **126** (9.4 mg, 23% yield) as a yellow solid. ¹H NMR (400 MHz, DMSO-*d*₆) δ 11.30 (s, 1H), 10.95 (s, 1H), 9.35 (s, 1H), 8.77 (s, 1H), 7.55 (d, *J* = 8.8 Hz, 1H), 7.35 (d, *J =* 8.8 Hz, 1H), 4.39 (s, 3H), 3.96 (s, 3H), 3.93 (s, 3H), 2.10-2.01 (m, 1H), 0.87-0.82 (m, 4H). LC-MS (ESI, Method 4) t_{R} = 2.86 min, m/z (M+H)⁺ = 421.3.

### Example 127

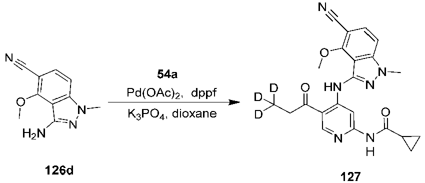

### N-(4-((5-cyano-4-methoxy-1-methyl-1H-indazol-3-yl)amino)-5-(propanoyl-3,3,3-d₃)pyridin-2-yl)cyclopropanecarboxamide (127)

To a mixture of **126d** (10 mg, 0.05 mmol) in dioxane (0.5 mL) was added **54a** (13 mg, 0.05 mmol), dppf (11 mg, 0.02 mmol), K₃PO₄ (26 mg, 0.12 mmol) and Pd(OAc)₂(2 mg, 0.01 mmol). The resulting mixture was stirred at 100 °C for 16 h under N₂ atmosphere. The reaction mixture was diluted with water (10 mL) and extracted with DCM (15 mL*3). The combined organic layer was dried over anhydrous Na₂SO₄, filtered and concentrated. The residue was purified by flash chromatography (PE:EA = 1:2) to give **127** (4.3 mg, 21% yield) as a yellow solid. LC-MS (ESI, Method 4) t_{R} = 2.92 min, m/z (M+H)⁺ = 422.3. ¹H NMR (400 MHz, DMSO-*d₆*) δ 12.33 (s, 1H), 10.93 (s, 1H), 9.36 (s, 1H), 8.94 (s, 1H), 7.56 (d, *J* = 8.8 Hz, 1H), 7.36 (d, *J =* 8.8 Hz, 1H), 4.43 (s, 3H), 3.96 (s, 3H), 3.13 (s, 2H), 2.11-2.04 (m, 1H), 0.89-0.83 (m, 4H).

### Example 128

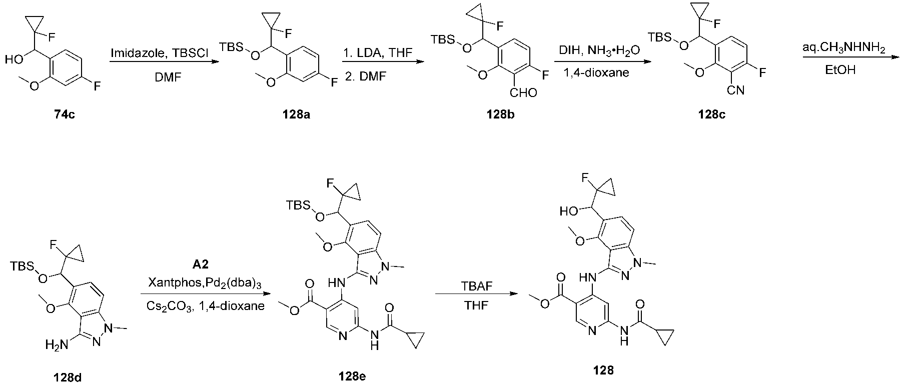

### Step 1. Tert-butyl((4-fluoro-2-methoxyphenyl) (1-fluorocyclopropyl) methoxy) dimethylsilane (128a)

To a solution of **74c** (220 mg, 1.03 mmol) and imidazole (104.9 mg, 1.54 mmol) in DMF (2 mL) was added TBSCl (232.2 mg, 1.54 mmol) at 30 °C. Then the mixture was stirred for 3 h. The mixture was diluted with *aq.* HCl (0.2 M, 1 mL) and extracted with EtOAc (10 mL*2). The organic layer was washed with brine (5 mL*3), dried over Na₂SO₄ and filtered. The filtrate was concentrated and purified by column chromatography (PE) to give **128a** (237 mg, 70% yield) as a yellow oil. ¹H NMR (400 MHz, DMSO-*d*₆) δ 7.40 (t, *J* = 7.2 Hz, 1H), 6.88 (dd, *J* = 11.6, 2.4 Hz, 1H), 6.86-6.75 (m, 1H), 5.24 (d, *J* = 15.6 Hz, 1H), 3.78 (s, 3H), 0.94-0.89 (m, 1H), 0.87-0.84 (m, 1H), 0.79 (s, 9H), 0.66-0.63 (m, 1H), 0.52-0.46 (m, 1H), 0.00 (s, 3H), -0.14 (s, 3H).

### Step 2. 3-(((Tert-butyldimethylsilyl) oxy) (1-fluorocyclopropyl) methyl)-6-fluoro-2-methoxybenzaldehyde (128b)

To a solution of **128a** (230 mg, 0.70 mmol) in THF (3 mL) was dropwise added LDA (0.7 mL, 1.4 mmol, 2 M in THF) at -50 °C under N₂ atmosphere. The mixture was stirred at - 50 °C for 1 h. Then DMF (154 mg, 2.10 mmol) was added to the mixture at -50°C and the mixture was stirred at -50 °C for 1 h. The reaction was quenched with saturated NH₄Cl (5 mL) at -50 °C and extracted with EtOAc (10 mL*2). The combined organic layer was concentrated to give the crude **128b** (249.6 mg, 100% yield) as a yellow oil which was used for the next step directly without further purification.

### Step 3. 3-(((Tert-butyldimethylsilyl) oxy) (1-fluorocyclopropyl) methyl)-6-fluoro-2-methoxybenzonitrile (128c)

To a solution **of 128b** (100 mg, 280.5 µmol) in 1,4-dioxane (0.5 mL) and ammonium hydroxide (0.5 mL) was added 1,3-diiodo-5,5-dimethylimidazolidine-2,4-dione (36.2 mg, 280.5 µmol) at 0 °C. Then the mixture was stirred at 30 °C for 3 h, TLC showed the starting material was disappeared. The mixture was diluted with water (5 mL) and extracted with EtOAc (5 mL*2). The organic layer was concentrated to give **128c** (99 mg, 100% yield) as a yellow oil which was used for the next step directly without further purification.

### Step 4. 5-(((Tert-butyldimethylsilyl) oxy) (1-fluorocyclopropyl) methyl)-4-methoxy-1-methyl-1H-indazol-3-amine (128d)

A mixture of 128c (100 mg, 282.90 µmol) and methylhydrazine (4 mL, 40% in water) in EtOH (2 mL) was stirred at 70 °C for 2 h. After cooling to room temperature, the mixture was diluted with water (5 mL) and extracted with EtOAc (10 mL*2). The combined organic layer was concentrated and purified by column chromatography (PE: EtOAc = 1:1) to give **128d** (20 mg, 19% yield) as a yellow oil. LC-MS (ESI, Method 3): t_{R}= 1.59 min, m/z (M+H)⁺ = 380.1.

### Step 5. Methyl 4-((5-(((tert-butyldimethylsilyl)oxy)(1-fluorocyclopropyl)methyl)-4-methoxy-1-methyl-1H-indazol-3-yl)amino)-6-(cyclopropanecarboxamido)nicotinate (128e)

A mixture of **128d** (20 mg, 52.7 µmol), A2 (14.8 mg, 58.0 µmol), Cs₂CO₃ (34.3 mg, 105.4 µmol), Pd₂(dba)₃ (9.7 mg, 10.54 µmol) and XantPhos (6.1 mg, 10.54 µmol) in 1,4-dioxane (0.5 mL) was stirred at 90 °C for 5 h under N₂ atmosphere. The mixture was concentrated and purified by column chromatography (DCM:MeOH = 19:1) to give the crude **128e** (20 mg, 64% yield) as a yellow solid. LC-MS (ESI, Method 3): t_{R} = 1.62 min, m/z (M+H)⁺ = 598.0.

### Step 6. Methyl 6-(cyclopropanecarboxamido)-4-((5-((1-fluorocyclopropyl)(hydroxy)methyl)-4-methoxy-1-methyl-1H-indazol-3-yl)amino)nicotinate (128)

To a solution **of 128e** (20 mg, 33.45 µmol) in THF (0.5 mL) was added TBAF (0.5 mL, 1.0 M in THF). After stirring at 15 °C for 1 h, the reaction mixture was diluted with water (2 mL) and extracted with EtOAc (5 mL*3). The combined organic layer was concentrated and purified by *Prep-TLC* (DCM:MeOH = 20:1) to give the crude product. The crude product was purified by *Prep-HPLC* (Method A) to give **128** (2.6 mg, 16% yield) as a white solid. LC-MS (ESI, Method 2): t_{R}= 1.14 min, m/z (M+H)⁺ = 484.1. ¹H NMR (400 MHz, DMSO-*d*₆) δ 10.90 (s, 1H), 10.86 (s, 1H), 9.21 (s, 1H), 8.76 (s, 1H), 7.59 (d, *J* = 8.8 Hz, 1H), 7.35 (d, *J* = 8.8 Hz, 1H), 5.69 (d, *J* = 4.8 Hz, 1H), 5.69 (dd, *J* = 14.0, 5.2 Hz, 1H), 3.95 (s, 3H), 3.92 (s, 3H), 3.86 (s, 3H), 2.00-1.83 (m, 1H), 1.00-0.74 (m, 8H).

### Example 129

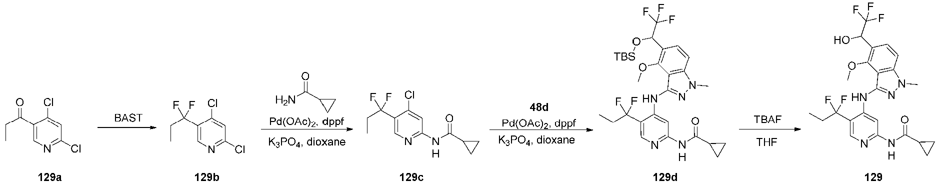

### Step 1. 2,4-Dichloro-5-(1,1-difluoropropyl)pyridine (129b)

A miture of **129a** (300 mg, 1.47 mmol) and BAST (1.63 g, 7.35 mmol) was stirred at 70 °C for 18 h. The mixture was cooled to room temperature and quenched with ice-water. Then it was basified by aq NaHCO₃, extracted with DCM (20 mL*3), washed with brine (20 mL), dried over Na₂SO₄, filtered and concentrated. The crude was purifed by column chromatography (PE:EtOAc = 6:1) to give compound 129b (200 mg, 60% yield) as a colorless oil. ¹H NMR (400 MHz, CDCl₃) δ 8.53 (s, 1H), 7.45 (s, 1H), 2.33 (tq, *J* = 16*.*8*,* 7.6 Hz, 2H), 1.01 (t, *J* = 7.6 Hz, 3H).

### Step 2. N-(4-chloro-5-(1,1-difluoropropyl)pyridin-2-yl)cyclopropanecarboxamide (129c)

A mixture of **129b** (50 mg, 0.22 mmol), cyclopropanecarboxamide (19 mg, 0.22 mmol), K₃PO₄ (94 mg, 0.44 mmol), dppf (25 mg, 0.044 mmol), Pd(OAc)₂ (5 mg, 0.022 mmol) in dioxane (1 mL) was stirred at 90 °C for 2 h under N₂ atmosphere. The mixture was concentrated and purified by column chromatography (PE:EtOAc = 3:1) to give the compound **129c** (50 mg, 82% yield) as a pale yellow solid. LC-MS (ESI, Method 4) t_{R} = 3.14 min, m/z (M+H)⁺ = 275.1.

### Step 3. N-(4-((5-(1-((tert-butyldimethylsilyl)oxy)-2,2,2-trifluoroethyl)-4-methoxy-1-methyl-1H-indazol-3-yl)amino)-5-(1,1-difluoropropyl)pyridin-2-yl)cyclopropanecarboxamide (129d)

A mixture **of 129c** (50 mg, 0.182 mmol), **48d** (71 mg, 0.182 mmol), dppf (20 mg, 0. 364 mmol), Pd(OAc)₂ (4 mg, 0.018 mmol), K₃PO₄ (77 mg, 0.364 mmol) in dioxane (1 mL) was stirred at 90 °C for 16 h under N₂ atmosphere. The mixture was concentrated and purified by column chromatography (PE:EtOAc = 1:3) to give a crude product **129d** (40 mg, 35% yield) as a yellow solid. LC-MS (ESI, Method 4) t_{R} = 4.20 min, m/z (M+H)⁺ = 628.4.

### Step 4. N-(5-(1,1-difluoropropyl)-4-((4-methoxy-1-methyl-5-(2,2,2-trifluoro-1-hydroxyethyl)-1H-indazol-3-yl)amino)pyridin-2-yl)cyclopropanecarboxamide (129)

To a mixture solution of crude product **129d** (40 mg, 0.064 mmol) in THF (1 mL) was added TBAF (0.1 mL, 0.1 mmol, 1 M in THF), then it was stirred at room temperature for 1 h. The mixture was diluted with H₂O (10 mL), extracted with EtOAc (10 mL*3), washed with brine (20 mL), dried over Na₂SO₄, filtered and concentrated. The crude was purifed by *prep-TLC* (DCM:MeOH = 30:1) to give the compound **129** (10 mg, 31% yield) as an off-white solid LC-MS (ESI, Method 4) t_{R} = 2.68 min, m/z (M+H)⁺ = 514.3. ¹H NMR (400 MHz, CDCl₃) δ 9.16 (s, 1H), 8.68 (s, 1H), 8.10 (s, 1H), 8.02 (t, *J* = 6.0 Hz, 1H), 7.56 (d, *J =* 8.8 Hz, 1H), 7.07 (d, *J =* 8.8 Hz, 1H), 5.49 (q, *J* = 6.8 Hz, 1H), 4.70-4.07 (m, 1H), 3.98 (s, 3H), 3.85 (s, 3H), 2.37-2.30 (m, 2H), 1.63-1.48 (m, 1H), 1.20-1.09 (m, 2H), 1.06 (t, *J =* 7.2 Hz, 3H), 0.92-0.83 (m, 2H).

### Example 130

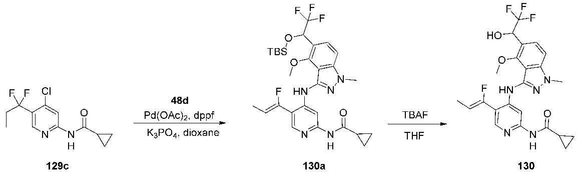

### Step 1. (Z)-N-(4-((5-(1-((tert-butyldimethylsilyl)oxy)-2,2,2-trifluoroethyl)-4-methoxy-1-methyl-1H-indazol-3-yl)amino)-5-(1-fluoroprop-1-en-1-yl)pyridin-2-yl)cyclopropanecarboxamide (130a)

A mixture of **129c** (50 mg, 0.182 mmol), **48d** (71 mg, 0.182 mmol), dppf (20 mg, 0. 364 mmol), Pd(OAc)₂ (4 mg, 0.018 mmol), K₃PO₄ (77 mg, 0.364 mmol) in dioxane (1 mL) was stirred at 90 °C for 16 h under N₂ atmosphere. The mixture was concentrated and purified by column chromatography (PE:EtOAc = 10:1 to 1:3) to give a crude product **130a** (40 mg, 35% yield) as a yellow solid. LC-MS (ESI, Method 4) t_{R} = 4.20 min, m/z (M+H)⁺ = 608.4.

### Step 2. (Z)-N-(5-(1-fluoroprop-1-en-1-yl)-4-((4-methoxy-1-methyl-5-(2,2,2-trifluoro-1-hydroxyethyl)-1H-indazol-3-yl)amino)pyridin-2-yl)cyclopropanecarboxamide (130)

To a mixture solution of crude product **130a** (40 mg, 0.064 mmol) in THF (1 mL) was added TBAF (0.1 mL, 0.1 mmol, 1 M in THF), then it was stirred at room temperature for 1 h. The mixture was diluted with H₂O (10 mL), extracted with EtOAc (10 mL*3), washed with brine (20 mL), dried over Na₂SO₄, filtered and concentrated. The crude was purifed by *prep*-TLC (DCM:MeOH = 30:1) to give the compound **130** (2 mg, 6% yield) as a pale yellow solid. LC-MS (ESI, Method 4) t_{R} = 2.60 min, m/z (M+H)⁺ = 494.3. ¹H NMR (400 MHz, DMSO-*d*₆) δ 10.64 (s, 1H), 8.47 (s, 1H), 8.09 (d, *J* = 4.4 Hz, 1H), 8.07 (d, *J* = 2.0 Hz, 1H), 7.52 (d, *J* = 8.8 Hz, 1H), 7.44 (d, *J* = 8.8 Hz, 1H), 6.84 (d, *J =* 5.6 Hz, 1H), 5.48-5.32 (m, 2H), 3.96 (s, 3H), 3.80 (s, 3H), 2.00-1.94 (m, 1H), 1.79 (dd, *J* = 6.8, 2.4 Hz, 3H), 0.82-0.70 (m, 4H).

### Example 131

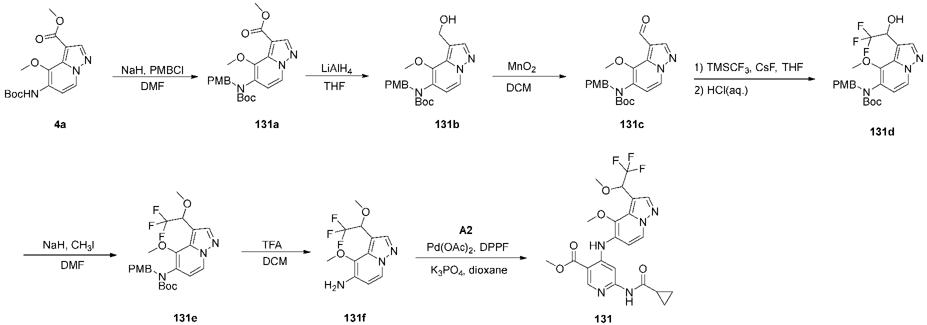

### Step 1. Methyl 5-((tert-butoxycarbonyl)(4-methoxybenzyl)amino)-4-methoxypyrazolo[1,5-a]pyridine-3-carboxylate (131a)

To a solution of **4a** (900 mg, 2.80 mmol) in DMF (25 mL) was added NaH (60% dispersion in oil) (224 mg, 5.60 mmol) at 0 °C under N₂ atmosphere. After the reaction mixture was stirred for 30 min, PMBCl (877 mg, 5.60 mmol) was added. Then the mixture was stirred at 0 °C to room temperature for another 3 h. The resulting solution was added with H₂O (20 mL) and extracted by EtOAc (30 mL), the combined organic layer was washed by brine (15 mL*3), dried over Na₂SO₄ and filtered. The filtrate was concentrated and purified by column chromatography (EtOAc:PE = 1:2) to afford **131a** (1.02 g, 82% yield) as a light-yellow oil. LC-MS (ESI, Method 4) t_{R} = 3.39 min, m/z (M+H)⁺ = 442.3.

### Step 2. Tert-butyl (3-(hydroxymethyl)-4-methoxypyrazolo[1,5-a]pyridin-5-yl)(4-methoxybenzyl)carbamate (131b)

To a solution of **131a** (1.02 g, 2.31 mmol) in THF (20 mL) was dropped with LiAlH₄ (2.31 mmol, 1 M, 2.3 mL) at 0 °C under N₂ atmosphere. The mixture was stirred at 0 °C to room temperature for 3 h. The resulting solution was quenched by NH₄Cl solution (30 mL) and extracted by EtOAc (40 mL). The combined organic layer was washed by brine (15 mL*2), dried over Na₂SO₄ and filtered. The filtrate was concentrated and purified by column chromatography (EtOAc:PE = 1:2) to afford **131b** (650 mg, 68% yield) as a white oil. LC-MS (ESI, Method 4) t_{R} = 2.88 min, m/z (M+H)⁺ = 414.3.

### Step 3. Tert-butyl (3-formyl-4-methoxypyrazolo[1,5-a]pyridin-5-yl)(4-methoxybenzyl)carbamate (131c)

To a solution of **131b** (650 mg, 1.57 mmol) in DCM (15 mL) was added MnO₂ (1.37 g, 15.72 mmol) at room temperature. The mixture was stirred at 40 °C for 6 h under N₂ atmosphere. The resulting solution was filtered, and the filtrate was concentrated to afford **131c** (500 mg, 77% yield) as a purple solid. LC-MS (ESI, Method 4) t_{R} = 3.27 min, m/z (M+H)⁺ = 412.3.

### Step 4. Tert-butyl (4-methoxy-3-(2,2,2-trifluoro-1-hydroxyethyl)pyrazolo[1,5-a]pyridin-5-yl)(4-methoxybenzyl)carbamate (131d)

To a solution of **131c** (500 mg, 1.22 mmol) in THF (15 mL) was added CsF (369 mg, 2.43 mmol) at 0 °C under N₂ atmosphere. Then trimethyl(trifluoromethyl)silane (346 mg, 2.43 mmol) was dropped slowly and the mixture was stirred at 0 °C for 2 h. Then the pH was adjusted to 2 by HCl (aqueous, 2 M) and stirred for another 2 h. The resulting solution was added H₂O (20 mL) and extracted by EtOAc (30 mL). The combined organic layer was washed by brine (15 mL*2), dried over Na₂SO₄ and filtered. The filtrate was concentrated and purified by column chromatography (EtOAc:PE = 1:2) to afford **131d** (450 mg, 77% yield) as a white solid. LC-MS (ESI, Method 4) t_{R} = 3.26 min, m/z (M+H)⁺ = 482.3.

### Step 5. Tert-butyl (4-methoxy-3-(2,2,2-trifluoro-1-methoxyethyl)pyrazolo[1,5-a]pyridin-5-yl)(4-methoxybenzyl)carbamate (131e)

To a solution **of 131d** (450 mg, 0.93 mmol) in DMF (15 mL) was added NaH (60% dispersion in oil, 75 mg, 1.87 mmol) at 0 °C under N₂ atmosphere. After the solution was stirred for 15 min, iodomethane (265 mg, 1.87 mmol) was added. And the mixture was stirred at 0 °C to room temperature for another 2 h. The resulting solution was added H₂O (20 mL) and extracted by EtOAc (30 mL), the combined organic layer was washed by brine (15 mL*3), dried over Na₂SO₄ and filtered. The filtrate was concentrated in vacuo to afford **131e** (400 mg, 86% yield) as a white oil without further purification. LC-MS (ESI, Method 4) t_{R} = 3.68 min, m/z (M+H)⁺ = 496.3.

### Step 6. 4-Methoxy-3-(2,2,2-trifluoro-1-methoxyethyl)pyrazolo[1,5-a]pyridin-5-amine (131f)

To a solution of **131e** (400 mg, 0.81 mmol) in DCM (5 mL) was added TFA (1.84 g, 16.15 mmol) at room temperature. The mixture was stirred at room temperature for 2 h. The reaction mixture was diluted with aq. Na₂CO₃ (30 mL) and extracted with EtOAc (30 mL*2). The combined organic layer was dried over anhydrous Na₂SO₄ and filtered. The filtrate was concentrated and purified by column chromatography (EtOAc:PE = 1:2) to afford **131f** (50 mg, 23% yield) as a white solid. LC-MS (ESI, Method 4) t_{R} = 2.27 min, m/z (M+H)⁺ = 276.2.

### Step 7. Methyl 6-(cyclopropanecarboxamido)-4-((4-methoxy-3-(2,2,2-trifluoro-1-methoxyethyl)pyrazolo[1,5-a]pyridin-5-yl)amino)nicotinate (131)

To a solution of **131f** (45 mg, 0.16 mmol), Pd(OAc)₂ (7 mg, 0.033 mmol), dppf (36 mg, 0.065 mmol), K₃PO₄ (87 mg, 0.41 mmol) in dioxane (2 mL) was added **A2** (42 mg, 0.16 mmol) at room temperature. The mixture was stirred at 100 °C for 3 h under N₂ atmosphere. The resulting solution was added H₂O (20 mL) and extracted by EtOAc (30 mL), the combined organic layer was washed by brine (15 mL* 2), dried over Na₂SO₄ and filtered. The filtrate was concentrated and purified by column chromatography and *Prep-HPLC* (Method D) to afford **131** (29 mg, 36% yield) as a white solid. LC-MS (ESI, Method 4) t_{R} = 2.92 min, m/z (M+H)⁺ = 494.3. ¹H NMR (400 MHz, DMSO-*d*₆) δ 10.97 (s, 1H), 9.68 (s, 1H), 8.73 (s, 1H), 8.62 (d, *J =* 7.6 Hz, 1H), 8.05 (s, 1H), 7.76 (s, 1H), 7.00 (d, *J* = 7.2 Hz, 1H), 5.37 (q, *J* = 6.8 Hz, 1H), 3.90 (s, 3H), 3.80 (s, 3H), 3.34 (s, 3H), 2.01-1.92 (m, 1H), 0.79-0.66 (m, 4H).

### Example 132

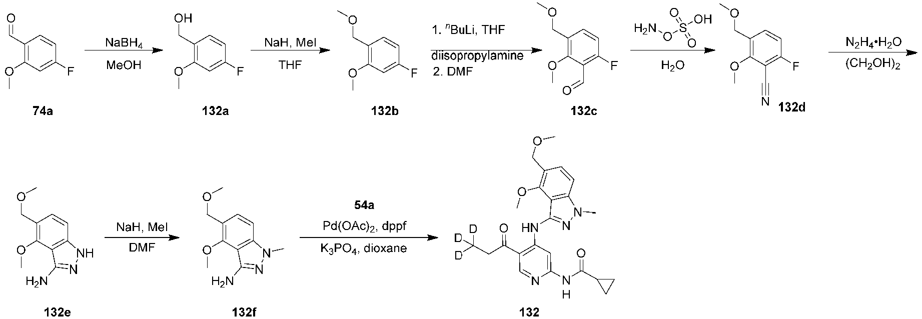

### Step 1. (4-Fluoro-2-methoxyphenyl)methanol (132a)

To a mixture of **74a** (4.50 g, 29.19 mmol) in MeOH (50 mL) was added NaBH₄ (1.21 g, 32.11 mmol) at 0 °C. The resulting mixture was stirred at 20 °C for 1 h. The reaction mixture was diluted with water (20 mL) and extracted with EtOAc (50 mL*2). The combined organic layer was washed with water (20 mL*2) and brine (20 mL*2), then it was dried over anhydrous Na₂SO₄ and filtered, the filtrate was concentrated to give **132a** (4.97 g, crude) as a colorless oil, which was used for the next step directly without further purification. ¹H NMR (400 MHz, CDCl₃) *δ* 7.22 (t, *J* = 7.2 Hz, 1H), 6.67-6.58 (m, 2H), 4.63 (s, 2H), 3.85 (s, 3H), 2.12 (s, 1H).

### Step 2. 4-Fluoro-2-methoxy-1-(methoxymethyl)benzene (132b)

To a mixture of **132a** (2.00 g, 12.81 mmol) in THF (20 mL) was added NaH (614.72 mg, 15.37 mmol, 60% in mineral oil) and CH₃I (2.73 g, 19.21 mmol, 1.20 mL) at 0 °C. The resulting mixture was stirred at 20 °C for 1 h. The reaction mixture was diluted with water (30 mL) and extracted with EtOAc (20 mL*2). The combined organic layer was washed with water (20 mL*2) and brine (20 mL*2), then it was dried over anhydrous Na₂SO₄, filtered and concentrated. The residue was purified by column chromatography (PE:EA = 99:1) to give **132b** (1.30 g, 60% yield) as a yellow solid. ¹H NMR (400 MHz, CDCl₃) *δ* 7.25-7.21 (m, 1H), 6.64-6.53 (m, 2H), 4.40 (s, 2H), 3.79 (s, 3H), 3.36 (s, 3H).

### Step 3. 6-Fluoro-2-methoxy-3-(methoxymethyl)benzaldehyde (132c)

To a mixture of diisopropylamine (1.78 g, 17.63 mmol, 2.48 mL) in THF (15 mL) was added n-BuLi (941 mg, 14.69 mmol, 5.88 mL, 2.5 M in hexane) at -78 °C under N₂ atmosphere. The mixture was stirred at -78 °C for 0.5 h. **132b** (1.00 g, 5.88 mmol) was added to the mixture and it was stirred at -78 °C for 1 h. DMF (644 mg, 8.81 mmol, 682 µL) was added to the mixture and it was stirred at -78 °C for 1 h. The reaction mixture was added to H₂O (30 mL) slowly and extracted with EtOAc (20 mL*3). The combined organics was washed with water (20 mL*2) and brine (20 mL*2), then it was dried over anhydrous Na₂SO₄, filtered and concentrated. The residue was purified by column chromatography (PE:EA = 98:2) to give **132c** (1.03 g, 88% yield) as a colorless oil. LC-MS (ESI, Method 4) t_{R} = 2.21 min, m/z (M+H)⁺ = 199.0.

### Step 4. 6-Fluoro-2-methoxy-3-(methoxymethyl)benzonitrile (132d)

To a mixture of **132c** (1.00 g, 5.05 mmol) in H₂O (10 mL) was added amino hydrogen sulfate (998 mg, 8.83 mmol). The resulting mixture was stirred at 50 °C for 16 h. The reaction mixture was diluted with water (30 mL) and extracted with DCM (20 mL*2). The combined organic layer was washed with water (20 mL*2) and brine (20 mL*2), then it was dried over anhydrous Na₂SO₄, filtered and concentrated. The residue was purified by column chromatography (PE:EA = 99:1) to give **132d** (393 mg, 40% yield) as a colorless oil. ¹H NMR (400 MHz, CDCl₃) *ð* 7.60 (dd, *J* = 8.8, 6.8 Hz, 1H), 6.93 (t, *J* = 8.4 Hz, 1H), 4.43 (s, 2H), 4.11 (s, 3H), 3.43 (s, 3H).

### Step 5. 4-Methoxy-5-(methoxymethyl)-1H-indazol-3-amine (132e)

To a mixture of **132d** (350 mg, 1.79 mmol) in ethylene glycol (2 mL), was added N₂H₄·H₂O (449 mg, 7.17 mmol, 80% in H₂O). The resulting mixture was stirred at 150 °C for 1 h under microwave. The reaction mixture was diluted with water (10 mL) and extracted with EtOAc (10 mL*2). The combined organic layer was washed with water (10 mL*2) and brine (10 mL*2), then it was dried over anhydrous Na₂SO₄, filtered and concentrated. The residue was purified by column chromatography (PE:EA = 58:42) to give **132e** (112 mg, 30% yield) as a white solid. ¹H NMR (400 MHz, CDCl₃) δ 7.33 (d, *J =* 8.4 Hz, 1H), 7.04 (d, *J =* 8.4 Hz, 1H), 4.53 (s, 2H), 3.98 (s, 3H), 3.41 (s, 3H).

### Step 6. 4-Methoxy-5-(methoxymethyl)-1-methyl-1H-indazol-3-amine (132f)

To a mixture of **132e** (100 mg, 0.48 mmol) in THF (20 mL) was added NaH (39 mg, 0.96 mmol, 60% in mineral oil) and CH₃I (82 mg, 0.58 mmol, 36 µL) at 0 °C. The resulting mixture was stirred at 20 °C for 1 h. The reaction mixture was diluted with water (10 mL) and extracted with EtOAc (10 mL*2). The combined organic layer was washed with water (10 mL*2) and brine (10 mL*2), then it was dried over anhydrous Na₂SO₄, filtered and concentrated. The residue was purified by flash chromatography (PE:EA = 68:32) to give **132f** (62 mg, 58% yield) as a yellow solid. ¹H NMR (400 MHz, CDCl₃) δ 7.33 (d, *J* = 8.4 Hz, 1H), 6.92 (d, *J* = 8.4 Hz, 1H), 4.52 (s, 2H), 3.98 (s, 3H), 3.80 (s, 3H), 3.40 (s, 3H).

### Step 7. N-(4-((4-methoxy-5-(methoxymethyl)-1-methyl-1H-indazol-3-yl)amino)-5-(propanoyl-3,3,3-d₃)pyridin-2-yl)cyclopropanecarboxamide (132)

To a solution of **132f** (10 mg, 0.045 mmol) in dioxane (0.5 mL) was added **54a** (12 mg, 0.045 mmol), dppf (10 mg, 0.02 mmol), K₃PO₄ (24 mg, 0.11 mmol) and Pd(OAc)₂(2 mg, 0.01 mmol). The reaction mixture was stirred at 100 °C for 16 h under N₂ atmosphere. The reaction mixture was concentrated in vacuo and diluted with water (10 mL), then it was extracted with EtOAc (10 mL*2). The combined organic layer was washed with water (10 mL*2) and brine (10 mL*2), then it was dried over anhydrous Na₂SO₄, filtered and concentrated. The residue was purified by *Prep-HPLC* (Method D) to give **132** (10.5 mg, 53% yield) as a grey solid. LC-MS (ESI, Method 4) t_{R} = 2.78 min, m/z (M+H)⁺ = 441.3. ¹H NMR (400 MHz, DMSO-*d*₆) δ 12.08 (s, 1H), 10.87 (s, 1H), 9.30 (s, 1H), 8.91 (s, 1H), 7.39 (d, *J* =8.4 Hz, 1H), 7.31 (d, *J =* 8.4 Hz, 1H), 4.52 (s, 2H), 3.91 (s, 3H), 3.90 (s, 3H), 3.31 (s, 3H), 3.13 (s, 2H), 2.11-2.03 (m, 1H), 0.86-0.81 (m, 4H).

### Example 133

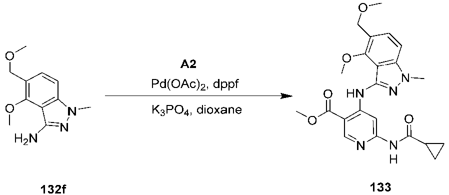

### Methyl 6-(cyclopropanecarboxamido)-4-((4-methoxy-5-(methoxymethyl)-1-methyl-1H-indazol-3-yl)amino)nicotinate (133)

To a solution of **132f** (20 mg, 0.090 mmol) in dioxane (0.5 mL) was added **A2** (23 mg, 0.090 mmol), dppf (20 mg, 0.036 mmol), K₃PO₄ (48 mg, 0.23 mmol) and Pd(OAc)₂ (4 mg, 0.018 mmol). The reaction mixture was stirred at 100 °C for 16 h under N₂ atmosphere. The reaction mixture was concentrated in vacuo and diluted with water (10 mL), then it was extracted with EtOAc (10 mL*2). The combined organic layer was washed with water (10 mL*2) and brine (10 mL*2), then it was dried over anhydrous Na₂SO₄, filtered and concentrated. The residue was purified by *Prep-HPLC* (Method D) to give **133** (19.2 mg, 48% yield) as a yellow solid. LC-MS (ESI, Method 4) t_{R} = 2.64 min, m/z (M+H)⁺ = 440.3. ¹H NMR (400 MHz, DMSO-*d*₆) δ 11.06 (s, 1H), 10.93 (s, 1H), 9.28 (s, 1H), 8.76 (s, 1H), 7.40 (d, *J =* 8.4 Hz, 1H), 7.31 (d, *J =* 8.4 Hz, 1H), 4.51 (s, 2H), 3.91 (s, 3H), 3.89 (s, 3H), 3.89 (s, 3H), 3.31 (s, 3H), 2.07-2.00 (m, 1H), 0.88-0.80 (m, 4H).

### Example 134

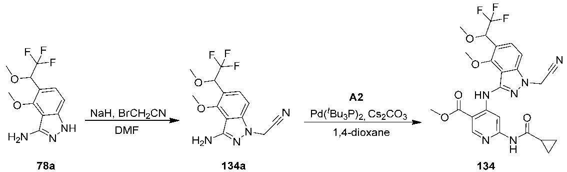

### Step 1. 2-(3-Amino-4-methoxy-5-(2,2,2-trifluoro-1-methoxyethyl)-1H-indazol-1-yl)acetonitrile (134a)

To a solution **of 78a** (200 mg, 0.727 mmol) in DMF (5 mL) was added NaH (58 mg, 1.45 mmol, 60% in mineral oil) at 0 °C. The mixture was stirred for 30 min at 25 °C. BrCH₂CN (174.3 mg, 1.45 mmol) was added to the reaction at 0 °C and the reaction mixture was stirred at 40 °C for 4 h. The mixture was diluted with H₂O (15 mL) and extracted with DCM (5 mL*3). The organic layer was washed with brine (5 mL), dried over Na₂SO₄ and filtered. The filtrate was concentrated and purified by column chromatography (PE:EA = 3:1 to 1:1) to give **134a** (150 mg, 66% yield) as a brown solid. LC-MS (ESI, Method 3): t_{R} = 1.16 min, m/z (M+H)⁺ = 315.2. ¹H NMR (400 MHz, DMSO-*d*₆) δ 7.41-7.35 (m, 2H), 5.60 (s, 2H), 5.43 (s, 2H), 5.22 (q, *J* = 6.8 Hz, 1H), 3.90 (s, 3H), 3.30 (s, 3H).

### Step 2. Methyl 4-((1-(cyanomethyl)-4-methoxy-5-(2,2,2-trifluoro-1-methoxyethyl)-1H-indazol-3-yl)amino)-6-(cyclopropanecarboxamido)nicotinate (134)

A mixture of **134a** (20 mg, 63.64 µmol), A2 (19.5 mg, 76.37 µmol), Bis(tri-tert-butylphosphine)palladium (6.5 mg, 12.73 µmol) and Cs₂CO₃ (41.5 mg, 127.2 µmol) in 1,4-dioxane (0.5 mL) was stirred at 90 °C for 2 h under N₂ atmosphere. The mixture was concentrated and purified by column chromatography (DCM:MeOH = 20:1) to give the crude product. Then the crude product was purified by *Prep-HPLC* (Method A) to give **134** (4.2 mg,12% yield) as a white solid. LC-MS (ESI, Method 2): t_{R} = 1.23 min, m/z (M+H)⁺ = 533.0. ¹H NMR (400 MHz, DMSO-*d*₆) δ 11.06 (s, 1H), 10.94 (s, 1H), 9.18 (s, 1H), 8.80 (s, 1H), 7.63 (d, *J* = 8.8 Hz, 1H), 7.54 (d, *J* = 8.8 Hz, 1H), 5.67 (s, 2H), 5.33 (q, *J* = 6.8 Hz, 1H), 3.94 (s, 3H), 3.93 (s, 3H), 3.34 (s, 3H), 2.10-2.01 (m, 1H), 0.85-0.80 (m, 4H).

### Example 135

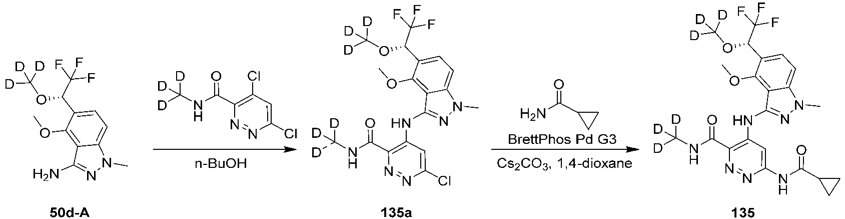

### Step 1. (S)-6-chloro-4-((4-methoxy-1-methyl-5-(2,2,2-trifluoro-1-(methoxy-d3)ethyl)-1H-indazol-3-yl)amino)-N-(methyl-d₃)pyridazine-3-carboxamide (135a)

A mixture of 50d-A (80.00 mg, 0.274 mmol) and 4,6-dichloro-N-(methyl-*d*₃)pyridazine-3-carboxamide (57.2 mg, 0.274 mmol) in n-BuOH (1 mL) was stirred at 80 °C for 10 h. The mixture was concentrated and purified by column chromatography (DCM:MeOH = 20:1) to give **135a** (95 mg, 75% yield) as a yellow solid. LC-MS (ESI, Method 3): t_{R} = 1.47 min, m/z (M+H)⁺ = 464.8.

### Step 2. (S)-6-(cyclopropanecarboxamido)-4-((4-methoxy-1-methyl-5-(2,2,2-trifluoro-1-(methoxy-d₃)ethyl)-1H-indazol-3-yl)amino)-N-(methyl-d₃)pyridazine-3-carboxamide (135)

A mixture **of 135a** (95 mg, 0.204 mmol), cyclopropanecarboxamide (69.6 mg, 0.817 mmol), BrettPhos Pd G3 (37.1 mg, 40.87 µmol) and Cs₂CO₃ (133.2 mg, 0.409 mmol) in 1,4-dioxane (1 mL) was stirred at 90 °C for 3 h under N₂ atmosphere in a sealed tube. The mixture was concentrated and purified by column chromatography (DCM:MeOH = 20:1) to give the crude product. The crude product was purified by *Prep-HPLC* (Method A) to give **135** (37.7 mg, 36% yield) as a white solid. LC-MS (ESI, Method 2): t_{R} = 1.27 min, m/z (M+H)⁺ = 514.1. ¹H NMR (400 MHz, DMSO-*d*₆) δ 12.04 (s, 1H), 11.30 (s, 1H), 9.44 (s, 1H), 9.20 (s, 1H), 7.47 (d, *J* = 8.8 Hz, 1H), 7.42 (d, *J* = 9.2 Hz, 1H), 5.31 (q, *J* = 6.8 Hz, 1H), 3.98 (s, 3H), 3.93 (s, 3H), 2.18-2.09 (m, 1H), 0.94-0.82 (m, 4H).

### Example 136

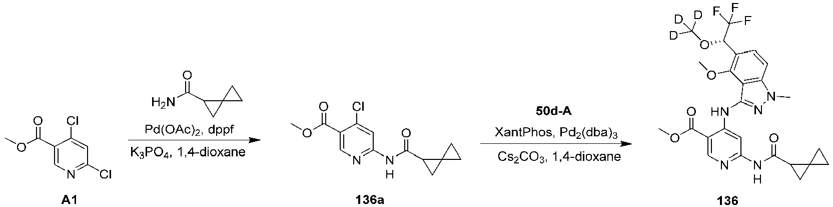

### Step 1. Methyl 4-chloro-6-(spiro[2.2]pentane-1-carboxamido)nicotinate (136a)

A mixture of **A1** (50 mg, 0.243 mmol), spiro[2.2]pentane-1-carboxamide (54 mg, 0.485 mmol), Pd(OAc)₂ (5.5 mg, 24.27 µmol), dppf (13.5 mg, 24.27 µmol) and K₃PO₄ (103 mg, 0.485 mmol) in 1,4-dioxane (0.5 mL) was stirred at 90 °C for 2 h under N₂ atmosphere. The reaction mixture was concentrated and purified by column chromatography (PE:EtOAc = 9:1) to give **136a** (20 mg, 29% yield) as a white solid. LC-MS (ESI, Method 3): t_{R} = 1.26 min, m/z (M+H)⁺ = 280.9.

### Step 2. Methyl 4-((4-methoxy-1-methyl-5-((S)-2,2,2-trifluoro-1-(methoxy-d₃)ethyl)-1H-indazol-3-yl)amino)-6-(spiro[2.2]pentane-1-carboxamido)nicotinate (136)

A mixture of **50d-A** (22.9 mg, 78.37 µmol), **136a** (20 mg, 71.25 µmol), XantPhos (8.3 mg, 14.25 µmol), Pd₂(dba)₃ (13.1 mg, 14.25 µmol) and Cs₂CO₃ (46.3 mg, 142.5 µmol) in 1,4-dioxane (0.5 mL) was stirred at 90 °C for 2 h under N₂ atmosphere. The reaction mixture was concentrated and purified by column chromatography (DCM:MeOH = 100:1) to give the crude product. The crude product was purified by *Prep-HPLC* (Method A) to give **136** (20 mg, 52% yield) as a white solid. LC-MS (ESI, Method 2): t_{R} = 1.27 min, m/z (M+H)⁺ = 537.1. ¹H NMR (400 MHz, DMSO-*d*₆) δ 10.89 (s, 1H), 10.66 (s, 1H), 9.14 (d, *J* = 2.4 Hz, 1H), 8.75 (s, 1H), 7.48 (d, *J* = 8.8 Hz, 1H), 7.42 (d, *J* = 8.8 Hz, 1H), 5.31-5.26 (m, 1H), 4.00 (s, 3H), 3.92 (s, 3H), 3.88 (s, 3H), 2.39-2.36 (m, 1H), 1.43-1.40 (m, 1H), 1.34-1.31 (m, 1H), 0.92-0.81 (m, 3H), 0.77-0.71 (m, 1H).

### Example 137

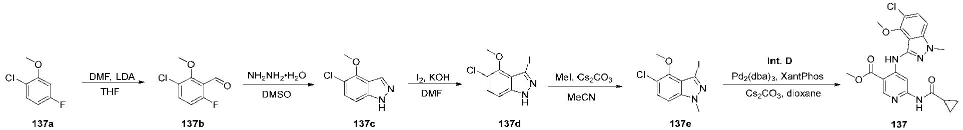

### Step 1. 3-Chloro-6-fluoro-2-methoxybenzaldehyde (137b)

To a stirred solution of **137a** (5.0 g, 31.14 mmol) in THF (50 mL) was added LDA (50 mL, 2 M, 100 mmol) dropwise at -78 °C under N₂ atmosphere. The resulting reaction mixture was stirred at -78 °C for 3 h, followed by the addition of DMF (9.1 g, 125.55 mmol). Then the final reaction mixture was stirred at -78 °C for 1 h and stirred at room temperature for 2 h. And it was quenched with 2N HCl (30 mL), extracted with EtOAc (40 mL*3). The combined organic phase was washed with brine (15 mL), dried over anhydrous Na₂SO₄ and filtered. The filtrate was concentrated to give a residue which was purified by column chromatography (EtOAc:PE = 89:11) to afford compound **137b** (3.38 g, 57% yield) as a light yellow solid. LC-MS (ESI, Method 4) t_{R} = 2.61 min, m/z (M+H)⁺ = 189.1.

### Step 2. 5-Chloro-4-methoxy-1H-indazole (137c)

A solution of **137b** (1.34 g, 31.14 mmol) and NH₂NH₂·H₂O (11 mL, 80% in water, 181.27 mmol) in DMSO (10 mL) was heated to 140°C and stirred for 48 h. Then it was diluted with water (30 mL) and extracted with EtOAc (20 mL*3). The combined organic phase was washed with brine (15 mL), dried over anhydrous Na₂SO₄ and filtered. The filtrate was concentrated to give a residue which was purified by column chromatography (MeOH:DCM = 97:3) to afford compound **137c** (120 mg, 9% yield) as a light brown solid. LC-MS (ESI, Method 4) t_{R} = 2.28 min, m/z (M+H)⁺ = 183.0.

### Step 3. 5-Chloro-3-iodo-4-methoxy-1H-indazole (137d)

To a mixture of **137c** (118 mg, 0.65 mmol) and KOH (145 mg, 2.58 mmol) in DMF (5 mL) was added I₂ (180 mg, 0.71 mmol) and the resulting reaction mixture was stirred at 50 °C for 1 h. Then it was diluted with water (15 mL) and extracted with EtOAc (15 mL*3). The combined organic phase was washed with brine (8 mL), dried over anhydrous Na₂SO₄ and filtered. The filtrate was concentrated to give a residue which was purified by column chromatography (EtOAc:PE = 2:5) to afford compound **137d** (197 mg, 98% yield) as a light yellow solid. LC-MS (ESI, Method 4) t_{R} = 2.80 min, m/z (M+H)⁺ = 309.0.

### Step 4. 5-Chloro-3-iodo-4-methoxy-1-methyl-1H-indazole (137e)

To a mixture **of 137d** (195 mg, 0.65 mmol) and Cs₂CO₃ (400 mg, 1.23 mmol) in MeCN (5 mL) was added MeI (90 mg, 0.64 mmol) and the resulting reaction mixture was stirred at 80 °C for 2 h. Then it was concentrated to give a residue which was purified by column chromatography (EtOAc:PE = 1:10) to afford compound **137e** (150 mg, 74% yield) as a white solid.

### Step 5. Methyl 4-((5-chloro-4-methoxy-1-methyl-1H-indazol-3-yl)amino)-6-(cyclopropanecarboxamido)nicotinate (137)

To a stirred mixture of **137e** (30 mg, 0.093 mmol), **Int.D** (40 mg, 0.12 mmol) and Cs₂CO₃ (61 mg, 0.19 mmol) in dioxane (2 mL) was added Pd₂(dba)₃ (8.5 mg, 0.009 mmol) and XantPhos (10.7 mg, 0.018 mmol). And the resulting reaction mixture was refluxed under N₂ atmosphere at 90 °C for 16 h. Then it was concentrated and the residue was purified by *Prep-*TLC (MeOH:DCM = 1:30) to afford a crude product, which was further purified by *Prep-HPLC* (Method D) to give **137** (9.8 mg, 25% yield) as a white solid. LC-MS (ESI, Method 4) t_{R} = 0.95 min, m/z (M+H)⁺ = 430.3. ¹H-NMR (400 MHz, DMSO-*d*₆) δ 11.08 (s, 1H), 10.94 (s, 1H), 9.26 (s, 1H), 8.77 (s, 1H), 7.45 (d, *J* = 8.8 Hz, 1H), 7.38 (d, *J* = 8.8 Hz, 1H), 4.03 (s, 3H), 3.96 (s, 3H), 3.92 (s, 3H), 2.07-2.01 (m, 1H), 0.87-0.81 (m, 4H).

### Example 138

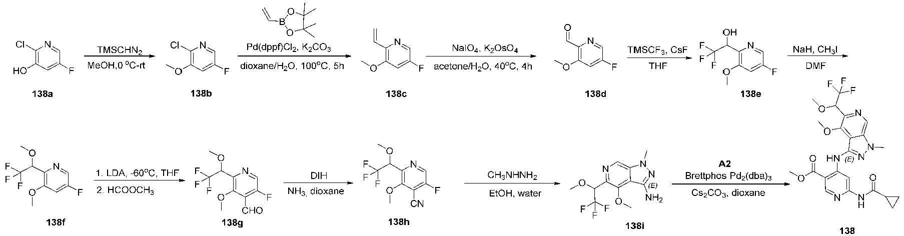

### Step 1. 2-Chloro-5-fluoro-3-methoxy-pyridine (138b)

To a solution **of 138a** (1 g, 6.78 mmol) in anhydrous MeOH (10 mL) was added TMSCHN₂ (20.4 mmol, 10.2 ml, 2 M in hexane) at 0 °C. The mixture was stirred for 4 h at 25 °C. The solvent was concentrated and purified by column chromatography (PE:EtOAc = 50:1 to 10:1) to give **138b** (370 mg, 34% yield) as a yellow oil. ¹H NMR (400 MHz, CDCl₃) δ 7.82 (d, *J* = 2.4 Hz, 1H), 6.82 (dd, *J* = 9.2 Hz, 2.4 Hz, 1H), 3.86 (s, 3H).

### Step 2. 5-Fluoro-3-methoxy-2-vinyl-pyridine (138c)

To a solution **of 138b** (370 mg, 2.29 mmol), 4,4,5,5-tetramethyl-2-vinyl-1,3,2-dioxaborolane (529 mg, 3.44 mmol), Pd(dppf)Cl₂ (168 mg, 0.229 mmol), K₂CO₃ (633 mg, 4.58 mmol) in dioxane (5 mL) and water (1 mL). The mixture was stirred for 5 h at 100 °C under N₂ atmosphere. The solvent was concentrated and purified by column chromatography (PE:EtOAc = 50:1 to 10:1) to give **138c** (280 mg, 80% yield) as yellow oil. LC-MS (ESI, Method 3): t_{R} = 1.00 min, m/z (M+H)⁺ = 154.0.

### Step 3. 5-Fluoro-3-methoxy-pyridine-2-carbaldehyde (138d)

To a solution of **138c** (280 mg, 1.83 mmol) and potassium osmate (VI) dihydrate (33.7 mg, 91.4 µmol) in acetone (5 mL) was added NaIO₄ (782.1 mg, 3.66 mmol) in H₂O (5 mL) at 0 °C and the mixture was stirred at 40 °C for 4 h. The mixture was diluted with H₂O (5 mL) and extracted with DCM (5*2 mL). The organic layer was washed with brine (2 mL), dried over Na₂SO₄ and filtered. The filtrate was concentrated to give **138d** (200 mg, 71% yield) as a brown oil. LC-MS (ESI, Method 3): t_{R} = 0.28 min, m/z (M+H)⁺ = 156.0.

### Step 4. 2,2,2-Trifluoro-1-(5-fluoro-3-methoxy-2-pyridyl)ethanol (138e)

To a solution of **138d** (200 mg, 1.29 mmol) and CsF (3.9 mg, 25.79 µmol) in anhydrous THF (5 mL) was slowly added TMSCF₃ (366.7 mg, 2.58 mmol) at 0 °C and the reaction mixture was stirred at 30 °C for 3 h. HCl (2M, 4 mL) was added to the mixture and the mixture was stirred at 30 °C for 3 h. The mixture was diluted with *sat.* NaHCO₃ (10 mL) and extracted with EtOAc (10 mL*3). The organic layer was washed with brine (10 mL), dried over Na₂SO₄ and filtered. The filtrate was concentrated to give the crude product which was purified by column chromatography (PE:EtOAc = 5:1 to 3:1) to give **138e** (140 mg, 48% yield). LC-MS (ESI, Method 3): t_{R} = 1.06 min, m/z (M+H)⁺ = 226.2. ¹H NMR (400 MHz, DMSO-*d*₆) δ 8.23 (d, *J* = 2.4 Hz, 1H), 7.62 (dd, *J* = 10.8, 2.4 Hz, 1H), 6.51 (d, *J* = 8.0 Hz, 1H), 5.43-5.35 (m, 1H), 3.89 (s, 3H).

### Step 5. 5-Fluoro-3-methoxy-2-(2,2,2-trifluoro-1-methoxy-ethyl)pyridine (138f)

To a solution of **138e** (140 mg, 0.622 mmol) in anhydrous DMF (2 mL) was added NaH (50 mg, 1.24 mmol, 60% purity in mineral oil) at 0 °C. The mixture was stirred for 30 min. CH₃I (132.4 mg, 0.933 mmol) was added and the mixture was stirred for 2 h at 0 °C. The reaction mixture was diluted with water (8 mL) and extracted with EtOAc (8 mL*3). The organic layer washed with brine (10 mL), dried over Na₂SO₄ and filtered. The filtrate was concentrated to give the crude product which was purified by column chromatography (PE:EtOAc = 2:1 to 1:1) to give **138f** (70 mg, 47% yield) as a yellow liquid. LC-MS (ESI, Method 3): t_{R} = 1.17 min, m/z (M+H)⁺ = 240.2. ¹H NMR (400 MHz, CDCl₃) δ 8.22 (s, 1H), 7.02 (dd, *J* = 9.6, 2.0 Hz, 1H), 5.19 (q, *J* = 6.4 Hz, 1H), 3.90 (s, 3H), 3.49 (s, 3H).

### Step 6. 5-Fluoro-3-methoxy-2-(2,2,2-trifluoro-1-methoxy-ethyl)pyridine-4-carbaldehyde (138g)

To a solution of **138f** (70 mg, 292.68 µmol) in anhydrous THF (2 mL) was added LDA (0.5 mL, 1 mmol, 2.0 M in THF) at -60 °C. The mixture was stirred for 1 h at -60 °C. Methyl formate (972 mg, 16.19 mmol) was added to the mixture and stirred for 1.5 h at -60 °C. The reaction was quenched with 4 M *aq.* HCl (5 mL) and extracted with EtOAc (5 mL*3). The organic layer was washed with brine (5 mL), dried over Na₂SO₄ and filtered. The filtrate was concentrated to get the crude **138g** (60 mg, 77% yield) as a yellow oil.

### Step 7. 5-Fluoro-3-methoxy-2-(2,2,2-trifluoro-1-methoxy-ethyl)pyridine-4-carbaldehyde (138h)

To a solution **of 138g** (60 mg, 224.57 µmol) in dioxane (1 mL) and NH₃·H₂O (1 mL) was added N, N'-Diiodo-5,5'-dimethylhydantoin (85.3 mg, 224.57 µmol) at 0 °C. The mixture was stirred for 8 h at 40 °C. After cooling to room temperature, the reaction mixture was diluted with *sat.* NH₄Cl (5 mL) and extracted with EtOAc (5 mL*3). The organic layer was washed with brine (5 mL) and dried over Na₂SO₄. Then it was filtered and concentrated to give **138h** (50 mg, 84% yield) as a yellow oil, which was used for the next step directly without further purification.

### Step 8. 4-Methoxy-1-methyl-5-(2,2,2-trifluoro-1-methoxy-ethyl)pyrazolo[3,4-c]pyridin-3-amine (138i)

To a solution **of 138h** (60 mg, 227.12 µmol) in EtOH (1 mL) was added CH₃NHNH₂ (1 mL, 40% in water) at room temperature. The mixture was stirred at 70 °C for 3 h. The reaction mixture was concentrated and purified by column chromatography (DCM:MeOH = 20:1) to give **138i** (15 mg, 23% yield) as a yellow solid. LC-MS (ESI, Method 3): t_{R} = 0.95 min, m/z (M+H)⁺ = 291.0.

### Step 9. Methyl 6-(cyclopropanecarbonylamino)-4-[[4-methoxy-1-methyl-5-(2,2,2-trifluoro-1-methoxy-ethyl)pyrazolo[3,4-c]pyridin-3-yl]amino]pyridine-3-carboxylate (138)

A mixture of **138i** (15 mg, 51.68 µmol), **A2** (19.7 mg, 77.52 µmol), Pd₂(dba)₃ (3 mg, 5.17 µmol), BrettPhos (5.6 mg, 10.34 µmol), Cs₂CO₃ (33.7 mg, 103.36 µmol) and anhydrous dioxane (0.5 mL) in a sealed tube was stirred for 5 h at 110 °C under N₂ atmosphere. The reaction mixture was concentrated and purified by *Prep-TLC* (PE:EtOAc = 1:1) to give the crude product, which was secondly purified by *Prep-HPLC* (Method A) to give **138** (1.5 mg, 6% yield) as a white solid. LC-MS (ESI, Method 2): t_{R} = 1.17 min, m/z (M+H)⁺ = 509.0. ¹H NMR (400 MHz, DMSO-*d*₆) δ 10.93 (s, 1H), 10.87 (s, 1H), 9.04 (s, 1H), 8.99 (s, 1H), 8.79 (s, 1H), 5.38-5.33 (m, 1H), 3.89 (s, 3H), 3.93 (s, 6H), 3.40 (s, 3H), 2.05-1.99 (m, 1H), 0.84-0.81 (m, 4H).

### Example 139

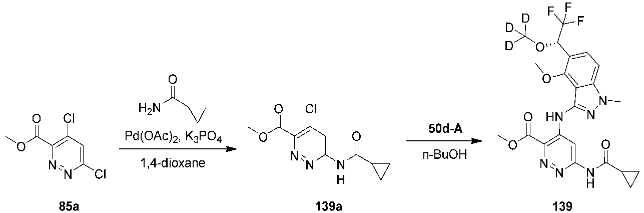

### Step 1. Methyl 4-chloro-6-(cyclopropanecarboxamido)pyridazine-3-carboxylate (139a)

To a solution **of 85a** (2 g, 9.66 mmol) and cyclopropanecarboxamide (822.2 mg, 9.66 mmol) in 1.4-dioxane (20 mL) was added K₃PO₄ (4.10 g, 19.32 mmol), Pd(OAc)₂ (216.9 mg, 0.966 mmol) and dppf (535.6 mg, 0.966 mmol). Then the mixture was stirred at 90 °C for 3 h under N₂ atmosphere. After cooling to room temperature, the reaction mixture was filtered, and the filter cake was washed with EtOAc (80 mL). The combined filtrate was washed with water (30 mL). The organic layer was concentrated and purified by column chromatography (PE:EtOAc = 3:1) to give **139a** (96 mg, 4% yield) as a yellow solid. LC-MS (ESI, Method 3): t_{R} = 0.61 min, m/z (M+H)⁺ = 256.1.

### Step 2. Methyl (S)-6-(cyclopropanecarboxamido)-4-((4-methoxy-1-methyl-5-(2,2,2-trifluoro-1-(methoxy-d₃)ethyl)-1H-indazol-3-yl)amino)pyridazine-3-carboxylate (139)

A mixture of **139a** (20 mg, 78.23 µmol) and **50d-A** (22.9 mg, 78.23 µmol) in n-BuOH (0.2 mL) was stirred at 120 °C for 3 h. The reaction mixture was concentrated and purified by column chromatography (DCM:MeOH = 20:1) to give the crude product. Then the crude product was re-purified by *Prep-HPLC* (Method A) to give **139** (11.4 mg, 28% yield) as a white solid. LC-MS (ESI, Method 2): t_{R} = 1.30 min, m/z (M+H)⁺ = 512.1. ¹H NMR (400 MHz, DMSO-*d*₆) δ 11.52 (s, 1H), 10.85 (s, 1H), 9.41 (s, 1H), 7.49 (d, *J* = 8.8 Hz, 1H), 7.43 (d, *J* = 8.8 Hz, 1H), 5.30 (q, *J* = 6.8 Hz, 1H), 4.01 (s, 3H), 3.99 (s, 3H), 3.92 (s, 3H), 2.18-2.06 (m, 1H), 0.94-0.78 (m, 4H).

### Example 140

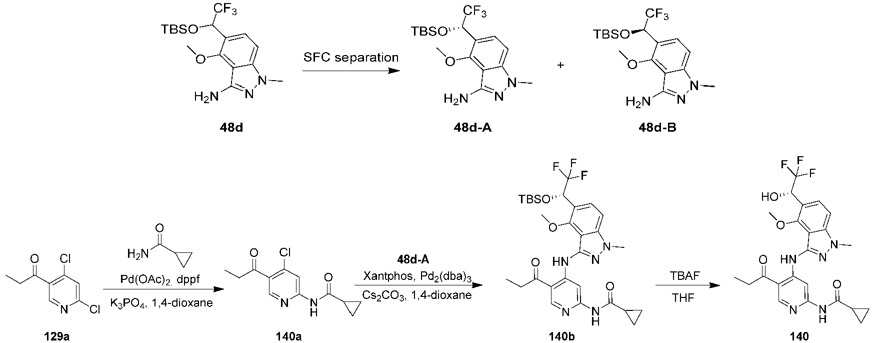

### Step 1. (S)-5-(1-((tert-butyldimethylsilyl)oxy)-2,2,2-trifluoroethyl)-4-methoxy-1-methyl-1H-indazol-3-amine (48d-A) and (R)-5-(1-((tert-butyldimethylsilyl)oxy)-2,2,2-trifluoroethyl)-4-methoxy-1-methyl-1H-indazol-3-amine (48d-B)

**48d** (240 mg, 0.177 mmol) was separated (Method H, Hex/IPA = 98/2, Flow rate: 25 mL/min) to give **48d-A** (96 mg, 40% yield) as a yellow solid and **48d-B** (106 mg, 44% yield) as a yellow solid.

**48d-A:** LC-MS (ESI, Method 3): t_{R} = 1.58 min, m/z (M+H)⁺ = 390.1. Chiral HPLC (Method 14) t_{R} = 12.11 min.

**48d-B:** LC-MS (ESI, Method 3): t_{R} = 1.57 min, m/z (M+H)⁺ = 390.1. Chiral HPLC (Method 14) t_{R} = 14.01 min.

### Step 2. N-(4-chloro-5-propionylpyridin-2-yl)cyclopropanecarboxamide (140a)

A mixture of **129a** (220 mg, 1.08 mmol), cyclopropanecarboxamide (100.9 mg, 1.19 mmol), dppf (29.9 mg, 53.91 µmol), K₃PO₄ (343.4 mg, 1.62 mmol) and Pd(OAc)₂ (12.1 mg, 53.91 µmol) in dioxane (1 mL) was stirred at 90 °C for 4 h under N₂ atmosphere. The reaction mixture was diluted with water (3 mL) and extracted with EtOAc (10 mL*3). The combined organic phase was dried over anhydrous Na₂SO₄, filtered and concentrated. The residue was purified by column chromatography (DCM:MeOH = 20:1) to give **140a** (60 mg, 22% yield) as a white solid. ¹H NMR (400 MHz, CDCl₃) δ 8.43 (s, 1H), 8.24 (s, 1H), 8.23 (s, 1H), 2.92 (q, *J* = 7.2 Hz, 2H), 1.55-1.43 (m, 1H), 1.14 (t, *J* = 7.2 Hz, 3H), 1.08-1.06 (m, 2H), 0.90-0.88 (m, 2H).

### Step 3. (S)-N-(4-((5-(1-((Tert-butyldimethylsilyl)oxy)-2,2,2-trifluoroethyl)-4-methoxy-1-methyl-1H-indazol-3-yl)amino)-5-propionylpyridin-2-yl)cyclopropanecarboxamide (140b)

A mixture of **140a** (55 mg, 0.218 mmol), **48d-A** (84.8 mg, 0.218 mmol), Cs₂CO₃ (141.8 mg, 0.435 mmol), XantPhos (25.2 mg, 43.52 µmol) and Pd₂(dba)₃ (39.9 mg, 43.52 µmol) in 1,4-dioxane (1 mL) was stirred at 90 °C under N₂ atmosphere for 3 h. The reaction mixture was filtered and concentrated to give the crude product **140b** which was used for the next step directly without further purification. LC-MS (ESI, Method 3): t_{R}= 1.58 min, m/z (M+H)⁺ = 606.0.

### Step 4. (S)-N-(4-((4-Methoxy-1-methyl-5-(2,2,2-trifluoro-1-hydroxyethyl)-1H-indazol-3-yl)amino)-5-propionylpyridin-2-yl)cyclopropanecarboxamide (140)

To a solution **of 140b** (90 mg, 0.149 mmol) in THF (1 mL) was added TBAF (1 mL, 1.0 M in THF) at 0 °C and the mixture was stirred at 25 °C for 1 h. The reaction mixture was diluted with H₂O (1 mL) and extracted with EtOAc (1 mL*2). The organic layer was concentrated and purified by column chromatography (DCM:MeOH = 20:1) to give **140** (25 mg, 34% yield) as a yellow solid. LC-MS (ESI, Method 2): t_{R} = 1.18 min, m/z (M+H)⁺ = 492.1. ¹H NMR (400 MHz, DMSO-*d*₆) δ 11.98 (s, 1H), 10.87 (s, 1H), 9.21 (s, 1H), 8.92 (s, 1H), 7.54 (d, *J* = 8.8 Hz, 1H), 7.42 (d, *J* = 8.8 Hz, 1H), 6.83 (d, *J* = 6.0 Hz, 1H), 5.48-5.44 (m, 1H), 3.96 (s, 3H), 3.87 (s, 3H), 3.15 (q, *J* = 7.2 Hz, 2H), 2.08-2.04 (m, 1H), 1.15 (t, *J* = 7.2 Hz, 3H), 0.85-0.81 (m, 4H).

### Example 141

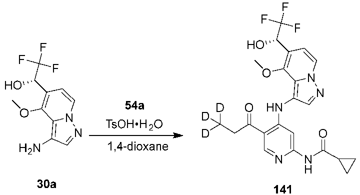

### (S)-N-(4-((4-methoxy-5-(2,2,2-trifluoro-1-hydroxyethyl)pyrazolo[1,5-a]pyridin-3-yl)amino)-5-(propanoyl-3,3,3-d₃)pyridin-2-yl)cyclopropanecarboxamide (141)

A mixture of **54a** (27 mg, 0.106 mmol), **30a** (27.6 mg, 0.106 mmol) and TsOH·H₂O (4.0 mg, 0.0211 mmol) in 1,4-dioxane (0.3 mL) was stirred at 90 °C for 3 h. The mixture was concentrated and purified by column chromatography (DCM:MeOH = 20:1) to give the crude product. The crude product was re-purified by *Prep-HPLC* (Method A) to afford **141** (14.4 mg, 28% yield) as a white solid. LC-MS (ESI, Method 2): t_{R} = 1.14 min, m/z (M+H)⁺ = 481.1. ¹H NMR (400 MHz, DMSO-*d*₆) δ 10.83 (s, 1H), 10.74 (s, 1H), 8.84 (s, 1H), 8.53 (d, *J=* 7.2 Hz, 1H), 8.15 (s, 1H), 7.71 (s, 1H), 7.03 (d, *J* = 5.6 Hz, 1H), 6.92 (d, *J* = 7.2 Hz, 1H), 5.46-5.35 (m, 1H), 3.67 (s, 3H), 3.09 (s, 2H), 2.04-1.92 (m, 1H), 0.80-0.68 (m, 4H).

### Example 142

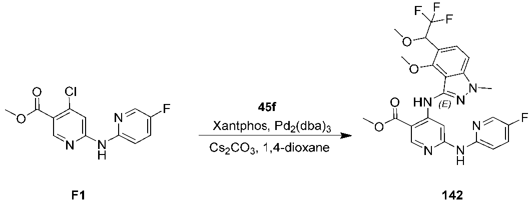

### Methyl 6-((5-fluoropyridin-2-yl)amino)-4-((4-methoxy-1-methyl-5-(2,2,2-trifluoro-1-methoxyethyl)-1H-indazol-3-yl)amino)nicotinate (142)

A mixture **of F1** (50 mg, 177.51 µmol), **45f** (51.4 mg, 177.51 µmol), Cs₂CO₃ (115.7 mg, 355.03 µmol), Pd₂(dba)₃ (32.5 mg, 35.50 µmol) and XantPhos (20.5 mg, 35.50 µmol) in 1,4-dioxane (1 mL) was stirred at 90 °C for 3 h under N₂ atmosphere. The mixture was concentrated and purified by column chromatography (DCM:MeOH = 19:1) to give the crude product. The crude compound was stirred in DMSO and MeOH (0.5 mL and 1mL) and filtrated to give **142** (35 mg, 37% yield) as a white solid. LC-MS (ESI, Method 2): t_{R} = 1.26 min, m/z (M+H)⁺ = 535.0. ¹H NMR (400 MHz, DMSO-*d*₆) δ 10.95 (s, 1H), 10.14 (s, 1H), 9.10 (s, 1H), 8.74 (s, 1H), 8.29 (d, *J* = 2.8 Hz, 1H), 7.71-7.67 (m, 1H), 7.66-7.60 (m, 1H), 7.48 (d, *J* = 8.8 Hz, 1H), 7.42 (d, *J =* 8.8 Hz, 1H), 5.30 (q, *J* = 6.8 Hz, 1H), 4.05 (s, 3H), 3.92 (s, 3H), 3.91 (s, 3H), 3.32 (s, 3H).

### Example 143

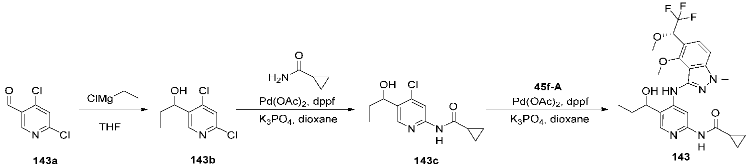

### Step 1. 1-(4,6-Dichloropyridin-3-yl)propan-1-ol (143b)

To a solution of **143a** (2 g, 11.36 mmol) in THF (20 mL) was added chloro(ethyl)magnesium (15.9 mL, 15.9 mmol, 1 M in THF) at -10 °C under N₂ atmosphere, then the mixture was stirred at 0 °C for 2 h. The mixture was quenched with aq NH₄Cl and extracted with EtOAc (10 mL*3), then it was washed with brine (20 mL), dried over Na₂SO₄ and filtered. The filtrate was concentrated and purifed by column chromatography (PE:EtOAc = 100:1 to 3:1) to give the compound **143b** (800 mg, 34% yield) as a yellow oil. LC-MS (ESI, Method 4) t_{R} = 2.46 min, m/z (M+H)⁺ = 206.0.

### Step 2. N-(4-chloro-5-(1-hydroxypropyl)pyridin-2-yl)cyclopropanecarboxamide (143c)

A mixture of **143b** (120 mg, 0.58 mmol), cyclopropanecarboxamide (50 mg, 0.58 mmol), K₃PO₄ (247 mg, 1.16 mmol), dppf (48 mg, 0.087 mmol), Pd(OAc)₂ (6 mg, 0.29 mmol) in dioxane (1.5 mL) was stirred at 90 °C for 2 h under N₂ atmosphere. The mixture was concentrated and purified by column chromatography (PE:EtOAc = 3:1) to give the compound **143c** (100 mg, 67% yield) as a pale yellow solid. LC-MS (ESI, Method 4) t_{R} = 2.26 min, m/z (M+H)⁺ = 255.1.

### Step 3. N-(5-(1-hydroxypropyl)-4-((4-methoxy-1-methyl-5-((S)-2,2,2-trifluoro-1-methoxyethyl)-1H-indazol-3-yl)amino)pyridin-2-yl)cyclopropanecarboxamide (143)

A mixture **of 143c** (100 mg, 0.39 mmol), **45f-A** (114 mg, 0.39 mol), Pd(OAc)₂ (4 mg, 0.02 mmol), K₃PO₄ (167 mg, 0.78 mmol), dppf (33 mg, 0.059 mmol) in dioxane (1.5 mL) was stirred at 95 °C for 16 h under N₂ atmosphere. The mixture was concentrated and purified by column chromatography (DCM:MeOH = 30:1) to give the crude compound and then purified by *Prep-HPLC* (Method D) to give compound **143** (80 mg, 40% yield) as a white solid. LC-MS (ESI, Method 4) t_{R} = 2.86 min, m/z (M+H)⁺=508.3. ¹H NMR (400 MHz, DMSO-*d*₆) δ 10.43 (s, 1H), 9.34 (s, 0.5H), 9.31 (s, 0.5H), 8.84 (s, 0.5H), 8.83 (0.5H), 7.87 (s, 0.5H), 7.86 (0.5H), 7.42 (d, *J* = 8.8 Hz, 1H), 7.37 (d, *J* = 8.8 Hz, 1H), 6.30-6.01 (m, 1H), 5.30-5.16 (m, 1H), 4.72-4.57 (m, 1H), 3.93 (s, 3H), 3.87 (s, 1.5H), 3.86 (s, 1.5H), 3.31 (s, 1.5H), 3.30 (s, 1.5H), 1.99-1.94 (m, 1H), 1.91-1.83 (m, 1H), 1.82-1.72 (m, 1H), 0.91-0.83 (m, 3H), 0.80-0.68 (m, 4H).

### Example 144

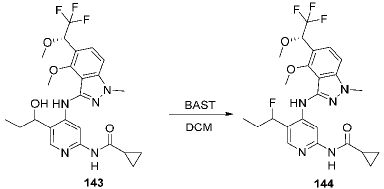

### N-(5-(1-fluoropropyl)-4-((4-methoxy-1-methyl-5-((S)-2,2,2-trifluoro-1-methoxyethyl)-1H-indazol-3-yl)amino)pyridin-2-yl)cyclopropanecarboxamide (144)

To a solution **of 143** (5 mg, 0.01 mmol) in DCM (0.5 mL) was added BAST (7 mg, 0.03 mol) at 0 °C, then the mixture was stirred at room temperature for 1 h. The mixture was diluted with ice-water, basified with aq NaHCO₃, extracted with DCM (10 mL*3), washed with brine (20 mL), dried over Na₂SO₄ and filtered. The filtrate was concentrated and purifed by *Prep*-HPLC (Method D) to give the compound **144** (1.4 mg, 28% yield) as a white solid. LC-MS (ESI, Method 4) t_{R} = 0.81 min, m/z (M+H)⁺ = 510.4. ¹H NMR (400 MHz, CDCl₃) δ 9.23 (s, 0.5H), 9.21 (s, 0.5H), 8.82 (s, 1H), 8.14 (s, 0.5 H), 8.1 (s, 0.5H), 7.89-7.87 (m, 1H), 7.51 (d, *J* = 8.8 Hz, 1H), 7.13 (d, *J* = 8.8 Hz, 1H), 5.51 (dt, *J* = 47.2, 7.2 Hz, 1H), 5.12-5.05 (m, 1H), 4.04 (s, 3H), 3.90 (s, 1.5H), 3.89 (s, 1.5H), 3.37 (s, 1.5H), 3.36 (s, 1.5H), 2.35-2.23 (m, 1H), 2.16-2.07 (m, 1H), 1.65-1.63 (m, 1H), 1.11-1.06 (m, 5H), 0.89-0.86 (m, 2H).

### Example 145

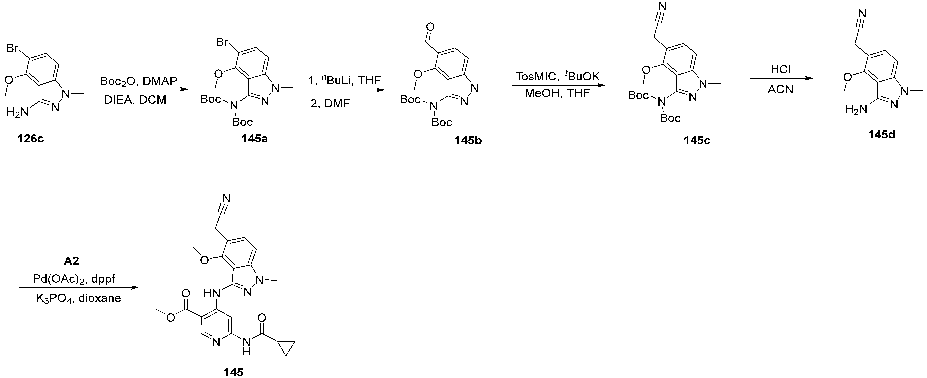

### Step 1. Tert-butyl N-(5-bromo-4-methoxy-1-methyl-indazol-3-yl)-N-tert-butoxycarbonyl-carbamate (145a)

To a solution of **126c** (800 mg, 3.12 mmol) in THF (15 mL) was added DIEA (4.04 g, 31.24 mmol, 5.44 mL), Boc₂O (3.41 g, 15.62 mmol, 3.58 mL) and DMAP (19 mg, 0.156 mmol) at 0 °C, then the mxiture was stirred at room temperature for 16 h. The mixture was diluted with H₂O (10 mL), extracted with EtOAc (10 mL*3), washed with brine (20 mL), dried over Na₂SO₄ and filtered. The filtrate was concentrated and purifed by flash chromatography (PE:EtOAc= 10:1 to 3:1) to give the compound **145a** (900 mg, 63% yield) as a white solid. LC-MS (ESI, Method 4) t_{R} = 3.70 min, m/z (M+H-100)⁺ = 356.1. ¹H NMR (400 MHz, CDCl₃) δ 7.46 (d, *J* = 8.8Hz, 1H), 7.00 (d, *J* = 8.8 Hz, 1H), 4.01 (s, 3H), 3.92(s, 3H), 1.44 (s, 18H).

### Step 2. Tert-butyl N-tert-butoxycarbonyl-N-(5-formyl-4-methoxy-1-methyl-indazol-3-yl)carbamate (145b)

To a solution of **145a** (300 mg, 0.66 mmol) in THF (5 mL) was added n-BuLi (0.32 mL, 0.79 mmol, 2.5 M in hexane) at -78 °C under N₂ atmosphere, then the mixture was stirred at -78 °C for 1 h. DMF (58 mg, 0.79 mmol) was added into the mixture at -78 °C , then the mixture was stirred at -78 °C for 1 h. The mixture was quenched with aq NH₄Cl (10 mL), extracted with EtOAc (10 mL*3), washed with brine (20 mL), dried over Na₂SO₄ and filtered. The filtrate was concentrated and purifed by column chromatography (PE:EtOAc = 100:1 to 3:1) to give the compound **145b** (150 mg, 56% yield) as yellow solid. LC-MS (ESI, Method 4) t_{R} = 1.02 min, m/z (M+H-100)⁺ = 306.2.

### Step 3. Tert-butyl N-tert-butoxycarbonyl-N-[5-(cyanomethyl)-4-methoxy-1-methyl-indazol-3-yl]carbamate (145c)

To a suspension of t-BuOK (111 mg, 0.987 mmol) in THF (2 mL) was added TosMIC (96 mg, 0.493 mmol) in THF (2 mL) at -60 °C, then the mixture was stirred at -60 °C for 15 min. A solution of **145b** (100 mg, 0.246 mmol) in THF (1 mL) was added into the mixture dropwise at -60 °C and the mixture was stirred for 1.5 hours at -60 °C. MeOH (5 mL) was added into the mixture and the mixture was stirred at 70 °C for 10 min. The mixture was concentrated and diluted with aq NH₄Cl (20 mL). Then it was extracted with EtOAc (15 mL*3), washed with brine, dried over Na₂SO₄ and concnetrated to give the compound **145c** (70 mg, 68% yield) as a yellow solid. LC-MS (ESI, Method 4) t_{R} = 1.00 min, m/z (M+H-100)⁺ = 317.0.

### Step 4. 2-(3-Amino-4-methoxy-1-methyl-1H-indazol-5-yl)acetonitrile (145d)

To a solution of 145c (70 mg, 0.168 mmol) in ACN (0.5 mL) was added HCl in dioxane (1 mL, 4 M), then the mixture was stirred at room temperature for 2h. The mixture was diluted with H₂O (10 mL), basified by aq NaHCO₃, extracted with EtOAc (10 mL*3), washed with brine (20 mL), dried over Na₂SO₄ and filtered. Then the filtrate was concentrated and purifed by column chromatography (PE:EtOAc= 10:1 to 1:1) to give the compound **145d** (20 mg, 55% yield) as a yellow solid. LC-MS (ESI, Method 4) t_{R} = 0.54 min, m/z (M+H)⁺ = 217.1.

### Step 5. Methyl 4-((5-(cyanomethyl)-4-methoxy-1-methyl-1H-indazol-3-yl)amino)-6-(cyclopropanecarboxamido)nicotinate (145)

A mixture of **145d** (20 mg, 0.092 mmol), **A2** (24 mg, 0.092 mmol), Pd(OAc)₂ (2 mg, 0.009 mmol), dppf (10 mg, 0.018 mmol), K₃PO₄ (39 mg, 0.184 mmol) in dioxane (1 mL) was stirred at 85 °C for 16 h under N₂ atmosphere. The mixture was concentrated and purified by column chromatography (PE:EtOAc = 1:2) to give the crude compound and then it was purified by *Prep-*HPLC (Method E) to give the compound **145** (6.8 mg, 17% yield) as a white solid. LC-MS (ESI, Method 4) t_{R} = 0.76 min, m/z (M+H)⁺ = 435.2. ¹H NMR (400 MHz, CDCl₃) δ 11.20 (s, 1H), 9.42 (s, 1H), 8.82 (s, 1H), 8.21 (s, 1H), 7.38 (d, *J =* 8.4 Hz, 1H), 7.09 (d, *J* = 8.4 Hz, 1H), 4.05 (s, 3H), 4.00 (s, 3H), 3.98 (s, 3H), 3.86 (s, 2H), 1.57-1.55 (m, 1H), 1.19-1.13 (m, 2H), 0.93-0.88 (m, 2H).

### Example 146

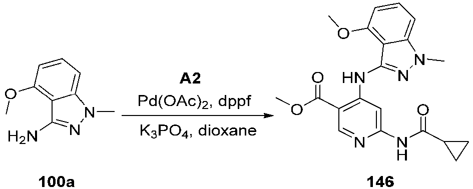

### Step 1. Methyl 6-(cyclopropanecarboxamido)-4-((4-methoxy-1-methyl-1H-indazol-3-yl)amino)nicotinate (146)

A mixture of **100a** (20 mg, 0.112 mmol), **A2** (29 mg, 0.112 mmol), Pd(OAc)₂ (2 mg, 0.009 mmol), dppf (10 mg, 0.018 mmol), K₃PO₄ (48 mg, 0.224 mmol) in dioxane (1 mL) was stirred at 85 °C for 16 h under N₂ atmosphere. The mixture was concentrated and purified by column chromatography (PE:EtOAc = 1:2) to give the crude compound, then it was purified by *Prep-HPLC* (Method E) to give the compound **146** (10 mg, 22% yield) as a white solid. LC-MS (ESI, Method 4) t_{R} = 0.82 min, m/z (M+H)⁺ = 396.2. ¹H NMR (400 MHz, CDCl₃) δ 11.37 (s, 1H), 9.51 (s, 1H), 8.79 (s, 1H), 8.33 (s, 1H), 7.27 (dd, *J* = 8.4 Hz, 7.6 Hz, 1H), 6.84 (d, *J* = 8.0 Hz, 1H), 6.40 (d, *J* = 7.6 Hz, 1H), 4.07 (s, 3H), 4.01 (s, 3H), 3.96 (s, 3H), 1.62-1.55 (m, 1H), 1.21-1.15 (m, 2H), 0.92-0.86 (m, 2H).

### Example 147

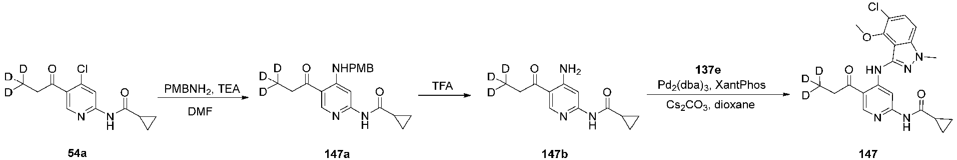

### Step 1. N-(4-((4-methoxybenzyl)amino)-5-(propanoyl-3,3,3-d₃)pyridin-2-yl)cyclopropanecarboxamide (147a)

To a stirred mixture of **54a** (65 mg, 0.25 mmol) and PMBNH₂ (68 mg, 0.50 mmol) in DMF (4 mL) was added TEA (95 mg, 0.94 mmol), and the resulting reaction mixture was stirred at 60 °C for 5 h. Then it was concentrated and purified by *Prep-TLC* (MeOH:DCM = 1:40) to afford compound **147a** (83 mg, 93% yield) as a yellow solid. LC-MS (ESI, Method 4) t_{R} = 0.68 min, m/z (M+H)⁺ = 357.2.

### Step 2. N-(4-amino-5-(propanoyl-3,3,3-d₃)pyridin-2-yl)cyclopropanecarboxamide 2,2,2-trifluoroacetate (147b)

A solution of **147a** (83 mg, 0.23 mmol) in TFA (3 mL) was stirred at room temperature for 2 h. Then it was concentrated and slurried in EtOAc and PE (1:5, 3mL). After filtration, the filter cake was washed with EtOAc and PE (1:5, 3mL) and dried to afford a pure compound **147b** (42 mg, 52% yield) as a light yellow solid. LC-MS (ESI, Method 4) t_{R} = 0.31 min, m/z (M+H)⁺ = 237.0.

### Step 3. N-(4-((5-chloro-4-methoxy-1-methyl-1H-indazol-3-yl)amino)-5-(propanoyl-3,3,3-d₃)pyridin-2-yl)cyclopropanecarboxamide (147)

To a stirred mixture of **147b** (20 mg, 0.057 mmol), **137e** (36 mg, 0.11 mmol) and Cs₂CO₃ (43 mg, 0.13 mmol) in dioxane (2 mL) was added Pd₂(dba)₃ (5 mg, 0.005 mmol) and XantPhos (6.4 mg, 0.011 mmol). And the resulting reaction mixture was stirred under N₂ atmosphere at 90 °C for 16 h. Then it was concentrated and purified by *Prep-TLC* (MeOH:DCM = 1:40) to afford a crude product, which was further purified by *Prep*-HPLC (Method D) to give compound **147** (11 mg, 45% yield) as a light yellow solid. LC-MS (ESI, Method 4) t_{R} = 0.97 min, m/z (M+H)⁺ = 431.2. ¹H NMR (400 MHz, DMSO-*d*₆) δ 12.14 (s, 1H), 10.89 (s, 1H), 9.28 (s, 1H), 8.92 (s, 1H), 7.44 (d, *J* = 8.8 Hz, 1H), 7.38 (d, *J* = 8.8 Hz, 1H), 4.05 (s, 3H), 3.95 (s, 3H), 3.13 (s, 2H), 2.10-2.04 (m, 1H), 0.87-0.82 (m, 4H).

### Example 148

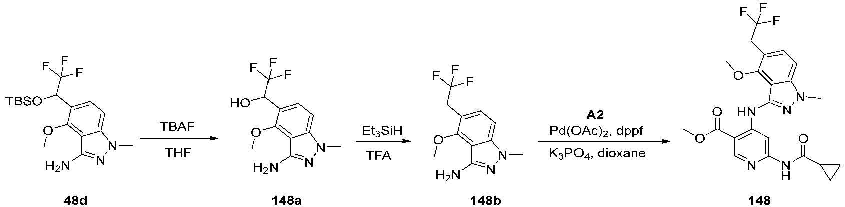

### Step 1. 1-(3-Amino-4-methoxy-1-methyl-1H-indazol-5-yl)-2,2,2-trifluoroethan-1-ol (148a)

To a solution of **48d** (200 mg, 0.51 mmol) in THF (2 mL) was added TBAF (0.5 mL, 0.5 mmol, 1 M in THF), then the reaction mixture was stirred at room temperature for 30 min. The mixture was diluted with H₂O (10 mL), extracted with EtOAc (10 mL*3), washed with brine (20 mL), dried over Na₂SO₄ and filtered. The filtrate was concentrated and purifed by column chromatography (DCM:MeOH = 100:1 to 30:1) to give the compound **148a** (140 mg, 99% yield) as a yellow solid. LC-MS (ESI, Method 4) t_{R} = 0.62 min, m/z (M+H)⁺ = 276.0.

### Step 2. 4-Methoxy-1-methyl-5-(2,2,2-trifluoroethyl)-1H-indazol-3-amine (148b)

To a solution of **148a** (100 mg, 0.36 mmol) in TFA (2 mL) was added triethylsilane (1 mL). Then the mixtue was stirred at 80 °C for 16 h. The mixture was concentrated, then it was diluted with H₂O, basified by aq NaHCO₃, extracted with EtOAc (10 mL*3), washed with brine (20 mL), dried over Na₂SO₄ and filtered, The filtrate was concentrated and purifed by column chromatography (DCM:MeOH = 100:1 to 30:1) to give the compound **148b** (24 mg, 25% yield) as a yellow solid. LC-MS (ESI, Method 4) t_{R} = 0.79 min, m/z (M+H)⁺ = 260.0.

### Step 3. Methyl 6-(cyclopropanecarboxamido)-4-((4-methoxy-1-methyl-5-(2,2,2-trifluoroethyl)-1H-indazol-3-yl)amino)nicotinate (148)

A mixture of **148b** (24 mg, 0.092 mmol), **A2** (24 mg, 0.092 mmol), dppf (10 mg, 0.029 mmol), Pd(OAc)₂ (2 mg, 0.009 mmol), K₃PO₄ (39 mg, 0.18 mmol) in 1,4-dioxane (2 mL) was stirred at 90 °C under N₂ atmosphere for 16 h. The mixture was concentrated and purified by column chromatography (DCM:MeOH = 30:1) to give crude compound, and then it was purified by *Prep-HPLC* (Method E) to give the compound **148** (15 mg, 34% yield) as a white solid. LC-MS (ESI, Method 4) t_{R}= 0.94 min, m/z (M+H)⁺ = 478.2. ¹H NMR (400 MHz, CDCl₃) δ 11.15 (s, 1H), 9.41 (s, 1H), 8.82 (s, 1H), 8.33 (s, 1H), 7.33 (d, *J* = 8.8 Hz, 1H), 7.06 (d, *J* = 8.8 Hz, 1H), 4.04 (s, 3H), 3.97 (s, 3H), 3.96 (s, 3H), 3.54 (q, *J* = 6.8 Hz, 2H), 1.60-1.54 (m, 1H), 1.18-1.14 (m, 2H), 0.94-0.86 (m, 2H).

### Example 149

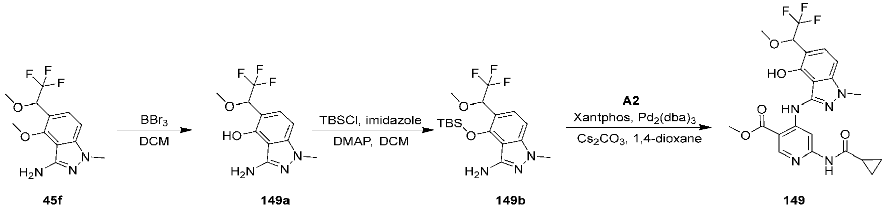

### Step 1. 3-Amino-1-methyl-5-(2,2,2-trifluoro-1-methoxyethyl)-1H-indazol-4-ol (149a)

To a solution of 45f (200 mg, 0.691 mmol) in DCM (2 mL) was added BBr₃ (2 mL, 2 mmol, 1.0 mol/L in THF) at 0 °C. Then the mixture was stirred at 30 °C for 4 h. The reaction mixture was concentrated and purified by column chromatography (DCM:MeOH = 20:1) to give **149a** (65 mg, 34% yield) as a yellow solid. LC-MS (ESI, Method 3): t_{R} = 1.14 min, m/z (M+H)⁺ = 275.8.

### Step 2. 4-((Tert-butyldimethylsilyl)oxy)-1-methyl-5-(2,2,2-trifluoro-1-methoxyethyl)-1H-indazol-3-amine (149b)

A mixture of **149a** (70 mg, 0.254 mmol), DMAP (3.1 mg, 25.4 µmol), imidazole (34.7 mg, 508.6 µmol) and TBSCl (76.7 mg, 508.6 µmol) in DCM (1 mL) was stirred at 30 °C for 4 h. The reaction mixture was diluted with water (5 mL) and extracted with DCM (6 mL*2). The combined organic layer was concentrated and purified by *Prep-HPLC* (Method A) to give **149b** (16 mg, 16% yield) as a yellow solid. LC-MS (ESI, Method 3): t_{R} = 1.04 min, m/z (M+H)⁺ = 390.2.

### Step 3. Methyl 6-(cyclopropanecarboxamido)-4-((4-hydroxy-1-methyl-5-(2,2,2-trifluoro-1-methoxyethyl)-1H-indazol-3-yl)amino)nicotinate (149)

A mixture of **149b** (16 mg, 41.08 µmol), **A2** (11.5 mg, 45.19 µmol), Cs₂CO₃ (26.8 mg, 82.16 µmol), Pd₂(dba)₃ (7.5 mg, 8.22 µmol) and XantPhos (4.8 mg, 8.22 µmol) in 1,4-dioxane (0.3 mL) was stirred at 90 °C for 2 h under N₂ atmosphere. The reaction mixture was concentrated and purified by *Prep-TLC* (DCM:MeOH = 20:1) to give the crude product. The crude product was re-purified by *Prep-HPLC* (Method A) to afford **149** (2.3 mg, 11% yield) as a yellow solid. LC-MS (ESI, Method 2): t_{R} = 1.20 min, m/z (M+H)⁺ = 494.1. ¹H NMR (400 MHz, DMSO-*d*₆) δ 11.32 (s, 1H), 10.84 (s, 1H), 10.62 (s, 1H), 9.42 (s, 1H), 8.75 (s, 1H), 7.23-7.20 (m, 1H), 7.08-7.02 (m, 1H), 5.61-5.48 (m, 1H), 3.89 (s, 6H), 3.27 (s, 3H), 2.13-1.92 (m, 1H), 0.93-0.73 (m, 4H).

### Example 150

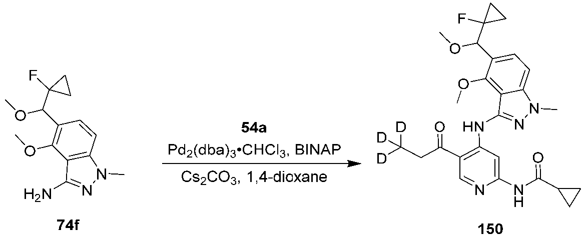

### N-(4-((5-((1-fluorocyclopropyl)(methoxy)methyl)-4-methoxy-1-methyl-1H-indazol-3-yl)amino)-5-(propanoyl-3,3,3-d₃)pyridin-2-yl)cyclopropanecarboxamide (150)

A mixture of **74f** (30 mg, 0.107 mmol), **54a** (27 mg, 0.106 mmol) (33.0 mg, 0.129 mmol), Cs₂CO₃ (70.0 mg, 0.215 mmol), BINAP (6.7 mg, 0.0107 mmol) and Pd₂(dba)₃·CHCl₃ (11.1 mg, 0.0107 mmol) in 1,4-dioxane (0.4 mL) was stirred at 110 °C for 2 h under N₂ atmosphere. The reaction mixture was concentrated and purified by column chromatography (DCM:MeOH = 20:1) to give the crude product. The crude product was slurried with ACN and MeOH (0.2 mL and 0.5 mL) to give **150** (16 mg, 30% yield) as a yellow solid. LC-MS (ESI, Method 2): t_{R} = 1.24 min, m/z (M+H)⁺ = 499.1. ¹H NMR (400 MHz, DMSO-*d*₆) δ 11.94 (s, 1H), 10.85 (s, 1H), 9.17 (s, 1H), 8.91 (s, 1H), 7.43 (d, *J* = 8.8 Hz, 1H), 7.37 (d, *J =* 8.8 Hz, 1H), 4.86 (d, *J* = 12.6 Hz, 1H), 3.96 (s, 3H), 3.86 (s, 3H), 3.23 (s, 3H), 3.13 (s, 2H), 2.10-2.01 (m, 1H), 1.11-0.89 (m, 2H), 0.88-0.70 (m, 6H).

### Example 151

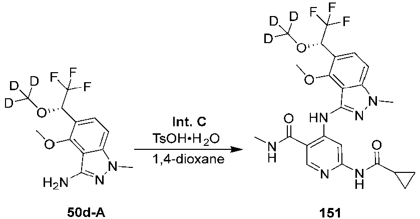

### (5)-6-(cyclopropanecarboxamido)-4-((4-methoxy-1-methyl-5-(2,2,2-trifluoro-1-(methoxy-d₃)ethyl)-1H-indazol-3-yl)amino)-N-methylnicotinamide (151)

To a solution of **50d-A** (550 mg, 1.88 mmol) and **Int. C** (454.5 mg, 1.80 mmol) in 1,4-dioxane (5 mL) was added TsOH·H₂O (35.8 mg, 0.188 mmol). The reaction mixture was stirred at 100 °C for 72 h. After the reaction mixture was cooled, it was concentrated and purified by column chromatography (DCM:MeOH = 20:1) to afford the crude product. The crude product was based with NH₃·H₂O (2 mL), Then it was re-purified by *Prep-HPLC* (Method A) to afford **151** (550 mg, 57% yield) as an off-white solid. LC-MS (ESI, Method 2): t_{R} = 1.21 min, m/z (M+H)⁺ = 510.1. ¹H NMR (400 MHz, DMSO-*d*₆) δ 11.60 (s, 1H), 10.71 (s, 1H), 9.13 (s, 1H), 8.66 (d, *J* = 4.4 Hz, 1H), 8.57 (s, 1H), 7.43 (d, *J* = 8.8 Hz, 1H), 7.39 (d, *J* = 8.8 Hz, 1H), 5.28 (q, *J* = 6.8 Hz, 1H), 3.96 (s, 3H), 3.90 (s, 3H), 2.84 (d, *J* = 4.4 Hz, 3H), 2.01-2.01 (m, 1H), 0.85-0.79 (m, 4H).

### Example 152

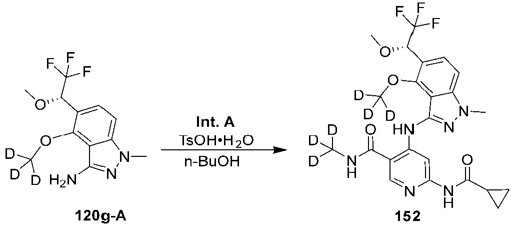

### (S)-6-(cyclopropanecarboxamido)-4-((4-(methoxy-d₃)-1-methyl-5-(2,2,2-trifluoro-1-methoxyethyl)-1H-indazol-3-yl)amino)-N-(methyl-d₃)nicotinamide (152)

To a solution of **120g-A** (20 mg, 68.43 µmol) and **Int. A** (21.1 mg, 82.12 µmol) in n-BuOH (0.2 mL) was added TsOH·H₂O (2.6 mg, 13.69 µmol) and stirred at 100 °C for 12 h. After the mixture was cooled to room temperature, the formed solid was collected by filtering and then it was purified by column chromatography (DCM:MeOH = 10:1) to give **152** (25 mg, 71% yield) as a white solid. LC-MS (ESI, Method 2): t_{R} = 1.21 min, m/z (M+H)⁺ = 513.1. ¹H NMR (400 MHz, DMSO-*d*₆) δ 11.61 (s, 1H), δ 10.70 (s, 1H), 9.13 (s, 1H), 8.63 (s, 1H), 8.57 (s, 1H), 7.43 (d, *J* = 8.8 Hz, 1H), 7.38 (d, *J* = 8.8 Hz, 1H), 5.29-5.27 (m, 1H), 3.95 (s, 3H), 3.35 (s, 3H), 2.04-2.01 (m, 1H), 0.85-0.79 (m, 4H).

### Example 153

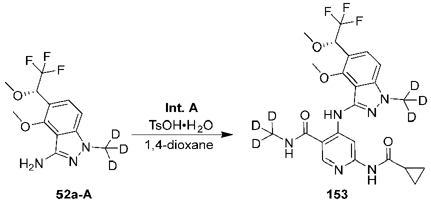

### (S)-6-(cyclopropanecarboxamido)-4-((4-methoxy-1-(methyl-d₃)-5-(2,2,2-trifluoro-1-methoxyethyl)-1H-indazol-3-yl)amino)-N-(methyl-d₃)nicotinamide (153)

A mixture of **52a-A** (15 mg, 51.32 µmol), **Int. A** (14.5 mg, 56.45 µmol) and TsOH·H₂O (2.0 mg, 10.26 µmol) in 1,4-dioxane (0.5 mL) was stirred at 100 °C for 16 h. The reaction mixture was concentrated and purified by *Prep-HPLC* (Method A) to give **153** (15.5 mg, 59% yield) as a white solid. LC-MS (ESI, Method 2): t_{R} = 1.21 min, m/z (M+H)⁺ = 513.1. ¹H NMR (400 MHz, DMSO-*d*₆) δ 11.60 (s, 1H), 10.70 (s, 1H), 9.12 (s, 1H), 8.62 (s, 1H), 8.57 (s, 1H), 7.43 (d, *J* = 8.8 Hz, 1H), 7.38 (d, *J* = 8.8 Hz, 1H), 5.28 (q, *J* = 6.8 Hz, 1H), 3.89 (s, 3H), 3.28 (s, 3H), 2.06-1.98 (m, 1H), 0.87-0.74 (m, 4H).

### Example 154

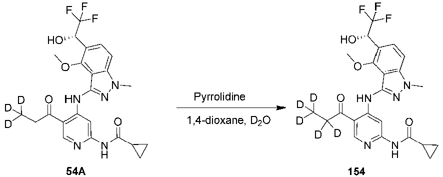

### (S)-N-(4-((4-methoxy-1-methyl-5-(2,2,2-trifluoro-1-hydroxyethyl)-1H-indazol-3-yl)amino)-5-(propanoyl-d₅)pyridin-2-yl)cyclopropanecarboxamide (154)

A solution of **54A** (25 mg, 50.56 µmol), pyrrolidine (0.4 mg, 5.06 µmol) in 1,4-dioxane and D₂O (0.5 mL and 0.5 mL) was stirred at 45 °C for 2 h. The mixture was purified by column chromatography (DCM:MeOH = 20:1) to give the crude product. The crude product was re-purified by *Prep-*HPLC (Method A) to give **154** (12 mg, 48% yield) as a yellow solid. LC-MS (ESI, Method 2): t_{R} = 1.18 min, m/z (M+H)⁺ = 497.1. ¹H NMR (400 MHz, DMSO-*d*₆) δ 11.99 (s, 1H), 10.87 (s, 1H), 9.22 (s, 1H), 8.92 (s, 1H), 7.55 (d, *J* = 8.8 Hz, 1H), 7.43 (d, *J* = 8.8 Hz, 1H), 6.84 (d, *J* = 6.4 Hz, 1H), 5.48-5.45 (m, 1H), 3.97 (s, 3H), 3.87 (s, 3H), 2.09-2.06 (m, 1H), 0.86-0.82 (m, 4H).

### Example 155

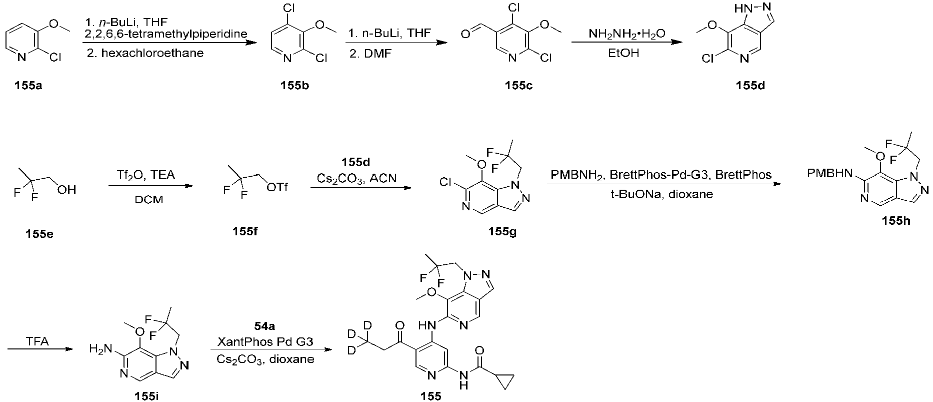

### Step 1. 2,4-Dichloro-3-methoxypyridine (155b)

To a solution of n-BuLi (5.5 mL, 13.93 mmol, 2.5 M in THF) in THF (25 mL) was added 2,2,6,6-tetramethylpiperidine (2.3 g, 16.72 mmol) at -78 °C under N₂ atmosphere. The reaction mixture was stirred at -78 °C for 15 min. Then to the reaction mixture was added a solution of **155a** (2.0 g, 13.93 mmol) in THF (25 mL). The reaction mixture was stirred at -78 °C for 4 h. A solution of hexachloroethane (6.6 g, 27.86 mmol) in THF (50 mL) was added dropwise to the reaction and the temperature was kept below -60 °C. After stirring for another 2 h at -78 °C, the reaction mixture was quenched with *sat.* NH₄Cl (20 mL), diluted with water (40 mL) and extracted with EtOAc (30 mL*3). The organic layer was washed with brine (20 mL), dried over Na₂SO₄, filtered and concentrated. The residue was purified by column chromatography (PE:EtOAc = 10:1) to afford compound 155b (700 mg, 28% yield) as a yellow oil. ¹H NMR (400 MHz, CDCl₃) δ 8.06 (d, *J* = 5.2 Hz, 1H), 7.29 (d, *J* = 5.6 Hz, 1H), 3.95 (s, 3H).

### Step 2. 4,6-Dichloro-5-methoxynicotinaldehyde (155c)

To a solution of n-BuLi (9.4 mL, 23.59 mmol, 2.5 M in THF) in THF (30 mL) was added **155b** (2.8 g, 15.73 mmol) at -78 °C. After stirring at -78 °C for 30 min, to the reaction mixture was added anhydrous DMF (1.7 g, 23.59 mmol) at -78 °C. After stirring at -78 °C for 2 h, the reaction was quenched with *sat.* NH₄Cl (20 mL) and extracted with EtOAc (100 mL). The organic layer was washed with brine (20 mL), dried over Na₂SO₄, filtered and concentrated. The residue was purified by column chromatography (PE:EtOAc = 5:1) to afford compound **155c** (2.0 g, 61% yield) as a white solid. ¹H NMR (400 MHz, DMSO-*d*₆) δ 10.25 (s, 1H), 8.60 (s, 1H), 3.93 (s, 3H).

### Step 3. 6-Chloro-7-methoxy-1H-pyrazolo[4,3-c]pyridine (155d)

A mixture **of 155c** (1.5 g, 7.28 mmol) and hydrazine hydrate (3.6 g, 21.84 mmol, 30% in water) in EtOH (20 mL) was stirred at 100 °C for 8 h. The reaction mixture was concentrated and purified by column chromatography (PE:EtOAc = 3:1) to afford compound **155d** (300 mg, 22% yield) as a white solid. ¹H NMR (400 MHz, DMSO-*d*₆) δ 13.95 (s, 1H), 8.67 (s, 1H), 8.37 (s, 1H), 4.04 (s, 3H).

### Step 4. 2,2-Difluoropropyl trifluoromethanesulfonate (155f)

To a stirred solution of **155e** (1.0 g, 10.4 mmol) and TEA (2.11 g, 20.85 mmol) in DCM (10 mL) was added Tf₂O (4.4 g, 15.60 mmol) dropwise at 0 °C, and the resulting reaction mixture was stirred at room temperature for 16 h. Then it was diluted with DCM (20 mL), washed with sat. Na₂CO₃ (20 mL) and brine (10 mL). The organic layer was dried over anhydrous Na₂SO₄ and filtrated, the filtrate was concentrated to give compound **155f** (1.3 g, crude) as a dark liquid.

### Step 5. 6-Chloro-1-(2,2-difluoropropyl)-7-methoxy-1H-pyrazolo[4,3-c]pyridine (155g)

To a mixture of **155f** (300 mg, crude) and Cs₂CO₃ (420 mg, 1.29 mmol) in MeCN (5 mL) was added **155d** (119 mg, 0.65 mmol) and the resulting reaction mixture was stirred at 80 °C for 2 h. Then it was concentrated to give a residue which was purified by column chromatography (EtOAc:PE = 1:4) to afford compound **155g** (83 mg, 49% yield) as a yellow solid. LC-MS (ESI, Method 4) t_{R} = 0.77 min, m/z (M+H)⁺ = 262.0.

### Step 6. 1-(2,2-Difluoropropyl)-7-methoxy-N-(4-methoxybenzyl)-1H-pyrazolo[4,3-c]pyridin-6-amine (155h)

To a stirred mixture of **155g** (83 mg, 0.32 mmol), PMBNH₂ (84 mg, 0.61 mmol) and t-BuONa (67 mg, 0.70 mmol) in dioxane (4 mL) was added BrettPhos Pd G3 (20 mg, 0.02 mmol) and BrettPhos (11 mg, 0.02 mmol). And the resulting reaction mixture was refluxed under N₂ atmosphere at 100 °C for 7 h. Then it was concentrated and purified by column chromatography (MeOH:DCM = 3:100) to afford compound **155h** (30 mg, 26% yield) as a yellow solid. LC-MS (ESI, Method 4) t_{R} = 0.88 min, m/z (M+H)⁺ = 363.2.

### Step 7. 1-(2,2-Difluoropropyl)-7-methoxy-1H-pyrazolo[4,3-c]pyridin-6-amine 2,2,2-trifluoroacetate (155i)

A solution of **155h** (30 mg, 0.083 mmol) in TFA (3 mL) was stirred at room temperature for 2 h. Then it was concentrated, the residue was slurried in EtOAc and PE (1:5, 3mL). After filtration, the filter cake was washed with EtOAc and PE (1:5, 3mL) and dried to afford a pure compound **155i** (22 mg, 74% yield) as a light yellow solid. LC-MS (ESI, Method 4) t_{R} = 0.35 min, m/z (M+H)⁺ = 243.2.

### Step 8. N-(4-((1-(2,2-difluoropropyl)-7-methoxy-1H-pyrazolo[4,3-c]pyridin-6-yl)amino)-5-(propanoyl-3,3,3-d₃)pyridin-2-yl)cyclopropanecarboxamide (155)

To a stirred mixture of **155h** (22 mg, 0.062 mmol) and 54a (20 mg, 0.078 mmol) in dioxane (2 mL) was added XantPhos Pd G3 (6 mg, 0.006 mmol) and Cs₂CO₃ (40 mg, 0.12 mmol), and the resulting reaction mixture was stirred under N₂ atmosphere at 100 °C for 7 h. Then it was concentrated and purified by *Prep*-TLC (MeOH:DCM = 1:40) to afford a crude product ,which was further purified by *Prep-*HPLC (Method D) to give **155** (15 mg, 50% yield) as a light yellow solid. LC-MS (ESI, Method 4) t_{R} = 0.78 min, m/z (M+H)⁺ = 462.2. ¹H NMR (400 MHz, DMSO-*d*₆) δ 12.00 (s, 1H), 10.90 (s, 1H), 9.31 (s, 1H), 8.93 (s, 1H), 8.73 (s, 1H), 8.36 (s, 1H), 4.95 (t, *J* = 13.0 Hz, 2H), 3.91 (s, 3H), 3.14 (s, 2H), 2.07 - 2.02 (m, 1H), 1.71 (t, *J* = 19.2 Hz, 3H), 0.85 - 0.80 (m, 4H).

### Example 156

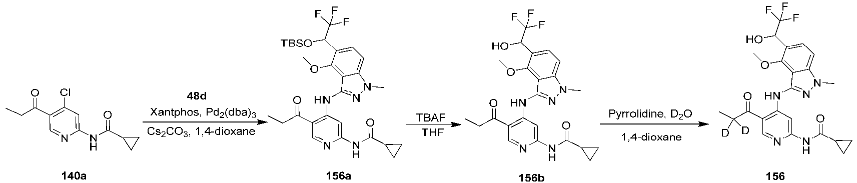

### Step 1. N-(4-((5-(1-((tert-butyldimethylsilyl)oxy)-2,2,2-trifluoroethyl)-4-methoxy-1-methyl-1H-indazol-3-yl)amino)-5-propionylpyridin-2-yl)cyclopropanecarboxamide (156a)

A mixture **of 140a** (50 mg, 0.198 mmol), **48d** (77.1 mg, 0.198 mmol), Cs₂CO₃ (128.9 mg, 0.396 mmol), XantPhos (22.9 mg, 39.57 µmol) and Pd₂(dba)₃ (36.2 mg, 39.57 µmol) in 1,4-dioxane (0.5 mL) was stirred at 90 °C under N₂ atmosphere for 3 h. After it was cooled to room temperature, the reaction mixture was filtered, and the filtrate was concentrated to give the crude product **156a** which was used for the next step directly without further purification. LC-MS (ESI, Method 3): t_{R} = 1.59 min, m/z (M+H)⁺ = 606.4.

### Step 2. N-(4-((4-methoxy-1-methyl-5-(2,2,2-trifluoro-1-hydroxyethyl)-1H-indazol-3-yl)amino)-5-propionylpyridin-2-yl)cyclopropanecarboxamide (156b)

To a solution **of 156a** (80 mg, 0.132 mmol) in THF (1 mL) was added TBAF (1 mL, 1.0 M in THF) at 0 °C and the reaction mixture was stirred at 25 °C for 1 h. The reaction mixture was diluted with H₂O (1 mL) and extracted with EtOAc (1 mL*2). The organic layer was concentrated and purified by column chromatography (DCM:MeOH = 20:1) to give **156b** (40 mg, 62% yield) as a yellow solid. LC-MS (ESI, Method 3): t_{R} = 1.23 min, m/z (M+H)⁺ = 491.9.

### Step 3. N-(4-((4-methoxy-1-methyl-5-(2,2,2-trifluoro-1-hydroxyethyl)-1H-indazol-3-yl)amino)-5-(propanoyl-2,2-d₂)pyridin-2-yl)cyclopropanecarboxamide (156)

To a solution **of 156b** (40 mg, 81.39 µmol) in 1,4-dioxane and D₂O (0.3 and 0.3 mL) was added pyrrolidine (1.2 mg, 16.28 µmol) at 25 °C and the reaction mixture was stirred at 45 °C for 4 h. After it was cooled to room temperature, the mixture was purified by *Prep*-HPLC (Method A) to give **156** (10 mg, 25% yield) as a yellow solid. LC-MS (ESI, Method 2): t_{R} = 1.18 min, m/z (M+H)⁺ = 494.1. ¹H NMR (400 MHz, DMSO-*d*₆) δ 11.98 (s, 1H), 10.87 (s, 1H), 9.21 (s, 1H), 8.92 (s, 1H), 7.54 (d, *J* = 8.8 Hz, 1H), 7.42 (d, *J* = 8.8 Hz, 1H), 6.83 (s, 1H), 5.47-5.45 (m, 1H), 3.96 (s, 3H), 3.87 (s, 3H), 2.08-2.04 (m, 1H), 1.15 (s, 3H), 0.85-0.81 (m, 4H).

### Example 157

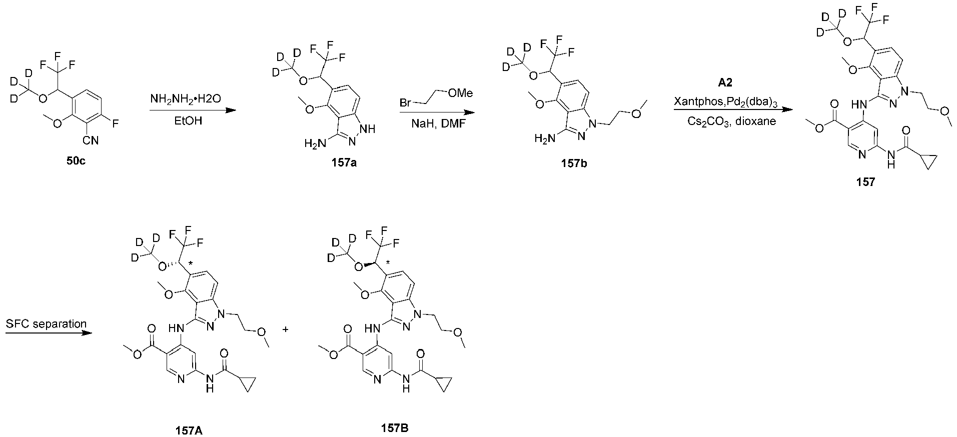

### Step 1. 4-Methoxy-5-(2,2,2-trifluoro-1-(methoxy-d₃)ethyl)-1H-indazol-3-amine (157a)

A solution of 50c (800 mg, 3.01 mmol) and NH₂NH₂·H₂O (5 mL) in EtOH (15 mL) was stirred at 90 °C for 4 h. After it was cooled to room temperature, the reaction mixture was extracted with EtOAc (10 mL*2). The combined organic phase was concentrated and purified by column chromatography (PE:EtOAc = 1:1) to give **157a** (500 mg, 60% yield) as a yellow solid. LC-MS (ESI, Method 3): t_{R} = 1.12 min, m/z (M+H)⁺ = 279.2.

### Step 2. 4-Methoxy-1-(2-methoxyethyl)-5-(2,2,2-trifluoro-1-(methoxy-d₃)ethyl)-1H-indazol-3-amine (157b)

To a solution **of 157a** (300 mg, 1.08 mmol) in DMF (3 mL) was added NaH (86.3 mg, 2.16 mmol, 60% dispersion in oil) at 0 °C. The mixture was stirred at 0 °C for 30 min. Then a solution of 1-bromo-2-methoxyethane (164.8 mg, 1.19 mmol) in DMF (0.5 mL) was added into the reaction mixture and the mixture was stirred at 20 °C for 1 h. The reaction mixture was quenched with ice-water (10 mL) and extracted with EtOAc (15 mL). The organic layer was concentrated and purified by column chromatography (PE:EtOAc = 3:1) to give **157b** (170 mg, 47% yield) as a yellow oil. LC-MS (ESI, Method 3): t_{R} = 0.84 min, m/z (M+H)⁺ = 337.2.

### Step 3. Methyl 6-(cyclopropanecarboxamido)-4-((4-methoxy-1-(2-methoxyethyl)-5-(2,2,2-trifluoro-1-(methoxy-d₃)ethyl)-1H-indazol-3-yl)amino)nicotinate (157)

A mixture **of 157b** (53 mg, 157.6 µmol), **A2** (44.2 mg, 173.3 µmol), Cs₂CO₃ (102.7 mg, 315.2 µmol), XantPhos (18.2 mg, 31.52 µmol) and Pd₂(dba)₃ (28.9 mg, 31.52 µmol) in 1,4-dioxane (0.5 mL) was stirred at 90 °C for 16 h under N₂ atmosphere. After the reaction mixture was cooled, it was diluted with water (10 mL) and extracted with EtOAc (20 mL*3). The combined organic layer was concentrated and purified by column chromatography (DCM:MeOH = 10:1) to give the crude product. The crude product was purified by *Prep-HPLC* (Method A) to give **157** (22 mg, 25% yield) as a white solid. LC-MS (ESI, Method 2): t_{R} = 1.26 min, m/z (M+H)⁺ = 555.1. ¹H NMR (400 MHz, DMSO-*d*₆) δ 10.92 (s, 1H), 10.91 (s, 1H), 9.32 (s, 1H), 8.78 (s, 1H), 7.52 (d, *J* = 8.8 Hz, 1H), 7.39 (d, *J* = 8.8 Hz, 1H), 5.29-5.27 (m, 1H), 4.45 (t, *J* = 5.2 Hz, 2H), 3.95 (t, *J* = 5.2 Hz, 2H), 3.93 (s, 3H), 3.90 (s, 3H), 3.24 (s, 3H), 2.06-2.03 (m, 1H), 0.83-0.80 (m, 4H).

### Step 4. Methyl (S*)-6-(cyclopropanecarboxamido)-4-((4-methoxy-1-(2-methoxyethyl)-5-(2,2,2-trifluoro-1-methoxyethyl)-1H-indazol-3-yl)amino)nicotinate (157A) and Methyl (R*)-6-(cyclopropanecarboxamido)-4-((4-methoxy-1-(2-methoxyethyl)-5-(2,2,2-trifluoro-1-methoxyethyl)-1H-indazol-3-yl)amino)nicotinate (157B)

157 (98 mg, 0.177 mmol) was separated by chiral *Prep-HPLC* (Method O) to give **157A** (6.6 mg, 6.7% yield) as a white solid and **157B** (21.3 mg, 22% yield) as a white solid.

157A: LC-MS (ESI, Method 2): t_{R} = 1.26 min, m/z (M+H)⁺ = 555.1. Chiral HPLC (Method 12, Hex:IPA:DEA = 80:20:0.2): t_{R} = 9.56 min. ¹H NMR (400 MHz, DMSO-*d*₆) δ 10.93 (s, 1H), 10.92 (s, 1H), 9.33 (s, 1H), 8.78 (s, 1H), 7.52 (d, *J* = 8.8 Hz, 1H), 7.39 (d, *J* = 8.8 Hz, 1H), 5.30-5.26 (m, 1H), 4.45 (t, *J* = 5.2 Hz, 2H), 3.96-3.93 (m, 5H), 3.90 (s, 3H), 3.34 (s, 3H), 2.06-2.03 (m, 1H), 0.85-0.82 (m, 4H).

**157B:** LC-MS (ESI, Method 2): t_{R} = 1.26 min, m/z (M+H)⁺ = 555.1. Chiral HPLC (Method 12, Hex:IPA:DEA = 80:20:0.2): t_{R} = 11.33 min. ¹H NMR (400 MHz, DMSO-*d*₆) δ 10.92 (s, 1H), 10.91 (s, 1H), 9.33 (s, 1H), 8.78 (s, 1H), 7.52 (d, *J* = 8.8 Hz, 1H), 7.39 (d, *J*= 8.*8* Hz, 1H), 5.29-5.26 (m, 1H), 4.45 (t, *J* = 5.2 Hz, 2H), 3.96-3.93 (m, 5H), 3.90 (s, 3H), 3.32 (s, 3H), 2.06-2.03 (m, 1H), 0.85-0.82 (m, 4H).

### Example 158

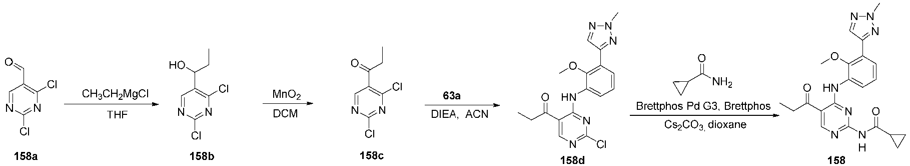

### Step 1. 1-(2,4-Dichloropyrimidin-5-yl)propan-1-ol (158b)

To a solution **of 158a** (1 g, 5.65 mmol) in THF (15 mL) was added CH₃CH₂MgCl (2 mol/L, 3.4 mL) at -78 °C under N₂ atmosphere. The mixture was stirred at -78 °C for 2 h. The resulting solution was added HCl solution (1 mol/L, 15 mL) and extracted by EtOAc (30 mL* 2), the combined organic layer was washed by brine (20 mL* 2), dried over Na₂SO₄ and filtered. The filtrate was concentrated and purified by column chromatography (EtOAc:PE = 1:1) to afford **158b** (120 mg, 10% yield) as a white oil. LC-MS (ESI, Method 4) t_{R} = 0.71 min, m/z (M+H)⁺ = 207.0.

### Step 2. 1-(2,4-Dichloropyrimidin-5-yl)propan-1-one (158c)

To a solution of **158b** (120 mg, 0.58 mmol) in DCM (10 mL) was added MnO₂ (50 mg, 0.58 mmol) at room temperature. The mixture was stirred at 43 °C for 12 h under N₂ atmosphere. The resulting solution was filtered, then the filtrate was concentrated and purified by column chromatography (EtOAc:PE = 1:2) to afford **158c** (90 mg, 76% yield) as a white oil. LC-MS (ESI, Method 4) t_{R} = 0.8 min, m/z (M+H)⁺ = 205.0.

### Step 3. 1-(2-Chloro-4-((2-methoxy-3-(2-methyl-2H-1,2,3-triazol-4-yl)phenyl)amino)pyrimidin-5-yl)propan-1-one (158d)

To a solution **of 158c** (80 mg, 0.39 mmol), **63a** (80 mg, 0.39 mmol) in ACN (8 mL) was added DIEA (101 mg, 0.78 mmol) at room temperature. The mixture was stirred at 85 °C for 2 h. The resulting solution was added H₂O (10 mL) and extracted by EtOAc (20 mL), the combined organic layer was dried over Na₂SO₄ and filtered. The filtrate was concentrated and purified by column chromatography (EtOAc:PE = 1:1) to afford **158d** (60 mg, 41% yield) as a light-yellow solid. LC-MS (ESI, Method 4) t_{R} = 1.05 min, m/z (M+H)⁺ = 373.0.

### Step 4. N-(4-((2-methoxy-3-(2-methyl-2H-1,2,3-triazol-4-yl)phenyl)amino)-5-propionylpyrimidin-2-yl)cyclopropanecarboxamide (158)

To a solution **of 158d** (45 mg, 0.12 mmol), BrettPhos Pd G3 (22 mg, 0.024 mmol), BrettPhos (26 mg, 0.048 mmol), Cs₂CO₃ (79 mg, 0.24 mmol) in dioxane (2 mL) was added cyclopropane carboxamide (51 mg, 0.6 mmol) at room temperature. The mixture was stirred at 100 °C for 3 h under N₂ atmosphere. The resulting solution was added H₂O (15 mL) and extracted by EtOAc (30 mL), the combined organic layer was dried over Na₂SO₄ and filtered. The filtrate was concentrated and purified by column chromatography (EtOAc:PE = 2:1) and *Prep*-HPLC (Method E) to afford 158 (8 mg, 16% yield) as a white solid. LC-MS (ESI, Method 4) t_{R}= 0.869 min, m/z (M+H)⁺ = 422.2 ¹H NMR (400 MHz, DMSO-*d*₆) δ 12.00 (s, 1H), 11.14 (s, 1H), 9.29 (dd, *J =* 8.0, 1.6 Hz, 1H), 9.10 (s, 1H), 8.16 (s, 1H), 7.55 (dd, *J =* 7.6, 1.2 Hz, 1H), 7.21 (t, *J* = 8.4 Hz, 1H), 4.23 (s, 3H), 3.73 (s, 3H), 3.11 (q, *J* = 7.2 Hz, 2H), 2.23-2.15 (m, 1H), 1.13 *(t, J= 7.2* Hz, 3H), 0.94-0.83 (m, 4H).

### Example 159

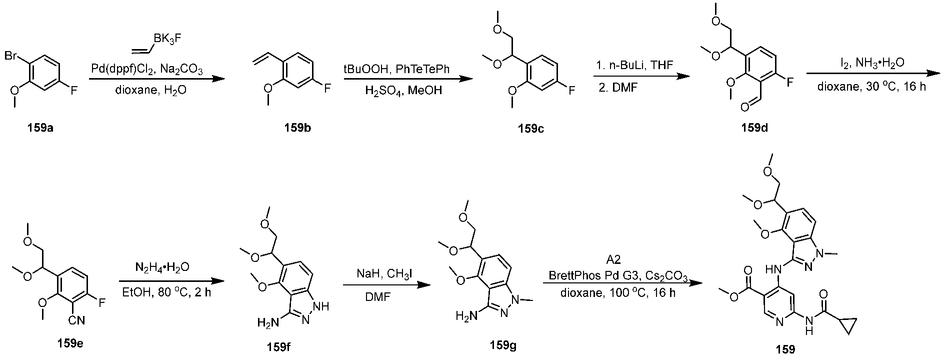

### Step 1. 4-Fluoro-2-methoxy-1-vinyl-benzene (159b)

A mixture of **159a** (2.10 g, 10.24 mmol), vinylboronic acid trifluoroborate potassium salt (1.65 g, 12.29 mmol), Na₂CO₃ (3.26 g, 10.73 mmol) and Pd(dppf)Cl₂·CH₂Cl₂ (502 mg, 0.615 mmol) in dioxane (30 mL) and water (3 mL) was stirred at 100 °C under N₂ atmosphere for 12 h. The reaction mixture was diluted with water (50 mL) and extracted with EtOAc (50 mL*3). The combined organic layer was washed with water (50 mL*3) and brine (50 mL*2), then it was dried over anhydrous Na₂SO₄, filtered and concentrated. The residue was purified by column chromatography (PE:EA = 9:1) to give **159b** (480 mg, 31% yield) as a colorless oil. ¹H NMR (400 MHz, CDCl₃): *δ* 7.41 (dd, *J* = 8.4, 6.8 Hz, 1H), 6.94 (dd, *J* = 17.6, 11.2 Hz, 1H), 6.67-6.55 (m, 2H), 5.66 (dd, *J* = 17.6, 1.6 Hz, 1H), 5.22 (dt, *J* = 11.2, 0.8 Hz, 1H), 3.83 (s, 3H).

### Step 2. 1-(1,2-Dimethoxyethyl)-4-fluoro-2-methoxy-benzene (159c)

To a mixture of **159b** (480 mg, 3.15 mmol) in MeOH (20 mL) was added diphenyl ditelluride (1.29 g, 3.15 mmol), conc. H₂SO₄ (631 mg, 6.31 mmol) and t-BuOOH (406 g, 3.15 mmol, 70% in water). The resulting mixture was stirred at 80 °C for 3 h. The reaction mixture was poured into water (50 mL) and extracted with EtOAc (30 mL*3). The combined organic layer was washed with water (50 mL*3) and brine (50 mL*2), then it was dried over anhydrous Na₂SO₄, filtered, and concentrated. The residue was purified by column chromatography (PE:EA = 95:5) to give **159c** (380 mg, 56% yield) as a colorless oil. ¹H NMR (400 MHz, CDCl₃): *δ* 7.31 (dd, *J* = 8.8, 6.8 Hz, 1H), 6.67 (dt, *J* = 8.8, 2.4 Hz, 1H), 6.60 (dd, *J* = 10.8, 2.4 Hz, 1H), 4.83 - 4.76 (m, 1H), 3.81 (s, 3H), 3.45-3.40 (m, 2H), 3.39 (s, 3H), 3.29 (s, 3H).

### Step 3. 3-(1,2-Dimethoxyethyl)-6-fluoro-2-methoxy-benzaldehyde (159d)

To a mixture **of 159c** (410 mg, 1.91 mmol) in anhydrous THF (10 mL) was added n-BuLi (1.53 mL, 3.83 mmol, 2.5M) at -60 °C under N₂ atmosphere. After the mixture was stirred for 30 min, DMF (210 mg, 2.87 mmol) was added into the above solution. The resulting mixture was stirred at -60 °C for 3 h under N₂ atmosphere. The reaction mixture was diluted with water (30 mL) and extracted with EtOAc (30 mL*3). The combined organic layer was washed with water (50 mL*3) and brine (50 mL*2), then it was dried over anhydrous Na₂SO₄, filtered and concentrated. The residue was purified by column chromatography (PE:EA = 7:3) to give **159d** (270 mg, 58% yield) as a yellow solid. HPLC (ESI, Method 4) t_{R} = 0.73 min. ¹H NMR (400 MHz, CDCl₃): *δ* 10.38 (s, 3H), 7.65 (t, *J* = 8.0 Hz, 1H), 6.99 (t, *J* = 9.2 Hz, 1H), 4.82-4.72 (m, 1H), 3.90 (s, 3H), 3.58-3.42 (m, 2H), 3.38 (s, 3H), 3.29 (s, 3H).

### Step 4. 3-(1,2-Dimethoxyethyl)-6-fluoro-2-methoxy-benzonitrile (159e)

A mixture of **159d** (270 mg, 1.11 mmol), I₂ (283 mg, 1.11 mmol) and NH₃·H₂O (4 mL, 30% in water) in dioxane (8 mL) was stirred at 20 °C for 12 h. The reaction mixture was diluted with EtOAc (50 mL) and washed with aq. Na₂S₂O₃ (50 mL*2), brine (50 mL*2), then it was dried over anhydrous Na₂SO₄, filtered and concentrated. The residue was purified by column chromatography (PE:EA = 7:3) to give **159e** (266 mg, 99% yield) as a light-yellow oil. LC-MS (ESI, Method 4) t_{R} = 0.82 min. ¹H NMR (400 MHz, CDCl₃): *δ* 7.62 (t, *J=* 8.0 Hz, 1H), 6.99 (t, *J* = 8.8 Hz, 1H), 4.75-4.67 (m, 1H), 4.13 (s, 3H), 3.56-3.40 (m, 2H), 3.37 (s, 3H), 3.28 (s, 3H).

### Step 5. 5-(1,2-Dimethoxyethyl)-4-methoxy-1H-indazol-3-amine (159f)

A mixture of **159e** (220 mg, 0.92 mmol) and N₂H₄·H₂O (2 mL, 80% in water) in ethanol (4 mL) was stirred at 80 °C for 12 h. The reaction mixture was poured into water (50 mL) and extracted with EtOAc (50 mL*3). The combined organic layer was washed with water (50 mL*3) and brine (50 mL*2), then it was dried over anhydrous Na₂SO₄, filtered and concentrated. The residue was purified by column chromatography (MeOH:DCM = 1:9) to give **159f** (160 mg, 69% yield) as a white solid. LC-MS (ESI, Method 4) t_{R} = 0.43 min, m/z (M+H)⁺ = 252.0.

### Step 6. 5-(1,2-Dimethoxyethyl)-4-methoxy-1-methyl-indazol-3-amine (159g)

To a mixture of **159f** (145 mg, 0.58 mmol) in DMF (5 mL) was added NaH (69 mg, 1.73 mmol, 60% in mineral oil) at 0 °C. The mixture was stirred for 30 min, then CH₃I (90 mg, 0.64 mmol) was added to the mixture. After stirring at 0-10 °C for 2 h, the reaction mixture was poured into water (30 mL) and extracted with EtOAc (30 mL*3). The combined organic layer was washed with water (50 mL*3) and brine (50 mL*2), then it was dried over anhydrous Na₂SO₄, filtered and concentrated. The residue was purified by column chromatography (PE:EA = 9:1 to 0:10) to give **159g** (120 mg, 78% yield) as a yellow solid. LC-MS (ESI, Method 4) t_{R} = 0.56 min, m/z (M+H)⁺ = 266.0. ¹H NMR (400 MHz, CDCl₃): *δ* 7.42 (d, *J =* 8.8 Hz, 1H), 6.99 (d, *J* = 8.8 Hz, 1H), 4.89-4.84 (m, 1H), 3.96 (s, 3H), 3.83 (s, 3H), 3.71-3.63 (m, 1H), 3.49-3.43 (m, 1H), 3.41 (s, 3H), 3.26 (s, 3H).

### Step 7. Methyl 6-(cyclopropanecarbonylamino)-4-[[5-(1,2-dimethoxyethyl)-4-methoxy-1-methyl-indazol-3-yl]amino]pyridine-3-carboxylate (159)

A mixture of **159g** (66 mg, 0.25 mmol), **A2** (51 mg, 0.20 mmol), Cs₂CO₃ (243 mg, 0.75 mmol) and BrettPhos Pd G3 (23 mg, 0.025 mmol) in dioxane (3 mL) was stirred at 100 °C under N₂ atmosphere for 12 h. The reaction mixture was diluted with water (30 mL) and extracted with EtOAc (30 mL*3). The combined organic layer was washed with water (50 mL*3), brine (50 mL*2), dried over anhydrous Na₂SO₄, filtered and concentrated. The residue was purified by column chromatography (MeOH:DCM = 1:9) and further purified by reversed-phase column chromatography (water in MeCN is 95-5%, 0.1% of HCOOH was contained in water) to give **159** (63.4 mg, 66% yield) as a white solid. LCMS (ESI, Method 4) t_{R} = 0.78 min, m/z (M+H)⁺ = 484.2. ¹H NMR (400 MHz, DMSO-*d*₆): *δ* 11.23 (s, 1H), 9.49 (s, 1H), 9.08 (brs, 1H), 8.78 (s, 1H), 7.42 (d, *J* = 8.8 Hz, 1H), 7.10 (d, *J* = 8.8 Hz, 1H), 5.00-4.94 (m, 1H), 4.04 (s, 3H), 3.98 (s, 6H), 3.71-3.63 (m, 1H), 3.52-3.46 (m, 1H), 3.41 (s, 3H), 3.27 (s, 3H), 1.69-1.59 (m, 1H), 1.19-1.11 (m, 2H), 0.95-0.87 (m, 2H).

### Example 160

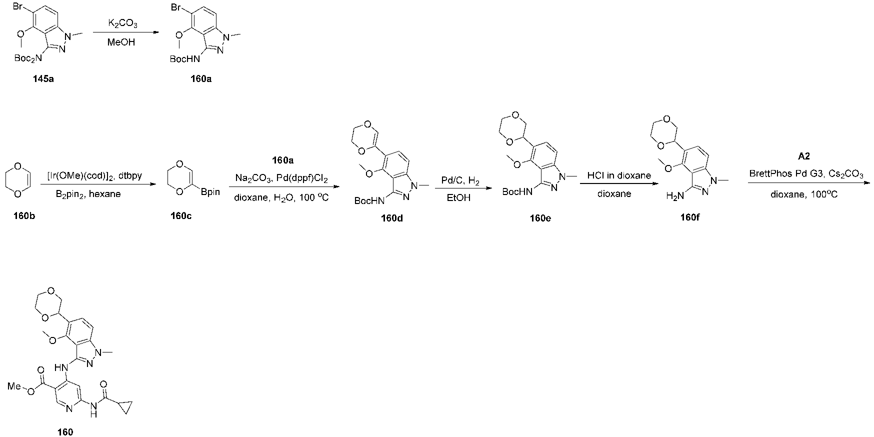

### Step 1. Tert-butyl (5-bromo-4-methoxy-1-methyl-1H-indazol-3-yl)carbamate (160a)

To a mixture of **145a** (1.10 g, 2.41 mmol) in MeOH (10 mL) was added K₂CO₃ (1.67 g, 12.05 mmol). The resulting mixture was stirred at 70 °C for 16 h under N₂ atmosphere. The reaction mixture was diluted with water (20 mL) and extracted with EtOAc (20 mL*2). The combined organic layer was washed with water (20 mL*2), brine (20 mL*2), dried over anhydrous Na₂SO₄, filtered and concentrated. The residue was purified by column chromatography (PE:EA = 89:11) to give **160a** (703 mg, 82% yield) as a white solid. LC-MS (ESI, Method 4) t_{R} = 0.95 min, m/z (M+H)⁺= 356.0.

### Step 2. 2-(5,6-Dihydro-1,4-dioxin-2-yl)-4,4,5,5-tetramethyl-1,3,2-dioxaborolane (160c)

To a solution of **160b** (500 mg, 5.81 mmol), B₂pin₂ (1.47 g, 5.81 mmol) in Heptane (30 mL) was added [Ir(OMe)(cod)]₂ (192.49 mg, 290.40 µmol), dtbpy (155.88 mg, 580.79 µmol), the mixture was stirred at 25 °C for 16 h under N₂ atmosphere. The reaction mixture was purified directly by column chromatography (PE:EtOAc = 9:1) to give **160c** (460 mg, 37% yield) as a colorless oil. LC-MS (ESI, Method 4) t_{R} = 0.82 min, m/z (M+H)⁺ = 213.0. ¹H NMR (400 MHz, Chloroform-*d*) δ 6.56 (s, 1H), 4.16-4.07 (m, 2H), 4.07-4.00 (m, 2H), 1.26 (s, 12H).

### Step 3. Tert-butyl (5-(5,6-dihydro-1,4-dioxin-2-yl)-4-methoxy-1-methyl-1H-indazol-3-yl)carbamate (160d)

To a solution of **160c** (133.94 mg, 631.64 µmol), **160a** (150 mg, 421.09 µmol), Pd(dppf)Cl₂ (68.78 mg, 84.22 µmol) in dioxane (3 mL) and water (0.5 mL) was added Na₂CO₃ (89.26 mg, 842.19 µmol, 35.28 µL), the mixture was stirred at 100 °C for 2 h under N₂ atmosphere. The reaction mixture was diluted with water (30 mL) and extracted with EtOAc (30 mL*2). The combined organic layer was dried over anhydrous Na₂SO₄, filtered and concentrated. The residue was purified by column chromatography (PE:EtOAc = 6:4) to give **160d** (127 mg, 83% yield) as a yellow oil. LC-MS (ESI, Method 4) t_{R}=0.89 min, m/z (M+H)⁺= 362.0.

### Step 4. Tert-butyl (5-(1,4-dioxan-2-yl)-4-methoxy-1-methyl-1H-indazol-3-yl)carbamate (160e)

To a solution of **160d** (120 mg, 332.05 µmol) in ethanol (3 mL) was added palladium on carbon (35.34 mg, 10% purity), the mixture was stirred at 20 °C for 2 h under H₂ atmosphere. The reaction mixture was filtered and concentrated. The residue was used directly in next step without further purification. LC-MS (ESI, Method 4) t_{R}= 0.82 min, m/z (M+H)⁺= 364.0.

### Step 5. 5-(1,4-Dioxan-2-yl)-4-methoxy-1-methyl-1H-indazol-3-amine (160f)

To a solution of 160e (130 mg, crude in last step) in dioxane (5 mL) was added HCl in dioxane (4 mol/L, 5 mL), the mixture was stirred at 20 °C for 3 h. The reaction mixture was concentrated. The residue was purified by column chromatography (DCM:MeOH = 95:5) to give **160f** (75 mg, 79% yield) as a brown oil. LC-MS (ESI, Method 4) t_{R}=0.58 min, m/z (M+H)⁺=264.0.

### Step 6. Methyl 4-((5-(1,4-dioxan-2-yl)-4-methoxy-1-methyl-1H-indazol-3-yl)amino)-6-(cyclopropanecarboxamido)nicotinate (160)

To a solution of **A2** (101.56 mg, 398.80 µmol), **160f** (70 mg, 265.86 µmol), Cs₂CO₃ (259.87 mg, 797.59 µmol) in dioxane (5 mL) was added BrettPhos Pd G3 (48.20 mg, 53.17 µmol), the mixture was stirred at 100 °C for 12 h under N₂ atmosphere. The reaction mixture was diluted with water (30 mL) and extracted with EtOAc (30 mL*2). The combined organic layer was dried over anhydrous Na₂SO₄, filtered and concentrated. The residue was purified by column chromatography (DCM:MeOH = 90:10) and *prep-HPLC* (Method D) to give **160** (8.7 mg, 6.8% yield) as a white solid. LC-MS (ESI, Method 4) t_{R}=0.79 min, m/z (M+H)⁺= 482.2. ¹H NMR (400 MHz, DMSO-*d*₆) δ 10.91 (s, 1H), 10.86 (s, 1H), 9.18 (s, 1H), 8.73 (s, 1H), 7.44 (d, *J* = 8.8 Hz, 1H), 7.33 (d, *J =* 8.8 Hz, 1H), 4.89 (dd, *J =* 10.0, 2.8 Hz, 1H), 3.91 (s, 3H), 3.89 (s, 3H), 3.85 (s, 3H), 3.82-3.58 (m, 5H), 3.54-3.44 (m, 1H), 2.01-1.99 (m, 1H), 0.93-0.70 (m, 4H).

### Example 161

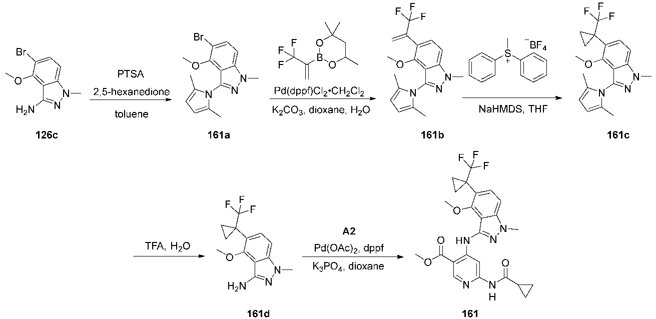

### Step 1. 5-Bromo-3-(2,5-dimethyl-1H-pyrrol-1-yl)-4-methoxy-1-methyl-1H-indazole (161a)

To a solution **of 126c** (1.0 g, 3.90 mmol) in toluene (15 mL) was added TsOH (67 mg, 0.39 mmol) and 2,5-hexanedione (535 mg, 4.69 mmol). Then the mixture was stirred at 110 °C for 16 h. The mixture was concentrated and diluted with EtOAc (50 mL), then it was washed with aq. NaHCO₃ (15 mL) and brine (20 mL), dried over Na₂SO₄, finally it was concentrated and purified by chromatography column (PE:EtOAc =100:1 to 4:1) to give the compound **161a** (1.2 g, 92% yield) as a gray solid. LC-MS (ESI, Method 4) t_{R} = 2.21 min, m/z (M+H)⁺ = 334.0.

### Step 2. 3-(2,5-Dimethyl-1H-pyrrol-1-yl)-4-methoxy-1-methyl-5-(3,3,3-trifluoroprop-1-en-2-yl)-1H-indazole (161b)

A mixture of **161a** (450 mg, 1.35 mmol), 4,4,6-trimethyl-2-(3,3,3-trifluoroprop-1-en-2-y1)-1,3,2-dioxaborinane (389 mg, 1.75 mmol), Pd(dppf)Cl₂·CH₂Cl₂ (110 mg, 0.135 mmol), K₂CO₃ (372 mg, 2.69 mmol) in dioxane (4 mL) and H₂O (1 mL) was stirred at 100 °C for 8 h under N₂ atmosphere. The mixture was concentrated and purified by chromatography column (PE:EtOAc = 100:1 to 10:1) to give the compound **161b** (466 mg, 99% yield) as a yellow solid. LC-MS (ESI, Method 4) t_{R} = 2.31 min, m/z (M+H)⁺ = 350.0. ¹H NMR (400 MHz, CDCl₃) δ 7.30 (d, *J* = 8.8 Hz, 1H), 7.17 (d, *J* = 8.8 Hz, 1H), 6.13 (q, *J* = 1.2 Hz, 1H), 5.91 (s, 2H), 5.65 (q, *J* = 1.2 Hz, 1H), 4.09 (s, 3H), 3.25 (s, 3H), 2.08 (s, 6H).

### Step 3. 3-(2,5-Dimethyl-1H-pyrrol-1-yl)-4-methoxy-1-methyl-5-(1-(trifluoromethyl)cyclopropyl)-1H-indazole (161c)

To a solution of **161b** (100 mg, 0.29 mmol) and methyldiphenylsulfonium tetrafluoroborate (107 mg, 0.37 mmol) in THF (2 mL) was added NaHMDS (0.46 mmol, 0.23 mL, 2 M in THF) at 0 °C, then the mixture was stirred at room temperature for 3 h. The mixture was diluted with aq. NH₄Cl (15 mL) and extracted with EtOAc (15 mL*3), then it was washed with brine (15 mL), dried over Na₂SO₄, concentrated and purified by chromatography column (PE:EtOAc = 100:1 to 10:1) to give the compound **161c** (100 mg, 96% yield) as a yellow oil. LC-MS (ESI, Method 4) t_{R} = 2.41 min, m/z (M+H)⁺ = 364.0.

### Step 4. 4-Methoxy-1-methyl-5-(1-(trifluoromethyl)cyclopropyl)-1H-indazol-3-amine (161d)

A solution of **161c** (100 mg, 0.27 mmol) in TFA and H₂O (1.5 mL, 1:2) was stirred at 110 °C for 16 h. The mixture was diluted with H₂O (10 mL) and basified by aq. NaHCO₃, then it was extracted with EtOAc, washed with brine, dried over Na₂SO₄, concentrated and purified by chromatography column (PE:EtOAc = 20:1 to 1:2) to give the compound **161d** (30 mg, 38% yield) as a yellow solid. LC-MS (ESI, Method 4,) t_{R} = 0.92 min, m/z (M+H)⁺ = 286.2.

### Step 5. Methyl 6-(cyclopropanecarboxamido)-4-((4-methoxy-1-methyl-5-(1-(trifluoromethyl)cyclopropyl)-1H-indazol-3-yl)amino)nicotinate (161)

A mixture of **161d** (16 mg, 0.056 mmol), **A2** (16 mg, 0.062 mmol), dppf (6 mg, 0.011 mmol), Pd(OAc)₂ (1 mg, 0.005 mmol), K₃PO₄ (24 mg, 0.11 mmol) was stirred at 90 °C for 16 h under N₂ atmosphere. The mixture was concentrated and purified by chromatography column (DCM:MeOH = 30:1) to get crude compound and then it was purified by *prep-HPLC* (Method D) to give the compound **161** (6 mg, 21% yield) as a white solid. LC-MS (ESI, Method 4) t_{R} = 1.91 min, m/z (M+H)⁺ = 504.2. ¹H NMR (400 MHz, CDCl₃) δ 11.20 (s, 1H), 9.64-9.37 (m, 2H), 8.75 (s, 1H), 7.49 (d, *J=* 8.8 Hz, 1H), 7.02 (d, *J=* 8.8 Hz, 1H), 4.10 (s, 3H), 4.03 (s, 3H), 3.98 (s, 3H), 1.74-1.66 (m, 1H), 1.50-1.40 (m, 2H), 1.26-1.24 (m, 2H), 1.18-1.11 (m, 2H), 0.93-0.87 (m, 2H).

### Example 162

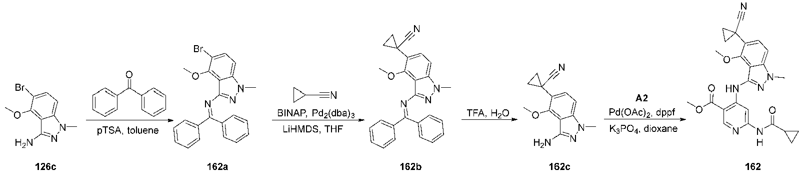

### Step 1. N-(5-bromo-4-methoxy-1-methyl-1H-indazol-3-yl)-1,1-diphenylmethanimine (162a)

To a solution of **126c** (1 g, 3.90 mmol), diphenylmethanone (783 mg, 4.30 mmol) in toluene (10 mL) was added pTSA (20 mg, 0.12 mmol) and 4Å molecular sieve (1g), then the mixture was stirred at 120 °C overnight. The mixture was diluted with EtOAc, filtrated and concentrated, finally it was purified by chromatography column (PE:EtOAc = 10:1 to 2:1) to give the compound **162a** (170 mg, 10% yield) as a yellow solid. LC-MS (ESI, Method 4) t_{R} = 1.18 min, m/z (M+H)⁺ = 420.0.

### Step 2. 1-(3-((Diphenylmethylene)amino)-4-methoxy-1-methyl-1H-indazol-5-yl)cyclopropane-1-carbonitrile (162b)

To a mixture of **162a** (40 mg, 0.095 mmol), cyclopropanecarbonitrile (13 mg, 0.19 mmol), BINAP (12 mg, 0.019 mmol), Pd₂(dba)₃ (9 mg, 0.01 mmol) in THF (1 mL) was added LiHMDS (0.4 mmol, 0.4 mL, 1 M in THF) at room temperature under N₂ atmosphere. The mixture was stirred at room temperature for 30 min, then it was stirred at 80 °C for 16 h. The mixture was diluted with aq. NH₄Cl, extracted with EtOAc, washed with brine and dried over Na₂SO₄, finally it was concentrated and purified by chromatography column (PE:EtOAc = 5:1 to 2:1) to give the compound **162b** (38 mg, 98% yield) as a yellow solid. LC-MS (ESI, Method 4) t_{R} = 1.10 min, m/z (M+H)⁺ = 407.2. ¹H NMR (400 MHz, CDCl₃) δ 7.91-7.85 (m, 2H), 7.54-7.48 (m, 1H), 7.46-7.40 (m, 2H), 7.25-7.17 (m, 5H), 7.15 *(d, J=* 8.8 Hz, 1H), 6.85 *(d, J=* 8.4 Hz, 1H), 4.11 (s, 3H), 3.79 (s, 3H), 1.65-1.60 (m, 2H), 1.24-1.19 (m, 2H).

### Step 3. 1-(3-Amino-4-methoxy-1-methyl-1H-indazol-5-yl)cyclopropane-1-carbonitrile (162c)

A solution of **162b** (100 mg, 0.27 mmol) in TFA and H₂O (1.5 mL, 1:2) was stirred at room temperature for 2 h. The mixture was diluted with H₂O (10 mL), basified by aq. NaHCO₃, extracted with EtOAc, washed with brine and dried over Na₂SO₄, then it was concentrated and purified by chromatography column (PE:EtOAc = 20:1 to 1:2) to give the compound **162c** (20 mg, 96% yield) as a yellow solid. LC-MS (ESI, Method 4) t_{R} = 0.65 min, m/z (M+H)⁺= 243.0.

### Step 4. Methyl 4-((5-(1-cyanocyclopropyl)-4-methoxy-1-methyl-1H-indazol-3-yl)amino)-6-(cyclopropanecarboxamido)nicotinate (162)

A mixture of **162c** (20 mg, 0.083 mmol), **A2** (23 mg, 0.091 mmol), dppf (9 mg, 0.017 mmol), Pd(OAc)₂ (2 mg, 0.008 mmol), K₃PO₄ (35 mg, 0.17 mmol) was stirred at 90 °C under N₂ atmosphere for 16 h. The mixture was concentrated and purified by chromatography column (DCM:MeOH = 30:1) to give compound **162** (16 mg, 42% yield) as a white solid. LC-MS (ESI, Method 4) t_{R} = 0.83 min, m/z (M+H)⁺= 461.2. ¹H NMR (400 MHz, CDCl₃) δ 11.25 (s, 1H), 9.47 (s, 1H), 8.82 (s, 1H), 8.42 (s, 1H), 7.36 (d, *J* = 8.8 Hz, 1H), 7.00 (d, *J* = 8.4 Hz, 1H), 4.26 (s, 3H), 4.02 (s, 3H), 3.98 (s, 3H), 1.75-1.72 (m, 2H), 1.61-1.54 (m, 1H), 1.49-1.43 (m, 2H), 1.19-1.13 (m, 2H), 0.93-0.86 (m, 2H).

### Example 163

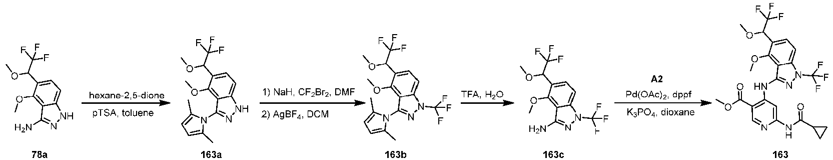

### Step 1. 3-(2,5-Dimethyl-1H-pyrrol-1-yl)-4-methoxy-5-(2,2,2-trifluoro-1-methoxyethyl)-1H-indazole (163a)

To a solution of **78a** (430 mg, 1.56 mmol) in toluene (8 mL) was added pTSA (27 mg, 0.156 mmol) and hexane-2,5-dione (214 mg, 1.87 mmol). Then the mixture was stirred at 110 °C for 16 h. The mixture was concentrated, diluted with EtOAc, washed with aq. NaHCO₃ and brine, then it was dried over Na₂SO₄, concentrated and purified by chromatography column (DCM:MeOH = 100:1 - 20:1) to give the compound **163a** (450 mg, 82% yield) as a gray solid. LC-MS (ESI, Method 4) t_{R} = 1.05 min, m/z (M+H)⁺= 354.0.

### Step 2. 3-(2,5-Dimethyl-1H-pyrrol-1-yl)-4-methoxy-5-(2,2,2-trifluoro-1-methoxyethyl)-1-(trifluoromethyl)-1H-indazole (163b)

To a solution of **163a** (400 mg, 1.13 mmol) in DMF (5 mL) was added NaH (136 mg, 3.40 mmol, 60% purity in mineral) at 0 °C, then the mixture was stirred at room temperature (15 °C) for 16 h. The mixture was diluted with H₂O (20 mL), extracted with EtOAc (15 mL*3) and washed with brine (15 mL), then it was dried over Na₂SO₄ and concentrated to give a yellow oil. The oil was dissolved in DCM (5 mL), AgBF₄ (661 mg, 3.40 mmol) was added to the mixture at -20 °C, then the mixture was stirred at room temperature for 24 h. The mixture was diluted with H₂O (20 mL) and extracted with DCM (15 mL*3), then it was washed with brine (15 mL), dried over Na₂SO₄, concentrated and purified by chromatography column (PE:EtOAc = 100:1 to 8:1) to give compound **163b** (120 mg, 25% yield) as a yellow oil. LC-MS (ESI, Method 4) t_{R} = 2.60 min, m/z (M+H)⁺= 422.2.

### Step 3. 4-Methoxy-5-(2,2,2-trifluoro-1-methoxyethyl)-1-(trifluoromethyl)-1H-indazol-3-amine (163c)

A solution of **163b** (100 mg, 0.24 mmol) in TFA and H₂O (1.8 mL, 1:2) was stirred at room temperature for 2 h. The mixture was diluted with H₂O (10 mL) and basified by aq. NaHCO₃, then it was extracted with EtOAc (10 mL*3), washed with brine (15 mL), dried over Na₂SO₄, finally it was concentrated and purified by chromatography column (PE:EtOAc = 20:1 to 1:2) to give the compound **163c** (30 mg, 37% yield) as a yellow solid. LC-MS (ESI, Method 4) t_{R} = 1.81 min, m/z (M+H)⁺= 344.0. ¹H NMR (400 MHz, CDCl₃) δ 7.64 (dd, *J* = 8.9, 1.2 Hz, 1H), 7.38 (dq, *J=* 8.8, 1.6 Hz, 1H), 5.07 (q, *J=* 6.8 Hz, 1H), 4.67 (s, 2H), 3.98 (s, 3H), 3.38 (s, 3H).

### Step 4. Methyl 6-(cyclopropanecarboxamido)-4-((4-methoxy-5-(2,2,2-trifluoro-1-methoxyethyl)-1-(trifluoromethyl)-1H-indazol-3-yl)amino)nicotinate (163)

A mixture of **163c** (15 mg, 0.044 mmol), **A2** (12 mg, 0.048 mmol), dppf (5 mg, 0.009 mmol), Pd(OAc)₂ (1 mg, 0.004 mmol), K₃PO₄ (19 mg, 0.088 mmol) was stirred at 80 °C under N₂ atmosphere for 16 h. The mixture was concentrated and purified by chromatography column (PE:EtOAc = 10:1 to 2:1) to give compound **163** (9 mg, 37% yield) as a white solid. LC-MS (ESI, Method 4) t_{R} = 2.32 min, m/z (M+H)⁺= 562.2. ¹H NMR (400 MHz, DMSO-*d*₆) δ 11.41 (s, 1H), 11.01 (s,1H), 9.44 (s, 1H), 8.85 (s, 1H), 7.73 (d, *J= 8.8* Hz, 1H), 7.66 (dd, *J* = 8.8, 2.0 Hz, 1H), 5.42 (q, *J* = 6.8 Hz, 1H), 4.01 (s, 3H), 3.95 (s, 3H), 3.38 (s, 3H), 2.10-2.01 (m, 1H), 0.88-0.81 (m, 4H).

### Example 164

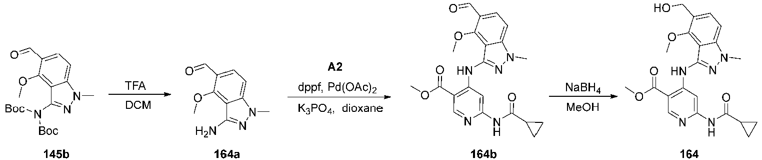

### Step 1. 3-Amino-4-methoxy-1-methyl-1H-indazole-5-carbaldehyde (164a)

To a solution **of 145b** (100 mg, 0.25 mmol) in DCM (2 mL) was added TFA (0.2 mL), the mixture was stirred at room temperature for 2 h. The mixture was diluted with H₂O (10 mL) and basified by aq. NaHCO₃, then it was extracted with EtOAc (10 mL*3), washed with brine (15 mL) and dried over Na₂SO₄, finally it was concentrated to give the crude compound **164a** (40 mg, 79% yield) as a yellow solid. LC-MS (ESI, Method 4) t_{R} = 0.58 min, m/z (M+H)⁺= 206.0.

### Step 2. Methyl 6-(cyclopropanecarboxamido)-4-((5-formyl-4-methoxy-1-methyl-1H-indazol-3-yl)amino)nicotinate (164b)

A mixture of **164a** (30 mg, 0.146 mmol), **A2** (37 mg, 0.146 mmol), dppf (16 mg, 0.029 mmol), Pd(OAc)₂ (3 mg, 0.015 mmol), K₃PO₄ (62 mg, 0.292 mmol) was stirred at 80 °C under N₂ atmosphere for 6 h. The mixture was concentrated and purified by chromatography column (DCM:MeOH = 100:1 to 20:1) to give compound **164b** (5 mg, 8% yield) as a white solid. LC-MS (ESI, Method 4) t_{R} = 1.28 min, m/z (M+H)⁺= 424.2.

### Step 3. Methyl 6-(cyclopropanecarboxamido)-4-((5-(hydroxymethyl)-4-methoxy-1-methyl-1H-indazol-3-yl)amino)nicotinate (164)

To a solution of **164b** (5 mg, 0.012mol) in MeOH (0.5 mL) was added NaBH₄ (1 mg, 0.026 mmol) at 0 °C, then the mixture was stirred at room temperature for 30 min. The mixture was filtered and purified by *prep-HPLC* (Method E) to give the compound **164** (1.0 mg, 20% yield) as a white solid. LC-MS (ESI, Method 4) t_{R} = 0.90 min, m/z (M+H)⁺= 426.2. ¹H NMR (400 MHz, CDCl₃) δ 11.22 (s, 1H), 9.41 (s, 1H), 8.81 (s, 1H), 8.19 (s, 1H), 7.40 (d, *J =* 8.8 Hz, 1H), 7.05 (d, *J* = 8.4 Hz, 1H), 5.34 (s, 1H), 4.83 (s, 2H), 4.04 (s, 3H), 4.03 (s, 3H), 3.97 (s, 3H), 1.87 (m, 1H), 1.18-1.12 (m, 2H), 0.91-0.87 (m, 2H).

### Example 165

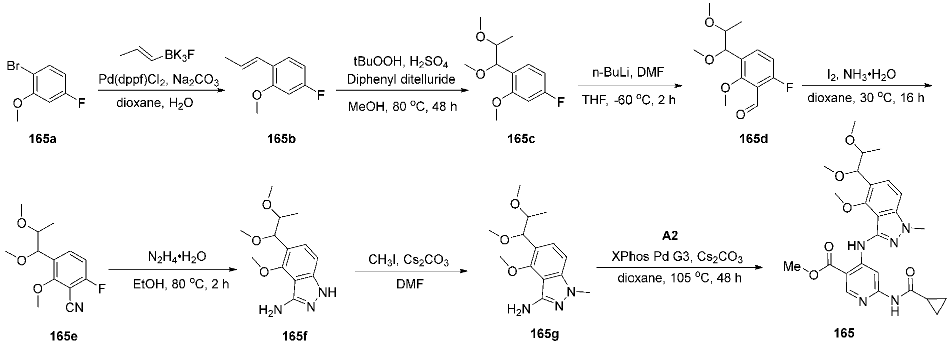

### Step 1. 4-Fluoro-2-methoxy-1-[(E)-prop-1-enyl]benzene (165b)

A mixture of **165a** (3.00 g, 14.63 mmol), Potassium *trans-*1-propenyltrifluoroborate (2.60 g, 17.56 mmol), Na₂CO₃ (4.65 g, 43.90 mmol) and Pd(dppf)Cl₂·CH₂Cl₂ (717 mg, 0.878 mmol) in dioxane (30 mL) and water (3 mL) was degassed and purged with nitrogen for 3 times. The resulting mixture was stirred at 100 °C under N₂ atmosphere for 12 h. The reaction mixture was diluted with water (50 mL) and extracted with EtOAc (50 mL*3). The combined organic layer was washed with water (50 mL*3) and brine (50 mL*2), then it was dried over anhydrous Na₂SO₄, filtered and concentrated. The residue was purified by chromatograph column (PE:EtOAc = 9:1) to give **165b** (1.80 g, 74% yield) as a colorless oil. ¹H NMR (400 MHz, CDCl₃): *δ* 7.31 (dd, *J =* 8.0, 6.8 Hz, 1H), 6.65-6.54 (m, 3H), 6.20-6.08 (m, 1H), 3.82 (s, 3H), 1.88 (d, *J* = 6.8 Hz, 3H).

### Step 2. 1-(1,2-Dimethoxypropyl)-4-fluoro-2-methoxy-benzene (165c)

To a mixture of **165b** (1.80 g, 10.83 mmol) in MeOH (100 mL) was added diphenyl ditelluride (4.43 g, 10.83 mmol), *conc.* H₂SO₄ (2.17 mg, 21.66 mmol) and *t*-BuOOH (1.39 g, 10.83 mmol, 70% in water). The resulting mixture was stirred at 80 °C for 3 h. The reaction mixture was poured into water (50 mL) and extracted with EtOAc (50 mL*3). The combined organic layer was washed with water (50 mL*3) and brine (50 mL*2), then it was dried over anhydrous Na₂SO₄, filtered, and concentrated under reduced pressure. The residue was purified by chromatography column (PE:EtOAc = 95:5) to give **165c** (it has 2 isomers, the first peak: 850 mg, 34% yield) as colorless oil. ¹H NMR (400 MHz, CDCl₃): *δ* 7.31 (dd, *J* = 8.8, 6.8 Hz, 1H), 6.67 (dt, *J* = 8.4, 2.4 Hz, 1H), 6.60 (dd, *J* = 10.8, 2.4 Hz, 1H), 4.75 (d, *J* = 3.6 Hz, 1H), 3.81 (s, 3H), 3.46 (dq, *J=* 6.4, 3.2 Hz, 1H), 3.39 (s, 3H), 3.29 (s, 3H), 1.04 (d, *J* = 6.8 Hz, 3H).

### Step 3. 3-(1,2-Dimethoxypropyl)-6-fluoro-2-methoxy-benzaldehyde (165d)

To a mixture of **165c** (850 mg, 3.72 mmol) in anhydrous THF (15 mL) was added n-BuLi (2.38 mL, 5.96 mmol, 2.5M) at -60 °C. After 30 min, DMF (327 mg, 4.47 mmol) was added to the above solution. The resulting mixture was stirred at -60 °C for 3 h under N₂ atmosphere. The reaction mixture was quenched with water (30 mL) and extracted with EtOAc (30 mL*3). The combined organic layer was washed with water (50 mL*3) and brine (50 mL*2), then it was dried over anhydrous Na₂SO₄, filtered and concentrated to give **165d** (950 mg, crude) as a yellow oil. ¹H NMR (400 MHz, CDCl₃): *δ* 10.39 (s, 1H), 7.66 (dd, *J* = 8.8, 6.4 Hz, 1H), 6.98 (dd, *J=* 10.0, 8.8 Hz, 1H), 4.57 (d, *J=* 4.8 Hz, 1H), 3.90 (s, 3H), 3.52-3.42 (m, 1H), 3.31 (s, 3H), 3.26 (s, 3H), 1.12 (d, *J* = 6.4 Hz, 3H).

### Step 4. 3-(1,2-Dimethoxypropyl)-6-fluoro-2-methoxy-benzonitrile (165e)

A mixture of **165d** (950 mg, 3.71 mmol), I₂ (941 mg, 3.71 mmol) and NH₃·H₂O (5 mL, 30% in water) in dioxane (10 mL) was stirred at 30 °C for 12 h. The reaction mixture was diluted with EtOAc (50 mL) and then washed with aq. Na₂S₂O₃ (50 mL*2) and brine (50 mL*2), then it was dried over anhydrous Na₂SO₄, filtered, and concentrated to give **165e** (930 mg, crude) as a yellow oil. LC-MS (ESI, Method 4) t_{R} = 0.91 min, m/z (M+H)⁺ = 254.0.

### Step 5. 5-(1,2-Dimethoxypropyl)-4-methoxy-1H-indazol-3-amine (165f)

A mixture of **165e** (930 mg, 3.67 mmol) and N₂H₄·H₂O (6 mL, 80% in water) in ethanol (6 mL) was stirred at 80 °C for 12 h. The reaction mixture was poured into water (50 mL) and extracted with EtOAc (50 mL*3). The combined organic layer was washed with water (50 mL*3) and brine (50 mL*2), then it was dried over anhydrous Na₂SO₄, filtered, and concentrated under reduced pressure. The residue was purified by chromatography column (DCM:MeOH = 9:1) to give **165f (360** mg, 37% yield for 3 steps) as a white solid. LC-MS (ESI, Method 4) t_{R} = 0.58 min, m/z (M+H)⁺ = 266.0. ¹H NMR (400 MHz, CDCl₃): *δ* 7.38 (d, *J* = 8.8 Hz, 1H), 7.07 (d, *J* = 8.8 Hz, 1H), 4.62 (d, *J* = 5.2 Hz, 1H), 4.38 (brs, 2H), 3.97 (s, 3H), 3.54-3.45 (m, 1H), 3.27 (s, 3H), 3.25 (s, 3H), 1.21 (d, *J* = 6.0 Hz, 3H).

### Step 6. 5-(1,2-Dimethoxypropyl)-4-methoxy-1-methyl-indazol-3-amine (165g)

To a mixture of **165f** (84 mg, 0.32 mmol) in DMF (3 mL) was added Cs₂CO₃ (155 mg, 0.47 mmol) at 20 °C. The mixture was stirred for 10 min, then CH₃I (54 mg, 0.38 mmol) was added to the reaction mixture. After stirring at 20 °C for 24 h, the reaction mixture was poured into water (30 mL) and extracted with EtOAc (30 mL*3). The combined organic layer was washed with water (50 mL*3) and brine (50 mL*2), then it was dried over anhydrous Na₂SO₄, filtered, and concentrated under reduced pressure. The residue was purified by chromatography column (PE:EtOAc = 4:6) to give **165g** (50 mg, 56% yield) as a yellow gum. LC-MS (ESI, Method 4) t_{R} = 0.68 min, m/z (M+H)⁺ = 280.2. ¹H NMR (400 MHz, CDCl₃): *δ* 7.48 (d, *J* = 8.8 Hz, 1H), 7.00 (d, *J =* 8.8 Hz, 1H), 4.56 (d, *J* = 5.6 Hz, 1H), 3.97 (s, 3H), 3.84 (s, 3H), 3.55-3.45 (m, 1H), 3.26 (s, 3H), 3.24 (s, 3H), 1.21 (d, *J* = 6.0 Hz, 3H).

### Step 7. Methyl 6-(cyclopropanecarboxamido)-4-((5-(1,2-dimethoxypropyl)-4-methoxy-1-methyl-1H-indazol-3-yl)amino)nicotinate (165)

A mixture of **165g** (50 mg, 0.18 mmol), **A2** (46 mg, 0.18 mmol), Cs₂CO₃ (175 mg, 0.54 mmol) and XPhos Pd G3 (30 mg, 0.036 mmol) in dioxane (3 mL) was degassed and purged with nitrogen for 3 times. The resulting mixture was stirred at 105 °C under N₂ atmosphere for 48 h. The reaction mixture was diluted with water (30 mL) and extracted with EtOAc (30 mL*3). The combined organic layer was washed with water (50 mL*3) and brine (50 mL*2), then it was dried over anhydrous Na₂SO₄, filtered and concentrated. The residue was purified by chromatography column (DCM:MeOH = 9:1) and further purified by reversed-phase chromatograph column (water:MeCN = 95:5 to 5:95, 0.1% HCOOH was contained in water) to give **165** (30.6 mg, 34% yield) as a white solid. LC-MS (ESI, Method 4) t_{R} = 0.87 min, m/z (M+H)⁺ = 498.2. ¹H NMR (400 MHz, DMSO-*d₆*): *δ* 11.38 (s, 1H), 10.00 (brs, 1H), 9.58 (s, 1H), 8.72 (s, 1H), 7.48 (d, *J* = 8.8 Hz, 1H), 7.12 (d, *J* = 8.8 Hz, 1H), 4.69 (d, *J* = 5.2 Hz, 1H), 4.07 (s, 3H), 4.01 (s, 3H), 3.99 (s, 3H), 3.61-3.50 (m, 1H), 3.27 (s, 3H), 3.24 (s, 3H), 1.82-1.70 (m, 1H), 1.22 (d, *J* = 6.4 Hz, 3H), 1.19-1.13(m, 2H), 0.99-0.91 (m, 2H).

### Example 166

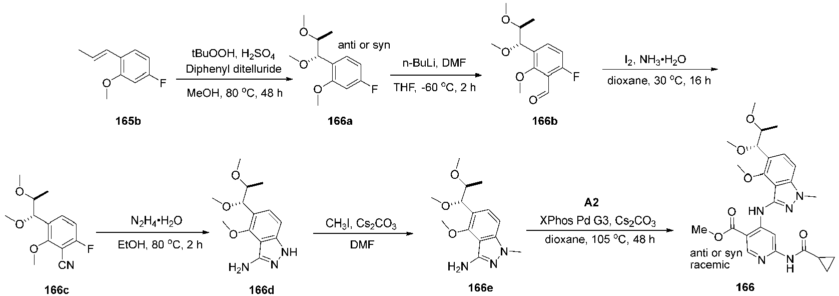

### Step 1. 1-((1S)-1,2-Dimethoxypropyl)-4-fluoro-2-methoxybenzene (166a)

To a mixture of **165b** (1.80 g, 10.83 mmol) in MeOH (100 mL) was added diphenyl ditelluride (4.43 g, 10.83 mmol), conc. H₂SO₄ (2.17 mg, 21.66 mmol) and t-BuOOH (1.39 g, 10.83 mmol, 70% in water). The resulting mixture was stirred at 80 °C for 3 h. The reaction mixture was poured into water (50 mL) and extracted with EtOAc (50 mL*3). The combined organic layer was washed with water (50 mL*3) and brine (50 mL*2), then it was dried over anhydrous Na₂SO₄, filtered, and concentrated under reduced pressure. The residue was purified by chromatography column (PE:EtOAc = 95:5) to give **166a** (it has 2 isomers, the second peak: 760 mg, 31% yield) as a colorless oil. ¹H NMR (400 MHz, CDCl₃): *δ* 7.29 (dd, *J* = 8.8, 6.8 Hz, 1H), 6.66 (dt, *J=* 8.4, 2.4 Hz, 1H), 6.59 (dd, *J* = 11.2, 2.4 Hz, 1H), 4.53 (d, *J=* 6.4 Hz, 1H), 3.80 (s, 3H), 3.52-3.43 (m, 1H), 3.35 (s, 3H), 3.21 (s, 3H), 0.99 (d, *J* = 6.4 Hz, 3H).

### Step 2. 3-((1S)-1,2-Dimethoxypropyl)-6-fluoro-2-methoxybenzaldehyde (166b)

To a mixture of **166a** (760 mg, 3.33 mmol) in anhydrous THF (15 mL) was added n-BuLi (2.13 mL, 5.33 mmol, 2.5M) at -60 °C. After 30 min, DMF (292 mg, 4.00 mmol) was added to the above solution. The resulting mixture was stirred at -60 °C for 3 h under N₂ atmosphere. The reaction mixture was quenched with water (30 mL) and extracted with EtOAc (30 mL*3). The combined organic layer was washed with water (50 mL*3) and brine (50 mL*2), then it was dried over anhydrous Na₂SO₄, filtered and concentrated to give **166b** (650 mg, crude) as a yellow oil. ¹H NMR (400 MHz, CDCl₃): *δ* 10.39 (s, 1H), 7.65 (dd, *J* = 8.8, 6.4 Hz, 1H), 6.98 (dd, *J* = 10.0, 8.8 Hz, 1H), 4.49 (d, *J* = 5.6 Hz, 1H), 3.89 (s, 3H), 3.52-3.44 (m, 1H), 3.34 (s, 3H), 3.25 (s, 3H), 1.07 (d, *J =* 6.4 Hz, 3H).

### Step 3. 3-((1S)-1,2-Dimethoxypropyl)-6-fluoro-2-methoxybenzonitrile (166c)

A mixture of **166b** (650 mg, 2.54 mmol), I₂ (644 mg, 2.54 mmol) and NH₃·H₂O (5 mL, 30% in water) in dioxane (10 mL) was stirred at 30 °C for 12 h. The reaction mixture was diluted with EtOAc (50 mL), then it was washed with aq. Na₂S₂O₃ (50 mL*2) and brine (50 mL*2), finally it was dried over anhydrous Na₂SO₄, filtered, and concentrated to give **166c** (600 mg, crude) as a yellow oil, which was used for the next step directly without further purification. LC-MS (ESI, Method 4) t_{R} = 0.86 min, m/z (M+H)⁺ = 254.0. ¹H NMR (400 MHz, CDCl₃): *δ* 7.61 (dd, *J* = 8.8, 6.8 Hz, 1H), 6.95 (t, *J* = 8.4 Hz, 1H), 4.47 (d, *J* = 5.2 Hz, 1H), 4.12 (s, 3H), 3.50-3.40 (m, 1H), 3.32 (s, 3H), 3.23 (s, 3H), 1.07 (d, *J* = 6.4 Hz, 3H).

### Step 4. 5-((1S)-1,2-Dimethoxypropyl)-4-methoxy-1H-indazol-3-amine (166d)

A mixture of **166c** (600 mg, 2.37 mmol) and N₂H₄·H₂O (4 mL, 80% in water) in ethanol (5 mL) was stirred at 80 °C for 2 h. The reaction mixture was poured into water (50 mL) and extracted with EtOAc (50 mL*3). The combined organic layer was washed with water (50 mL*3) and brine (50 mL*2), then it was dried over anhydrous Na₂SO₄, filtered and concentrated under reduced pressure. The residue was purified by chromatography column (DCM:MeOH = 9:1) to give **166d** (155 mg, 25% yield for 3 steps) as a white solid. LC-MS (ESI, Method 4) t_{R} = 0.48 min, m/z (M+H)⁺ = 266.2. ¹H NMR (400 MHz, CDCl₃): *δ* 7.32 (d, *J =* 8.8 Hz, 1H), 7.06 (d, *J* = 8.8 Hz, 1H), 4.57 (d, *J* = 6.8 Hz, 1H), 3.96 (s, 3H), 3.64-3.55 (m, 1H), 3.45 (s, 3H), 3.25 (s, 3H), 0.98 (d, *J* = 6.4 Hz, 3H).

### Step 5. 5-((1S)-1,2-Dimethoxypropyl)-4-methoxy-1-methyl-1H-indazol-3-amine (166e)

To a mixture of **166d** (80 mg, 0.30 mmol) in DMF (3 mL) was added NaH (60 mg, 1.51 mmol, 60% in mineral oil) at 0 °C. The mixture was stirred for 30 min, then CH₃I (43 mg, 0.30 mmol) was added the reaction mixture. After stirring at 0-10 °C for 2 h, the reaction mixture was poured into water (30 mL) and extracted with EtOAc (30 mL*3). The combined organic layer was washed with water (50 mL*3) and brine (50 mL*2), then it was dried over anhydrous Na₂SO₄, filtered and concentrated under reduced pressure. The residue was purified by chromatography column (PE:EtOAc = 90:10 to 0:100) to give **166e** (47 mg, 56% yield) as a yellow gum. LC-MS (ESI, Method 4) t_{R} = 0.60 min, m/z (M+H)⁺ = 280.2. ¹H NMR (400 MHz, CDCl₃): *δ* 7.32 (d, *J* = 8.8 Hz, 1H), 6.97 (d, *J* = 8.8 Hz, 1H), 4.56 (d, *J* = 7.2 Hz, 1H), 3.95 (s, 3H), 3.81 (s, 3H), 3.63-3.55 (m, 1H), 3.44 (s, 3H), 3.24 (s, 3H), 0.98 (d, *J* = 6.4 Hz, 3H).

### Step 6. Methyl 6-(cyclopropanecarboxamido)-4-((5-((1S)-1,2-dimethoxypropyl)-4-methoxy-1-methyl-1H-indazol-3-yl)amino)nicotinate (166)

A mixture of **166e** (47 mg, 0.17 mmol), **A2** (51 mg, 0.20 mmol), Cs₂CO₃ (165 mg, 0.50 mmol) and XPhos Pd G3 (28 mg, 0.034 mmol) in dioxane (3 mL) was degassed and purged with nitrogen for 3 times. The resulting mixture was stirred at 105 °C under N₂ atmosphere for 48 h. The reaction mixture was diluted with water (30 mL) and extracted with EtOAc (30 mL*3). The combined organic layer was washed with water (50 mL*3) and brine (50 mL*2), then it was dried over anhydrous Na₂SO₄, filtered and concentrated. The residue was purified by chromatography column (DCM:MeOH = 9:1) and further purified by reversed-phase chromatograph column (water:MeCN = 95:5 to 5:95, 0.1% HCOOH was contained in water) to give **166** (28.9 mg, 34% yield) as a white solid. LCMS (ESI, Method 4) t_{R} = 0.79 min, m/z (M+H)⁺ = 498.2. ¹H NMR (400 MHz, CDCl₃): *δ* 11.20 (s, 1H), 9.46 (s, 1H), 9.06 (brs, 1H), 8.77 (s, 1H), 7.43 (d, *J =* 8.8 Hz, 1H), 7.10 (d, *J* = 8.8 Hz, 1H), 4.62 (d, *J* = 6.4 Hz, 1H), 4.06 (s, 3H), 3.99 (s, 3H), 3.97 (s, 3H), 3.64-3.56 (m, 1H), 3.41 (s, 3H), 3.26 (s, 3H), 1.72-1.59 (m, 1H), 1.18-1.12 (m, 2H), 1.04 (d, *J =* 6.4 Hz, 3H), 0.96-0.89(m, 2H).

### Example 167

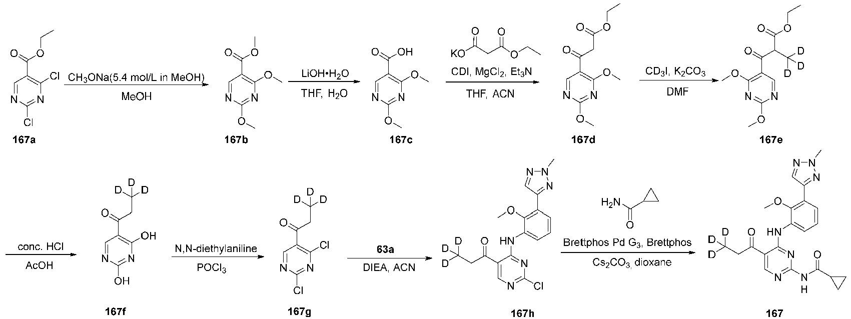

### Step 1. Methyl 2,4-dimethoxypyrimidine-5-carboxylate (167b)

To a solution of **167a** (4 g, 18.10 mmol) in MeOH (30 mL) was added CH₃ONa (5.4 M in MeOH, 16.76 mL) at room temperature. The mixture was stirred at room temperature for 12 h. The resulting solution was diluted with H₂O (30 mL) and extracted with EtOAc (50 mL*2), then the combined organic layer was dried over Na₂SO₄ and filtered. The filtrate was concentrated and purified by chromatography column (EtOAc:PE = 1:1) to afford **167b** (3.25 g, 91% yield) as a white solid. LC-MS (ESI, Method 4) t_{R} = 0.58 min, m/z (M+H)⁺= 199.0.

### Step 2. 2,4-Dimethoxypyrimidine-5-carboxylic acid (167c)

To a solution of **167b** (3.3 g, 16.65 mmol) in THF (25 mL) and water (10 mL) was added LiOH·H₂O (1.40 g, 33.30 mmol) at room temperature. The mixture was stirred at room temperature for 12 h. The resulting solution was diluted with H₂O (30 mL), and the pH was adjusted to 5-6 by HCl solution (1 mol/L). The solution was extracted with EtOAc (80 mL* 8), then the organic layer was dried over Na₂SO₄ and filtered. The filtrate was concentrated to afford **167c** (1.33 g, 43% yield) as a white solid. LC-MS (ESI, Method 4) t_{R} = 0.33 min, m/z (M+H)⁺= 185.0.

### Step 3. Ethyl 3-(2,4-dimethoxypyrimidin-5-yl)-3-oxopropanoate (167d)

To a solution of **167c** (1.33 g, 7.22 mmol) in THF (10 mL) and ACN (10 mL) was added CDI (1.52 g, 9.39 mmol) at room temperature. The mixture was stirred at room temperature for 2 h. Then MgCl₂ (825 mg, 8.67 mmol) and Et₃N (2.19 g, 21.67 mmol) was added, followed by potassium 3-ethoxy-3-oxopropanoate (1.87 g, 10.83 mmol). The mixture was stirred at room temperature for 12 h. The resulting solution was diluted with H₂O (20 mL) and extracted with EtOAc (40 mL), the combined organic layer was dried over Na₂SO₄ and filtered. The filtrate was concentrated and purified by chromatography column (EtOAc:PE = 1:2) to afford **167d** (800 mg, 44% yield) as a white solid. LC-MS (ESI, Method 4) t_{R} = 0.69 min, m/z (M+H)⁺= 255.0.

### Step 4. Ethyl 2-(2,4-dimethoxypyrimidine-5-carbonyl)propanoate-3,3,3-d3 (167e)

To a solution of **167d** (850 mg, 3.34 mmol), K₂CO₃ (693 mg, 5.01 mmol) in DMF (10 mL) was added CD₃I (582 mg, 4.01 mmol) at 0 °C. The mixture was stirred at 0 °C to room temperature for 12 h. The resulting solution was diluted with H₂O (20 mL) and extracted with EtOAc (30 mL), the combined organic layer was washed by brine (15 mL* 2), dried over Na₂SO₄ and filtered. The filtrate was concentrated and purified by chromatography column (EtOAc:PE = 1:2) to afford **167e** (510 mg, 56% yield) as a yellow oil. LC-MS (ESI, Method 4) t_{R} = 0.78 min, m/z (M+H)⁺= 272.0.

### Step 5. 1-(2,4-Dihydroxypyrimidin-5-yl)propan-1-one-3,3,3-d3 (167f)

To a solution of **167e** (500 mg, 1.84 mmol) in AcOH (10 mL) was dropped conc. HCl (2 mL) at room temperature. The mixture was stirred at 100 °C for 3 h. The resulting solution was concentrated to afford **167f** (160 mg, 51% yield) as a light-yellow solid without further purification. LC-MS (ESI, Method 4) t_{R} = 0.78 min, m/z (M+H)⁺= 172.0.

### Step 6. 1-(2,4-Dichloropyrimidin-5-yl)propan-1-one-3,3,3-d3 (167g)

To a solution of **167f** (120 mg, 0.70 mmol) in POCl₃ (9.90 g, 64.57 mmol, 6 mL) was added *N, N*-diethylaniline (1.87 g, 12.54 mmol, 2 mL) at room temperature. The mixture was stirred at 120 °C for 2 h. The resulting solution was poured into 40 g ice and extracted by EtOAc (30 mL). The combined organic layer was washed by brine (15 mL*3), dried over Na₂SO₄ and filtered. The filtrate was concentrated and purified by chromatography column (EtOAc:PE = 1:5) to afford **167g** (40 mg, 27% yield) as a light-red oil. LC-MS (ESI, Method 4) t_{R} = 0.80 min, m/z (M+H)⁺= 208.0.

### Step 7. 1-(2-Chloro-4-((2-methoxy-3-(2-methyl-2H-1,2,3-triazol-4-yl)phenyl)amino)pyrimidin-5-yl)propan-1-one-3,3,3-d3 (167h)

To a solution of **167g** (40 mg, 0.19 mmol), DIEA (50 mg, 0.38 mmol) in ACN (2 mL) was added **63a** (39 mg, 0.19 mmol) at room temperature. The mixture was stirred at 85 °C for 2 h. The resulting solution was diluted with H₂O (15 mL) and extracted with EtOAc (30 mL), the combined organic layer was washed by brine (10 mL*2), dried over Na₂SO₄ and filtered. The filtrate was concentrated and purified by chromatography column (EtOAc:PE = 1:3) to afford **167h** (50 mg, 69% yield) as a light-yellow solid. LC-MS (ESI, Method 4) t_{R} = 1.04 min, m/z (M+H)⁺= 376.2.

### Step 8. N-(4-((2-methoxy-3-(2-methyl-2H-1,2,3-triazol-4-yl)phenyl)amino)-5-(propanoyl-3,3,3-d3)pyrimidin-2-yl)cyclopropanecarboxamide (167)

To a solution of **167h** (45 mg, 0.12 mmol), BrettPhos Pd G3 (22 mg, 0.024 mmol), BrettPhos (26 mg, 0.048 mmol), Cs₂CO₃ (78 mg, 0.24 mmol) in dioxane (2 mL) was added cyclopropanecarboxamide (51 mg, 0.60 mmol) at room temperature. The mixture was stirred at 100 °C for 3 h under N₂ atmosphere. The resulting solution was diluted with H₂O (15 mL) and extracted with EtOAc (30 mL), the combined organic layer was dried over Na₂SO₄ and filtered. The filtrate was concentrated and purified by chromatography column (EtOAc:PE = 2:1) and *prep-HPLC* (Method E) to afford **167** (7 mg, 13% yield) as a white solid. LC-MS (ESI, Method 4) t_{R} = 7.55 min, m/z (M+H)⁺= 425.2. ¹H NMR (400 MHz, DMSO-*d*₆) δ 12.00 (s, 1H), 11.14 (s, 1H), 9.29 (dd, *J =* 8.4, 1.6 Hz, 1H), 9.10 (s, 1H), 8.16 (s, 1H), 7.55 (dd, *J* = 7.6, 1.6 Hz, 1H), 7.21 (t, *J* = 8.0 Hz, 1H), 4.23 (s, 3H), 3.73 (s, 3H), 3.09 (s, 2H), 2.22-2.16 (m, 1H), 0.94-0.75 (m, 4H).

### Example 168

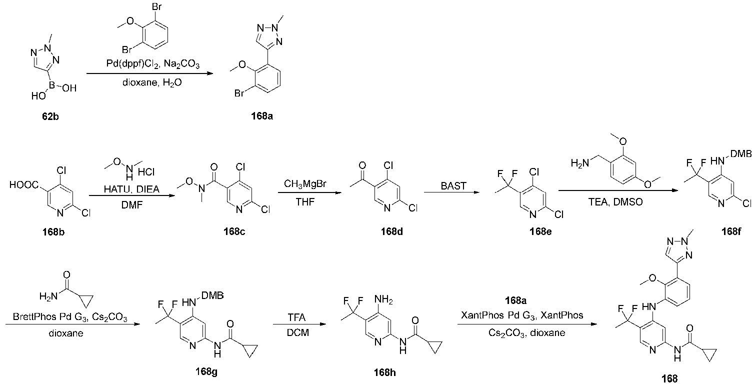

### Step 1. 4-(3-Bromo-2-methoxyphenyl)-2-methyl-2H-1,2,3-triazole (168a)

To a solution of 62b (100 mg, 0.78 mmol) and 1,3-dibromo-2-methoxybenzene (419 mg, 1.58 mmol) in dioxane (2 mL) and H₂O (0.4 mL) was added Pd(dppf)Cl₂ (64 mg, 0.079 mmol) and Na₂CO₃ (167 mg, 1.58 mmol) under N₂ atmosphere, the mixture was stirred at 40 °C for 2 h. The reaction mixture was diluted with water (50 mL) and extracted with EtOAc (50 mL*2). The combined organic layer was washed with brine (30 mL*2) and dried over anhydrous Na₂SO₄, then it was filtered and concentrated. The residue was purified by chromatography column (EtOAc:PE = 1:1) to give **168a** (127 mg, 60% yield) as a colorless oil. LC-MS (ESI, Method 4) t_{R} = 1.00 min, m/z (M+H)⁺ = 268.0.

### Step 2. 4,6-Dichloro-N-methoxynicotinamide (168c)

To a solution of **168b** (5 g, 26.04 mmol) and *N*,*O*-dimethylhydroxylamine hydrochloride (3.05 g, 31.25 mmol) in DMF (60 mL) was added DIEA (6.73 g, 52.08 mmol, 9.07 mL) and HATU (9.90 g, 26.04 mmol), the mixture was stirred at 25 °C for 12 h. The reaction mixture was diluted with water (200 mL) and extracted with EtOAc (100 mL*2). The combined organic layer was washed with brine (100 mL*2) and dried over anhydrous Na₂SO₄, then it was filtered and concentrated. The residue was purified by chromatography column (EtOAc:PE = 1:1) to give **168c** (5.95 g, 97% yield) as a colorless oil. LC-MS (ESI, Method 4) t_{R} = 0.70 min, m/z (M+H)⁺= 235.0.

### Step 3. 1-(4,6-Dichloropyridin-3-yl)ethan-1-one (168d)

To a solution of **168c** (2 g, 8.51 mmol) in THF (40 mL) was added CH₃MgBr (4.25 mL, 12.76 mmol, 3 M in Et₂O) under N₂ atmosphere at 0 °C, the mixture was stirred at 0 °C for 30 min and room temperature for 2 h. The reaction mixture was quenched with saturated NH₄Cl (100 mL) and extracted with EtOAc (50 mL*2). The combined organic layer was washed with brine (50 mL*2) and dried over anhydrous Na₂SO₄, then it was filtered and concentrated. The residue was purified by chromatography column (EtOAc:PE = 15:85) to give **168d** (1.35 g, 84% yield) as a white solid. LC-MS (ESI, Method 4) t_{R} = 0.79 min, m/z (M+H)⁺= 190.0.

### Step 4. 2,4-Dichloro-5-(1,1-difluoroethyl)pyridine (168e)

Compound **168d** (500 mg, 2.63 mmol) was added to BAST (2.91 g, 13.16 mmol, 2.43 mL) at 0 °C, the mixture was stirred for 10 min, then it was heated to 70 °C and stirred for 2 h. The reaction mixture was quenched with saturated NaHCO₃ (100 mL) and extracted with EtOAc (50 mL*2). The combined organic layer was washed with brine (50 mL*2) and dried over anhydrous Na₂SO₄, then it was filtered and concentrated. The residue was purified by chromatography column (EtOAc:PE = 1:9) to give **168e** (388 mg, 70% yield) as a white solid. LC-MS (ESI, Method 4) t_{R} = 0.95 min. ¹H NMR (400 MHz, CDCl₃) δ 8.58 (s, 1H), 7.46 (s, 1H), 2.05 (t, *J* = 18.4 Hz, 3H).

### Step 5. 2-Chloro-5-(1,1-difluoroethyl)-N-(3,4-dimethylbenzyl)pyridin-4-amine (168f)

To a solution of **168e** (293 mg, 1.38 mmol) and TEA (210 mg, 2.07 mmol, 0.29 mL) in DMSO (5 mL) was added (2,4-dimethoxyphenyl)methanamine (347 mg, 2.07 mmol, 0.31 mL), the mixture was stirred at 50 °C for 2 h. The reaction mixture was diluted with water (50 mL) and extracted with EtOAc (50 mL*2). The combined organic layer was washed with brine (50 mL*2) and dried over anhydrous Na₂SO₄, then it was filtered and concentrated. The residue was purified by *Prep*-TLC (EtOAc:PE = 1:4) to give **168f** (300 mg, 63% yield) as a colorless oil. LC-MS (ESI, Method 4) t_{R} = 1.04 min, m/z (M+H)⁺ = 343.2.

### Step 6. N-(5-(1,1-difluoroethyl)-4-((3,4-dimethylbenzyl)amino)pyridin-2-yl)cyclopropanecarboxamide (168g)

To a solution of **168f** (300 mg, 0.88 mmol) and cyclopropanecarboxamide (74 mg, 0.88 mmol) in dioxane (8 mL) was added BrettPhos Pd G3 (79 mg, 0.088 mmol) and Cs₂CO₃ (570 mg, 1.75 mmol) under N₂ atmosphere, the mixture was stirred at 90 °C for 2 h. The reaction mixture was diluted with water (50 mL) and extracted with EtOAc (50 mL*2). The combined organic layer was washed with brine (50 mL*2) and dried over anhydrous Na₂SO₄, then it was filtered and concentrated. The residue was purified by *Prep*-TLC (EtOAc:PE = 1:1) to give **168g** (180 mg, 53% yield) as a white solid. LC-MS (ESI, Method 4) t_{R} = 0.71 min, m/z (M+H)⁺= 392.2.

### Step 7. N-(4-amino-5-(1,1-difluoroethyl)pyridin-2-yl)cyclopropanecarboxamide (168h)

To a solution of **168g** (130 mg, 0.33 mmol) in DCM (1.5 mL) was added TFA (0.3 mL), the mixture was stirred at 25 °C for 1 h. The reaction mixture was quenched with saturated NaHCO₃ (50 mL) and extracted with EtOAc (50 mL*2). The combined organic layer was washed with brine (50 mL*2) and dried over anhydrous Na₂SO₄, then it was filtered and concentrated. The residue was purified by chromatography column (EtOAc:PE = 1:1) to give 168h (76 mg, 95% yield) as a white solid. LC-MS (ESI, Method 4) t_{R} = 0.34 min, m/z (M+H)⁺= 242.0. ¹H NMR (400 MHz, DMSO-*d*₆) *δ* 10.47 (s, 1H), 7.93 (s, 1H), 7.46 (s, 1H), 6.13 (s, 2H), 2.03-1.88 (m, 4H), 0.86-0.71 (m, 4H).

### Step 8. N-(5-(1,1-difluoroethyl)-4-((2-methoxy-3-(2-methyl-2H-1,2,3-triazol-4-yl)phenyl)amino)pyridin-2-yl)cyclopropanecarboxamide (168)

To a solution of **168h** (35 mg, 0.15 mmol) and **168a** (39 mg, 0.15 mmol) in dioxane (5 mL) was added XantPhos Pd G3 (28 mg, 0.029mol), XantPhos (34 mg, 0.058 mmol) and Cs₂CO₃ (95 mg, 0.29 mmol) under N₂ atmosphere, the mixture was stirred at 100 °C for 3 h. The reaction mixture was diluted with water (50 mL) and extracted with EtOAc (50 mL*2). The combined organic layer was washed with brine (50 mL*2) and dried over anhydrous Na₂SO₄, then it was filtered and concentrated. The residue was purified by *Prep-HPLC* (Method D) to give **168** (8 mg, 14% yield) as an off-white solid. LC-MS (ESI, Method 4) t_{R} = 0.73 min, m/z (M+H)⁺= 429.2. ¹H NMR (400 MHz, DMSO-*d*6) *δ* 10.73 (s, 1H), 8.20 (s, 1H), 8.09 (s, 1H), 7.80 (s, 1H), 7.70 (dd, *J=* 8.0, 1.6 Hz, 1H), 7.47 (t, *J* = 3.6 Hz, 1H), 7.37 (dd, *J=* 8.0, 1.6 Hz, 1H), 7.27 (t, *J=* 8.0 Hz, 1H), 4.23 (s, 3H), 3.60 (s, 3H), 2.14 (t, *J=* 19.2 Hz, 3H), 2.00-1.90 (m, 1H), 0.76-0.69 (m, 4H).

### Example 169

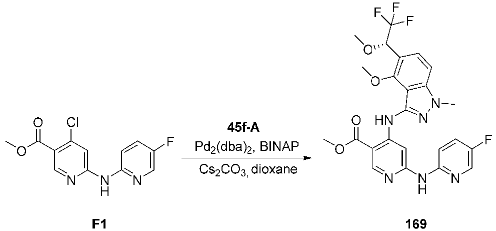

### Step 1. Methyl (S)-6-((5-fluoropyridin-2-yl)amino)-4-((4-methoxy-1-methyl-5-(2,2,2-trifluoro-1-methoxyethyl)-1H-indazol-3-yl)amino)nicotinate (169)

To a solution of **F1** (100 mg, 0.36 mmol), Pd₂(dba)₃ (65 mg, 0.071 mmol), BINAP (66 mg, 0.11 mmol), Cs₂CO₃ (289 mg, 0.89 mmol) in dioxane (8 mL) was added **45f-A** (103 mg, 0.36 mmol) at room temperature. The mixture was stirred at 100 °C for 12 h under N₂ atmosphere. The resulting solution was diluted with H₂O (15 mL) and extracted with EtOAc (30 mL), the combined organic layer was washed by brine (15 mL* 2), dried over Na₂SO₄ and filtered. The filtrate was concentrated and purified by chromatography column (DCM:MeOH = 9:1) and *Prep*-HPLC(Method D) to afford **169** (11 mg, 6% yield) as a white solid. LC-MS (ESI, Method 4) t_{R} = 0.865 min, m/z (M+H)⁺= 535.2. ¹H NMR (400 MHz, DMSO-*d*₆) δ 10.97 (s, 1H), 10.17 (s, 1H), 9.12 (s, 1H), 8.74 (s, 1H), 8.30 (d, *J* = 2.8 Hz, 1H), 7.70 (td, *J* = 8.8, 3.2 Hz, 1H), 7.62 (dd, *J* = 9.2, 4.0 Hz, 1H), 7.49 (d, *J* = 9.2 Hz, 1H), 7.42 (d, *J* = 8*.*8 Hz, 1H), 5.30 (q, *J* = 6.8 Hz, 1H), 4.05 (s, 3H), 3.92 (d, *J* = 4.8 Hz, 6H), 3.32 (s, 3H).

### Example 170

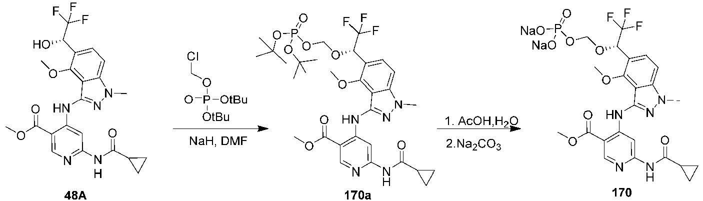

### Step 1. (S)-methyl 6-(cyclopropanecarboxamido)-4-((5-(1-(((di-tert-butoxyphosphoryl)oxy)methoxy)-2,2,2-trifluoroethyl)-4-methoxy-1-methyl-1H-indazol-3-yl)amino)nicotinate (170a)

To a solution of **48A** (500 mg, 1.01 mmol) in DMF (5 mL) was added NaH (40.53 mg, 1.01 mmol, 60% in mineral oil) at 0 °C. The mixture was stirred at 0 °C for 30 min. Di*-tert-*butyl chloromethyl phosphate (393 mg, 1.52 mmol) was added to the mixture. After stirring at 20 °C for 4 h, the mixture was diluted with H₂O (5 mL) and extracted with EtOAc (10 mL *2). The combined organic layer was concentrated and purified by *Prep*-HPLC (Method A) to afford **170a** (100 mg, 12% yield) as a white solid. LC-MS (ESI, Method 3): t_{R} = 1.20 min, m/z (M+H)⁺ = 716.5.

### Step 2. Sodium (S)-(1-(3-((2-(cyclopropanecarboxamido)-5-(methoxycarbonyl)pyridin-4-yl)amino)-4-methoxy-1-methyl-1H-indazol-5-yl)-2,2,2-trifluoroethoxy)methyl phosphate (170)

A mixture of **170a** (100 mg, 0.139 mmol) in AcOH and H₂O (2 mL and 0.5 mL) was stirred at 35 °C for 16 h. The reaction was concentrated and purified by *Prep*-HPLC (ACN:water = 5:95 to 95:5) to afford the solution of phosphonic acid. Saturated Na₂CO₃ (0.2 mL) was added to the solution, and then the solution was lyophilized to afford **170** (4.5 mg, 12% yield). LC-MS (ESI, Method 2): t_{R} = 1.09 min, m/z (M-2Na+H)⁺= 604.0. ¹H NMR (400 MHz, DMSO-*d*₆) δ 10.96 (s, 1H), 10.90 (s, 1H), 9.26 (s, 1H), 8.76 (s, 1H), 7.44-7.41 (m, 2H), 5.91-5.99 (m, 1H), 5.03-5.01 (m, 1H), 4.72-4.69 (m, 1H), 3.97-3.93 (m, 9H), 2.06-2.02 (m, 1H), 0.86-0.81 (m, 4H).

### Example 171

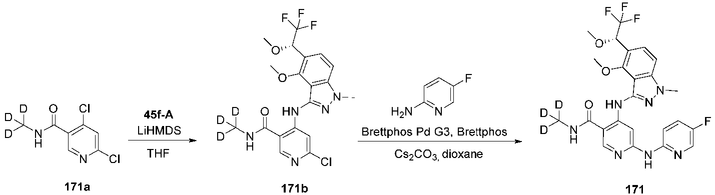

### Step 1. (S)-6-chloro-4-((4-methoxy-1-methyl-5-(2,2,2-trifluoro-1-methoxyethyl)-1H-indazol-3-yl)amino)-N-(methyl-d3)nicotinamide (171b)

To a solution of **45f-A** (150 mg, 0.52 mmol), **171a** (108 mg, 0.52 mmol) in THF (10 mL) was dropped LiHMDS (1 M, 2.07 mL) at -60 °C under N₂ atmosphere. The mixture was stirred at -60 °C to room temperature for 4 h. The resulting solution was diluted with H₂O (15 mL) and extracted by EtOAc (30 mL), the combined organic layer was washed by brine (15 mL* 2), dried over Na₂SO₄ and filtered. The filtrate was concentrated and purified by chromatography column (EtOAc:PE = 1:2) to afford **171b** (210 mg, 88% yield) as a light-yellow solid. LC-MS (ESI, Method 4) t_{R} = 0.77 min, m/z (M+H)⁺= 461.2.

### Step 2. (S)-6-((5-fluoropyridin-2-yl)amino)-4-((4-methoxy-1-methyl-5-(2,2,2-trifluoro-1-methoxyethyl)-1H-indazol-3-yl)amino)-N-(methyl-d3)nicotinamide (171)

To a solution of **171b** (100 mg, 0.22 mmol), BrettPhos Pd G3 (39 mg, 0.043 mmol), BrettPhos (47 mg, 0.087 mmol), Cs₂CO₃ (177 mg, 0.54 mmol) in dioxane (8 mL) was added 5-fluoropyridin-2-amine (49 mg, 0.43 mmol) at room temperature. The mixture was stirred at 100 °C for 12 h under N₂ atmosphere. The resulting solution was diluted with H₂O (20 mL) and extracted by EtOAc (30 mL), the combined organic layer was washed by brine (20 mL* 2), dried over Na₂SO₄ and filtered. The filtrate was concentrated and purified by chromatography column (DCM:MeOH = 9:1) and *Prep*-HPLC(Method D) to afford **171** (48 mg, 41% yield) as a white solid. LC-MS (ESI, Method 4) t_{R} = 5.87 min, m/z (M+H)⁺= 537.2 ¹H NMR (400 MHz, DMSO-*d*₆) δ 11.65 (s, 1H), 9.89 (s, 1H), 8.96 (s, 1H), 8.51-8.48 (m, 2H), 8.22 (t, *J* = 1.6 Hz, 1H), 7.66-7.60 (m, 2H), 7.41 (d, *J* = 8.8 Hz, 1H), 7.36 (d, *J=* 9.2 Hz, 1H), 5.26 (q, *J* = 6.8 Hz, 1H), 3.99 (s, 3H), 3.89 (s, 3H), 3.28 (s, 3H).

### Example 172

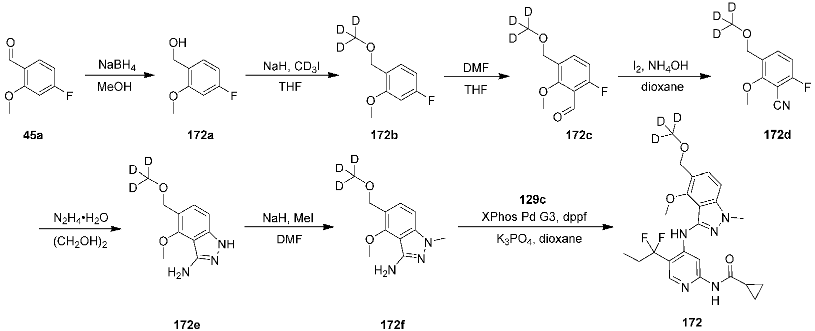

### Step 1. (4-Fluoro-2-methoxyphenyl)methanol (172a)

To a mixture of **45a** (8.00 g, 51.90 mmol) in MeOH (50 mL) was added NaBH₄ (2.16 g, 57.09 mmol) at 0 °C. The resulting mixture was stirred at 20 °C for 2 h under N₂ atmosphere. The reaction mixture was diluted with water (100 mL) and extracted with EtOAc (50 mL*3). The combined organic layer was washed with water (50 mL*2) and brine (50 mL*2), then it was dried over anhydrous Na₂SO₄, filtered and concentrated to give **172a** (7.60 g, 94% yield) as a white solid, which was used for the next step directly without further purification.

### Step 2. 4-Fluoro-2-methoxy-1-((methoxy-d3)methyl)benzene (172b)

To a mixture of **172a** (4.00 g, 25.62 mmol) in THF (50 mL) was added NaH (1.23 g, 30.74 mmol, 60% in mineral oil) and CD₃I (5.57 g, 38.42 mmol, 2.39 mL) at 0 °C. The resulting mixture was stirred at 0 °C for 3 h under N₂ atmosphere. The reaction mixture was diluted with water (20 mL) and extracted with EtOAc (20 mL*2). The combined organic layer was washed with water (20 mL*2), brine (20 mL*2) and dried over anhydrous Na₂SO₄, then it was filtered and concentrated. The residue was purified by chromatography column (EtOAc:PE = 6:94) to give **172b** (4.10 g, 92% yield) as a colorless oil.

### Step 3. 6-Fluoro-2-methoxy-3-((methoxy-d3)methyl)benzaldehyde (172c)

To a mixture of **172b** (1.00 g, 5.77 mmol) in THF (10 mL) was added *n*-BuLi (8.66 mmol, 2.5 M, 3.46 mL). The resulting mixture was stirred at -78 °C for 1 h under N₂ atmosphere. DMF (8.66 mmol, 670 µL) was added to the mixture and the mixture was stirred at -78 °C for 1 h. The reaction mixture was diluted with water (20 mL) and extracted with EtOAc (20 mL*2). The combined organic layer was washed with water (20 mL*2), brine (20 mL*2) and dried over anhydrous Na₂SO₄, then it was filtered and concentrated. The residue was purified by chromatography column (EtOAc:PE = 5:95) to give **172c** (230 mg, 13% yield) as a white solid. LC-MS (ESI, Method 4) t_{R} = 0.72 min, m/z (M+H)⁺= 202.0.

### Step 4. 6-Fluoro-2-methoxy-3-((methoxy-d3)methyl)benzonitrile (172d)

To a mixture of **172c** (230 mg, 0.77 mmol) in H₂O (5 mL) was added (hydrazineyloxy)sulfonic acid (152 mg, 1.35 mmol). The resulting mixture was stirred at 50 °C for 16 h under N₂ atmosphere. The reaction mixture was diluted with water (10 mL) and extracted with EtOAc (20 mL*2). The combined organic layer was washed with water (20 mL*2), brine (20 mL*2) and dried over anhydrous Na₂SO₄, then it was filtered and concentrated to give **172d** (73 mg, crude) as a colorless oil, which was used for the next step directly without further purification.

### Step 5. 4-Methoxy-5-((methoxy-d3)methyl)-1H-indazol-3-amine (172e)

To a mixture of **172d** (73 mg, 0.40 mmol) in ethylene glycol (5 mL) was added hydrazine (101 mg, 1.61 mmol, 80% in H₂O). The resulting mixture was stirred at 150 °C for 1 h under microwave. The reaction mixture was diluted with water (20 mL) and extracted with EtOAc (20 mL*2). The combined organic layer was washed with water (20 mL*2), brine (20 mL*2) and dried over anhydrous Na₂SO₄, then it was filtered and concentrated. The residue was purified by chromatography column (EtOAc:PE = 71:29) to give **172e** (65 mg, 77% yield) as a colorless oil. LC-MS (ESI, Method 4) t_{R} = 0.44 min, m/z (M+H)⁺= 211.0.

### Step 6. 4-Methoxy-5-((methoxy-d3)methyl)-1-methyl-1H-indazol-3-amine (172f)

To a mixture of **172e** (65 mg, 0.31 mmol) in THF (5 mL) was added NaH (25 mg, 0.62 mmol, 60% in mineral oil) and MeI (53 mg, 0.37 mmol, 23 µL). The resulting mixture was stirred at 0 °C for 1 h under N₂ atmosphere. The reaction mixture was diluted with water (20 mL) and extracted with EtOAc (20 mL*2). The combined organic layer was washed with water (20 mL*2), brine (20 mL*2) and dried over anhydrous Na₂SO₄, then it was filtered and concentrated. The residue was purified by chromatography column (EtOAc:PE = 32:68) to give **172f** (33 mg, 47% yield) as a colorless oil. ¹H NMR (400 MHz, CDCl₃) *δ* 7.39 (d, *J* = 8.8 Hz, 1H), 6.93 (d, *J=* 8.8 Hz, 1H), 4.52 (s, 2H), 3.99 (s, 3H), 3.81 (s, 3H).

### Step 7. N-(5-(1,1-difluoropropyl)-4-((4-methoxy-5-((methoxy-d3)methyl)-1-methyl-1H-indazol-3-yl)amino)pyridin-2-yl)cyclopropanecarboxamide (172)

A mixture of **172f** (40 mg, 0.18 mmol), **129c** (45 mg, 0.16 mmol), K₃PO₄ (87 mg, 0.41 mmol), dppf (36 mg, 0.66 mmol), and XPhos Pd G3 (28 mg, 0.033 mmol) in dioxane (3 mL) was degassed and purged with nitrogen for 3 times. The resulting mixture was stirred at 85 °C under N₂ atmosphere for 48 h. The reaction mixture was diluted with water (30 mL) and extracted with EtOAc (30 mL*3). The combined organic layer was washed with water (50 mL*3), brine (50 mL*2) and dried over anhydrous Na₂SO₄, then it was filtered and concentrated. The residue was purified by chromatography column (DCM:MeOH = 9:1) and further purified by reversed-phase chromatograph column (water:MeCN = 95:5 to 5:95, 0.1% HCOOH was contained in water) to give **172** (20.6 mg, 27% yield) as a white solid. LCMS (ESI, Method 4) t_{R} = 0.82 min, m/z (M+H)⁺ = 463.2. ¹H NMR (400 MHz, DMSO*-d₆*): *δ* 9.41 (s, 1H), 8.56 (brs, 1H), 8.40 (t, *J* = 5.6 Hz, 1H), 8.11 (s, 1H), 7.36 (d, *J* = 8.4 Hz, 1H), 7.01 (d, *J* = 8.4 Hz, 1H), 4.54 (s, 2H), 4.00 (s, 3H), 3.97 (s, 3H), 2.41-2.22 (m, 2H), 1.66-1.53 (m, 1H), 1.17-1.10 (m, 2H), 1.07 (t, *J* = 7.2 Hz, 3H), 0.92-0.83 (m, 2H).

### Example 173

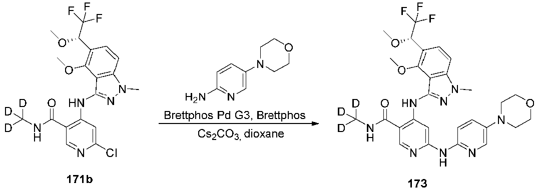

### Step 1. (S)-4-((4-methoxy-1-methyl-5-(2,2,2-trifluoro-1-methoxyethyl)-1H-indazol-3-yl)amino)-N-(methyl-d3)-6-((5-morpholinopyridin-2-yl)amino)nicotinamide (173)

To a solution of **171b** (40 mg, 0.087 mmol), BrettPhos Pd G3 (16 mg, 0.017 mmol), BrettPhos (19 mg, 0.035 mmol), Cs₂CO₃ (71 mg, 0.22 mmol) in dioxane (2 mL) was added 5-morpholinopyridin-2-amine (31 mg, 0.17 mmol) at room temperature. The mixture was stirred at 100 °C for 12 h under N₂ atmosphere. The resulting solution was diluted with H₂O (15 mL) and extracted with EtOAc (30 mL), the combined organic layer was washed by brine (20 mL* 2), dried over Na₂SO₄ and filtered. The filtrate was concentrated and purified by chromatography column (DCM:MeOH = 9:1) and *Prep*-HPLC(Method D) to afford **173** (18 mg, 34% yield) as a white solid. LC-MS (ESI, Method 4) t_{R} = 0.82 min, m/z (M)⁺= 604.4 ¹H NMR (400 MHz, DMSO-*d*₆) δ 11.61 (s, 1H), 9.53 (s, 1H), 8.86 (s, 1H), 8.47 (s, 1H), 8.44 (s, 1H), 7.93 (d, *J=* 2.8 Hz, 1H), 7.50 (d, *J* = 9.2 Hz, 1H), 7.43-7.32 (m, 3H), 5.26 (q, *J* = 6.8 Hz, 1H), 3.98 (s, 3H), 3.88 (s, 3H), 3.76-3.69 (m, 4H), 3.28 (s, 3H), 3.08-3.01 (m, 4H).

### Example 174

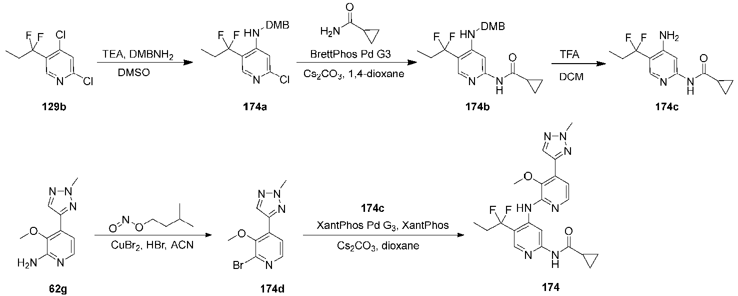

### Step 1. 2-Chloro-5-(1,1-difluoropropyl)-N-(2,4-dimethoxybenzyl)pyridin-4-amine (174a)

To a solution of **129b** (450 mg, 1.99 mmol) in DMSO (4.5 mL) was added (2,4-dimethoxyphenyl)methanamine (832 mg, 4.98 mmol) and TEA (403 mg, 3.98 mmol). Then the mixture was stirred at 70 °C for 6 h. After cooling to room temperature, the reaction mixture was diluted with water (10 mL) and extracted with EtOAc (15 mL*3). The combined organic layer was concentrated and purified by chromatography column (PE:EtOAc = 5:1) to afford **174a** (530 mg, 75% yield) as yellow oil. LC-MS (ESI, Method 3): t_{R} = 1.62 min, m/z (M+H)⁺= 356.9.

### Step 2. N-(5-(1,1-difluoropropyl)-4-((2,4-dimethoxybenzyl)amino)pyridin-2-yl)cyclopropanecarboxamide (174b)

A mixture of **174a** (480 mg, 1.35 mmol), cyclopropanecarboxamide (572.5 mg, 6.73 mmol), BrettPhos Pd G3 (122 mg, 0.135 mmol) and Cs₂CO₃ (1.10 g, 3.36 mmol) in 1,4-dioxane (4.8 mL) was stirred at 90 °C for 2 h under N₂ atmosphere. After cooling to room temperature, the mixture was diluted with water (5 mL) and extracted with EtOAc (10 mL*3). The combined organic layer was concentrated. The residue was purified by chromatography column (PE:EtOAc = 5:1) to afford **174b** (500 mg, 92% yield) as a yellow solid. LC-MS (ESI, Method 3): t_{R} = 1.20 min, m/z (M+H)⁺= 406.3.

### Step 3. N-(4-amino-5-(1,1-difluoropropyl)pyridin-2-yl)cyclopropanecarboxamide (174c)

A mixture of **174b** (260 mg, 641 µmol) in TFA and DCM (0.75 mL and 2.25 mL) was stirred at 15 °C for 2 h. The solvent was removed by pumping nitrogen stream. The residue was adjusted pH>7 with TEA and concentrated. Then the residue was purified by chromatography column (DCM:MeOH = 20:1) to afford **174c** (161 mg, 98% yield) as a yellow solid. LC-MS (ESI, Method 3): t_{R} = 1.19 min, m/z (M+H)⁺= 256.0.

### Step 4. 2-Bromo-3-methoxy-4-(2-methyl-2H-1,2,3-triazol-4-yl)pyridine (174d)

To a solution of **62g** (80 mg, 0.39 mmol) and CuBr₂ (174 mg, 0.78 mmol) in ACN (3 mL) and HBr (3 mL, 48% wt. in H₂O) was added isoamyl nitrite (913 mg, 7.80 mmol, 1.04 mL) at 0 °C under N₂ atmosphere, the mixture was stirred at 65 °C for 0.5 h. The reaction mixture was quenched with saturated NaHCO₃ (50 mL) and extracted with EtOAc (50 mL*2). The combined organic layer was washed with brine (50 mL*2) and dried over anhydrous Na₂SO₄, then it was filtered and concentrated. The residue was purified by chromatography column (EtOAc:PE = 15:85) to give **174d** (55 mg, 0.20 mmol, 52% yield) as a white solid. LC-MS (ESI, Method 4) t_{R} = 0.82 min, m/z (M+H)⁺ = 269.0. ¹H NMR (400 MHz, DMSO-*d*₆) δ 8.30 (s, 1H), 8.23 (d, *J* = 5.2 Hz, 1H), 7.88 (d, *J=* 5.2 Hz, 1H), 4.27 (s, 3H), 3.82 (s, 3H).

### Step 5. N-(5-(1,1-difluoropropyl)-4-((3-methoxy-4-(2-methyl-2H-1,2,3-triazol-4-yl)pyridin-2-yl)amino)pyridin-2-yl)cyclopropanecarboxamide (174)

To a solution of **174d** (32 mg, 0.12 mmol) and **174c** (30 mg, 0.12 mmol) in dioxane (4 mL) was added XantPhos Pd G3 (22 mg, 0.024 mmol), XantPhos (27 mg, 0.047 mmol), Cs₂CO₃ (77 mg, 0.24 mmol) and molecular sieve under N₂ atmosphere, the mixture was stirred at 120 °C for 12 h. The reaction mixture was diluted with water (50 mL) and extracted with EtOAc (50 mL*2). The combined organic layer was washed with brine (50 mL*2) and dried over anhydrous Na₂SO₄, then it was filtered and concentrated. The residue was purified by *Prep-*HPLC (Method D) to give **174** (15 mg, 28% yield) as a white solid. LC-MS (ESI, Method 4) t_{R} =0.91 min, m/z (M+H)⁺= 444.2. ¹H NMR (400 MHz, DMSO-*d*₆) *δ* 10.82 (s, 1H), 9.41 (s, 1H), 8.30 (s, 1H), 8.26 (s, 1H), 8.21 (t, *J* = 5.6 Hz, 1H), 8.12 (d, *J* = 5.2 Hz, 1H), 7.44 (d, *J* = 5.2 Hz, 1H), 4.27 (s, 3H), 3.73 (s, 3H), 2.48-2.30 (m, 2H), 2.08-1.95 (m, 1H), 1.04 (t, *J* = 7.2 Hz, 3H), 0.86-0.76 (m, 4H).

### Example 175

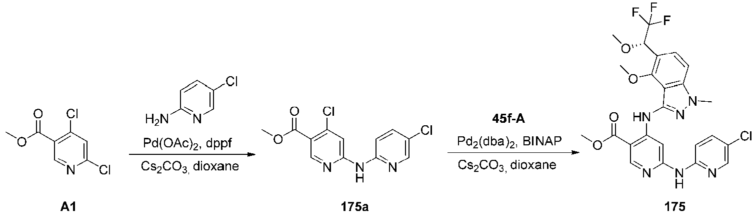

### Step 1. Methyl 4-chloro-6-((5-chloropyridin-2-yl)amino)nicotinate (175a)

To a solution of **A1** (500 mg, 2.43 mmol), Pd(OAc)₂ (109 mg, 0.49 mmol), dppf (404 mg, 0.73 mmol), Cs₂CO₃ (1.98 g, 6.07 mmol) in dioxane (10 mL) was added 5-chloropyridin-2-amine (312 mg, 2.43 mmol) at room temperature. The mixture was stirred at 90 °C for 3 h under N₂ atmosphere. The resulting solution was diluted with H₂O (15 mL) and extracted by EtOAc (30 mL), the combined organic layer was washed by brine (15 mL* 2), dried over Na₂SO₄ and filtered. The filtrate was concentrated and purified by chromatography column (EtOAc:PE = 1:2) to afford **175a** (290 mg, 40% yield) as a white solid. LC-MS (ESI, Method 4) t_{R} = 1.01 min, m/z (M+H)⁺= 298.0.

### Step 2. Methyl (S)-6-((5-chloropyridin-2-yl)amino)-4-((4-methoxy-1-methyl-5-(2,2,2-trifluoro-1-methoxyethyl)-1H-indazol-3-yl)amino)nicotinate (175)

To a solution of **45f-A** (60 mg, 0.21 mmol), Pd₂(dba)₃ (38 mg, 0.041 mmol), BINAP (39 mg, 0.062 mmol), Cs₂CO₃ (169 mg, 0.52 mmol) in dioxane (8 mL) was added **175a** (62 mg, 0.21 mmol) at room temperature. The mixture was stirred at 100 °C for 12 h under N₂ atmosphere. The resulting solution was diluted with H₂O (15 mL) and extracted by EtOAc (30 mL), the combined organic layer was washed by brine (15 mL* 2), dried over Na₂SO₄ and filtered. The filtrate was concentrated and purified by chromatography column (EtOAc:PE = 1:1) and *Prep*-HPLC(Method E) to afford **175** (37 mg, 33% yield) as a white solid. LC-MS (ESI, Method 4) t_{R} = 0.932 min, m/z (M+H)⁺= 551.2 ¹H NMR (400 MHz, DMSO-*d*₆) δ 10.98 (s, 1H), 10.28 (s, 1H), 9.11 (s, 1H), 8.76 (s, 1H), 8.31 (dd, *J* = 2.8, 0.8 Hz, 1H), 7.81 (dd, *J =* 8.8, 2.4 Hz, 1H), 7.64 (d, *J* = 8.8 Hz, 1H), 7.49 (d, *J* = 8.8 Hz, 1H), 7.42 (d, *J* = 8.8 Hz, 1H), 5.30 (q, *J* = 6.8 Hz, 1H), 4.06 (s, 3H), 3.92 (d, *J* = 2.8 Hz, 6H), 3.32 (s, 3H).

### Example 176

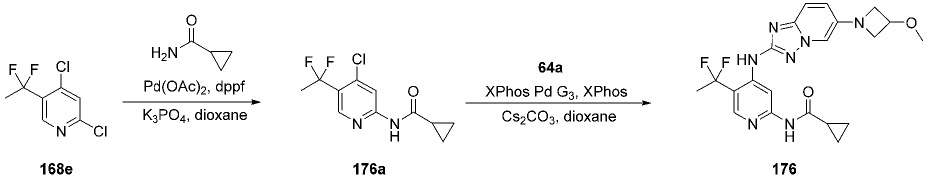

### Step 1. N-(4-chloro-5-(1,1-difluoroethyl)pyridin-2-yl)cyclopropanecarboxamide (176a)

To a solution of **168e** (378 mg, 1.78 mmol) and cyclopropanecarboxamide (152 mg, 1.78 mmol) in dioxane (8 mL) was added Pd(OAc)₂ (80 mg, 0.36 mmol), dppf (395 mg, 0.71 mmol) and K₃PO₄ (1.14 g, 5.35 mmol) under N₂ atmosphere, the mixture was stirred at 80 °C for 2 h. The reaction mixture was diluted with water (50 mL) and extracted with EtOAc (50 mL*2). The combined organic layer was washed with brine (30 mL*2) and dried over anhydrous Na₂SO₄, then it was filtered and concentrated. The residue was purified by chromatography column (EtOAc:PE = 15:85) to give **176a** (190 mg, 81% yield) as an off-white solid. LC-MS (ESI, Method 4) t_{R} = 0.94 min, m/z (M+H)⁺ = 261.0. ¹H NMR (400 MHz, CDCl₃) *δ* 8.43 (s, 1H), 8.39 (s, 1H), 2.04 (t, *J* = 18.4 Hz, 3H), 1.64-1.49 (m, 1H), 1.18-1.10 (m, 2H), 0.99-0.91 (m, 2H).

### Step 2. N-(5-(1,1-difluoroethyl)-4-((6-(3-methoxyazetidin-1-yl)-[1,2,4]triazolo[1,5-a]pyridin-2-yl)amino)pyridin-2-yl)cyclopropanecarboxamide (176)

To a solution of **176a** (20 mg, 0.077 mmol) and **64a** (17 mg, 0.077 mmol) in dioxane (4 mL) was added XPhos Pd G3 (6 mg, 0.0077 mmol), XPhos (7 mg, 0.015 mmol), Cs₂CO₃ (50 mg, 0.15 mmol) and molecular sieve under N₂ atmosphere, the mixture was stirred at 90 °C for 1 h. The reaction mixture was diluted with water (50 mL), and extracted with EtOAc (50 mL*2). The combined organic layer was washed with brine (50 mL*2) and dried over anhydrous Na₂SO₄, then it was filtered and concentrated. The residue was purified by *Prep-HPLC* (Method D) to give **176** (8 mg, 0.018 mmol, 24% yield) as an off-white solid. LC-MS (ESI, Method 4) t_{R} = 0.72 min, m/z (M+H)⁺ = 444.2. ¹H NMR (400 MHz, DMSO-*d*6) *δ* 10.81 (s, 1H), 8.93 (s, 1H), 8.28 (s, 1H), 7.92 (d, *J* = 2.4 Hz, 1H), 7.83 (t, *J* = 4.4 Hz, 1H), 7.55 (d, *J* = 9.6 Hz, 1H), 7.12 (dd, *J* = 9.6, 2.4 Hz, 1H), 4.39-4.27 (m, 1H), 4.18-4.04 (m, 2H), 3.66 (dd, *J* = 8.4, 4.4 Hz, 2H), 3.25 (s, 3H), 2.14 (t, *J* = 19.6 Hz, 3H), 2.04-1.97 (m, 1H), 0.89-0.73 (m, 4H).

### Example 177

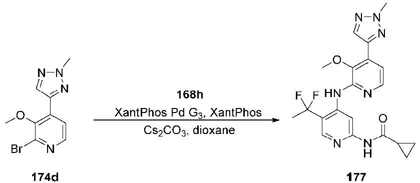

### Step 1. N-(5-(1,1-difluoroethyl)-4-((3-methoxy-4-(2-methyl-2H-1,2,3-triazol-4-yl)pyridin-2-yl)amino)pyridin-2-yl)cyclopropanecarboxamide (177)

To a solution of 174d (45 mg, 0.19 mmol) and 168h (50 mg, 0.19 mmol) in dioxane (4 mL) was added XantPhos Pd G3 (35 mg, 0.037mol), XantPhos (43 mg, 0.074 mmol), Cs2CO3 (121 mg, 0.37 mmol) and molecular sieve under N2 atmosphere, the mixture was stirred at 90 °C for 2 h. The reaction mixture was diluted with water (50 mL) and extracted with EtOAc (50 mL*2). The combined organic layer was washed with brine (50 mL*2) and dried over anhydrous Na2SO4, then it was filtered and concentrated. The residue was purified by Prep-HPLC (Method D) to give 177 (19 mg, 24% yield) as a white solid. LC-MS (ESI, Method 4) tR = 0.82 min, m/z (M+H)+ = 430.2. 1H NMR (400 MHz, DMSO-d6) δ 10.84 (s, 1H), 9.47 (s, 1H), 8.34 (s, 1H), 8.32-8.27 (m, 2H), 8.12 (d, J = 5.2 Hz, 1H), 7.44 (d, J = 5.2 Hz, 1H), 4.27 (s, 3H), 3.73 (s, 3H), 2.19 (t, J = 20.0 Hz, 3H), 2.08-2.00 (m, 1H), 0.86-0.77 (m, 4H).

### Example 178

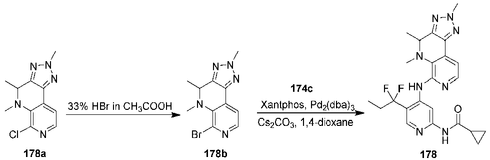

### Step 1. 6-Bromo-2,4,5-trimethyl-4,5-dihydro-2H-[1,2,3]triazolo[4,5-c][1,7]naphthyridine (178b)

A mixture of **178a** (120 mg, 0.481 mmol, WO 2022175747 P130, Example 25) in HBr (1 mL, 33% in acetic acid) was stirred at 50 °C for 16 h. After cooling to room temperature, the suspension was filtered, and the filtered cake was washed with ACN (6 mL). The filtered cake was dried to afford **178b** (120 mg, 85% yield) as a yellow solid. LC-MS (ESI, Method 3): t_{R} = 1.20 min, m/z (M+H)⁺= 294.0.

### Step 2. N-(5-(1,1-difluoropropyl)-4-((2,4,5-trimethyl-4,5-dihydro-2H-[1,2,3]triazolo[4,5-c][1,7]naphthyridin-6-yl)amino)pyridin-2-yl)cyclopropanecarboxamide (178)

A mixture of **174c** (40 mg, 0.157 mmol), **178b** (69.0 mg, 0.235 mmol), XantPhos (18.1 mg, 0.031 mmol), Pd₂(dba)₃ (28.7 mg, 0.031 mmol) and Cs₂CO₃ (153.2 mg, 0.47 mmol) in 1,4-dioxane (0.5 mL) was stirred at 100 °C for 2 h under N₂ atmosphere. The mixture was concentrated and purified by chromatography column (DCM:MeOH = 20:1) to give the crude product. The crude product was re-purified by *Prep-*HPLC (Method C) to afford **178** (1.7 mg, 2% yield) as a white solid. LC-MS (ESI, Method 2): t_{R} = 1.18 min, m/z (M+H)⁺= 469.1. ¹H NMR (400 MHz, DMSO-*d*₆) δ 10.78 (s, 1H), 9.48 (s, 1H), 8.69 (t, *J* = 4.8 Hz, 1H), 8.24 (s, 1H), 8.15 (d, *J* = 5.2 Hz, 1H), 7.26 (d, *J* = 4.8 Hz, 1H), 4.53 (q, *J* = 6.8 Hz, 1H), 4.24 (s, 3H), 2.48 (s, 3H), 2.45-2.32 (m, 2H), 2.07-1.99 (m, 1H), 1.13 (d, *J* = 7.2 Hz, 3H), 1.04 (t, *J* = 7.2 Hz, 3H), 0.88-0.77 (m, 4H).

### Example 179

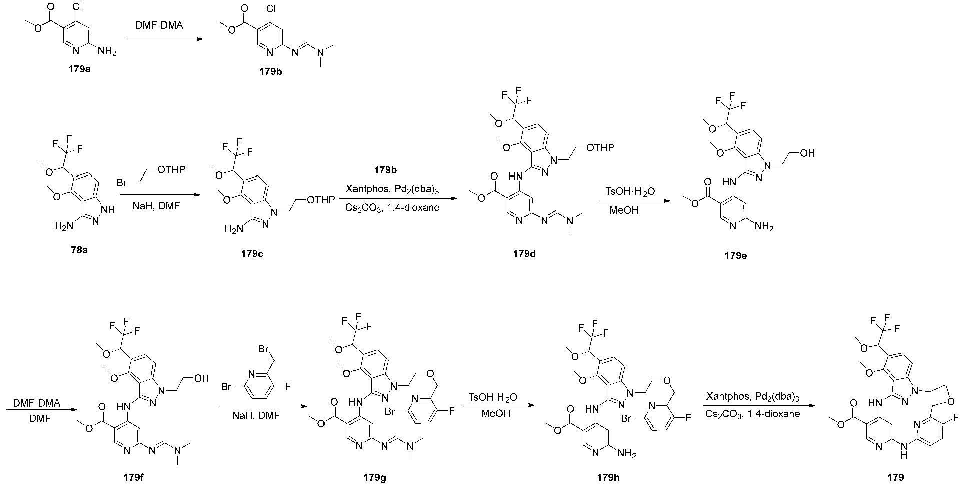

### Step 1. Methyl 4-chloro-6-(((dimethylamino)methylene)amino)nicotinate (179b)

To a solution of **179a** (1 g, 5.37 mmol) in DMF (10 mL) was added DMF-DMA (1.07 g, 8.97 mmol, 1.19 mL). Then the mixture was stirred at 60 °C for 2 h. The mixture was concentrated to **179b** (1.30 g, 100% yield) as yellow oil, which was used for the next step directly without further purification. LC-MS (ESI, Method 3): t_{R} = 0.85 min, m/z (M+H)⁺ = 242.2.

### Step 2. 4-Methoxy-1-(2-((tetrahydro-2H-pyran-2-yl)oxy)ethyl)-5-(2,2,2-trifluoro-1-methoxyethyl)-1H-indazol-3-amine (179c)

To a solution of **78a** (1.2 g, 4.36 mmol) in DMF (25 mL) was added NaH (209.3 mg, 5.23 mmol, 60% dispersion in mineral oil) at 0 °C. The mixture was stirred for 30 mins, then 2-(2-bromoethoxy)tetrahydro-2H-pyran (1.00 g, 4.80 mmol) was added to the mixture. After stirring for 2 h at 15 °C, the reaction mixture was diluted with water (30 ml) and extracted with EtOAc (10 mL*3). The combined organic phase was dried over anhydrous Na₂SO₄, filtered and concentrated. The residue was purified by chromatography column (PE:EtOAc = 1:1) to afford **179c** (1.4 g, 80% yield) as red solid. LC-MS (ESI, Method 3): t_{R} = 1.30 min, m/z (M+H)⁺ = 404.2.

### Step 3. Methyl 6-(((dimethylamino)methylene)amino)-4-((4-methoxy-1-(2-((tetrahydro-2H-pyran-2-yl)oxy)ethyl)-5-(2,2,2-trifluoro-1-methoxyethyl)-1H-indazol-3-yl)amino)nicotinate (179d)

A mixture of **179c** (1.2 g, 2.97 mmol), **179b** (1.01 g, 4.16 mmol), XantPhos (344.3 mg, 0.595 mmol), Pd₂(dba)₃ (544.8 mg, 0.595 mmol) and Cs₂CO₃ (1.94 g, 5.95 mmol) in 1,4-dioxane (12 mL) was stirred at 100 °C for 16 h under N₂ atmosphere. After cooling to room temperature, the reaction mixture was diluted with water (20 mL) and extracted with EtOAc (50 mL*3). The organic layer was concentrated and purified by chromatography column (DCM:MeOH = 20:1) to afford crude **179d** (1.5 g, 83% yield) as a yellow oil. LC-MS (ESI, Method 3): t_{R} = 1.32 min, m/z (M+H)⁺ = 609.3.

### Step 4. Methyl 6-amino-4-((1-(2-hydroxyethyl)-4-methoxy-5-(2,2,2-trifluoro-1-methoxyethyl)-1H-indazol-3-yl)amino)nicotinate (179e)

A mixture of **179d** (1.5 g, 2.46 mmol) and TsOH·H₂O (468.8 mg, 2.46 mmol) in MeOH (10 mL) was stirred at 50 °C for 5 h. After cooling to room temperature, the suspension was filtered, and the filter cake was dried to afford **179e** (950 mg, 82% yield) as a yellow solid. LC-MS (ESI, Method 3): t_{R} = 1.17 min, m/z (M+H)⁺ = 470.2.

### Step 5. Methyl 6-(((dimethylamino)methylene)amino)-4-((1-(2-hydroxyethyl)-4-methoxy-5-(2,2,2-trifluoro-1-methoxyethyl)-1H-indazol-3-yl)amino)nicotinate (179f)

To a solution of **179e** (350 mg, 0.746 mmol) in DMF (3.5 mL) was added DMF-DMA (266.5 mg, 2.24 mmol) at 20 °C. Then the mixture was stirred at 60 °C for 3 h and concentrated to afford **179f** (380 mg, crude) as yellow oil, which was used for the next step directly without further purification. LC-MS (ESI, Method 3): t_{R} = 0.72 min, m/z (M+H)⁺ = 525.3.

### Step 6. Methyl 4-((1-(2-((6-bromo-3-fluoropyridin-2-yl)methoxy)ethyl)-4-methoxy-5-(2,2,2-trifluoro-1-methoxyethyl)-1H-indazol-3-yl)amino)-6-(((dimethylamino)methylene)amino)nicotinate (179g)

To a solution of **179f** (350 mg, 0.667 mmol) and 6-bromo-2-(bromomethyl)-3-fluoropyridine (538.3 mg, 2.00 mmol, CAS 1187836-89-0) in DMF (3 mL) was added NaH (80 mg, 2.00 mmol, 60% dispersion in mineral oil) at 0 °C. Then the mixture was stirred at 0 °C for 1 h and 15 °C for 1 h. The reaction was quenched with ice-water (8 mL) and extracted with DCM/ACN (15 mL*2, v:v = 5:1). The combined organic layer was concentrated and purified by *Prep-*HPLC (Method A) to afford 179g (100 mg, 21% yield) as a yellow solid. LC-MS (ESI, Method 3): t_{R} = 0.84 min, m/z (M+H)⁺ = 712.2.

### Step 7. Methyl 6-amino-4-((1-(2-((6-bromo-3-fluoropyridin-2-yl)methoxy)ethyl)-4-methoxy-5-(2,2,2-trifluoro-1-methoxyethyl)-1H-indazol-3-yl)amino)nicotinate (179h)

A mixture of **179g** (100 mg, 0.140 mmol) and TsOH·H₂O (53.4 mg, 0.281 mmol) in MeOH (1 mL) was stirred at 70 °C for 4 h. The mixture was purified by *Prep*-HPLC (Method A) to afford **179h** (18 mg, 19% yield) as a yellow solid. LC-MS (ESI, Method 3): t_{R} = 1.36 min, m/z (M+H)⁺ = 659.0.

### Step 8. Methyl 12-fluoro-23-methoxy-22-(2,2,2-trifluoro-1-methoxyethyl)-15-oxa-2,6,8,18,25,26-hexaazapentacyclo[16.6.1.1^{3,7}.1^{9,13}.0^{19,24}]heptacosa-1(25),3(27),4,6,9,11,13(26),19,21,23-decaene-4-carboxylate (179)

A mixture of **179h** (18 mg, 0.0274 mmol), XantPhos (3.2 mg, 0.0055 mmol), Pd₂(dba)₃ (5.0 mg, 0.0055 mmol) and Cs₂CO₃ (17.8 mg, 0.0548 mmol) in 1,4-dioxane (2 mL) was stirred at 90 °C for 3 h under N₂ atmosphere. The mixture was concentrated and purified by *Prep*-TLC (DCM:MeOH = 10:1) to give crude product. The crude product was re-purified by *Prep-HPLC* (Method A) to afford **179** (2.5 mg, 16% yield) as a white solid. LC-MS (ESI, Method 2): t_{R} = 1.26 min, m/z (M+H)⁺= 577.0. ¹H NMR (400 MHz, DMSO-*d*₆) δ 10.75 (s, 1H), 10.10 (s, 1H), 9.57 (s, 1H), 8.72 (s, 1H), 7.62 (d, *J* = 9.2 Hz, 1H), 7.57 (d, *J* = 8.8 Hz, 1H), 7.40 (d, *J* = 8.8 Hz, 1H), 7.09 (dd, *J* = 3.2, 9.2 Hz, 1H), 5.33-5.25 (m, 1H), 4.67-4.61 (m, 4H), 3.96 (t, *J* = 4.4 Hz, 2H), 3.91 (s, 3H), 3.87 (s, 3H), 3.30 (s, 3H).

### Example 180

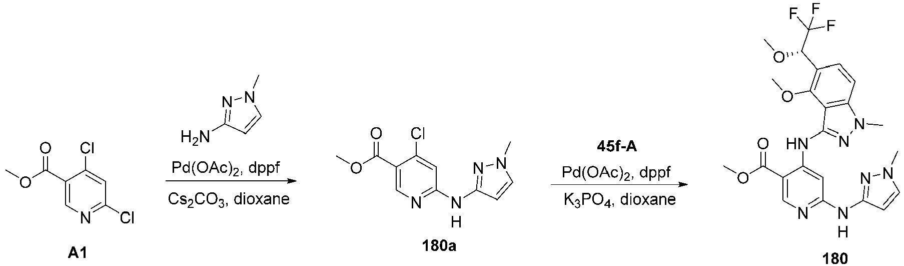

### Step 1. Methyl 4-chloro-6-((1-methyl-1H-pyrazol-3-yl)amino)nicotinate (180a)

A mixture of **A1** (100 mg, 0.49 mmol), 1-methyl-1*H*-pyrazol-3-amine (47 mg, 0.49 mmol), dppf (54 mg, 0.097 mmol), Pd(OAc)₂ (11 mg, 0.049 mmol), Cs₂CO₃ (316 mg, 0.97 mmol) in dioxane (2 mL) was stirred at 90 °C for 5 h under N₂ atmosphere. The mixture was concentrated and purified by chromatography column (PE:EtOAc = 1:1) to give the compound **180a** (100 mg, 77% yield) as a white solid. LC-MS (ESI, Method 4) t_{R} = 0.72 min, m/z (M+H)⁺ = 267.0. ¹H NMR (400 MHz, CDCl₃) δ 8.76 (s, 1H), 7.49 (s, 1H), 7.41 (s, 1H), 7.30 (d,*J* = 2.4 Hz, 1H), 6.14 (d, *J=* 2.0 Hz, 1H), 3.90 (s, 3H), 3.86 (s, 3H).

### Step 2. Methyl (S)-4-((4-methoxy-1-methyl-5-(2,2,2-trifluoro-1-methoxyethyl)-1H-indazol-3-yl)amino)-6-((1-methyl-1H-pyrazol-3-yl)amino)nicotinate (180)

A mixture of **180a** (20 mg, 0.075 mmol), **45f-A** (20 mg, 0.069 mmol), K₃PO₄ (29 mg, 0.136 mmol), dppf (8 mg, 0.014 mmol) and Pd(OAc)₂ (2 mg, 0.007 mmol) in dioxane (1 mL) was stirred at 90 °C for 16 h under N₂ atmosphere. The mixture was concentrated and purified by chromatography column (DCM:MeOH = 25:1) to give the crude compound, then it was purified by *Prep*-HPLC (Method D) to give the compound **180** (3.5 mg, 10% yield) as a white solid. LC-MS (ESI, Method 4) t_{R} = 0.86 min, m/z (M+H)⁺ = 520.2. ¹H NMR (400 MHz, DMSO-*d*₆) δ 10.91 (s, 1H), 9.62 (s, 1H), 8.63 (s, 1H), 8.47 (s, 1H), 7.55 (d, *J* = 2.4 Hz, 1H), 7.43 (d, *J* = 9.2 Hz, 1H), 7.38 (d, *J* = 9.2 Hz, 1H), 6.18 (d, *J* = 2.4 Hz, 1H), 5.26 (q, *J* = 6.8 Hz, 1H), 3.99 (s, 3H), 3.87 (s, 3H), 3.85 (s, 3H), 3.76 (s, 3H), 3.28 (s, 3H).

### Example 181

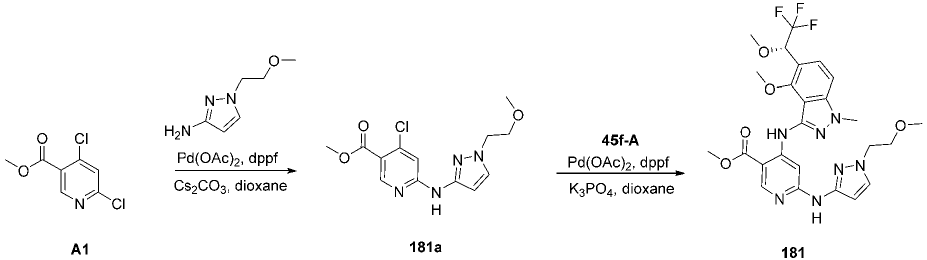

### Step 1. Methyl 4-chloro-6-((1-(2-methoxyethyl)-1H-pyrazol-3-yl)amino)nicotinate (181a)

A mixture of **A1** (50 mg, 0.24 mmol), 1-(2-methoxyethyl)-1*H*-pyrazol-3-amine (33 mg, 0.23mmol), dppf (27 mg, 0.048 mmol), Pd(OAc)₂ (4 mg, 0.024 mmol), Cs₂CO₃ (158 mg, 0.49 mmol) in dioxane (1 mL) was stirred at 90 °C for 5 h under N₂ atmosphere. The mixture was concentrated and purified by chromatography column (PE:EtOAc = 1:1) to give the compound **181a** (50 mg, 66% yield) as a white solid. LC-MS (ESI, Method 4) t_{R} = 1.11 min, m/z (M+H)⁺ = 311.0. ¹H NMR (400 MHz, CDCl₃) δ 8.75 (s, 1H), 7.73 (s, 1H), 7.51 (s, 1H), 7.41 (d, *J* = 2.0 Hz, 1H), 6.15 (d, *J* = 2.0 Hz, 1H), 4.23 (t, *J* = 4.8 Hz, 2H), 3.91 (s, 3H), 3.75 (t, *J* = 4.8 Hz, 2H), 3.35 (s, 3H).

### Step 2. Methyl (S)-4-((4-methoxy-1-methyl-5-(2,2,2-trifluoro-1-methoxyethyl)-1H-indazol-3-yl)amino)-6-((1-(2-methoxyethyl)-1H-pyrazol-3-yl)amino)nicotinate (181)

A mixture of 181a (45 mg, 0.145 mmol), **45f-A** (30 mg, 0.104 mmol), K3PO4 (44 mg, 0.207 mmol), dppf (12 mg, 0.021 mmol) and Pd(OAc)₂ (2 mg, 0.01 mmol) in dioxane (1 mL) was stirred at 90 °C for 16 h under N₂ atmosphere. The mixture was concentrated and purified by chromatography column (DCM:MeOH = 25:1) to give the compound **181** (2.4 mg, 4% yield) as a white solid. LC-MS (ESI, Method 4) t_{R} = 1.52 min, m/z (M+H)⁺ = 564.2. ¹H NMR (400 MHz, CDCl₃) δ 11.30 (s, 1H), 8.72 (s, 1H), 8.65 (s, 1H), 7.55 (d, *J* = 9.2 Hz, 1H), 7.44 (s, 1H), 7.16 (d, *J* = 8.8 Hz, 1H), 6.41 (s, 1H), 5.25-5.07 (m, 1H), 4.32-4.15 (m, 2H), 4.02 (s, 3H), 4.00 (s, 3H), 3.97 (s, 3H), 3.81-3.66 (m, 2H), 3.37 (s, 3H), 3.34 (s, 3H).

### Example 182

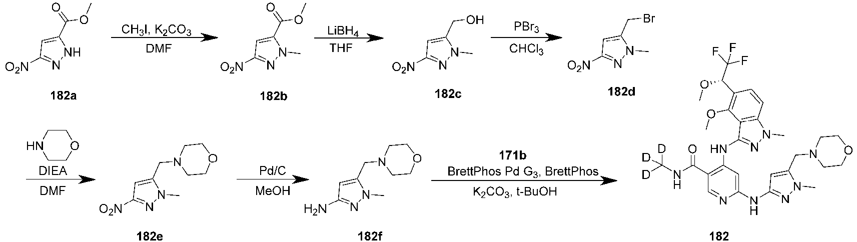

### Step 1. Methyl 1-methyl-3-nitro-1H-pyrazole-5-carboxylate (182b)

To a solution of **182a** (2 g, 11.69 mmol) and K₂CO₃ (1.94 g, 14.03 mmol) in DMF (20 mL) was added CH₃I (1.82 g, 12.86 mmol, 0.80 mL) slowly at 0 °C, the mixture was stirred at 0 °C for 20 min and 25 °C for 12 h. The reaction mixture was diluted with water (100 mL) and extracted with EtOAc (80 mL*2). The combined organic layer was washed with brine (80 mL*2) and dried over anhydrous Na₂SO₄, then it was filtered and concentrated. The residue was purified by chromatography column (EtOAc:PE = 1:4) to give **182b** (2.06 g, 95% yield) as a white solid. LC-MS (ESI, Method 4) t_{R} = 0.71 min, m/z (M+H)⁺ = 186.0. ¹H NMR (400 MHz, DMSO-*d*₆) δ 7.56 (s, 1H), 4.19 (s, 3H), 3.89 (s, 3H).

### Step 2. (1-Methyl-3-nitro-1H-pyrazol-5-yl)methanol (182c)

To a solution of **182b** (2.06 g, 11.13 mmol) in THF (40 mL) was added LiBH₄ (11.13 mL, 22.25 mmol, 2 M in THF) under N₂ atmosphere at 0 °C, the mixture was stirred at 0 °C for 20 min and 25 °C for 3 h. The reaction mixture was quenched with saturated Na₂CO₃ (100 mL) and extracted with EtOAc (80 mL*2). The combined organic layer was washed with brine (80 mL*2) and dried over anhydrous Na₂SO₄, then it was filtered and concentrated. The residue was purified by chromatography column (EtOAc:PE = 1:1) to give **182c** (1.21 g, 69% yield) as a white solid. LC-MS (ESI, Method 4) t_{R} = 0.53 min, m/z (M+H)⁺ = 158.0. ¹H NMR (400 MHz, DMSO-*d*₆) δ 6.94 (s, 1H), 5.57 (t, *J* = 5.6 Hz, 1H), 4.55 (d, *J* = 5.6 Hz, 2H), 3.91 (s, 3H).

### Step 3. 5-(Bromomethyl)-1-methyl-3-nitro-1H-pyrazole (182d)

To a solution of **182c** (500 mg, 3.18 mmol) in CHCl₃ (20 mL) was added PBr₃ (861 mg, 3.18 mmol, 0.30 mL) at 0 °C, the mixture was stirred at 60 °C for 2 h. The reaction mixture was quenched with saturated NaHCO₃ (50 mL) and extracted with EtOAc (50 mL*2). The combined organic layer was washed with brine (50 mL*2) and dried over anhydrous Na₂SO₄, then it was filtered and concentrated. The residue was purified by chromatography column (PE:EtOAc = 1:3) to give **182d** (696 mg, 3.16 mmol, 99% yield) as a white solid. LC-MS (ESI, Method 4) t_{R} = 0.73 min, m/z (M+H)⁺ = 220.0.

### Step 4. 4-((1-Methyl-3-nitro-1H-pyrazol-5-yl)methyl)morpholine (182e)

To a solution of **182d** (300 mg, 1.36 mmol) and morpholine (143 mg, 1.64 mmol, 0.14 mL) in DMF (20 mL) was added DIEA (441 mg, 3.41 mmol, 0.59 mL), the mixture was stirred at 65 °C for 4 h. The reaction mixture was diluted with water (50 mL) and extracted with EtOAc (50 mL*2). The combined organic layer was washed with brine (50 mL*2) and dried over anhydrous Na₂SO₄, then it was filtered and concentrated. The residue was purified by chromatography column (PE:EtOAc = 1:1) to give **182e** (272 mg, 88% yield) as a white solid. LC-MS (ESI, Method 4) t_{R} = 0.33 min, m/z (M+H)⁺ = 227.0. ¹H NMR (400 MHz, DMSO-*d*₆) *δ* 6.98 (s, 1H), 3.94 (s, 3H), 3.61-3.52 (m, 6H), 2.39 (t, *J* = 4.8 Hz, 4H).

### Step 5. 1-Methyl-5-(morpholinomethyl)-1H-pyrazol-3-amine (182f)

To a solution of **182e** (30 mg, 0.13 mmol) in MeOH (4 mL) was added Pd/C (3 mg, 10% on carbon) under H₂ atmosphere, the mixture was stirred at 50 °C for 1 h. The reaction mixture was filtered, and the filtrate was concentrated to give **182f** (35 mg, crude) as a white solid, which was used for the next step directly without further purification. LC-MS (ESI, Method 4) t_{R} = 0.21 min, m/z (M+H)⁺ = 197.0.

### Step 6. (S)-4-((4-methoxy-1-methyl-5-(2,2,2-trifluoro-1-methoxyethyl)-1H-indazol-3-yl)amino)-N-(methyl-d3)-6-((1-methyl-5-(morpholinomethyl)-1H-pyrazol-3-yl)amino)nicotinamide (182)

To a solution of **182f** (13 mg, 0.065 mmol) and **171b** (20 mg, 0.043 mmol) in t-BuOH (2 mL) was added BrettPhos Pd G3 (6 mg, 0.0065 mmol), BrettPhos (6 mg, 0.011 mmol) and K₂CO₃ (21 mg, 0.15 mmol) under N₂ atmosphere, the mixture was stirred at 110 °C for 4 h. The reaction mixture was diluted with water (30 mL) and extracted with EtOAc (30 mL*2). The combined organic layer was washed with brine (20 mL*2) and dried over anhydrous Na₂SO₄, then it was filtered and concentrated. The residue was purified by *Prep*-TLC (DCM:MeOH = 92:8) to give **182** (19 mg, 69% yield) as an off-white solid. LC-MS (ESI, Method 4) t_{R} = 0.71 min, m/z (M+H)⁺ = 621.4. ¹H NMR (400 MHz, DMSO-*d*₆) δ 11.72 (s, 1H), 8.74-8.12 (m, 3H), 7.53-7.35 (m, 2H), 6.17 (s, 1H), 5.30 (q, *J* = 6.8 Hz, 1H), 4.02 (s, 3H), 3.91 (s, 3H), 3.80 (s, 3H), 3.58 (t, *J* = 4.8 Hz, 4H), 3.53 (s, 2H), 3.32 (s, 3H), 2.40 (s, 4H).

### Example 183

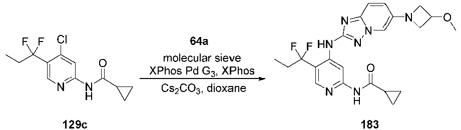

### Step 1. N-(5-(1,1-difluoropropyl)-4-((6-(3-methoxyazetidin-1-yl)-[1,2,4]triazolo[1,5-a]pyridin-2-yl)amino)pyridin-2-yl)cyclopropanecarboxamide (183)

To a solution of **129c** (40 mg, 0.15 mmol) and **64a** (32 mg, 0.15 mmol) in dioxane (3 mL) was added XPhos Pd G3 (12 mg, 0.015 mmol), XPhos (14 mg, 0.029 mmol), Cs₂CO₃ (95 mg, 0.29 mmol) and molecular sieve under N₂ atmosphere, the mixture was stirred at 90 °C for 2 h. The reaction mixture was diluted with water (50 mL) and extracted with EtOAc (50 mL*2). The combined organic layer was washed with brine (50 mL*2) and dried over anhydrous Na₂SO₄, then it was filtered and concentrated. The residue was purified by *Prep-HPLC* (Method D) to give **183** (23 mg, 34% yield) as a white solid. LC-MS (ESI, Method 4) t_{R} = 0.80 min, m/z (M+H)⁺ = 458.2. ¹H NMR (400 MHz, DMSO-*d*₆) *δ* 10.79 (s, 1H), 8.87 (s, 1H), 8.20 (s, 1H), 7.94-7.90 (m, 1H), 7.86 (t, *J* = 4.4 Hz, 1H), 7.58-7.51 (m, 1H), 7.11 (dd, *J* = 9.6, 2.4 Hz, 1H), 4.36-4.27 (m, 1H), 4.14-4.06 (m, 2H), 3.66 (dd, *J* = 8.4, 4.4 Hz, 2H), 3.25 (s, 3H), 2.43-2.30 (m, 2H), 2.07-1.95 (m, 1H), 1.01 (t, *J* = 7.6 Hz, 3H), 0.84-0.76 (m, 4H).

### Example 184

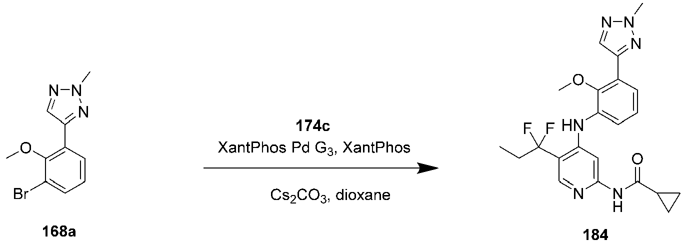

### Step 1. N-(5-(1,1-difluoropropyl)-4-((2-methoxy-3-(2-methyl-2H-1,2,3-triazol-4-yl)phenyl)amino)pyridin-2-yl)cyclopropanecarboxamide (184)

To a solution of **168a** (42 mg, 0.16 mmol) and **174c** (4 mL) was added XantPhos Pd G3 (30 mg, 0.031 mmol), XantPhos (36 mg, 0.063 mmol), Cs₂CO₃ (103 mg, 0.31 mmol) and molecular sieve under N₂ atmosphere, the mixture was stirred at 120 °C for 12 h. The reaction mixture was diluted with water (50 mL) and extracted with EtOAc (50 mL*2). The combined organic layer was washed with brine (50 mL*2) and dried over anhydrous Na₂SO₄, then it was filtered, and concentrated. The residue was purified by *Prep-*HPLC (Method D) to give **184** (7 mg, 9% yield) as a white solid. LC-MS (ESI, Method 4) t_{R} = 0.83 min, m/z (M+H)⁺ = 443.2. ¹H NMR (400 MHz, DMSO-*d*₆) *δ* 10.71 (s, 1H), 8.13 (s, 1H), 8.09 (s, 1H), 7.75 (s, 1H), 7.71 (dd, *J* = 8.0, 1.6 Hz, 1H), 7.45-7.41 (m, 1H), 7.37-7.32 (m, 1H), 7.26 (t, *J* = 8.0 Hz, 1H), 4.23 (s, 3H), 3.59 (s, 3H), 2.47-2.29 (m, 2H), 1.99-1.88 (m, 1H), 1.02 (t, *J* = 7.2 Hz, 3H), 0.77-0.66 (m, 4H).

### Example 185

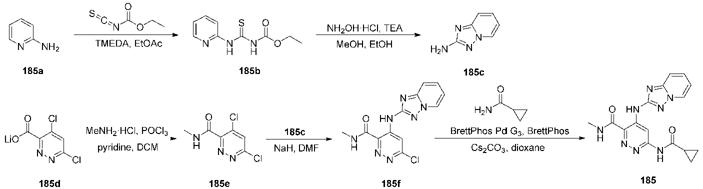

### Step 1. Ethyl N-[(2-pyridinylamino)thioxomethyl]carbamate (185b)

To a solution of **185a** (500 mg, 5.31 mmol) and TMEDA (62 mg, 0.53 mmol, 0.08 mL) in EtOAc (8 mL) was added ethyl isothiocyanatoformate (766 mg, 5.84 mmol, 0.69 mL), the mixture was stirred at 25 °C for 3 h. The mixture was concentrated and purified by chromatography column (EtOAc:PE = 1:4) to give **185b** (1.12 g, 94% yield) as a white solid. LC-MS (ESI, Method 4) t_{R} = 0.78 min, m/z (M+H)⁺ = 226.0.

### Step 2. [1,2,4]Triazolo[1,5-a]pyridin-2-amine (185c)

To a solution of **185b** (500 mg, 2.22 mmol) and TEA (449 mg, 4.44 mmol, 0.62 mL) in MeOH (5 mL) and EtOH (5 mL) was added hydroxylamine hydrochloride (463 mg, 6.66 mmol), the mixture was stirred at 60 °C for 2 h. The mixture was concentrated and purified by chromatography column (EtOAc) to give **185c** (221 mg, 1.65 mmol, 74% yield) as a white solid. LC-MS (ESI, Method 4) t_{R} = 0.35 min, m/z (M+H)⁺ = 135.0. ¹H NMR (400 MHz, Chloroform-*d*) δ 8.29 (m, 1H), 7.42-7.37 (m, 2H), 6.82 (m, 1H), 4.43 (s, 2H).

### Step 3. 4,6-Dichloro-N-methylpyridazine-3-carboxamide (185e)

To a solution of **185d** (1 g, 5.03 mmol) and methylamine hydrochloride (441 mg, 6.54 mmol) in DCM (10 mL) was added pyridine (1.99 g, 25.14 mmol, 2.02 mL) at 0 °C, then to the reaction mixture was added POCl₃ (3.85 g, 25.14 mmol, 2.34 mL), and it was stirred at 25 °C for 12 h. The reaction mixture was quenched with saturated NaHCO₃ (50 mL) and extracted with EtOAc (50 mL*2). The combined organic layer was washed with brine (50 mL*2) and dried over anhydrous Na₂SO₄, then it was filtered and concentrated. The residue was purified by chromatography column (EtOAc:PE = 1:1) to give **185e** (718 mg, 69% yield) as a white solid. LC-MS (ESI, Method 4) t_{R} = 0.40 min, m/z (M+H)⁺ = 206.0. ¹H NMR (400 MHz, DMSO-*d*₆) δ 8.92 (s, 1H), 8.49 (s, 1H), 2.83 (d, *J* = 4.8 Hz, 3H).

### Step 4. 4-([1,2,4]Triazolo[1,5-a]pyridin-2-ylamino)-6-chloro-N-methylpyridazine-3-carboxamide (185f)

To a solution of **185e** (150 mg, 0.73 mmol) and **185c** (98 mg, 0.73 mmol) in DMF (5 mL) was added NaH (87 mg, 2.18 mmol, 60% in mineral oil) , the mixture was stirred at 25 °C for 3 h. The reaction mixture was quenched with saturated NH₄Cl (50 mL), an off-white precipitate was formed. The precipitate was collected by filtration to give **185f** (450 mg, crude) as an off-white solid. The crude product was used in the next step without further purification. LC-MS (ESI, Method 4) t_{R} = 0.80 min, m/z (M+H)⁺ = 304.0.

### Step 5. 4-([1,2,4]Triazolo[1,5-a]pyridin-2-ylamino)-6-(cyclopropanecarboxamido)-N-methylpyridazine-3-carboxamide (185)

To a solution of **185f** (400 mg, 1.32 mmol) and cyclopropanecarboxamide (224 mg, 2.63 mmol) in dioxane (10 mL) was added BrettPhos Pd G3 (239 mg, 0.26 mmol), BrettPhos (283 mg, 0.53 mmol) and Cs₂CO₃ (1.29 g, 3.95 mmol) under N₂ atmosphere, the mixture was stirred at 90 °C for 3 h. The reaction mixture was diluted with water (30 mL) and extracted with EtOAc (30 mL*2). The combined organic layer was washed with brine (20 mL*2) and dried over anhydrous Na₂SO₄, then it was filtered and concentrated. The residue was purified by *Prep-*TLC (MeOH:DCM = 5:95) to give **185** (9 mg, 2% yield) as an off-white solid. LC-MS (ESI, Method 4) t_{R} = 0.70 min, m/z (M+H)⁺ = 353.0. ¹H NMR (400 MHz, DMSO-*d*₆) *δ* 12.14 (s, 1H), 11.43 (s, 1H), 9.44 (s, 1H), 9.37-9.30 (m, 1H), 8.83 (d, *J* = 6.8 Hz, 1H), 7.73-7.63 (m, 2H), 7.18-7.12 (m, 1H), 2.87 (d, *J* = 4.8 Hz, 3H), 2.21-2.08 (m, 1H), 0.93-0.87 (m, 4H).

### Example 186

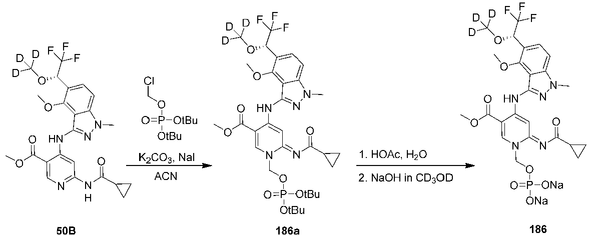

### Step 1. Methyl (S,E)-6-((cyclopropanecarbonyl)imino)-1-(((di-tert-butoxyphosphoryl)oxy)methyl)-4-((4-methoxy-1-methyl-5-(2,2,2-trifluoro-1-(methoxy-d3)ethyl)-1H-indazol-3-yl)amino)-1,6-dihydropyridine-3-carboxylate (186a)

A mixture of **50B** (150 mg, 0.293 mmol), K₂CO₃ (121.8 mg, 0.881 mmol) in acetonitrile (5 mL) was stirred for 30 min at room temperature. To the reaction mixture was added NaI (132.1 mg, 0.881 mmol) followed by di-*tert*-butyl (chloromethyl) phosphate (228 mg, 0.881 mmol). The reaction was stirred at 50 °C for 18 h. After cooling to room temperature, the reaction mixture was diluted with DCM and MeOH (5 mL and 5 mL), then it was filtered and concentrated to afford the crude compound **186a** (215 mg) as a yellow solid which was used for the next step without purification. LC-MS (ESI, Method 3): t_{R} = 1.39 min, m/z (M+H)⁺ = 733.4.

### Step 2. Sodium (S,E)-(2-((cyclopropanecarbonyl)imino)-4-((4-methoxy-1-methyl-5-(2,2,2-trifluoro-1-(methoxy-d3)ethyl)-1H-indazol-3-yl)amino)-5-(methoxycarbonyl)pyridin-1(2H)-yl)methyl phosphate (186)

A mixture of **186a** (110 mg, 0.150 mmol) in AcOH (1 mL) and H₂O (0.5 mL) was stirred at 45 °C for 2 h. After cooling to room temperature, the reaction mixture was diluted with water (15 mL) and the formed brown solid was collected by filtration. The solid was sonicated in DCM (5 mL) for 0.5 h and filtered to afford the phosphoric acid (60 mg, 64% yield). The solid (10 mg, 0.016 mmol) was suspension in 0.2 mL of CD₃OD, and NaOH solution (2.0 mg, 0.05 mmol, in 0.3 mL of CD₃OD) was added to the mixture until the solid was dissolved to afford **186** (91% purity in CD₃OD). LC-MS (ESI, Method 3): t_{R} = 1.11 min, m/z (M-2Na+H)⁺ = 621.0. ¹H NMR (400 MHz, CD₃OD) δ 9.18 (s, 1H), 8.91 (s, 1H), 7.50 (d, *J* = 8.8 Hz, 1H), 7.32 (d, *J* = 8.8 Hz, 1H), 5.83 (d, *J* = 8.4 Hz, 2H), 5.21-5.19 (m, 1H), 4.04 (s, 3H), 3.96 (s, 3H), 3.47 (s, 3H), 1.75-1.70 (m, 1H), 1.02-1.00 (m, 2H), 0.78-0.75 (m, 2H).

### Example 187

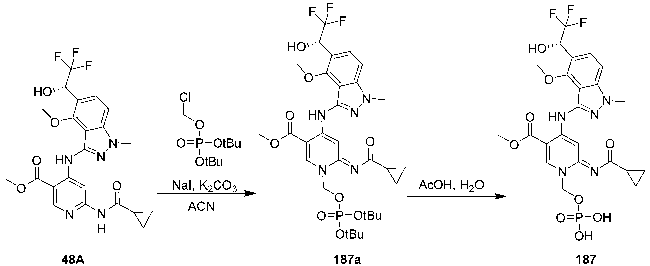

### Step 1. Methyl (S,E)-6-((cyclopropanecarbonyl)imino)-1-(((di-tert-butoxyphosphoryl)oxy)methyl)-4-((4-methoxy-1-methyl-5-(2,2,2-trifluoro-1-hydroxyethyl)-1H-indazol-3-yl)amino)-1,6-dihydropyridine-3-carboxylate (187a)

A mixture of **48A** (100 mg, 0.203 mmol) and K₂CO₃ (84.0 mg, 0.608 mmol) in acetonitrile (2 mL) was stirred for 30 min at room temperature. To the reaction mixture was added NaI (91.1 mg, 0.608 mmol) followed by di-*tert*-butyl (chloromethyl) phosphate (157.3 mg, 0.608 mmol). The reaction was stirred at 50 °C for 18 h. After cooling to room temperature, the reaction mixture was diluted with DCM and MeOH (5 mL and 5 mL), then it was filtered and concentrated to afford the crude compound **187a** (145 mg) as a yellow solid which was used for the next step without purification. LC-MS (ESI, Method 3): t_{R} = 1.02 min, m/z (M+H)⁺ = 716.3.

### Step 2. Methyl (S,E)-6-((cyclopropanecarbonyl)imino)-4-((4-methoxy-1-methyl-5-(2,2,2-trifluoro-1-hydroxyethyl)-1H-indazol-3-yl)amino)-1-((phosphonooxy)methyl)-1,6-dihydropyridine-3-carboxylate (187)

A mixture of **187a** (145 mg, 0.203 mmol) in AcOH (2 mL) and H₂O (1 mL) was stirred for 2 h at 45 °C. After cooling to room temperature, the reaction mixture was diluted with water (10 mL) and concentrated. The residue was diluted with 5 mL of water and lyophilized. The obtained crude product was sonicated in DCM (5 mL) for 0.5 h and filtered to afford **187** (28 mg, 23% yield) as a yellow solid. LC-MS (ESI, Method 3): t_{R} = 0.73 min, m/z (M+H)⁺ = 604.2. ¹H NMR (400 MHz, DMSO-*d*₆) δ 11.49 (s, 1H), 9.41 (s, 1H), 9.26 (s, 1H), 7.60 (d, *J* = 8.8 Hz, 1H), 7.53 (d, *J* = 8.8 Hz, 1H), 6.67 (s, 1H), 5.92 (d, *J* = 12.0 Hz, 2H), 5.28-5.17 (m, 1H), 4.01 (s, 3H), 3.91 (s, 3H), 3.72 (s, 3H), 2.01-1.95 (m, 1H), 0.96-0.93 (m, 2H), 0.85-0.82 (m, 2H).

### Example 188

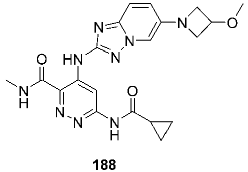

### Synthesis of 6-(Cyclopropanecarboxamido) -4- ((6-(3-methoxyazetidin-1-yl) -[1,2,4] triazolo [1,5-a] pyridin-2-yl)amino)-N-methylpyridazine-3-carboxamide (188)

The synthesis refers to the patent (WO2021170046A1, page 28-30). LC-MS (ESI, Method 4), tR = 2.96 min, m/z (M+H)⁺ = 438.1. ¹H NMR (400 MHz, DMSO-*d*₆) δ 12.00 (s, 1H), 11.37 (s, 1H), 9.34 (s, 1H), 9.30 (s, 1H), 7.92 (s, 1H), 7.57 (d, *J* = 9.2 Hz, 1H), 7.12 (d, *J* = 8.4 Hz, 1H), 4.33 (s, 1H), 4.11 (t, *J* = 7.2 Hz, 2H), 3.71-3.65 (m, 2H), 3.25 (s, 3H), 2.87 (d, *J* = 4.8 Hz, 3H), 2.14 (s, 1H), 0.90-0.87 (m, 4H).

### Example 189

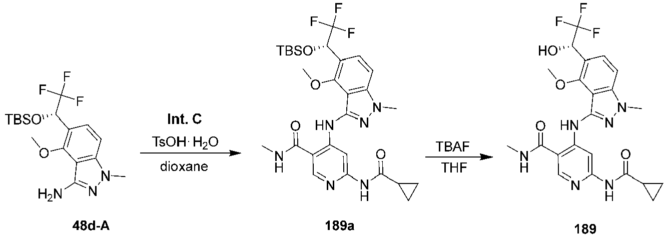

### Step 1. (S)-4-((5-(1-((tert-butyldimethylsilyl)oxy)-2,2,2-trifluoroethyl)-4-methoxy-1-methyl-1H-indazol-3-yl)amino)-6-(cyclopropanecarboxamido)-N-methylnicotinamide (189a)

A mixture of **48d-A** (100 mg, 0.257 mmol), **Int. C** (78.2 mg, 0.308 mmol) and TsOH·H₂O (4.98 mg, 0.026 mmol) in 1,4-dioxane (1 mL) was stirred at 110 °C for 16 h. After the mixture was cooled, it was basified with NH₃·H₂O (0.3 mL) and concentrated. The residue was purified by chromatography column (DCM:MeOH = 20:1) to afford **189a** (150 mg, 96% yield) as a yellow solid. LC-MS (ESI, Method 3): t_{R} = 1.60 min, m/z (M+H)⁺ = 607.3.

### Step 2. (S)-6-(cyclopropanecarboxamido)-4-((4-methoxy-1-methyl-5-(2,2,2-trifluoro-1-hydroxyethyl)-1H-indazol-3-yl)amino)-N-methylnicotinamide (189)

To a solution of **189a** (150 mg, 0.247 mmol) in THF (1.0 mL) was added TBAF (0.5 mL, 1 M in THF) at 25 °C. After stirring at 25 °C for 2 h, the reaction mixture was diluted with water (10 mL) and extracted with EtOAc (20 mL*2). The combined organic layer was concentrated and purified by chromatography column (PE:EtOAc = 1:1) to afford **189** (102 mg, 84% yield) as a white solid. LC-MS (ESI, Method 2): t_{R} = 1.11 min, m/z (M+H)⁺ = 493.1. ¹H NMR (400 MHz, DMSO-*d*₆) δ 11.59 (s, 1H), 10.72 (s, 1H), 9.15 (s, 1H), 8.65 (d, *J* = 4.4 Hz, 1H), 8.57 (s, 1H), 7.52 (d, *J* = 8.8 Hz, 1H), 7.40 (d, *J* = 8.8 Hz, 1H), 6.82 (d, *J* = 5.2 Hz, 1H), 5.46-5.42 (m, 1H), 3.94 (s, 3H), 3.87 (s, 3H), 2.83 (d, *J* = 4.0 Hz, 3H), 2.03-2.01 (m, 1H), 0.85-0.78 (m, 4H).

### Example 190

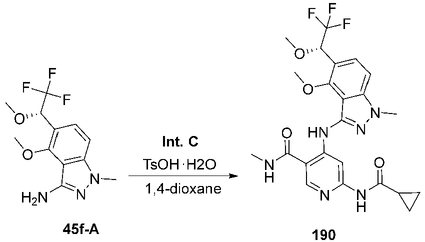

### Step 1. (S)-6-(cyclopropanecarboxamido)-4-((4-methoxy-1-methyl-5-(2,2,2-trifluoro-1-methoxyethyl)-1H-indazol-3-yl)amino)-N-methylnicotinamide (190)

A mixture of **45f-A** (30 mg, 0.104 mmol), **Int. C** (31.6 mg, 0.124 mmol) and TsOH·H₂O (2.0 mg, 0.01 mol) in 1,4-dioxane (0.5 mL) was stirred at 110 °C for 16 h. After the mixture was cooled, it was basified with NH₃·H₂O (0.3 mL) and concentrated. The residue was purified by *Prep-HPLC* (Method A) to afford **190** (22 mg, 42% yield) as a white solid. LC-MS (ESI, Method 2): t_{R} = 1.20 min, m/z (M+H)⁺ = 507.1. ¹H NMR (400 MHz, DMSO-*d*₆) δ 11.61 (s, 1H), 10.72 (s, 1H), 9.14 (s, 1H), 8.67 (d, *J* = 4.0 Hz, 1H), 8.57 (s, 1H), 7.44 (d, *J* = 8.8 Hz, 1H), 7.39 (d, *J* = 8.8 Hz, 1H), 5.29-5.25 (m, 1H), 3.96 (s, 3H), 3.89 (s, 3H), 3.31 (s, 3H), 2.83 (d, *J* = 4.0 Hz, 3H), 2.03-2.01 (m, 1H), 0.82-0.79 (m, 4H).

### Example 191

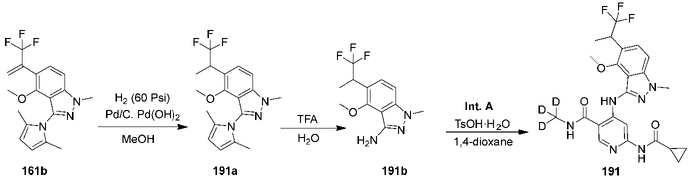

### Step 1. 3-(2,5-Dimethyl-1H-pyrrol-1-yl)-4-methoxy-1-methyl-5-(1,1,1-trifluoropropan-2-yl)-1H-indazole (191a)

A mixture of **161b** (130 mg, 0.372 mmol), Pd(OH)₂ (13 mg, 10% W) and Pd/C (13 mg, 10% on carbon) in MeOH (3 mL) was stirred at room temperature for 5 h under H₂ (60 Psi) atmosphere. The reaction mixture was filtered and concentrated to afford **191a** (120 mg, 92% yield) as yellow oil which was used for the next step without purification. LC-MS (ESI, Method 3): t_{R} = 1.75 min, m/z (M+H)⁺ = 351.8. ¹H NMR (400 MHz, DMSO-*d*₆) δ 7.53 (d, *J* = 8.8 Hz, 1H), 7.47 (d, *J* = 8.8 Hz, 1H), 5.86 (s, 2H), 4.07-4.05 (m, 1H), 3.95 (s, 3H), 3.17 (s, 3H), 1.96 (s, 6H), 1.45 (d, *J* = 6.8 Hz, 3H).

### Step 2. 4-Methoxy-1-methyl-5-(1,1,1-trifluoropropan-2-yl)-1H-indazol-3-amine (191b)

A mixture of **191a** (130 mg, 369.99 µmol) in TFA (0.5 mL) and H₂O (1.5 mL) was stirred 4 h at 100 °C. After cooling to room temperature, the reaction was diluted with EtOAc (10 mL) and washed with *sat.* NaHCO₃ (3 mL*2). The separated organic layer was concentrated and purified by *Prep-*HPLC (Method A) to afford **191b** (20 mg, 20% yield) as a yellow solid. LC-MS (ESI, Method 3): t_{R} = 1.22 min, m/z (M+H)⁺ = 274.2.

### Step 3. 6-(Cyclopropanecarboxamido)-4-((4-methoxy-1-methyl-5-(1,1,1-trifluoropropan-2-yl)-1H-indazol-3-yl)amino)-N-(methyl-d3)nicotinamide (191)

A mixture of **191b** (20 mg, 0.0732 mmol), **Int. A** (28.2 mg, 0.110 mmol) and TsOH·H₂O (3 mg, 0.0158 mmol) in 1,4-dioxane (0.5 mL) was stirred at 110 °C for 16 h. After the mixture was cooled, 0.1 mL NH₃·H₂O was added to it. Then the mixture was purified by reversed-phase chromatograph column (water:MeCN = 95:5 to 5:95, 0.1% HCOOH was contained in water) to afford **191** (18 mg, 50% yield) as a white solid. LC-MS (ESI, Method 2): t_{R} = 1.22 min, m/z (M+H)⁺ = 494.1. ¹H NMR (400 MHz, DMSO-*d*₆) δ 11.59 (s, 1H), 10.71 (s, 1H), 9.16 (s, 1H), 8.62 (s, 1H), 8.56 (s, 1H), 7.42 (d, *J* = 8.8 Hz, 1H), 7.38 (d, *J* = 8.8 Hz, 1H), 4.19-4.07 (m, 1H), 3.93 (s, 3H), 3.84 (s, 3H), 2.09-1.97 (m, 1H), 1.47 (d, *J* = 7.2 Hz, 3H), 0.86-0.76 (m, 4H).

### Example 192

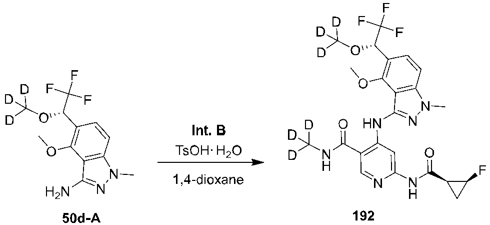

### Step 1. 6-((1S,2S)-2-fluorocyclopropane-1-carboxamido)-4-((4-methoxy-1-methyl-5-((S)-2,2,2-trifluoro-1-(methoxy-d3)ethyl)-1H-indazol-3-yl)amino)-N-(methyl-d3)nicotinamide (192)

A mixture of **50d-A** (35.1 mg, 0.12 mmol), Int. B (30 mg, 0.109 mmol) and TsOH·H2O (2.1 mg, 0.011 mmol) in 1,4-dioxane (0.5 mL) was stirred at 110 °C for 16 h. After the mixture was cooled and concentrated, it was purified by chromatography column (DCM:MeOH = 20:1) to afford **192** (39 mg, 67% yield) as a white solid. LC-MS (ESI, Method 3): t_{R} = 1.19 min, m/z (M+H)⁺ = 531.1. ¹H NMR (400 MHz, DMSO-*d*₆) δ 11.64 (s, 1H), 10.77 (s, 1H), 9.14 (s, 1H), 8.66 (s, 1H), 8.59 (s, 1H), 7.45 (d, *J* = 8.8 Hz, 1H), 7.39 (d, *J* = 8.8 Hz, 1H), 5.26-5.31 (m, 1H), 4.92 (dd, *J* = 66.5, 5.1 Hz, 1H), 3.98 (s, 3H), 3.90 (s, 3H), 2.22 (t, *J* = 6.8 Hz, 1H), 1.62-1.69 (m, 1H), 1.13-1.24 (m, 1H).

### Example 193

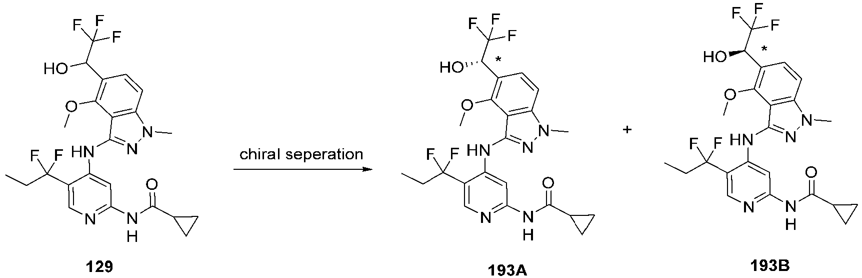

### Step 1. (S*)-N-(5-(1,1-difluoropropyl)-4-((4-methoxy-1-methyl-5-(2,2,2-trifluoro-1-hydroxyethyl)-1H-indazol-3-yl)amino)pyridin-2-yl)cyclopropanecarboxamide (193A) and

### (R*)-N-(5-(1,1-difluoropropyl)-4-((4-methoxy-1-methyl-5-(2,2,2-trifluoro-1-hydroxyethyl)-1H-indazol-3-yl)amino)pyridin-2-yl)cyclopropanecarboxamide (193B)

Compound **129** (90 mg, 0.175 mmol) was separated by chiral *prep*-HPLC (Method I, Hex:IPA = 70:30) to give compound **193A** (30.3 mg, 34% yield) as white solid and **193B** (22.8 mg, 25% yield) as a white solid.
**193A:** LC-MS (Method 4) t_{R} = 2.68 min, m/z (M+H)⁺ = 514.3. ¹H NMR (400 MHz, DMSO-*d*₆) δ 10.69 (s, 1H), 8.55 (s,1H), 8.17 (s, 1H), 8.08 (s, 1H), 7.53 (d, *J* = 8.8 Hz, 1H), 7.46 (d, *J* = 8.8 Hz, 1H), 6.84 (d, *J* = 6.0 Hz, 1H), 5.48-5.33 (m, 1H), 3.97 (s, 3H), 3.80 (s, 3H), 2.47-2.38 (m, 2H), 2.00-1.94 (m, 1H), 1.05 (t, *J* = 7.2 Hz, 3H), 0.81-0.68 (m, 4H). Chiral-HPLC (Method 6, Hex:IPA = 70:30) t_{R} = 5.80 min.
**193B:** LC-MS (Method 4) t_{R} = 2.68 min, m/z (M+H)⁺ = 514.3. ¹H NMR (400 MHz, DMSO-*d*₆) δ 10.68 (s, 1H), 8.56 (s, 1H), 8.17 (s, 1H), 8.08 (s, 1H), 7.53 (d, *J* = 8.8 Hz, 1H), 7.46 (d, *J* = 8.8 Hz, 1H), 6.84 (d, *J* = 6.0 Hz, 1H), 5.50-5.33 (m, 1H), 3.97 (s, 3H), 3.80 (s, 3H), 2.47-2.37 (m, 2H), 2.05-1.89 (m, 1H), 1.05 (t, *J* = 7.2 Hz, 3H), 0.81-0.67 (m, 4H). Chiral-HPLC (Method 6, Hex:IPA = 70:30) t_{R} = 7.80 min.

### Example 194

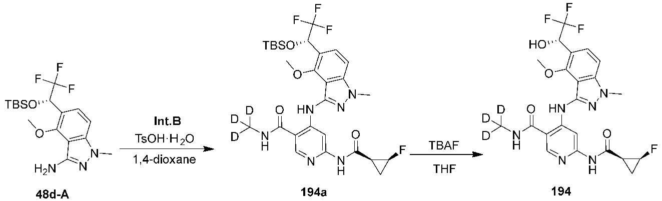

### Step 1. 4-((5-((S)-1-((tert-butyldimethylsilyl)oxy)-2,2,2-trifluoroethyl)-4-methoxy-1-methyl-1H-indazol-3-yl)amino)-6-((1S,2S)-2-fluorocyclopropane-1-carboxamido)-N-(methyl-d3)nicotinamide (194a)

A mixture of **Int**. **B** (50 mg, 0.182 mmol), **48d-A** (92.2 mg, 0.236 mmol) and TsOH·H₂O (3.5. mg, 0.018 mmol) in dioxane (0.5 mL) was stirred at 110 °C for 16 h. After the mixture was cooled, 0.3 mL NH₃·H₂O was added to it. The mixture was extracted with EtOAc (5 mL*2) and washed with brine, then it was dried over Na₂SO₄ and filtered. The filtrate was concentrated to afford crude **194a** (100 mg, 88% yield) as a yellow solid. LC-MS (ESI, Method 2): t_{R} = 1.48 min, m/z (M+H)⁺ = 628.5.

### Step 2. 6-((1S,2S)-2-fluorocyclopropane-1-carboxamido)-4-((4-methoxy-1-methyl-5-((S)-2,2,2-trifluoro-1-hydroxyethyl)-1H-indazol-3-yl)amino)-N-(methyl-d3)nicotinamide (194)

To a solution of **194a** (100 mg, 0.159 mmol) in THF (1 mL) was added TBAF (0.5 mL) at 25 °C. Then the mixture was stirred at 25 °C for 2 h. The reaction mixture was diluted with water (10 mL) and extracted with EtOAc (10 mL*2). The organic layer was concentrated and purified by chromatography column (DCM:MeOH = 10:1) to afford **194** (75 mg, 92% yield) as a white solid. LC-MS (ESI, Method 2): t_{R} = 1.11 min, m/z (M+H)⁺ = 514.1. ¹H NMR (400 MHz, DMSO-*d*₆) δ 11.62 (s, 1H), 10.76 (s, 1H), 9.17 (s, 1H), 8.64 (s, 1H), 8.58 (s, 1H), 7.52 (d, *J* = 8.8 Hz, 1H), 7.40 (d, *J* = 8.8 Hz, 1H), 6.82 (d, *J* = 5.2 Hz, 1H), 5.46-5.41 (m, 1H), 4.92 (dd, *J* = 66.4, 5.3 Hz, 1H), 3.96 (s, 3H), 3.87 (s, 3H), 2.24-2.19 (m, 1H), 1.69-1.61 (m, 1H), 1.16-1.13 (m, 1H).

### Example 195

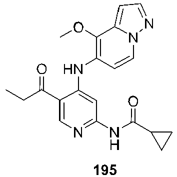

### N-[4-[(4-Methoxypyrazolo[1,5-a]pyridin-5-yl)amino]-5-propanoyl-2-pyridyl]cyclopropanecarboxamide (195)

The synthesis of compound **195** refers to the patent (CN115466257A, page 24-26). LC-MS (ESI, Method 4) t_{R} = 0.72 min, m/z (M+H)⁺ = 380.2. ¹H NMR (400 MHz, CDCl₃): *δ* 11.02 (brs, 1H), 8.71 (s, 1H), 8.42 (brs, 1H), 8.29 (d, *J* = 7.2 Hz, 1H), 7.93 (s, 1H), 7.90 (d, *J* = 2.0 Hz, 1H), 6.87 (d, *J* = 7.2 Hz, 1H), 6.61 (d, *J* = 2.0 Hz, 1H), 4.00 (s, 3H), 3.03 (q, *J* = 7.2 Hz, 2H), 1.58-1.49 (m, 1H), 1.26 (t, *J* = 7.2 Hz, 3H), 1.09-1.03 (m, 2H), 0.92-0.85 (m, 2H).

### Example 196

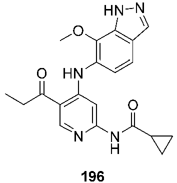

### N-(4-((7-Methoxy-1H-indazol-6-yl)amino)-5-propionylpyridin-2-yl)cyclopropanecarboxamide (196)

The synthesis of compound **196** refers to the patent (CN115466257A, page 36-37). LC-MS (Method 2): t_{R} = 1.10 min, m/z (M+H) ⁺= 380.0. ¹H NMR (400 MHz, DMSO-*d*₆): δ 13.39 (s, 1H), 10.90 (s, 1H), 10.85 (s, 1H), 8.85 (s, 1H), 8.11 (s, 1H), 7.70 (s, 1H), 7.52 (d,*J* = 8.4 Hz, 1H), 7.08 (d, *J* = 8.8 Hz, 1H), 3.89 (s, 3H), 3.12 (q, *J* = 7.2 Hz, 2H), 1.99-1.95 (m, 1H), 1.13 (t, *J* = 7.2 Hz, 3H), 0.76-0.72 (m, 4H).

### Example 197

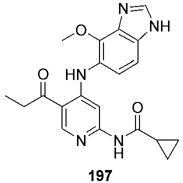

### N-[4-[(4-Methoxy-1H-benzimidazol-5-yl)amino]-5-propanoyl-2-pyridyl] cyclopropanecarboxamide (197)

The synthesis of compound **197** refers to the patent (CN115466257A, page 33-34). LC-MS (ESI, Method 4) t_{R} = 0.52 min, m/z (M+H)⁺ = 380.2. ¹H NMR (400 MHz, CDCl₃): *δ* 10.98 (s, 1H), 9.82 (brs, 1H), 8.61 (s, 1H), 8.61 (s, 1H), 7.94 (s, 1H), 7.70 (s, 1H), 7.23-7.13 (m, 2H), 4.28 (s, 3H), 3.03 (q, *J* = 7.2 Hz, 2H), 1.72-1.62 (m, 1H), 1.27 (t, *J* = 7.2 Hz, 3H), 1.03-0.95 (m, 2H), 0.91-0.82 (m, 2H).

### Example 198

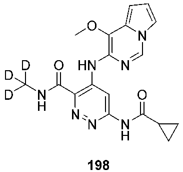

### 6-(Cyclopropanecarboxamido)-4-((4-methoxypyrrolo[1,2-c]pyrimidin-3-yl)amino)-N-(methyl-d3)pyridazine-3-carboxamide (198)

The synthesis of compound **198** refers to the patent (CN116262739A, page 35-36). LC-MS (ESI, Method 4) t_{R} = 0.76 min, m/z (M+H)⁺ = 385.2. ¹H NMR (400 MHz, DMSO-*d*₆): δ 11.50 (s, 1H), 11.24 (s, 1H), 9.14 (s, 1H), 9.01 (s, 1H), 8.99 (d, *J* = 0.8 Hz, 1H), 7.67 (dd, *J =* 2.8, 1.2 Hz, 1H), 6.90 (dd, *J* = 3.6, 2.8 Hz, 1H), 6.56-6.53 (m, 1H), 3.99 (s, 3H), 2.13-2.07 (m, 1H), 0.84 (d, *J* = 6.4 Hz, 4H).

### Example 199

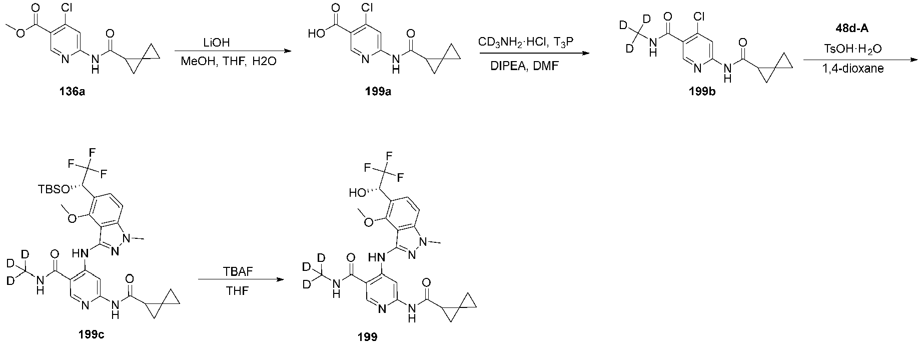

### Step 1. 4-Chloro-6-(spiro[2.2]pentane-1-carboxamido)nicotinic acid (199a)

A mixture of **136a** (45 mg, 0.160 mmol), LiOH·H₂O (33.6 mg, 0.801 mmol) in THF (1 mL), MeOH (1 mL) and H₂O (1 mL) was stirred at 25 °C for 6 h. The mixture was concentrated, and the aqueous layer was adjusted by 1 M HCl to pH = 3. The formed solid was collected by filtration and dried to give **199a** (35 mg, 82% yield) as a white solid. LC-MS (ESI, Method 3): t_{R} = 1.11 min, m/z (M+H)⁺ = 267.2.

### Step 2. 4-Chloro-N-(methyl-d₃)-6-(spiro[2.2]pentane-1-carboxamido)nicotinamide (199b)

A mixture of **199a** (35 mg, 0.131 mol), methyl-*d*₃-amine hydrochloride (27.8 mg, 0.394 mmol), T₃P (334 mg, 0.525 mmol, 50 wt.% in EtOAc), DIPEA (67.9 mg, 0.525 mmol) in DMF (0.5 mL) was stirred for 36 h at 50 °C. After cooling to room temperature, the reaction mixture was purified by *Prep-*HPLC (Method A) to give **199b** (10 mg, 27% yield) as a white solid. LC-MS (ESI, Method 3): t_{R} = 1.05 min, m/z (M+H)⁺ = 282.8.

### Step 3. 4-((5-((S)-1-((tert-butyldimethylsilyl)oxy)-2,2,2-trifluoroethyl)-4-methoxy-1-methyl-1H-indazol-3-yl)amino)-N-(methyl-d₃)-6-(spiro[2.2]pentane-1-carboxamido)nicotinamide (199c)

A mixture of **199b** (10 mg, 0.035 mmol), **48d-A** (20.7 mg, 0.053 mmol) and TsOH·H₂O (3.4 mg, 0.018 mmol) in dioxane (0.5 mL) was stirred at 110 °C for 20 h. After cooling to room temperature, the mixture was basified with NH₃·H₂O (0.3 mL) and concentrated to afford **199c** (22.4 mg, 100% yield) as yellow oil which was used for the next step without purification. LC-MS (ESI, Method 3): t_{R} = 1.55 min, m/z (M+H)⁺ = 636.5.

### Step 4. 4-((4-Methoxy-1-methyl-5-((S)-2,2,2-trifluoro-1-hydroxyethyl)-1H-indazol-3-yl)amino)-N-(methyl-d₃)-6-(spiro[2.2]pentane-1-carboxamido)nicotinamide (199)

To a solution of **199c** (20 mg, 0.031 mmol) in THF (0.5 mL) was added TBAF (0.2 mL, 1M in THF) at 25 °C. Then the mixture was stirred at 25 °C for 2 h. The reaction mixture was diluted with water (10 mL) and extracted with EtOAc (10 mL*2). The organic layer was concentrated and purified by chromatography column (DCM:MeOH = 10:1) to afford crude product. The crude product was purified by *Prep-*HPLC (Method A) to afford **199** (1.0 mg, 6% yield) as a white solid. LC-MS (ESI, Method 2): t_{R} = 1.15 min, m/z (M+H)⁺ = 522.1. ¹H NMR (400 MHz, CD₃OD) δ 9.19 (s, 1H), 8.42 (s, 1H), 7.60 (d, *J* = 8.8 Hz, 1H), 7.28 (d, *J* = 8.8 Hz, 1H), 5.54-5.49 (m, 1H), 4.03 (s, 3H), 3.94 (s, 3H), 2.26-2.17 (m, 1H), 1.42-1.28 (m, 2H), 0.97-0.91 (m, 4H).

A Summary of representative compounds are shown in **Table 1.**

**Table 1. Exemplary Compounds**

| **Example** | **Structure** | **Name** | **Mol Weight** |
|---|---|---|---|
| **1** | 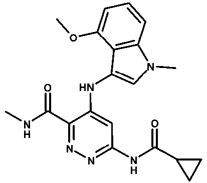 | 6-(Cyclopropanecarboxamido)-4-((4-methoxy-1-methyl-1H-indol-3-yl)amino)-N-methylpyridazine-3-carboxamide | 394.43 |
| **2** | 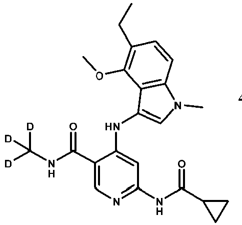 | 6-(Cyclopropanecarboxamido)-4-((5-ethyl-4-methoxy-1-methyl-1H-indol-3-yl)amino)-N-(methyl-d3)nicotinamide | 424.51 |
| **3** | 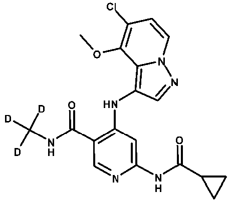 | 4-((5-Chloro-4-methoxypyrazolo[1,5-a]pyridin-3-yl)amino)-6-(cyclopropanecarboxamido)-N-(methyl-d3)nicotinamide | 417.86 |
| **4** | 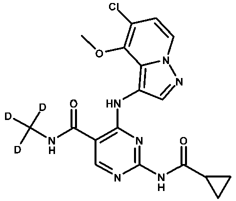 | 4-((5-Chloro-4-methoxypyrazolo[1,5-a]pyridin-3-yl)amino)-2-(cyclopropanecarboxamido)-N-(methyl-d3)pyrimidine-5-carboxamide | 418.85 |
| **5** | 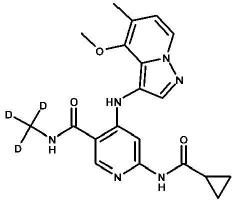 | 6-(Cyclopropanecarboxamido)-4-((4-methoxy-5-methylpyrazolo[1,5-a]pyridin-3-yl)amino)-N-(methyl-d3)nicotinamide | 397.44 |
| **6** | 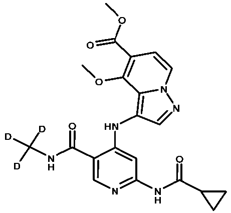 | Methyl 3-((2-(cyclopropanecarboxamido)-5-((methyl-d3)carbamoyl)pyridin-4-yl)amino)-4-methoxypyrazolo[1,5-a]pyridine-5-carboxylate | 441.45 |
| **7** | 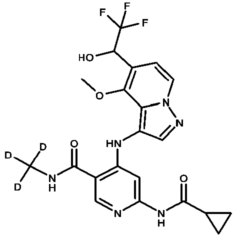 | 6-(Cyclopropanecarboxamido)-4-((4-methoxy-5-(2,2,2-trifluoro-1-hydroxyethyl)pyrazolo[1,5-a]pyridin-3-yl)amino)-N-(methyl-d3)nicotinamide | 481.44 |
| **7A** | 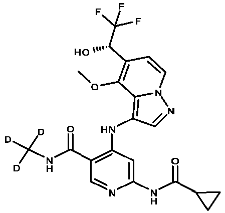 | (S)-6-(cyclopropanecarboxamido)-4-((4-methoxy-5-(2,2,2-trifluoro-1-hydroxyethyl)pyrazolo[1,5-a]pyridin-3-yl)amino)-N-(methyl-d3)nicotinamide | 481.44 |
| **7B** | 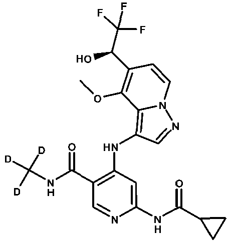 | (R)-6-(cyclopropanecarboxamido)-4-((4-methoxy-5-(2,2,2-trifluoro-1-hydroxyethyl)pyrazolo[1,5-a]pyridin-3-yl)amino)-N-(methyl-d3)nicotinamide | 481.44 |
| **8** | 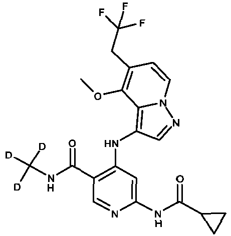 | 6-(Cyclopropanecarboxamido)-4-((4-methoxy-5-(2,2,2-trifluoroethyl)pyrazolo[1,5-a]pyridin-3-yl)amino)-N-(methyl-d3)nicotinamide | 465.44 |
| **9** | 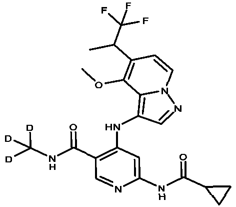 | 6-(Cyclopropanecarboxamido)-4-((4-methoxy-5-(1,1,1-trifluoropropan-2-yl)pyrazolo[1,5-a]pyridin-3-yl)amino)-N-(methyl-d3)nicotinamide | 479.47 |
| **9A** | 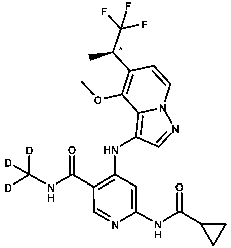 | (R*) -6-(cyclopropanecarboxamido)-4-((4-methoxy-5-(1,1,1-trifluoropropan-2-yl)pyrazolo[1,5-a]pyridin-3-yl)amino)-N-(methyl-d3)nicotinamide | 479.47 |
| **9B** | 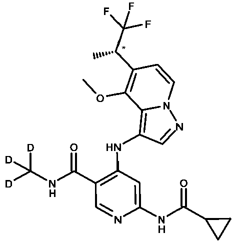 | (S*)-6-(cyclopropanecarboxamido)-4-((4-methoxy-5-(1,1,1-trifluoropropan-2-yl)pyrazolo[1,5-a]pyridin-3-yl)amino)-N-(methyl-d3)nicotinamide | 479.47 |
| **10** | 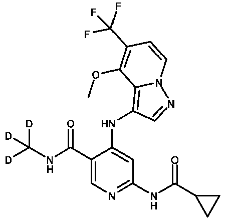 | 6-(Cyclopropanecarboxamido)-4-((4-methoxy-5-(trifluoromethyl)pyrazolo[1,5-a]pyridin-3-yl)amino)-N-(methyl-d3)nicotinamide | 451.42 |
| **11** | 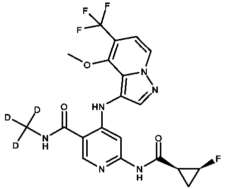 | 6-((1S,2S)-2-fluorocyclopropane-1-carboxamido)-4-((4-methoxy-5-(trifluoromethyl)pyrazolo[1,5-a]pyridin-3-yl)amino)-N-(methyl-d3)nicotinamide | 469.41 |
| **12** | 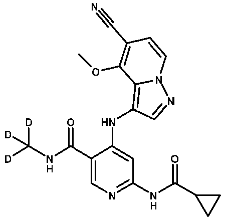 | 4-((5-Cyano-4-methoxypyrazolo[1,5-a]pyridin-3-yl)amino)-6-(cyclopropanecarboxamido)-N-(methyl-d3)nicotinamide | 408.43 |
| **13** | 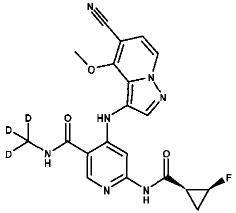 | 4-((5-Cyano-4-methoxypyrazolo[1,5-a]pyridin-3-yl)amino)-6-((1S,2S)-2-fluorocyclopropane-1-carboxamido)-N-(methyl-d3)nicotinamide | 426.42 |
| **14A** | 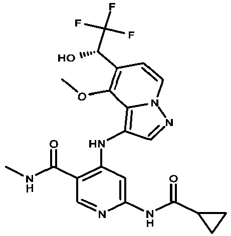 | (S)-6-(Cyclopropanecarboxamido)-4-((4-methoxy-3-(2,2,2-trifluoro-1-hydroxyethyl)pyrazolo[1,5-a]pyridin-5-yl)amino)-N-methylnicotinamide | 478.42 |
| **14B** | 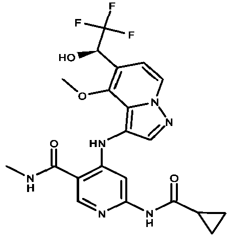 | (R)-6-(Cyclopropanecarboxamido)-4-((4-methoxy-3-(2,2,2-trifluoro-1-hydroxyethyl)pyrazolo[1,5-a]pyridin-5-yl)amino)-N-methylnicotinamide | 478.42 |
| **15A** | 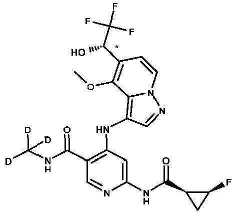 | 6-((1S,2S)-2-fluorocyclopropane-1-carboxamido)-4-((4-methoxy-5-((S*)-2,2,2-trifluoro-1-hydroxyethyl)pyrazolo[1,5-a]pyridin-3-yl)amino)-N-(methyl-d3)nicotinamide | 499.43 |
| **15B** | 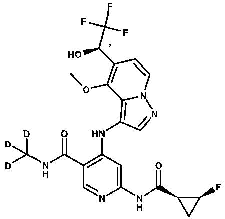 | 6-((1S,2S)-2-fluorocyclopropane-1-carboxamido)-4-((4-methoxy-5-((R*)-2,2,2-trifluoro-1-hydroxyethyl)pyrazolo[1,5-a]pyridin-3-yl)amino)-N-(methyl-d3)nicotinamide | 499.43 |
| **16** | 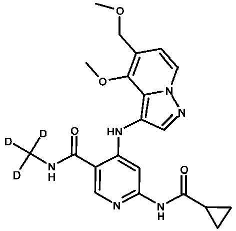 | 6-(Cyclopropanecarboxamido)-4-((4-methoxy-5-(methoxymethyl)pyrazolo[1,5-a]pyridin-3-yl)amino)-N-(methyl-d3)nicotinamide | 427.47 |
| **17** | 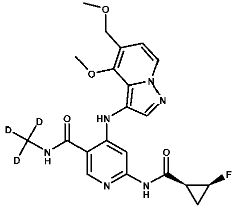 | 6-((1S,2S)-2-fluorocyclopropane-1-carboxamido)-4-((4-methoxy-5-(methoxymethyl)pyrazolo[1,5-a]pyridin-3-yl)amino)-N-(methyl-d3)nicotinamide | 445.46 |
| **18** | 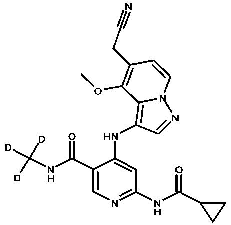 | 4-((5-(Cyanomethyl)-4-methoxypyrazolo[1,5-a]pyridin-3-yl)amino)-6-(cyclopropanecarboxamido)-N-(methyl-d3)nicotinamide | 422.45 |
| **19** | 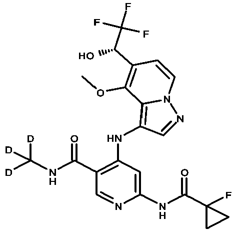 | (S)-6-(1-fluorocyclopropane-1-carboxamido)-4-((4-methoxy-5-(2,2,2-trifluoro-1-hydroxyethyl)pyrazolo[1,5-a]pyridin-3-yl)amino)-N-(methyl-d3)nicotinamide | 499.43 |
| **20** | 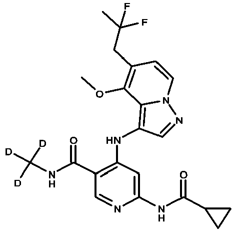 | 6-(Cyclopropanecarboxamido)-4-((5-(2,2-difluoropropyl)-4-methoxypyrazolo[1,5-a]pyridin-3-yl)amino)-N-(methyl-d3)nicotinamide | 461.48 |
| **21** | 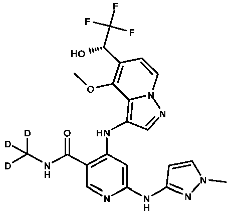 | (S)-4-((4-methoxy-5-(2,2,2-trifluoro-1-hydroxyethyl)pyrazolo[1,5-a]pyridin-3-yl)amino)-N-(methyl-d3)-6-((1-methyl-1H-pyrazol-3-yl)amino)nicotinamide | 493.46 |
| **22** | 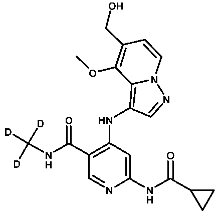 | 6-(Cyclopropanecarboxamido)-4-((5-(hydroxymethyl)-4-methoxypyrazolo[1,5-a]pyridin-3-yl)amino)-N-(methyl-d3)nicotinamide | 413.44 |
| **23** | 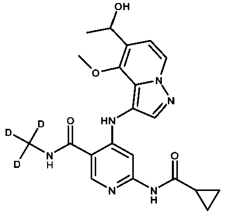 | 6-(Cyclopropanecarboxamido)-4-((5-(1-hydroxyethyl)-4-methoxypyrazolo[1,5-a]pyridin-3-yl)amino)-N-(methyl-d3)nicotinamide | 427.47 |
| **24** | 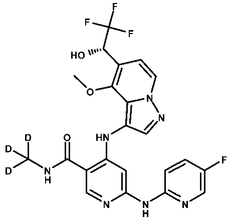 | (S)-6-((5-fluoropyridin-2-yl)amino)-4-((4-methoxy-5-(2,2,2-trifluoro-1-hydroxyethyl)pyrazolo[1,5-a]pyridin-3-yl)amino)-N-(methyl-d3)nicotinamide | 508.44 |
| **25** | 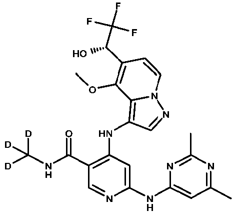 | (S)-6-((2,6-Dimethylpyrimidin-4-yl)amino)-4-((4-methoxy-5-(2,2,2-trifluoro-1-hydroxyethyl)pyrazolo[1,5-a]pyridin-3-yl)amino)-N-(methyl-d3)nicotinamide | 519.49 |
| **26** | 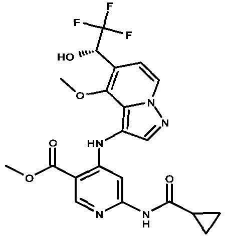 | Methyl (S)-6-(cyclopropanecarboxamido)-4-((4-methoxy-5-(2,2,2-trifluoro-1-hydroxyethyl)pyrazolo[1,5-a]pyridin-3-yl)amino)nicotinate | 479.41 |
| **27** | 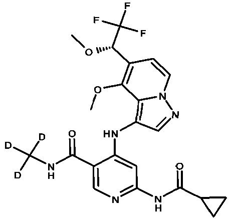 | (S)-6-(cyclopropanecarboxamido)-4-((4-methoxy-5-(2,2,2-trifluoro-1-methoxyethyl)pyrazolo[1,5-a]pyridin-3-yl)amino)-N-(methyl-d3)nicotinamide | 495.47 |
| **28** | 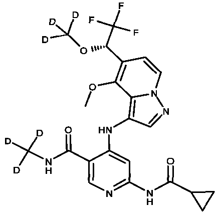 | (S)-6-(cyclopropanecarboxamido)-4-((4-methoxy-5-(2,2,2-trifluoro-1-(methoxy-d3)ethyl)pyrazolo[1,5-a]pyridin-3-yl)amino)-N-(methyl-d3)nicotinamide | 498.49 |
| **29** | 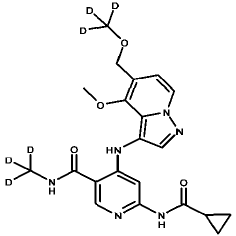 | 6-(Cyclopropanecarboxamido)-4-((4-methoxy-5-((methoxy-d3)methyl)pyrazolo[1,5-a]pyridin-3-yl)amino)-N-(methyl-d3)nicotinamide | 430.49 |
| **30** | 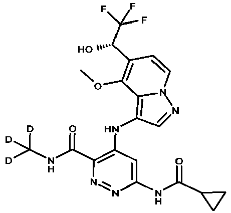 | (S)-6-(cyclopropanecarboxamido)-4-((4-methoxy-5-(2,2,2-trifluoro-1-hydroxyethyl)pyrazolo[1,5-a]pyridin-3-yl)amino)-N-(methyl-d3)pyridazine-3-carboxamide | 482.43 |
| **31** | 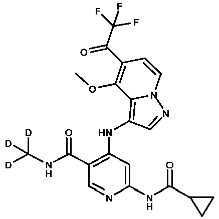 | 6-(Cyclopropanecarboxamido)-4-((4-methoxy-5-(2,2,2-trifluoroacetyl)pyrazolo[1,5-a]pyridin-3-yl)amino)-N-(methyl-d3)nicotinamide | 479.43 |
| **32** | 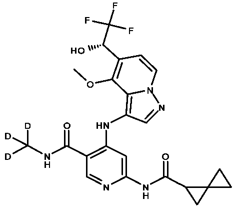 | 4-((4-Methoxy-5-((S)-2,2,2-trifluoro-1-hydroxyethyl)pyrazolo[1,5-a]pyridin-3-yl)amino)-N-(methyl-d3)-6-(spiro[2.2]pentane-1-carboxamido)nicotinamide | 507.48 |
| **32A** | 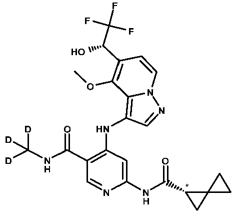 | 4-((4-Methoxy-5-((S)-2,2,2-trifluoro-1-hydroxyethyl)pyrazolo[1,5-a]pyridin-3-yl)amino)-N-(methyl-d3)-6-((S*)-spiro[2.2]pentane-1-carboxamido)nicotinamide | 507.48 |
| **32B** | 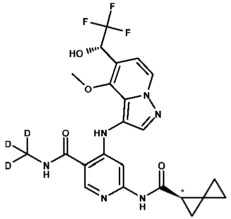 | 4-((4-Methoxy-5-((S)-2,2,2-trifluoro-1-hydroxyethyl)pyrazolo[1,5-a]pyridin-3-yl)amino)-N-(methyl-d3)-6-((R*)-spiro[2.2]pentane-1-carboxamido)nicotinamide | 507.48 |
| **33** | 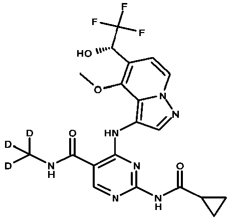 | (S)-2-(cyclopropanecarboxamido)-4-((4-methoxy-5-(2,2,2-trifluoro-1-hydroxyethyl)pyrazolo[1,5-a]pyridin-3-yl)amino)-N-(methyl-d3)pyrimidine-5-carboxamide | 482.43 |
| **34** | 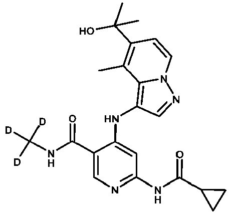 | 6-(Cyclopropanecarboxamido)-4-((5-(2-hydroxypropan-2-yl)-4-methylpyrazolo[1,5-a]pyridin-3-yl)amino)-N-(methyl-d3)nicotinamide | 425.50 |
| **35** | 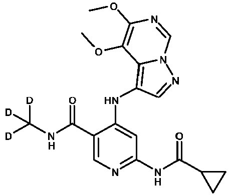 | 6-(Cyclopropanecarboxamido)-4-((4,5-dimethoxypyrazolo[1,5-c]pyrimidin-3-yl)amino)-N-(methyl-d3)nicotinamide | 414.43 |
| **36** | 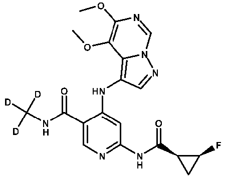 | 4-((4,5-Dimethoxypyrazolo[1,5-c]pyrimidin-3-yl)amino)-6-((1S,2S)-2-fluorocyclopropane-1-carboxamido)-N-(methyl-d3)nicotinamide | 432.42 |
| **37** | 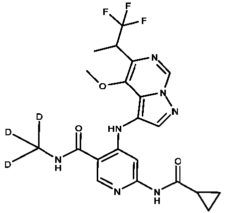 | 6-(Cyclopropanecarboxamido)-4-((4-methoxy-5-(1,1,1-trifluoropropan-2-yl)pyrazolo[1,5-c]pyrimidin-3-yl)amino)-N-(methyl-d3)nicotinamide | 480.46 |
| **38** | 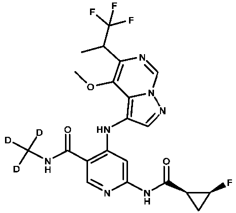 | 6-((1S,2S)-2-fluorocyclopropane-1-carboxamido)-4-((4-methoxy-5-(1,1,1-trifluoropropan-2-yl)pyrazolo[1,5-c]pyrimidin-3-yl)amino)-N-(methyl-d3)nicotinamide | 498.45 |
| **39** | 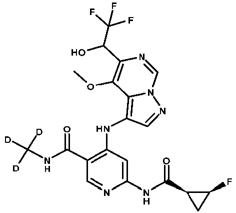 | 6-((1S,2S)-2-fluorocyclopropane-1-carboxamido)-4-((4-methoxy-5-(2,2,2-trifluoro-1-hydroxyethyl)pyrazolo[1,5-c]pyrimidin-3-yl)amino)-N-(methyl-d3)nicotinamide | 500.42 |
| **40** | 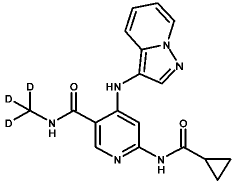 | 6-(Cyclopropanecarboxamido)-N-(methyl-d3)-4-(pyrazolo[1,5-a]pyridin-3-ylamino)nicotinamide | 353.39 |
| **41** | 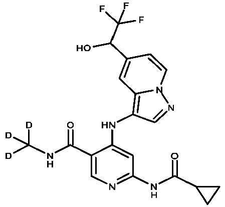 | 6-(Cyclopropanecarboxamido)-N-(methyl-d3)-4-((5-(2,2,2-trifluoro-1-hydroxyethyl)pyrazolo[1,5-a]pyridin-3-yl)amino)nicotinamide | 451.42 |
| **42** | 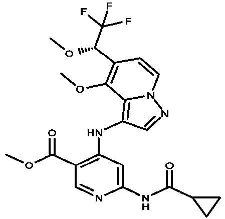 | Methyl (S)-6-(cyclopropanecarboxamido)-4-((4-methoxy-5-(2,2,2-trifluoro-1-methoxyethyl)pyrazolo[1,5-a]pyridin-3-yl)amino)nicotinate | 493.43 |
| **43** | 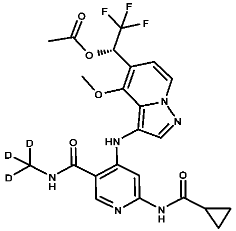 | (S)-1-(3-((2-(cyclopropanecarboxamido)-5-((methyl-d3)carbamoyl)pyridin-4-yl)amino)-4-methoxypyrazolo[1,5-a]pyridin-5-yl)-2,2,2-trifluoroethyl acetate | 523.48 |
| **44** | 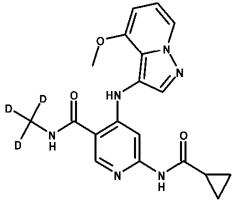 | 6-(cyclopropanecarboxamido)-4-((4-methoxypyrazolo[1,5-a]pyridin-3-yl)amino)-N-(methyl-d3)nicotinamide | 383.42 |
| **45** | 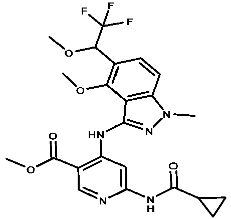 | Methyl 6-(cyclopropanecarboxamido)-4-((4-methoxy-1-methyl-5-(2,2,2-trifluoro-1-methoxyethyl)-1H-indazol-3-yl)amino)nicotinate | 507.46 |
| **45A** | 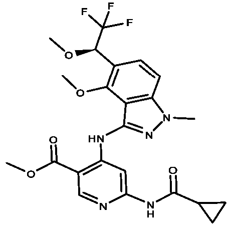 | Methyl (R)-6-(cyclopropanecarboxamido)-4-((4-methoxy-1-methyl-5-(2,2,2-trifluoro-1-methoxyethyl)-1H-indazol-3-yl)amino)nicotinate | 507.46 |
| **45B** | 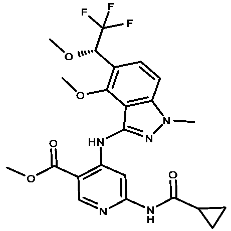 | Methyl (S)-6-(cyclopropanecarboxamido)-4-((4-methoxy-1-methyl-5-(2,2,2-trifluoro-1-methoxyethyl)-1H-indazol-3-yl)amino)nicotinate | 507.46 |
| **46** | 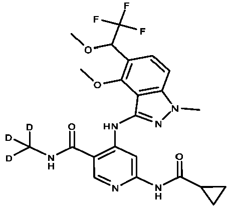 | 6-(Cyclopropanecarboxamido)-4-((4-methoxy-1-methyl-5-(2,2,2-trifluoro-1-methoxyethyl)-1H-indazol-3-yl)amino)-N-(methyl-d3)nicotinamide | 509.50 |
| **46A** | 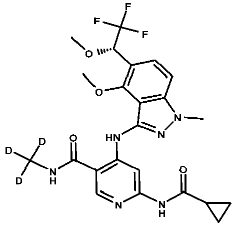 | (S)-6-(cyclopropanecarboxamido)-4-((4-methoxy-1-methyl-5-(2,2,2-trifluoro-1-methoxyethyl)-1H-indazol-3-yl)amino)-N-(methyl-d3)nicotinamide | 509.50 |
| **46B** | 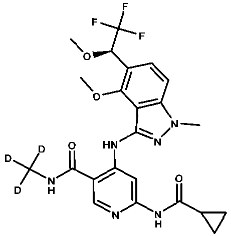 | (R)-6-(cyclopropanecarboxamido)-4-((4-methoxy-1-methyl-5-(2,2,2-trifluoro-1-methoxyethyl)-1H-indazol-3-yl)amino)-N-(methyl-d3)nicotinamide | 509.50 |
| **47** | 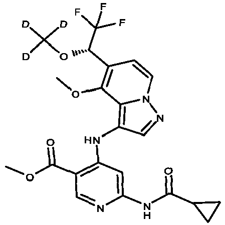 | Methyl (S)-6-(cyclopropanecarboxamido)-4-((4-methoxy-5-(2,2,2-trifluoro-1-(methoxy-d3)ethyl)pyrazolo[1,5-a]pyridin-3-yl)amino)nicotinate | 496.45 |
| **48** | 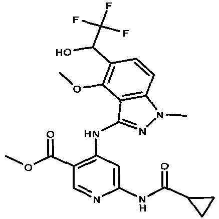 | Methyl 6-(cyclopropanecarboxamido)-4-((4-methoxy-1-methyl-5-(2,2,2-trifluoro-1-hydroxyethyl)-1H-indazol-3-yl)amino)nicotinate | 493.43 |
| **48A** | 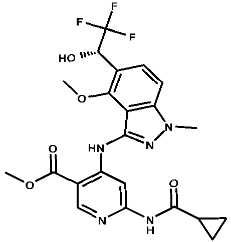 | Methyl (S)-6-(cyclopropanecarboxamido)-4-((4-methoxy-1-methyl-5-(2,2,2-trifluoro-1-hydroxyethyl)-1H-indazol-3-yl)amino)nicotinate | 493.43 |
| **48B** | 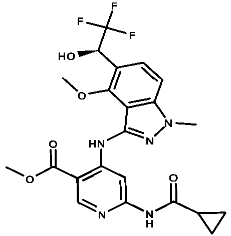 | Methyl (R)-6-(cyclopropanecarboxamido)-4-((4-methoxy-1-methyl-5-(2,2,2-trifluoro-1-hydroxyethyl)-1H-indazol-3-yl)amino)nicotinate | 493.43 |
| **49** | 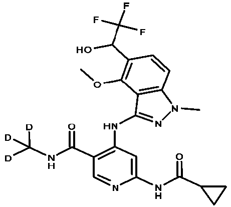 | 6-(Cyclopropanecarboxamido)-4-((4-methoxy-1-methyl-5-(2,2,2-trifluoro-1-hydroxyethyl)-1H-indazol-3-yl)amino)-N-(methyl-d3)nicotinamide | 495.47 |
| **49A** | 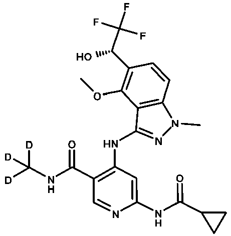 | (S)-6-(cyclopropanecarboxamido)-4-((4-methoxy-1-methyl-5-(2,2,2-trifluoro-1-hydroxyethyl)-1H-indazol-3-yl)amino)-N-(methyl-d3)nicotinamide | 495.47 |
| **49B** | 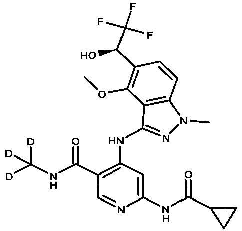 | (R)-6-(cyclopropanecarboxamido)-4-((4-methoxy-1-methyl-5-(2,2,2-trifluoro-1-hydroxyethyl)-1H-indazol-3-yl)amino)-N-(methyl-d3)nicotinamide | 495.47 |
| **50A** | 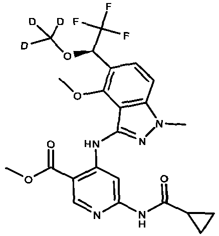 | Methyl (R)-6-(cyclopropanecarboxamido)-4-((4-methoxy-1-methyl-5-(2,2,2-trifluoro-1-(methoxy-d3)ethyl)-1H-indazol-3-yl)amino)nicotinate | 510.48 |
| **50B** | 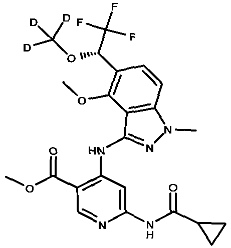 | Methyl (S)-6-(cyclopropanecarboxamido)-4-((4-methoxy-1-methyl-5-(2,2,2-trifluoro-1-(methoxy-d3)ethyl)-1H-indazol-3-yl)amino)nicotinate | 510.48 |
| **51A** | 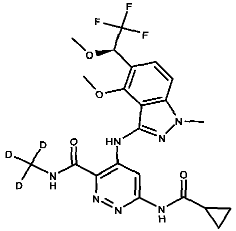 | (R)-6-(cyclopropanecarboxamido)-4-((4-methoxy-1-methyl-5-(2,2,2-trifluoro-1-methoxyethyl)-1H-indazol-3-yl)amino)-N-(methyl-d3)pyridazine-3-carboxamide | 510.48 |
| **51B** | 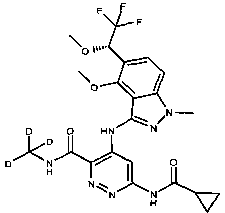 | (S)-6-(cyclopropanecarboxamido)-4-((4-methoxy-1-methyl-5-(2,2,2-trifluoro-1-methoxyethyl)-1H-indazol-3-yl)amino)-N-(methyl-d3)pyridazine-3-carboxamide | 510.48 |
| **52A** | 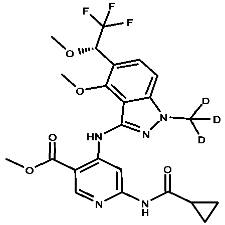 | Methyl (S)-6-(cyclopropanecarboxamido)-4-((4-methoxy-1-(methyl-d3)-5-(2,2,2-trifluoro-1-methoxyethyl)-1H-indazol-3-yl)amino)nicotinate | 510.48 |
| **52B** | 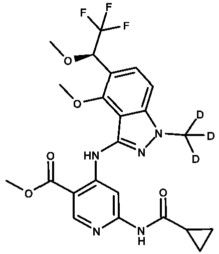 | Methyl (R)-6-(cyclopropanecarboxamido)-4-((4-methoxy-1-(methyl-d3)-5-(2,2,2-trifluoro-1-methoxyethyl)-1H-indazol-3-yl)amino)nicotinate | 510.48 |
| **53A** | 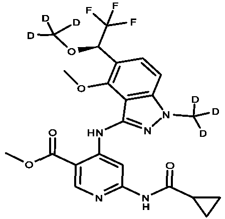 | Methyl (R)-6-(cyclopropanecarboxamido)-4-((4-methoxy-1-(methyl-d3)-5-(2,2,2-trifluoro-1-(methoxy-d3)ethyl)-1H-indazol-3-yl)amino)nicotinate | 513.50 |
| **53B** | 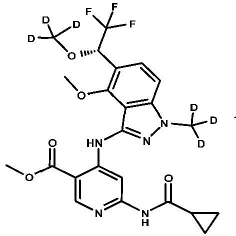 | Methyl (S)-6-(cyclopropanecarboxamido)-4-((4-methoxy-1-(methyl-d3)-5-(2,2,2-trifluoro-1-(methoxy-d3)ethyl)-1H-indazol-3-yl)amino)nicotinate | 513.50 |
| **54A** | 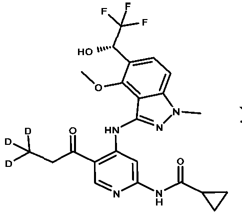 | (S)-N-(4-((4-methoxy-1-methyl-5-(2,2,2-trifluoro-1-hydroxyethyl)-1H-indazol-3-yl)amino)-5-(propanoyl-3,3,3-d3)pyridin-2-yl)cyclopropanecarboxamide | 494.48 |
| **54B** | 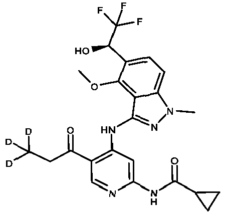 | (R)-N-(4-((4-methoxy-1-methyl-5-(2,2,2-trifluoro-1-hydroxyethyl)-1H-indazol-3-yl)amino)-5-(propanoyl-3,3,3-d3)pyridin-2-yl)cyclopropanecarboxamide | 494.48 |
| **55A** | 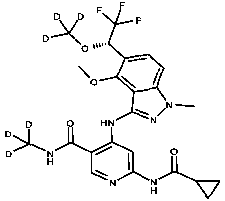 | (S)-6-(cyclopropanecarboxamido)-4-((4-methoxy-1-methyl-5-(2,2,2-trifluoro-1-(methoxy-d3)ethyl)-1H-indazol-3-yl)amino)-N-(methyl-d3)nicotinamide | 512.51 |
| **55B** | 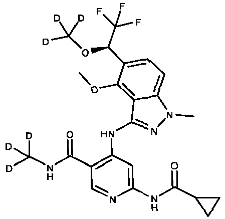 | (R)-6-(cyclopropanecarboxamido)-4-((4-methoxy-1-methyl-5-(2,2,2-trifluoro-1-(methoxy-d3)ethyl)-1H-indazol-3-yl)amino)-N-(methyl-d3)nicotinamide | 512.51 |
| **56** | 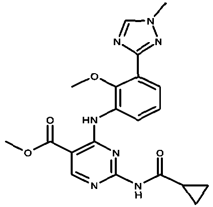 | Methyl 2-(cyclopropanecarboxamido)-4-((2-methoxy-3-(1-methyl-1H-1,2,4-triazol-3-yl)phenyl)amino)pyrimidine-5-carboxylate | 423.42 |
| **57** | 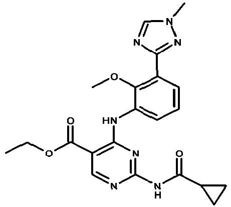 | Ethyl 2-(cyclopropanecarboxamido)-4-((2-methoxy-3-(1-methyl-1H-1,2,4-triazol-3-yl)phenyl)amino)pyrimidine-5-carboxylate | 437.45 |
| **58** | 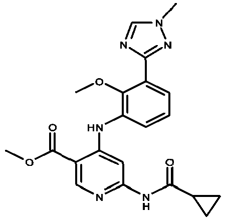 | Methyl 6-(cyclopropanecarboxamido)-4-((2-methoxy-3-(1-methyl-1H-1,2,4-triazol-3-yl)phenyl)amino)nicotinate | 422.44 |
| **59** | 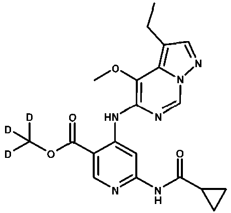 | Methyl-d3 6-(cyclopropanecarboxamido)-4-((3-ethyl-4-methoxypyrazolo[1,5-c]pyrimidin-5-yl)amino)nicotinate | 413.44 |
| **60** | 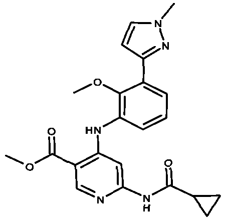 | Methyl 6-(cyclopropanecarboxamido)-4-((2-methoxy-3-(1-methyl-1*H*-pyrazol-3-yl)phenyl)amino)nicotinate | 421.45 |
| **61** | 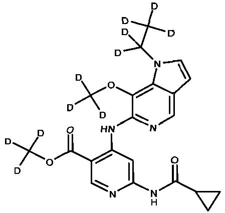 | Methyl-d3 6-(cyclopropanecarboxamido)-4-((1-(ethyl-d5)-7-(methoxy-d3)-1H-pyrrolo[3,2-c] pyridin-6-yl) amino) nicotinate | 420.51 |
| **62** | 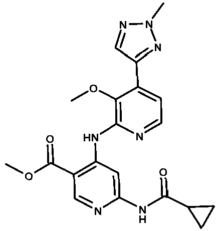 | Methyl 6-(cyclopropanecarboxamido)-4-((3-methoxy-4-(2-methyl-2*H*-1,2,3-triazol-4-yl)pyridin-2-yl)amino)nicotinate | 423.42 |
| **63** | 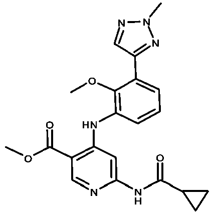 | Methyl 6-(cyclopropanecarboxamido)-4-((2-methoxy-3-(2-methyl-2H-1,2,3-triazol-4-yl)phenyl)amino)nicotinate | 422.44 |
| **64** | 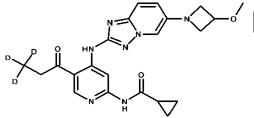 | *N-*[4-[[6-(3-methoxyazetidin-1-yl)-[1,2,4]triazolo[1,5-*a*]pyridin-2-yl]amino]-5-(3,3,3-trideuteriopropanoyl)-2-pyridyl]cyclopropanecarboxamide | 438.50 |
| **65A** | 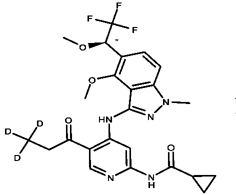 | (R*)-N-(4-((4-methoxy-1-methyl-5-(2,2,2-trifluoro-1-methoxyethyl)-1H-indazol-3-yl)amino)-5-(propanoyl-3,3,3-d3)pyridin-2-yl)cyclopropanecarboxamide | 508.51 |
| **65B** | 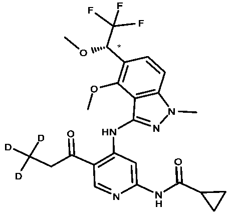 | (S*)-N-(4-((4-methoxy-1-methyl-5-(2,2,2-trifluoro-1-methoxyethyl)-1H-indazol-3-yl)amino)-5-(propanoyl-3,3,3-d3)pyridin-2-yl)cyclopropanecarboxamide | 508.51 |
| **66A** | 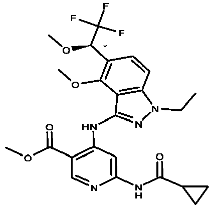 | Methyl (R*)-6-(cyclopropanecarboxamido)-4-((1-ethyl-4-methoxy-5-(2,2,2-trifluoro-1-methoxyethyl)-1H-indazol-3-yl)amino)nicotinate | 521.49 |
| **66B** | 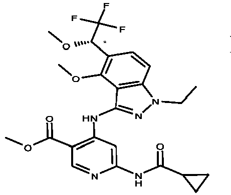 | Methyl (S*)-6-(cyclopropanecarboxamido)-4-((1-ethyl-4-methoxy-5-(2,2,2-trifluoro-1-methoxyethyl)-1H-indazol-3-yl)amino)nicotinate | 521.49 |
| **67** | 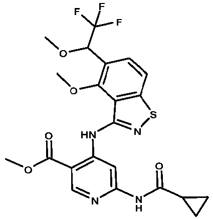 | Methyl 6-(cyclopropanecarboxamido)-4-((4-methoxy-5-(2,2,2-trifluoro-1-methoxyethyl)benzo[d]isothiazol-3-yl)amino)nicotinate | 510.49 |
| **67A** | 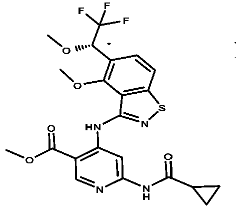 | Methyl (S*)-6-(cyclopropanecarboxamido)-4-((4-methoxy-5-(2,2,2-trifluoro-1-methoxyethyl)benzo[d]isothiazol-3-yl)amino)nicotinate | 510.49 |
| **67B** | 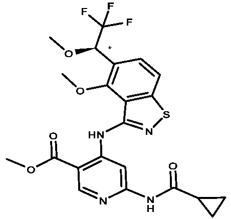 | Methyl (R*)-6-(cyclopropanecarboxamido)-4-((4-methoxy-5-(2,2,2-trifluoro-1-methoxyethyl)benzo[d]isothiazol-3-yl)amino)nicotinate | 510.49 |
| **68** | 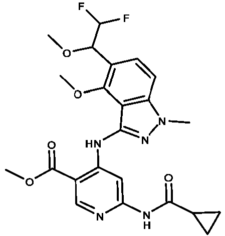 | Methyl 6-(cyclopropanecarboxamido)-4-((5-(2,2-difluoro-1-methoxyethyl)-4-methoxy-1-methyl-1H-indazol-3-yl)amino)nicotinate | 489.47 |
| **69** | 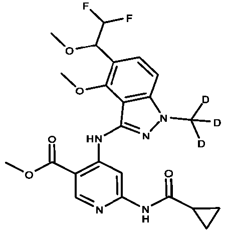 | Methyl 6-(cyclopropanecarboxamido)-4-((5-(2,2-difluoro-1-methoxyethyl)-4-methoxy-1-(methyl-d3)-1H-indazol-3-yl)amino)nicotinate | 492.49 |
| **70A** | 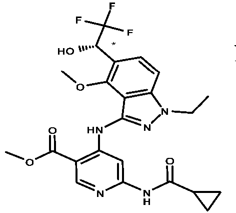 | Methyl (S*)-6-(cyclopropanecarboxamido)-4-((1-ethyl-4-methoxy-5-(2,2,2-trifluoro-1-hydroxyethyl)-1H-indazol-3-yl)amino)nicotinate | 507.46 |
| **70B** | 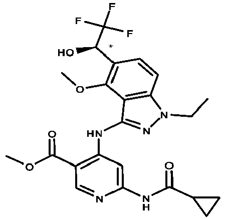 | Methyl (R*)-6-(cyclopropanecarboxamido)-4-((1-ethyl-4-methoxy-5-(2,2,2-trifluoro-1-hydroxyethyl)-1H-indazol-3-yl)amino)nicotinate | 507.46 |
| **71A** | 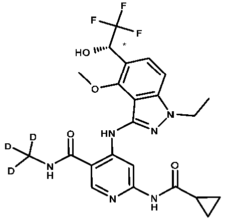 | (S*)-6-(cyclopropanecarboxamido)-4-((1-ethyl-4-methoxy-5-(2,2,2-trifluoro-1-hydroxyethyl)-1H-indazol-3-yl)amino)-N-(methyl-d3)nicotinamide | 509.5 |
| **71B** | 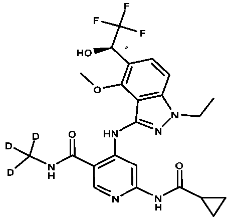 | (R*)-6-(cyclopropanecarboxamido)-4-((1-ethyl-4-methoxy-5-(2,2,2-trifluoro-1-hydroxyethyl)-1H-indazol-3-yl)amino)-N-(methyl-d3)nicotinamide | 509.5 |
| **72** | 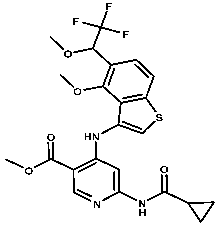 | 6-(Cyclopropanecarboxamido)-4-((4-methoxy-5-(2,2,2-trifluoro-1-methoxyethyl)benzo[b]thiophen-3-yl)amino)-N-(methyl-d3)nicotinamide | 509.5 |
| **73** | 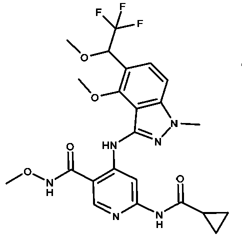 | 6-(Cyclopropanecarboxamido)-N-methoxy-4-((4-methoxy-1-methyl-5-(2,2,2-trifluoro-1-methoxyethyl)-1H-indazol-3-yl)amino)nicotinamide | 522.48 |
| **74** | 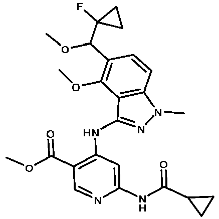 | Methyl 6-(cyclopropanecarboxamido)-4-((5-((1-fluorocyclopropyl) (methoxy)methyl)-4-methoxy-1-methyl-1H-indazol-3-yl)amino)nicotinate | 497.52 |
| **75** | 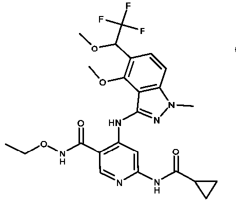 | 6-(Cyclopropanecarboxamido)-N-ethoxy-4-((4-methoxy-1-methyl-5-(2,2,2-trifluoro-1-methoxyethyl)-1H-indazol-3-yl)amino)nicotinamide | 536.5 |
| **76** | 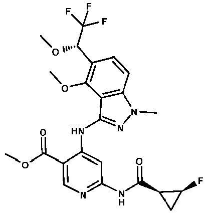 | Methyl 6-((1S,2S)-2-fluorocyclopropane-1-carboxamido)-4-((4-methoxy-1-methyl-5-((S)-2,2,2-trifluoro-1-methoxyethyl)-1H-indazol-3-yl)amino)nicotinate | 525.45 |
| **77** | 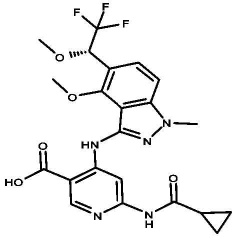 | (S)-6-(cyclopropanecarboxamido)-4-((4-methoxy-1-methyl-5-(2,2,2-trifluoro-1-methoxyethyl)-1H-indazol-3-yl)amino)nicotinic acid | 493.43 |
| **78** | 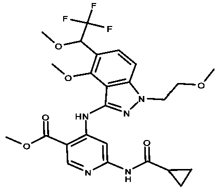 | Methyl 6-(cyclopropanecarboxamido)-4-((4-methoxy-1-(2-methoxyethyl)-5-(2,2,2-trifluoro-1-methoxyethyl)-1H-indazol-3-yl)amino)nicotinate | 551.51 |
| **79** | 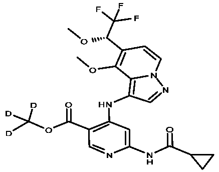 | Methyl-d3 (S)-6-(cyclopropanecarboxamido)-4-((4-methoxy-5-(2,2,2-trifluoro-1-methoxyethyl)pyrazolo[1,5-a]pyridin-3-yl)amino)nicotinate | 496.45 |
| **80** | 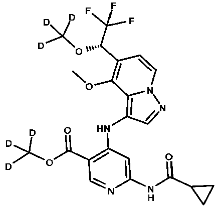 | Methyl-d3 (S)-6-(cyclopropanecarboxamido)-4-((4-methoxy-5-(2,2,2-trifluoro-1-(methoxy-d3)ethyl)pyrazolo[1,5-a]pyridin-3-yl)amino)nicotinate | 499.47 |
| **81** | 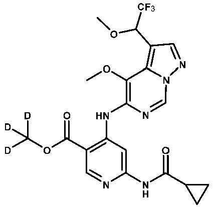 | Methyl-d3 6-(cyclopropanecarboxamido)-4-((4-methoxy-3-(2,2,2-trifluoro-1-methoxyethyl)pyrazolo[1,5-c]pyrimidin-5-yl)amino)nicotinate | 497.45 |
| **82A** | 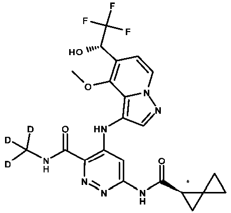 | 4-((4-Methoxy-5-((S)-2,2,2-trifluoro-1-hydroxyethyl)pyrazolo[1,5-a]pyridin-3-yl)amino)-N-(methyl-d3)-6-((R*)-spiro[2.2]pentane-1-carboxamido)pyridazine-3-carboxamide | 508.47 |
| **82B** | 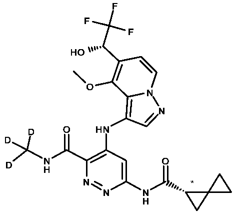 | 4-((4-Methoxy-5-((S)-2,2,2-trifluoro-1-hydroxyethyl)pyrazolo[1,5-a]pyridin-3-yl)amino)-N-(methyl-d3)-6-((S*)-spiro[2.2]pentane-1-carboxamido)pyridazine-3-carboxamide | 508.47 |
| **83** | 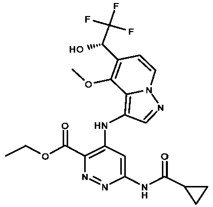 | (S)-ethyl 6-(cyclopropanecarboxamido)-4-((4-methoxy-5-(2,2,2-trifluoro-1-hydroxyethyl)pyrazolo[1,5-a]pyridin-3-yl)amino)nicotinate | 494.42 |
| **84** | 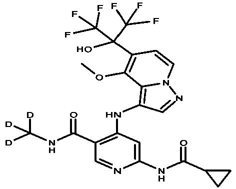 | 6-(Cyclopropanecarboxamido)-4-((5-(1,1,1,3,3,3-hexafluoro-2-hydroxypropan-2-yl)-4-methoxypyrazolo[1,5-a]pyridin-3-yl)amino)-N-(methyl-d3)nicotinamide | 549.44 |
| **85** | 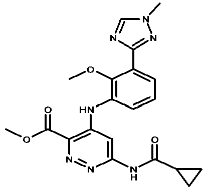 | Methyl 6-(cyclopropanecarboxamido)-4-((2-methoxy-3-(1-methyl-1H-1,2,4-triazol-3-yl)phenyl)amino)pyridazine-3-carboxylate | 423.42 |
| **86** | 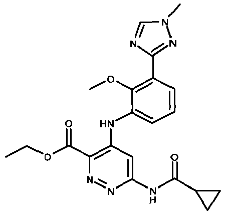 | Ethyl 6-(cyclopropanecarboxamido)-4-((2-methoxy-3-(1-methyl-1H-1,2,4-triazol-3-yl)phenyl)amino)pyridazine-3-carboxylate | 437.45 |
| **87** | 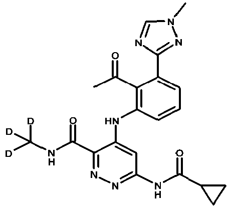 | 4-((2-Acetyl-3-(1-methyl-1H-1,2,4-triazol-3-yl)phenyl)amino)-6-(cyclopropanecarboxamido)-N-(methyl-d3)pyridazine-3-carboxamide | 437.47 |
| **88** | 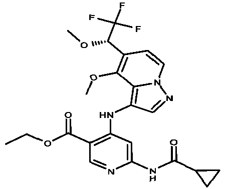 | Ethyl (S)-6-(cyclopropanecarboxamido)-4-((4-methoxy-5-(2,2,2-trifluoro-1-methoxyethyl)pyrazolo[1,5-a]pyridin-3-yl)amino)nicotinate | 507.46 |
| **89** | 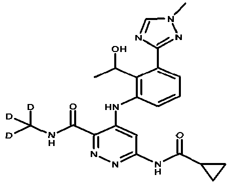 | 6-(Cyclopropanecarboxamido)-4-((2-(1-hydroxyethyl)-3-(1-methyl-1H-1,2,4-triazol-3-yl)phenyl)amino)-N-(methyl-d3)pyridazine-3-carboxamide | 439.48 |
| **90** | 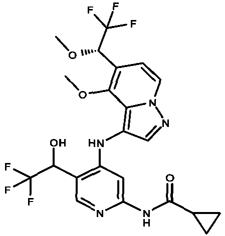 | N-(4-((4-methoxy-5-((S)-2,2,2-trifluoro-1-methoxyethyl)pyrazolo[1,5-a]pyridin-3-yl)amino)-5-(2,2,2-trifluoro-1-hydroxyethyl)pyridin-2-yl)cyclopropanecarboxamide | 533.42 |
| **91** | 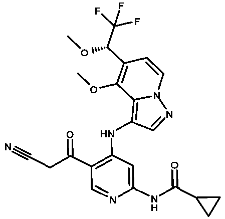 | (S)-N-(5-(2-cyanoacetyl)-4-((4-methoxy-5-(2,2,2-trifluoro-1-methoxyethyl)pyrazolo[1,5-a]pyridin-3-yl)amino)pyridin-2-yl)cyclopropanecarboxamide | 502.45 |
| **92** | 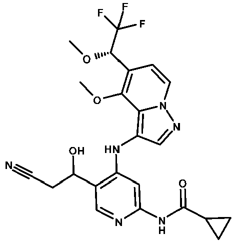 | N-(5-(2-cyano-1-hydroxyethyl)-4-((4-methoxy-5-((S)-2,2,2-trifluoro-1-methoxyethyl)pyrazolo[1,5-a]pyridin-3-yl)amino)pyridin-2-yl)cyclopropanecarboxamide | 504.46 |
| **93** | 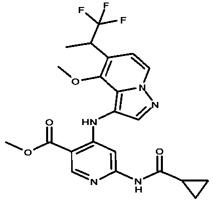 | Methyl 6-(cyclopropanecarbonylamino)-4-[[4-methoxy-5-(2,2,2-trifluoro-1-methylethyl)pyrazolo[1,5-a]pyridin-3-yl]amino]pyridine-3-carboxylate | 477.44 |
| **94** | 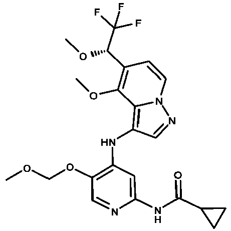 | (S)-N-(4-((4-methoxy-5-(2,2,2-trifluoro-1-methoxyethyl)pyrazolo[1,5-a]pyridin-3-yl)amino)-5-(methoxymethoxy)pyridin-2-yl)cyclopropanecarboxamide | 495.45 |
| **95** | 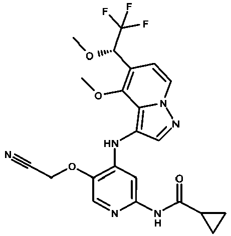 | (S)-N-(5-(cyanomethoxy)-4-((4-methoxy-5-(2,2,2-trifluoro-1-methoxyethyl)pyrazolo[1,5-a]pyridin-3-yl)amino)pyridin-2-yl)cyclopropanecarboxamide | 490.43 |
| **96** | 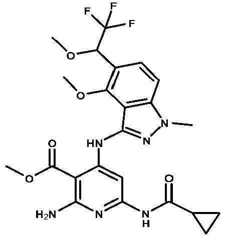 | Methyl 2-amino-6-(cyclopropanecarboxamido)-4-((4-methoxy-1-methyl-5-(2,2,2-trifluoro-1-methoxyethyl)-1H-indazol-3-yl) amino) nicotinate | 522.48 |
| **97** | 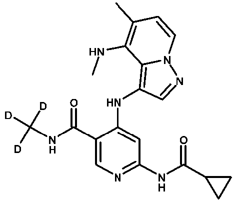 | 6-(Cyclopropanecarboxamido)-N-(methyl-d3)-4-((5-methyl-4-(methylamino)pyrazolo[1,5-a]pyridin-3-yl)amino)nicotinamide | 396.46 |
| **98A** | 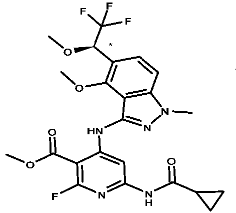 | Methyl (R*)-6-(cyclopropanecarboxamido)-2-fluoro-4-((4-methoxy-1-methyl-5-(2,2,2-trifluoro-1-methoxyethyl)-1H-indazol-3-yl)amino)nicotinate | 525.45 |
| **98B** | 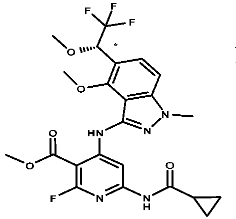 | Methyl (S*)-6-(cyclopropanecarboxamido)-2-fluoro-4-((4-methoxy-1-methyl-5-(2,2,2-trifluoro-1-methoxyethyl)-1H-indazol-3-yl)amino)nicotinate | 525.45 |
| **99** | 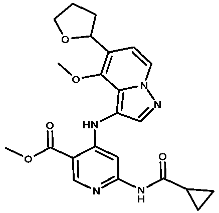 | Methyl 6-(cyclopropanecarboxamido)-4-((4-methoxy-5-(tetrahydrofuran-2-yl)pyrazolo[1,5-a]pyridin-3-yl)amino)nicotinate | 451.47 |
| **100** | 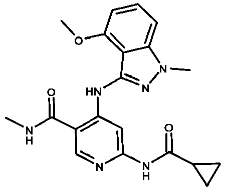 | 6-(Cyclopropanecarboxamido)-4-((4-methoxy-1-methyl-1H-indazol-3-yl)amino)-N-methylnicotinamide | 394.44 |
| **101** | 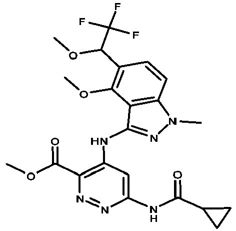 | Methyl 6-(cyclopropanecarboxamido)-4-((4-methoxy-1-methyl-5-(2,2,2-trifluoro-1-methoxyethyl)-1H-indazol-3-yl)amino)pyridazine-3-carboxylate | 508.45 |
| **102** | 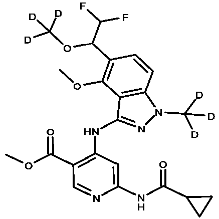 | Methyl 6-(cyclopropanecarboxamido)-4-((5-(2,2-difluoro-1-(methoxy-d3)ethyl)-4-methoxy-1-(methyl-d3)-1H-indazol-3-yl)amino)nicotinate | 495.51 |
| **103** | 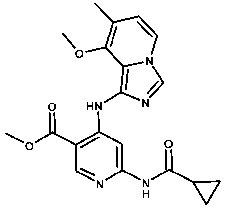 | Methyl 6-(cyclopropanecarboxamido)-4-((8-methoxy-7-methylimidazo[1,5-a]pyridin-1-yl)amino)nicotinate | 395.41 |
| **104** | 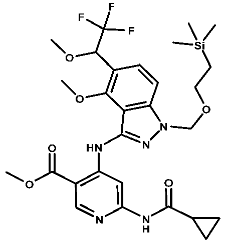 | Methyl 6-(cyclopropanecarboxamido)-4-((4-methoxy-5-(2,2,2-trifluoro-1-methoxyethyl)-1-((2-(trimethylsilyl)ethoxy)methyl)-1H-indazol-3-yl)amino)nicotinate | 623.69 |
| **105** | 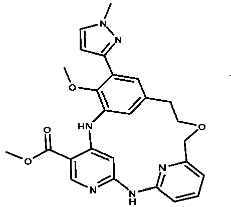 | Methyl 10-methoxy-11-(1-methyl-1H-pyrazol-3-yl)-16-oxa-2,4,8,22-tetraazatetracyclo[16,3.1,1^{3,7}.1^{9,13}] tetracosa-1(22),3,5,7(24),9(23),10,12,18,20-nonaene-6-carboxylate | 486.53 |
| **106** | 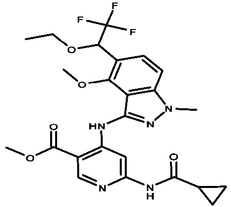 | Methyl 6-(cyclopropanecarboxamido)-4-((5-(1-ethoxy-2,2,2-trifluoroethyl)-4-methoxy-1-methyl-1H-indazol-3-yl)amino)nicotinate | 521.49 |
| **106A** | 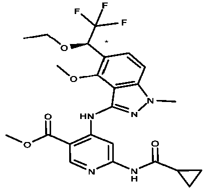 | Methyl (R*)-6-(cyclopropanecarboxamido)-4-((5-(1-ethoxy-2,2,2-trifluoroethyl)-4-methoxy-1-methyl-1H-indazol-3-yl)amino)nicotinate | 521.49 |
| **106B** | 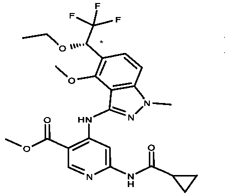 | Methyl (S*)-6-(cyclopropanecarboxamido)-4-((5-(1-ethoxy-2,2,2-trifluoroethyl)-4-methoxy-1-methyl-1H-indazol-3-yl)amino)nicotinate | 521.49 |
| **107** | 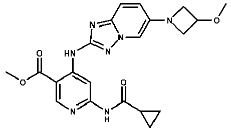 | Methyl 6-(cyclopropanecarbonylamino)-4-[[6-(3-methoxyazetidin-1-yl)-[1,2,4]triazolo[1,5-a]pyridin-2-yl]amino]pyridine-3-carboxylate | 437.45 |
| **108** | 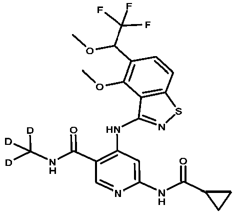 | 6-(Cyclopropanecarboxamido)-4-((4-methoxy-5-(2,2,2-trifluoro-1-methoxyethyl)benzo[d]isothiazol-3-yl)amino)-N-(methyl-d3)nicotinamide | 512.52 |
| **109** | 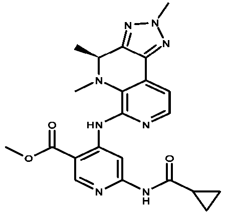 | Methyl (S)-6-(cyclopropanecarboxamido)-4-((2,4,5-trimethyl-4,5-dihydro-2H-[1,2,3]triazolo[4,5-c][1,7]naphthyridin-6-yl)amino)nicotinate | 448.48 |
| **110** | 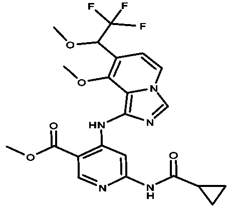 | Methyl 6-(cyclopropanecarboxamido)-4-((8-methoxy-7-(2,2,2-trifluoro-1-methoxyethyl)imidazo[1,5-a]pyridin-1-yl)amino)nicotinate | 493.43 |
| **111** | 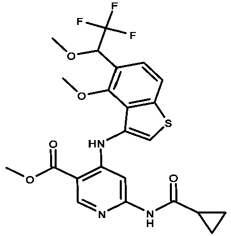 | Methyl 6-(cyclopropanecarboxamido)-4-((4-methoxy-5-(2,2,2-trifluoro-1-methoxyethyl)benzo[b]thiophen-3-yl)amino)nicotinate | 509.50 |
| **112** | 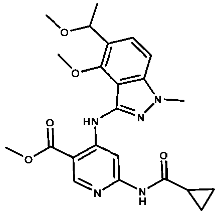 | Methyl 6-(cyclopropanecarboxamido)-4-((4-methoxy-5-(1-methoxyethyl)-1-methyl-1H-indazol-3-yl)amino)nicotinate | 453.49 |
| **113** | 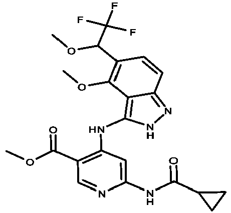 | Methyl 6-(cyclopropanecarboxamido)-4-((4-methoxy-5-(2,2,2-trifluoro-1-methoxyethyl)-1H-indazol-3-yl)amino)nicotinate | 493.43 |
| **114** | 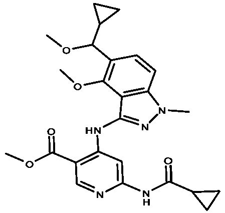 | Methyl 6-(cyclopropanecarboxamido)-4-((5-(cyclopropyl(methoxy)methyl)-4-methoxy-1-methyl-1H-indazol-3-yl)amino)nicotinate | 479.53 |
| **115** | 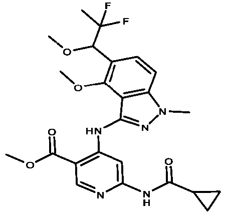 | Methyl 6-(cyclopropanecarboxamido)-4-((5-(2,2-difluoro-1-methoxypropyl)-4-methoxy-1-methyl-1H-indazol-3-yl)amino)nicotinate | 503.50 |
| **115A** | 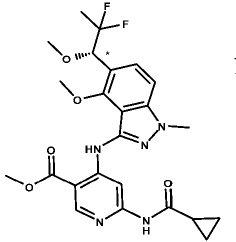 | Methyl (S*)-6-(cyclopropanecarboxamido)-4-((5-(2,2-difluoro-1-methoxypropyl)-4-methoxy-1-methyl-1H-indazol-3-yl)amino)nicotinate | 503.50 |
| **115B** | 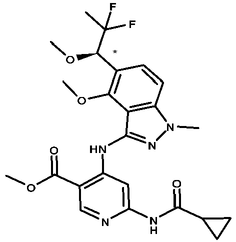 | Methyl (R*)-6-(cyclopropanecarboxamido)-4-((5-(2,2-difluoro-1-methoxypropyl)-4-methoxy-1-methyl-1H-indazol-3-yl)amino)nicotinate | 503.50 |
| **116** | 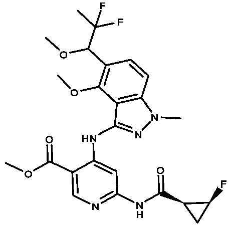 | Methyl 4-((5-(2,2-difluoro-1-methoxypropyl)-4-methoxy-1-methyl-1H-indazol-3-yl)amino)-6-((1S,2S)-2-fluorocyclopropane-1-carboxamido)nicotinate | 521.49 |
| **117** | 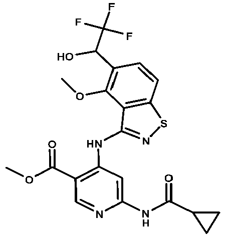 | Methyl 6-(cyclopropanecarboxamido)-4-((4-methoxy-5-(2,2,2-trifluoro-1-hydroxyethyl)benzo[d]isothiazol-3-yl)amino)nicotinate | 496.46 |
| **118** | 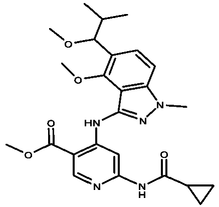 | Methyl 6-(cyclopropanecarboxamido)-4-((4-methoxy-5-(1-methoxy-2-methylpropyl)-1-methyl-1H-indazol-3-yl)amino)nicotinate | 481.54 |
| **119** | 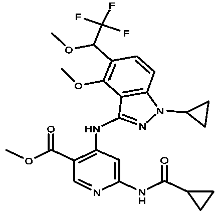 | Methyl 6-(cyclopropanecarboxamido)-4-((1-cyclopropyl-4-methoxy-5-(2,2,2-trifluoro-1-methoxyethyl)-1H-indazol-3-yl)amino)nicotinate | 533.50 |
| **120A** | 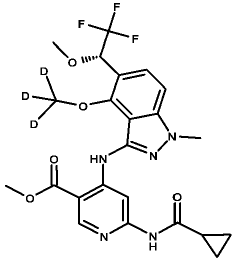 | Methyl (S)-6-(cyclopropanecarboxamido)-4-((4-(methoxy-d3)-1-methyl-5-(2,2,2-trifluoro-1-methoxyethyl)-1H-indazol-3-yl)amino)nicotinate | 510.48 |
| **120B** | 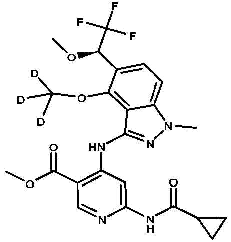 | Methyl (R)-6-(cyclopropanecarboxamido)-4-((4-(methoxy-d3)-1-methyl-5-(2,2,2-trifluoro-1-methoxyethyl)-1H-indazol-3-yl)amino)nicotinate | 510.48 |
| **121** | 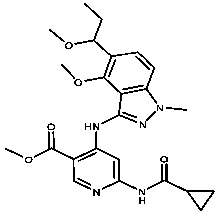 | Methyl 6-(cyclopropanecarboxamido)-4-((4-methoxy-5-(1-methoxypropyl)-1-methyl-1H-indazol-3-yl)amino)nicotinate | 467.52 |
| **122** | 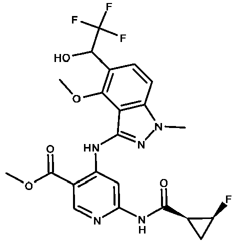 | Methyl 6-((1S,2S)-2-fluorocyclopropane-1-carboxamido)-4-((4-methoxy-1-methyl-5-(2,2,2-trifluoro-1-hydroxyethyl)-1H-indazol-3-yl) amino) nicotinate | 511.43 |
| **122A** | 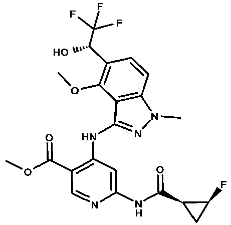 | Methyl 6-((1S,2S)-2-fluorocyclopropane-1-carboxamido)-4-((4-methoxy-1-methyl-5-((S*)-2,2,2-trifluoro-1-hydroxyethyl)-1H-indazol-3-yl) amino) nicotinate | 511.43 |
| **122B** | 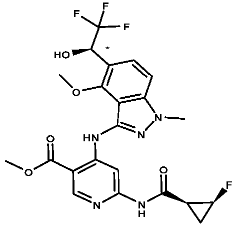 | Methyl 6-((1S,2S)-2-fluorocyclopropane-1-carboxamido)-4-((4-methoxy-1-methyl-5-((R*)-2,2,2-trifluoro-1-hydroxyethyl)-1H-indazol-3-yl) amino) nicotinate | 511.43 |
| **123** | 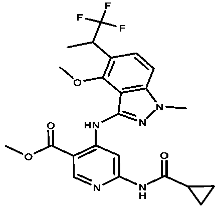 | Methyl 6-(cyclopropanecarboxamido)-4-((4-methoxy-1-methyl-5-(1,1,1-trifluoropropan-2-yl)-1H-indazol-3-yl)amino)nicotinate | 491.46 |
| **124** | 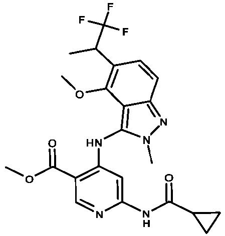 | Methyl 6-(cyclopropanecarboxamido)-4-((4-methoxy-2-methyl-5-(1,1,1-trifluoropropan-2-yl)-2H-indazol-3-yl)amino)nicotinate | 491.46 |
| **125** | 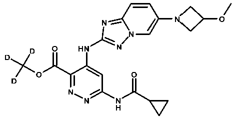 | Methyl-d3 6-(cyclopropanecarboxamido)-4-((6-(3-methoxyazetidin-1-yl)-[1,2,4]triazolo[1,5-a]pyridin-2-yl)amino)pyridazine-3-carboxylate | 441.46 |
| **126** | 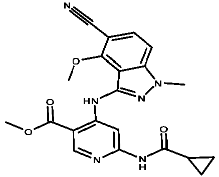 | Methyl 4-((5-cyano-4-methoxy-1-methyl-1H-indazol-3-yl)amino)-6-(cyclopropanecarboxamido)nicotinate | 420.42 |
| **127** | 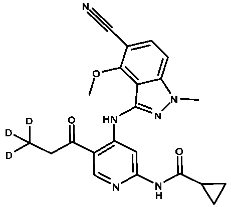 | N-(4-((5-cyano-4-methoxy-1-methyl-1H-indazol-3-yl)amino)-5-(propanoyl-3,3,3-d3)pyridin-2-yl)cyclopropanecarboxamide | 421.47 |
| **128** | 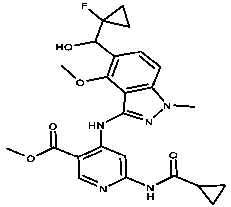 | Methyl 6-(cyclopropanecarboxamido)-4-((5-((1-fluorocyclopropyl)(hydroxy)methyl)-4-methoxy-1-methyl-1H-indazol-3-yl)amino)nicotinate | 483.49 |
| **129** | 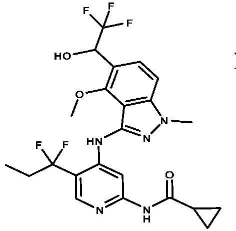 | N-(5-(1,1-difluoropropyl)-4-((4-methoxy-1-methyl-5-(2,2,2-trifluoro-1-hydroxyethyl)-1H-indazol-3-yl)amino)pyridin-2-yl)cyclopropanecarboxamide | 513.46 |
| **130** | 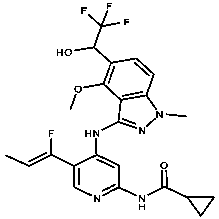 | (Z)-N-(5-(1-fluoroprop-1-en-1-yl)-4-((4-methoxy-1-methyl-5-(2,2,2-trifluoro-1-hydroxyethyl)-1H-indazol-3-yl)amino)pyridin-2-yl)cyclopropanecarboxamide | 493.45 |
| **131** | 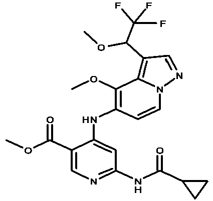 | Methyl 6-(cyclopropanecarboxamido)-4-((4-methoxy-3-(2,2,2-trifluoro-1-methoxyethyl)pyrazolo[1,5-a]pyridin-5-yl)amino)nicotinate | 493.43 |
| **132** | 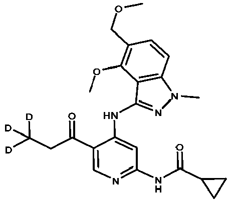 | N-(4-((4-methoxy-5-(methoxymethyl)-1-methyl-1H-indazol-3-yl)amino)-5-(propanoyl-3,3,3-d3)pyridin-2-yl)cyclopropanecarboxamide | 440.51 |
| **133** | 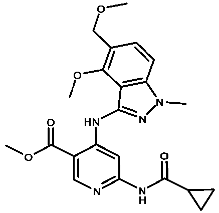 | Methyl 6-(cyclopropanecarboxamido)-4-((4-methoxy-5-(methoxymethyl)-1-methyl-1H-indazol-3-yl)amino)nicotinate | 439.46 |
| **134** | 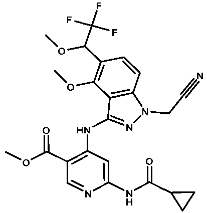 | Methyl 4-((1-(cyanomethyl)-4-methoxy-5-(2,2,2-trifluoro-1-methoxyethyl)-1H-indazol-3-yl)amino)-6-(cyclopropanecarboxamido)nicotinate | 532.47 |
| **135** | 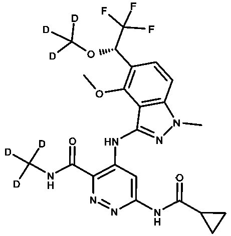 | (S)-6-(cyclopropanecarboxamido)-4-((4-methoxy-1-methyl-5-(2,2,2-trifluoro-1-(methoxy-d3)ethyl)-1H-indazol-3-yl)amino)-N-(methyl-d3)pyridazine-3-carboxamide | 513.50 |
| **136** | 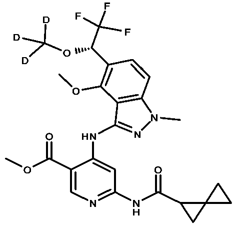 | Methyl 4-((4-methoxy-1-methyl-5-((S)-2,2,2-trifluoro-1-(methoxy-d3)ethyl)-1H-indazol-3-yl)amino)-6-(spiro[2.2]pentane-1-carboxamido)nicotinate | 536.52 |
| **137** | 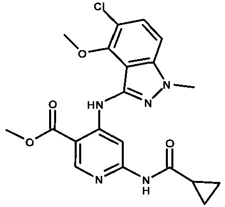 | Methyl 4-((5-chloro-4-methoxy-1-methyl-1H-indazol-3-yl)amino)-6-(cyclopropanecarboxamido)nicotinate | 429.86 |
| **138** | 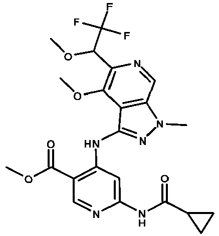 | Methyl 6-(cyclopropanecarbonylamino)-4-[[4-methoxy-1-methyl-5-(2,2,2-trifluoro-1-methoxy-ethyl)pyrazolo[3,4-c]pyridin-3-yl]amino]pyridine-3-carboxylate | 508.45 |
| **139** | 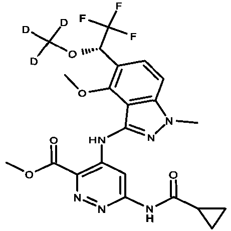 | Methyl (S)-6-(cyclopropanecarboxamido)-4-((4-methoxy-1-methyl-5-(2,2,2-trifluoro-1-(methoxy-d3)ethyl)-1H-indazol-3-yl)amino)pyridazine-3-carboxylate | 511.47 |
| **140** | 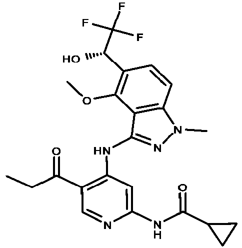 | (S)-N-(4-((4-Methoxy-1-methyl-5-(2,2,2-trifluoro-1-hydroxyethyl)-1H-indazol-3-yl)amino)-5-propionylpyridin-2-yl)cyclopropanecarboxamide | 491.46 |
| **141** | 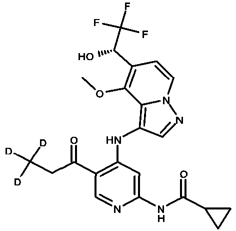 | (S)-N-(4-((4-methoxy-5-(2,2,2-trifluoro-1-hydroxyethyl)pyrazolo[1,5-a]pyridin-3-yl)amino)-5-(propanoyl-3,3,3-d3)pyridin-2-yl)cyclopropanecarboxamide | 480.45 |
| **142** | 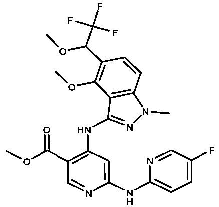 | Methyl 6-((5-fluoropyridin-2-yl)amino)-4-((4-methoxy-1-methyl-5-(2,2,2-trifluoro-1-methoxyethyl)-1H-indazol-3-yl)amino)nicotinate | 534.46 |
| **143** | 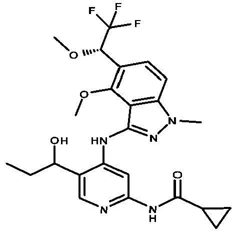 | N-(5-(1-hydroxypropyl)-4-((4-methoxy-1-methyl-5-((S)-2,2,2-trifluoro-1-methoxyethyl)-1H-indazol-3-yl)amino)pyridin-2-yl)cyclopropanecarboxamide | 507.50 |
| **144** | 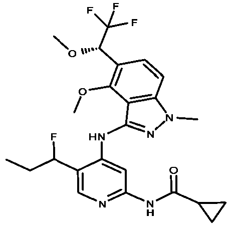 | N-(5-(1-fluoropropyl)-4-((4-methoxy-1-methyl-5-((S)-2,2,2-trifluoro-1-methoxyethyl)-1H-indazol-3-yl)amino)pyridin-2-yl)cyclopropanecarboxamide | 509.50 |
| **145** | 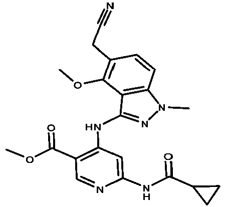 | Methyl 4-((5-(cyanomethyl)-4-methoxy-1-methyl-1H-indazol-3-yl)amino)-6-(cyclopropanecarboxamido)nicotinate | 434.45 |
| **146** | 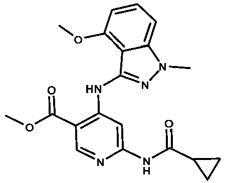 | Methyl 6-(cyclopropanecarboxamido)-4-((4-methoxy-1-methyl-1H-indazol-3-yl)amino)nicotinate | 395.41 |
| **147** | 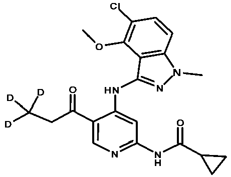 | N-(4-((5-chloro-4-methoxy-1-methyl-1H-indazol-3-yl)amino)-5-(propanoyl-3,3,3-d3)pyridin-2-yl)cyclopropanecarboxamide | 430.90 |
| **148** | 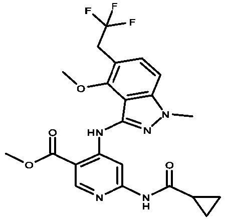 | Methyl 6-(cyclopropanecarboxamido)-4-((4-methoxy-1-methyl-5-(2,2,2-trifluoroethyl)-1H-indazol-3-yl)amino)nicotinate | 477.44 |
| **149** | 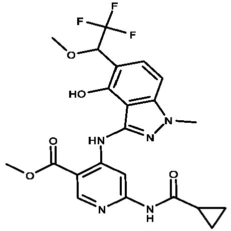 | Methyl 6-(cyclopropanecarboxamido)-4-((4-hydroxy-1-methyl-5-(2,2,2-trifluoro-1-methoxyethyl)-1H-indazol-3-yl)amino)nicotinate | 493.43 |
| **150** | 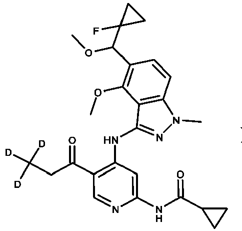 | N-(4-((5-((1-fluorocyclopropyl)(methoxy)methyl)-4-methoxy-1-methyl-1H-indazol-3-yl)amino)-5-(propanoyl-3,3,3-d3)pyridin-2-yl)cyclopropanecarboxamide | 498.56 |
| **151** | 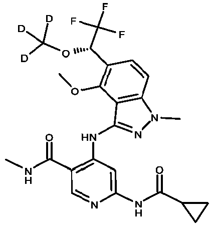 | (S)-6-(cyclopropanecarboxamido)-4-((4-methoxy-1-methyl-5-(2,2,2-trifluoro-1-(methoxy-d3)ethyl)-1H-indazol-3-yl)amino)-N-methylnicotinamide | 509.50 |
| **152** | 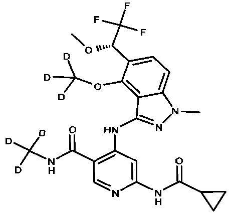 | (S)-6-(cyclopropanecarboxamido)-4-((4-(methoxy-d3)-1-methyl-5-(2,2,2-trifluoro-1-methoxyethyl)-1H-indazol-3-yl)amino)-N-(methyl-d3)nicotinamide | 512.51 |
| **153** | 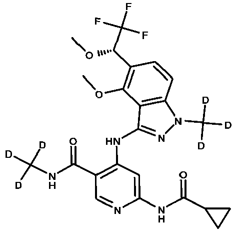 | (S)-6-(cyclopropanecarboxamido)-4-((4-methoxy-1-(methyl-d3)-5-(2,2,2-trifluoro-1-methoxyethyl)-1H-indazol-3-yl)amino)-N-(methyl-d3)nicotinamide | 512.51 |
| **154** | 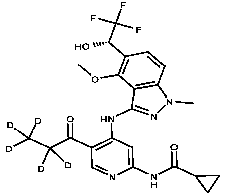 | (S)-N-(4-((4-methoxy-1-methyl-5-(2,2,2-trifluoro-1-hydroxyethyl)-1H-indazol-3-yl)amino)-5-(propanoyl-d5)pyridin-2-yl)cyclopropanecarboxamide | 496.49 |
| **155** | 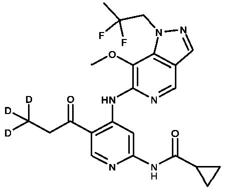 | N-(4-((1-(2,2-difluoropropyl)-7-methoxy-1H-pyrazolo[4,3-c]pyridin-6-yl)amino)-5-(propanoyl-3,3,3-d3)pyridin-2-yl)cyclopropanecarboxamide | 461.48 |
| **156** | 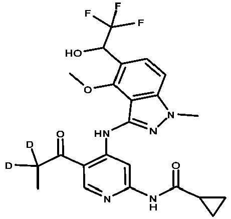 | N-(4-((4-methoxy-1-methyl-5-(2,2,2-trifluoro-1-hydroxyethyl)-1H-indazol-3-yl)amino)-5-(propanoyl-2,2-d2)pyridin-2-yl)cyclopropanecarboxamide | 493.47 |
| **157** | 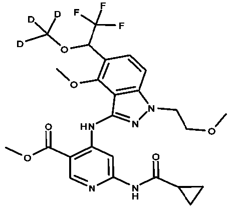 | Methyl 6-(cyclopropanecarboxamido)-4-((4-methoxy-1-(2-methoxyethyl)-5-(2,2,2-trifluoro-1-(methoxy-d3)ethyl)-1H-indazol-3-yl)amino)nicotinate | 554.53 |
| **157A** | 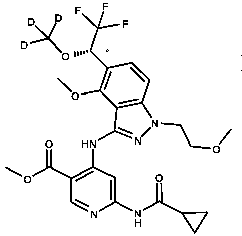 | Methyl (S*)-6-(cyclopropanecarboxamido)-4-((4-methoxy-1-(2-methoxyethyl)-5-(2,2,2-trifluoro-1-methoxyethyl)-1H-indazol-3-yl)amino)nicotinate | 554.53 |
| **157B** | 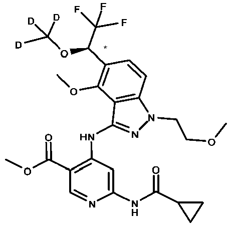 | Methyl (R*)-6-(cyclopropanecarboxamido)-4-((4-methoxy-1-(2-methoxyethyl)-5-(2,2,2-trifluoro-1-methoxyethyl)-1H-indazol-3-yl)amino)nicotinate | 554.53 |
| **158** | 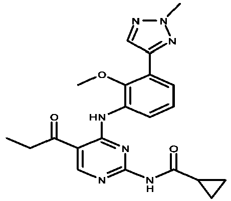 | N-(4-((2-methoxy-3-(2-methyl-2H-1,2,3-triazol-4-yl)phenyl)amino)-5-propionylpyrimidin-2-yl)cyclopropanecarboxamide | 421.45 |
| **159** | 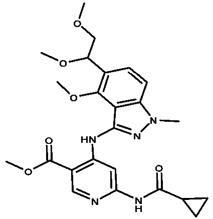 | Methyl 6-(cyclopropanecarbonylamino)-4-[[5-(1,2-dimethoxyethyl)-4-methoxy-1-methyl-indazol-3-yl]amino]pyridine-3-carboxylate | 483.52 |
| **160** | 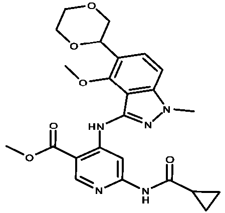 | Methyl 4-((5-(1,4-dioxan-2-yl)-4-methoxy-1-methyl-1H-indazol-3-yl)amino)-6-(cyclopropanecarboxamido)nicotinate | 481.50 |
| **161** | 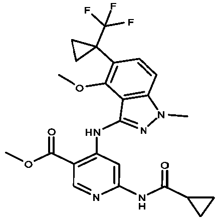 | Methyl 6-(cyclopropanecarboxamido)-4-((4-methoxy-1-methyl-5-(1-(trifluoromethyl)cyclopropyl)-1H-indazol-3-yl)amino)nicotinate | 503.47 |
| **162** | 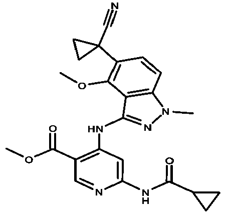 | Methyl 4-((5-(1-cyanocyclopropyl)-4-methoxy-1-methyl-1H-indazol-3-yl)amino)-6-(cyclopropanecarboxamido)nicotinate | 460.48 |
| **163** | 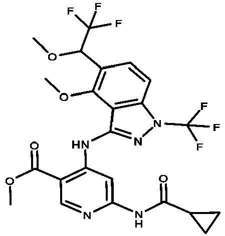 | Methyl 6-(cyclopropanecarboxamido)-4-((4-methoxy-5-(2,2,2-trifluoro-1-methoxyethyl)-1-(trifluoromethyl)-1H-indazol-3-yl)amino)nicotinate | 561.43 |
| **164** | 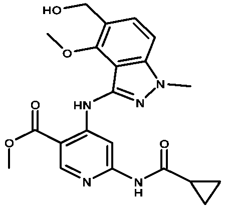 | Methyl 6-(cyclopropanecarboxamido)-4-((5-(hydroxymethyl)-4-methoxy-1-methyl-1H-indazol-3-yl)amino)nicotinate | 425.44 |
| **165** | 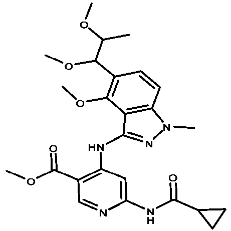 | Methyl 6-(cyclopropanecarboxamido)-4-((5-(1,2-dimethoxypropyl)-4-methoxy-1-methyl-1H-indazol-3-yl)amino)nicotinate | 497.54 |
| **166** | 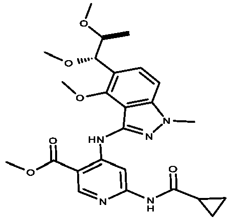 | Methyl 6-(cyclopropanecarboxamido)-4-((5-((1S)-1,2-dimethoxypropyl)-4-methoxy-1-methyl-1H-indazol-3-yl)amino)nicotinate | 497.54 |
| **167** | 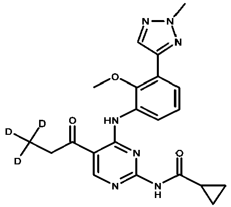 | N-(4-((2-Methoxy-3-(2-methyl-2H-1,2,3-triazol-4-yl)phenyl)amino)-5-(propanoyl-3,3,3-d3)pyrimidin-2-yl)cyclopropanecarboxamide | 424.47 |
| **168** | 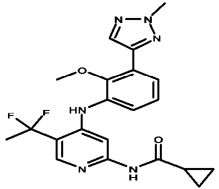 | N-(5-(1,1-Difluoroethyl)-4-((2-methoxy-3-(2-methyl-2H-1,2,3-triazol-4-yl)phenyl)amino)pyridin-2-yl)cyclopropanecarboxamide | 428.43 |
| **169** | 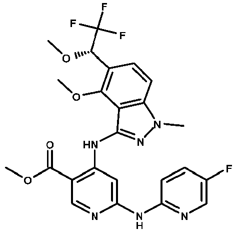 | Methyl (S)-6-((5-fluoropyridin-2-yl)amino)-4-((4-methoxy-1-methyl-5-(2,2,2-trifluoro-1-methoxyethyl)-1H-indazol-3-yl)amino)nicotinate | 534.46 |
| **170** | 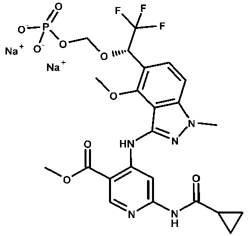 | Sodium (S)-(1-(3-((2-(cyclopropanecarboxamido)-5-(methoxycarbonyl)pyridin-4-yl)amino)-4-methoxy-1-methyl-1H-indazol-5-yl)-2,2,2-trifluoroethoxy)methyl phosphate | 647.40 |
| **171** | 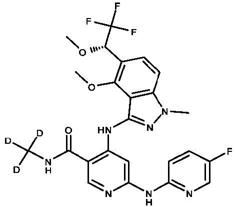 | (S)-6-((5-Fluoropyridin-2-yl)amino)-4-((4-methoxy-1-methyl-5-(2,2,2-trifluoro-1-methoxyethyl)-1H-indazol-3-yl)amino)-N-(methyl-d3)nicotinamide | 536.50 |
| **172** | 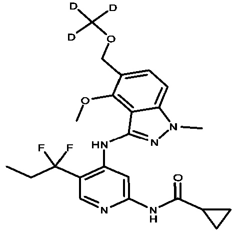 | N-(5-(1,1-Difluoropropyl)-4-((4-methoxy-5-((methoxy-d3)methyl)-1-methyl-1H-indazol-3-yl)amino)pyridin-2-yl)cyclopropanecarboxamide | 462.51 |
| **173** | 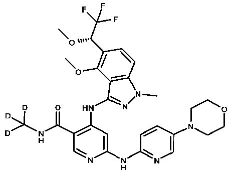 | (S)-4-((4-Methoxy-1-methyl-5-(2,2,2-trifluoro-1-methoxyethyl)-1H-indazol-3-yl)amino)-N-(methyl-d3)-6-((5-morpholinopyridin-2-yl)amino)nicotinamide | 603.61 |
| **174** | 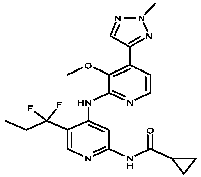 | N-(5-(1,1-Difluoropropyl)-4-((3-methoxy-4-(2-methyl-2H-1,2,3-triazol-4-yl)pyridin-2-yl)amino)pyridin-2-yl)cyclopropanecarboxamide | 443.67 |
| **175** | 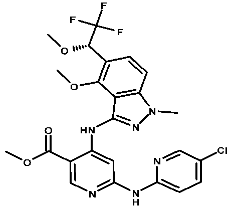 | Methyl (S)-6-((5-chloropyridin-2-yl)amino)-4-((4-methoxy-1-methyl-5-(2,2,2-trifluoro-1-methoxyethyl)-1H-indazol-3-yl)amino)nicotinate | 550.92 |
| **176** | 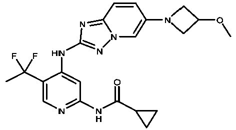 | N-(5-(1,1-Difluoroethyl)-4-((6-(3-methoxyazetidin-1-yl)-[1,2,4]triazolo[1,5-a]pyridin-2-yl)amino)pyridin-2-yl)cyclopropanecarboxamide | 443.45 |
| **177** | 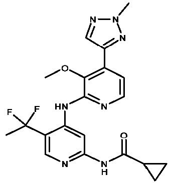 | N-(5-(1,1-Difluoroethyl)-4-((3-methoxy-4-(2-methyl-2H-1,2,3-triazol-4-yl)pyridin-2-yl)amino)pyridin-2-yl)cyclopropanecarboxamide | 429.42 |
| **178** | 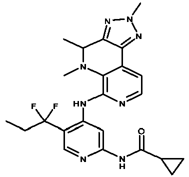 | N-(5-(1,1-Difluoropropyl)-4-((2,4,5-trimethyl-4,5-dihydro-2H-[1,2,3]triazolo[4,5-c][1,7]naphthyridin-6-yl)amino)pyridin-2-yl)cyclopropanecarboxamide | 468.50 |
| **179** | 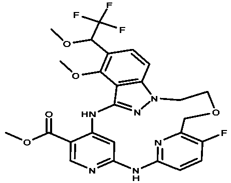 | Methyl 12-fluoro-23-methoxy-22-(2,2,2-trifluoro-1-methoxyethyl)-15-oxa-2,6,8,18,25,26-hexaazapentacyclo[16.6.1,1^ {3,7}.1^{9,13 }.0^{19,24}]heptacosa-1(25),3(27),4,6,9,11,13(26),19,21,23-decaene-4-carboxylate | 576.50 |
| **180** | 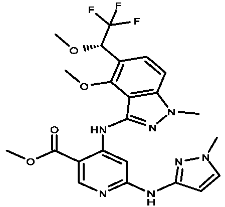 | Methyl (S)-4-((4-methoxy-1-methyl-5-(2,2,2-trifluoro-1-methoxyethyl)-1H-indazol-3-yl)amino)-6-((1-methyl-1H-pyrazol-3-yl)amino)nicotinate | 519.48 |
| **181** | 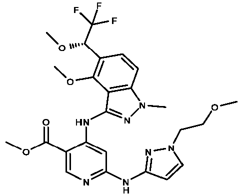 | Methyl (S)-4-((4-methoxy-1-methyl-5-(2,2,2-trifluoro-1-methoxyethyl)-1H-indazol-3-yl)amino)-6-((1-(2-methoxyethyl)-1H-pyrazol-3-yl)amino)nicotinate | 563.53 |
| **182** | 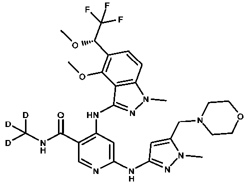 | (S)-4-((4-Methoxy-1-methyl-5-(2,2,2-trifluoro-1-methoxyethyl)-1H-indazol-3-yl)amino)-N-(methyl-d3)-6-((1-methyl-5-(morpholinomethyl)-1H-pyrazol-3-yl)amino)nicotinamide | 620.64 |
| **183** | 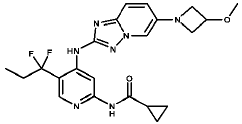 | N-(5-(1,1-Difluoropropyl)-4-((6-(3-methoxyazetidin-1-yl)-[1,2,4]triazolo[1,5-a]pyridin-2-yl)amino)pyridin-2-yl)cyclopropanecarboxamide | 457.49 |
| **184** | 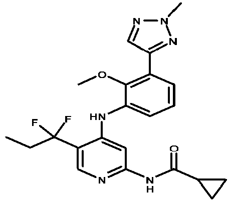 | N-(5-(1,1-Difluoropropyl)-4-((2-methoxy-3-(2-methyl-2H-1,2,3-triazol-4-yl)phenyl)amino)pyridin-2-yl)cyclopropanecarboxamide | 442.47 |
| **185** | 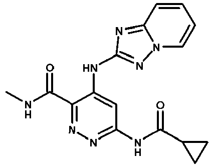 | 4-([1,2,4]Triazolo[1,5-a]pyridin-2-ylamino)-6-(cyclopropanecarboxamido)-N-methylpyridazine-3-carboxamide | 352.36 |
| **186** | 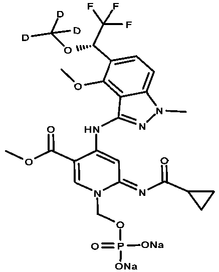 | Sodium (S,E)-(2-((cyclopropanecarbonyl)imino)-4-((4-methoxy-1-methyl-5-(2,2,2-trifluoro-1-(methoxy-d3)ethyl)- 1H-indazol-3-yl)amino)-5-(methoxycarbonyl)pyridin-1(2H)-yl)methyl phosphate | 664.46 |
| **187** | 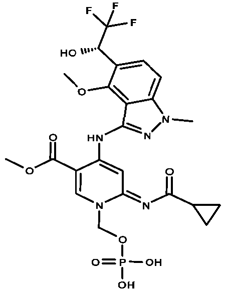 | Methyl (S,E)-6-((cyclopropanecarbonyl)imino)-4-((4-methoxy-1-methyl-5-(2,2,2-trifluoro-1-hydroxyethyl)-1H-indazol-3-yl)amino)-1-((phosphonooxy)methyl)-1,6-dihydropyridine-3-carboxylate | 603.45 |
| **188** | 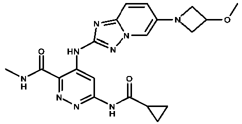 | 6-(Cyclopropanecarboxamido)-4-((6-(3-methoxyazetidin-1-yl)-[1,2,4]triazolo[1,5-a]pyridin-2-yl)amino)-N-methylpyridazine-3-carboxamide | 437.46 |
| **189** | 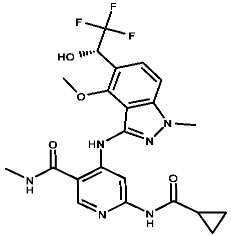 | (S)-6-(Cyclopropanecarboxamido)-4-((4-methoxy-1-methyl-5-(2,2,2-trifluoro-1-hydroxyethyl)-1H-indazol-3-yl)amino)-N-methylnicotinamide | 492.46 |
| **190** | 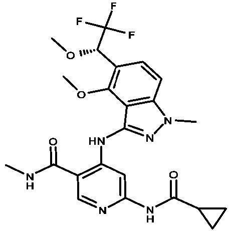 | (S)-6-(Cyclopropanecarboxamido)-4-((4-methoxy-1-methyl-5-(2,2,2-trifluoro-1-methoxyethyl)-1H-indazol-3-yl)amino)-N-methylnicotinamide | 506.49 |
| **191** | 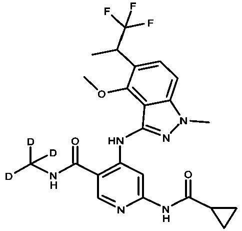 | 6-(Cyclopropanecarboxamido)-4-((4-methoxy-1-methyl-5-(1,1,1-trifluoropropan-2-yl)-1H-indazol-3-yl)amino)-N-(methyl-d3)nicotinamide | 493.51 |
| **192** | 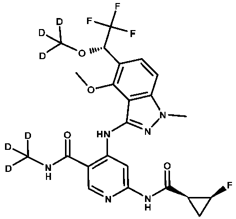 | 6-((1S,2S)-2-Fluorocyclopropane-1-carboxamido)-4-((4-methoxy-1-methyl-5-((S)-2,2,2-trifluoro-1-(methoxy-d3)ethyl)-1H-indazol-3-yl)amino)-N-(methyl-d3)nicotinamide | 530.51 |
| **193A** | 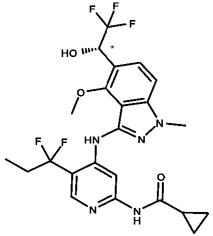 | (S*)-N-(5-(1,1-Difluoropropyl)-4-((4-methoxy-1-methyl-5-(2,2,2-trifluoro-1-hydroxyethyl)-1H-indazol-3-yl)amino)pyridin-2-yl)cyclopropanecarboxamide | 513.47 |
| **193B** | 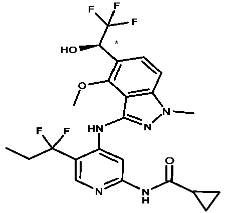 | (R*)-N-(5-(1,1-Difluoropropyl)-4-((4-methoxy-1-methyl-5-(2,2,2-trifluoro-1-hydroxyethyl)-1H-indazol-3-yl)amino)pyridin-2-yl)cyclopropanecarboxamide | 513.47 |
| **194** | 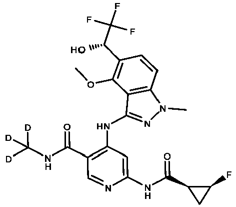 | 6-((1S,2S)-2-Fluorocyclopropane-1-carboxamido)-4-((4-methoxy-1-methyl-5-((S)-2,2,2-trifluoro-1-hydroxyethyl)-1H-indazol-3-yl)amino)-N-(methyl-d3)nicotinamide | 513.47 |
| **199** | 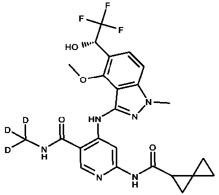 | 4-((4-Methoxy-1-methyl-5-((S)-2,2,2-trifluoro-1-hydroxyethyl)-1H-indazol-3-yl)amino)-N-(methyl-d3)-6-(spiro[2.2]pentane-1-carboxamido)nicotinamide | 521.52 |

Note: A asterisk * at the chiral center in the chemical structures or the S* or R* in the names means the compound is a chiral pure but absolute configuration unknown single isomer.

### Binding Activity test for JH2 domain of JAK1 and TYK2

The compounds are prepared in DMSO for 200x top dose, then serial dilute it with 27-fold for 3 more points. Add 8µL/well in echo source plate, echo will add 75nL/well with 3-fold serial dilution for 11 points in assay plate. 75nL DMSO for high control and low control.

Prepare 3X working solutions (1.5nM) of JAK1-JH2 domain or TYK2-JH2 domain enzyme in assay buffer, add 5µL of enzyme working solutions per well to the assay plate including high control. For low control, add 5µL/well assay buffer. And then spin down at 1000rpm and centrifuge for 30 seconds. After enzyme system prepared, add 5µL of Tb-antibody solution into each well of assay plate. Spin down at 1000 rpm and centrifuge for 30sec. After Tb-antibody added, also add 5µL of tracer into the assay plate. Spin down at 1000rpm and centrifuge for 30sec. Incubate for 60mins at 25°C firstly and then continue to incubate the plate at 4°C overnight. Read by envision in FRET mode. The Luminescence value was recorded by a multi-label reader Envision (PerkinElmer). Inhibition Rate was calculated relative to vehicle (DMSO) treated control wells using following formula: $% Inhibition = 100 - \frac{{RLU}_{compound} - {RLU}_{□} lowcontrol}{{RLU}_{□} high control - {RLU}_{□} low control} \times 100 %$ wherein
RLU_compound = the relative light unit of cells treated with test compounds
RLU_low control = the relative light unit of medium with DMSO only
RLU_high control = the relative light unit of cells treated with DMSO only

The dose-response (percent inhibition) curve was plotted and IC50 values (the concentration that causes 50% growth inhibition) were determined by GraphPad software. The IC50 of tested compounds are shown in Table 2.

**Table 2. IC50 on JH2 domain activity (nM)**

| **Example** | **TYK2 JH2 Binding IC50(nM)** | **JAK1 JH2 Binding IC50(nM)** | **JAK1/TYK2 selectivity** |
|---|---|---|---|
| **1** | 2.331 | 19.527 | 8.4 |
| **2** | 0.333 | 5.236 | 15.7 |
| **3** | 0.223 | 13.352 | 59.9 |
| **4** | 0.164 | 15.329 | 93.5 |
| **5** | 0.137 | 6.619 | 48.3 |
| **6** | 0.482 | 7.446 | 15.4 |
| **7** | 0.147 | 11.007 | 74.9 |
| **7A** | 0.097 | 10.436 | 107.6 |
| **7B** | 0.528 | 12.867 | 24.4 |
| **8** | 0.106 | 3.39 | 32.0 |
| **9** | 0.158 | 8.756 | 55.4 |
| **9A** | 0.424 | 38.961 | 91.9 |
| **9B** | 0.08 | 5.039 | 63.0 |
| **10** | 0.224 | 9.706 | 43.3 |
| **11** | 0.182 | 17.571 | 96.5 |
| **12** | 0.308 | 21.084 | 68.5 |
| **13** | 0.172 | 20.978 | 122.0 |
| **14A** | 0.102 | 10.344 | 101.4 |
| **14B** | 0.583 | 15.254 | 26.2 |
| **15A** | 0.078 | 12.545 | 160.8 |
| **15B** | 0.375 | 19.977 | 53.3 |
| **16** | 0.22 | 14.081 | 64.0 |
| **17** | 0.102 | 12.745 | 125.0 |
| **18** | 0.13 | 1.627 | 12.5 |
| **19** | 0.273 | 276.237 | 1011.9 |
| **20** | 0.058 | 13.643 | 235.2 |
| **21** | 0.077 | 10.986 | 142.7 |
| **22** | 0.185 | 7.505 | 40.6 |
| **23** | 0.185 | 13.455 | 72.7 |
| **24** | 0.066 | 18.599 | 281.8 |
| **25** | 0.068 | 11.497 | 169.1 |
| **26** | 0.183 | 61.869 | 338.1 |
| **27** | 0.064 | 8.191 | 128.0 |
| **28** | 0.073 | 7.483 | 102.5 |
| **29** | 0.219 | 16.734 | 76.4 |
| **30** | 0.088 | 10.948 | 124.4 |
| **31** | 0.183 | 149.259 | 815.6 |
| **32** | 0.171 | 23.455 | 137.2 |
| **32A** | 0.099 | 18.574 | 187.6 |
| **32B** | 0.108 | 11.469 | 106.2 |
| **33** | 0.082 | 34.303 | 418.3 |
| **34** | 161.249 | >298.5 | >1.85 |
| **35** | 8.274 | 92.335 | 11.2 |
| **36** | 4.548 | 254.872 | 56.0 |
| **37** | 1.648 | 110.61 | 67.1 |
| **38** | 0.761 | 143.581 | 188.7 |
| **39** | 0.561 | >298.5 | >532.1 |
| **40** | 186.126 | >298.5 | >1.6 |
| **41** | 46.84 | >298.5 | >6.37 |
| **42** | 0.202 | 79.026 | 391.2 |
| **43** | 0.175 | 71.263 | 407.2 |
| **44** | 2.8 | 60.185 | 21.5 |
| **45** | 0.209 | 64.27 | 307.51 |
| **45A** | 0.826 | >298.5 | >361.38 |
| **45B** | 0.124 | 24.465 | 197.30 |
| **46** | 0.113 | 6.815 | 60.31 |
| **46A** | 0.074 | 3.007 | 40.64 |
| **46B** | 0.18 | / | / |
| **47** | 0.221 | 46.455 | 210.20 |
| **48** | 0.166 | 33.647 | 202.69 |
| **48A** | 0.145 | 23.039 | 158.89 |
| **48B** | 1.68 | 85.902 | 51.13 |
| **49** | 0.136 | 4.098 | 30.13 |
| **49A** | 0.107 | 5.28 | 49.3 |
| **49B** | 0.211 | 6.069 | 28.76 |
| **50A** | 1.039 | >298.5 | >287.30 |
| **50B** | 0.177 | 28.84 | 162.94 |
| **51A** | 0.154 | 6.29 | 40.84 |
| **51B** | 0.171 | 1.27 | 7.43 |
| **52A** | 0.082 | 41.14 | 501.71 |
| **52B** | 0.616 | >298.5 | >484.60 |
| **53A** | 0.627 | / | / |
| **53B** | 0.15 | / | / |
| **54A** | 0.118 | 3.82 | 32.37 |
| **54B** | 0.433 | 7.085 | 16.36 |
| **55A** | 0.052 | 2.544 | 48.9 |
| **55B** | 0.08 | 12.283 | 153.54 |
| **56** | 0.967 | 52.627 | 54.42 |
| **57** | 7.325 | >298.5 | >40.75 |
| **58** | 0.304 | 11.946 | 39.30 |
| **59** | 0.834 | 44.149 | 52.94 |
| **60** | 0.546 | 17.947 | 32.87 |
| **61** | 1.363 | / | / |
| **62** | 0.98 | 153.835 | 156.97 |
| **63** | 0.285 | / | / |
| **64** | 0.141 | 34.686 | 246 |
| **65A** | 0.54 | >298.5 | >553.80 |
| **65B** | 0.09 | 12.46 | 144.84 |
| **66A** | 2.81 | >298.5 | 106.23 |
| **66B** | 0.13 | 90.70 | 719.87 |
| **67** | 0.15 | / | / |
| **67A** | 0.21 | 18.631 | 88.72 |
| **67B** | 0.32 | 119.351 | 372.97 |
| **68** | 0.14 | 16.291 | 116.36 |
| **69** | 0.13 | 14.86 | 114.31 |
| **70A** | 0.15 | 21.768 | 145.12 |
| **70B** | 2.12 | 47.181 | 22.26 |
| **71A** | 0.14 | 7.257 | 51.84 |
| **71B** | 0.25 | / | / |
| **72** | 0.14 | / | / |
| **73** | 0.14 | / | / |
| **74** | 0.18 | / | / |
| **75** | 0.09 | 24.717 | 274.63 |
| **76** | 0.09 | / | / |
| **77** | 0.08 | 184.637 | 2307.96 |
| **78** | 0.17 | / | / |
| **79** | 0.28 | 42.69 | 150.32 |
| **80** | 0.26 | 45.96 | 176.08 |
| **81** | 4.02 | >298.5 | >74.25 |
| **82A** | 0.21 | 7.96 | 38.29 |
| **82B** | 0.23 | 6.32 | 27.48 |
| **83** | 0.76 | >298.5 | >393.28 |
| **84** | 4.24 | >298.5 | >70.42 |
| **85** | 197.98 | >298.5 | >1.51 |
| **86** | >298.5 | >298.5 | / |
| **87** | >298.5 | >298.5 | / |
| **88** | 1.70 | >298.5 | >175.18 |
| **89** | >298.5 | >298.5 | / |
| **90** | >298.5 | >298.5 | / |
| **91** | 5.24 | >298.5 | >56.93 |
| **92** | 26.54 | 79.63 | 3.00 |
| **93** | 1.24 | 63.68 | 51.39 |
| **94** | 140.69 | 192.69 | 1.37 |
| **95** | 68.27 | >298.5 | >4.37 |
| **96** | 93.59 | >298.5 | >3.19 |
| **97** | 2.37 | / | / |
| **98A** | >298.5 | / | / |
| **98B** | 3.88 | / | / |
| **99** | 1.79 | / | / |
| **100** | 0.97 | 112.9 | 116.4 |
| **101** | >298.5 | / | / |
| **102** | 0.19 | 18.47 | 99.81 |
| **103** | 5.84 | / | / |
| **104** | 13.68 | / | / |
| **105** | 1.208 | 231.865 | 191.94 |
| **106** | 1.31 | / | / |
| **106A** | 4.31 | / | / |
| **106B** | 0.21 | 15.790 | 75.19 |
| **107** | 0.25 | / | / |
| **109** | 0.836 | / | / |
| **110** | 2.468 | / | / |
| **111** | 0.364 | / | / |
| **112** | 0.162 | / | / |
| **113** | 0.258 | 74.511 | 288.80 |
| **114** | 0.209 | / | / |
| **115** | 0.182 | / | / |
| **115A** | 0.119 | / | / |
| **115B** | 1.215 | / | / |
| **116** | 0.188 | / | / |
| **118** | 0.943 | / | / |
| **119** | 0.308 | / | / |
| **120A** | 0.089 | / | / |
| **120B** | 0.442 | / | / |
| **121** | 0.405 | / | / |
| **122** | 0.178 | / | / |
| **122A** | 0.072 | 15.573 | 216.29 |
| **122B** | 0.733 | / | / |
| **123** | 0.37 | 174.023 | 470.33 |
| **124** | 90.276 | / | / |
| **125** | 52.39 | >298.5 | >5.70 |
| **126** | 1.793 | / | / |
| **127** | 0.256 | / | / |
| **128** | 0.26 | / | / |
| **129** | 0.466 | / | / |
| **130** | 0.688 | / | / |
| **131** | 12.662 | / | / |
| **132** | 0.174 | / | / |
| **133** | 0.288 | / | / |
| **134** | 1.52 | / | / |
| **135** | 0.07 | / | / |
| **136** | 0.21 | 77.832 | 370.63 |
| **137** | 0.377 | / | / |
| **138** | 1.218 | / | / |
| **139** | / | / | / |
| **140** | 0.061 | 4.357 | 71.43 |
| **141** | 0.075 | / | / |
| **142** | 0.185 | / | / |
| **143** | 34.698 | / | / |
| **144** | 42.116 | / | / |
| **145** | 0.151 | / | / |
| **146** | 9.8 | / | / |
| **147** | 0.146 | / | / |
| **148** | 0.091 | / | / |
| **149** | 0.747 | / | / |
| **150** | 0.154 | / | / |
| **151** | 0.053 | / | / |
| **152** | 0.071 | / | / |
| **153** | 0.055 | / | / |
| **154** | 0.066 | 6.882 | 104.27 |
| **155** | 0.891 | / | / |
| **156** | 0.163 | 14.16 | 86.87 |
| **157** | 0.15 | / | / |
| **157A** | 0.095 | / | / |
| **157B** | 1.41 | / | / |
| **158** | 0.496 | / | / |
| **159** | 0.203 | 8.211 | 40.45 |
| **160** | 0.709 | / | / |
| **161** | 0.568 | / | / |
| **162** | 0.261 | / | / |
| **163** | 0.488 | / | / |
| **164** | 0.481 | / | / |
| **165** | 2.71 | / | / |
| **166** | 1.106 | / | / |
| **167** | 0.275 | 31.993 | 116.34 |
| **168** | 0.27 | 1.03 | 3.81 |
| **169** | 0.19 | >298.5 | >1571 |
| **170** | 0.074 | / | / |
| **171** | 0.171 | 16.958 | 99.17 |
| **172** | 0.79 | 18.165 | 22.99 |
| **173** | 0.161 | 2.388 | 14.83 |
| **174** | 0.86 | 61.45 | 71.45 |
| **175** | 0.384 | >298.5 | >777.34 |
| **176** | 0.768 | 17.055 | 22.21 |
| **177** | 0.749 | 6.367 | 8.50 |
| **178** | 3.156 | / | / |
| **179** | 0.481 | / | / |
| **180** | 0.176 | >298.5 | >1696 |
| **181** | 0.309 | >298.5 | >966 |
| **182** | 0.141 | 1.968 | 13.95 |
| **184** | 0.216 | 6.851 | 31.71 |
| **185** | 0.565 | 9.207 | 16.30 |
| **189** | 0.054 | 3.505 | 64.91 |
| **190** | 0.076 | 1.954 | 25.71 |
| **191** | 0.132 | 5.960 | 45.15 |
| **192** | 0.075 | 2.631 | 35.08 |
| **193A** | 0.417 | / | / |
| **193B** | 1.178 | / | / |
| **194** | 0.080 | 3.903 | 48.8 |
| **195** | 2.96 | 67.124 | 22.68 |
| **196** | 3.93 | 31.6 | 8.04 |
| **197** | 2.716 | 17.402 | 6.41 |
| **198** | 1.48 | 10.36 | 7.0 |
| **199** | 0.091 | 5.582 | 61.34 |

Exemplary compounds of the invention showed good selectivity over JAK1. These compounds can be used to reduce or eliminate the side effects caused by JAK1 inhibition in autoimmune disease.

### Biochemical assay

### Testing for JAK1, JAK2 and TYK2 Kinase Activities

JAK activity was determined in the reaction buffer 50 mM HEPES, 0.01% Brij35, 10 mM MgCl₂, 2 mM DTT by a microfluidic assay. The phosphorylation of a FAM labeled peptide substrate was monitored in the Caliper EZ Reader II (Perkin Elmer). The assay condition for each batch of enzyme (Carna Biosciences) was optimized to obtain 10% conversion rate of peptide substrate.

The test compounds were dissolved in DMSO to a stock concentration of 10 mM. Three-fold serially diluted compounds with top concentration of 5 µM were pre-incubated with JAK1, JAK2 or TYK2 for 10 min at ambient temperature. The final DMSO concentration of assay mixture was 1%. FAM labeled peptide substrate (final concentration 3 µM) and ATP (Km concentration or 1mM) were sequentially added to initiate the kinase reaction at 28°C for 90 min (JAK), 15 min (JAK2) and 30 min (TYK2), respectively. The reaction was stopped by adding 50 mM EDTA.

The well in the test plate without enzyme was defined as 100% inhibition. And the well without compound but with equivalent DMSO was defined as no inhibition. The percent inhibition was calculated by the following formula: $% Inhibition = \frac{Conversion % _ max - Conversion % _ sample}{Conversion % _ max - Conversion % _ min} \times 100$ wherein
Conversion%_ ₘₐₓ = the conversion rate in the positive well without addition of compound
Conversion%_ ₛₐₘₚₗₑ = the conversion rate of test compounds
Conversion%_ ₘᵢₙ = the conversion rate in the well without addition of enzyme

The dose-response (percent inhibition) curve was plotted and IC50 values were determined by GraphPad software. The IC50 values of tested compounds were list in **Table 3.**

**Table 3. JAK1, JAK2 and TYK2 IC50 Values of Illustrative Compounds**

| **Example** | **JAK1** (10 nM) (1 mM ATP), nM | **JAK2** (1.5 nM) (1 mM ATP), nM | **TYK2** (20 nM) (1 mM ATP), nM |
|---|---|---|---|
| **7A** | >20000 | >20000 | >20000 |
| **7B** | >20000 | >20000 | >20000 |
| **21** | >20000 | >20000 | >20000 |
| **25** | >20000 | >20000 | >20000 |
| **27** | >20000 | >20000 | >20000 |
| **30** | >20000 | >20000 | >20000 |
| **48A** | >5000 | >5000 | >5000 |
| **49A** | >5000 | >10000 | >5000 |
| **50B** | >5000 | >5000 | >5000 |
| **55A** | >5000 | >10000 | >5000 |

Exemplary compounds of the invention showed excellent selectivity over JAK1, JAK2 and TYK2 kinase domain inhibition.

### Anti-proliferative assay

Dimerization domain of Tel protein fused with JAK kinase domain was permanently transduced into Ba/F3 cells, whose proliferation is dependent on JAK activity in the absence of IL-3 induction. These engineered Ba/F3-FL-TYK2-P760L cells were used to monitor JAK inhibitory activities of the compounds in the cellular. Ba/F3 cells were cultured in RPMI-1640 (Corning) containing 10% fetal bovine serum. Cells were seeded at 2000/well of white flat bottom 96-well plates. The well containing medium only was used as background control. After 24h growth, cells were treated with compounds. The test compounds were dissolved in DMSO to a stock concentration of 20 mM. 3-fold serially diluted compounds for 9 concentrations with top concentration of 20 µM was added into the each well. The final DMSO concentration was 0.1%. The cells continued to grow at 37°C in 5% CO₂ for 72 h after compound treatment. The viability was measured by cellular ATP determination using the Cell-Titer Glo luciferase reagent (Promega). The Luminescence value was recorded by a multi-label reader Envision (PerkinElmer). Inhibition Rate was calculated relative to vehicle (DMSO) treated control wells using following formula: $% Inhibition = 100 - \frac{RLU _ compound - RLU _ blank}{RLU _ control - RLU _ blank} \times 100 %$ wherein
RLU_compound = the relative light unit of cells treated with test compounds
RLU_blank = the relative light unit of medium with DMSO only
RLU_control = the relative light unit of cells treated with DMSO only

The dose-response (percent inhibition) curve was plotted and IC50 values (the concentration that causes 50% growth inhibition) were determined by GraphPad software. The IC50 of tested compounds are shown in **Table 4.**

**Table 4. Ba/F3-FL-TYK2-P760L Cells IC50 Values of Exemplary Compounds**

| **Example** | **Baf3-FL-T2P760L (nM)** | **Example** | **Baf3-FL-T2P760L (nM)** |
|---|---|---|---|
| **1** | 1173 | **92** | 877.9 |
| **2** | 17.04 | **93** | 577.2 |
| **3** | 43.65 | **94** | 4629 |
| **4** | 38.92 | **95** | >5000.0 |
| **5** | 31.99 | **96** | 2993 |
| **6** | 146.4 | **97** | / |
| **7** | 9.32 | **98A** | / |
| **7A** | 5.4 | **98B** | / |
| **7B** | 223.25 | **99** | / |
| **8** | 31.26 | **100** | 504.7 |
| **9** | 51.65 | **101** | / |
| **9A** | 2142 | **102** | 3.905 |
| **9B** | 54.19 | **103** | / |
| **10** | 119.5 | **104** | / |
| **11** | 54.49 | **105** | 66.77 |
| **12** | 193.6 | **106** | / |
| **13** | 130.9 | **106A** | / |
| **14A** | 7.41 | **106B** | 5.486 |
| **14B** | 207.2 | **107** | 27.76 |
| **15A** | 10.58 | **108** | / |
| **15B** | 870.4 | **109** | / |
| **16** | 87.03 | **110** | / |
| **17** | 84.28 | **111** | 19 |
| **18** | 90.28 | **112** | 11.36 |
| **19** | 137.3 | **113** | >1000.0 |
| **20** | 23.13 | **114** | 21.93 |
| **21** | 15.69 | **115** | 5.581 |
| **22** | 127.4 | **115A** | 9.828 |
| **23** | 165.7 | **115B** | / |
| **24** | 3.87 | **116** | 4.649 |
| **25** | 5.01 | **117** | / |
| **26** | 35.25 | **118** | / |
| **27** | 8.54 | **119** | / |
| **28** | 10.67 | **120A** | 2.706 |
| **29** | 80.945 | **120B** | 107.1 |
| **30** | 7.96 | **121** | / |
| **31** | 60.26 | **122** | 1.158 |
| **32** | 42.31 | **122A** | 0.8 |
| **32A** | 33.03 | **122B** | / |
| **32B** | 27.34 | **123** | 88.34 |
| **33** | 16.02 | **124** | / |
| **34** | >5000.0 | **125** | / |
| **35** | 2344 | **126** | / |
| **36** | 1768 | **127** | 52.29 |
| **37** | 767.7 | **128** | 17.28 |
| **38** | 1345 | **129** | 1.411 |
| **39** | 350.5 | **130** | 157.2 |
| **40** | >5000.0 | **131** | / |
| **41** | >5000.0 | **132** | 25.93 |
| **42** | 104.4 | **133** | 53.28 |
| **43** | 20.6 | **134** | / |
| **44** | 356.2 | **135** | 0.327 |
| **45** | 8.606 | **136** | 21.23 |
| **45A** | 110.255 | **137** | 99.8 |
| **45B** | 2.526 | **138** | / |
| **46** | 0.618 | **139** | / |
| **46A** | 0.31 | **140** | 0.463 |
| **46B** | 11.24 | **141** | 11.32 |
| **47** | 70.637 | **142** | 12.04 |
| **48** | 2.189 | **143** | / |
| **48A** | 1.496 | **144** | / |
| **48B** | 161.9 | **145** | 15.16 |
| **49** | 0.729 | **146** | / |
| **49A** | 0.464 | **147** | 23.8 |
| **49B** | 18.04 | **148** | 7.084 |
| **50A** | 131.2 | **149** | / |
| **50B** | 2.854 | **150** | 32.68 |
| **51A** | 12.32 | **151** | 0.213 |
| **51B** | 0.399 | **152** | 0.284 |
| **52A** | 3.556 | **153** | 0.235 |
| **52B** | 119.1 | **154** | 0.455 |
| **53A** | / | **155** | / |
| **53B** | 2.841 | **156** | 1.757 |
| **54A** | 0.577 | **157** | 9.215 |
| **54B** | / | **157A** | 2.511 |
| **55A** | 0.352 | **157B** | / |
| **55B** | 16.22 | **158** | 68.44 |
| **56** | 148.1 | **159** | 6.289 |
| **57** | 718.6 | **160** | 119 |
| **58** | 32.373 | **161** | 73.97 |
| **59** | 208.2 | **162** | 26.59 |
| **60** | 94.39 | **163** | 56.14 |
| **61** | / | **164** | 50.1 |
| **62** | >1000.0 | **165** | 280.9 |
| **63** | 48.35 | **166** | 112.6 |
| **64** | 12.97 | **167** | 60.93 |
| **65A** | 117.4 | **168** | 19.12 |
| **65B** | 2.1 | **169** | 8.329 |
| **66A** | / | **170** | / |
| **66B** | 12.51 | **171** | 1.028 |
| **67** | 2.496 | **172** | 21.92 |
| **67A** | / | **173** | 0.401 |
| **67B** | / | **174** | 15.43 |
| **68** | 3.603 | **175** | 18.09 |
| **69** | / | **176** | 13.31 |
| **70A** | 2.346 | **177** | 15.2 |
| **70B** | / | **178** | 34.95 |
| **71A** | 0.412 | **179** | 30.86 |
| **71B** | 17.92 | **180** | 14.3 |
| **72** | 2.649 | **181** | 35.19 |
| **73** | 7.564 | **182** | 0.817 |
| **74** | 30.74 | **183** | 14.54 |
| **75** | / | **184** | 14.90 |
| **76** | 3.49 | **185** | 89.14 |
| **77** | >1000.0 | **186** | / |
| **78** | 5.259 | **187** | / |
| **79** | 108.85 | **188** | / |
| **80** | 88.86 | **189** | 0.119 |
| **81** | 306.2 | **190** | 0.196 |
| **82A** | 34.95 | **191** | 2.509 |
| **82B** | 27.015 | **192** | 0.088 |
| 83 | 172.65 | **193A** | 0.395 |
| 84 | 634.1 | **193B** | 45.010 |
| 85 | >5000.0 | **194** | 0.241 |
| 87 | 1862 | **195** | 329.4 |
| 88 | 718.9 | **196** | 856.9 |
| **89** | >5000.0 | **197** | 425.8 |
| **90** | >5000.0 | **198** | 259.7 |
| **91** | 1383 | | |

### IFN-γ Release in NK92 Cells Stimulated by IL-12

*Purpose:* To evaluate the inhibition activity of TYK2 inhibitors in NK92 cells on TYK2 pathway through ELISA method.

### Materials:

| **Items** | **Vendor** | **Cat. No.** |
|---|---|---|
| PBS | BI | 02-024-01ACS |
| α-MEM | Gibco | 12561056 |
| Inactivated Horse Serum | Gibco | 26050088 |
| FBS | Gibco | 10091148 |
| β- Mercaptoethanol | procell | PB180633 |
| Penicillin-Streptomycin | Gibco | 15140-122 |
| Folic Acid | MCE | HY-16637A |
| i-Inositol | MCE | HY-B1411 |
| Recombinant human IL-2 | PEPROTECH | 200-02-10UG |
| Recombinant human IL-12 P70 | PEPROTECH | 200-12-50UG |
| ELISA MAX^{™} Deluxe Set Human IFN-γ | BD | 555142 |
| Nunc Edge 2 96-well plate | Thermo | 167425 |
| TMB | Solarbio | PR1200 |
| ELISA Plate | Corning | 9018 |
| Stop Solution | Elabscience | E-ELIR-006 |

*Procedure:* 1) Seeding NK92 cells into 96-well plate, cell density is 1.5E5 cells/well, 100uL/well; 2)Add prepared test articles into indicated wells, 50uL/well, and continue incubate for 1h, at 37°C; 3) After compound treatment, add 50uL/well IL-12 into indicated wells, final concentration of IL-12 is 2.5ng/mL; 4)Incubate cytokine stimulated cells at 37°C for another 15hrs in CO₂ incubator; 5) After cytokine stimulation, transfer culture supernatant into a new plate and centrifuge for 5min at 350x g, and detect IFN-γ concentration in the supernatant with ELISA kit; 6) Data analysis with GrapdPad Prism6.0 software.

**Table 5. IC50 Values of Exemplary Compounds on TYK2 Pathway**

| **Example** | **IC50 on TYK2 pathway (nM)** |
|---|---|
| **7A** | 9.10 |
| **15A** | 15.70 |
| **21** | 14.49 |
| **25** | 7.18 |
| **26** | 17.6 |
| **27** | 6.02 |
| **30** | 6.72 |
| **43** | 22.63 |
| **100** | 86.76 |
| **195** | 1500 |
| **196** | 922.8 |
| **197** | 515.1 |

Exemplary compounds of the invention showed good TYK2 pathway inhibition activity in cells.

### Phosphorylation Inhibition of STAT in Human Whole Blood Testing:

The inhibitory potential of test articles in human whole blood assay was evaluated by the method of flow cytometry.

IFN-alpha will activate JAK1 and TYK2 kinase in T cells by binding to IFN receptors, and then lead to phosphorylation of STAT1 and STAT2. The phosphorylated STAT1 enter nuclear and promote transcription and expression of IFN-gamma. To evaluate the efficacy of TYK2i in T cells, freshly collected human whole blood will be stimulated with certain unit of human IFN-alpha (Universal Type I IFN(1MU), R&D, 11200-2) for 20 mins in incubator. After stimulation, fix cells with Phosflow^{™} Fix Buffer I (BD, 557870), and then collect fixed cells by centrifugation (500 g, 8min). Wash cells once with pre-cooled PBS, and then permeabilize cells with cold Perm Buffer III buffer (BD, 558050) for 45 mins on ice. Collect cells by centrifugation (600 g, 8min) and washed twice with cold PBS. Stain cells with anti-human CD3(FITC Mouse Anti-Human CD3, BD, 555332) and anti-pSTAT1_Y701(Alexa Fluor 647 Mouse Anti-Stat1 (pY701), BD, 612597) antibodies. Detect pSTAT1 by flow cytometry (CytoFlex S) and analyze data with FlowJo and GraphPad Prism 8 software. IC50 values of test articles were determined using 4- parameter logistic equation.

Exemplary results are summarized in **Table 6.**

**Table 6. IC50 Values of Exemplary Compounds in Human Whole Blood**

| **Example** | **pSTAT1 inhibition in hWB (IC50,nM)** | **Example** | **pSTAT1 inhibition in hWB (IC50,nM)** |
|---|---|---|---|
| **2** | 140.66 | **32A** | 99.43 |
| **3** | 176.6 | **43** | 173.8 |
| **4** | 312.3 | **46A** | 32.98 |
| **5** | 139.34 | **48A** | 29.12 |
| **7** | 67.43 | **49** | 27.85 |
| **7A** | 31.15 | **49A** | 10.49 |
| **7B** | 1005 | **50B** | 56.49 |
| **8** | 75.46 | **51B** | 76.52 |
| **9B** | 289.7 | **54A** | 10.79 |
| **14A** | 35.91 | **55A** | 24.84 |
| **15A** | 34.83 | **70A** | 43.67 |
| **18** | 84.33 | **71A** | 54.85 |
| **21** | 22.71 | **107** | 401.3 |
| **22** | 80.81 | **141** | 177.2 |
| **24** | 34.58 | **159** | 128.7 |
| **25** | 30.83 | **192** | 24.60 |
| **27** | 51.22 | **195** | >5000 |
| **30** | 52.21 | **196** | >10000 |
| **31** | 863.3 | **198** | 1469 |

### TYK2 Inhibitors Activities Detection in Human Microglia Cells (HMC3) and Astrocyte (CCF-STTG1)

### Method 1: Phosphorylation of STAT1 inhibition detection in HMC3 and CCF-STTG1 cells by Jess

Seeding HMC3 cells into 12-well plate and incubate overnight. Pre-stimulation with recombinant human IFNα (Sino biological, H08Y), treat cells with compound with different concentration for 1hr. After compound treatment, stimulate HMC3 cells with 50ng/mL hINFα for another 1 hour (for CCF-STTG1 cells IFNα induction time is 30 mins). And then wash cells with DPBS once gently and lyse cells with pre-cooled RIPA buffer (Sigma, R0278-500ML) for 30min on ice to harvest cells. Centrifuge cell lysate for 20 mins at 12000rpm with freezer centrifuge (Thermo Scientific, Heraeus Fresco 21) to extract protein. Prepare samples with Jess Simple Wes kit (SM-WO04) and then run western blot on Jess simple western system (Protein simple, JS-4631). Collect data and analyzed with GraphPad Prism.

### Method2: Phosphorylation of STAT1 inhibition detection in HMC3 cells with HTRF assay

Seeding HMC3 cells into 96-well plate and incubate overnight. Pre-stimulation with recombinant human IFNα (Sino biological, H08Y), treat cells with compound with different concentration for 1hr. After compound treatment, stimulate cells with 50ng/mL hINFα for another 1 hour (for CCF-STTG1 cells IFNα induction time is 30 mins). After compound treatment and cytokine stimulation, dump old medium and lyse cells with supplemented lysis buffer provided by the HTRF kit (Revvity, 63ADK026PEG) for 30 min at room temperature under shaking. After homogenization by pipetting up and down, transfer 16 µL of cell lysate from the 96-well cell-culture plate to a small volume white 384-well plate, and then add 4 µL of pre-mixed two ready-to-use antibody working solution into each wells. Cover the plate with a plate sealer and incubate it overnight at room temperature. Read the fluorescence emission at two different wavelengths (665nm and 620nm) on microplate reader (Molecular Device, SpectraMax i3X). Collect data and analyzed with GraphPad Prism.

Exemplary results are summarized in **Table 7, Table 8 and Table 9.**

**Table 7. Inhibition in Microglia Cells by Jess Method**

| **Example** | **Jess_HMC3 IC50 (IFNa pSTAT1, nM)** |
|---|---|
| **7A** | 1.040 |
| **45B** | 4.800 |
| **48A** | 1.030 |
| **49A** | 0.084 |
| **50B** | 9.300 |
| **68** | 3.168 |
| **122A** | 0.239 |
| **193A** | 4.279 |

**Table 8. Inhibition in Microglia Cells by HTRF Method**

| **Example** | **HTRF_HMC3 IC 50 (IFNa pSTAT1, nM)** |
|---|---|
| **48A** | <0.061 |
| **49A** | <0.061 |
| **54A** | <0.061 |
| **55A** | 0.192 |
| **68** | <0.061 |
| **70A** | <0.061 |
| **122A** | <0.061 |
| **159** | 0.082 |
| **193A** | 0.821 |

**Table 9. Inhibition in Astrocyte Cells by Jess Method**

| **Example** | **Jess_CCF-STTG1 IC50 (IFNa pSTAT1, nM)** |
|---|---|
| **7A** | 6.82 |
| **45B** | 3.5 |
| **48A** | 0.74 |
| **50B** | 3.73 |

### ADME

### Microsomal Metabolic Stability Assay

Microsomes were pre-incubated with test compound or control compounds for 5 min at 37°C in 100 mM potassium phosphate buffer, pH 7.4, 1.0 mM EDTA. The reaction was initiated by addition of 15 µL of the NADPH regenerating system to 30 µL of each incubation mixture per time point. The final incubation condition was composed of 0.5 mg/mL microsomal protein, 1 µM test article/positive control, 2 mM NADPH. The 0-minute samples were prepared by addition of a 30 µL aliquot of each incubation mixture to 135 µL quench reagent to precipitate proteins. And then a 15 µL aliquot of the NADPH regenerating system was added. At 5, 15, 30 and 45 minutes, the reaction will be stopped by the addition of cold acetonitrile solution containing internal standard. The samples taken at all time points were centrifuged at 5000×g for 15 minutes. 50 µL of supernatant are taken into 96-well assay plates pre-added with 50 µL ultrapure water, and then analyzed by LC/MS/MS.

Concentrations of test articles, control compounds in the samples were determined by using LC/MS/MS method. Plotting of the chromatograms and peak area integrations are carried out by Analyst (AB Sciex).

In the determination of the *in vitro* elimination constant (ke) of the control compounds, the analyte/internal standard peak area ratios will be converted to percentage remaining (%Remaining) with the following equation: $%Remaining = \frac{Peak area ratio of analyte to IS at each time point}{Peak area ratio of analyte to IS at t = 0} \times 100 %$

The CLᵢₙₜ of microsomes was calculated using the formula: CL_{int (mic)}=0.693/T_{1/2} /mg microsome protein per mL. The slope was measured by the natural logarithm of the percentage of the residual compound and time, T1/2 was calculated according to the following formula. $T = \frac{0.693}{- slope}$

Exemplary results are summarized in **Table 10.**

**Table 10. T1/2 and CLᵢₙₜ Values of Exemplary Compounds**

| **Example** | **Human LM T1/2 (min)** | **Human LM Clint (uL/min/mg)** |
|---|---|---|
| **7A** | 88.7 | 15.63 |
| **7B** | 128.3 | 10.80 |
| **13** | 62.99 | 22.00 |
| **14A** | 74.25 | 18.67 |
| **15A** | 116.42 | 11.91 |
| **17** | 112.21 | 12.35 |
| **21** | 60.72 | 22.83 |
| **22** | 308.09 | 4.5 |
| **23** | 7938.23 | 0.17 |
| **24** | 56.24 | 24.64 |
| **29** | 49.64 | 27.92 |
| **30** | 81.46 | 17.01 |
| **35** | 242.29 | 5.72 |
| **37** | 113 | 12.27 |
| **38** | 188.78 | 7.34 |
| **45B** | 75.44 | 18.37 |
| **48** | 71.92 | 19.27 |
| **49** | 88.39 | 15.68 |
| **49A** | 71.69 | 19.33 |
| **50B** | 126.58 | 10.95 |
| **51B** | 172.03 | 8.06 |
| **53B** | 104.78 | 13.23 |
| **66B** | 97.49 | 14.22 |
| **67** | 75.7 | 18.31 |
| **102** | 46.56 | 29.77 |
| **122A** | 89.6 | 15.47 |
| **156** | 52.87 | 26.22 |
| **157** | 53.83 | 25.75 |

### Caco-2 Cell Permeability

Compounds permeability was evaluated using Caco-2 cell lines. HBSS buffer was used as transfer buffer. Appropriate dosing and receiver solutions were applied to the donor and receiver chambers to initiate the transport assay in apical to basolateral (A to B) or basolateral to apical (B to A) directions. Compounds were tested at 10.0 µM in the absence of p-gp inhibitor bidirectionally in duplicates. After incubation at 37°C for 90 mins, donor and receiver samples were analyzed by LC-MS/MS. The intestinal absorption potential was assessed using Pₐₚₚ value and efflux ratio.

Exemplary results are summarized in **Table 11.**

**Table 11. Papp and Efflux Ratio Values of Exemplary Compounds**

| **Example** | **Papp (10⁻⁶ cm·s⁻¹) A-B** | **Papp (10⁻⁶ cm·s⁻¹) B-A** | **Efflux Ratio: Papp(B-A)/Papp(A-B)** |
|---|---|---|---|
| **21** | 0.71 | 47.38 | 66.99 |
| **23** | 0.46 | 37.64 | 82.67 |
| **25** | 2.57 | 62.31 | 24.22 |
| **33** | 0.39 | 46.52 | 120.68 |
| **49A** | 1.32 | 66.57 | 50.31 |
| **45B** | 7.27 | 5.65 | 0.78 |
| **48A** | 29.90 | 29.01 | 0.97 |
| **122A** | 27.4 | 45.31 | 1.65 |
| **BMS986165** | 0.98 | 55.12 | 56.50 |

| | | | |
|---|---|---|---|
| BMS986165, See, PCT/CN2022/139649 (filed on December 16, 2022), Example 41. | | | |

Exemplary ester compounds 45B,48A and 122A have lower efflux ratio numbers then the amide compounds 21,23,25,33,49A and BMS986165 which makes them can be easier to penetrate the blood-brain barrier.

### Kinetic Solubility

Aliquots of 8 µL of reference and test compound stock solutions (10 mM/5 mM) are added into 792 µL of 100 mM pH 7.4 phosphate buffer. The final DMSO concentration is 1%. Sample tubes are shaken for 2 hours at 1000 rpm at room temperature. Samples are centrifuged at 12000 rpm for 10 min to precipitate un-dissolved particles. And transfer the supernatants to a new tube or plate. Add 5 µL of samples (no diluted, 10 times diluted, and 100 times diluted) and standard curve samples to 95 µL of ACN containing IS for LC-MS/MS analysis.
Exemplary results are summarized in **Table 12**

**Table 12. Kinetic Solubilities Values of Exemplary Compounds**

| **Example** | **Kinetics Solubility_PBS7.4(µM)** | **Example** | **Kinetics Solubility_PBS7.4(µM)** |
|---|---|---|---|
| **2** | 15.30 | **27** | 14.25 |
| **3** | 15.10 | **29** | 67.20 |
| **5** | 57.40 | **30** | 41.95 |
| **7** | 42.90 | **31** | 39.85 |
| **7A** | 52.14 | **32** | 62.20 |
| **7B** | 55.80 | **32A** | 25.50 |
| **8** | 18.55 | **33** | 59.35 |
| **9** | 60.02 | **38** | 37.20 |
| **9A** | 35.80 | **43** | 33.30 |
| **9B** | 45.35 | **45B** | 9.37 |
| **12** | 15.14 | **46A** | 8.16 |
| **13** | 11.18 | **48A** | 6.98 |
| **14A** | 45.46 | **49** | 83.05 |
| **15A** | 23.91 | **49A** | 45.5 |
| **19** | 22.70 | **49B** | 37.45 |
| **20** | 15.00 | **50B** | 7.23 |
| **21** | 57.20 | **55A** | 7.78 |
| **24** | 10.37 | **122A** | 10.3 |
| **25** | 43.00 | **188** | 1.01 |

Exemplary compounds of the invention showed ideal solubility for further development.

### Brian Pharmacokinetic Studies in Rats

Pharmacokinetic profiles of test articles were evaluated in fasted Sprague-Dawley rats. Different kinds of formulations were designed to ensure test articles fully dissolved and wouldn't precipitate in 1:10 SGF or FaSSIF dilution. Typically, Rats were dosed with 3 mg/kg and 5 mg/kg by intravenous injection and oral gavage, respectively. The animal was restrained manually at the designated time points, approximately 150 µL of blood sample was collected via jugular vein for serial bleeding into K2EDTA tubes. The blood samples were maintained in wet ice first and centrifuged to obtain plasma (2000g, 4°C, 5 min) within 30 minutes post sampling. Anesthetizing the rat with the Isoflurane vaporizer to 2%~3% and place its back on the operation table. Spread the animal limbs and pin the limbs on the operation table. Make a cut along the sternum about 4cm long, low enough to expose the sternum's end. Expose the heart and use a 25-gauge needle connected with a 20 mL syringe filled with 20 mL of cold saline to pierce the left ventricle. Maintain the placement of the needle, and slowly push the syringe plunger to wash the brain with saline. The perfusion will be finished within about 2 min and then take the brain out of the body carefully without damaging the tissue. Dry the tissue on filtrate paper, which should be placed on ice, and then weigh the brain tissue and store at -70 °C and analyzed by LC/MS/MS for quantification. PK parameter values, including, but not necessarily limited to, the maximum plasma concentrations (Cmax), and the area under the plasma concentration vs. time curve (AUC) from time zero to 24-hour (AUC0-24h) were determined using WinNonlin program.

**Table 13. Systemic Pharmacokinetic Parameters in Sprague-Dawley Rats**

| **Example** | **Final State of the Formulation** | **Administration Route** | **Dose (mg/kg)** | **AUC (h*ng/ml)** | **T1/2 (h)** |
|---|---|---|---|---|---|
| **26** | solution | *iv* | 3 | 1289 | 1.53 |
| **42** | solution | *iv* | 3 | 646 | 0.34 |
| **45B** | solution | *iv* | 3 | 1283 | 2.28 |
| **48A** | solution | *iv* | 3 | 1532 | 1.62 |
| **54A** | solution | *iv* | 2 | 1063 | 1.70 |
| **BMS986165** | solution | *po* | 5 | 4088 | 1.82 |

**Table 14. Brain Pharmacokinetic Parameters in Sprague-Dawley Rats**

| **Example** | **Final State of the Formulation** | **Administration Route** | **Dose (mg/kg)** | **T1/2 (h)** | **AUC (h*ng/g)** | **Brain AUC/Systemic AUC** |
|---|---|---|---|---|---|---|
| **26** | solution | *iv* | 3 | 1.99 | 140 | 0.11 |
| **42** | solution | *iv* | 3 | 0.25 | 1286 | 1.99 |
| **45B** | solution | *iv* | 3 | 2.23 | 1235 | 0.96 |
| **48A** | solution | *iv* | 3 | 2.04 | 288 | 0.19 |
| **54A** | solution | *iv* | 2 | 1.64 | 188 | 0.18 |
| **BMS986165** | solution | *po* | 5 | 7.85 | 82.6 | 0.02 |

Conclusion: Examples 26, 42, 45B, 48A and 54A showed good brain exposure in Rats.

### Brian Pharmacokinetic Studies in Dogs

Pharmacokinetic profiles of test articles were evaluated in Beagle dogs. Different kinds of formulations were designed to ensure test articles fully dissolved and wouldn't precipitate in 1:10 SGF or FaSSIF dilution. The animal was restrained manually at the designated time points, approximately 500 µL of blood sample was collected via cephalic vein into K2EDTA tubes. The blood samples were maintained in wet ice first and centrifuged to obtain plasma (2000g, 4°C, 5 min) within 15 minutes post sampling. The animal was euthanized with pure propofol inhalation. Shave the hair on the back of head and neck, and clean the skin of the area with iodine and 75% ethanol. Inject about 4-5 L saline from carotid artery for perfusion. Brain was collected after the perfusion fluid (saline) was gradually clarified. Dry the tissue on filtrate paper on ice, and then weigh the brain tissue and store at -70°C for later analysis. PK parameter values, including, but not necessarily limited to, the maximum plasma concentrations (Cmax), and the area under the plasma concentration vs. time curve (AUC) from time zero to 4-hour (AUC0-4h) were determined using WinNonlin program.

**Table 15. Systemic Pharmacokinetic Parameters in Beagle Dogs**

| **Example** | **Final State of the Formulation** | **Administration Route** | **Dose (mg/kg)** | **AUC (h*ng/ml)** |
|---|---|---|---|---|
| **45B** | solution | *iv* | 2 | 1759 |
| **50B** | solution | *iv* | 2 | 1367 |

**Table 16. Brain Pharmacokinetic Parameters in Beagle Dogs**

| **Example** | **Final State of the Formulation** | **Adminis tration Route** | **Dose (mg/kg)** | **AUC (h*ng/g)** | **Brain AUC/Systemic AUC** |
|---|---|---|---|---|---|
| **45B** | solution | *iv* | 2 | 1963 | 1.12 |
| **50B** | solution | *iv* | 2 | 1856 | 1.36 |

Conclusion: Examples 45B and 50B showed good brain exposure in Dogs.

### Mouse, Rat and Dog Pharmacokinetic Studies:

Pharmacokinetic profiles of test articles were evaluated in fasted mouse, Sprague-Dawley rats and Beagle dogs. After dosing, blood samples were collected at each time point. For IV injection group, time points were set at 5, 15, 30 min, and then 1, 2, 4, 8 and 24 hours after dosing. For oral gavage group,#time points were set at 15, 30 min, and then 1, 2, 4, 8, and 24 hours. Blood was collected into appropriately labeled tubes containing K₂EDTA as the anticoagulant. Plasma was obtained within 1 hours of blood collection by centrifugation at 8000×g and 4 °C for 6 minutes, and then stored at -20 °C until analyzed by LC/MS/MS for quantification. PK parameter values, including, but not necessarily limited to, the maximum plasma concentrations (Cmax), the time to reach the maximum concentrations (Tmax), and the area under the plasma concentration vs. time curve (AUC) from time zero to 24-hour (AUC0-24h) were determined using WinNonlin program.

**Table 17. Pharmacokinetic Parameters in Beagle dogs by Intravenous Administration**

| **Example** | **Final State of the Formulation** | **Dose (mg/kg)** | **AUC (h*ng/ml)** | **T1/2 (h)** | **Cl (mL/min/Kg)** | **Vd (L/Kg)** |
|---|---|---|---|---|---|---|
| **55A** | solution | 2 | 9980 | 3.64 | 3.52 | 0.678 |

**Table 18. Pharmacokinetic Parameters in Sprague-Dawley Rats by Intravenous Administration**

| **Example** | **Final State of the Formulation** | **Dose (mg/kg)** | **AUC (h*ng/ml)** | **T1/2 (h)** | **Cl (mL/min/Kg)** | **Vd (L/Kg)** |
|---|---|---|---|---|---|---|
| **7A** | solution | 1 | 317 | 0.84 | 53.17 | 3.3 |
| **49A** | solution | 2 | 904 | 1.04 | 38.17 | 2.2 |
| **55A** | solution | 3 | 3646 | 2.56 | 13.6 | 2.05 |

**Table 19. Pharmacokinetic Parameters in Sprague-Dawley Rats by Oral Administration**

| **Example** | **Final State of the Formulation** | **Dose (mg/kg)** | **Cmax (ng/ml)** | **T1/2 (h)** | **AUC (h*ng/ml)** | **F(%)** |
|---|---|---|---|---|---|---|
| **7A** | solution | 10 | 31.3 | 4.91 | 258 | 8.1 |
| **49A** | solution | 10 | 74.2 | 3.14 | 418 | 9.25 |
| **55A** | solution | 5 | 409 | 2.67 | 2954 | 48.6 |

**Table 20. Pharmacokinetic Parameters in Mice by Intravenous Administration**

| **Example** | **Final State of the Formulation** | **Dose (mg/kg)** | **AUC (h*ng/ml)** | **T1/2 (h)** | **Cl (mL/min/Kg)** | **Vd (L/Kg)** |
|---|---|---|---|---|---|---|
| **55A** | solution | 3 | 7599 | 2.57 | 6.57 | 0.819 |

**Table 21. Pharmacokinetic Parameters in Mice by Oral Administration**

| **Example** | **Final State of the Formulation** | **Dose (mg/kg)** | **Cmax (ng/ml)** | **T1/2 (h)** | **AUC (h*ng/ml)** | **F(%)** |
|---|---|---|---|---|---|---|
| **55A** | solution | 5 | 2300 | 2.38 | 7494 | 59.2 |

### Mice Colon Pharmacokinetic Studies:

Pharmacokinetic studies were investigated using female Balb/c mice to determine plasma and colon exposure of tested compounds. Compounds were administrated via oral gavage in a formulation of 0.5% Methocel (A4M) + 0.1% Tween80 in water to Balb/c mic. Animals were anesthetized at the designated time points, plasma, colon tissue and colonic contents samples were collected. Tissue samples were homogenized using MeOH : PBS(1:1). All samples were analyzed by LC-MS/MS.

Exemplary results are summarized in **Table 22**

**Table 22**

| Mice Colon Pharmacokinetic Parameters | | | | | | | |
|---|---|---|---|---|---|---|---|
| **Example** | **Dose (mg/kg)** | **Plasma T_{1/2} (h)** | **Plasma Tₘₐₓ (h)** | **Plasma Cₘₐₓ (ng/mL)** | **Plasma AUC₀₋₂₄ (ng*h/mL)** | **Colon AUC₀₋₂₄ (ng*h/mL)** | **Colon content AUC₀₋₂₄ (ng*h/mL)** |
| **7A** | 10 | 1.75 | 0.25 | 426 | 599 | 45772 | 1431730 |
| **49A** | 10 | 1.03 | 0.25 | 1344 | 1219 | 21308 | 1590537 |

### SCID Mice Colitis by Anti-CD40-driven Pharmacodynamics study

This study aimed to obtain the pharmacodynamics of the test article in colitis model by Anti-CD40-driven after oral administration. C.B17-SCID mice obtained from the Zhejiang Vital River Laboratory Animal Technology Co., Ltd. Starting on Day -1 and continuing daily through Day 4, mice (n = 10 per group) were dosed with 0 (vehicle control), 15, 45, or 100 mg/kg PO BID compound 7A in vehicle containing 5% DMSO, 65% PEG400, and 30% DDH₂O (v/v/v). In addition, on Day 0, 30 minutes after administration, colitis was induced with a single intraperitoneal injection of 200 µL of anti-CD40 mAb (BE0016-2, BioXcell) in DPBS, the Sham group mice were intraperitoneally injected with 200 µL of DPBS.

Each mouse was scored daily for pathological features, including stool consistency, presence of blood stool, and body weight loss. Individual scores were combined to generate the Disease Activity Index (DAI) which was calculated daily for each mouse, the maximum score was 12. On Day 5, all animals were euthanized, colon was collected for measuring colon index (weight, length, and density).

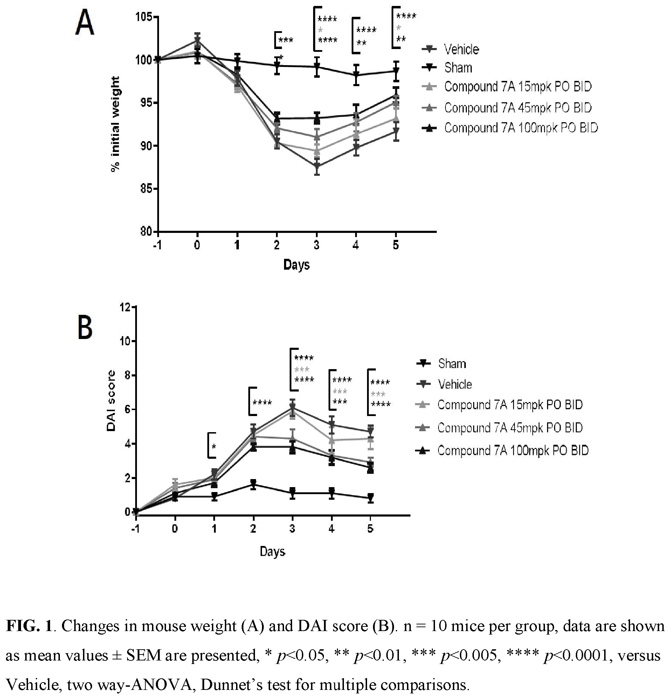

In this study, the impact of compound **7A** on colon inflammation was investigated *in vivo.* Our results indicated the significant protective effect of compound **7A** on Anti-CD40-induced colitis, as evidenced by a reduction in body weight loss (**FIG. 1****. A**) and DAI score (**FIG. 1****. B**), improved the colon length and weight index (**Table 23**) in immunological colitis in mice.

**Table 23. Colon Index**

| **Group** | **Colon weight (mg)** | **Colon length (cm)** | **Colon density (mg/cm)** |
|---|---|---|---|
| Sham | 193.20±5.68^{****} | 8.14±0.10^{****} | 23.79±0.80^{****} |
| Vehicle | 282.90±5.19 | 5.44±0.09 | 52.14±1.15 |
| Compound 7A 15mpk PO BID | 260.20±10.58 | 5.61±0.20 | 46.99±2.71 |
| Compound 7A 45mpk PO BID | 234.20±9.04^{****} | 6.34±0.13^{****} | 37.00±1.36^{****} |
| Compound 7A 100mpk PO BID | 208.40±5.53^{****} | 7.15±0.15^{****} | 29.23±0.91^{****} |

| | | | |
|---|---|---|---|
| n = 10 mice per group, data are shown as mean values ± SEM are presented, * *p*<0.05, ** *p*<0.01, *** *p*<0.005, *****p*<0.0001, versus Vehicle, two way-ANOVA, Dunnet's test for multiple comparisons. | | | |

### SCID mice colitis by T cell transfer pharmacodynamics study

This study aimed to obtain the pharmacodynamics of the test article in T cell transfer colitis model after oral administration. For induction of colitis, naive CD4⁺ T cells (CD4⁺CD44^{low}CD62L^{high}) were adoptively transferred from MHC-compatible Balb/c mice to C.B17-SCID recipients. Balb/c splenocytes were positively selected for CD4⁺ T cells using the Mouse Naïve CD4⁺ T Cell Isolation Kit (Cat #19765, STEMCELL), 1.5×10⁶ cells in a volume of 100 µL PBS were transferred to each recipient by i.v. injection on Day 0. C.B17-SCID mice obtained from the Beijing Vital River Laboratory Animal Technology Co., Ltd. Starting on Day 0 and continuing daily through Day 32, mice (n = 8 per group) were dosed with 0 (vehicle control), 45 mg/kg PO BID compound **7A** in a vehicle containing 5% DMSO, 65% PEG400, and 30% DDH₂O (v/v/v).

Each mouse was scored three times weekly for pathological features, including stool consistency, and body weight loss. Individual scores were combined to generate the DAI, the maximum score was 6. At the termination of the study, the colon was collected for measuring colon index (weight, length, and density), emptied of fecal contents opened longitudinally along the mesenteric border, and formed a Swiss-Roll from the proximal to the distal end, processed to paraffin-embedded blocks to generate 5-µm-thick sections for hematoxylin and eosin (H&E) staining. Slices were evaluated in a blinded manner and histological scores were assessed based on the following parameters: inflammation, epithelial defects, crypt atrophy, dysplasia, and the area affected by dysplasia.

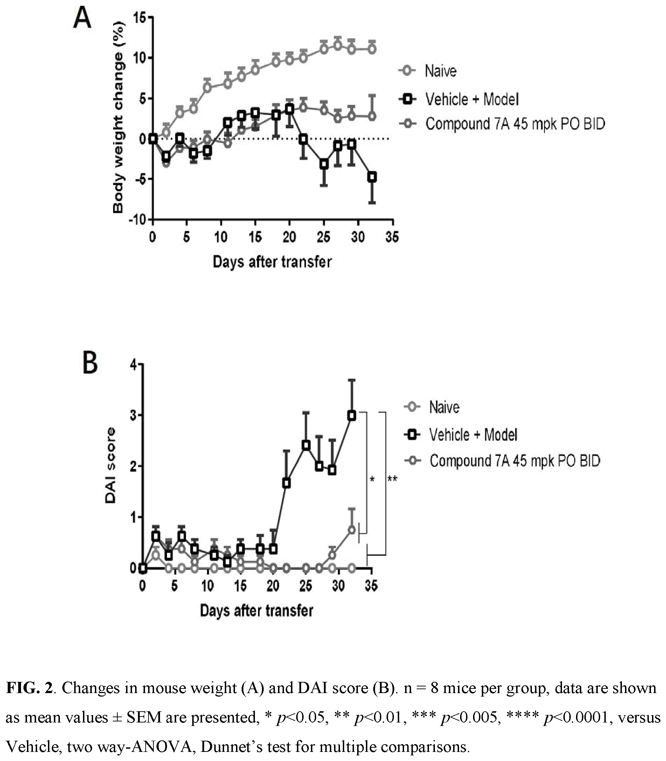

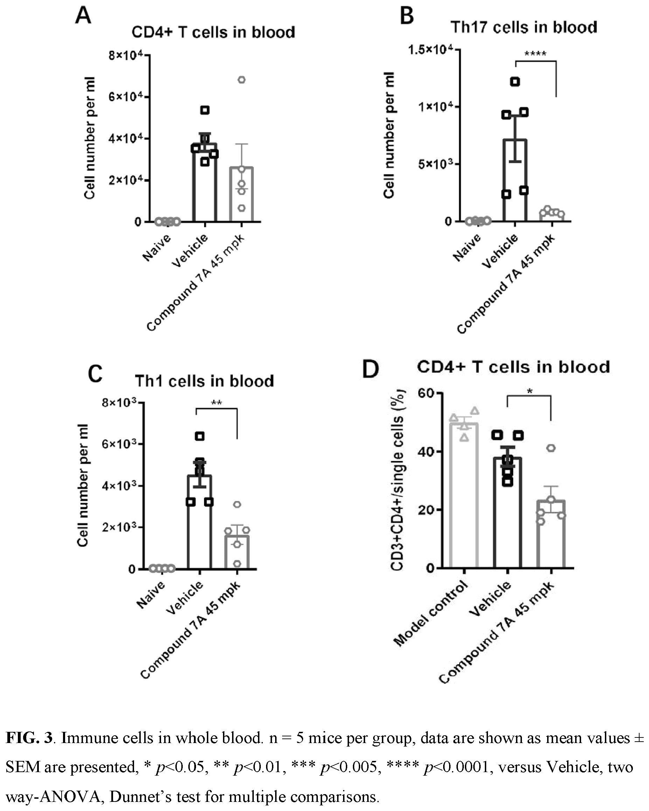

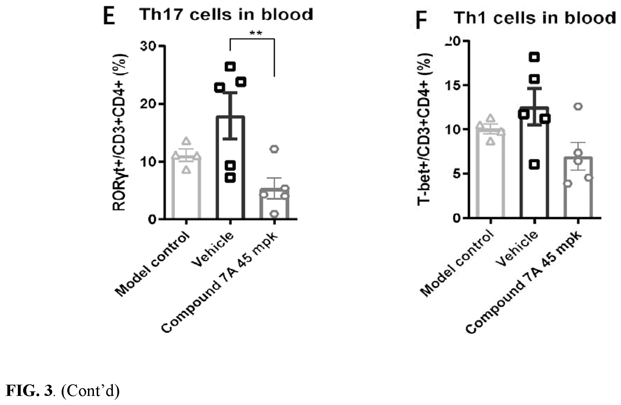

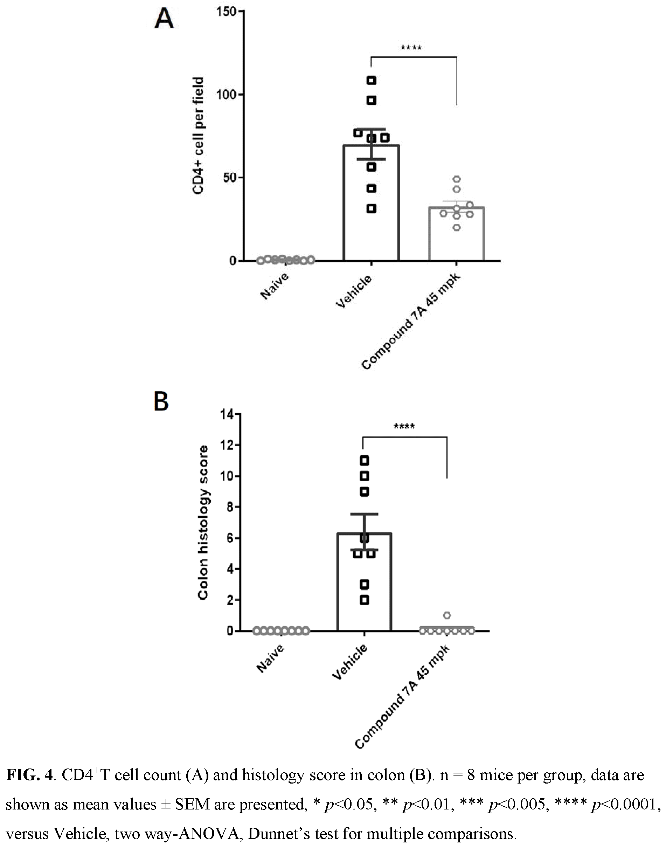

In the adoptive naive CD4⁺T cells transfer colitis model, the model control group showed more severe disease progression, including more weight loss, higher DAI scores, and colon weight/colon length ratio, more severe colonic histopathological damage, inflammatory cell infiltration, and splenomegaly. Compound **7A** administered orally twice daily alleviated colitis symptoms, specifically by inhibiting disease-related weight loss (**FIG. 2****. A**), DAI scores (**FIG. 2****. B**), colon weight, colon weight/colon length ratio **(Table 24),** and colonic histopathological changes (**FIG. 4****. B**). In addition, compound 7A significantly reduced the absolute value of Th1 and Th17 cells (**FIG. 3****. B-C**), and the percentage value of CD4⁺T cells and Th17 cells in whole blood (**FIG. 3****. D-E**) and CD4⁺T cells in colonic tissue (**FIG. 4****. A**).

**Table 24. Colon and Spleen Index**

| **Group** | **Colon weight (mg)** | **Colon length (cm)** | **Colon density (mg/cm)** | **Spleen index (mg/g)** |
|---|---|---|---|---|
| Naive | 166.21±10.03^{****} | 6.56±0.21 | 25.64±2.00^{****} | 1.63±0.12^{****} |
| Vehicle | 321.40±29.43 | 6.76±0.35 | 48.50±5.42 | 3.67±0.45 |
| Compound 7A 45mpk PO BID | 218.38±10.13^{**} | 7.94±0.32^{*} | 27.66±1.24^{****} | 3.08±0.36 |

| | | | | |
|---|---|---|---|---|
| n = 8 mice per group, data are shown as mean values ± SEM are presented, * *p*<0.05, ** *p*<0.01, *** *p*<0.005, *****p*<0.0001, versus Vehicle, two way-ANOVA, Dunnet's test for multiple comparisons. | | | | |

Applicant's disclosure is described herein in preferred embodiments with reference to the Figures, in which like numbers represent the same or similar elements. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

The described features, structures, or characteristics of Applicant's disclosure may be combined in any suitable manner in one or more embodiments. In the description, herein, numerous specific details are recited to provide a thorough understanding of embodiments of the invention. One skilled in the relevant art will recognize, however, that Applicant's composition and/or method may be practiced without one or more of the specific details, or with other methods, components, materials, and so forth. In other instances, well-known structures, materials, or operations are not shown or described in detail to avoid obscuring aspects of the disclosure.

Unless defined otherwise, all technical and scientific terms used herein have the same meaning as commonly understood by one of ordinary skill in the art. Although any methods and materials similar or equivalent to those described herein can also be used in the practice or testing of the present disclosure, the preferred methods and materials are now described. Methods recited herein may be carried out in any order that is logically possible, in addition to a particular order disclosed.

### Incorporation by Reference

References and citations to other documents, such as patents, patent applications, patent publications, journals, books, papers, web contents, have been made in this disclosure. All such documents are hereby incorporated herein by reference in their entirety for all purposes. Any material, or portion thereof, that is said to be incorporated by reference herein, but which conflicts with existing definitions, statements, or other disclosure material explicitly set forth herein is only incorporated to the extent that no conflict arises between that incorporated material and the present disclosure material. In the event of a conflict, the conflict is to be resolved in favor of the present disclosure as the preferred disclosure.

### Equivalents

The representative examples are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples and the references to the scientific and patent literature included herein. The examples contain important additional information, exemplification and guidance that can be adapted to the practice of this invention in its various embodiments and equivalents thereof.

Also provided are the following embodiments:
1. A compound having the structural formula (I): 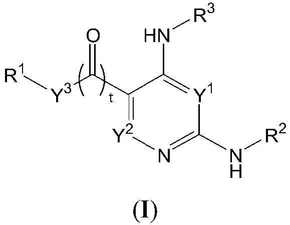 or a pharmaceutically acceptable form or an isotope derivative thereof,
   wherein
   Y¹ is CH, CF or N;
   Y² is CH or N;
   Y³ is NR, O, CH₂, CD₂, CF₂ or O-NH;
   *t* is 0 or 1;
   R¹ is H, F, CD₃, or C₁₋₃ alkyl, provided that R¹ is not F when Y³ is N, O or O-NH;
   R² is
      R^{2'}, wherein R^{2'} is C₁-C₆ alkyl, C₃₋₆ cycloalkyl, C₅₋₇ spirocycloalkyl, or C₃₋₆ heterocycloalkyl, each substituted with 0-2 R^{2a}, wherein R^{2a} is selected from the group consisting of halogen, CN, OR, NRR', alkyl, cycloalkyl and heterocycle;
      an aryl or heteroaryl group, each substituted with 0-2 R^{2a};
      (C=O)R^{2b}; or
      (C=O)NHR^{2b};
   R³ is 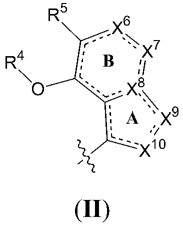 wherein
      X⁶ is CR⁶ or N;
      X⁷ is CR⁷ or N;
      X⁸ is C or N;
      X⁹ is CR⁹, O, S, N or NR⁹;
      X¹⁰ is CR¹⁰, O, S, N or NR¹⁰; and wherein each of Ring A and Ring B is independently an aryl or heteroaryl group;
      R²⁶ is C₁₋₆ alkyl, C₃₋₆ cycloalkyl, C₅₋₇ spirocycloalkyl, aryl or heteroaryl, each substituted with 0-4 R^{2c};
      R^{2c} at each occurrence is independently halo, CN, OR, NRR', OCF₃, CF₃, C₁₋₆ alkyl, C₁₋₆ haloalkyl, C₂₋₆ alkenyl, C₂₋₆ alkynyl, wherein said alkyl, haloalkyl, alkenyl, alkynyl, R and R' are substituted with 0-3 R^{2a}; and
      R⁴ is C₁₋₃ alkyl, substituted with 0-5 R^{4a}, wherein R^{4a} is selected from D, F and Cl;
      R⁵ is H, CN, halo, OCH₃, C(=O)OR, NHC(=O)R, NRR', NO₂, C₁₋₆ alkyl, C₃₋₆ cycloalkyl or heterocyclic, wherein said alkyl, cycloalkyl or heterocyclic is substituted with 0-3 R^{5a}, wherein each R^{5a} is independently selected from OH, D, F, Cl, CN, CH₂F, CHF₂, CF₃, OCH₃, OCD₃, OCF₃ and OC(=O)CH₃;
      each of R⁶, R⁷, R⁹ and R¹⁰ is independently selected from H, F, Cl, CN, CD₃, CH₂CF₃, CF₃, OR, NRR', C₁₋₃ alkyl and C₃₋₅ cycloalkyl, wherein said alkyl, cycloalkyl, R and R' are substituted with 0-2 R^{2a}; and
      each of R and R' is independently H or a C₁-C₆ alkyl or acyl, or R and R', together with the nitrogen atom to which they are bonded, form a 4- to 7-membered ring comprising 0-2 heteroatoms selected from O, NR, S and SO₂.
2. The compound of embodiment [1], wherein t is 1, having the structural formula: 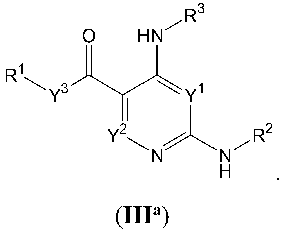
3. The compound of embodiment [1], wherein t is 0, having the structural formula: 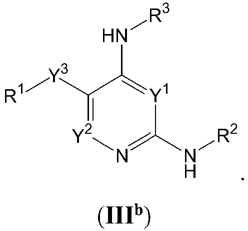
4. The compound of any one of embodiments [1]-[3], wherein Ring A is a heteroaryl group.
5. The compound of any one of embodiments [1]-[3], wherein X⁶ is CH and X⁷ is CH, with R³ having the structure: 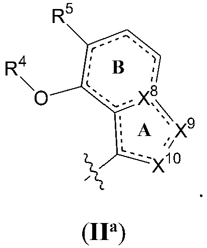
6. The compound of any one of embodiments [1]-[3], wherein X⁷ is CH and X⁸ is C, with R³ having the structure: 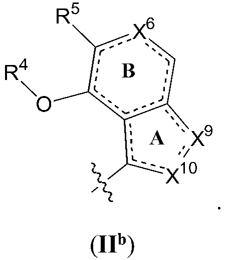
7. The compound of any one of embodiments [1]-[3], wherein X⁶ is CH and X⁸ is C, with R³ having the structure: 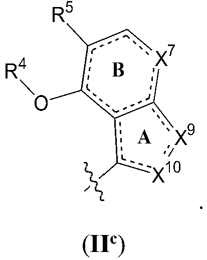
8. The compound of any one of embodiments [1]-[3], wherein X⁷ is CH and X¹⁰ is CH, with R³ having the structure: 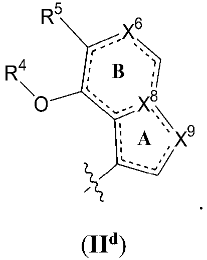
9. The compound of any one of embodiments [1]-[8], wherein R³ is selected from: 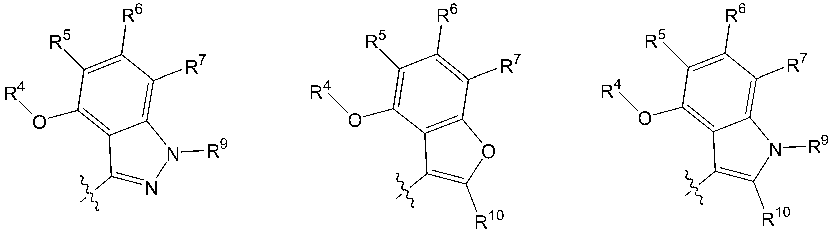 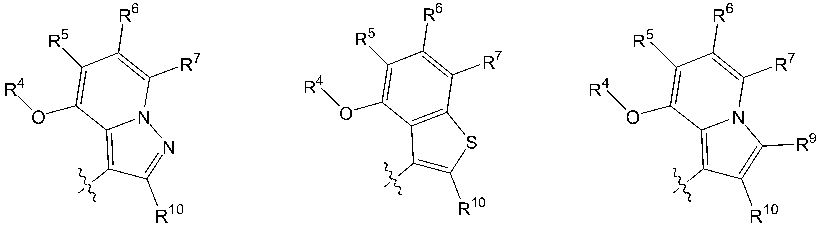 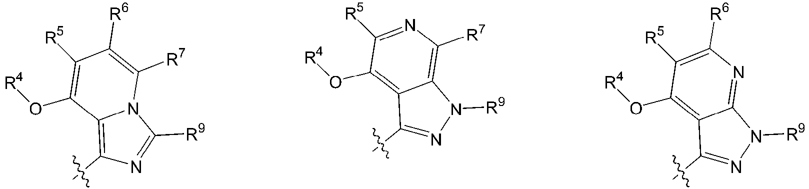 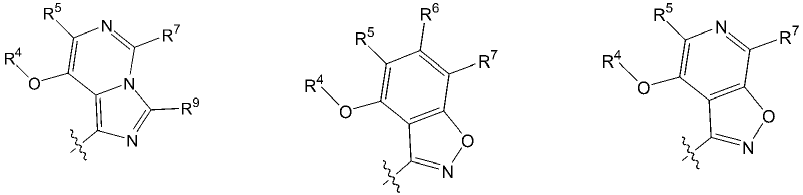 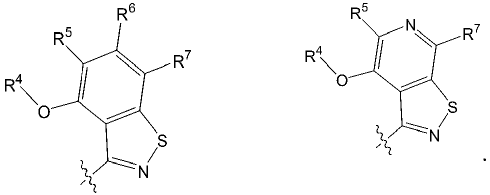
10. The compound of embodiment [9], wherein R⁴ is CH₃.
11. The compound of embodiment [9], wherein R⁴ is CD₃.
12. The compound of embodiment [9], wherein R³ is: 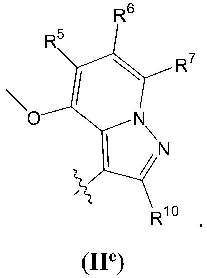
13. The compound of any one of embodiments [1]-[12], wherein R¹⁰ if present is H.
14. The compound of embodiment [9], wherein R³ is: 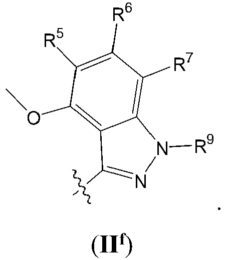
15. The compound of any one of embodiments [1]-[14], wherein R⁹ if present is C₁₋₃ alkyl or cyclopropyl, each optionally substituted with C₁₋₃ alkoxy, CF₃ or NRR'.
16. The compound of embodiment [15], wherein R⁹ is C₁₋₃ alkyl.
17. The compound of embodiment [16], wherein R⁹ is CH₃.
18. The compound of embodiment [16], wherein R⁹ is CD₃.
19. The compound of any one of embodiments [1]-[18], wherein R⁵ is C₁₋₄ alkyl, substituted with an OH.
20. The compound of any one of embodiments [1]-[18], wherein R⁵ is: 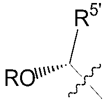 wherein
   R^{5'} is a C₁₋₃ alkyl or cyclopropyl, substituted with 0-5 F's; and
   R is H, C₁₋₃ alkyl or acyl.
21. The compound of embodiment [20], wherein R is CH₃.
22. The compound of embodiment [20], wherein R is CD₃.
23. The compound of any one of embodiments [1]-[18], wherein R⁵ is: 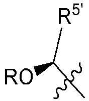 wherein
   R^{5'} is a C₁₋₃ alkyl or cyclopropyl, substituted with 0-5 F's; and
   R is H, C₁₋₃ alkyl or acyl.
24. The compound of embodiment [23], wherein R is CH₃.
25. The compound of embodiment [23], wherein R is CD₃.
26. The compound of embodiment [20] or [23], wherein R is C(=O)CH₃ or C(=O)CD₃.
27. The compound of any one of embodiments [20]-[26], wherein R^{5'} is CF₃.
28. The compound of any one of embodiments [20]-[26], wherein R^{5'} is CHF₂.
29. The compound of any one of embodiments [1]-[28], wherein Y³ is NH.
30. The compound of embodiment [29], wherein Y¹ is CH and Y² is CH: 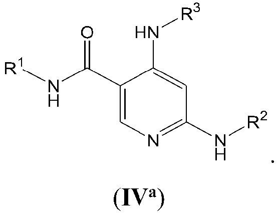
31. The compound of embodiment [29], wherein Y¹ is CH and Y² is N: 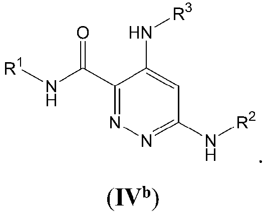
32. The compound of embodiment [29], wherein Y¹ is N and Y² is CH: 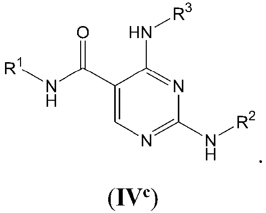
33. The compound of embodiment [29], wherein Y¹ is N and Y² is N: 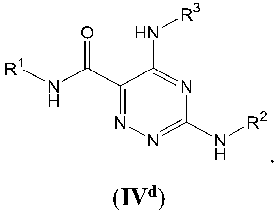
34. The compound of any one of embodiments [1]-[28], wherein Y³ is O.
35. The compound of embodiment [34], wherein Y¹ is CH and Y² is CH: 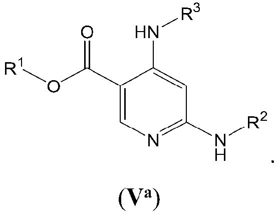
36. The compound of embodiment [34], wherein Y¹ is CH and Y² is N: 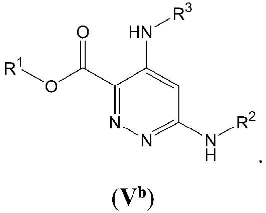
37. The compound of embodiment [34], wherein Y¹ is N and Y² is CH: 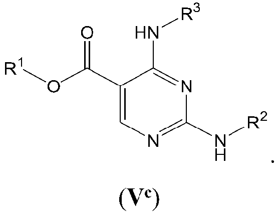
38. The compound of embodiment [34], wherein Y¹ is N and Y² is N: 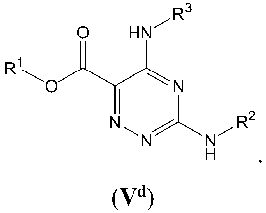
39. The compound of any one of embodiments [1]-[28], wherein Y³ is CH₂.
40. The compound of embodiment [39], wherein Y¹ is CH and Y² is CH: 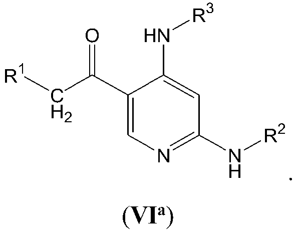
41. The compound of embodiment [39], wherein Y¹ is CH and Y² is N: 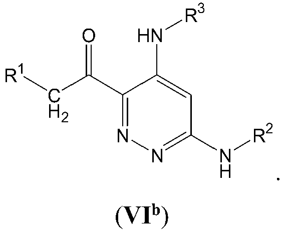
42. The compound of embodiment [39], wherein Y¹ is N and Y² is CH: 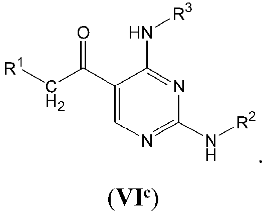
43. The compound of embodiment [39], wherein Y¹ is N and Y² is N: 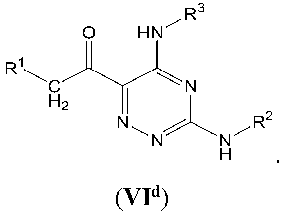
44. The compound of any one of embodiments [1]-[28], wherein Y³ is CD₂.
45. The compound of any one of embodiments [1]-[28], wherein Y³ is CF₂ and t is 0.
46. The compound of embodiment [45], wherein Y¹ is CH and Y² is CH: 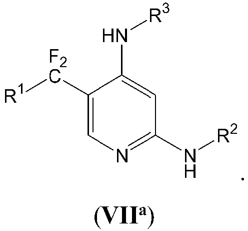
47. The compound of embodiment [45], wherein Y¹ is CH and Y² is N: 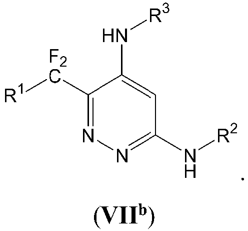
48. The compound of embodiment [45], wherein Y¹ is N and Y² is CH: 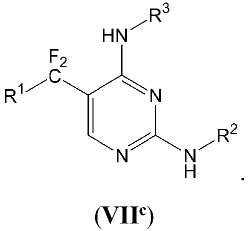
49. The compound of embodiment [45], wherein Y¹ is N and Y² is N: 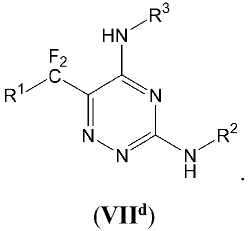
50. The compound of any one of embodiments [1]-[49], wherein R⁶ and R⁷, if present, is H.
51. The compound of any one of embodiments [1]-[50], wherein R² is R^{2'}.
52. The compound of any one of embodiments [1]-[50], wherein R² is (C=O)R^{2b}.
53. The compound of embodiment [52], wherein R^{2b} is selected from C₁-C₆ alkyl, substituted with 0-3 R^{2c}.
54. The compound of embodiment [52], wherein R^{2b} is C₃₋₆ cycloalkyl, substituted with 0-3 R^{2c}.
55. The compound of embodiment [54], wherein R^{2b} is cyclopropyl.
56. The compound of embodiment [54], wherein R^{2b} is cyclopropyl, substituted with F.
57. The compound of embodiment [52], wherein R^{2b} is C₅₋₇ spirocycloalkyl, substituted with 0-3 R^{2c}.
58. The compound of embodiment [57], wherein R^{2b} is C₅ spiro[2.2]pentyl.
59. The compound of any one of embodiments [1]-[49], wherein R² is (C=O)NHR^{2b}.
60. The compound of any one of embodiments [1]-[49], wherein R² is pyridinyl substituted with 0-2 R^{2c}.
61. The compound of any one of embodiments [1]-[49], wherein R² is phenyl substituted with 0-2 R^{2c}.
62. The compound of any one of embodiments [1]-[49], wherein R² is pyrazolyl substituted with 0-2 R^{2c}.
63. The compound of any one of embodiments [1]-[49], wherein R² is pyrimidyl substituted with 0-2 R^{2c}.
64. The compound of any one of embodiments [1]-[63], wherein R¹ is CH₃.
65. The compound of any one of embodiments [1]-[63], wherein R¹ is CD₃.
66. The compound of embodiment [1], having the structural formula: 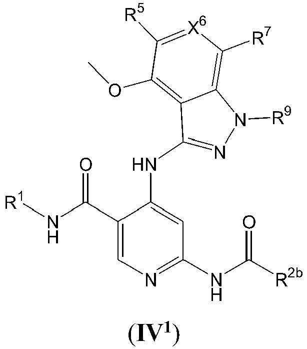 wherein X⁶ is N or CH.
67. The compound of embodiment [1], having a structural formula selected from: 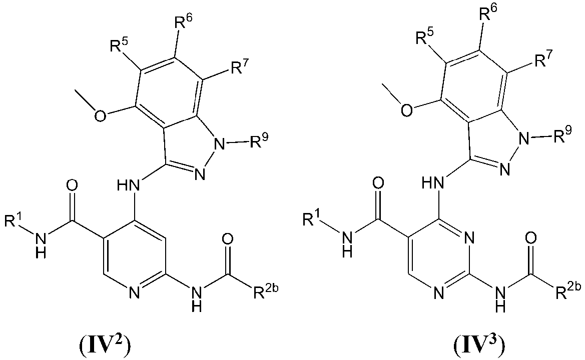 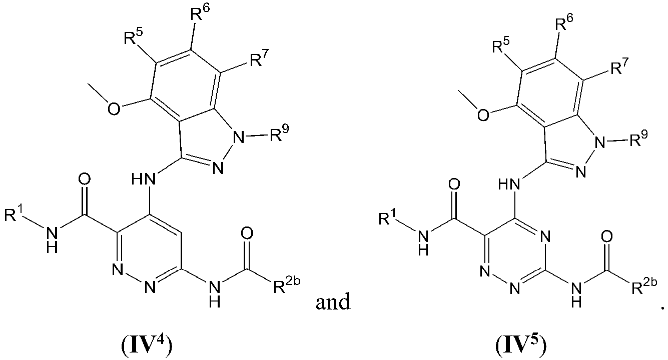
68. The compound of embodiment [1], having a structural formula selected from: 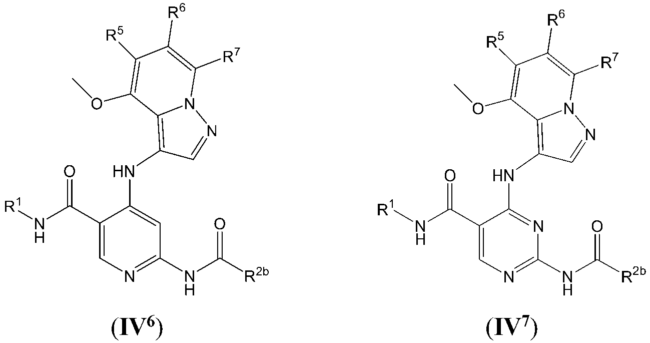 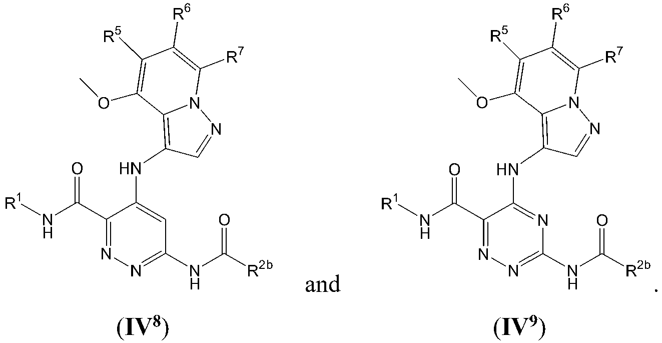
69. The compound of embodiment [1], having a structural formula selected from: 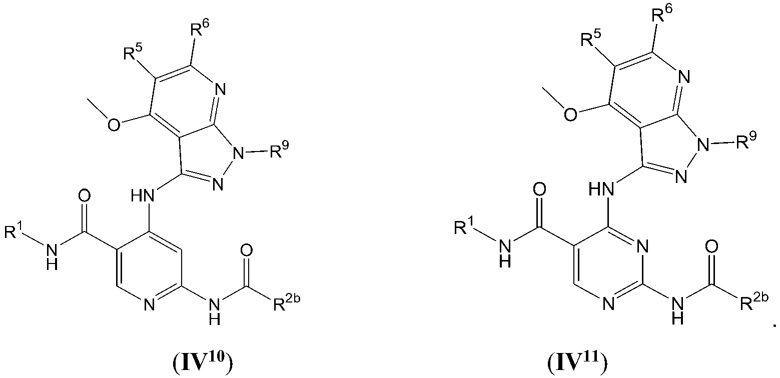 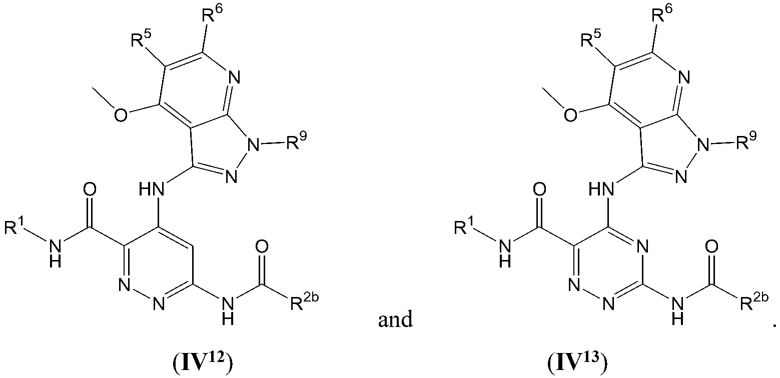
70. The compound of embodiment [1], having a structural formula selected from: 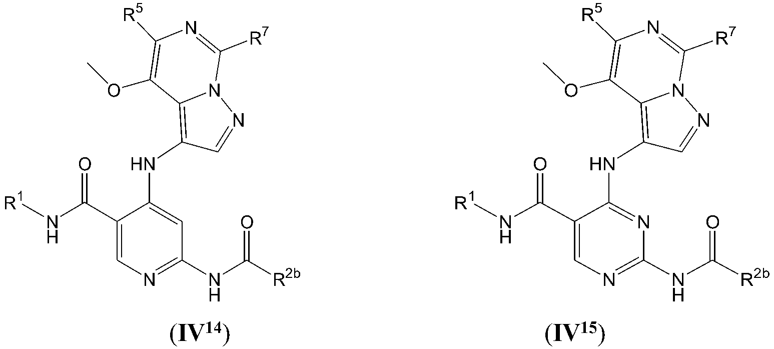 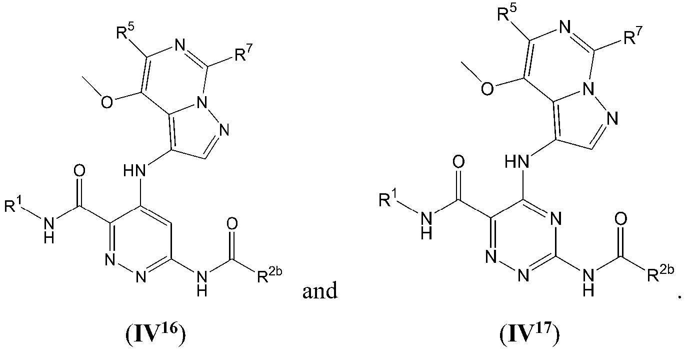
71. The compound of embodiment [1], having a structural formula selected from: 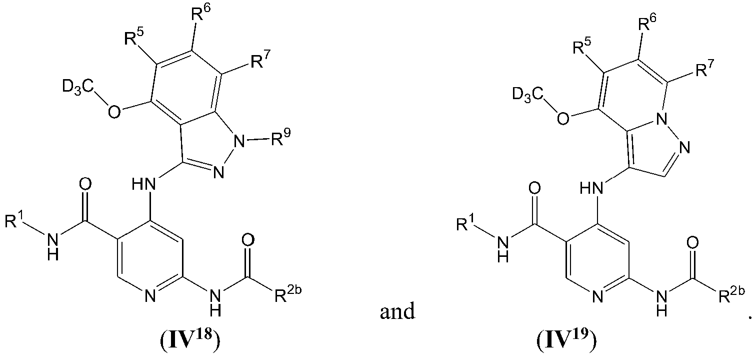
72. The compound of embodiment [1], having the structural formula: 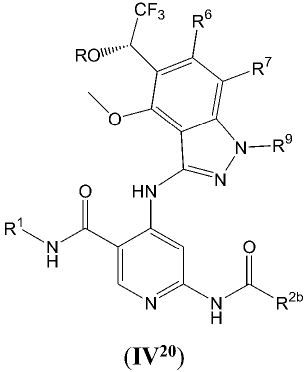 wherein R is H, CD₃, C₁₋₃ alkyl or acyl.
73. The compound of embodiment [1], having the structural formula: 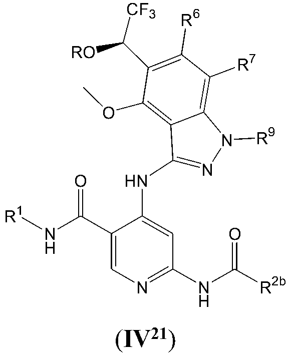 wherein R is H, CD₃, C₁₋₃ alkyl or acyl.
74. The compound of embodiment [1], having the structural formula: 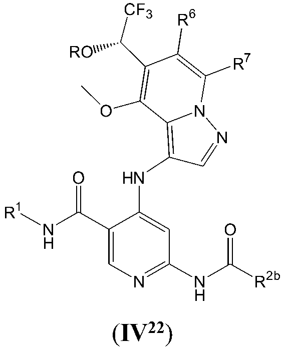 wherein R is H, CD₃, C₁₋₃ alkyl or acyl.
75. The compound of embodiment [1], having the structural formula: 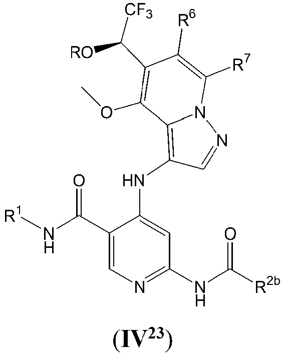 wherein R is H, CD₃, C₁₋₃ alkyl or acyl.
76. The compound of embodiment [1], having the structural formula: 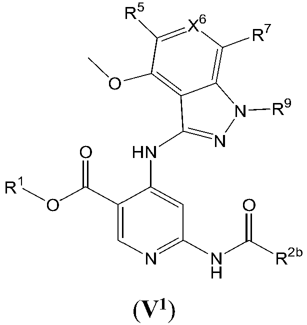 wherein X⁶ is N or CH.
77. The compound of embodiment [1], having a structural formula selected from: 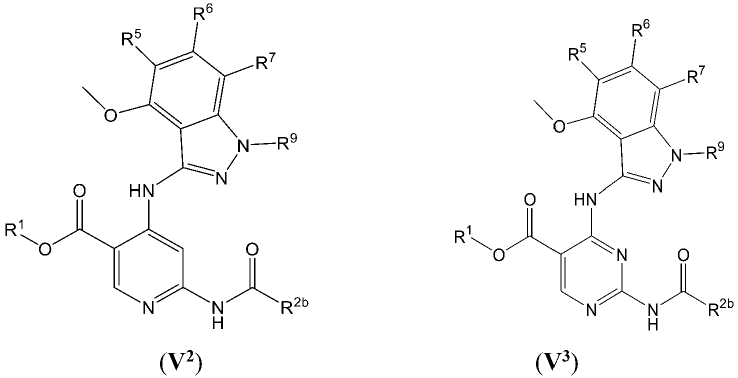 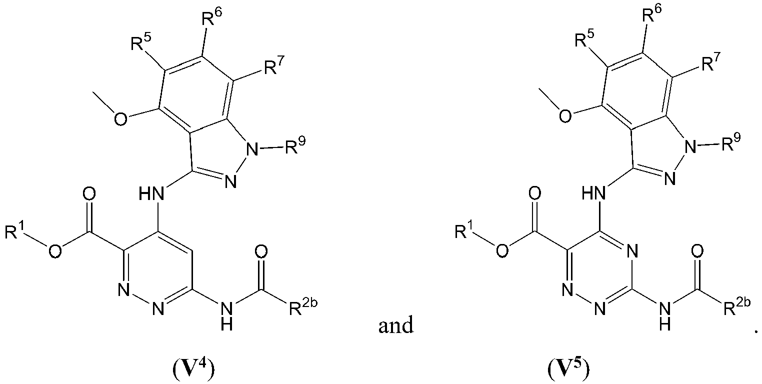
78. The compound of embodiment [1], having a structural formula selected from: 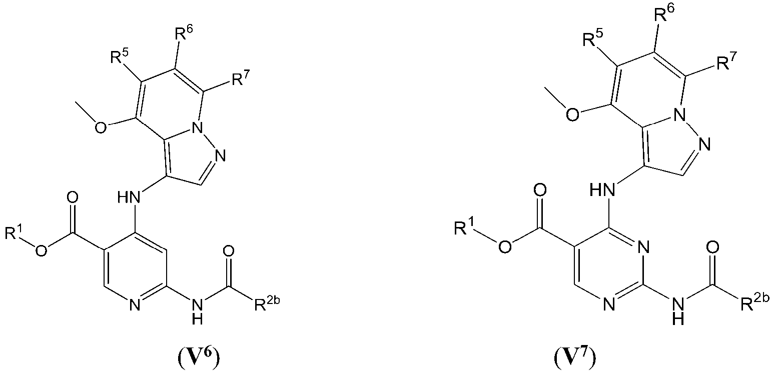 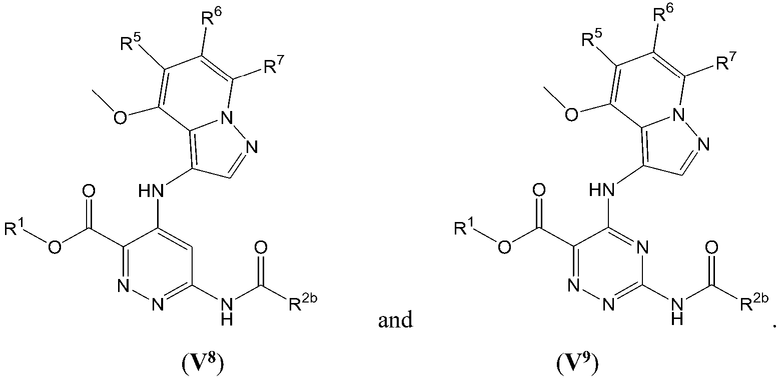
79. The compound of embodiment [1], having a structural formula selected from: 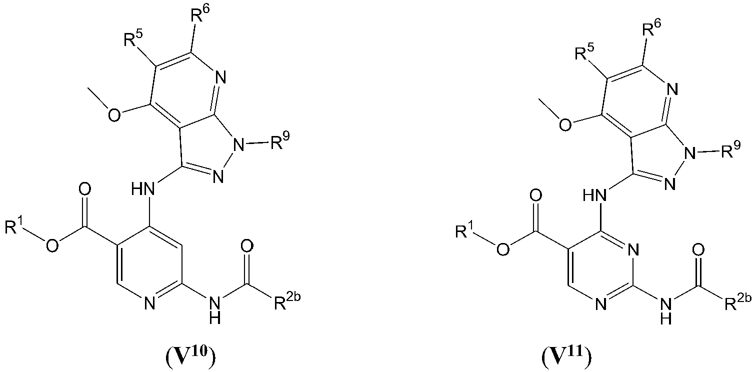 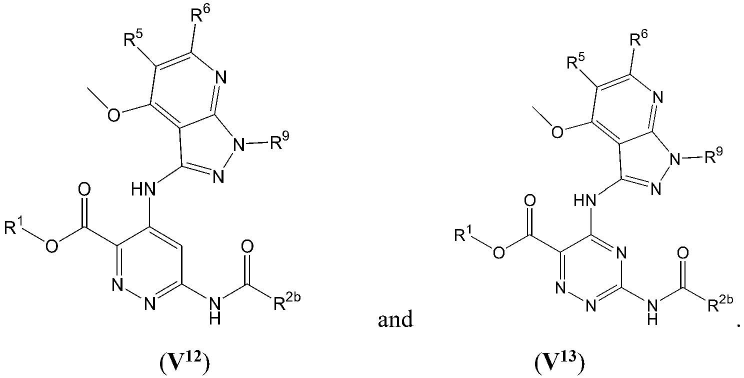
80. The compound of embodiment [1], having a structural formula selected from: 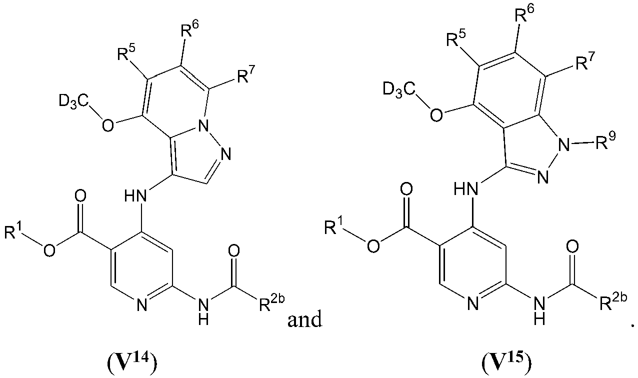
81. The compound of embodiment [1], having a structural formula selected from: 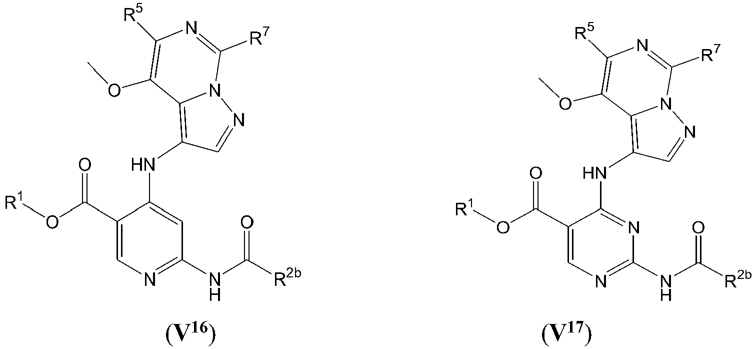 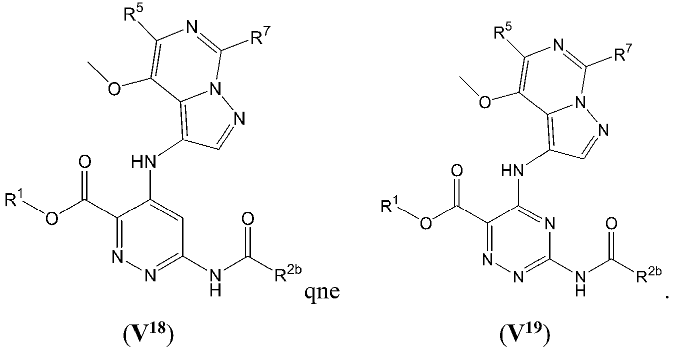
82. The compound of embodiment [1], having the structural formula: 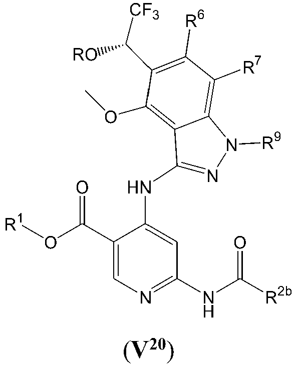 wherein R is H, CD₃, C₁₋₃ alkyl or acyl.
83. The compound of embodiment [1], having the structural formula: 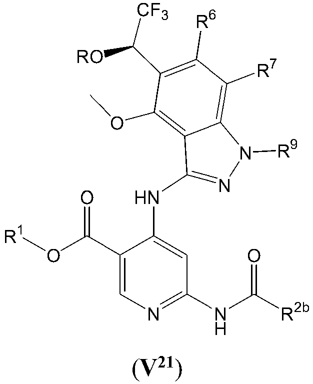 wherein R is H, CD₃, C₁₋₃ alkyl or acyl.
84. The compound of embodiment [1], having the structural formula: 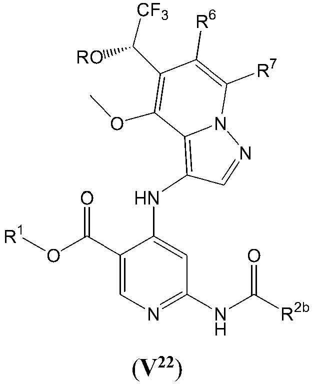 wherein R is H, CD₃, C₁₋₃ alkyl or acyl.
85. The compound of embodiment [1], having the structural formula: 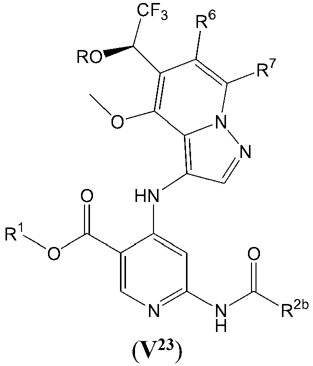 wherein R is H, CD₃, C₁₋₃ alkyl or acyl.
86. The compound of embodiment [1], having the structural formula: 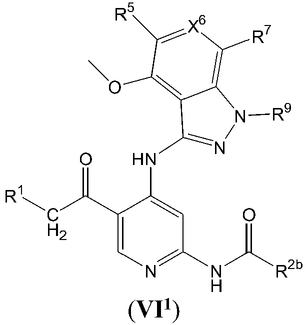 wherein X⁶ is N or CH.
87. The compound of embodiment [1], having a structural formula selected from: 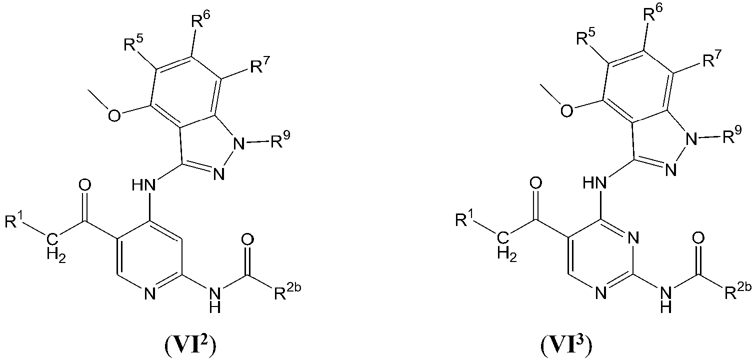 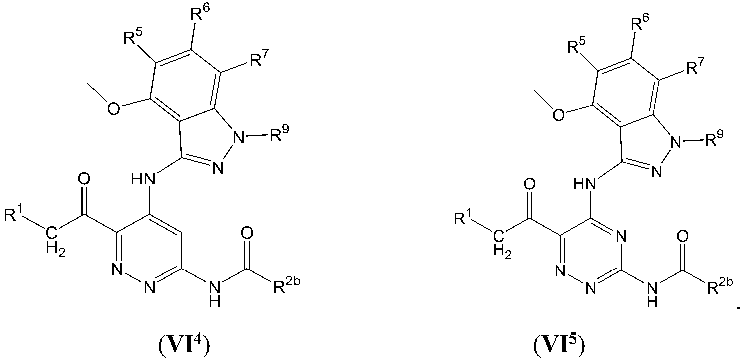
88. The compound of embodiment [1], having a structural formula selected from: 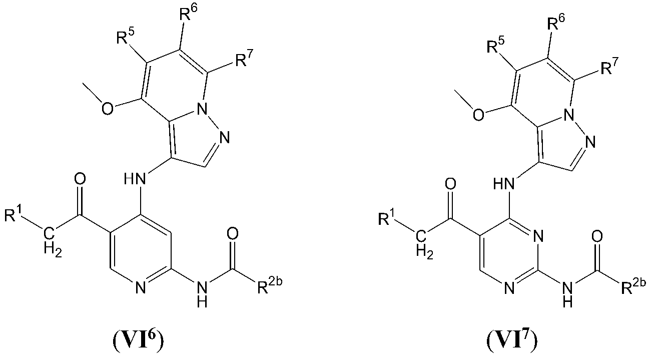 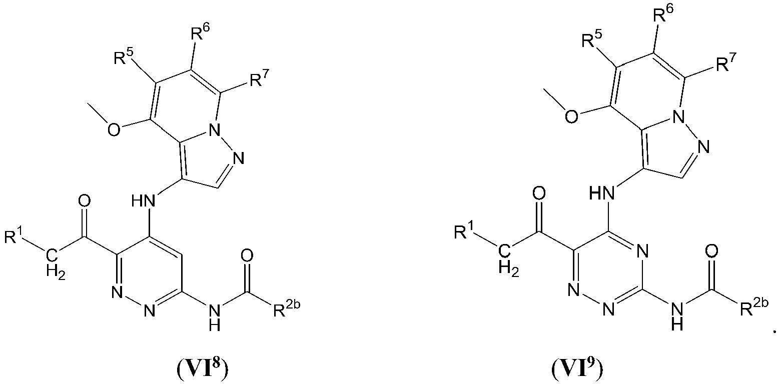
89. The compound of embodiment [1], having a structural formula selected from: 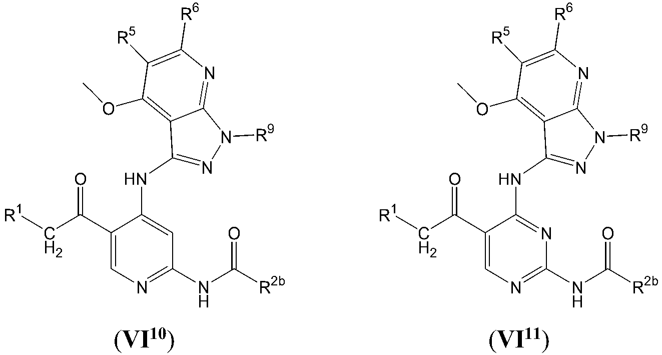 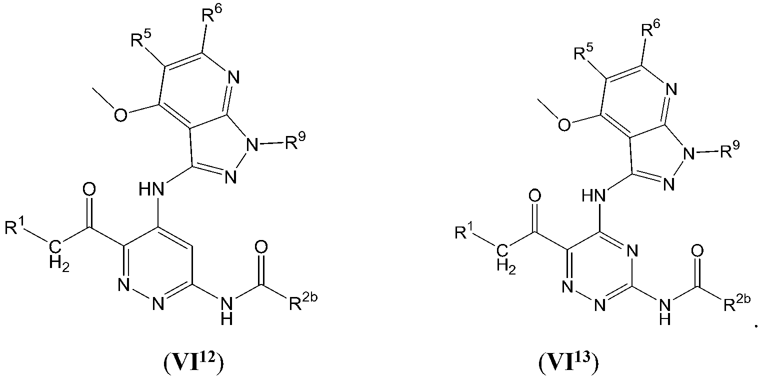
90. The compound of embodiment [1], having a structural formula selected from: 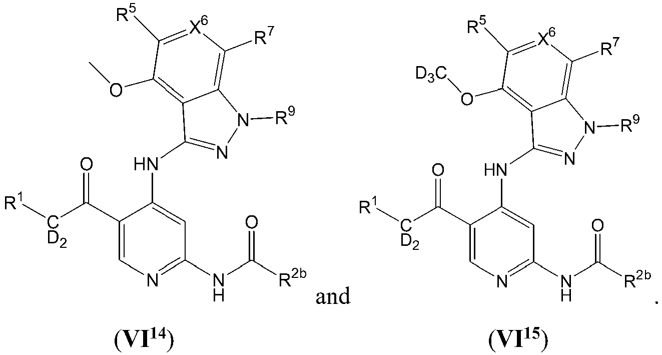
91. The compound of embodiment [1], having a structural formula selected from: 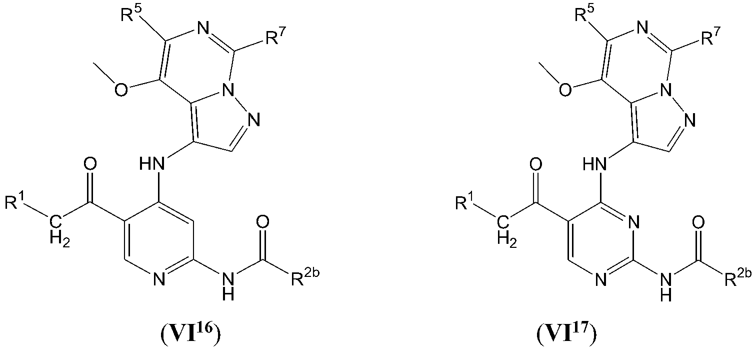 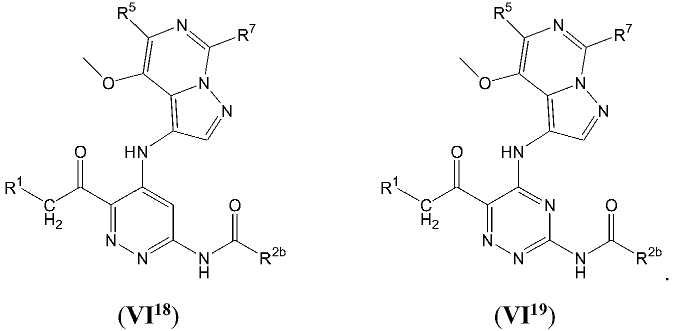
92. The compound of embodiment [1], having the structural formula: 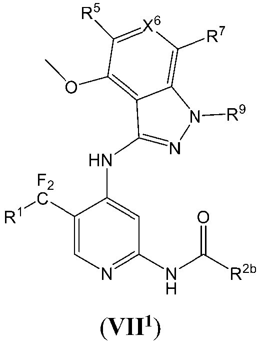 wherein X⁶ is N or CH.
93. The compound of embodiment [1], having a structural formula: 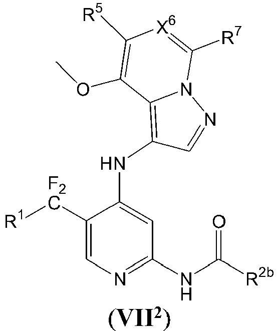 wherein X⁶ is N or CH.
94. The compound of any one of embodiments [66]-[93], wherein R⁶ and R⁷, if present, is H.
95. The compound of any one of embodiments [1]-[94], wherein R⁹ if present is C₁₋₃ alkyl or cyclopropyl, each optionally substituted with C₁₋₃ alkoxy, CF₃ or NRR'.
96. The compound of embodiment [95], wherein R⁹ is C₁₋₃ alkyl or cyclopropyl.
97. The compound of embodiment [96], wherein R⁹ is CH₃.
98. The compound of embodiment [96], wherein R⁹ is CD₃.
99. The compound of any one of embodiments [66]-[98], wherein R^{2b} is C₁-C₆ alkyl, cyclopropyl or cyclobutyl, substituted with 0-2 R^{2c}.
100. The compound of embodiment [99], wherein R^{2b} is cyclopropyl.
101. The compound of any one of embodiments [66]-[100], wherein R⁵ is: 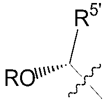 wherein
   R^{5'} is a C₁₋₃ alkyl or cyclopropyl, substituted with 0-5 F's; and
   R is H, C₁₋₃ alkyl or acyl.
102. The compound of embodiment [101], wherein R is H.
103. The compound of embodiment [101], wherein R is CH₃.
104. The compound of embodiment [101], wherein R is CD₃.
105. The compound of any one of embodiments [66]-[100], wherein R⁵ is: 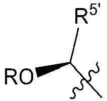 wherein
   R^{5'} is a C₁₋₃ alkyl or cyclopropyl, substituted with 0-5 F's; and
   R is H, C₁₋₃ alkyl or acyl.
106. The compound of embodiment [105], wherein R is H.
107. The compound of embodiment [105], wherein R is CH₃.
108. The compound of embodiment [105], wherein R is CD₃.
109. The compound of any one of embodiments [101]-[108], wherein R^{5'} is CF₃.
110. The compound of any one of embodiments [101]-[108], wherein R^{5'} is CHF₂.
111. The compound of any one of embodiments [101]-[108], wherein R^{5'} is a C₂₋₃ alkyl, substituted with 2-5 F's.
112. The compound of any one of embodiments [66]-[111], wherein R¹ is CH₃.
113. The compound of any one of embodiments [66]-[111], wherein R¹ is CD₃.
114. A compound having the structural formula (VIII): 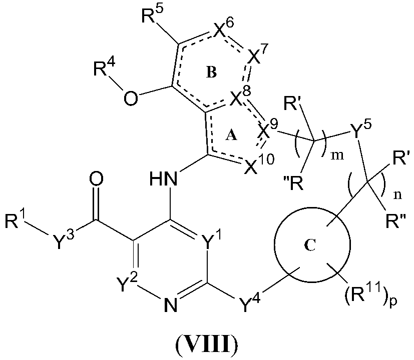 or a pharmaceutically acceptable form or an isotope derivative thereof,
   wherein
   X⁶ is CR⁶ or N;
   X⁷ is CR⁷ or N;
   X⁸ is C or N;
   X⁹ is CR⁹, O, S, N or NR⁹;
   X¹⁰ is CR¹⁰, O, S, N or NR¹⁰;
   Y¹ is CH, CF or N;
   Y² is CH or N;
   Y³ is NR, O, CH₂, CD₂, CF₂ or O-NH;
   Y⁴ is NR, CH₂ or CF₂;
   Y⁵ is NR, CH₂, O, S, SO or SO₂;
   *m* is 0, 1, 2 and 3;
   *n* is 0, 1, 2 and 3;
   *p is 0, 1, 2 and* 3;
   each of Ring A and Ring B is independently an aryl or heteroaryl group;
   Ring C is a 5- or 6-membered aryl or heteroaryl group;
   R¹ is H, F, CD₃, or C₁₋₃ alkyl, provided that R¹ is not F when Y³ is N, O or O-NH;
   R⁴ is a C₁₋₃ alkyl, substituted with 0-5 R^{4a}, wherein R^{4a} is selected from D, F and Cl;
   R⁵ is H, CN, halo, OCH₃, C(=O)OR, NHC(=O)R, NRR', NO₂, C₁₋₆ alkyl, C₃₋₆ cycloalkyl or heterocyclic, wherein said alkyl, cycloalkyl or heterocyclic is substituted with 0-3 R^{5a}, wherein each R^{5a} is independently selected from OH, D, F, Cl, CN, CH₂F, CHF₂, CF₃, OCH₃, OCD₃, OCF₃ and OC(=O)CH₃;
   each of R⁶, R⁷, R⁹, R¹⁰ and R¹¹ is independently selected from H, F, Cl, CN, CD₃, CH₂CF₃, CF₃, OR, NRR', C₁₋₃ alkyl and C₃₋₅ cycloalkyl, wherein said alkyl, cycloalkyl, R and R' are substituted with 0-2 R^{2a};
   R^{2a} is selected from F, OCF₃, CF₃, CN, NO₂, OR, NRR' and C₁₋₆ alkyl; and
   each of R and R' independently H, C₁-C₆ alkyl or acyl, or R and R' together with the nitrogen or carbon atom to which they are bonded, form a 3- to 6-membered
   ring comprising 0-2 heteroatoms selected from O, NR, S and SO₂.
115. The compound of embodiment [114], wherein Y¹ is CH and Y² is CH.
116. The compound of embodiment [114], wherein Y¹ is N and Y² is CH.
117. The compound of embodiment [114], wherein Y¹ is CH and Y² is N.
118. The compound of embodiment [114], wherein Y¹ is N and Y² is N.
119. The compound of any one of embodiments [114]-[118], wherein Y³ is NH.
120. The compound of any one of embodiments [114]-[118], wherein Y³ is O.
121. The compound of any one of embodiments [114]-[118], wherein Y³ is CH₂.
122. The compound of any one of embodiments [114]-[118], wherein Y³ is CD₂.
123. The compound of any one of embodiments [114]-[122], wherein Y⁴ is NH.
124. The compound of any one of embodiments [114]-[122], wherein Y⁴ is CH₂.
125. The compound of any one of embodiments [114]-[122], wherein R¹ is CH₃.
126. The compound of any one of embodiments [114]-[122], wherein R¹ is CD₃.
127. The compound of any one of embodiments [114]-[126], wherein R⁴ is CH₃.
128. The compound of any one of embodiments [114]-[126], wherein R⁴ is CD₃.
129. A compound selected from **Table** 1 or a pharmaceutically acceptable form or an isotope derivative thereof.
130. A pharmaceutical composition comprising a compound according to any of embodiments [1]-[129], effective to treat or reduce one or more diseases or disorders, in a mammal, including a human, and a pharmaceutically acceptable excipient, carrier, or diluent.
131. The pharmaceutical composition of embodiment [130], being suitable for oral administration.
132. The pharmaceutical composition of embodiment [130], being suitable for topical administration.
133. The pharmaceutical composition of embodiment [130], being suitable for GI-restricted administration.
134. The pharmaceutical composition of any one of embodiments [130]-[133], being useful to treat or reduce one or more of inflammatory diseases, immune-mediated diseases and cancers, or a related disease or disorder.
135. The pharmaceutical composition of embodiment [134], wherein the disease or disorder is an inflammatory disease.
136. The pharmaceutical composition of embodiment [134], wherein the disease or disorder is an immune-mediated disease.
137. The pharmaceutical composition of embodiment [134], wherein the disease or disorder is a neuroinflammatory disease.
138. The pharmaceutical composition of embodiment [134], wherein the disease or disorder is cancer.
139. The pharmaceutical composition of embodiment [134], wherein the disease or disorder is selected from: inflammatory bowel disease, psoriasis, psoriatic arthritis, alopecia areata, eczema, ankylosing spondylitis (AS), vitiligo, atopic dermatitis, discoid lupus erythematosus (DLE), subacute cutaneous lupus erythematosus (SCLE), systemic lupus erythematosus (SLE), Sjogren's syndrome, scleroderma, Crohn's Disease (CD), rheumatoid arthritis (RA), T-cell acute lymphoblastic leukemia (T-ALL), cutaneous T-cell lymphomas (CTCL), multiple sclerosis (MS), Alzheimer's Disease (AD), Parkinson's Disease (PD), type I diabetes, asthma, kidney fibrosis, diabetic nephropathy, polycystic kidney disease, HIV-associated nephropathy, chronic myelogenous leukemia (CML), essential thrombocythemia (ET), polycythemia vera (PV), myelofibrosis (MF), breast cancer and ovarian cancer.
140. A unit dosage form comprising a pharmaceutical composition according to any of embodiments [130]-[139].
141. The unit dosage form of embodiment [140], being a tablet.
142. The unit dosage form of embodiment [140], being a capsule.
143. The unit dosage form of embodiment [140], being a topical formulation.
144. A method for treating, reducing or preventing a disease or disorder, comprising administering to a subject in need thereof a therapeutically effective amount of a compound according to any one of embodiments [1]-[129], wherein the disease or disorder is selected from inflammatory diseases, immune-mediated diseases, cancer, or a related disease or disorder thereof, in a mammal, including a human.
145. The method of embodiment [144], wherein the disease or disorder is an inflammatory disease.
146. The method of embodiment [144], wherein the disease or disorder is an immune-mediated disease.
147. The method of embodiment [144], wherein the disease or disorder is a neuroinflammatory disease.
148. The method of embodiment [144], wherein the disease or disorder is cancer.
149. The method of embodiment [144], wherein the disease or disorder is selected from: inflammatory bowel disease, psoriasis, psoriatic arthritis, alopecia areata, eczema, ankylosing spondylitis (AS), vitiligo, atopic dermatitis, discoid lupus erythematosus (DLE), subacute cutaneous lupus erythematosus (SCLE), systemic lupus erythematosus (SLE), Sjogren's syndrome, scleroderma, Crohn's Disease (CD), rheumatoid arthritis (RA), T-cell acute lymphoblastic leukemia (T-ALL), cutaneous T-cell lymphomas (CTCL), multiple sclerosis (MS), Alzheimer's Disease (AD), Parkinson's Disease (PD), type I diabetes, asthma, kidney fibrosis, diabetic nephropathy, polycystic kidney disease, HIV-associated nephropathy, chronic myelogenous leukemia (CML), essential thrombocythemia (ET), polycythemia vera (PV), myelofibrosis (MF), breast cancer and ovarian cancer.
150. The method of any of embodiments [144]-[149], wherein administration is via oral administration.
151. The method of any of embodiments [144]-[149], wherein administration is via topical administration.
152. The method of any of embodiments [144]-[149], wherein administration is via GI-restricted administration.
153. Use of a compound of any of embodiments [1]-[129], and a pharmaceutically acceptable excipient, carrier, or diluent, in preparation of a medicament for treating a disease or disorder.
154. The use of embodiment [153], wherein the disease or disorder is one or more of inflammatory diseases, immune-mediated diseases and cancer.
155. The use of embodiment [154], wherein the disease or disorder is an inflammatory disease.
156. The use of embodiment [154], wherein the disease or disorder is an immune-mediated disease.
157. The use of embodiment [154], wherein the disease or disorder is cancer.
158. The use of any one of embodiments [153]-[157], wherein the medicament is for oral administration.
159. The use of any one of embodiments [153]-[157], wherein the medicament is for topical administration.
160. The use of any one of embodiments [153]-[157], wherein the medicament is for GI restriction administration.
161. A method for preparing a compound of any one of embodiments [1]-[129].

## Claims

1. A compound having a structural formula selected from V20 and V22:
| | |
|---|---|
| V²⁰ | 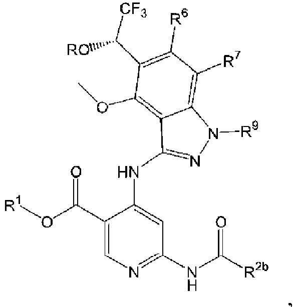 and |
| V²² | 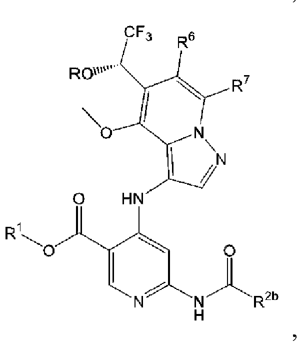 |
or a pharmaceutically acceptable salt thereof,
wherein
R¹ is H, CD₃, or C₁₋₃ alkyl;
R^{2b} is C₁₋₆ alkyl, C₃₋₆ cycloalkyl, C₅₋₇ spirocycloalkyl, aryl or heteroaryl, each substituted with 0-4 R^{2c};
R^{2c} at each occurrence is independently halo, CN, OR, NRR', OCF₃, CF₃, C₁₋₆ alkyl, C₁₋₆ haloalkyl, C₂₋₆ alkenyl, C₂₋₆ alkynyl, wherein said alkyl, haloalkyl, alkenyl, alkynyl, R and R' are substituted with 0-3 R^{2a};
each of R⁶, R⁷, and R⁹ is independently selected from H, F, Cl, CN, CD₃, CH₂CF₃, CF₃, OR, NRR', C₁₋₃ alkyl and C₃₋₅ cycloalkyl, wherein said alkyl, cycloalkyl, R and R' are substituted with 0-2 R^{2a};
R^{2a} is selected from the group consisting of halogen, CN, OR, NRR', alkyl, cycloalkyl and heterocycle;
R' is independently H or a C₁-C₆ alkyl or acyl, or R and R', together with the nitrogen atom to which they are bonded, form a 4- to 7-membered ring comprising 0-2 heteroatoms selected from O, NR, S and SO₂; and
R is independently H, CD₃, C₁-C₃ alkyl, or acyl.

2. The compound of claim 1, wherein R⁶ and R⁷ are H.

3. The compound of claim 2 having structural formula V20.

4. The compound of claim 2 having structural formula V20, wherein R⁹ is CH₃ or CD₃.

5. The compound of claim 2 having structural formula V22.

6. The compound of claim 1, wherein the compound is compound 42, or a pharmaceutically acceptable salt thereof:
| | | |
|---|---|---|
| 42 | 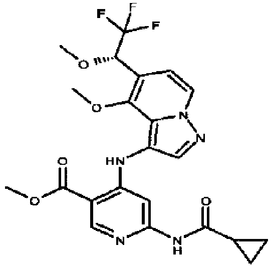 | Methyl (S)-6-(cyclopropanecarboxamido)-4-((4-methoxy-5-(2,2,2-trifluoro-1-methoxyethyl)pyrazolo[ 1,5-a]pyridin-3-yl)amino)nicotinate. |

7. The compound of claim 1, wherein the compound is compound 45B, or a pharmaceutically acceptable salt thereof:
| | | |
|---|---|---|
| 45B | 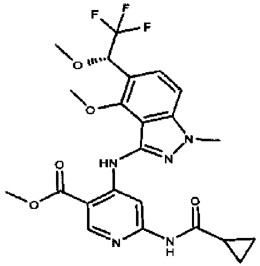 | Methyl (S)-6-(cyclopropanecarboxamido)-4-((4-methoxy-1-methyl-5-(2,2,2-trifluoro-1-methoxyethyl)-1H-indazol-3-yl)amino)nicotinate. |

8. The compound of claim 1, wherein the compound is compound 47, or a pharmaceutically acceptable salt thereof:
| | | |
|---|---|---|
| 47 | 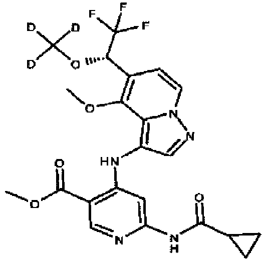 | Methyl (S)-6-( cyclopropanecarboxamido)-4-((4-methoxy-5-(2,2,2-trifluoro-1-(methoxy-d3)ethyl)pyrazolo[1,5-a]pyridin-3-yl)amino)nicotinate. |

9. The compound of claim 1, wherein the compound is compound 50B, or a pharmaceutically acceptable salt thereof:
| | | |
|---|---|---|
| 50B | 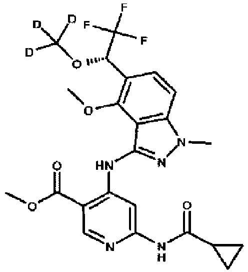 | Methyl (S)-6-( cyclopropanecarboxamido)-4-((4-methoxy-l-methyl-5-(2,2,2-trifluoro-1-(methoxy-d3)ethyl)-1H-indazol-3-yl)amino )nicotinate. |

10. The compound of claim 1, wherein the compound is compound 52A, or a pharmaceutically acceptable salt thereof:
| | | |
|---|---|---|
| 52A | 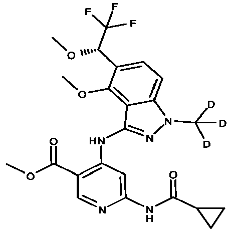 | Methyl (S)-6-(cyclopropanecarboxamido)-4-((4-methoxy-1-(methyl-d3)-5-(2,2,2-trifluoro-1-methoxyethyl)-1H-indazol-3-yl)amino)nicotinate. |

11. The compound of claim 1, wherein the compound is compound 53B, or a pharmaceutically acceptable salt thereof:
| | | |
|---|---|---|
| 53B | 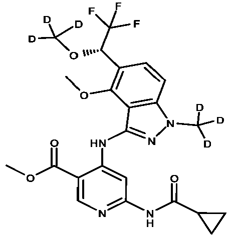 | Methyl (S)-6-(cyclopropanecarboxamido)-4-((4-methoxy-1-(methyl-d3)-5-(2,2,2-trifluoro-1-(methoxy-d3)ethyl)-1H-indazol-3-yl)amino)nicotinate. |

12. The compound of claim 1, wherein the compound is compound 120A, or a pharmaceutically acceptable salt thereof:
| | | |
|---|---|---|
| 120A | 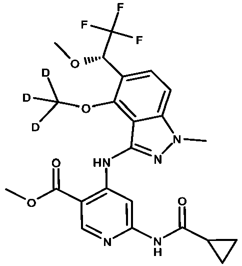 | Methyl (S)-6-(cyclopropanecarboxamido)-4-((4-(methoxy-d3)-1-methyl-5-(2,2,2-trifluoro-1-methoxyethyl)-1H-indazol-3-yl)amino)nicotinate. |

13. A pharmaceutical composition comprising a compound according to any of claims 1-12, or a pharmaceutically acceptable form or an isotope derivative thereof, and a pharmaceutically acceptable excipient, carrier, or diluent;
optionally wherein:
a) the pharmaceutical composition is suitable for oral administration; topical administration; or GI-restricted administration; and/or
b) the pharmaceutical composition is for use as a medicament to treat or reduce one or more diseases or disorders in a mammal, including a human;
further optionally wherein the pharmaceutical composition is for use in treating or reducing one or more of inflammatory diseases (such as neuroinflammatory disease), immune-mediated diseases and cancers, or a related disease or disorder,
yet further optionally wherein the disease or disorder is selected from: inflammatory bowel disease, psoriasis, psoriatic arthritis, alopecia areata, eczema, ankylosing spondylitis (AS), vitiligo, atopic dermatitis, discoid lupus erythematosus (DLE), subacute cutaneous lupus erythematosus (SCLE), systemic lupus erythematosus (SLE), Sjogren's syndrome, scleroderma, Crohn's Disease (CD), rheumatoid arthritis (RA), T-cell acute lymphoblastic leukemia (T-ALL), cutaneous T-cell lymphomas (CTCL), multiple sclerosis (MS), Alzheimer's Disease (AD), Parkinson's Disease (PD), type I diabetes, asthma, kidney fibrosis, diabetic nephropathy, polycystic kidney disease, HIV-associated nephropathy, chronic myelogenous leukemia (CML), essential thrombocythemia (ET), polycythemia vera (PV), myelofibrosis (MF), breast cancer and ovarian cancer.

14. A unit dosage form comprising a pharmaceutical composition according to claim 13, optionally wherein the unit dosage form is a tablet, a capsule, or a topical formulation.

15. A method for preparing a compound of any one of claims 1-12.
